(19) Europäisches Patentamt
European Patent Office
Office européen des brevets

(11) **EP 2 210 898 A1**

(12) **EUROPEAN PATENT APPLICATION**

(43) Date of publication:
**28.07.2010 Bulletin 2010/30**

(51) Int Cl.:
***C07K 14/32*** (2006.01)     ***C07K 14/195*** (2006.01)
***C12N 15/31*** (2006.01)

(21) Application number: **10159320.0**

(22) Date of filing: **29.11.2007**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **29.11.2006 US 861992 P**

(83) **Declaration under Rule 32(1) EPC (expert solution)**

(62) Document number(s) of the earlier application(s) in accordance with Art. 76 EPC:
**07853214.0 / 2 099 818**

(71) Applicant: **Novozymes Inc.**
**Davis, CA 95616 (US)**

(72) Inventor: **The designation of the inventor has not yet been filed**

(74) Representative: **Lindum, Peter Würtz**
**Novozymes A/S**
**Patents**
**Krogshøjvej 36**
**2880 Bagsværd (DK)**

Remarks:
This application was filed on 08-04-2010 as a divisional application to the application mentioned under INID code 62.

(54) **Bacillus Licheniformis chromosome**

(57) The present invention relates to an isolated polynucleotide of *Bacillus licheniformis* SJ1904 (ATCC PTA-7992) that encodes a biologically active substance and to nucleic acid constructs, vectors, and host cells comprising the polynucleotides as well as methods for producing biologically active substances encoded by the polynucleotides and to methods of using the isolated polynucleotide.

EP 2 210 898 A1

**Description**

**Reference to a Sequence Listing**

Incorporated herein by reference are 2 copies of the Sequence Listing on compact disk. Copy 1 is done on a Intel x86 machine format, in Windows XP operating system compatibility, there is one file saved as 01 10930.204-WO SeqList.txt, and is 20,210 KB, and was created on November 28, 2007. Copy 2 is identical to Copy 1. The content of the attached compact disks are the same and includes no new matter.

**Reference to a Deposit of Biological Material**

This application contains a reference to a deposit of biological material, which deposit is incorporated herein by reference.

**Field of the Invention**

The present invention relates to an isolated polynucleotide molecule comprising the complete chromosome of *Bacillus licheniformis* SJ1904. The present invention also relates to features (polynucleotides) of the complete chromosomal DNA molecule of *Bacillus licheniformis* SJ1904 that encode biologically active substances and to nucleic acid constructs, vectors, and host cells comprising the features. The present invention also relates to methods for producing biologically active substances encoded by the features and to methods of using the isolated features derived from the complete chromosomal DNA molecule of *Bacillus licheniformis* SJ 1904.

**Description of the Related Art**

Microbes have evolved for some 3.8 billion years and make up most of the earth's biomass. They are found in virtually every environment, surviving and thriving in extremes of heat, cold, radiation, pressure, salt, acidity, and darkness. Often in these environments, no other forms of life are found and the only nutrients come from inorganic matter. The diversity and range of their environmental adaptations indicate that microbes long ago "solved" many problems for which scientists are still actively seeking solutions. The value in determining the complete genome sequence of microbes is that it provides a detailed blueprint for the organism revealing biochemical pathways, substrates, intermediates, and end products as well as regulatory networks, and evolutionary relationships to other microbes. A complete manifest of proteins, both structural and catalytic, is encoded as a list of features in the DNA molecule comprising the genome, as well as their likely cellular location.

Knowledge about the enormous range of microbial capacities has broad and far-reaching implications for environmental, energy, health, and industrial applications, such as cleanup of toxic-waste, production of novel therapeutic and preventive agents (drugs and vaccines), energy generation and development of renewable energy sources, production of chemical catalysts, reagents, and enzymes to improve efficiency of industrial processes, management of environmental carbon, nitrogen and nutrient cycling, detection of disease-causing organisms, monitoring of the safety of food and water supplies, use of genetically altered bacteria as living sensors (biosensors) to detect harmful chemicals in soil, air, or water, and understanding of specialized systems used by microbial cells to live in natural environments.

*Bacillus licheniformis* is a gram positive spore-forming bacterium that is widely distributed as a saprophytic organism in the environment. Unlike most other bacilli that are predominantly aerobic, *Bacillus licheniformis* is a facultative anaerobe that may allow it to grow in additional ecological niches. This species produces a diverse assortment of extracellular enzymes that are believed to contribute to the process of nutrient cycling in nature (Claus, D. and Berkeley, R.C.W., 1986, In Bergey's Manual of Systematic Bacteriology, Vol. 2., eds. Sneath, P.H.A. et al., Williams and Wilkins Co., Baltimore, MD, pp. 1105-1139). Certain *Bacillus licheniformis* isolates are capable of denitrification but, the relevance of this characteristic to environmental denitrification may be small since the species generally persists in soil as endospores (Alexander, M., 1977, Introduction to Soil Microbiology. John Wiley and Sons, Inc., New York).

There are numerous industrial and agricultural uses for *Bacillus licheniformis* and its extracellular products. The species has been used for decades in the manufacture of industrial enzymes including several proteases, alpha-amylase, penicillinase, pentosanase, cycloglucosyltransferase, beta-mannanase, and several pectinolytic enzymes, owing largely to its ability to secrete sizeable amounts of degradative enzymes. *Bacillus licheniformis* is also used to produce peptide antibiotics such as bacitracin and proticin, in addition to a number of specialty chemicals such as citric acid, inosine, inosinic acid, and poly-gamma-glutamic acid. The proteases from *Bacillus licheniformis* are used in the detergent industry as well as for dehairing and batting of leather (Eveleigh, D.E., 1981, Scientific American 245, 155-178). Amylases from *Bacillus licheniformis* are deployed for the hydrolysis of starch, desizing of textiles, and sizing of paper (Erickson, R.J., 1976, In Microbiology, ed. Schlesinger, D. (Am. Soc. Microbiol., Washington, DC), pp. 406-419.). Certain strains of *Bacillus licheniformis* have shown efficacy to destroy fungal pathogens affecting maize, grasses, and vegetable crops (U.S. No. Patent 5,589,381; U.S. No. Patent 5,665,354). As an endospore-forming bacterium, the ability of the organism to survive under unfavorable environmental conditions may enhance its potential as a natural control agent.

*Bacillus licheniformis* can be differentiated from other bacilli on the basis of metabolic and physiological tests (Logan, N.A. and Berkeley, R.C.W., 1981, In The Aerobic Endospore-Forming Bacteria: Classification and Identification, eds. Berkeley, R.C.W. and Goodfellow, M., Academic Press, Inc., London, pp. 106-140; O'Donnell, A.G., Norris, J.R., Berkeley, R.C.W., Claus, D., Kanero, T., Logan, N.A., and Nozaki, R., 1980, Internat. J. Systematic Bacteriol. 30: 448-459). However, biochemical and phenotypic characteristics may be ambiguous among closely related species. Lapidus *et al.* (Lapidus, A., Galleron, N., Andersen, J.T., Jørgensen, P.L. Ehrlich, S.D., and Sorokin, A., 2002, FEMS Microbiol. Lett. 209: 23-30) constructed a physical map of the *Bacillus licheniformis* strain ATCC 14580 chromosome using a PCR approach, and established a number of regions of co-linearity where gene content and organization were conserved with the *Bacillus subtilis* chromosome. In addition, Rey *et al.* (Rey, M.W, Ramaiya, P, Nelson, B.A., Brody-Karpin, S.D., Zaretsky, E.J., Tang, M., Lopez de Leon, A., Xiang, H., Gusti, V., Clausen, I.G., Olsen, P.B., Rasmussen, M.D., Andersen, J.T., Jørgensen, P.L., Larsen, T.S., Sorokin, A., Bolotin, A., Lapidus, A., Galleron, N., Ehrlich, S.D., and Berka, R.M. 2004. Complete genome sequence of the industrial bacterium Bacillus licheniformis and comparisons with closely related Bacillus species. Genome Biol. 5: R77) published the complete genome sequence of the *Bacillus licheniformis* type strain ATCC 14580. They determined that the genome of *Bacillus licheniformis* ATCC 14580 comprises a circular chromosome of 4,222,336 base-pairs (bp) containing 4,208 predicted protein-coding genes with an average size of 873 bp, seven rRNA operons, and 72 tRNA genes. The *Bacillus licheniformis* ATCC 14580 chromosome contains large regions that are colinear with the genomes of *Bacillus subtilis* strain 168 and *Bacillus halodurans* strain C-125, and approximately 80% of the predicted *Bacillus licheniformis* ATCC 14580 coding sequences have *Bacillus subtilis* orthologs. However, despite the unmistakable organizational similarities between these *Bacillus licheniformis* and *Bacillus subtilis* genomes, there are notable differences in the numbers and locations of prophages, transposable elements and a number of extracellular enzymes and secondary metabolic pathway operons that distinguish these species. These differences include a region of more than 80 kilobases (kb) that comprises a cluster of polyketide synthase genes and a second operon of 38 kb encoding plipastatin synthase enzymes that are absent in the *Bacillus licheniformis* ATCC 14580 genome.

Public databases now contain a multitude of complete bacterial genomes, including several genomes from different strains of the same species. Recent analyses have shown, using pairwise whole genome alignments, that different strains of the same species may differ substantially in gene content. For example, genome comparisons of *Escherichia coli* strains CFT073, EDL933 and MG1655 revealed that only 39.2% of their combined set of proteins (gene products) are common to all three strains highlighting the astonishing diversity among strains of the same species (Welch et al., 2002, Proc. Nat. Acad. Sci. USA 99: 17020-17024; Perna et al., 2001, Nature 409: 529-533; Hayashi et al., 2001, DNA Res. 8: 11-22; Blattner et al., 1997, Science 277: 1453-1474). Furthermore, the genome sequence of E. *coli* strain CFT073 revealed 1,623 strain-specific genes (21.2%). From comparisons of this type, it is clearly seen that bacterial genomes are segmented into a common conserved backbone and strain-specific sequences. Typically the genome of a given strain within a species shows a mosaic structure in terms of the distribution of conserved "backbone" genes conserved among all strains and non-conserved genes that may have been acquired by horizontal transfer (Welch et al., 2002, Proc. Nat. Acad. Sci. USA 99: 17020-17024; Brzuszkiewicz et al., 2006, Proc. Nat. Acad. Sci. USA 103: 12879-12884).

Therefore, it would be advantageous to the art to have available the complete primary structure of the chromosomal DNA molecule of the *Bacillus licheniformis* strain SJ1904, an industrial strain that differs from the type strain ATCC 14580. With the complete chromosome data in hand, it should be possible to do comparative genomics and proteomics studies that can lead to improved industrial strains as well as to a better understanding of genome evolution among closely-related bacilli in the *subtilis-licheniformis* group.

The present invention relates to an isolated polynucleotide of the complete chromosome of *Bacillus licheniformis* strain SJ1904.

## Summary of the Invention

The present invention relates to an isolated polynucleotide of the complete chromosomal DNA molecule of *Bacillus licheniformis* SJ1904 (ATCC PTA-7992) having the nucleotide sequence of SEQ ID NO: 1.

The present invention also relates to isolated features (polynucleotides) of the complete chromosomal DNA molecule of *Bacillus licheniformis* SJ1904 encoding biologically active substances selected from the group consisting of SEQ ID NOs: 2-4876 and subsequences thereof encoding fragments having biological activity.

The present invention also relates to isolated features (polynucleotides) encoding biologically active substances, selected from the group consisting of:

(a) a polynucleotide comprising a nucleotide sequence having preferably at least 60% identity, more preferably at least 65% identity, more preferably at least 70% identity, more preferably at least 75% identity, more preferably at least 80% identity, more preferably at least 85% identity, more preferably at least 90% identity, even more preferably at least 90% identity, most preferably at least 95% identity, and even most preferably at least 97% identity with a polynucleotide selected from the group consisting of SEQ ID NOs: 2-4876 and subsequences thereof encoding fragments having biological activity;

(b) a polynucleotide comprising a nucleotide sequence that hybridizes under preferably at least medium stringency conditions, more preferably at least medium-high stringency conditions, or most preferably at least high stringency conditions with a polynucleotide selected from the group consisting of SEQ ID NOs: 2-4876 and full-length complementary strands thereof;

(c) a polynucleotide encoding a biologically active substance comprising an amino acid sequence having preferably at least 60% identity, more preferably at least 65% identity, more preferably at least 70% identity, more preferably at least 75% identity, more preferably at least 80% identity, more preferably at least 85% identity, even more preferably at least 90% identity, most preferably at least 95% identity, and even most preferably at least 97% identity with an amino acid sequence selected from the group consisting of SEQ ID NOs: 4877-9751 and fragments thereof retaining biological activity; and

(d) a polynucleotide encoding an artificial variant comprising a substitution, deletion, and/or insertion of one or more (several) amino acids of an amino acid sequence selected from the group consisting of SEQ ID NOs: 4877-9751 and fragments thereof retaining biological activity.

The present invention also relates to nucleic acid constructs, vectors, and host cells comprising the isolated polynucleotides.

The present invention also relates to isolated biologically active substances comprising an amino acid sequence selected from the group consisting of SEQ ID NOs: 4877-9751 and fragments thereof retaining biological activity.

The present invention also relates to isolated biologically active substances, selected from the group consisting of:

(a) a biologically active substance comprising an amino acid sequence having preferably at least 60% identity, more preferably at least 65% identity, more preferably at least 70% identity, more preferably at least 75% identity, more preferably at least 80% identity, more preferably at least 85% identity, even more preferably at least 90% identity, most preferably at least 95% identity, and even most preferably at least 97% identity with an amino acid sequence selected from the group consisting of SEQ ID NOs: 4877-9751 and fragments thereof retaining biological activity;

(b) a biologically active substance encoded by a polynucleotide comprising a nucleotide sequence having preferably at least 60% identity, more preferably at least 65% identity, more preferably at least 70% identity, more preferably at least 75% identity, more preferably at least 80% identity, more preferably at least 85% identity, even more preferably at least 90% identity, most preferably at least 95% identity, and even most preferably at least 97% identity with a polynucleotide selected from the group consisting of SEQ ID NOs: 2-4876 and subsequences thereof encoding fragments having biological activity;

(c) a biologically active substance encoded by a polynucleotide comprising a nucleotide sequence that hybridizes under preferably at least medium stringency conditions, more preferably at least medium-high stringency conditions, or most preferably at least high stringency conditions with a polynucleotide selected from the group consisting of SEQ ID NOs: 2-4876 and full-length complementary strands thereof; and

(d) an artificial variant comprising a substitution, deletion, and/or insertion of one or more (several) amino acids of an amino acid sequence selected from the group consisting of SEQ ID NOs: 4877-9751 and fragments thereof retaining biological activity.

The present invention also relates to methods for producing such substances having biological activity comprising (a) cultivating a recombinant host cell comprising a nucleic acid construct comprising a polynucleotide encoding the biologically active substance under conditions suitable for production of the biologically active substance; and (b) recovering the biologically active substance.

The present invention also relates to methods for monitoring differential expression of a plurality of genes in a first bacterial cell relative to expression of the same genes in one or more (several) second bacterial cells, comprising:

(a) adding a mixture of detection reporter-labeled nucleic acids isolated from the bacterial cells to a substrate containing an array of *Bacillus licheniformis* polynucleotides selected from the group consisting of SEQ ID NOs: 2-4876, full-length complementary strands of SEQ ID NOs: 2-4876, and fragments of SEQ ID NOs: 2-4876, under conditions where the detection reporter-labeled nucleic acids hybridize to complementary sequences of the *Bacillus licheniformis* polynucleotides on the array, wherein the nucleic acids from the first bacterial cell and the one or more (several) second bacterial cells are labeled with a first detection reporter and one or more (several) different second detection reporters, respectively; and

(b) examining the array under conditions wherein the relative expression of the genes in the bacterial cells is determined by the observed detection signal of each spot on the array in which (i) the *Bacillus licheniformis* polynucleotides on the array that hybridize to the nucleic acids obtained from either the first or the one or more (several) second bacterial cells produce a distinct first detection signal or one or more (several) second detection signals, respectively, and (ii) the *Bacillus licheniformis* polynucleotides on the array that hybridize to the nucleic acids obtained from both the first and one or more (several) second bacterial produce a distinct combined detection signal.

The present invention also relates to methods for isolating a polynucleotide encoding an enzyme, comprising:

(a) adding a mixture of labeled first nucleic acid probes, isolated from a microbial strain cultured on medium without an inducing substrate, and labeled second nucleic acid probes, isolated from the microbial strain cultured on medium with the inducing substrate, to an array of *Bacillus licheniformis* polynucleotides selected from the group consisting of the polynucleotides of SEQ ID NOs: 2-4876, full-length complementary strands of SEQ ID NOs: 2-4876, and fragments of SEQ ID NOs: 2-4876, under conditions where the labeled nucleic acid probes hybridize to complementary sequences of the *Bacillus licheniformis* polynucleotides on the array, wherein the first nucleic acid probes are labeled with a first reporter and the second nucleic acid probes are labeled with a second reporter;
(b) examining the array under conditions wherein the relative expression of the genes of the microbial strain is determined by the observed hybridization reporter signal of each spot on the array in which (i) the *Bacillus licheniformis* polynucleotides on the array that hybridize to the first nucleic acid probes produce a distinct first hybridization reporter signal or the second nucleic acid probes produce a distinct second hybridization reporter signal, and (ii) the *Bacillus licheniformis* polynucleotides on the array that hybridize to both the first and second nucleic acid probes produce a distinct combined hybridization reporter signal; and
(c) isolating a polynucleotide from the microbial strain that encodes an enzyme that degrades or converts the substrate.

The present invention also relates to genes or polynucleotides isolated by such methods and nucleic acid constructs, vectors, and host cells containing the isolated genes or polynucleotides.

## Definitions

**Biologically active substance:** The term "substance having biological activity" or "biologically active substance" is defined herein as any substance having biological activity encoded by a single gene or polynucleotide. Such substances include, but are not limited to, polypeptides (*e.g.*, enzymes) and RNA (*e.g.*, mRNA, tRNA, rRNA, and ncRNA). For purposes of the present invention, biological activity is determined according to procedures known in the art such as those described by Carpenter and Sabatini, 2004, Nature 5: 11-22; Sordie et al., 2003, Proceedings of the National Academy of Sciences USA 100: 11964-11969; Braun and LaBaer, 2003, TRENDS in Biotechnology 21: 383-388; and Kaberdin and McDowall, 2003, Genome Research 13: 1961-1965.

In a preferred aspect, the biologically active substance is a polypeptide. The polypeptide may be any polypeptide having a biological activity of interest. The term "polypeptide" is not meant herein to refer to a specific length of the encoded product and, therefore, encompasses peptides, oligopeptides, and proteins.

In a more preferred aspect, the polypeptide is an antibody, antigen, antimicrobial peptide, enzyme, growth factor, hormone, immunodilator, neurotransmitter, receptor, reporter protein, structural protein, transcription factor, and transporter.

In an even more preferred aspect, the polypeptide is an oxidoreductase, transferase, hydrolase, lyase, isomerase, or ligase.

In a most preferred aspect, the polypeptide is an aminopeptidase, amylase, carbohydrase, carboxypeptidase, catalase, cellulase, chitinase, cutinase, cyclodextrin glycosyltransferase, deoxyribonuclease, esterase, alpha-galactosidase, beta-galactosidase, glucoamylase, glucocerebrosidase, alpha-glucosidase, beta-glucosidase, haloperoxidase, invertase, laccase, lipase, mannosidase, mutanase, oxidase, pectinolytic enzyme, peptidoglutaminase, peroxidase, phospholipase, phytase, polyphenoloxidase, proteolytic enzyme, ribonuclease, transglutaminase, urokinase, or xylanase

In another more preferred aspect, the polypeptide is an albumin, collagen, tropoelastin, elastin, or gelatin.

**Isolated biologically active substance:** The term "isolated biologically active substance" is defined herein as a substance that is at least 1% pure, preferably at least 5% pure, more preferably at least 10% pure, more preferably at least 20% pure, more preferably at least 40% pure, more preferably at least 60% pure, even more preferably at least 80% pure, and most preferably at least 90% pure, as determined by SDS-PAGE, HPLC, capillary electrophoresis, or any other method used in the art.

**Substantially pure biologically active substance or pure biologically active substance**: The term "substantially pure biologically active substance" is defined herein as a biologically active substance preparation that contains at most 10%, preferably at most 8%, more preferably at most 6%, more preferably at most 5%, more preferably at most 4%, more preferably at most 3%, even more preferably at most 2%, most preferably at most 1%, and even most preferably at most 0.5% by weight of other material with which it is natively associated. It is, therefore, preferred that the substantially pure biologically active substance is at least 92% pure, preferably at least 94% pure, more preferably at least 95% pure, more preferably at least 96% pure, more preferably at least 96% pure, more preferably at least 97% pure, even more preferably at least 98% pure, most preferably at least 99%, and even most preferably at least 99.5% pure by weight of the total material present in the preparation. The term "pure biologically active substance" is defined as a biologically active substance preparation that contains no other material with which it is natively associated. The biologically active substances of the present invention are preferably in a substantially pure form. In particular, it is preferred that the biologically active substances are in "essentially pure form", *i.e.*, that the biologically active substance preparation is essentially free of other material with which it is natively or recombinantly associated. This can be accomplished, for example, by preparing the biologically active substance by means of well-known recombinant methods or by classical purification methods.

**Identity**: The relatedness between two amino acid sequences or between two nucleotide sequences is described by the parameter "identity".

For purposes of the present invention, the degree of sequence identity between two amino acid sequences is determined by the Smith-Waterman Protein method for the GENEMATCHER2™ as implemented by Paracel Inc. (Pasadena, CA), or the BLASTP method as described by Altschul et al., 1990, Journal of Molecular Biology 215: 403-410.

For purposes of the present invention, the degree of sequence identity between two nucleotide sequences is determined by the Smith Waterman nucleotide method for the GENEMATCHER2™ or BLASTN for the BlastMachine as implemented by Paracel Inc.

**Polypeptide Fragment:** The term "polypeptide fragment" is defined herein as a polypeptide, which retains biological activity, having one or more (several) amino acids deleted from the amino and/or carboxyl terminus of a polypeptide encoded by any of the polynucleotides of the present invention, *i.e.*, polypeptides of SEQ ID NOs: 4877-9751. Preferably, a fragment contains at least 80%, preferably at least 85%, more preferably at least 90%, even more preferably at least 95%, and most preferably at least 97% of the amino acid residues of the mature polypeptide product.

**Subsequence**: The term "subsequence" is defined herein as a polynucleotide comprising a nucleotide sequence of any of SEQ ID NOs: 2-4876 except that one or more (several) nucleotides have been deleted from the 5' and/or 3' end. Preferably, a subsequence contains at least 80%, preferably at least 85%, more preferably at least 90%, even more preferably at least 95%, and most preferably at least 97% of the nucleotides of any of the isolated polynucleotides of the present invention.

**Substantially pure polynucleotide or pure polynucleotide:** The term "substantially pure polynucleotide" as used herein refers to a polynucleotide preparation free of other extraneous or unwanted nucleotides and in a form suitable for use within genetically engineered protein production systems. Thus, a substantially pure polynucleotide contains at most 10%, preferably at most 8%, more preferably at most 6%, more preferably at most 5%, more preferably at most 4%, more preferably at most 3%, even more preferably at most 2%, most preferably at most 1%, and even most preferably at most 0.5% by weight of other polynucleotide material with which it is natively or recombinantly associated. A substantially pure polynucleotide may, however, include naturally occurring 5' and 3' untranslated regions, such as promoters and terminators. It is preferred that the substantially pure polynucleotide is at least 90% pure, preferably at least 92% pure, more preferably at least 94% pure, more preferably at least 95% pure, more preferably at least 96% pure, more preferably at least 97% pure, even more preferably at least 98% pure, most preferably at least 99%, and even most preferably at least 99.5% pure by weight. The polynucleotides of the present invention are preferably in a substantially pure form, *i.e.*, that the polynucleotide preparation is essentially free of other polynucleotide material with which it is natively or recombinantly associated. The polynucleotides may be of genomic, cDNA, RNA, semisynthetic, synthetic origin, or any combinations thereof. The term "pure polynucleotide" is defined as a polynucleotide preparation that contains no other material with which it is natively associated.

**Nucleic acid construct:** The term "nucleic acid construct" as used herein refers to a nucleic acid molecule, either single- or double-stranded, which is isolated from a naturally occurring gene or which is modified to contain segments of nucleic acids in a manner that would not otherwise exist in nature or which is synthetic. The term nucleic acid construct is synonymous with the term "expression cassette" when the nucleic acid construct contains the control sequences required for expression of a coding sequence of the present invention.

**Control sequence:** The term "control sequences" is defined herein to include all components, which are necessary or advantageous for the expression of a biologically active substance of the present invention. Each control sequence may be native or foreign to the polynucleotide encoding the substance. Such control sequences include, but are not limited to, a leader, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the polynucleotide encoding a biologically active substance.

**Operably linked:** The term "operably linked" as used herein refers to a configuration in which a control sequence is placed at an appropriate position relative to the coding sequence of the DNA sequence, such that the control sequence directs the expression of a biologically active substance.

**Coding sequence:** When used herein the term "coding sequence" is intended to cover a nucleotide sequence, which directly specifies the amino acid sequence of its protein product. The boundaries of the coding sequence are generally determined by an open reading frame, which usually begins with the ATG start codon or alternative start codons such as GTG and TTG.

**Expression:** The term "expression" includes any step involved in the production of a biologically active substance including, but not limited to, transcription, post-transcriptional modification, translation, post-translational modification, and secretion.

**Expression vector:** The term "expression vector" herein covers a DNA molecule, linear or circular, that comprises a segment encoding a biologically active substance of the invention, and is operably linked to additional segments that provide for its transcription.

**Host cell:** The term "host cell", as used herein, includes any cell type that is susceptible to transformation, transfection, conjugation, electroporation, etc. with a nucleic acid construct, plasmid, or vector.

**Modification:** The term "modification" means herein any chemical modification of a biological substance, *e.g.*, polypeptide, as well as genetic manipulation of the DNA encoding that biological substance. The modification can be a substitution, a deletion and/or an insertion of one or more (several) amino acids as well as replacements of one or more (several) amino acid side chains.

**Artificial variant:** When used herein, the term "artificial variant" means a polypeptide having biological activity produced by an organism expressing a modified nucleotide sequence, or the mature polypeptide coding region thereof. The modified nucleotide sequence is obtained through human intervention by modification of the nucleotide sequence disclosed or a homologous sequence thereof, or the mature polypeptide coding region thereof.

**Detailed Description of the Invention**

***Bacillus licheniformis* SJ1904 Chromosome and Features (Polynucleotides) Thereof**

The present invention relates to an isolated polynucleotide of the complete chromosomal DNA molecule of *Bacillus licheniformis* SJ1904 (ATCC PTA-7992) having the polynucleotide sequence of SEQ ID NO: 1. *Bacillus licheniformis* SJ1904 consists of a circular molecule of 4,345,159 base pairs with a mean %G+C content of 46.7%. The chromosome contains 4875 predicted protein-coding genes (SEQ ID NOs: 2-4876) with an average size of 789 bp, 7 rRNA operons, and 72 tRNA genes. The deduced amino acid sequences of the 4876 predicted protein-coding genes are shown in SEQ ID NOs: 4877-9851. SEQ ID NO: 4877 corresponds to SEQ ID NO: 2, SEQ ID NO: 4878 corresponds to SEQ ID NO: 3, SEQ ID NO: 4879 corresponds to SEQ ID NO: 4, etc. The predicted functions of the 4875 gene products are shown in Table 1.

The present invention also relates to isolated features (polynucleotides) of the complete chromosomal DNA molecule of *Bacillus licheniformis* SJ1904 encoding biologically active substances, selected from the group consisting of SEQ ID NOs: 2-4876 and subsequences thereof encoding fragments having biological activity.

The present invention also relates to isolated features (polynucleotides) encoding biologically active substances, selected from the group consisting of:

(a) a polynucleotide comprising a nucleotide sequence having preferably at least 60% identity, more preferably at least 65% identity, more preferably at least 70% identity, more preferably at least 75% identity, more preferably at least 80% identity, more preferably at least 85% identity, even more preferably at least 90% identity, most preferably at least 95% identity, and even most preferably at least 97% identity with a polynucleotide selected from the group consisting of SEQ ID NOs: 2-4876 and subsequences thereof encoding fragments having biological activity;

(b) a polynucleotide comprising a nucleotide sequence that hybridizes under preferably at least medium stringency conditions, more preferably at least medium-high stringency conditions, or most preferably at least high stringency conditions with a polynucleotide selected from the group consisting of SEQ ID NOs: 2-4876 and full-length complementary strands thereof;

(c) a polynucleotide encoding a biologically active substance comprising an amino acid sequence having preferably at least 60% identity, more preferably at least 65% identity, more preferably at least 70% identity, more preferably at least 75% identity, more preferably at least 80% identity, more preferably at least 85% identity, even more preferably at least 90% identity, most preferably at least 95% identity, and even most preferably at least 97% identity with an amino acid sequence selected from the group consisting of SEQ ID NOs: 4877-9751 and fragments thereof retaining biological activity; and

(d) a polynucleotide encoding an artificial variant comprising a substitution, deletion, and/or insertion of one or more (several) amino acids of an amino acid sequence selected from the group consisting of SEQ ID NOs: 4877-9751 and fragments thereof retaining biological activity.

In a first aspect, the present invention relates to an isolated polynucleotide having a degree of sequence identity to a polynucleotide selected from the group consisting of SEQ ID NOs: 2-4876 of at least 60%, preferably at least 65%, more preferably at least 70%, more preferably at least 75%, more preferably at least 80%, more preferably at least 85%, even more preferably at least 90%, most preferably at least 95%, and even most preferably at least 96%, 97%, 98%, or 99%, which encode biologically active substances having a particular biological activity (hereinafter "homologous biologically active substances").

In a preferred aspect, the present invention relates to an isolated polynucleotide comprising a nucleotide sequence having preferably at least 60% identity, more preferably at least 65% identity, more preferably at least 70% identity, more preferably at least 75% identity, more preferably at least 80% identity, more preferably at least 85% identity, even more preferably at least 90% identity, most preferably at least 95% identity, and even most preferably at least 97% identity with a polynucleotide selected from the group consisting of SEQ ID NOs: 50, 55, 77, 82, 91, 98, 103, 110, 125, 169, 171, 172, 174, 176, 177, 178, 179, 180, 181, 182, 183, 185, 186, 187, 189, 192, 194, 195, 196, 198, 199, 201, 203, 204, 205, 206, 207, 208, 209, 210,211,212,213,214,215,216,217,218,219,220,221,222,223,225,226,227,228,229, 230,240,241,242,243,244,245,246,247,248,249,250,251,252,253,254,255,256,257,　258,259,260,261,262,263,264, 265,266,287,306,308,322,323,329,350,354,360,363,　367,381,445,469,487,488,516,523,524,537,541,575,592,607, 621,627,628,666,672,　688,691,702,710,719,726,729,748,751,773,774,777,791,831,833,842,847,848,849, 850, 851, 855, 857, 885, 886, 887, 901, 944, 982, 991, 994, 1018, 1032, 1034, 1058, 1078, 1088, 1098, 1108, 1112, 1128, 1148, 1153, 1154, 1158, 1160, 1164, 1175, 1176, 1177, 1180, 1181, 1183, 1184, 1193, 1194, 1199, 1222, 1237, 1244, 1262, 1263, 1265, 1266, 1278, 1302, 1313,1314,1315,1316,1317,1318,1345,1355,1373,1374,1401,1424,1432,1433,1440, 1442, 1448, 1455, 1470, 1496, 1497, 1501, 1509, 1527, 1539, 1556, 1565, 1589, 1619, 1629, 1630, 1634, 1635, 1636, 1638, 1639, 1640, 1641, 1642, 1643, 1644, 1645, 1646, 1647, 1649, 1650, 1651, 1652, 1653, 1654, 1655, 1657, 1658, 1659, 1660, 1661, 1662, 1663, 1664, 1665, 1666, 1667, 1668, 1669, 1670, 1671, 1672, 1673, 1675, 1676, 1677, 1679, 1684, 1688, 1692, 1694, 1703, 1704, 1728, 1732, 1734, 1736, 1775, 1783, 1816, 1831, 1838, 1851, 1853, 1866, 1887,2002,2007,2022,　2038,2044,2067,2070,2072,2076,2094,2106,2119,2120,2121,　2124,2131,2139,2147,2 148, 2166,2171,2194,2196,　2198,2205,2211,2213,2216,2221,　2230,2236,2240,2241,2255,2258,2263,2273,2277,　2286, 2288,2289,2290,2291,　2292,　2293,2294,2295,2296,2297,2298,2299,2300,2301,2302,2303,2304,2305,2324,　2327, 2337,2341,2347,2351,　2364,2381,2388,2400,2403,2408,2410,2411,2414,2415,2424,　2425,2430,2435,2439, 2457,2467,2468,2469,2483,　2491,2498,2504,2505,2506,2507,　2508,2509,2510,2511,2512,2514,2515,2516,2517, 2518,2519,2520,2521,2522,　2524,　2525,2526,2527,2528,2529,2530,2531,2532,2533,2534,2535,2536,2537,2538, 2539, 2540,2541,2542,2543,　2544,2545,2546,2547,2548,2549,2550,2551,2552,2553,2554,　2555,2556,2557,　2558, 2559,2560,2561,2562,2564,　2565,2566,2567,2568,2569,2570,　2571,2572,2573,2574,2575,2576,2577,2578,　2579, 2580,2581,2582,2583,2584,　2585,　2586,2587,2588,2590,2591,2592,2593,2594,2595,2596,2597,2598,2599,　2600, 2601, 2602, 2603, 2604, 2605, 2606, 2607, 2608, 2609, 2610, 2611, 2612, 2613, 2614, 2615, 2616, 2617, 2618, 2619, 2620, 2621, 2622, 2623, 2624, 2625, 2626, 2627, 2628, 2629, 2630, 2631, 2632, 2633, 2634, 2635, 2636, 2637, 2638, 2639, 2640, 2641, 2642, 2643, 2644, 2645, 2646, 2647,2648,2649,2650,2651,2652,2653,2654,2655,　2656, 2657,2658,2659,2661,2664,　2665,2666,　2667,2669,2670,2671,2672,2673,2674,2675,2676,2677,2679,　2680,2681, 2684,2685,2686,2688,2689,2690,2691,　2692,2693,2696,2697,2698,2706,2723,2730,　2733,2751,2762,　2795,2806, 2813,2834,2835,2841,2850,2866,2867,　2874,2878,2898, 2902, 2904, 2917, 2947, 2957, 2960, 2969, 2972, 2973, 2999, 3004, 3019, 3028, 3054, 3064, 3082, 3093, 3123, 3133, 3134, 3135, 3136, 3137, 3138, 3139, 3140, 3141, 3142, 3143, 3144, 3145, 3146, 3147, 3148, 3149, 3150, 3162, 3222, 3224, 3225, 3226, 3227, 3228, 3229, 3231, 3244, 3248, 3259, 3264, 3265, 3287, 3291, 3303, 3310, 3311, 3321, 3324, 3355, 3362, 3371, 3429, 3434, 3442, 3446, 3458, 3471, 3486, 3496, 3499, 3503, 3504, 3508, 3549, 3558, 3577, 3593, 3598, 3601, 3603, 3606, 3611, 3642, 3653, 3655, 3670, 3673, 3682, 3690, 3694, 3707, 3716, 3727, 3736, 3737, 3744, 3753, 3765, 3790, 3798, 3799,, 3872, 3881, 3885, 3915, 3916, 3922, 3931, 3938, 3969, 4011, 4017, 4056, 4059, 4068, 4076, 4077, 4081, 4082, 4085, 4090, 4106, 4112, 4117, 4144, 4145, 4158, 4159, 4160, 4161, 4162, 4163, 4164, 4165, 4166, 4167, 4168, 4169, 4170, 4171, 4172, 4173, 4174, 4175, 4176, 4179, 4181, 4182, 4201, 4235, 4285, 4288, 4305, 4323, 4333, 4336, 4353, 4398, 4409, 4417, 4421, 4423, 4429, 4435, 4448, 4450, 4469, 4472, 4476, 4482, 4488, 4513, 4518, 4544, 4559, 4560, 4569, 4571, 4578, 4607, 4622, 4631, 4643, 4647, 4648, 4650, 4658, 4669, 4672, 4682, 4687, 4688, 4704, 4718, 4754, 4767, 4786, 4792, 4797, 4804, 4812, 4814, 4823, 4830, 4833, 4838, 4848, 4849, 4859, 4862, and 4872; preferably at least 75% identity, more preferably at least 80% identity, more preferably at least 85% identity, even more preferably at least 90% identity, most preferably at least 95% identity, and even most preferably at least 97% identity with a polynucleotide selected from the group consisting of SEQ ID NOs: 909, 911, 1367, 1498, 2663, 2668, 2682, and 2683; preferably at least 80% identity, more preferably at least 85% identity, even more preferably at least 90% identity, most preferably at least 95% identity, and even most preferably at least 97% identity with a polynucleotide selected from the group consisting of SEQ ID NOs: 167, 654, 2513, 2589, and 2694; preferably at least 85% identity, more preferably at least 90% identity, most preferably at least 95% identity, and even most preferably at least 97% identity with a polynucleotide selected from the group consisting of SEQ ID NOs: 163, 184, 910, 1674, 2412, 2660, 2662, 2695, and 3231; preferably at least 90% identity, more preferably at least 95% identity, and most preferably at least 97% identity with a polynucleotide selected from the group consisting of SEQ ID NOs: 27, 166, 168, 170, 193, 202, 231, 543, 776, 888, 889, 890, 898, 908, 916, 1028, 1123, 1133, 1155, 1156, 1157, 1173, 1211, 1212, 1218, 1229, 1230, 1277, 1356, 1357, 1359, 1360, 1368, 1372, 1391, 1500, 1584, 1604, 1627, 1631, 1632, 1637, 1648, 1680, 2101, 2102, 2239, 2358, 2359, 2360, 2404, 2417, 2429, 2494, 2563, 2678, 2724, 3111, 3539, 3850, 4078, 4079, 4080, 4083, 4084, 4180, 4221, 4642, 4689, 4707, and 4727; or preferably at least 95% identity and more preferably at least 97% identity with a polynucleotide selected from the group consisting of SEQ ID NOs: 2, 3, 4, 5, 6, 7, 8, 9, 10, 11, 12, 13, 14, 15, 16, 17, 18, 19, 20, 21, 22, 23, 24, 25, 26, 28, 29, 30, 31, 32, 33, 34, 35, 36, 37, 38, 39, 40, 41, 42, 43, 44, 45, 46, 47, 48, 49, 51, 52, 53, 54, 56, 57, 58, 59, 60, 61, 62, 63, 64, 65, 66, 67, 68, 69, 70, 71, 72, 73, 74, 75, 76, 78, 79, 80, 81, 83, 84, 85, 86, 87, 88, 89, 90, 92, 93, 94, 95, 96, 97, 99, 100, 101, 102, 104, 105, 106, 107, 108, 109, 111, 112, 113, 114, 115, 116, 117, 118, 119, 120, 121, 122, 123, 124, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 164, 165, 173, 175, 188, 190, 191, 197, 200, 224, 232, 233, 234, 235, 236, 237, 238, 239, 267, 268, 269, 270, 271, 272, 273, 274, 275, 276, 277, 278, 279, 280, 281, 282, 283, 284, 285, 286, 288, 289, 290, 291, 292, 293, 294, 295, 296, 297, 298, 299, 300, 301, 302, 303, 304, 305, 307, 309, 310, 311, 312, 313, 314, 315, 316, 317, 318, 319, 320, 321, 324, 325, 326, 327, 328, 330, 331, 332, 333, 334, 335, 336, 337, 338, 339, 340, 341, 342, 343, 344, 345, 346, 347, 348, 349, 351, 352, 353, 355, 356, 357, 358, 359, 361, 362, 364, 365, 366, 368, 369, 370, 371, 372, 373, 374, 375, 376, 377, 378, 379, 380, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 446, 447, 448, 449, 450, 451, 452, 453, 454, 455, 456, 457, 458, 459, 460, 461, 462, 463, 464, 465, 466, 467, 468, 470, 471, 472, 473, 474, 475, 476, 477, 478, 479, 480, 481, 482, 483, 484, 485, 486, 489, 490, 491, 492, 493, 494, 495, 496, 497, 498, 499, 500, 501, 502, 503, 504, 505, 506, 507, 508, 509, 510, 511, 512, 513, 514, 515, 517, 518, 519, 520, 521, 522, 525, 526, 527, 528, 529, 530, 531, 532, 533, 534, 535, 536, 538, 539, 540, 542, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 576, 577, 578, 579, 580, 581, 582, 583, 584, 585, 586, 587, 588, 589, 590, 591, 593, 594, 595, 596, 597, 598, 599, 600, 601, 602, 603, 604, 605, 606, 608, 609, 610, 611, 612, 613, 614, 615, 616, 617, 618, 619, 620, 622, 623, 624, 625, 626, 629, 630, 631, 632, 633, 634, 635, 636, 637, 638, 639, 640, 641, 642, 643, 644, 645, 646, 647, 648, 649, 650, 651, 652, 653, 655, 656, 657, 658, 659, 660, 661, 662, 663, 664, 665, 667, 668, 669, 670, 671, 673, 674, 675, 676, 677, 678, 679, 680, 681, 682, 683, 684, 685, 686, 687, 689, 690, 692, 693, 694, 695, 696, 697, 698, 699, 700, 701, 703, 704, 705, 706, 707, 708, 709, 711, 712, 713, 714, 715, 716, 717, 718, 720, 721, 722, 723, 724, 725, 727, 728, 730, 731, 732, 733, 734, 735, 736, 737, 738, 739, 740, 741, 742, 743, 744, 745, 746, 747, 749, 750, 752, 753, 754, 755, 756, 757, 758, 759, 760, 761, 762, 763, 764, 765, 766, 767, 768, 769, 770, 771, 772, 775, 778, 779, 780, 781, 782, 783, 784, 785, 786, 787, 788, 789, 790, 792, 793, 794, 795, 796, 797, 798, 799, 800, 801, 802, 803, 804, 805, 806, 807, 808, 809, 810, 811, 812, 813, 814, 815, 816, 817, 818, 819, 820, 821, 822, 823, 824, 825, 826, 827, 828, 829, 830, 832, 834, 835, 836, 837, 838, 839, 840, 841, 843, 844, 845, 846, 852, 853, 854, 856, 858, 859, 860, 861, 862, 863, 864, 865, 866, 867, 868, 869, 870, 871, 872, 873, 874, 875, 876, 877, 878, 879, 880, 881, 882, 883, 884, 891, 892, 893, 894, 895, 896, 897, 899, 900, 902, 903, 904, 905, 906, 907, 912, 913, 914, 915, 917, 918, 919, 920, 921, 922, 923, 924, 925, 926, 927, 928, 929, 930, 931, 932, 933, 934, 935, 936, 937, 938, 939, 940, 941, 942, 943, 945, 946, 947, 948, 949, 950, 951, 952, 953, 954, 955, 956, 957, 958, 959, 960, 961, 962, 963, 964, 965, 966, 967, 968, 969, 970, 971, 972, 973, 974, 975, 976, 977, 978, 979, 980, 981, 983, 984, 985, 986, 987, 988, 989, 990, 992, 993, 995, 996, 997, 998, 999, 1000, 1001, 1002, 1003, 1004, 1005, 1006, 1007, 1008, 1009, 1010, 1011, 1012, 1013, 1014, 1015, 1016, 1017, 1019, 1020, 1021, 1022, 1023, 1024, 1025, 1026, 1027, 1029, 1030, 1031, 1033, 1035, 1036, 1037, 1038, 1039, 1040, 1041, 1042, 1043, 1044, 1045, 1046, 1047, 1048, 1049, 1050, 1051, 1052, 1053, 1054, 1055, 1056, 1057, 1059, 1060, 1061, 1062, 1063, 1064, 1065, 1066, 1067, 1068, 1069, 1070, 1071, 1072, 1073, 1074, 1075, 1076, 1077, 1079, 1080, 1081, 1082, 1083, 1084, 1085, 1086, 1087, 1089, 1090, 1091, 1092, 1093, 1094, 1095, 1096, 1097, 1099, 1100, 1101, 1102, 1103, 1104, 1105, 1106, 1107, 1109, 1110, 1111, 1113, 1114, 1115, 1116, 1117, 1118, 1119, 1120, 1121, 1122, 1124, 1125, 1126, 1127, 1129, 1130, 1131, 1132, 1134, 1135, 1136, 1137, 1138, 1139, 1140, 1141, 1142, 1143, 1144, 1145, 1146, 1147, 1149, 1150, 1151, 1152, 1159, 1161, 1162, 1163, 1165, 1166, 1167, 1168, 1169, 1170, 1171, 1172, 1174, 1178, 1179, 1182, 1185, 1186, 1187, 1188, 1189, 1190, 1191, 1192, 1195, 1196, 1197, 1198, 1200, 1201, 1202, 1203, 1204, 1205, 1206, 1207, 1208, 1209, 1210, 1213, 1214, 1215, 1216, 1217, 1219, 1220, 1221, 1223, 1224, 1225, 1226, 1227, 1228, 1231, 1232, 1233, 1234, 1235, 1236, 1238, 1239, 1240, 1241, 1242, 1243, 1245, 1246, 1247, 1248, 1249, 1250, 1251, 1252, 1253, 1254, 1255, 1256, 1257, 1258, 1259, 1260, 1261, 1264, 1267, 1268, 1269, 1270, 1271, 1272, 1273, 1274, 1275, 1276, 1279, 1280, 1281, 1282, 1283, 1284, 1285, 1286, 1287, 1288, 1289, 1290, 1291, 1292, 1293, 1294, 1295, 1296, 1297, 1298, 1299, 1300, 1301, 1303, 1304, 1305, 1306, 1307, 1308, 1309, 1310, 1311, 1312, 1319, 1320, 1321, 1322, 1323, 1324, 1325, 1326, 1327, 1328, 1329, 1330, 1331, 1332, 1333, 1334, 1335, 1336, 1337, 1338, 1339, 1340, 1341, 1342, 1343, 1344, 1346, 1347, 1348, 1349, 1350, 1351, 1352, 1353, 1354, 1358, 1361, 1362, 1363, 1364, 1365, 1366, 1369, 1370, 1371, 1375, 1376, 1377, 1378, 1379, 1380, 1381, 1382, 1383, 1384, 1385, 1386, 1387, 1388, 1389, 1390, 1392, 1393, 1394, 1395, 1396, 1397, 1398, 1399, 1400, 1402, 1403, 1404, 1405, 1406, 1407, 1408, 1409, 1410, 1411, 1412, 1413, 1414, 1415, 1416, 1417, 1418, 1419, 1420, 1421, 1422, 1423, 1425, 1426, 1427, 1428, 1429, 1430, 1431, 1434, 1435, 1436, 1437, 1438, 1439, 1441, 1443, 1444, 1445, 1446, 1447, 1449, 1450, 1451, 1452, 1453, 1454, 1456, 1457, 1458, 1459, 1460, 1461, 1462, 1463, 1464, 1465, 1466, 1467, 1468, 1469, 1471, 1472, 1473, 1474, 1475, 1476, 1477, 1478, 1479, 1480, 1481, 1482, 1483, 1484, 1485, 1486, 1487, 1488, 1489, 1490, 1491, 1492, 1493, 1494, 1495, 1499, 1502, 1503, 1504, 1505, 1506, 1507, 1508, 1510, 1511, 1512, 1513, 1514, 1515, 1516, 1517, 1518, 1519, 1520, 1521, 1522, 1523, 1524, 1525, 1526, 1528, 1529, 1530, 1531, 1532, 1533, 1534, 1535, 1536, 1537, 1538, 1540, 1541, 1542, 1543, 1544, 1545, 1546, 1547, 1548, 1549, 1550, 1551, 1552, 1553, 1554, 1555, 1557, 1558, 1559, 1560, 1561, 1562, 1563, 1564, 1566, 1567, 1568, 1569, 1570, 1571, 1572, 1573, 1574, 1575, 1576, 1577, 1578, 1579, 1580, 1581, 1582, 1583, 1585, 1586, 1587, 1588, 1590, 1591, 1592, 1593, 1594, 1595, 1596, 1597, 1598, 1599, 1600, 1601, 1602, 1603, 1605, 1606, 1607, 1608, 1609, 1610, 1611, 1612, 1613, 1614, 1615, 1616, 1617, 1618, 1620, 1621, 1622, 1623, 1624, 1625, 1626, 1628, 1633, 1656, 1678, 1681, 1682, 1683, 1685, 1686, 1687, 1689, 1690, 1691, 1693, 1695, 1696, 1697, 1698, 1699, 1700, 1701, 1702, 1705, 1706, 1707, 1708, 1709, 1710, 1711, 1712, 1713, 1714, 1715, 1716, 1717, 1718, 1719, 1720, 1721, 1722, 1723, 1724, 1725, 1726, 1727, 1729, 1730, 1731, 1733, 1735, 1737, 1738, 1739, 1740, 1741, 1742, 1743, 1744, 1745, 1746, 1747, 1748, 1749, 1750, 1751, 1752, 1753, 1754, 1755, 1756, 1757, 1758, 1759, 1760, 1761, 1762, 1763, 1764, 1765, 1766, 1767, 1768, 1769, 1770, 1771, 1772, 1773, 1774, 1776, 1777, 1778, 1779, 1780, 1781, 1782, 1784, 1785, 1786, 1787, 1788, 1789, 1790, 1791, 1792, 1793, 1794, 1795, 1796, 1797, 1798, 1799, 1800, 1801, 1802, 1803, 1804, 1805, 1806, 1807, 1808, 1809, 1810, 1811, 1812, 1813, 1814, 1815, 1817, 1818, 1819, 1820, 1821, 1822, 1823, 1824, 1825, 1826, 1827, 1828, 1829, 1830, 1832, 1833, 1834, 1835, 1836, 1837, 1839, 1840, 1841, 1842, 1843, 1844, 1845, 1846, 1847, 1848, 1849, 1850, 1852, 1854, 1855, 1856, 1857, 1858, 1859, 1860, 1861, 1862, 1863, 1864, 1865, 1867, 1868, 1869, 1870, 1871, 1872, 1873, 1874, 1875, 1876, 1877, 1878, 1879, 1880, 1881, 1882, 1883, 1884, 1885, 1886, 1888, 1889, 1890, 1891, 1892, 1893, 1894, 1895, 1896, 1897, 1898, 1899, 1900, 1901, 1902, 1903, 1904, 1905, 1906, 1907, 1908, 1909, 1910, 1911, 1912, 1913, 1914, 1915, 1916, 1917, 1918, 1919, 1920, 1921, 1922, 1923, 1924, 1925, 1926, 1927, 1928, 1929, 1930, 1931, 1932, 1933, 1934, 1935, 1936, 1937, 1938, 1939, 1940, 1941, 1942, 1943, 1944, 1945, 1946, 1947, 1948, 1949, 1950, 1951, 1952, 1953, 1954, 1955, 1956, 1957, 1958, 1959, 1960, 1961, 1962, 1963, 1964, 1965, 1966, 1967, 1968, 1969, 1970, 1971, 1972, 1973, 1974, 1975, 1976, 1977, 1978, 1979, 1980, 1981, 1982, 1983, 1984, 1985, 1986, 1987, 1988, 1989, 1990, 1991, 1992, 1993, 1994, 1995, 1996, 1997, 1998, 1999, 2000, 2001, 2003, 2004, 2005, 2006, 2008, 2009, 2010, 2011, 2012, 2013, 2014, 2015, 2016, 2017, 2018, 2019, 2020, 2021, 2023, 2024, 2025, 2026, 2027, 2028, 2029, 2030, 2031, 2032, 2033, 2034, 2035, 2036, 2037, 2039, 2040, 2041, 2042, 2043, 2045, 2046, 2047, 2048, 2049, 2050, 2051, 2052, 2053, 2054, 2055, 2056, 2057, 2058, 2059, 2060, 2061, 2062, 2063, 2064, 2065, 2066, 2068, 2069, 2071, 2073, 2074, 2075, 2077, 2078, 2079, 2080, 2081, 2082, 2083, 2084, 2085, 2086, 2087, 2088, 2089, 2090, 2091, 2092, 2093, 2095, 2096, 2097, 2098, 2099, 2100, 2103, 2104, 2105, 2107, 2108, 2109, 2110, 2111, 2112, 2113, 2114, 2115, 2116, 2117, 2118, 2122, 2123, 2125, 2126, 2127, 2128, 2129, 2130, 2132, 2133, 2134, 2135, 2136, 2137, 2138, 2140, 2141, 2142, 2143, 2144, 2145, 2146, 2149, 2150, 2151, 2152, 2153, 2154, 2155, 2156, 2157, 2158, 2159, 2160, 2161, 2162, 2163, 2164, 2165, 2167, 2168, 2169, 2170, 2172, 2173, 2174, 2175, 2176, 2177, 2178, 2179, 2180, 2181, 2182, 2183, 2184, 2185, 2186, 2187, 2188, 2189, 2190, 2191, 2192, 2193, 2195, 2197, 2199, 2200, 2201, 2202, 2203, 2204, 2206, 2207, 2208, 2209, 2210, 2212, 2214, 2215, 2217, 2218, 2219, 2220, 2222, 2223, 2224, 2225, 2226, 2227, 2228, 2229, 2231, 2232, 2233, 2234, 2235, 2237, 2238, 2242, 2243, 2244, 2245, 2246, 2247, 2248, 2249, 2250, 2251, 2252, 2253, 2254, 2256, 2257, 2259, 2260, 2261, 2262, 2264, 2265, 2266, 2267, 2268, 2269, 2270, 2271, 2272, 2274, 2275, 2276, 2278, 2279, 2280, 2281, 2282, 2283, 2284, 2285, 2287, 2306, 2307, 2308, 2309, 2310, 2311, 2312, 2313, 2314, 3231, 2316, 2317, 2318, 2319, 2320, 2321, 2322, 2323, 2325, 2326, 2328, 2329, 2330, 2331, 2332, 2333, 2334, 2335, 2336, 2338, 2339, 2340, 2342, 2343, 2344, 2345, 2346, 2348, 2349, 2350, 2352, 2353, 2354, 2355, 2356, 2357, 2361, 2362, 2363, 2365, 2366, 2367, 2368, 2369, 2370, 2371, 2372, 2373, 2374, 2375, 2376, 2377, 2378, 2379, 2380, 2382, 2383, 2384, 2385, 2386, 2387, 2389, 2390, 2391, 2392, 2393, 2394, 2395, 2396, 2397, 2398, 2399, 2401, 2402, 2405, 2406, 2407, 2409, 2413, 2416, 2418, 2419, 2420, 2421, 2422, 2423, 2426, 2427, 2428, 2431, 2432, 2433, 2434, 2436, 2437, 2438, 2440, 2441, 2442, 2443, 2444, 2445, 2446, 2447, 2448, 2449, 2450, 2451, 2452, 2453, 2454, 2455, 2456, 2458, 2459, 2460, 2461, 2462, 2463, 2464, 2465, 2466, 2470, 2471, 2472, 2473, 2474, 2475, 2476, 2477, 2478, 2479, 2480, 2481, 2482, 2484, 2485, 2486, 2487, 2488, 2489, 2490, 2492, 2493, 2495, 2496, 2497, 2499, 2500, 2501, 2502, 2503, 2523, 2687, 3231, 2700, 2701, 2702, 2703, 2704, 2705, 2707, 2708, 2709, 2710, 2711, 2712, 2713, 2714, 2715, 2716, 2717, 2718, 2719, 2720, 2721, 2722, 2725, 2726, 2727, 2728, 2729, 2731, 2732, 2734, 2735, 2736, 2737, 2738, 2739, 2740, 2741, 2742, 2743, 2744, 2745, 2746, 2747, 2748, 2749, 2750, 2752, 2753, 2754, 2755, 2756, 2757, 2758, 2759, 2760, 2761, 2763, 2764, 2765, 2766, 2767, 2768, 2769, 2770, 2771, 2772, 2773, 2774, 2775, 2776, 2777, 2778, 2779, 2780, 2781, 2782, 2783, 2784, 2785, 2786, 2787, 2788, 2789, 2790, 2791, 2792, 2793, 2794, 2796, 2797, 2798, 2799, 2800, 2801, 2802, 2803, 2804, 2805, 2807, 2808, 2809, 2810, 2811, 2812, 2814, 2815, 2816, 2817, 2818, 2819, 2820, 2821, 2822, 2823, 2824, 2825, 2826, 2827, 2828, 2829, 2830, 2831, 2832, 2833, 2836, 2837, 2838, 2839, 2840, 2842, 2843, 2844, 2845, 2846, 2847, 2848, 2849, 2851, 2852, 2853, 2854, 2855, 2856, 2857, 2858, 2859, 2860, 2861, 2862, 2863, 2864, 2865, 2868, 2869, 2870, 2871, 2872, 2873, 2875, 2876, 2877, 2879, 2880, 2881, 2882, 2883, 2884, 2885, 2886, 2887, 2888, 2889, 2890, 2891, 2892, 2893, 2894, 2895, 2896, 2897, 2899, 2900, 2901, 2903, 2905, 2906, 2907, 2908, 2909, 2910, 2911, 2912, 2913, 2914, 2915, 2916, 2918, 2919, 2920, 2921, 2922, 2923, 2924, 2925, 2926, 2927, 2928, 2929, 2930, 2931, 2932, 2933, 2934, 2935, 2936, 2937, 2938, 2939, 2940, 2941, 2942, 2943, 2944, 2945, 2946, 2948, 2949, 2950, 2951, 2952, 2953, 2954, 2955, 2956, 2958, 2959, 2961, 2962, 2963, 2964, 2965, 2966, 2967, 2968, 2970, 2971, 2974, 2975, 2976, 2977, 2978, 2979, 2980, 2981, 2982, 2983, 2984, 2985, 2986, 2987, 2988, 2989, 2990, 2991, 2992, 2993, 2994, 2995, 2996, 2997, 2998, 3000, 3001, 3002, 3003, 3005, 3006, 3007, 3008, 3009, 3010, 3011, 3012, 3013, 3014, 3015, 3016, 3017, 3018, 3020, 3021, 3022, 3023, 3024, 3025, 3026, 3027, 3029, 3030, 3031, 3032, 3033, 3034, 3035, 3036, 3037, 3038, 3039, 3040, 3041, 3042, 3043, 3044, 3045, 3046, 3047, 3048, 3049, 3050, 3051, 3052, 3053, 3055, 3056, 3057, 3058, 3059, 3060, 3061, 3062, 3063, 3065, 3066, 3067, 3068, 3069, 3070, 3071, 3072, 3073, 3074, 3075, 3076, 3077, 3078, 3079, 3080, 3081, 3083, 3084, 3085, 3086, 3087, 3088, 3089, 3090, 3091, 3092, 3094, 3095, 3096, 3097, 3098, 3099, 3100, 3101, 3102, 3103, 3104, 3105, 3106, 3107, 3108, 3109, 3110, 3112, 3113, 3114, 3115, 3116, 3117, 3118, 3119, 3120, 3121, 3122, 3124, 3125, 3126, 3127, 3128, 3129, 3130, 3131, 3132, 3151, 3152, 3153, 3154, 3155, 3156, 3157, 3158, 3159, 3160, 3161, 3163, 3164, 3165, 3166, 3167, 3168, 3169, 3170, 3171, 3172, 3173, 3174, 3175, 3176, 3177, 3178, 3179, 3180, 3181, 3182, 3183, 3184, 3185, 3186, 3187, 3188, 3189, 3190, 3191, 3192, 3193, 3194, 3195, 3196, 3197, 3198, 3199, 3200, 3201, 3202, 3203, 3204, 3205, 3206, 3207, 3208, 3209, 3210, 3211, 3212, 3213, 3214, 3215, 3216, 3217, 3218, 3219, 3220, 3221, 3223, 3232, 3233, 3234, 3235, 3236, 3237, 3238, 3239, 3240, 3241, 3242, 3243, 3245, 3246, 3247, 3249, 3250, 3251, 3252, 3253, 3254, 3255, 3256, 3257, 3258, 3260, 3261, 3262, 3263, 3266, 3267, 3268, 3269, 3270, 3271, 3272, 3273, 3274, 3275, 3276, 3277, 3278, 3279, 3280, 3281, 3282, 3283, 3284, 3285, 3286, 3288, 3289, 3290, 3292, 3293, 3294, 3295, 3296, 3297, 3298, 3299, 3300, 3301, 3302, 3304, 3305, 3306, 3307, 3308, 3309, 3312, 3313, 3314, 3315, 3316, 3317, 3318, 3319, 3320, 3322, 3323, 3325, 3326, 3327, 3328, 3329, 3330, 3331, 3332, 3333, 3334, 3335, 3336, 3337, 3338, 3339, 3340, 3341, 3342, 3343, 3344, 3345, 3346, 3347, 3348, 3349, 3350, 3351, 3352, 3353, 3354, 3356, 3357, 3358, 3359, 3360, 3361, 3363, 3364, 3365, 3366, 3367, 3368, 3369, 3370, 3372, 3373, 3374, 3375, 3376, 3377, 3378, 3379, 3380, 3381, 3382, 3383, 3384, 3385, 3386, 3387, 3388, 3389, 3390, 3391, 3392, 3393, 3394, 3395, 3396, 3397, 3398, 3399, 3400, 3401, 3402, 3403, 3404, 3405, 3406, 3407, 3408, 3409, 3410, 3411, 3412, 3413, 3414, 3415, 3416, 3417, 3418, 3419, 3420, 3421, 3422, 3423, 3424, 3425, 3426, 3427, 3428, 3430, 3431, 3432, 3433, 3435, 3436, 3437, 3438, 3439, 3440, 3441, 3443, 3444, 3445, 3447, 3448, 3449, 3450, 3451, 3452, 3453, 3454, 3455, 3456, 3457, 3459, 3460, 3461, 3462, 3463, 3464, 3465, 3466, 3467, 3468, 3469, 3470, 3472, 3473, 3474, 3475, 3476, 3477, 3478, 3479, 3480, 3481, 3482, 3483, 3484, 3485, 3487, 3488, 3489, 3490, 3491, 3492, 3493, 3494, 3495, 3497, 3498, 3500, 3501, 3502, 3505, 3506, 3507, 3509, 3510, 3511, 3512, 3513, 3514, 3515, 3516, 3517, 3518, 3519, 3520, 3521, 3522, 3523, 3524, 3525, 3526, 3527, 3528, 3529, 3530, 3531, 3532, 3533, 3534, 3535, 3536, 3537, 3538, 3540, 3541, 3542, 3543, 3544, 3545, 3546, 3547, 3548, 3550, 3551, 3552, 3553, 3554, 3555, 3556, 3557, 3559, 3560, 3561, 3562, 3563, 3564, 3565, 3566, 3567, 3568, 3569, 3570, 3571, 3572, 3573, 3574, 3575, 3576, 3578, 3579, 3580, 3581, 3582, 3583, 3584, 3585, 3586, 3587, 3588, 3589, 3590, 3591, 3592, 3594, 3595, 3596, 3597, 3599, 3600, 3602, 3604, 3605, 3607, 3608, 3609, 3610, 3612, 3613, 3614, 3615, 3616, 3617, 3618, 3619, 3620, 3621, 3622, 3623, 3624, 3625, 3626, 3627, 3628, 3629, 3630, 3631, 3632, 3633, 3634, 3635, 3636, 3637, 3638, 3639, 3640, 3641, 3643, 3644, 3645, 3646, 3647, 3648, 3649, 3650, 3651, 3652, 3654, 3656, 3657, 3658, 3659, 3660, 3661, 3662, 3663, 3664, 3665, 3666, 3667, 3668, 3669, 3671, 3672, 3674, 3675, 3676, 3677, 3678, 3679, 3680, 3681, 3683, 3684, 3685, 3686, 3687, 3688, 3689, 3691, 3692, 3693, 3695, 3696, 3697, 3698, 3699, 3700, 3701, 3702, 3703, 3704, 3705, 3706, 3708, 3709, 3710, 3711, 3712, 3713, 3714, 3715, 3717, 3718, 3719, 3720, 3721, 3722, 3723, 3724, 3725, 3726, 3728, 3729, 3730, 3731, 3732, 3733, 3734, 3735, 3738, 3739, 3740, 3741, 3742, 3743, 3745, 3746, 3747, 3748, 3749, 3750, 3751, 3752, 3754, 3755, 3756, 3757, 3758, 3759, 3760, 3761, 3762, 3763, 3764, 3766, 3767, 3768, 3769, 3770, 3771, 3772, 3773, 3774, 3775, 3776, 3777, 3778, 3779, 3780, 3781, 3782, 3783, 3784, 3785, 3786, 3787, 3788, 3789, 4463, 3792, 3793, 3794, 3795, 3796, 3797, 3800, 3801, 3802, 3803, 3804, 3805, 3806, 3807, 3808, 3809, 3810, 3811, 3812, 3813, 3814, 3815, 3816, 3817, 3818, 3819, 3820, 3821, 3822, 3823, 3824, 3825, 3826, 3827, 3828, 3829, 3830, 3831, 3832, 3833, 3834, 3835, 3837, 3838, 3839, 3840, 3841, 3842, 3843, 3844, 3845, 3846, 3847, 3848, 3849, 3851, 3852, 3853, 3854, 3855, 3856, 3857, 3858, 3859, 3860, 3861, 3862, 3863, 3864, 3865, 3866, 3867, 3868, 3869, 3870, 3871, 3873, 3874, 3875, 3876, 3877, 3878, 3879, 3880, 3882, 3883, 3884, 3886, 3887, 3888, 3889, 3890, 3891, 3892, 3893, 3894, 3895, 3896, 3897, 3898, 3899, 3900, 3901, 3902, 3903, 3904, 3905, 3906, 3907, 3908, 3909, 3910, 3911, 3912, 3913, 3914, 3917, 3918, 3919, 3920, 3921, 3923, 3924, 3925, 3926, 3927, 3928, 3929, 3930, 3932, 3933, 3934, 3935, 3936, 3937, 3939, 3940, 3941, 3942, 3943, 3944, 3945, 3946, 3947, 3948, 3949, 3950, 3951, 3952, 3953, 3954, 3955, 3956, 3957, 3958, 3959, 3960, 3961, 3962, 3963, 3964, 3965, 3966, 3967, 3968, 3970, 3971, 3972, 3973, 3974, 3975, 3976, 3977, 3978, 3979, 3980, 3981, 3982, 3983, 3984, 3985, 3986, 3987, 3988, 3989, 3990, 3991, 3992, 3993, 3994, 3995, 3996, 3997, 3998, 3999, 4000, 4001, 4002, 4003, 4004, 4005, 4006, 4007, 4008, 4009, 4010, 4012, 4013, 4014, 4015, 4016, 4018, 4019, 4020, 4021, 4022, 4023, 4024, 4025, 4026, 4027, 4028, 4029, 4030, 4031, 4032, 4033, 4034, 4035, 4036, 4037, 4038, 4039, 4040, 4041, 4042, 4043, 4044, 4045, 4046, 4047, 4048, 4049, 4050, 4051, 4052, 4053, 4054, 4055, 4057, 4058, 4060, 4061, 4062, 4063, 4064, 4065, 4066, 4067, 4069, 4070, 4071, 4072, 4073, 4074, 4075, 4086, 4087, 4088, 4089, 4091, 4092, 4093, 4094, 4095, 4096, 4097, 4098, 4099, 4100, 4101, 4102, 4103, 4104, 4105, 4107, 4108, 4109, 4110, 4111, 4113, 4114, 4115, 4116, 4118, 4119, 4120, 4121, 4122, 4123, 4124, 4125, 4126, 4127, 4128, 4129, 4130, 4131, 4132, 4133, 4134, 4135, 4136, 4137, 4138, 4139, 4140, 4141, 4142, 4143, 4146, 4147, 4148, 4149, 4150, 4151, 4152, 4153, 4154, 4155, 4156, 4157, 4177, 4178, 4183, 4184, 4185, 4186, 4187, 4188, 4189, 4190, 4191, 4192, 4193, 4194, 4195, 4196, 4197, 4198, 4199, 4200, 4202, 4203, 4204, 4205, 4206, 4207, 4208, 4209, 4210, 4211, 4212, 4213, 4214, 4215, 4216, 4217, 4218, 4219, 4220, 4222, 4223, 4224, 4225, 4226, 4227, 4228, 4229, 4230, 4231, 4232, 4233, 4234, 4236, 4237, 4238, 4239, 4240, 4241, 4242, 4243, 4244, 4245, 4246, 4247, 4248, 4249, 4250, 4251, 4252, 4253, 4254, 4255, 4256, 4257, 4258, 4259, 4260, 4261, 4262, 4263, 4264, 4265, 4266, 4267, 4268, 4269, 4270, 4271, 4272, 4273, 4274, 4275, 4276, 4277, 4278, 4279, 4280, 4281, 4282, 4283, 4284, 4286, 4287, 4289, 4290, 4291, 4292, 4293, 4294, 4295, 4296, 4297, 4298, 4299, 4300, 4301, 4302, 4303, 4304, 4306, 4307, 4308, 4309, 4310, 4311, 4312, 4313, 4314, 4315, 4316, 4317, 4318, 4319, 4320, 4321, 4322, 4324, 4325, 4326, 4327, 4328, 4329, 4330, 4331, 4332, 4334, 4335, 4337, 4338, 4339, 4340, 4341, 4342, 4343, 4344, 4345, 4346, 4347, 4348, 4349, 4350, 4351, 4352, 4354, 4355, 4356, 4357, 4358, 4359, 4360, 4361, 4362, 4363, 4364, 4365, 4366, 4367, 4368, 4369, 4370, 4371, 4372, 4373, 4374, 4375, 4376, 4377, 4378, 4379, 4380, 4381, 4382, 4383, 4384, 4385, 4386, 4387, 4388, 4389, 4390, 4391, 4392, 4393, 4394, 4395, 4396, 4397, 4399, 4400, 4401, 4402, 4403, 4404, 4405, 4406, 4407, 4408, 4410, 4411, 4412, 4413, 4414, 4415, 4416, 4418, 4419, 4420, 4422, 4424, 4425, 4426, 4427, 4428, 4430, 4431, 4432, 4433, 4434, 4436, 4437, 4438, 4439, 4440, 4441, 4442, 4443, 4444, 4445, 4446, 4447, 4449, 4451, 4452, 4453, 4454, 4455, 4456, 4457, 4458, 4459, 4460, 4461, 4462, 4463, 4464, 4465, 4466, 4467, 4468, 4470, 4471, 4473, 4474, 4475, 4477, 4478, 4479, 4480, 4481, 4483, 4484, 4485, 4486, 4487, 4489, 4490, 4491, 4492, 4493, 4494, 4495, 4496, 4497, 4498, 4499, 4500, 4501, 4502, 4503, 4504, 4505, 4506, 4507, 4508, 4509, 4510, 4511, 4512, 4514, 4515, 4516, 4517, 4519, 4520, 4521, 4522, 4523, 4524, 4525, 4526, 4527, 4528, 4529, 4530, 4531, 4532, 4533, 4534, 4535, 4536, 4537, 4538, 4539, 4540, 4541, 4542, 4543, 4545, 4546, 4547, 4548, 4549, 4550, 4551, 4552, 4553, 4554, 4555, 4556, 4557, 4558, 4561, 4562, 4563, 4564, 4565, 4566, 4567, 4568, 4570, 4572, 4573, 4574, 4575, 4576, 4577, 4579, 4580, 4581, 4582, 4583, 4584, 4585, 4586, 4587, 4588, 4589, 4590, 4591, 4592, 4593, 4594, 4595, 4596, 4597, 4598, 4599, 4600, 4601, 4602, 4603, 4604, 4605, 4606, 4608, 4609, 4610, 4611, 4612, 4613, 4614, 4615, 4616, 4617, 4618, 4619, 4620, 4621, 4623, 4624, 4625, 4626, 4627, 4628, 4629, 4630, 4632, 4633, 4634, 4635, 4636, 4637, 4638, 4639, 4640, 4641, 4644, 4645, 4646, 4649, 4651, 4652, 4653, 4654, 4655, 4656, 4657, 4659, 4660, 4661, 4662, 4663, 4664, 4665, 4666, 4667, 4668, 4670, 4671, 4673, 4674, 4675, 4676, 4677, 4678, 4679, 4680, 4681, 4683, 4684, 4685, 4686, 4690, 4691, 4692, 4693, 4694, 4695, 4696, 4697, 4698, 4699, 4700, 4701, 4702, 4703, 4705, 4706, 4708, 4709, 4710, 4711, 4712, 4713, 4714, 4715, 4716, 4717, 4719, 4720, 4721, 4722, 4723, 4724, 4725, 4726, 4728, 4729, 4730, 4731, 4732, 4733, 4734, 4735, 4736, 4737, 4738, 4739, 4740, 4741, 4742, 4743, 4744, 4745, 4746, 4747, 4748, 4749, 4750, 4751, 4752, 4753, 4755, 4756, 4757, 4758, 4759, 4760, 4761, 4762, 4763, 4764, 4765, 4766, 4768, 4769, 4770, 4771, 4772, 4773, 4774, 4775, 4776, 4777, 4778, 4779, 4780, 4781, 4782, 4783, 4784, 4785, 4787, 4788, 4789, 4790, 4791, 4793, 4794, 4795, 4796, 4798, 4799, 4800, 4801, 4802, 4803, 4805, 4806, 4807, 4808, 4809, 4810, 4811, 4813, 4815, 4816, 4817, 4818, 4819, 4820, 4821, 4822, 4824, 4825, 4826, 4827, 4828, 4829, 4831, 4832, 4834, 4835, 4836, 4837, 4839, 4840, 4841, 4842, 4843, 4844, 4845, 4846, 4847, 4850, 4851, 4852, 4853, 4854, 4855, 4856, 4857, 4858, 4860, 4861, 4863, 4864, 4865, 4866, 4867, 4868, 4869, 4870, 4871, 4873, 4874, 4875, and 4876.

In a second aspect, the present invention relates to an isolated polynucleotide comprising a nucleotide sequence that hybridizes under very low stringency conditions, preferably low stringency conditions, more preferably medium stringency conditions, more preferably medium-high stringency conditions, even more preferably high stringency conditions, and most preferably very high stringency conditions with any of (i) the polynucleotides of SEQ ID NOs: 2-4876, or subsequences thereof, or (ii) full-length complementary strands thereof (J. Sambrook, E.F. Fritsch, and T. Maniatus, 1989, Molecular Cloning, A Laboratory Manual, 2d edition, Cold Spring Harbor, New York). Subsequences of SEQ ID NOs: 2-4876 may be preferably at least 90 nucleotides, more preferably at least 150 nucleotide, and most preferably at least 200 nucleotides. Moreover, the subsequences may encode fragments of a gene product that have biological activity.

In a preferred aspect, the present invention relates to an isolated polynucleotide comprising a nucleotide sequence that hybridizes under preferably at least medium stringency conditions, more preferably at least medium-high stringency conditions, or most preferably at least high stringency conditions with a polynucleotide selected from the group consisting of SEQ ID NOs: 2-4876 and full-length complementary strands thereof.

The nucleotide sequences of SEQ ID NOs: 2-4876 or subsequences thereof, as well as the amino acid sequences of SEQ ID NOs: 4877-9751 or fragments thereof, may be used to design nucleic acid probes to identify and clone DNA encoding biologically active substances from strains of different genera or species according to methods well known in the art. In particular, such probes can be used for hybridization with the genomic DNA of the genus or species of interest, following standard Southern blotting procedures, in order to identify and isolate the corresponding gene therein. Such probes can be considerably shorter than the entire sequence, but should be at least 14, preferably at least 25, more preferably at least 35 nucleotides in length, such as at least 70 nucleotides in length. It is preferred, however, that the nucleic acid probes are at least 100 nucleotides in length. For example, the nucleic acid probes may be at least 200 nucleotides, at least 300 nucleotides, at least 400 nucleotides, or at least 500 nucleotides in length. Even longer probes may be used, *e.g.*, nucleic acid probes that are at least 600 nucleotides, at least 700 nucleotides, at least 800 nucleotides, or at least 900 nucleotides in length. Both DNA and RNA probes can be used. The probes are typically labeled for detecting the corresponding gene (for example, with $^{32}$P, $^3$H, $^{35}$S, biotin, or avidin). Such probes are encompassed by the present invention.

A genomic DNA library prepared from such other organisms may, therefore, be screened for DNA that hybridizes with the probes described above and encodes a biologically active substance. Genomic DNA from such other organisms may be separated by agarose or polyacrylamide gel electrophoresis, or other separation techniques. DNA from the libraries or the separated DNA may be transferred to and immobilized on nitrocellulose or other suitable carrier material. In order to identify a clone or DNA that is homologous with any of SEQ ID NOs: 2-4876 or subsequences thereof, the carrier material is used in a Southern blot.

For purposes of the present invention, hybridization indicates that a polynucleotide hybridizes to a labeled gene having the nucleotide sequence shown in any of SEQ ID NOs: 2-4876, full-length complementary strands thereof, or subsequences thereof, under very low to very high stringency conditions. Molecules to which the nucleic acid probe hybridizes under these conditions can be detected using X-ray film.

In a preferred aspect, the nucleic acid probe is any of the polynucleotides of SEQ ID NOs: 2-4876, or subsequences thereof. In another preferred aspect, the nucleic acid probe is the mature polypeptide coding region of any of the polynucleotides of SEQ ID NOs: 2-4876. In another preferred aspect, the nucleic acid probe is the polynucleotide of any of SEQ ID NOs: 2-4876 contained in chromosome of *Bacillus licheniformis* SJ1904. In another preferred aspect, the nucleic acid probe is the mature polypeptide coding region of any of the polynucleotides of SEQ ID NOs: 2-4876 contained in *Bacillus licheniformis* SJ1904.

For long probes of at least 100 nucleotides in length, very low to very high stringency conditions are defined as prehybridization and hybridization at 42°C in 5X SSPE, 0.3% SDS, 200 $\mu$g/ml sheared and denatured salmon sperm DNA, and either 25% formamide for very low and low stringencies, 35% formamide for medium and medium-high stringencies, or 50% formamide for high and very high stringencies, following standard Southern blotting procedures.

For long probes of at least 100 nucleotides in length, the carrier material is finally washed three times each for 15 minutes using 2X SSC, 0.2% SDS preferably at least at 45°C (very low stringency), more preferably at least at 50°C (low stringency), more preferably at least at 55°C (medium stringency), more preferably at least at 60°C (medium-high stringency), even more preferably at least at 65°C (high stringency), and most preferably at least at 70°C (very high stringency).

For short probes of about 14 nucleotides to about 70 nucleotides in length, stringency conditions are defined as prehybridization, hybridization, and washing post-hybridization at about 5°C to about 10°C below the calculated $T_m$ using the calculation according to Bolton and McCarthy (1962, Proceedings of the National Academy of Sciences USA 48:1390) in 0.9 M NaCl, 0.09 M Tris-HCl pH 7.6, 6 mM EDTA, 0.5% NP-40, 1X Denhardt's solution, 1 mM sodium pyrophosphate, 1 mM sodium monobasic phosphate, 0.1 mM ATP, and 0.2 mg of yeast RNA per ml following standard Southern blotting procedures.

For short probes of about 14 nucleotides to about 70 nucleotides in length, the carrier material is washed once in 6X SCC plus 0.1% SDS for 15 minutes and twice each for 15 minutes using 6X SSC at 5°C to 10°C below the calculated $T_m$.

Under salt-containing hybridization conditions, the effective $T_m$ is what controls the degree of sequence identity required between the probe and the filter bound DNA for successful hybridization. The effective $T_m$ may be determined using the formula below to determine the degree of sequence identity required for two DNAs to hybridize under various stringency conditions.

$$\text{Effective } T_m = 81.5 + 16.6(\log M[Na^+]) + 0.41(\%G+C) - 0.72(\% \text{ formamide})$$

The %G+C content of any of the polynucleotides of SEQ ID NOs: 2-4876 can easily be determined. For medium stringency, for example, the concentration of formamide is 35% and the Na[+] concentration for 5X SSPE is 0.75 M. Applying this formula to these values, the Effective $T_m$ in °C can be calculated. Another relevant relationship is that a 1% mismatch of two DNAs lowers the $T_m$ 1.4°C. To determine the degree of sequence identity required for two DNAs to hybridize under medium stringency conditions at 42°C, the following formula is used:

$$\% \text{ Homology} = 100 - [(\text{Effective } T_m - \text{Hybridization Temperature})/1.4]$$

Applying this formula, the degree of sequence identity required for two DNAs to hybridize under medium stringency conditions at 42°C can be calculated.

Similar calculations can be made under other stringency conditions, as defined herein.

The present invention also relates to isolated polynucleotides obtained by (a) hybridizing a population of DNA under very low, low, medium, medium-high, high, or very high stringency conditions with any of (i) the polynucleotides of SEQ ID NOs: 2-4876, or subsequences thereof, or (ii) full-length complementary strands thereof; and (b) isolating the hybridizing polynucleotide from the population of DNA. In a preferred aspect, the hybridizing polynucleotide encodes a polypeptide of any of SEQ ID NOs: 2-4876, or homologous polypeptides thereof.

In a preferred aspect, the present invention relates to isolated polynucleotides obtained by (a) hybridizing a population of DNA under preferably at least medium stringency conditions, more preferably at least medium-high stringency conditions, or most preferably at least high stringency conditions with a polynucleotide selected from the group consisting of SEQ ID NOs: 2-4876 and full-length complementary strands thereof; and (b) isolating the hybridizing polynucleotide from the population of DNA.

In a third aspect, the present invention relates to an isolated polynucleotide encoding a biologically active substance comprising an amino acid sequence having preferably at least 60% identity, more preferably at least 65% identity, more preferably at least 70% identity, more preferably at least 75% identity, more preferably at least 80% identity, more preferably at least 85% identity, more preferably at least 90% identity, even more preferably at least 90% identity, most preferably at least 95% identity, and even most preferably at least 97% identity with an amino acid sequence selected from the group consisting of SEQ ID NOs: 4877-9751 and fragments thereof retaining biological activity.

In a preferred aspect, the biological substance comprises an amino acid sequence an amino acid sequence having a degree of sequence identity of preferably at least 60% identity, more preferably at least 65% identity, more preferably at least 70% identity, more preferably at least 75% identity, more preferably at least 80%, more preferably at least 85%, even more preferably at least 90%, most preferably at least 95%, and even most preferably at least 97% with an amino acid sequence selected from the group consisting of SEQ ID NOs: 4944, 4985, 5051, 5058, 5064, 5076, 5079, 5097, 5098, 5099, 5106, 5115, 5117, 5136, 5137, 5197, 5204, 5286, 5459, 5529, 5604, 5723, 5724, 5725, 5726, 5761, 5923, 6056, 6097, 6153, 6192, 6371, 6372, 6376, 6456, 6504, 6531, 6545, 6546, 6547, 6548, 6550, 6945, 6965, 7053, 7116, 7163, 7164, 7165, 7166, 7169, 7170, 7174, 7176, 7177, 7363, 7392, 7397, 7399, 7402, 7420, 7421, 7423, 7425, 7438, 7449, 7453, 7468, 7507, 7562, 7563, 7565, 7573, 7641, 7815, 8003, 8008, 8009, 8010, 8011, 8014, 8015, 8018, 8020, 8021, 8022, 8046, 8099, 8100, 8103, 8140, 8323, 8710, 8711, 8966, 9034, 9035, 9036, 9049, 9051, 9054, 9056, 9057, 9224, 9516, 9525, 9547, 9562, 9564, 4900, 4925, 4930, 4952, 4957, 4966, 4973, 4978, 5000, 5010, 5044, 5046, 5047, 5049, 5052, 5053, 5054, 5055, 5056, 5057, 5060, 5061, 5062, 5067, 5069, 5070, 5071, 5073, 5074, 5078, 5080, 5081, 5082, 5083, 5084, 5085, 5086, 5087, 5088, 5089, 5090, 5091, 5092, 5093, 5094, 5095, 5096, 5100, 5101, 5102, 5103, 5104, 5105, 5112, 5116, 5118, 5119, 5120, 5121, 5122, 5123, 5124, 5125, 5126, 5127, 5128, 5129, 5130, 5131, 5132, 5133, 5134, 5135, 5138, 5139, 5140, 5141, 5151, 5162, 5173, 5180, 5181, 5183, 5192, 5198, 5225, 5229, 5235, 5236, 5238, 5242, 5249, 5256, 5290, 5297, 5307, 5320, 5344, 5347, 5353, 5354, 5362, 5363, 5366, 5373, 5391, 5396, 5398, 5399, 5412, 5416, 5428, 5445, 5450, 5456, 5467, 5482, 5493, 5496, 5502, 5503, 5506, 5541, 5547, 5563, 5566, 5574, 5577, 5585, 5594, 5601, 5609, 5623, 5626, 5639, 5648, 5649, 5652, 5664, 5666, 5702, 5704, 5706, 5708, 5717, 5722, 5730, 5732, 5760, 5762, 5776, 5784, 5819, 5832, 5852, 5857, 5866, 5869, 5890, 5893, 5897, 5907, 5909, 5912, 5933, 5953, 5955, 5963, 5966, 5973, 5983, 5986, 5987, 5993, 6003, 6005, 6023, 6026, 6028, 6029, 6033, 6035, 6039, 6045, 6050, 6051, 6052, 6055, 6058, 6059, 6068, 6069, 6074, 6083, 6090, 6095, 6112, 6119, 6137, 6138, 6140, 6141, 6167, 6177, 6188, 6189, 6190, 6191, 6193, 6195, 6209, 6220, 6230, 6245, 6248, 6249, 6276, 6286, 6294, 6296, 6299, 6307, 6308, 6315, 6317, 6323, 6330, 6341, 6345, 6384, 6402, 6414, 6431, 6440, 6445, 6464, 6494, 6506, 6509, 6510, 6511, 6513, 6514, 6515, 6516, 6517, 6518, 6519, 6520, 6521, 6522, 6524, 6525, 6526, 6527, 6528, 6529, 6530, 6532, 6533, 6534, 6535, 6536, 6537, 6538, 6539, 6540, 6541, 6542, 6543, 6544, 6552, 6557, 6559, 6563, 6567, 6569, 6572, 6578, 6579, 6603, 6607, 6609, 6611, 6626, 6650, 6654, 6658, 6661, 6667, 6670, 6673, 6691, 6706, 6711, 6713, 6726, 6728, 6741, 6758, 6762, 6819, 6821, 6877, 6882, 6897, 6913, 6919, 6923, 6940, 6942, 6947, 6951, 6958, 6969, 6981, 6984, 6994, 6995, 6996, 6999, 7006, 7014, 7022, 7023, 7041, 7046, 7069, 7071, 7073, 7079, 7080, 7086, 7088, 7091, 7096, 7100, 7105, 7111, 7115, 7129, 7130, 7133, 7138, 7148, 7152, 7157, 7161, 7167, 7171, 7172, 7173, 7175, 7178, 7179, 7180, 7199, 7200, 7202, 7211, 7212, 7216, 7222, 7226, 7229, 7237, 7239, 7256, 7263, 7275, 7278, 7279, 7283, 7285, 7286, 7287, 7289, 7290, 7299, 7300, 7305, 7310, 7314, 7321, 7324, 7332, 7339, 7342, 7343, 7344, 7351, 7358, 7366, 7373, 7375, 7379, 7380, 7381, 7382, 7383, 7384, 7385, 7386, 7387, 7388, 7389, 7390, 7391, 7393, 7394, 7395, 7396, 7400, 7401, 7403, 7404, 7405, 7406, 7407, 7408, 7409, 7410, 7411, 7412, 7413, 7414, 7415, 7416, 7417, 7418, 7419, 7422, 7424, 7426, 7427, 7428, 7429, 7430, 7431, 7432, 7433, 7434, 7435, 7436, 7437, 7439, 7440, 7442, 7443, 7444, 7445, 7446, 7447, 7448, 7450, 7451, 7452, 7454, 7455, 7456, 7457, 7458, 7459, 7460, 7461, 7462, 7463, 7464, 7465, 7466, 7467, 7469, 7470, 7471, 7472, 7473, 7474, 7475, 7476, 7477, 7478, 7479, 7480, 7481, 7482, 7483, 7484, 7485, 7486, 7487, 7488, 7489, 7490, 7491, 7492, 7493, 7494, 7495, 7496, 7497, 7498, 7499, 7500, 7501, 7502, 7503, 7504, 7505, 7506, 7508, 7509, 7510, 7511, 7513, 7514, 7515, 7516, 7517, 7518, 7519, 7520, 7521, 7522, 7523, 7524, 7525, 7526, 7527, 7528, 7529, 7530, 7531, 7532, 7533, 7534, 7536, 7539, 7540, 7542, 7544, 7545, 7546, 7547, 7548, 7549, 7550, 7551, 7552, 7553, 7554, 7555, 7556, 7559, 7560, 7561, 7564, 7566, 7567, 7568, 7571, 7572, 7581, 7583, 7592, 7595, 7598, 7605, 7608, 7626, 7637, 7646, 7670, 7681, 7688, 7709, 7710, 7716, 7725, 7741, 7742, 7749, 7753, 7758, 7766, 7773, 7777, 7779, 7792, 7822, 7832, 7835, 7844, 7847, 7848, 7874, 7879, 7893, 7894, 7903, 7921, 7929, 7939, 7944, 7957, 7962, 7968, 7987, 7998, 8012, 8016, 8017, 8019, 8023, 8024, 8025, 8037, 8090, 8097, 8101, 8102, 8104, 8106, 8119, 8123, 8134, 8136, 8139, 8162, 8166, 8174, 8178, 8185, 8186, 8196, 8198, 8199, 8208, 8230, 8237, 8246, 8299, 8304, 8306, 8309, 8317, 8321, 8332, 8333, 8346, 8361, 8368, 8371, 8374, 8378, 8379, 8383, 8384, 8394, 8424, 8433, 8452, 8462, 8468, 8473, 8476, 8478, 8481, 8486, 8517, 8528, 8530, 8545, 8548, 8557, 8565, 8569, 8582, 8584, 8591, 8602, 8610, 8611, 8612, 8619, 8628, 8640, 8645, 8655, 8665, 8667, 8673, 8674, 8693, 8700, 8714, 8747, 8754, 8756, 8760, 8779, 8786, 8790, 8791, 8797, 8801, 8806, 8813, 8844, 8886, 8892, 8893, 8908, 8912, 8923, 8930, 8931, 8934, 8943, 8951, 8952, 8953, 8956, 8957, 8960, 8961, 8965, 8981, 8987, 8992, 9019, 9020, 9033, 9037, 9038, 9039, 9040, 9041, 9042, 9043, 9044, 9045, 9046, 9047, 9048, 9050, 9076, 9096, 9110, 9114, 9118, 9160, 9163, 9180, 9198, 9208, 9211, 9228, 9245, 9273, 9284, 9292, 9296, 9298, 9304, 9310, 9321, 9323, 9325, 9337, 9344, 9347, 9349, 9351, 9357, 9362, 9363, 9374, 9384, 9385, 9388, 9393, 9419, 9433, 9434, 9435, 9436, 9444, 9446, 9453, 9475, 9480, 9482, 9497, 9506, 9508, 9518, 9520, 9522, 9523, 9524, 9533, 9544, 9557, 9568, 9579, 9583, 9593, 9597, 9629, 9642, 9661, 9667, 9672, 9679, 9687, 9689, 9698, 9705, 9708, 9713, 9723, 9724, 9729, 9734, 9737, and 9747; preferably at least 65% identity, more preferably at least 70% identity, more preferably at least 75% identity, more preferably at least 80%, more preferably at least 85%, even more preferably at least 90%, most preferably at least 95%, and even most preferably at least 97% with an amino acid sequence selected from the group consisting of SEQ ID NOs: 4991, 5068, 5308, 5463, 5919, 6549, 6648, 6680, 6948, 7298, 7541, 7896, 7911, 8497, 8702, 8729, 8737, 9052, 9690, and 9709; preferably at least 70% identity, more preferably at least 75% identity, more preferably at least 80%, more preferably at least 85%, even more preferably at least 90%, most preferably at least 95%, and even most preferably at least 97% with an amino acid sequence selected from the group consisting of SEQ ID NOs: 4986, 5041, 5043, 5418, 5427, 5500, 5515, 5696, 5713, 5785, 5939, 6106, 6109, 6171, 6260, 6503, 6677, 6724, 7054, 7260, 7295, 7316, 7341, 7512, 7538, 7591, 7628, 7735, 7912, 8050, 8076, 8206, 8207, 8240, 8327, 8406, 8428, 8860, 8955, 9061, 9099, 9115, 9262, 9591, 9641, and 9720; preferably at least 75% identity, more preferably at least 80%, more preferably at least 85%, even more preferably at least 90%, most preferably at least 95%, and even most preferably at least 97% with an amino acid sequence selected from the group consisting of SEQ ID NOs: 5038, 5059, 5240, 5403, 5444, 5508, 5559, 5562, 5642, 5651, 5846, 5885, 5921, 5942, 6015, 6067, 6071, 6164, 6215, 6293, 6373, 6390, 6446, 6624, 6704, 6717, 6749, 6759, 6808, 6847, 6848, 6852, 6854, 7292, 7297, 7308, 7317, 7537, 7543, 7557, 7603, 7651, 7858, 7949, 7954, 7996, 8074, 8200, 8221, 8263, 8288, 8614, 8684, 8720, 8728, 8740, 8749, 8787, 8864, 8958, 9055, 9176, 9196, 9283, 9308, 9352, 9491, 9512, 9602, 9653, 9684, 9719, and 9731; preferably at least 80% identity, more preferably at least 85% identity, even more preferably at least 90% identity, most preferably at least 95% identity, and even most preferably at least 97% identity with an amino acid sequence selected from the group consisting of SEQ ID NOs: 4891, 4956, 5042, 5072, 5169, 5190, 5195, 5217, 5241, 5266, 5305, 5369, 5374, 5377, 5409, 5435, 5512, 5524, 5538, 5595, 5613, 5647, 5657, 5665, 5667, 5735, 5743, 5830, 5878, 5900, 5903, 5977, 5988, 6000, 6060, 6066, 6070, 6073, 6075, 6105, 6120, 6139, 6152, 6174, 6291, 6335, 6375, 6437, 6466, 6492, 6505, 6507, 6523, 6553, 6592, 6651, 6694, 6730, 6732, 6774, 6843, 6929, 6943, 6964, 7093, 7132, 7144, 7224, 7349, 7374, 7398, 7570, 7578, 7632, 7635, 7643, 7680, 7682, 7685, 7700, 7713, 7727, 7800, 7843, 7864, 7918, 7947, 7982, 7989, 7991, 7999, 8055, 8057, 8085, 8095, 8170, 8182, 8191, 8210, 8255, 8261, 8328, 8365, 8399, 8410, 8479, 8551, 8561, 8577, 8589, 8649, 8679, 8742, 8744, 8784, 8789, 8796, 8818, 8832, 8835, 8845, 8854, 8858, 8868, 8878, 8900, 8950, 9007, 9102, 9108, 9151, 9169, 9207, 9241, 9246, 9248, 9250, 9299, 9341, 9343, 9412, 9443, 9470, 9476, 9511, 9528, 9559, 9565, 9577, 9584, 9594, 9595, 9682, and 9714; preferably at least 85% identity, more preferably at least 90% identity, most preferably at least 95% identity, and even most preferably at least 97% identity with an amino acid sequence selected from the group consisting of SEQ ID NOs: 4913, 4948, 4968, 5025, 5034, 5066, 5109, 5110, 5146, 5160, 5199, 5200, 5203, 5211, 5221, 5223, 5226, 5252, 5269, 5273, 5293, 5309, 5318, 5342, 5352, 5393, 5394, 5421, 5430, 5431, 5434, 5447, 5458, 5492, 5507, 5511, 5514, 5525, 5526, 5530, 5544, 5570, 5616, 5617, 5621, 5640, 5661, 5663, 5691, 5716, 5738, 5751, 5753, 5757, 5759, 5786, 5802, 5820, 5826, 5855, 5858, 5871, 5872, 5902, 5905, 5908, 5915, 5936, 5937, 5938, 5947, 6006, 6011, 6012, 6025, 6030, 6032, 6034, 6057, 6080, 6084, 6093, 6096, 6110, 6131, 6178, 6183, 6199, 6232, 6236, 6243, 6257, 6258, 6313, 6331, 6354, 6360, 6362, 6386, 6417, 6421, 6435, 6455, 6468, 6472, 6475, 6476, 6478, 6495, 6555, 6566, 6568, 6604, 6608, 6641, 6662, 6663, 6665, 6692, 6699, 6736, 6752, 6763, 6807, 6809, 6810, 6840, 6842, 6844, 6853, 6855, 6857, 6866, 6906, 6908, 6915, 6922, 6953, 6962, 6977, 6989, 7004, 7025, 7032, 7042, 7045, 7058, 7072, 7085, 7134, 7139, 7145, 7182, 7188, 7194, 7208, 7227, 7249, 7261, 7272, 7293, 7309, 7320, 7331, 7333, 7334, 7354, 7367, 7371, 7558, 7597, 7602, 7623, 7664, 7677, 7679, 7687, 7732, 7734, 7739, 7744, 7752, 7765, 7769, 7790, 7801, 7818, 7823, 7833, 7837, 7860, 7867, 7877, 7882, 7915, 7931, 7932, 7946, 7948, 7959, 7967, 7973, 7975, 7977, 7978, 7986, 8071, 8072, 8096, 8107, 8129, 8146, 8148, 8153, 8164, 8165, 8169, 8203, 8211, 8216, 8218, 8234, 8245, 8253, 8275, 8297, 8315, 8334, 8340, 8357, 8362, 8372, 8386, 8398, 8425, 8438, 8455, 8460, 8470, 8480, 8490, 8506, 8531, 8533, 8572, 8593, 8600, 8615, 8623, 8627, 8630, 8634, 8664, 8687, 8703, 8704, 8705, 8726, 8739, 8750, 8752, 8753, 8769, 8780, 8781, 8805, 8816, 8825, 8846, 8869, 8870, 8883, 8913, 8938, 8944, 8945, 8948, 8959, 8962, 8973, 8976, 8980, 8991, 9000, 9006, 9017, 9068, 9093, 9097, 9103, 9109, 9146, 9155, 9159, 9255, 9258, 9260, 9266, 9269, 9271, 9278, 9293, 9350, 9373, 9394, 9415, 9420, 9449, 9466, 9472, 9477, 9486, 9500, 9504, 9514, 9517, 9535, 9573, 9590, 9599, 9601, 9623, 9645, 9648, 9652, 9654, 9659, 9726, 9728, 9736, and 9748; preferably at least 90% identity, more preferably at least 95% identity, and most preferably at least 97% identity with an amino acid sequence selected from the group consisting ofSEQ ID NOs: 4890, 4895, 4921, 4923, 4927, 4933, 4934, 4937, 4939, 4943, 4946, 4950, 4951, 4955, 4958, 4967, 4970, 4981, 4982, 4987, 4989, 4999, 5017, 5022, 5029, 5048, 5111, 5113, 5145, 5152, 5157, 5165, 5171, 5178, 5179, 5182, 5186, 5202, 5213, 5214, 5215, 5218, 5227, 5230, 5233, 5245, 5268, 5272, 5274, 5279, 5281, 5283, 5298, 5303, 5315, 5324, 5326, 5328, 5329, 5330, 5336, 5337, 5338, 5339, 5346, 5357, 5360, 5376, 5380, 5387, 5400, 5401, 5414, 5417, 5419, 5422, 5424, 5436, 5442, 5446, 5448, 5455, 5457, 5466, 5475, 5499, 5501, 5510, 5537, 5539, 5542, 5550, 5552, 5557, 5565, 5571, 5573, 5586, 5589, 5612, 5615, 5620, 5622, 5628, 5632, 5634, 5638, 5645, 5646, 5660, 5670, 5677, 5678, 5681, 5688, 5705, 5707, 5710, 5739, 5748, 5754, 5756, 5763, 5764, 5765, 5766, 5768, 5779, 5782, 5783, 5787, 5791, 5792, 5793, 5799, 5808, 5813, 5821, 5827, 5828, 5834, 5835, 5837, 5842, 5850, 5856, 5859, 5864, 5870, 5884, 5906, 5914, 5917, 5945, 5946, 5958, 5964, 5965, 5985, 5989, 5994, 5998, 6007, 6009, 6016, 6017, 6019, 6022, 6031, 6048, 6062, 6064, 6079, 6081, 6086, 6089, 6098, 6103, 6107, 6113, 6121, 6122, 6125, 6156, 6163, 6165, 6181, 6205, 6212, 6223, 6235, 6238, 6239, 6242, 6244, 6250, 6251, 6255, 6261, 6262, 6265, 6267, 6269, 6281, 6283, 6284, 6290, 6292, 6300, 6301, 6318, 6324, 6327, 6332, 6333, 6338, 6339, 6344, 6349, 6353, 6361, 6363, 6374, 6377, 6383, 6387, 6396, 6397, 6399, 6400, 6413, 6423, 6449, 6450, 6462, 6465, 6467, 6471, 6484, 6493, 6501, 6512, 6565, 6573, 6574, 6575, 6576, 6580, 6583, 6585, 6589, 6594, 6598, 6600, 6605, 6619, 6629, 6635, 6636, 6637, 6638, 6642, 6647, 6664, 6669, 6686, 6688, 6695, 6696, 6697, 6698, 6700, 6710, 6712, 6729, 6735, 6740, 6742, 6743, 6744, 6745, 6768, 6779, 6780, 6781, 6782, 6786, 6792, 6793, 6795, 6800, 6801, 6802, 6804, 6823, 6827, 6829, 6833, 6834, 6836, 6838, 6861, 6862, 6863, 6867, 6868, 6871, 6872, 6887, 6903, 6918, 6920, 6927, 6933, 6939, 6944, 6949, 6960, 6966, 6973, 6976, 6979, 7000, 7002, 7015, 7017, 7019, 7036, 7040, 7051, 7055, 7056, 7063, 7075, 7077, 7087, 7090, 7092, 7097, 7102, 7103, 7104, 7109, 7112, 7117, 7118, 7122, 7123, 7125, 7126, 7127, 7158, 7162, 7181, 7186, 7196, 7201, 7203, 7206, 7209, 7210, 7219, 7221, 7230, 7231, 7232, 7233, 7235, 7236, 7244, 7248, 7251, 7252, 7257, 7268, 7271, 7274, 7281, 7304, 7326, 7335, 7352, 7356, 7357, 7360, 7364, 7577, 7579, 7601, 7607, 7615, 7624, 7625, 7639, 7650, 7658, 7672, 7696, 7712, 7718, 7728, 7729, 7730, 7745, 7747, 7748, 7751, 7754, 7756, 7774, 7780, 7786, 7808, 7810, 7826, 7831, 7840, 7849, 7854, 7871, 7872, 7886, 7892, 7914, 7925, 7928, 7933, 7934, 7937, 7941, 7945, 7969, 7970, 7980, 8032, 8039, 8040, 8060, 8063, 8065, 8066, 8075, 8077, 8078, 8086, 8122, 8133, 8138, 8149, 8157, 8158, 8159, 8177, 8180, 8184, 8188, 8195, 8205, 8209, 8220, 8223, 8235, 8241, 8243, 8247, 8250, 8252, 8257, 8271, 8280, 8282, 8283, 8295, 8298, 8305, 8310, 8325, 8342, 8344, 8347, 8358, 8363, 8366, 8373, 8377, 8382, 8387, 8392, 8400, 8404, 8405, 8407, 8413, 8417, 8419, 8421, 8426, 8429, 8434, 8436, 8442, 8450, 8457, 8458, 8461, 8463, 8469, 8491, 8494, 8501, 8504, 8507, 8508, 8516, 8523, 8532, 8536, 8544, 8550, 8552, 8554, 8564, 8566, 8587, 8603, 8622, 8629, 8632, 8635, 8636, 8646, 8647, 8652, 8653, 8654, 8657, 8682, 8690, 8698, 8701, 8709, 8712, 8724, 8725, 8736, 8755, 8767, 8772, 8776, 8798, 8803, 8804, 8817, 8836, 8843, 8852, 8857, 8859, 8861, 8866, 8880, 8881, 8882, 8884, 8885, 8891, 8894, 8901, 8904, 8905, 8910, 8911, 8927, 8933, 8954, 8963, 8971, 8975, 8977, 8984, 8995, 8997, 9004, 9008, 9023, 9026, 9031, 9063, 9078, 9080, 9083, 9091, 9092, 9112, 9120, 9127, 9134, 9140, 9149, 9156, 9164, 9168, 9185, 9189, 9190, 9193, 9197, 9202, 9204, 9214, 9217, 9220, 9232, 9233, 9236, 9243, 9259, 9274, 9275, 9280, 9286, 9291, 9300, 9305, 9306, 9317, 9329, 9356, 9364, 9366, 9367, 9378, 9382, 9389, 9390, 9392, 9396, 9399, 9405, 9417, 9422, 9425, 9428, 9438, 9441, 9451, 9454, 9462, 9463, 9468, 9473, 9496, 9499, 9501, 9502, 9510, 9539, 9540, 9550, 9556, 9561, 9570, 9574, 9585, 9587, 9604, 9605, 9606, 9613, 9616, 9618, 9630, 9632, 9633, 9635, 9650, 9651, 9655, 9656, 9670, 9680, 9683, 9686, 9699, 9704, 9716, 9727, and 9749; or preferably at least 95% identity and more preferably at least 97% identity with an amino acid sequence selected from the group consisting of SEQ ID NOs: 4877, 4878, 4879, 4880, 4881, 4882, 4883, 4884, 4885, 4886, 4887, 4888, 4889, 4892, 4893, 4894, 4896, 4897, 4898, 4899, 4901, 4902, 4903, 4904, 4905, 4906, 4907, 4908, 4909, 4910, 4911, 4912, 4914, 4915, 4916, 4917, 4918, 4919, 4920, 4922, 4924, 4926, 4928, 4929, 4931, 4932, 4935, 4936, 4938, 4940, 4941, 4942, 4945, 4947, 4949, 4953, 4954, 4959, 4960, 4961, 4962, 4963, 4964, 4965, 4969, 4971, 4972, 4974, 4975, 4976, 4977, 4979, 4980, 4983, 4984, 4988, 4990, 4992, 4993, 4994, 4995, 4996, 4997, 4998, 5001, 5002, 5003, 5004, 5005, 5006, 5007, 5008, 5009, 5011, 5012, 5013, 5014, 5015, 5016, 5018, 5019, 5020, 5021, 5023, 5024, 5026, 5027, 5028, 5030, 5031, 5032, 5033, 5035, 5036, 5037, 5039, 5045, 5050, 5063, 5065, 5075, 5077, 5107, 5108, 5114, 5142, 5143, 5144, 5147, 5148, 5149, 5150, 5153, 5154, 5155, 5156, 5158, 5159, 5161, 5163, 5164, 5166, 5167, 5168, 5170, 5172, 5174, 5175, 5176, 5177, 5184, 5185, 5187, 5188, 5189, 5191, 5193, 5194, 5196, 5201, 5205, 5206, 5207, 5208, 5209, 5210, 5212, 5216, 5219, 5220, 5222, 5224, 5228, 5231, 5232, 5234, 5237, 5239, 5243, 5244, 5246, 5247, 5248, 5250, 5251, 5254, 5255, 5257, 5258, 5259, 5260, 5261, 5262, 5263, 5264, 5265, 5267, 5270, 5271, 5275, 5276, 5277, 5278, 5280, 5282, 5284, 5285, 5287, 5288, 5289, 5291, 5292, 5294, 5295, 5296, 5299, 5300, 5301, 5302, 5304, 5306, 5310, 5311, 5312, 5313, 5314, 5316, 5317, 5319, 5321, 5322, 5323, 5325, 5327, 5331, 5332, 5333, 5334, 5335, 5340, 5341, 5343, 5345, 5348, 5349, 5350, 5351, 5355, 5356, 5358, 5359, 5361, 5364, 5365, 5367, 5368, 5370, 5371, 5372, 5375, 5379, 5381, 5382, 5383, 5384, 5385, 5386, 5388, 5389, 5390, 5392, 5395, 5397, 5402, 5404, 5405, 5406, 5407, 5408, 5410, 5411, 5413, 5415, 5420, 5423, 5425, 5426, 5429, 5432, 5433, 5437, 5438, 5439, 5440, 5441, 5443, 5449, 5451, 5452, 5453, 5454, 5460, 5461, 5462, 5464, 5465, 5468, 5469, 5470, 5471, 5472, 5473, 5474, 5476, 5477, 5478, 5479, 5480, 5481, 5483, 5484, 5485, 5486, 5487, 5488, 5489, 5490, 5491, 5494, 5495, 5497, 5498, 5504, 5505, 5509, 5513, 5516, 5517, 5518, 5519, 5520, 5521, 5522, 5523, 5527, 5528, 5531, 5532, 5533, 5534, 5535, 5536, 5540, 5543, 5545, 5546, 5548, 5549, 5551, 5553, 5554, 5555, 5556, 5558, 5560, 5561, 5564, 5567, 5568, 5569, 5572, 5575, 5576, 5578, 5579, 5580, 5581, 5582, 5583, 5584, 5587, 5588, 5590, 5591, 5592, 5593, 5596, 5597, 5598, 5599, 5600, 5602, 5603, 5605, 5606, 5607, 5608, 5610, 5611, 5614, 5618, 5619, 5624, 5625, 5627, 5629, 5630, 5631, 5633, 5635, 5636, 5637, 5641, 5643, 5644, 5650, 5653, 5654, 5655, 5656, 5658, 5659, 5662, 5668, 5669, 5671, 5672, 5673, 5674, 5675, 5676, 5679, 5680, 5682, 5683, 5684, 5685, 5686, 5687, 5689, 5690, 5692, 5693, 5694, 5695, 5697, 5698, 5699, 5700, 5701, 5703, 5709, 5711, 5712, 5714, 5715, 5718, 5719, 5720, 5721, 5728, 5729, 5731, 5733, 5734, 5736, 5737, 5740, 5741, 5742, 5744, 5745, 5746, 5747, 5749, 5750, 5752, 5755, 5758, 5767, 5769, 5770, 5771, 5772, 5773, 5774, 5775, 5777, 5778, 5780, 5781, 5788, 5789, 5790, 5794, 5795, 5796, 5797, 5798, 5800, 5801, 5803, 5804, 5805, 5806, 5807, 5809, 5810, 5811, 5812, 5814, 5815, 5816, 5817, 5818, 5822, 5823, 5824, 5825, 5829, 5831, 5833, 5836, 5838, 5839, 5840, 5841, 5843, 5844, 5845, 5847, 5848, 5849, 5851, 5853, 5854, 5860, 5861, 5862, 5863, 5865, 5867, 5868, 5873, 5874, 5875, 5876, 5877, 5879, 5880, 5881, 5882, 5883, 5886, 5887, 5888, 5889, 5891, 5892, 5894, 5895, 5896, 5899, 5901, 5904, 5910, 5911, 5913, 5916, 5918, 5920, 5922, 5924, 5925, 5926, 5927, 5928, 5929, 5930, 5931, 5932, 5934, 5935, 5940, 5941, 5943, 5944, 5948, 5949, 5950, 5951, 5952, 5954, 5956, 5957, 5959, 5960, 5961, 5962, 5967, 5968, 5969, 5970, 5971, 5972, 5974, 5975, 5976, 5978, 5979, 5980, 5981, 5982, 5984, 5990, 5991, 5992, 5995, 5996, 5997, 5999, 6001, 6002, 6004, 6008, 6010, 6013, 6014, 6018, 6020, 6021, 6024, 6027, 6036, 6037, 6038, 6040, 6041, 6042, 6043, 6044, 6046, 6047, 6049, 6053, 6054, 6061, 6063, 6065, 6072, 6076, 6077, 6078, 6082, 6085, 6087, 6088, 6091, 6092, 6094, 6099, 6100, 6101, 6102, 6104, 6108, 6111, 6114, 6115, 6116, 6117, 6118, 6123, 6124, 6126, 6127, 6128, 6129, 6130, 6132, 6133, 6134, 6135, 6136, 6142, 6143, 6144, 6145, 6146, 6147, 6148, 6149, 6150, 6151, 6154, 6155, 6157, 6158, 6159, 6160, 6161, 6162, 6166, 6168, 6169, 6170, 6172, 6173, 6175, 6176, 6179, 6180, 6182, 6184, 6185, 6186, 6187, 6194, 6196, 6197, 6198, 6200, 6201, 6202, 6203, 6204, 6206, 6207, 6208, 6210, 6211, 6213, 6214, 6216, 6217, 6218, 6219, 6221, 6222, 6224, 6225, 6226, 6227, 6228, 6229, 6231, 6233, 6234, 6237, 6240, 6246, 6247, 6252, 6253, 6254, 6256, 6259, 6263, 6264, 6266, 6268, 6270, 6271, 6272, 6273, 6274, 6275, 6277, 6278, 6279, 6280, 6282, 6285, 6287, 6288, 6289, 6295, 6297, 6298, 6302, 6303, 6304, 6305, 6306, 6309, 6310, 6311, 6312, 6314, 6316, 6319, 6320, 6321, 6322, 6325, 6326, 6328, 6329, 6336, 6337, 6340, 6342, 6343, 6346, 6347, 6348, 6350, 6351, 6352, 6355, 6356, 6357, 6358, 6359, 6364, 6365, 6366, 6367, 6368, 6369, 6378, 6379, 6380, 6381, 6382, 6385, 6388, 6389, 6391, 6392, 6393, 6394, 6395, 6398, 6401, 6403, 6404, 6405, 6406, 6407, 6408, 6409, 6410, 6411, 6412, 6415, 6416, 6418, 6419, 6420, 6422, 6424, 6425, 6426, 6427, 6428, 6429, 6430, 6432, 6433, 6434, 6436, 6438, 6439, 6441, 6442, 6443, 6444, 6447, 6448, 6451, 6452, 6453, 6454, 6457, 6458, 6459, 6460, 6461, 6463, 6469, 6470, 6473, 6474, 6477, 6479, 6480, 6481, 6482, 6483, 6485, 6486, 6487, 6488, 6489, 6490, 6491, 6496, 6497, 6498, 6499, 6500, 6502, 6508, 6556, 6558, 6560, 6561, 6562, 6564, 6570, 6571, 6577, 6581, 6582, 6584, 6586, 6587, 6588, 6590, 6591, 6593, 6595, 6596, 6597, 6599, 6601, 6602, 6606, 6610, 6612, 6613, 6614, 6615, 6616, 6617, 6618, 6620, 6621, 6622, 6623, 6625, 6627, 6628, 6630, 6631, 6632, 6633, 6634, 6639, 6640, 6643, 6644, 6645, 6646, 6649, 6652, 6653, 6655, 6656, 6657, 6659, 6660, 6666, 6668, 6671, 6672, 6674, 6675, 6676, 6678, 6679, 6681, 6682, 6683, 6684, 6685, 6687, 6689, 6690, 6693, 6701, 6702, 6703, 6705, 6707, 6708, 6709, 6714, 6715, 6716, 6718, 6719, 6720, 6721, 6722, 6723, 6725, 6727, 6731, 6733, 6734, 6737, 6738, 6739, 6746, 6747, 6748, 6750, 6751, 6753, 6754, 6755, 6756, 6757, 6760, 6761, 6764, 6765, 6766, 6767, 6769, 6770, 6771, 6772, 6773, 6775, 6776, 6777, 6778, 6783, 6784, 6785, 6787, 6788, 6789, 6790, 6791, 6794, 6796, 6797, 6798, 6799, 6803, 6805, 6806, 6811, 6812, 6813, 6814, 6815, 6816, 6817, 6818, 6820, 6822, 6824, 6825, 6826, 6828, 6830, 6831, 6832, 6835, 6837, 6839, 6841, 6845, 6846, 6849, 6850, 6851, 6856, 6858, 6859, 6860, 6864, 6865, 6869, 6870, 6873, 6874, 6875, 6876, 6878, 6879, 6880, 6881, 6883, 6884, 6885, 6886, 6888, 6889, 6890, 6891, 6892, 6893, 6894, 6895, 6896, 6898, 6899, 6900, 6901, 6902, 6904, 6905, 6907, 6909, 6910, 6911, 6912, 6914, 6916, 6917, 6921, 6924, 6925, 6926, 6928, 6930, 6931, 6932, 6934, 6935, 6936, 6937, 6938, 6941, 6950, 6952, 6954, 6955, 6956, 6957, 6959, 6961, 6967, 6968, 6970, 6971, 6972, 6974, 6975, 6978, 6980, 6982, 6983, 6985, 6986, 6987, 6988, 6990, 6991, 6992, 6993, 6997, 6998, 7001, 7003, 7005, 7007, 7008, 7009, 7010, 7011, 7012, 7013, 7016, 7018, 7020, 7021, 7024, 7026, 7027, 7028, 7029, 7030, 7031, 7033, 7034, 7035, 7037, 7038, 7039, 7043, 7044, 7047, 7048, 7049, 7050, 7052, 7057, 7059, 7060, 7061, 7062, 7064, 7065, 7066, 7067, 7068, 7070, 7074, 7076, 7078, 7081, 7082, 7083, 7084, 7089, 7094, 7095, 7098, 7099, 7101, 7106, 7107, 7108, 7110, 7113, 7114, 7119, 7120, 7121, 7124, 7128, 7131, 7135, 7136, 7137, 7140, 7141, 7142, 7143, 7146, 7147, 7149, 7150, 7151, 7153, 7154, 7155, 7156, 7159, 7160, 7183, 7184, 7185, 7187, 7189, 7190, 7191, 7192, 7193, 7197, 7198, 7204, 7205, 7207, 7213, 7214, 7215, 7217, 7218, 7220, 7223, 7225, 7228, 7234, 7238, 7240, 7241, 7242, 7243, 7245, 7246, 7247, 7250, 7253, 7254, 7255, 7258, 7259, 7262, 7264, 7265, 7266, 7267, 7269, 7270, 7273, 7276, 7277, 7280, 7282, 7284, 7288, 7291, 7294, 7296, 7301, 7302, 7303, 7306, 7307, 7311, 7312, 7313, 7315, 7318, 7319, 7322, 7323, 7325, 7327, 7328, 7329, 7330, 7336, 7337, 7338, 7340, 7345, 7346, 7347, 7348, 7350, 7355, 7359, 7361, 7362, 7365, 7368, 7369, 7370, 7372, 7376, 7377, 7378, 7574, 7575, 7576, 7580, 7582, 7584, 7585, 7586, 7587, 7588, 7589, 7590, 7593, 7594, 7596, 7599, 7600, 7604, 7606, 7609, 7610, 7611, 7612, 7613, 7614, 7616, 7617, 7618, 7619, 7620, 7621, 7622, 7627, 7629, 7630, 7631, 7633, 7634, 7636, 7638, 7640, 7642, 7644, 7645, 7647, 7648, 7649, 7652, 7653, 7654, 7655, 7656, 7657, 7659, 7660, 7661, 7662, 7663, 7665, 7666, 7667, 7668, 7669, 7671, 7673, 7674, 7675, 7676, 7678, 7683, 7684, 7686, 7689, 7690, 7691, 7692, 7693, 7694, 7695, 7697, 7698, 7699, 7701, 7702, 7703, 7704, 7705, 7706, 7707, 7708, 7711, 7714, 7715, 7717, 7719, 7720, 7721, 7722, 7723, 7724, 7726, 7731, 7733, 7736, 7737, 7738, 7740, 7743, 7746, 7750, 7755, 7757, 7759, 7760, 7761, 7762, 7763, 7764, 7767, 7768, 7770, 7771, 7772, 7775, 7776, 7778, 7781, 7782, 7783, 7784, 7785, 7787, 7788, 7789, 7791, 7793, 7794, 7795, 7796, 7797, 7798, 7799, 7802, 7803, 7804, 7805, 7806, 7807, 7809, 7811, 7812, 7813, 7814, 7816, 7817, 7819, 7820, 7821, 7824, 7825, 7827, 7828, 7829, 7830, 7834, 7836, 7838, 7839, 7841, 7842, 7845, 7846, 7850, 7851, 7852, 7853, 7855, 7856, 7857, 7859, 7861, 7862, 7863, 7865, 7866, 7868, 7869, 7870, 7873, 7875, 7876, 7878, 7880, 7881, 7883, 7884, 7885, 7887, 7888, 7889, 7891, 7895, 7897, 7898, 7899, 7900, 7901, 7902, 7904, 7905, 7906, 7907, 7908, 7909, 7910, 7913, 7916, 7917, 7919, 7920, 7922, 7923, 7924, 7926, 7927, 7930, 7935, 7936, 7938, 7940, 7942, 7943, 7950, 7951, 7952, 7953, 7955, 7956, 7958, 7960, 7961, 7963, 7964, 7965, 7966, 7971, 7972, 7974, 7976, 7979, 7981, 7983, 7984, 7985, 7988, 7990, 7992, 7993, 7994, 7995, 7997, 8000, 8001, 8002, 8004, 8005, 8006, 8007, 8026, 8027, 8028, 8029, 8030, 8031, 8033, 8034, 8035, 8036, 8038, 8041, 8042, 8043, 8044, 8045, 8047, 8048, 8049, 8051, 8052, 8053, 8054, 8056, 8058, 8059, 8061, 8062, 8064, 8067, 8068, 8069, 8070, 8073, 8079, 8080, 8081, 8082, 8083, 8084, 8087, 8088, 8089, 8091, 8092, 8093, 8094, 8098, 8105, 8108, 8109, 8110, 8111, 8112, 8113, 8114, 8115, 8116, 8117, 8118, 8120, 8121, 8124, 8125, 8126, 8127, 8128, 8130, 8131, 8132, 8135, 8137, 8141, 8142, 8143, 8144, 8145, 8147, 8150, 8151, 8152, 8154, 8155, 8156, 8160, 8161, 8163, 8167, 8168, 8171, 8172, 8173, 8175, 8176, 8179, 8181, 8183, 8187, 8189, 8190, 8192, 8193, 8194, 8197, 8201, 8202, 8204, 8212, 8213, 8214, 8215, 8217, 8219, 8222, 8224, 8225, 8226, 8227, 8228, 8229, 8231, 8232, 8233, 8236, 8238, 8239, 8242, 8244, 8248, 8249, 8251, 8254, 8256, 8258, 8259, 8260, 8262, 8264, 8265, 8266, 8267, 8268, 8269, 8270, 8272, 8273, 8274, 8276, 8277, 8278, 8279, 8281, 8284, 8285, 8286, 8287, 8289, 8290, 8291, 8292, 8293, 8294, 8296, 8300, 8301, 8302, 8303, 8307, 8308, 8311, 8312, 8313, 8314, 8316, 8318, 8319, 8320, 8322, 8324, 8326, 8329, 8330, 8331, 8335, 8336, 8337, 8338, 8339, 8341, 8343, 8345, 8348, 8349, 8350, 8351, 8352, 8353, 8354, 8355, 8356, 8359, 8360, 8364, 8367, 8369, 8370, 8375, 8376, 8380, 8381, 8385, 8388, 8389, 8390, 8391, 8393, 8395, 8396, 8397, 8401, 8402, 8403, 8408, 8409, 8411, 8412, 8414, 8415, 8416, 8418, 8420, 8422, 8423, 8427, 8430, 8431, 8432, 8435, 8437, 8439, 8440, 8441, 8443, 8444, 8445, 8446, 8447, 8448, 8449, 8451, 8453, 8454, 8456, 8459, 8464, 8465, 8466, 8467, 8471, 8472, 8474, 8475, 8477, 8482, 8483, 8484, 8485, 8487, 8488, 8489, 8492, 8493, 8495, 8496, 8498, 8499, 8500, 8502, 8503, 8505, 8509, 8510, 8511, 8512, 8513, 8514, 8515, 8518, 8519, 8520, 8521, 8522, 8524, 8525, 8526, 8527, 8529, 8534, 8535, 8537, 8538, 8539, 8540, 8541, 8542, 8543, 8546, 8547, 8549, 8553, 8555, 8556, 8558, 8559, 8560, 8562, 8563, 8567, 8568, 8570, 8571, 8573, 8574, 8575, 8576, 8578, 8579, 8580, 8581, 8583, 8585, 8586, 8588, 8590, 8592, 8594, 8595, 8596, 8597, 8598, 8599, 8601, 8604, 8605, 8606, 8607, 8608, 8609, 8613, 8616, 8617, 8618, 8620, 8621, 8624, 8625, 8626, 8631, 8633, 8637, 8638, 8639, 8641, 8642, 8643, 8644, 8648, 8650, 8651, 8656, 8658, 8659, 8660, 8661, 8662, 8663, 8666, 8668, 8669, 8670, 8671, 8672, 8675, 8676, 8677, 8678, 8680, 8681, 8683, 8685, 8686, 8688, 8689, 8691, 8692, 8694, 8695, 8696, 8697, 8699, 8706, 8707, 8708, 8713, 8715, 8716, 8717, 8718, 8719, 8721, 8722, 8723, 8727, 8730, 8731, 8732, 8733, 8734, 8735, 8738, 8741, 8743, 8745, 8746, 8748, 8751, 8757, 8758, 8759, 8761, 8762, 8763, 8764, 8765, 8766, 8768, 8770, 8771, 8773, 8774, 8775, 8777, 8778, 8782, 8783, 8785, 8788, 8792, 8793, 8794, 8795, 8799, 8800, 8802, 8807, 8808, 8809, 8810, 8811, 8812, 8814, 8815, 8819, 8820, 8821, 8822, 8823, 8824, 8826, 8827, 8828, 8829, 8830, 8831, 8833, 8834, 8837, 8838, 8839, 8840, 8841, 8842, 8847, 8848, 8849, 8850, 8851, 8853, 8855, 8856, 8862, 8863, 8865, 8867, 8871, 8872, 8873, 8874, 8875, 8876, 8877, 8879, 8887, 8888, 8889, 8890, 8895, 8896, 8897, 8898, 8899, 8902, 8903, 8906, 8907, 8909, 8914, 8915, 8916, 8917, 8918, 8919, 8920, 8921, 8922, 8924, 8925, 8926, 8928, 8929, 8932, 8935, 8936, 8937, 8939, 8940, 8941, 8942, 8946, 8947, 8949, 8964, 8967, 8968, 8969, 8970, 8972, 8974, 8979, 8982, 8983, 8985, 8986, 8988, 8989, 8990, 8993, 8994, 8996, 8998, 8999, 9001, 9002, 9003, 9005, 9009, 9010, 9011, 9012, 9013, 9014, 9015, 9016, 9018, 9021, 9022, 9024, 9025, 9027, 9028, 9029, 9030, 9032, 9053, 9058, 9059, 9060, 9062, 9064, 9065, 9066, 9067, 9069, 9070, 9071, 9072, 9073, 9074, 9075, 9077, 9079, 9081, 9082, 9084, 9085, 9086, 9087, 9088, 9089, 9090, 9094, 9095, 9098, 9100, 9101, 9104, 9105, 9106, 9107, 9111, 9113, 9116, 9117, 9119, 9121, 9122, 9123, 9124, 9125, 9126, 9128, 9129, 9130, 9131, 9132, 9133, 9135, 9136, 9137, 9138, 9139, 9141, 9142, 9143, 9144, 9145, 9147, 9148, 9150, 9152, 9153, 9154, 9157, 9158, 9161, 9162, 9165, 9166, 9167, 9170, 9171, 9172, 9173, 9174, 9175, 9177, 9178, 9179, 9181, 9182, 9183, 9184, 9186, 9187, 9188, 9191, 9192, 9194, 9195, 9199, 9200, 9201, 9203, 9205, 9206, 9209, 9210, 9212, 9213, 9215, 9216, 9218, 9219, 9221, 9222, 9223, 9225, 9226, 9227, 9229, 9230, 9231, 9234, 9235, 9237, 9238, 9239, 9240, 9242, 9244, 9247, 9249, 9251, 9252, 9253, 9254, 9256, 9257, 9261, 9263, 9264, 9265, 9267, 9268, 9270, 9272, 9276, 9277, 9279, 9281, 9282, 9285, 9287, 9288, 9289, 9290, 9294, 9295, 9297, 9301, 9302, 9303, 9307, 9309, 9311, 9312, 9313, 9314, 9315, 9316, 9318, 9319, 9320, 9322, 9324, 9326, 9327, 9328, 9330, 9331, 9332, 9333, 9334, 9335, 9336, 9338, 9339, 9340, 9342, 9345, 9346, 9348, 9353, 9354, 9355, 9358, 9359, 9360, 9361, 9365, 9368, 9369, 9370, 9371, 9372, 9375, 9376, 9377, 9379, 9380, 9381, 9383, 9386, 9387, 9391, 9395, 9397, 9398, 9400, 9401, 9402, 9403, 9404, 9406, 9407, 9408, 9409, 9410, 9411, 9413, 9414, 9416, 9418, 9421, 9423, 9424, 9426, 9427, 9429, 9430, 9431, 9432, 9437, 9439, 9440, 9442, 9445, 9448, 9450, 9452, 9455, 9456, 9457, 9458, 9459, 9460, 9461, 9464, 9465, 9467, 9469, 9471, 9474, 9478, 9479, 9481, 9483, 9484, 9485, 9487, 9488, 9489, 9490, 9492, 9493, 9494, 9495, 9498, 9503, 9505, 9507, 9509, 9513, 9515, 9519, 9521, 9526, 9527, 9529, 9530, 9531, 9532, 9534, 9536, 9537, 9538, 9541, 9542, 9543, 9545, 9546, 9548, 9549, 9551, 9552, 9553, 9554, 9555, 9558, 9560, 9566, 9567, 9569, 9571, 9572, 9575, 9576, 9578, 9580, 9581, 9582, 9586, 9588, 9589, 9592, 9596, 9598, 9600, 9603, 9607, 9608, 9609, 9610, 9611, 9612, 9614, 9615, 9617, 9619, 9620, 9621, 9622, 9624, 9625, 9626, 9627, 9628, 9631, 9634, 9636, 9637, 9638, 9639, 9640, 9643, 9644, 9646, 9647, 9649, 9657, 9658, 9660, 9662, 9663, 9664, 9665, 9666, 9668, 9669, 9671, 9673, 9674, 9675, 9676, 9677, 9678, 9681, 9685, 9688, 9691, 9692, 9693, 9694, 9695, 9696, 9697, 9700, 9701, 9702, 9703, 9706, 9707, 9710, 9711, 9712, 9715, 9717, 9718, 9721, 9722, 9725, 9730, 9732, 9733, 9735, 9738, 9739, 9740, 9741, 9742, 9743, 9744, 9745, 9746, 9750, and 9751

In a fourth aspect, the present invention relates to a polynucleotide encoding an artificial variant comprising a substitution, deletion, and/or insertion of one or more (several) amino acids of any of SEQ ID NOs: 4877-9751 or a homologous sequence thereof; or the mature polypeptide thereof. Preferably, amino acid changes are of a minor nature, that is conservative amino acid substitutions or insertions that do not significantly affect the folding and/or activity of the protein; small deletions, typically of one to about 30 amino acids; small amino- or carboxyl-terminal extensions, such as an amino-terminal methionine residue; a small linker peptide of up to about 20-25 residues; or a small extension that facilitates purification by changing net charge or another function, such as a poly-histidine tract, an antigenic epitope or a binding domain.

Examples of conservative substitutions are within the group of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. The most commonly occurring exchanges are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

In addition to the 20 standard amino acids, non-standard amino acids (such as 4-hydroxyproline, 6-N-methyl lysine, 2-aminoisobutyric acid, isovaline, and alpha-methyl serine) may be substituted for amino acid residues of a wild-type polypeptide. A limited number of non-conservative amino acids, amino acids that are not encoded by the genetic code, and unnatural amino acids may be substituted for amino acid residues. "Unnatural amino acids" have been modified after protein synthesis, and/or have a chemical structure in their side chain(s) different from that of the standard amino acids. Unnatural amino acids can be chemically synthesized, and preferably, are commercially available, and include pipecolic acid, thiazolidine carboxylic acid, dehydroproline, 3- and 4-methylproline, and 3,3-dimethylproline.

Alternatively, the amino acid changes are of such a nature that the physico-chemical properties of the polypeptides are altered. For example, amino acid changes may improve the thermal stability of the polypeptide, alter the substrate specificity, change the pH optimum, and the like.

Essential amino acids in the parent polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for biological activity to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64. The identities of essential amino acids can also be inferred from analysis of identities with polypeptides that are related to a polypeptide according to the invention.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (*e.g.*, Lowman et al., 1991, Biochem. 30: 10832-10837; U.S. Patent No. 5,223,409; WO 92/06204), and region-directed mutagenesis (Derbyshire et al., 1986, Gene 46: 145; Ner et al., 1988, DNA 7: 127).

Mutagenesis/shuffling methods can be combined with high-throughput, automated screening methods to detect activity of cloned, mutagenized polypeptides expressed by host cells (Ness et al., 1999, Nature Biotechnology 17: 893-896). Mutagenized DNA molecules that encode active polypeptides can be recovered from the host cells and rapidly sequenced using standard methods in the art. These methods allow the rapid determination of the importance of individual amino acid residues in a polypeptide of interest, and can be applied to polypeptides of unknown structure.

The total number of amino acid substitutions, deletions and/or insertions of any of SEQ ID NOs: 4877-9751 is 10, preferably 9, more preferably 8, more preferably 7, more preferably at most 6, more preferably 5, more preferably 4, even more preferably 3, most preferably 2, and even most preferably 1.

**Biologically Active Substances**

The present invention also relates to isolated biologically active substances, selected from the group consisting of:

(a) a biologically active substance comprising an amino acid sequence having preferably at least 60% identity, more preferably at least 65% identity, more preferably at least 70% identity, more preferably at least 75% identity, more preferably at least 80% identity, more preferably at least 85% identity, more preferably at least 90% identity, even more preferably at least 90% identity, most preferably at least 95% identity, and even most preferably at least 97% identity with an amino acid sequence selected from the group consisting of SEQ ID NOs: 4877-9751 and fragments thereof retaining biological activity;

(b) a biologically active substance encoded by a polynucleotide comprising a nucleotide sequence having preferably at least 60% identity, more preferably at least 65% identity, more preferably at least 70% identity, more preferably at least 75% identity, more preferably at least 80% identity, more preferably at least 85% identity, more preferably at least 90% identity, even more preferably at least 90% identity, most preferably at least 95% identity, and even most preferably at least 97% identity with a polynucleotide selected from the group consisting of SEQ ID NOs: 2-4876 and subsequences thereof encoding fragments having biological activity;

(c) a biologically active substance encoded by a polynucleotide comprising a nucleotide sequence that hybridizes under preferably at least medium stringency conditions, more preferably at least medium-high stringency conditions, or most preferably at least high stringency conditions with a polynucleotide selected from the group consisting of SEQ ID NOs: 2-4876 and full-length complementary strands thereof; and

(d) an artificial variant comprising a substitution, deletion, and/or insertion of one or more (several) amino acids of an amino acid sequence selected from the group consisting of SEQ ID NOs: 4877-9751 and fragments thereof retaining biological activity.

In a first aspect, the present invention also relates to an isolated biologically active substance comprising an amino acid sequence having a degree of sequence identity to any of SEQ ID NOs: 4877-9751 of at least 60%, preferably at least 65%, more preferably at least 70%, more preferably at least 75%, more preferably at least 80%, more preferably at least 85%, even more preferably at least 90%, most preferably at least 95%, and even most preferably at least 97%, which have biological activity (hereinafter "homologous polypeptides"). In a preferred aspect, the homologous polypeptides have an amino acid sequence that differs by ten amino acids, preferably by five amino acids, more preferably by four amino acids, even more preferably by three amino acids, most preferably by two amino acids, and even most preferably by one amino acid from the amino acid sequences of SEQ ID NOs: 4877-9751.

In a preferred aspect, the present invention relates to an isolated biological substance comprising an amino acid sequence having a degree of sequence identity of preferably at least 60% identity, more preferably at least 65% identity, more preferably at least 70% identity, more preferably at least 75% identity, more preferably at least 80%, more preferably at least 85%, even more preferably at least 90%, most preferably at least 95%, and even most preferably at least 97% with an amino acid sequence selected from the group consisting of SEQ ID NOs: 4944, 4985, 5051, 5058, 5064, 5076, 5079, 5097, 5098, 5099, 5106, 5115, 5117, 5136, 5137, 5197, 5204, 5286, 5459, 5529, 5604, 5723, 5724, 5725, 5726, 5761, 5923, 6056, 6097, 6153, 6192, 6371, 6372, 6376, 6456, 6504, 6531, 6545, 6546, 6547, 6548, 6550, 6945, 6965, 7053, 7116, 7163, 7164, 7165, 7166, 7169, 7170, 7174, 7176, 7177, 7363, 7392, 7397, 7399, 7402, 7420, 7421, 7423, 7425, 7438, 7449, 7453, 7468, 7507, 7562, 7563, 7565, 7573, 7641, 7815, 8003, 8008, 8009, 8010, 8011, 8014, 8015, 8018, 8020, 8021, 8022, 8046, 8099, 8100, 8103, 8140, 8323, 8710, 8711, 8966, 9034, 9035, 9036, 9049, 9051, 9054, 9056, 9057, 9224, 9516, 9525, 9547, 9562, 9564, 4900, 4925, 4930, 4952, 4957, 4966, 4973, 4978, 5000, 5010, 5044, 5046, 5047, 5049, 5052, 5053, 5054, 5055, 5056, 5057, 5060, 5061, 5062, 5067, 5069, 5070, 5071, 5073, 5074, 5078, 5080, 5081, 5082, 5083, 5084, 5085, 5086, 5087, 5088, 5089, 5090, 5091, 5092, 5093, 5094, 5095, 5096, 5100, 5101, 5102, 5103, 5104, 5105, 5112, 5116, 5118, 5119, 5120, 5121, 5122, 5123, 5124, 5125, 5126, 5127, 5128, 5129, 5130, 5131, 5132, 5133, 5134, 5135, 5138, 5139, 5140, 5141, 5151, 5162, 5173, 5180, 5181, 5183, 5192, 5198, 5225, 5229, 5235, 5236, 5238, 5242, 5249, 5256, 5290, 5297, 5307, 5320, 5344, 5347, 5353, 5354, 5362, 5363, 5366, 5373, 5391, 5396, 5398, 5399, 5412, 5416, 5428, 5445, 5450, 5456, 5467, 5482, 5493, 5496, 5502, 5503, 5506, 5541, 5547, 5563, 5566, 5574, 5577, 5585, 5594, 5601, 5609, 5623, 5626, 5639, 5648, 5649, 5652, 5664, 5666, 5702, 5704, 5706, 5708, 5717, 5722, 5730, 5732, 5760, 5762, 5776, 5784, 5819, 5832, 5852, 5857, 5866, 5869, 5890, 5893, 5897, 5907, 5909, 5912, 5933, 5953, 5955, 5963, 5966, 5973, 5983, 5986, 5987, 5993, 6003, 6005, 6023, 6026, 6028, 6029, 6033, 6035, 6039, 6045, 6050, 6051, 6052, 6055, 6058, 6059, 6068, 6069, 6074, 6083, 6090, 6095, 6112, 6119, 6137, 6138, 6140, 6141, 6167, 6177, 6188, 6189, 6190, 6191, 6193, 6195, 6209, 6220, 6230, 6245, 6248, 6249, 6276, 6286, 6294, 6296, 6299, 6307, 6308, 6315, 6317, 6323, 6330, 6341, 6345, 6384, 6402, 6414, 6431, 6440, 6445, 6464, 6494, 6506, 6509, 6510, 6511, 6513, 6514, 6515, 6516, 6517, 6518, 6519, 6520, 6521, 6522, 6524, 6525, 6526, 6527, 6528, 6529, 6530, 6532, 6533, 6534, 6535, 6536, 6537, 6538, 6539, 6540, 6541, 6542, 6543, 6544, 6552, 6557, 6559, 6563, 6567, 6569, 6572, 6578, 6579, 6603, 6607, 6609, 6611, 6626, 6650, 6654, 6658, 6661, 6667, 6670, 6673, 6691, 6706, 6711, 6713, 6726, 6728, 6741, 6758, 6762, 6819, 6821, 6877, 6882, 6897, 6913, 6919, 6923, 6940, 6942, 6947, 6951, 6958, 6969, 6981, 6984, 6994, 6995, 6996, 6999, 7006, 7014, 7022, 7023, 7041, 7046, 7069, 7071, 7073, 7079, 7080, 7086, 7088, 7091, 7096, 7100, 7105, 7111, 7115, 7129, 7130, 7133, 7138, 7148, 7152, 7157, 7161, 7167, 7171, 7172, 7173, 7175, 7178, 7179, 7180, 7199, 7200, 7202, 7211, 7212, 7216, 7222, 7226, 7229, 7237, 7239, 7256, 7263, 7275, 7278, 7279, 7283, 7285, 7286, 7287, 7289, 7290, 7299, 7300, 7305, 7310, 7314, 7321, 7324, 7332, 7339, 7342, 7343, 7344, 7351, 7358, 7366, 7373, 7375, 7379, 7380, 7381, 7382, 7383, 7384, 7385, 7386, 7387, 7388, 7389, 7390, 7391, 7393, 7394, 7395, 7396, 7400, 7401, 7403, 7404, 7405, 7406, 7407, 7408, 7409, 7410, 7411, 7412, 7413, 7414, 7415, 7416, 7417, 7418, 7419, 7422, 7424, 7426, 7427, 7428, 7429, 7430, 7431, 7432, 7433, 7434, 7435, 7436, 7437, 7439, 7440, 7442, 7443, 7444, 7445, 7446, 7447, 7448, 7450, 7451, 7452, 7454, 7455, 7456, 7457, 7458, 7459, 7460, 7461, 7462, 7463, 7464, 7465, 7466, 7467, 7469, 7470, 7471, 7472, 7473, 7474, 7475, 7476, 7477, 7478, 7479, 7480, 7481, 7482, 7483, 7484, 7485, 7486, 7487, 7488, 7489, 7490, 7491, 7492, 7493, 7494, 7495, 7496, 7497, 7498, 7499, 7500, 7501, 7502, 7503, 7504, 7505, 7506, 7508, 7509, 7510, 7511, 7513, 7514, 7515, 7516, 7517, 7518, 7519, 7520, 7521, 7522, 7523, 7524, 7525, 7526, 7527, 7528, 7529, 7530, 7531, 7532, 7533, 7534, 7536, 7539, 7540, 7542, 7544, 7545, 7546, 7547, 7548, 7549, 7550, 7551, 7552, 7553, 7554, 7555, 7556, 7559, 7560, 7561, 7564, 7566, 7567, 7568, 7571, 7572, 7581, 7583, 7592, 7595, 7598, 7605, 7608, 7626, 7637, 7646, 7670, 7681, 7688, 7709, 7710, 7716, 7725, 7741, 7742, 7749, 7753, 7758, 7766, 7773, 7777, 7779, 7792, 7822, 7832, 7835, 7844, 7847, 7848, 7874, 7879, 7893, 7894, 7903, 7921, 7929, 7939, 7944, 7957, 7962, 7968, 7987, 7998, 8012, 8016, 8017, 8019, 8023, 8024, 8025, 8037, 8090, 8097, 8101, 8102, 8104, 8106, 8119, 8123, 8134, 8136, 8139, 8162, 8166, 8174, 8178, 8185, 8186, 8196, 8198, 8199, 8208, 8230, 8237, 8246, 8299, 8304, 8306, 8309, 8317, 8321, 8332, 8333, 8346, 8361, 8368, 8371, 8374, 8378, 8379, 8383, 8384, 8394, 8424, 8433, 8452, 8462, 8468, 8473, 8476, 8478, 8481, 8486, 8517, 8528, 8530, 8545, 8548, 8557, 8565, 8569, 8582, 8584, 8591, 8602, 8610, 8611, 8612, 8619, 8628, 8640, 8645, 8655, 8665, 8667, 8673, 8674, 8693, 8700, 8714, 8747, 8754, 8756, 8760, 8779, 8786, 8790, 8791, 8797, 8801, 8806, 8813, 8844, 8886, 8892, 8893, 8908, 8912, 8923, 8930, 8931, 8934, 8943, 8951, 8952, 8953, 8956, 8957, 8960, 8961, 8965, 8981, 8987, 8992, 9019, 9020, 9033, 9037, 9038, 9039, 9040, 9041, 9042, 9043, 9044, 9045, 9046, 9047, 9048, 9050, 9076, 9096, 9110, 9114, 9118, 9160, 9163, 9180, 9198, 9208, 9211, 9228, 9245, 9273, 9284, 9292, 9296, 9298, 9304, 9310, 9321, 9323, 9325, 9337, 9344, 9347, 9349, 9351, 9357, 9362, 9363, 9374, 9384, 9385, 9388, 9393, 9419, 9433, 9434, 9435, 9436, 9444, 9446, 9453, 9475, 9480, 9482, 9497, 9506, 9508, 9518, 9520, 9522, 9523, 9524, 9533, 9544, 9557, 9568, 9579, 9583, 9593, 9597, 9629, 9642, 9661, 9667, 9672, 9679, 9687, 9689, 9698, 9705, 9708, 9713, 9723, 9724, 9729, 9734, 9737, and 9747 ; preferably at least 65% identity, more preferably at least 70% identity, more preferably at least 75% identity, more preferably at least 80%, more preferably at least 85%, even more preferably at least 90%, most preferably at least 95%, and even most preferably at least 97% with an amino acid sequence selected from the group consisting of SEO ID NOs: 4991, 5068, 5308, 5463, 5919, 6549, 6648, 6680, 6948, 7298, 7541, 7896, 7911, 8497, 8702, 8729, 8737, 9052, 9690, and 9709; preferably at least 70% identity, more preferably at least 75% identity, more preferably at least 80%, more preferably at least 85%, even more preferably at least 90%, most preferably at least 95%, and even most preferably at least 97% with an amino acid sequence selected from the group consisting of SEQ ID NOs: 4986, 5041, 5043, 5418, 5427, 5500, 5515, 5696, 5713, 5785, 5939, 6106, 6109, 6171, 6260, 6503, 6677, 6724, 7054, 7260, 7295, 7316, 7341, 7512, 7538, 7591, 7628, 7735, 7912, 8050, 8076, 8206, 8207, 8240, 8327, 8406, 8428, 8860, 8955, 9061, 9099, 9115, 9262, 9591, 9641, and 9720; preferably at least 75% identity, more preferably at least 80%, more preferably at least 85%, even more preferably at least 90%, most preferably at least 95%, and even most preferably at least 97% with an amino acid sequence selected from the group consisting of SEQ ID NOs: 5038, 5059, 5240, 5403, 5444, 5508, 5559, 5562, 5642, 5651, 5846, 5885, 5921, 5942, 6015, 6067, 6071, 6164, 6215, 6293, 6373, 6390, 6446, 6624, 6704, 6717, 6749, 6759, 6808, 6847, 6848, 6852, 6854, 7292, 7297, 7308, 7317, 7537, 7543, 7557, 7603, 7651, 7858, 7949, 7954, 7996, 8074, 8200, 8221, 8263, 8288, 8614, 8684, 8720, 8728, 8740, 8749, 8787, 8864, 8958, 9055, 9176, 9196, 9283, 9308, 9352, 9491, 9512, 9602, 9653, 9684, 9719, and 9731; preferably at least 80% identity, more preferably at least 85% identity, even more preferably at least 90% identity, most preferably at least 95% identity, and even most preferably at least 97% identity with an amino acid sequence selected from the group consisting of SEQ ID NOs: 4891, 4956, 5042, 5072, 5169, 5190, 5195, 5217, 5241, 5266, 5305, 5369, 5374, 5377, 5409, 5435, 5512, 5524, 5538, 5595, 5613, 5647, 5657, 5665, 5667, 5735, 5743, 5830, 5878, 5900, 5903, 5977, 5988, 6000, 6060, 6066, 6070, 6073, 6075, 6105, 6120, 6139, 6152, 6174, 6291, 6335, 6375, 6437, 6466, 6492, 6505, 6507, 6523, 6553, 6592, 6651, 6694, 6730, 6732, 6774, 6843, 6929, 6943, 6964, 7093, 7132, 7144, 7224, 7349, 7374, 7398, 7570, 7578, 7632, 7635, 7643, 7680, 7682, 7685, 7700, 7713, 7727, 7800, 7843, 7864, 7918, 7947, 7982, 7989, 7991, 7999, 8055, 8057, 8085, 8095, 8170, 8182, 8191, 8210, 8255, 8261, 8328, 8365, 8399, 8410, 8479, 8551, 8561, 8577, 8589, 8649, 8679, 8742, 8744, 8784, 8789, 8796, 8818, 8832, 8835, 8845, 8854, 8858, 8868, 8878, 8900, 8950, 9007, 9102, 9108, 9151, 9169, 9207, 9241, 9246, 9248, 9250, 9299, 9341, 9343, 9412, 9443, 9470, 9476, 9511, 9528, 9559, 9565, 9577, 9584, 9594, 9595, 9682, and 9714; preferably at least 85% identity, more preferably at least 90% identity, most preferably at least 95% identity, and even most preferably at least 97% identity with an amino acid sequence selected from the group consisting of SEQ ID NOs: 4913, 4948, 4968, 5025, 5034, 5066, 5109, 5110, 5146, 5160, 5199, 5200, 5203, 5211, 5221, 5223, 5226, 5252, 5269, 5273, 5293, 5309, 5318, 5342, 5352, 5393, 5394, 5421, 5430, 5431, 5434, 5447, 5458, 5492, 5507, 5511, 5514, 5525, 5526, 5530, 5544, 5570, 5616, 5617, 5621, 5640, 5661, 5663, 5691, 5716, 5738, 5751, 5753, 5757, 5759, 5786, 5802, 5820, 5826, 5855, 5858, 5871, 5872, 5902, 5905, 5908, 5915, 5936, 5937, 5938, 5947, 6006, 6011, 6012, 6025, 6030, 6032, 6034, 6057, 6080, 6084, 6093, 6096, 6110, 6131, 6178, 6183, 6199, 6232, 6236, 6243, 6257, 6258, 6313, 6331, 6354, 6360, 6362, 6386, 6417, 6421, 6435, 6455, 6468, 6472, 6475, 6476, 6478, 6495, 6555, 6566, 6568, 6604, 6608, 6641, 6662, 6663, 6665, 6692, 6699, 6736, 6752, 6763, 6807, 6809, 6810, 6840, 6842, 6844, 6853, 6855, 6857, 6866, 6906, 6908, 6915, 6922, 6953, 6962, 6977, 6989, 7004, 7025, 7032, 7042, 7045, 7058, 7072, 7085, 7134, 7139, 7145, 7182, 7188, 7194, 7208, 7227, 7249, 7261, 7272, 7293, 7309, 7320, 7331, 7333, 7334, 7354, 7367, 7371, 7558, 7597, 7602, 7623, 7664, 7677, 7679, 7687, 7732, 7734, 7739, 7744, 7752, 7765, 7769, 7790, 7801, 7818, 7823, 7833, 7837, 7860, 7867, 7877, 7882, 7915, 7931, 7932, 7946, 7948, 7959, 7967, 7973, 7975, 7977, 7978, 7986, 8071, 8072, 8096, 8107, 8129, 8146, 8148, 8153, 8164, 8165, 8169, 8203, 8211, 8216, 8218, 8234, 8245, 8253, 8275, 8297, 8315, 8334, 8340, 8357, 8362, 8372, 8386, 8398, 8425, 8438, 8455, 8460, 8470, 8480, 8490, 8506, 8531, 8533, 8572, 8593, 8600, 8615, 8623, 8627, 8630, 8634, 8664, 8687, 8703, 8704, 8705, 8726, 8739, 8750, 8752, 8753, 8769, 8780, 8781, 8805, 8816, 8825, 8846, 8869, 8870, 8883, 8913, 8938, 8944, 8945, 8948, 8959, 8962, 8973, 8976, 8980, 8991, 9000, 9006, 9017, 9068, 9093, 9097, 9103, 9109, 9146, 9155, 9159, 9255, 9258, 9260, 9266, 9269, 9271, 9278, 9293, 9350, 9373, 9394, 9415, 9420, 9449, 9466, 9472, 9477, 9486, 9500, 9504, 9514, 9517, 9535, 9573, 9590, 9599, 9601, 9623, 9645, 9648, 9652, 9654, 9659, 9726, 9728, 9736, and 9748; preferably at least 90% identity, more preferably at least 95% identity, and most preferably at least 97% identity with an amino acid sequence selected from the group consisting ofSEQ ID NOs: 4890, 4895, 4921, 4923, 4927, 4933, 4934, 4937, 4939, 4943, 4946, 4950, 4951, 4955, 4958, 4967, 4970, 4981, 4982, 4987, 4989, 4999, 5017, 5022, 5029, 5048, 5111, 5113, 5145, 5152, 5157, 5165, 5171, 5178, 5179, 5182, 5186, 5202, 5213, 5214, 5215, 5218, 5227, 5230, 5233, 5245, 5268, 5272, 5274, 5279, 5281, 5283, 5298, 5303, 5315, 5324, 5326, 5328, 5329, 5330, 5336, 5337, 5338, 5339, 5346, 5357, 5360, 5376, 5380, 5387, 5400, 5401, 5414, 5417, 5419, 5422, 5424, 5436, 5442, 5446, 5448, 5455, 5457, 5466, 5475, 5499, 5501, 5510, 5537, 5539, 5542, 5550, 5552, 5557, 5565, 5571, 5573, 5586, 5589, 5612, 5615, 5620, 5622, 5628, 5632, 5634, 5638, 5645, 5646, 5660, 5670, 5677, 5678, 5681, 5688, 5705, 5707, 5710, 5739, 5748, 5754, 5756, 5763, 5764, 5765, 5766, 5768, 5779, 5782, 5783, 5787, 5791, 5792, 5793, 5799, 5808, 5813, 5821, 5827, 5828, 5834, 5835, 5837, 5842, 5850, 5856, 5859, 5864, 5870, 5884, 5906, 5914, 5917, 5945, 5946, 5958, 5964, 5965, 5985, 5989, 5994, 5998, 6007, 6009, 6016, 6017, 6019, 6022, 6031, 6048, 6062, 6064, 6079, 6081, 6086, 6089, 6098, 6103, 6107, 6113, 6121, 6122, 6125, 6156, 6163, 6165, 6181, 6205, 6212, 6223, 6235, 6238, 6239, 6242, 6244, 6250, 6251, 6255, 6261, 6262, 6265, 6267, 6269, 6281, 6283, 6284, 6290, 6292, 6300, 6301, 6318, 6324, 6327, 6332, 6333, 6338, 6339, 6344, 6349, 6353, 6361, 6363, 6374, 6377, 6383, 6387, 6396, 6397, 6399, 6400, 6413, 6423, 6449, 6450, 6462, 6465, 6467, 6471, 6484, 6493, 6501, 6512, 6565, 6573, 6574, 6575, 6576, 6580, 6583, 6585, 6589, 6594, 6598, 6600, 6605, 6619, 6629, 6635, 6636, 6637, 6638, 6642, 6647, 6664, 6669, 6686, 6688, 6695, 6696, 6697, 6698, 6700, 6710, 6712, 6729, 6735, 6740, 6742, 6743, 6744, 6745, 6768, 6779, 6780, 6781, 6782, 6786, 6792, 6793, 6795, 6800, 6801, 6802, 6804, 6823, 6827, 6829, 6833, 6834, 6836, 6838, 6861, 6862, 6863, 6867, 6868, 6871, 6872, 6887, 6903, 6918, 6920, 6927, 6933, 6939, 6944, 6949, 6960, 6966, 6973, 6976, 6979, 7000, 7002, 7015, 7017, 7019, 7036, 7040, 7051, 7055, 7056, 7063, 7075, 7077, 7087, 7090, 7092, 7097, 7102, 7103, 7104, 7109, 7112, 7117, 7118, 7122, 7123, 7125, 7126, 7127, 7158, 7162, 7181, 7186, 7196, 7201, 7203, 7206, 7209, 7210, 7219, 7221, 7230, 7231, 7232, 7233, 7235, 7236, 7244, 7248, 7251, 7252, 7257, 7268, 7271, 7274, 7281, 7304, 7326, 7335, 7352, 7356, 7357, 7360, 7364, 7577, 7579, 7601, 7607, 7615, 7624, 7625, 7639, 7650, 7658, 7672, 7696, 7712, 7718, 7728, 7729, 7730, 7745, 7747, 7748, 7751, 7754, 7756, 7774, 7780, 7786, 7808, 7810, 7826, 7831, 7840, 7849, 7854, 7871, 7872, 7886, 7892, 7914, 7925, 7928, 7933, 7934, 7937, 7941, 7945, 7969, 7970, 7980, 8032, 8039, 8040, 8060, 8063, 8065, 8066, 8075, 8077, 8078, 8086, 8122, 8133, 8138, 8149, 8157, 8158, 8159, 8177, 8180, 8184, 8188, 8195, 8205, 8209, 8220, 8223, 8235, 8241, 8243, 8247, 8250, 8252, 8257, 8271, 8280, 8282, 8283, 8295, 8298, 8305, 8310, 8325, 8342, 8344, 8347, 8358, 8363, 8366, 8373, 8377, 8382, 8387, 8392, 8400, 8404, 8405, 8407, 8413, 8417, 8419, 8421, 8426, 8429, 8434, 8436, 8442, 8450, 8457, 8458, 8461, 8463, 8469, 8491, 8494, 8501, 8504, 8507, 8508, 8516, 8523, 8532, 8536, 8544, 8550, 8552, 8554, 8564, 8566, 8587, 8603, 8622, 8629, 8632, 8635, 8636, 8646, 8647, 8652, 8653, 8654, 8657, 8682, 8690, 8698, 8701, 8709, 8712, 8724, 8725, 8736, 8755, 8767, 8772, 8776, 8798, 8803, 8804, 8817, 8836, 8843, 8852, 8857, 8859, 8861, 8866, 8880, 8881, 8882, 8884, 8885, 8891, 8894, 8901, 8904, 8905, 8910, 8911, 8927, 8933, 8954, 8963, 8971, 8975, 8977, 8984, 8995, 8997, 9004, 9008, 9023, 9026, 9031, 9063, 9078, 9080, 9083, 9091, 9092, 9112, 9120, 9127, 9134, 9140, 9149, 9156, 9164, 9168, 9185, 9189, 9190, 9193, 9197, 9202, 9204, 9214, 9217, 9220, 9232, 9233, 9236, 9243, 9259, 9274, 9275, 9280, 9286, 9291, 9300, 9305, 9306, 9317, 9329, 9356, 9364, 9366, 9367, 9378, 9382, 9389, 9390, 9392, 9396, 9399, 9405, 9417, 9422, 9425, 9428, 9438, 9441, 9451, 9454, 9462, 9463, 9468, 9473, 9496, 9499, 9501, 9502, 9510, 9539, 9540, 9550, 9556, 9561, 9570, 9574, 9585, 9587, 9604, 9605, 9606, 9613, 9616, 9618, 9630, 9632, 9633, 9635, 9650, 9651, 9655, 9656, 9670, 9680, 9683, 9686, 9699, 9704, 9716, 9727, and 9749; or preferably at least 95% identity and more preferably at least 97% identity with an amino acid sequence selected from the group consisting of SEQ ID NOs: 4877, 4878, 4879, 4880, 4881, 4882, 4883, 4884, 4885, 4886, 4887, 4888, 4889, 4892, 4893, 4894, 4896, 4897, 4898, 4899, 4901, 4902, 4903, 4904, 4905, 4906, 4907, 4908, 4909, 4910, 4911, 4912, 4914, 4915, 4916, 4917, 4918, 4919, 4920, 4922, 4924, 4926, 4928, 4929, 4931, 4932, 4935, 4936, 4938, 4940, 4941, 4942, 4945, 4947, 4949, 4953, 4954, 4959, 4960, 4961, 4962, 4963, 4964, 4965, 4969, 4971, 4972, 4974, 4975, 4976, 4977, 4979, 4980, 4983, 4984, 4988, 4990, 4992, 4993, 4994, 4995, 4996, 4997, 4998, 5001, 5002, 5003, 5004, 5005, 5006, 5007, 5008, 5009, 5011, 5012, 5013, 5014, 5015, 5016, 5018, 5019, 5020, 5021, 5023, 5024, 5026, 5027, 5028, 5030, 5031, 5032, 5033, 5035, 5036, 5037, 5039, 5045, 5050, 5063, 5065, 5075, 5077, 5107, 5108, 5114, 5142, 5143, 5144, 5147, 5148, 5149, 5150, 5153, 5154, 5155, 5156, 5158, 5159, 5161, 5163, 5164, 5166, 5167, 5168, 5170, 5172, 5174, 5175, 5176, 5177, 5184, 5185, 5187, 5188, 5189, 5191, 5193, 5194, 5196, 5201, 5205, 5206, 5207, 5208, 5209, 5210, 5212, 5216, 5219, 5220, 5222, 5224, 5228, 5231, 5232, 5234, 5237, 5239, 5243, 5244, 5246, 5247, 5248, 5250, 5251, 5254, 5255, 5257, 5258, 5259, 5260, 5261, 5262, 5263, 5264, 5265, 5267, 5270, 5271, 5275, 5276, 5277, 5278, 5280, 5282, 5284, 5285, 5287, 5288, 5289, 5291, 5292, 5294, 5295, 5296, 5299, 5300, 5301, 5302, 5304, 5306, 5310, 5311, 5312, 5313, 5314, 5316, 5317, 5319, 5321, 5322, 5323, 5325, 5327, 5331, 5332, 5333, 5334, 5335, 5340, 5341, 5343, 5345, 5348, 5349, 5350, 5351, 5355, 5356, 5358, 5359, 5361, 5364, 5365, 5367, 5368, 5370, 5371, 5372, 5375, 5379, 5381, 5382, 5383, 5384, 5385, 5386, 5388, 5389, 5390, 5392, 5395, 5397, 5402, 5404, 5405, 5406, 5407, 5408, 5410, 5411, 5413, 5415, 5420, 5423, 5425, 5426, 5429, 5432, 5433, 5437, 5438, 5439, 5440, 5441, 5443, 5449, 5451, 5452, 5453, 5454, 5460, 5461, 5462, 5464, 5465, 5468, 5469, 5470, 5471, 5472, 5473, 5474, 5476, 5477, 5478, 5479, 5480, 5481, 5483, 5484, 5485, 5486, 5487, 5488, 5489, 5490, 5491, 5494, 5495, 5497, 5498, 5504, 5505, 5509, 5513, 5516, 5517, 5518, 5519, 5520, 5521, 5522, 5523, 5527, 5528, 5531, 5532, 5533, 5534, 5535, 5536, 5540, 5543, 5545, 5546, 5548, 5549, 5551, 5553, 5554, 5555, 5556, 5558, 5560, 5561, 5564, 5567, 5568, 5569, 5572, 5575, 5576, 5578, 5579, 5580, 5581, 5582, 5583, 5584, 5587, 5588, 5590, 5591, 5592, 5593, 5596, 5597, 5598, 5599, 5600, 5602, 5603, 5605, 5606, 5607, 5608, 5610, 5611, 5614, 5618, 5619, 5624, 5625, 5627, 5629, 5630, 5631, 5633, 5635, 5636, 5637, 5641, 5643, 5644, 5650, 5653, 5654, 5655, 5656, 5658, 5659, 5662, 5668, 5669, 5671, 5672, 5673, 5674, 5675, 5676, 5679, 5680, 5682, 5683, 5684, 5685, 5686, 5687, 5689, 5690, 5692, 5693, 5694, 5695, 5697, 5698, 5699, 5700, 5701, 5703, 5709, 5711, 5712, 5714, 5715, 5718, 5719, 5720, 5721, 5728, 5729, 5731, 5733, 5734, 5736, 5737, 5740, 5741, 5742, 5744, 5745, 5746, 5747, 5749, 5750, 5752, 5755, 5758, 5767, 5769, 5770, 5771, 5772, 5773, 5774, 5775, 5777, 5778, 5780, 5781, 5788, 5789, 5790, 5794, 5795, 5796, 5797, 5798, 5800, 5801, 5803, 5804, 5805, 5806, 5807, 5809, 5810, 5811, 5812, 5814, 5815, 5816, 5817, 5818, 5822, 5823, 5824, 5825, 5829, 5831, 5833, 5836, 5838, 5839, 5840, 5841, 5843, 5844, 5845, 5847, 5848, 5849, 5851, 5853, 5854, 5860, 5861, 5862, 5863, 5865, 5867, 5868, 5873, 5874, 5875, 5876, 5877, 5879, 5880, 5881, 5882, 5883, 5886, 5887, 5888, 5889, 5891, 5892, 5894, 5895, 5896, 5899, 5901, 5904, 5910, 5911, 5913, 5916, 5918, 5920, 5922, 5924, 5925, 5926, 5927, 5928, 5929, 5930, 5931, 5932, 5934, 5935, 5940, 5941, 5943, 5944, 5948, 5949, 5950, 5951, 5952, 5954, 5956, 5957, 5959, 5960, 5961, 5962, 5967, 5968, 5969, 5970, 5971, 5972, 5974, 5975, 5976, 5978, 5979, 5980, 5981, 5982, 5984, 5990, 5991, 5992, 5995, 5996, 5997, 5999, 6001, 6002, 6004, 6008, 6010, 6013, 6014, 6018, 6020, 6021, 6024, 6027, 6036, 6037, 6038, 6040, 6041, 6042, 6043, 6044, 6046, 6047, 6049, 6053, 6054, 6061, 6063, 6065, 6072, 6076, 6077, 6078, 6082, 6085, 6087, 6088, 6091, 6092, 6094, 6099, 6100, 6101, 6102, 6104, 6108, 6111, 6114, 6115, 6116, 6117, 6118, 6123, 6124, 6126, 6127, 6128, 6129, 6130, 6132, 6133, 6134, 6135, 6136, 6142, 6143, 6144, 6145, 6146, 6147, 6148, 6149, 6150, 6151, 6154, 6155, 6157, 6158, 6159, 6160, 6161, 6162, 6166, 6168, 6169, 6170, 6172, 6173, 6175, 6176, 6179, 6180, 6182, 6184, 6185, 6186, 6187, 6194, 6196, 6197, 6198, 6200, 6201, 6202, 6203, 6204, 6206, 6207, 6208, 6210, 6211, 6213, 6214, 6216, 6217, 6218, 6219, 6221, 6222, 6224, 6225, 6226, 6227, 6228, 6229, 6231, 6233, 6234, 6237, 6240, 6246, 6247, 6252, 6253, 6254, 6256, 6259, 6263, 6264, 6266, 6268, 6270, 6271, 6272, 6273, 6274, 6275, 6277, 6278, 6279, 6280, 6282, 6285, 6287, 6288, 6289, 6295, 6297, 6298, 6302, 6303, 6304, 6305, 6306, 6309, 6310, 6311, 6312, 6314, 6316, 6319, 6320, 6321, 6322, 6325, 6326, 6328, 6329, 6336, 6337, 6340, 6342, 6343, 6346, 6347, 6348, 6350, 6351, 6352, 6355, 6356, 6357, 6358, 6359, 6364, 6365, 6366, 6367, 6368, 6369, 6378, 6379, 6380, 6381, 6382, 6385, 6388, 6389, 6391, 6392, 6393, 6394, 6395, 6398, 6401, 6403, 6404, 6405, 6406, 6407, 6408, 6409, 6410, 6411, 6412, 6415, 6416, 6418, 6419, 6420, 6422, 6424, 6425, 6426, 6427, 6428, 6429, 6430, 6432, 6433, 6434, 6436, 6438, 6439, 6441, 6442, 6443, 6444, 6447, 6448, 6451, 6452, 6453, 6454, 6457, 6458, 6459, 6460, 6461, 6463, 6469, 6470, 6473, 6474, 6477, 6479, 6480, 6481, 6482, 6483, 6485, 6486, 6487, 6488, 6489, 6490, 6491, 6496, 6497, 6498, 6499, 6500, 6502, 6508, 6556, 6558, 6560, 6561, 6562, 6564, 6570, 6571, 6577, 6581, 6582, 6584, 6586, 6587, 6588, 6590, 6591, 6593, 6595, 6596, 6597, 6599, 6601, 6602, 6606, 6610, 6612, 6613, 6614, 6615, 6616, 6617, 6618, 6620, 6621, 6622, 6623, 6625, 6627, 6628, 6630, 6631, 6632, 6633, 6634, 6639, 6640, 6643, 6644, 6645, 6646, 6649, 6652, 6653, 6655, 6656, 6657, 6659, 6660, 6666, 6668, 6671, 6672, 6674, 6675, 6676, 6678, 6679, 6681, 6682, 6683, 6684, 6685, 6687, 6689, 6690, 6693, 6701, 6702, 6703, 6705, 6707, 6708, 6709, 6714, 6715, 6716, 6718, 6719, 6720, 6721, 6722, 6723, 6725, 6727, 6731, 6733, 6734, 6737, 6738, 6739, 6746, 6747, 6748, 6750, 6751, 6753, 6754, 6755, 6756, 6757, 6760, 6761, 6764, 6765, 6766, 6767, 6769, 6770, 6771, 6772, 6773, 6775, 6776, 6777, 6778, 6783, 6784, 6785, 6787, 6788, 6789, 6790, 6791, 6794, 6796, 6797, 6798, 6799, 6803, 6805, 6806, 6811, 6812, 6813, 6814, 6815, 6816, 6817, 6818, 6820, 6822, 6824, 6825, 6826, 6828, 6830, 6831, 6832, 6835, 6837, 6839, 6841, 6845, 6846, 6849, 6850, 6851, 6856, 6858, 6859, 6860, 6864, 6865, 6869, 6870, 6873, 6874, 6875, 6876, 6878, 6879, 6880, 6881, 6883, 6884, 6885, 6886, 6888, 6889, 6890, 6891, 6892, 6893, 6894, 6895, 6896, 6898, 6899, 6900, 6901, 6902, 6904, 6905, 6907, 6909, 6910, 6911, 6912, 6914, 6916, 6917, 6921, 6924, 6925, 6926, 6928, 6930, 6931, 6932, 6934, 6935, 6936, 6937, 6938, 6941, 6950, 6952, 6954, 6955, 6956, 6957, 6959, 6961, 6967, 6968, 6970, 6971, 6972, 6974, 6975, 6978, 6980, 6982, 6983, 6985, 6986, 6987, 6988, 6990, 6991, 6992, 6993, 6997, 6998, 7001, 7003, 7005, 7007, 7008, 7009, 7010, 7011, 7012, 7013, 7016, 7018, 7020, 7021, 7024, 7026, 7027, 7028, 7029, 7030, 7031, 7033, 7034, 7035, 7037, 7038, 7039, 7043, 7044, 7047, 7048, 7049, 7050, 7052, 7057, 7059, 7060, 7061, 7062, 7064, 7065, 7066, 7067, 7068, 7070, 7074, 7076, 7078, 7081, 7082, 7083, 7084, 7089, 7094, 7095, 7098, 7099, 7101, 7106, 7107, 7108, 7110, 7113, 7114, 7119, 7120, 7121, 7124, 7128, 7131, 7135, 7136, 7137, 7140, 7141, 7142, 7143, 7146, 7147, 7149, 7150, 7151, 7153, 7154, 7155, 7156, 7159, 7160, 7183, 7184, 7185, 7187, 7189, 7190, 7191, 7192, 7193, 7197, 7198, 7204, 7205, 7207, 7213, 7214, 7215, 7217, 7218, 7220, 7223, 7225, 7228, 7234, 7238, 7240, 7241, 7242, 7243, 7245, 7246, 7247, 7250, 7253, 7254, 7255, 7258, 7259, 7262, 7264, 7265, 7266, 7267, 7269, 7270, 7273, 7276, 7277, 7280, 7282, 7284, 7288, 7291, 7294, 7296, 7301, 7302, 7303, 7306, 7307, 7311, 7312, 7313, 7315, 7318, 7319, 7322, 7323, 7325, 7327, 7328, 7329, 7330, 7336, 7337, 7338, 7340, 7345, 7346, 7347, 7348, 7350, 7355, 7359, 7361, 7362, 7365, 7368, 7369, 7370, 7372, 7376, 7377, 7378, 7574, 7575, 7576, 7580, 7582, 7584, 7585, 7586, 7587, 7588, 7589, 7590, 7593, 7594, 7596, 7599, 7600, 7604, 7606, 7609, 7610, 7611, 7612, 7613, 7614, 7616, 7617, 7618, 7619, 7620, 7621, 7622, 7627, 7629, 7630, 7631, 7633, 7634, 7636, 7638, 7640, 7642, 7644, 7645, 7647, 7648, 7649, 7652, 7653, 7654, 7655, 7656, 7657, 7659, 7660, 7661, 7662, 7663, 7665, 7666, 7667, 7668, 7669, 7671, 7673, 7674, 7675, 7676, 7678, 7683, 7684, 7686, 7689, 7690, 7691, 7692, 7693, 7694, 7695, 7697, 7698, 7699, 7701, 7702, 7703, 7704, 7705, 7706, 7707, 7708, 7711, 7714, 7715, 7717, 7719, 7720, 7721, 7722, 7723, 7724, 7726, 7731, 7733, 7736, 7737, 7738, 7740, 7743, 7746, 7750, 7755, 7757, 7759, 7760, 7761, 7762, 7763, 7764, 7767, 7768, 7770, 7771, 7772, 7775, 7776, 7778, 7781, 7782, 7783, 7784, 7785, 7787, 7788, 7789, 7791, 7793, 7794, 7795, 7796, 7797, 7798, 7799, 7802, 7803, 7804, 7805, 7806, 7807, 7809, 7811, 7812, 7813, 7814, 7816, 7817, 7819, 7820, 7821, 7824, 7825, 7827, 7828, 7829, 7830, 7834, 7836, 7838, 7839, 7841, 7842, 7845, 7846, 7850, 7851, 7852, 7853, 7855, 7856, 7857, 7859, 7861, 7862, 7863, 7865, 7866, 7868, 7869, 7870, 7873, 7875, 7876, 7878, 7880, 7881, 7883, 7884, 7885, 7887, 7888, 7889, 7891, 7895, 7897, 7898, 7899, 7900, 7901, 7902, 7904, 7905, 7906, 7907, 7908, 7909, 7910, 7913, 7916, 7917, 7919, 7920, 7922, 7923, 7924, 7926, 7927, 7930, 7935, 7936, 7938, 7940, 7942, 7943, 7950, 7951, 7952, 7953, 7955, 7956, 7958, 7960, 7961, 7963, 7964, 7965, 7966, 7971, 7972, 7974, 7976, 7979, 7981, 7983, 7984, 7985, 7988, 7990, 7992, 7993, 7994, 7995, 7997, 8000, 8001, 8002, 8004, 8005, 8006, 8007, 8026, 8027, 8028, 8029, 8030, 8031, 8033, 8034, 8035, 8036, 8038, 8041, 8042, 8043, 8044, 8045, 8047, 8048, 8049, 8051, 8052, 8053, 8054, 8056, 8058, 8059, 8061, 8062, 8064, 8067, 8068, 8069, 8070, 8073, 8079, 8080, 8081, 8082, 8083, 8084, 8087, 8088, 8089, 8091, 8092, 8093, 8094, 8098, 8105, 8108, 8109, 8110, 8111, 8112, 8113, 8114, 8115, 8116, 8117, 8118, 8120, 8121, 8124, 8125, 8126, 8127, 8128, 8130, 8131, 8132, 8135, 8137, 8141, 8142, 8143, 8144, 8145, 8147, 8150, 8151, 8152, 8154, 8155, 8156, 8160, 8161, 8163, 8167, 8168, 8171, 8172, 8173, 8175, 8176, 8179, 8181, 8183, 8187, 8189, 8190, 8192, 8193, 8194, 8197, 8201, 8202, 8204, 8212, 8213, 8214, 8215, 8217, 8219, 8222, 8224, 8225, 8226, 8227, 8228, 8229, 8231, 8232, 8233, 8236, 8238, 8239, 8242, 8244, 8248, 8249, 8251, 8254, 8256, 8258, 8259, 8260, 8262, 8264, 8265, 8266, 8267, 8268, 8269, 8270, 8272, 8273, 8274, 8276, 8277, 8278, 8279, 8281, 8284, 8285, 8286, 8287, 8289, 8290, 8291, 8292, 8293, 8294, 8296, 8300, 8301, 8302, 8303, 8307, 8308, 8311, 8312, 8313, 8314, 8316, 8318, 8319, 8320, 8322, 8324, 8326, 8329, 8330, 8331, 8335, 8336, 8337, 8338, 8339, 8341, 8343, 8345, 8348, 8349, 8350, 8351, 8352, 8353, 8354, 8355, 8356, 8359, 8360, 8364, 8367, 8369, 8370, 8375, 8376, 8380, 8381, 8385, 8388, 8389, 8390, 8391, 8393, 8395, 8396, 8397, 8401, 8402, 8403, 8408, 8409, 8411, 8412, 8414, 8415, 8416, 8418, 8420, 8422, 8423, 8427, 8430, 8431, 8432, 8435, 8437, 8439, 8440, 8441, 8443, 8444, 8445, 8446, 8447, 8448, 8449, 8451, 8453, 8454, 8456, 8459, 8464, 8465, 8466, 8467, 8471, 8472, 8474, 8475, 8477, 8482, 8483, 8484, 8485, 8487, 8488, 8489, 8492, 8493, 8495, 8496, 8498, 8499, 8500, 8502, 8503, 8505, 8509, 8510, 8511, 8512, 8513, 8514, 8515, 8518, 8519, 8520, 8521, 8522, 8524, 8525, 8526, 8527, 8529, 8534, 8535, 8537, 8538, 8539, 8540, 8541, 8542, 8543, 8546, 8547, 8549, 8553, 8555, 8556, 8558, 8559, 8560, 8562, 8563, 8567, 8568, 8570, 8571, 8573, 8574, 8575, 8576, 8578, 8579, 8580, 8581, 8583, 8585, 8586, 8588, 8590, 8592, 8594, 8595, 8596, 8597, 8598, 8599, 8601, 8604, 8605, 8606, 8607, 8608, 8609, 8613, 8616, 8617, 8618, 8620, 8621, 8624, 8625, 8626, 8631, 8633, 8637, 8638, 8639, 8641, 8642, 8643, 8644, 8648, 8650, 8651, 8656, 8658, 8659, 8660, 8661, 8662, 8663, 8666, 8668, 8669, 8670, 8671, 8672, 8675, 8676, 8677, 8678, 8680, 8681, 8683, 8685, 8686, 8688, 8689, 8691, 8692, 8694, 8695, 8696, 8697, 8699, 8706, 8707, 8708, 8713, 8715, 8716, 8717, 8718, 8719, 8721, 8722, 8723, 8727, 8730, 8731, 8732, 8733, 8734, 8735, 8738, 8741, 8743, 8745, 8746, 8748, 8751, 8757, 8758, 8759, 8761, 8762, 8763, 8764, 8765, 8766, 8768, 8770, 8771, 8773, 8774, 8775, 8777, 8778, 8782, 8783, 8785, 8788, 8792, 8793, 8794, 8795, 8799, 8800, 8802, 8807, 8808, 8809, 8810, 8811, 8812, 8814, 8815, 8819, 8820, 8821, 8822, 8823, 8824, 8826, 8827, 8828, 8829, 8830, 8831, 8833, 8834, 8837, 8838, 8839, 8840, 8841, 8842, 8847, 8848, 8849, 8850, 8851, 8853, 8855, 8856, 8862, 8863, 8865, 8867, 8871, 8872, 8873, 8874, 8875, 8876, 8877, 8879, 8887, 8888, 8889, 8890, 8895, 8896, 8897, 8898, 8899, 8902, 8903, 8906, 8907, 8909, 8914, 8915, 8916, 8917, 8918, 8919, 8920, 8921, 8922, 8924, 8925, 8926, 8928, 8929, 8932, 8935, 8936, 8937, 8939, 8940, 8941, 8942, 8946, 8947, 8949, 8964, 8967, 8968, 8969, 8970, 8972, 8974, 8979, 8982, 8983, 8985, 8986, 8988, 8989, 8990, 8993, 8994, 8996, 8998, 8999, 9001, 9002, 9003, 9005, 9009, 9010, 9011, 9012, 9013, 9014, 9015, 9016, 9018, 9021, 9022, 9024, 9025, 9027, 9028, 9029, 9030, 9032, 9053, 9058, 9059, 9060, 9062, 9064, 9065, 9066, 9067, 9069, 9070, 9071, 9072, 9073, 9074, 9075, 9077, 9079, 9081, 9082, 9084, 9085, 9086, 9087, 9088, 9089, 9090, 9094, 9095, 9098, 9100, 9101, 9104, 9105, 9106, 9107, 9111, 9113, 9116, 9117, 9119, 9121, 9122, 9123, 9124, 9125, 9126, 9128, 9129, 9130, 9131, 9132, 9133, 9135, 9136, 9137, 9138, 9139, 9141, 9142, 9143, 9144, 9145, 9147, 9148, 9150, 9152, 9153, 9154, 9157, 9158, 9161, 9162, 9165, 9166, 9167, 9170, 9171, 9172, 9173, 9174, 9175, 9177, 9178, 9179, 9181, 9182, 9183, 9184, 9186, 9187, 9188, 9191, 9192, 9194, 9195, 9199, 9200, 9201, 9203, 9205, 9206, 9209, 9210, 9212, 9213, 9215, 9216, 9218, 9219, 9221, 9222, 9223, 9225, 9226, 9227, 9229, 9230, 9231, 9234, 9235, 9237, 9238, 9239, 9240, 9242, 9244, 9247, 9249, 9251, 9252, 9253, 9254, 9256, 9257, 9261, 9263, 9264, 9265, 9267, 9268, 9270, 9272, 9276, 9277, 9279, 9281, 9282, 9285, 9287, 9288, 9289, 9290, 9294, 9295, 9297, 9301, 9302, 9303, 9307, 9309, 9311, 9312, 9313, 9314, 9315, 9316, 9318, 9319, 9320, 9322, 9324, 9326, 9327, 9328, 9330, 9331, 9332, 9333, 9334, 9335, 9336, 9338, 9339, 9340, 9342, 9345, 9346, 9348, 9353, 9354, 9355, 9358, 9359, 9360, 9361, 9365, 9368, 9369, 9370, 9371, 9372, 9375, 9376, 9377, 9379, 9380, 9381, 9383, 9386, 9387, 9391, 9395, 9397, 9398, 9400, 9401, 9402, 9403, 9404, 9406, 9407, 9408, 9409, 9410, 9411, 9413, 9414, 9416, 9418, 9421, 9423, 9424, 9426, 9427, 9429, 9430, 9431, 9432, 9437, 9439, 9440, 9442, 9445, 9448, 9450, 9452, 9455, 9456, 9457, 9458, 9459, 9460, 9461, 9464, 9465, 9467, 9469, 9471, 9474, 9478, 9479, 9481, 9483, 9484, 9485, 9487, 9488, 9489, 9490, 9492, 9493, 9494, 9495, 9498, 9503, 9505, 9507, 9509, 9513, 9515, 9519, 9521, 9526, 9527, 9529, 9530, 9531, 9532, 9534, 9536, 9537, 9538, 9541, 9542, 9543, 9545, 9546, 9548, 9549, 9551, 9552, 9553, 9554, 9555, 9558, 9560, 9566, 9567, 9569, 9571, 9572, 9575, 9576, 9578, 9580, 9581, 9582, 9586, 9588, 9589, 9592, 9596, 9598, 9600, 9603, 9607, 9608, 9609, 9610, 9611, 9612, 9614, 9615, 9617, 9619, 9620, 9621, 9622, 9624, 9625, 9626, 9627, 9628, 9631, 9634, 9636, 9637, 9638, 9639, 9640, 9643, 9644, 9646, 9647, 9649, 9657, 9658, 9660, 9662, 9663, 9664, 9665, 9666, 9668, 9669, 9671, 9673, 9674, 9675, 9676, 9677, 9678, 9681, 9685, 9688, 9691, 9692, 9693, 9694, 9695, 9696, 9697, 9700, 9701, 9702, 9703, 9706, 9707, 9710, 9711, 9712, 9715, 9717, 9718, 9721, 9722, 9725, 9730, 9732, 9733, 9735, 9738, 9739, 9740, 9741, 9742, 9743, 9744, 9745, 9746, 9750, and 9751.

A biologically active substance preferably comprises the amino acid sequence of any of SEQ ID NOs: 4877-9751; or fragments thereof that have biological activity. In a more preferred aspect, a biologically active substance comprises the amino acid sequence of any of SEQ ID NOs: 4877-9751. In another preferred aspect, a biologically active substance comprises the mature polypeptide region of any of SEQ ID NOs: 4877-9751, or fragments thereof that have biological activity. In another preferred aspect, a biologically active substance comprises the mature polypeptide region of any of SEQ ID NOs: 4877-9751. In another preferred aspect, a biologically active substance consists of the amino acid sequence of any of SEQ ID NOs: 4877-9751; or fragments thereof that have biological activity. In another preferred aspect, a biologically active substance consists of the amino acid sequence of any of SEQ ID NOs: 4877-9751. In another preferred aspect, a biologically active substance consists of the mature polypeptide region of any of SEQ ID NOs: 4877-9751; or fragments thereof that have biological activity. In another preferred aspect, a biologically active substance consists of the mature polypeptide region of any of SEQ ID NOs: 4877-9751.

In a second aspect, the present invention relates to a biologically active substance encoded by a polynucleotide comprising a nucleotide sequence having preferably at least 60% identity, more preferably at least 65% identity, more preferably at least 70% identity, more preferably at least 75% identity, more preferably at least 80% identity, more preferably at least 85% identity, more preferably at least 90% identity, even more preferably at least 90% identity, most preferably at least 95% identity, and even most preferably at least 97% identity with a polynucleotide selected from the group consisting of SEQ ID NOs: 2-4876 and subsequences thereof encoding fragments having biological activity.

In preferred aspect, the biologically active substance is encoded by a polynucleotide comprising a nucleotide sequence having preferably at least 60% identity, more preferably at least 65% identity, more preferably at least 70% identity, more preferably at least 75% identity, more preferably at least 80% identity, more preferably at least 85% identity, even more preferably at least 90% identity, most preferably at least 95% identity, and even most preferably at least 97% identity with a polynucleotide selected from the group consisting of SEQ ID NOs: 50, 55, 77, 82, 91, 98, 103, 110, 125, 169, 171, 172, 174, 176, 177, 178, 179, 180, 181, 182, 183, 185, 186, 187, 189, 192, 194, 195, 196, 198, 199, 201, 203, 204, 205, 206, 207, 208, 209, 210, 211, 212, 213, 214, 215, 216, 217, 218, 219, 220, 221, 222, 223, 225, 226, 227, 228, 229, 230, 240, 241, 242, 243, 244, 245, 246, 247, 248, 249, 250, 251, 252, 253, 254, 255, 256, 257, 258, 259, 260, 261, 262, 263, 264, 265, 266, 287, 306, 308, 322, 323, 329, 350, 354, 360, 363, 367, 381, 445, 469, 487, 488, 516, 523, 524, 537, 541, 575, 592, 607, 621, 627, 628, 666, 672, 688, 691, 702, 710, 719, 726, 729, 748, 751, 773, 774, 777, 791, 831, 833, 842, 847, 848, 849, 850, 851, 855, 857, 885, 886, 887, 901, 944, 982, 991, 994, 1018, 1032, 1034, 1058, 1078, 1088, 1098, 1108, 1112, 1128, 1148, 1153, 1154, 1158, 1160, 1164, 1175, 1176, 1177, 1180, 1181, 1183, 1184, 1193, 1194, 1199, 1222, 1237, 1244, 1262, 1263, 1265, 1266, 1278, 1302, 1313, 1314, 1315, 1316, 1317, 1318, 1345, 1355, 1373, 1374, 1401, 1424, 1432, 1433, 1440, 1442, 1448, 1455, 1470, 1496, 1497, 1501, 1509, 1527, 1539, 1556, 1565, 1589, 1619, 1629, 1630, 1634, 1635, 1636, 1638, 1639, 1640, 1641, 1642, 1643, 1644, 1645, 1646, 1647, 1649, 1650, 1651, 1652, 1653, 1654, 1655, 1657, 1658, 1659, 1660, 1661, 1662, 1663, 1664, 1665, 1666, 1667, 1668, 1669, 1670, 1671, 1672, 1673, 1675, 1676, 1677, 1679, 1684, 1688, 1692, 1694, 1703, 1704, 1728, 1732, 1734, 1736, 1775, 1783, 1816, 1831, 1838, 1851, 1853, 1866, 1887, 2002, 2007, 2022, 2038, 2044, 2067, 2070, 2072, 2076, 2094, 2106, 2119, 2120, 2121, 2124, 2131, 2139, 2147, 2148, 2166, 2171, 2194, 2196, 2198, 2205, 2211, 2213, 2216, 2221, 2230, 2236, 2240, 2241, 2255, 2258, 2263, 2273, 2277, 2286, 2288, 2289, 2290, 2291, 2292, 2293, 2294, 2295, 2296, 2297, 2298, 2299, 2300, 2301, 2302, 2303, 2304, 2305, 2324, 2327, 2337, 2341, 2347, 2351, 2364, 2381, 2388, 2400, 2403, 2408, 2410, 2411, 2414, 2415, 2424, 2425, 2430, 2435, 2439, 2457, 2467, 2468, 2469, 2483, 2491, 2498, 2504, 2505, 2506, 2507, 2508, 2509, 2510, 2511, 2512, 2514, 2515, 2516, 2517, 2518, 2519, 2520, 2521, 2522, 2524, 2525, 2526, 2527, 2528, 2529, 2530, 2531, 2532, 2533, 2534, 2535, 2536, 2537, 2538, 2539, 2540, 2541, 2542, 2543, 2544, 2545, 2546, 2547, 2548, 2549, 2550, 2551, 2552, 2553, 2554, 2555, 2556, 2557, 2558, 2559, 2560, 2561, 2562, 2564, 2565, 2566,

2567, 2568, 2569, 2570, 2571, 2572, 2573, 2574, 2575, 2576, 2577, 2578, 2579, 2580, 2581, 2582, 2583, 2584, 2585, 2586, 2587, 2588, 2590, 2591, 2592, 2593, 2594, 2595, 2596, 2597, 2598, 2599, 2600, 2601, 2602, 2603, 2604, 2605, 2606, 2607, 2608, 2609, 2610, 2611, 2612, 2613, 2614, 2615, 2616, 2617, 2618, 2619, 2620, 2621, 2622, 2623, 2624, 2625, 2626, 2627, 2628, 2629, 2630, 2631, 2632, 2633, 2634, 2635, 2636, 2637, 2638, 2639, 2640, 2641, 2642, 2643, 2644, 2645, 2646, 2647, 2648, 2649, 2650, 2651, 2652, 2653, 2654, 2655, 2656, 2657, 2658, 2659, 2661, 2664, 2665, 2666, 2667, 2669, 2670, 2671, 2672, 2673, 2674, 2675, 2676, 2677, 2679, 2680, 2681, 2684, 2685, 2686, 2688, 2689, 2690, 2691, 2692, 2693, 2696, 2697, 2698, 2706, 2723, 2730, 2733, 2751, 2762, 2795, 2806, 2813, 2834, 2835, 2841, 2850, 2866, 2867, 2874, 2878, 2898, 2902, 2904, 2917, 2947, 2957, 2960, 2969, 2972, 2973, 2999, 3004, 3019, 3028, 3054, 3064, 3082, 3093, 3123, 3133, 3134, 3135, 3136, 3137, 3138, 3139, 3140, 3141, 3142, 3143, 3144, 3145, 3146, 3147, 3148, 3149, 3150, 3162, 3222, 3224, 3225, 3226, 3227, 3228, 3229, 3231, 3244, 3248, 3259, 3264, 3265, 3287, 3291, 3303, 3310, 3311, 3321, 3324, 3355, 3362, 3371, 3429, 3434, 3442, 3446, 3458, 3471, 3486, 3496, 3499, 3503, 3504, 3508, 3549, 3558, 3577, 3593, 3598, 3601, 3603, 3606, 3611, 3642, 3653, 3655, 3670, 3673, 3682, 3690, 3694, 3707, 3716, 3727, 3736, 3737, 3744, 3753, 3765, 3790, 3798, 3799, , 3872, 3881, 3885, 3915, 3916, 3922, 3931, 3938, 3969, 4011, 4017, 4056, 4059, 4068, 4076, 4077, 4081, 4082, 4085, 4090, 4106, 4112, 4117, 4144, 4145, 4158, 4159, 4160, 4161, 4162, 4163, 4164, 4165, 4166, 4167, 4168, 4169, 4170, 4171, 4172, 4173, 4174, 4175, 4176, 4179, 4181, 4182, 4201, 4235, 4285, 4288, 4305, 4323, 4333, 4336, 4353, 4398, 4409, 4417, 4421, 4423, 4429, 4435, 4448, 4450, 4469, 4472, 4476, 4482, 4488, 4513, 4518, 4544, 4559, 4560, 4569, 4571, 4578, 4607, 4622, 4631, 4643, 4647, 4648, 4650, 4658, 4669, 4672, 4682, 4687, 4688, 4704, 4718, 4754, 4767, 4786, 4792, 4797, 4804, 4812, 4814, 4823, 4830, 4833, 4838, 4848, 4849, 4859, 4862, and 4872; preferably at least 75% identity, more preferably at least 80% identity, more preferably at least 85% identity, even more preferably at least 90% identity, most preferably at least 95% identity, and even most preferably at least 97% identity with a polynucleotide selected from the group consisting of SEO ID NOs: 909, 911, 1367, 1498, 2663, 2668, 2682, and 2683; preferably at least 80% identity, more preferably at least 85% identity, even more preferably at least 90% identity, most preferably at least 95% identity, and even most preferably at least 97% identity with a polynucleotide selected from the group consisting of SEQ ID NOs: 167, 654, 2513, 2589, and 2694; preferably at least 85% identity, more preferably at least 90% identity, most preferably at least 95% identity, and even most preferably at least 97% identity with a polynucleotide selected from the group consisting of SEQ ID NOs: 163, 184, 910, 1674, 2412, 2660, 2662, 2695, and 3231; preferably at least 90% identity, more preferably at least 95% identity, and most preferably at least 97% identity with a polynucleotide selected from the group consisting ofSEQ ID NOs: 27, 166, 168, 170, 193, 202, 231, 543, 776, 888, 889, 890, 898, 908, 916, 1028, 1123, 1133, 1155, 1156, 1157, 1173, 1211, 1212, 1218, 1229, 1230, 1277, 1356, 1357, 1359, 1360, 1368, 1372, 1391, 1500, 1584, 1604, 1627, 1631, 1632, 1637, 1648, 1680, 2101, 2102, 2239, 2358, 2359, 2360, 2404, 2417, 2429, 2494, 2563, 2678, 2724, 3111, 3539, 3850, 4078, 4079, 4080, 4083, 4084, 4180, 4221, 4642, 4689, 4707, and 4727; or preferably at least 95% identity and more preferably at least 97% identity with a polynucleotide selected from the group consisting of SEQ ID NOs: 2, 3, 4, 5, 6, 7, 8, 9, 10, 11, 12, 13, 14, 15, 16, 17, 18, 19, 20, 21, 22, 23, 24, 25, 26, 28, 29, 30, 31, 32, 33, 34, 35, 36, 37, 38, 39, 40, 41, 42, 43, 44, 45, 46, 47, 48, 49, 51, 52, 53, 54, 56, 57, 58, 59, 60, 61, 62, 63, 64, 65, 66, 67, 68, 69, 70, 71, 72, 73, 74, 75, 76, 78, 79, 80, 81, 83, 84, 85, 86, 87, 88, 89, 90, 92, 93, 94, 95, 96, 97, 99, 100, 101, 102, 104, 105, 106, 107, 108, 109, 111, 112, 113, 114, 115, 116, 117, 118, 119, 120, 121, 122, 123, 124, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 164, 165, 173, 175, 188, 190, 191, 197, 200, 224, 232, 233, 234, 235, 236, 237, 238, 239, 267, 268, 269, 270, 271, 272, 273, 274, 275, 276, 277, 278, 279, 280, 281, 282, 283, 284, 285, 286, 288, 289, 290, 291, 292, 293, 294, 295, 296, 297, 298, 299, 300, 301, 302, 303, 304, 305, 307, 309, 310, 311, 312, 313, 314, 315, 316, 317, 318, 319, 320, 321, 324, 325, 326, 327, 328, 330, 331, 332, 333, 334, 335, 336, 337, 338, 339, 340, 341, 342, 343, 344, 345, 346, 347, 348, 349, 351, 352, 353, 355, 356, 357, 358, 359, 361, 362, 364, 365, 366, 368, 369, 370, 371, 372, 373, 374, 375, 376, 377, 378, 379, 380, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 446, 447, 448, 449, 450, 451, 452, 453, 454, 455, 456, 457, 458, 459, 460, 461, 462, 463, 464, 465, 466, 467, 468, 470, 471, 472, 473, 474, 475, 476, 477, 478, 479, 480, 481, 482, 483, 484, 485, 486, 489, 490, 491, 492, 493, 494, 495, 496, 497, 498, 499, 500, 501, 502, 503, 504, 505, 506, 507, 508, 509, 510, 511, 512, 513, 514, 515, 517, 518, 519, 520, 521, 522, 525, 526, 527, 528, 529, 530, 531, 532, 533, 534, 535, 536, 538, 539, 540, 542, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 576, 577, 578, 579, 580, 581, 582, 583, 584, 585, 586, 587, 588, 589, 590, 591, 593, 594, 595, 596, 597, 598, 599, 600, 601, 602, 603, 604, 605, 606, 608, 609, 610, 611, 612, 613, 614, 615, 616, 617, 618, 619, 620, 622, 623, 624, 625, 626, 629, 630, 631, 632, 633, 634, 635, 636, 637, 638, 639, 640, 641, 642, 643, 644, 645, 646, 647, 648, 649, 650, 651, 652, 653, 655, 656, 657, 658, 659, 660, 661, 662, 663, 664, 665, 667, 668, 669, 670, 671, 673, 674, 675, 676, 677, 678, 679, 680, 681, 682, 683, 684, 685, 686, 687, 689, 690, 692, 693, 694, 695, 696, 697, 698, 699, 700, 701, 703, 704, 705, 706, 707, 708, 709, 711, 712, 713, 714, 715, 716, 717, 718, 720, 721, 722, 723, 724, 725, 727, 728, 730, 731, 732, 733, 734, 735, 736, 737, 738, 739, 740, 741, 742, 743, 744, 745, 746, 747, 749, 750, 752, 753, 754, 755, 756, 757, 758, 759, 760, 761, 762, 763, 764, 765, 766, 767, 768, 769, 770, 771, 772, 775, 778, 779, 780, 781, 782, 783, 784, 785, 786, 787, 788, 789, 790, 792, 793, 794, 795, 796, 797, 798, 799, 800, 801, 802, 803, 804, 805, 806, 807, 808, 809, 810, 811, 812, 813, 814, 815, 816, 817, 818, 819, 820, 821, 822, 823, 824, 825, 826, 827, 828, 829, 830, 832, 834, 835, 836, 837, 838, 839, 840, 841, 843, 844, 845, 846, 852, 853, 854, 856, 858, 859, 860, 861, 862, 863, 864, 865, 866, 867, 868, 869, 870, 871, 872, 873, 874, 875, 876, 877, 878, 879, 880, 881, 882, 883, 884, 891, 892, 893, 894, 895, 896, 897, 899, 900, 902, 903, 904, 905, 906, 907, 912, 913, 914, 915, 917, 918, 919, 920, 921, 922, 923, 924, 925, 926, 927, 928, 929, 930, 931, 932, 933, 934, 935, 936, 937, 938, 939, 940, 941, 942, 943, 945, 946, 947, 948, 949, 950, 951, 952, 953, 954, 955, 956, 957, 958, 959, 960, 961, 962, 963, 964, 965, 966, 967, 968, 969, 970, 971, 972, 973, 974, 975, 976, 977, 978, 979, 980, 981, 983, 984, 985, 986, 987, 988, 989, 990, 992, 993, 995, 996, 997, 998, 999, 1000, 1001, 1002, 1003, 1004, 1005, 1006, 1007, 1008, 1009, 1010, 1011, 1012, 1013, 1014, 1015, 1016, 1017, 1019, 1020, 1021, 1022, 1023, 1024, 1025, 1026, 1027, 1029, 1030, 1031, 1033, 1035, 1036, 1037, 1038, 1039, 1040, 1041, 1042, 1043, 1044, 1045, 1046, 1047, 1048, 1049, 1050, 1051, 1052, 1053, 1054, 1055, 1056, 1057, 1059, 1060, 1061, 1062, 1063, 1064, 1065, 1066, 1067, 1068, 1069, 1070, 1071, 1072, 1073, 1074, 1075, 1076, 1077, 1079, 1080, 1081, 1082, 1083, 1084, 1085, 1086, 1087, 1089, 1090, 1091, 1092, 1093, 1094, 1095, 1096, 1097, 1099, 1100, 1101, 1102, 1103, 1104, 1105, 1106, 1107, 1109, 1110, 1111, 1113, 1114, 1115, 1116, 1117, 1118, 1119, 1120, 1121, 1122, 1124, 1125, 1126, 1127, 1129, 1130, 1131, 1132, 1134, 1135, 1136, 1137, 1138, 1139, 1140, 1141, 1142, 1143, 1144, 1145, 1146, 1147, 1149, 1150, 1151, 1152, 1159, 1161, 1162, 1163, 1165, 1166, 1167, 1168, 1169, 1170, 1171, 1172, 1174, 1178, 1179, 1182, 1185, 1186, 1187, 1188, 1189, 1190, 1191, 1192, 1195, 1196, 1197, 1198, 1200, 1201, 1202, 1203, 1204, 1205, 1206, 1207, 1208, 1209, 1210, 1213, 1214, 1215, 1216, 1217, 1219, 1220, 1221, 1223, 1224, 1225, 1226, 1227, 1228, 1231, 1232, 1233, 1234, 1235, 1236, 1238, 1239, 1240, 1241, 1242, 1243, 1245, 1246, 1247, 1248, 1249, 1250, 1251, 1252, 1253, 1254, 1255, 1256, 1257, 1258, 1259, 1260, 1261, 1264, 1267, 1268, 1269, 1270, 1271, 1272, 1273, 1274, 1275, 1276, 1279, 1280, 1281, 1282, 1283, 1284, 1285, 1286, 1287, 1288, 1289, 1290, 1291, 1292, 1293, 1294, 1295, 1296, 1297, 1298, 1299, 1300, 1301, 1303, 1304, 1305, 1306, 1307, 1308, 1309, 1310, 1311, 1312, 1319, 1320, 1321, 1322, 1323, 1324, 1325, 1326, 1327, 1328, 1329, 1330, 1331, 1332, 1333, 1334, 1335, 1336, 1337, 1338, 1339, 1340, 1341, 1342, 1343, 1344, 1346, 1347, 1348, 1349, 1350, 1351, 1352, 1353, 1354, 1358, 1361, 1362, 1363, 1364, 1365, 1366, 1369, 1370, 1371, 1375, 1376, 1377, 1378, 1379, 1380, 1381, 1382, 1383, 1384, 1385, 1386, 1387, 1388, 1389, 1390, 1392, 1393, 1394, 1395, 1396, 1397, 1398, 1399, 1400, 1402, 1403, 1404, 1405, 1406, 1407, 1408, 1409, 1410, 1411, 1412, 1413, 1414, 1415, 1416, 1417, 1418, 1419, 1420, 1421, 1422, 1423, 1425, 1426, 1427, 1428, 1429, 1430, 1431, 1434, 1435, 1436, 1437, 1438, 1439, 1441, 1443, 1444, 1445, 1446, 1447, 1449, 1450, 1451, 1452, 1453, 1454, 1456, 1457, 1458, 1459, 1460, 1461, 1462, 1463, 1464, 1465, 1466, 1467, 1468, 1469, 1471, 1472, 1473, 1474, 1475, 1476, 1477, 1478, 1479, 1480, 1481, 1482, 1483, 1484, 1485, 1486, 1487, 1488, 1489, 1490, 1491, 1492, 1493, 1494, 1495, 1499, 1502, 1503, 1504, 1505, 1506, 1507, 1508, 1510, 1511, 1512, 1513, 1514, 1515, 1516, 1517, 1518, 1519, 1520, 1521, 1522, 1523, 1524, 1525, 1526, 1528, 1529, 1530, 1531, 1532, 1533, 1534, 1535, 1536, 1537, 1538, 1540, 1541, 1542, 1543, 1544, 1545, 1546, 1547, 1548, 1549, 1550, 1551, 1552, 1553, 1554, 1555, 1557, 1558, 1559, 1560, 1561, 1562, 1563, 1564, 1566, 1567, 1568, 1569, 1570, 1571, 1572, 1573, 1574, 1575, 1576, 1577, 1578, 1579, 1580, 1581, 1582, 1583, 1585, 1586, 1587, 1588, 1590, 1591, 1592, 1593, 1594, 1595, 1596, 1597, 1598, 1599, 1600, 1601, 1602, 1603, 1605, 1606, 1607, 1608, 1609, 1610, 1611, 1612, 1613, 1614, 1615, 1616, 1617, 1618, 1620, 1621, 1622, 1623, 1624, 1625, 1626, 1628, 1633, 1656, 1678, 1681, 1682, 1683, 1685, 1686, 1687, 1689, 1690, 1691, 1693, 1695, 1696, 1697, 1698, 1699, 1700, 1701, 1702, 1705, 1706, 1707, 1708, 1709, 1710, 1711, 1712, 1713, 1714, 1715, 1716, 1717, 1718, 1719, 1720, 1721, 1722, 1723, 1724, 1725, 1726, 1727, 1729, 1730, 1731, 1733, 1735, 1737, 1738, 1739, 1740, 1741, 1742, 1743, 1744, 1745, 1746, 1747, 1748, 1749, 1750, 1751, 1752, 1753, 1754, 1755, 1756, 1757, 1758, 1759, 1760, 1761, 1762, 1763, 1764, 1765, 1766, 1767, 1768, 1769, 1770, 1771, 1772, 1773, 1774, 1776, 1777, 1778, 1779, 1780, 1781, 1782, 1784, 1785, 1786, 1787, 1788, 1789, 1790, 1791, 1792, 1793, 1794, 1795, 1796, 1797, 1798, 1799, 1800, 1801, 1802, 1803, 1804, 1805, 1806, 1807, 1808, 1809, 1810, 1811, 1812, 1813, 1814, 1815, 1817, 1818, 1819, 1820, 1821, 1822, 1823, 1824, 1825, 1826, 1827, 1828, 1829, 1830, 1832, 1833, 1834, 1835, 1836, 1837, 1839, 1840, 1841, 1842, 1843, 1844, 1845, 1846, 1847, 1848, 1849, 1850, 1852, 1854, 1855, 1856, 1857, 1858, 1859, 1860, 1861, 1862, 1863, 1864, 1865, 1867, 1868, 1869, 1870, 1871, 1872, 1873, 1874, 1875, 1876, 1877, 1878, 1879, 1880, 1881, 1882, 1883, 1884, 1885, 1886, 1888, 1889, 1890, 1891, 1892, 1893, 1894, 1895, 1896, 1897, 1898, 1899, 1900, 1901, 1902, 1903, 1904, 1905, 1906, 1907, 1908, 1909, 1910, 1911, 1912, 1913, 1914, 1915, 1916, 1917, 1918, 1919, 1920, 1921, 1922, 1923, 1924, 1925, 1926, 1927, 1928, 1929, 1930, 1931, 1932, 1933, 1934, 1935, 1936, 1937, 1938, 1939, 1940, 1941, 1942, 1943, 1944, 1945, 1946, 1947, 1948, 1949, 1950, 1951, 1952, 1953, 1954, 1955, 1956, 1957, 1958, 1959, 1960, 1961, 1962, 1963, 1964, 1965, 1966, 1967, 1968, 1969, 1970, 1971, 1972, 1973, 1974, 1975, 1976, 1977, 1978, 1979, 1980, 1981, 1982, 1983, 1984, 1985, 1986, 1987, 1988, 1989, 1990, 1991, 1992, 1993, 1994, 1995, 1996, 1997, 1998, 1999, 2000, 2001, 2003, 2004, 2005, 2006, 2008, 2009, 2010, 2011, 2012, 2013, 2014, 2015, 2016, 2017, 2018, 2019, 2020, 2021, 2023, 2024, 2025, 2026, 2027, 2028, 2029, 2030, 2031, 2032, 2033, 2034, 2035, 2036, 2037, 2039, 2040, 2041, 2042, 2043, 2045, 2046, 2047, 2048, 2049, 2050, 2051, 2052, 2053, 2054, 2055, 2056, 2057, 2058, 2059, 2060, 2061, 2062, 2063, 2064, 2065, 2066, 2068, 2069, 2071, 2073, 2074, 2075, 2077, 2078, 2079, 2080, 2081, 2082, 2083, 2084, 2085, 2086, 2087, 2088, 2089, 2090, 2091, 2092, 2093, 2095, 2096, 2097, 2098, 2099, 2100, 2103, 2104, 2105, 2107, 2108, 2109, 2110, 2111, 2112, 2113, 2114, 2115, 2116, 2117, 2118, 2122, 2123, 2125, 2126, 2127, 2128, 2129, 2130, 2132, 2133, 2134, 2135, 2136, 2137, 2138, 2140, 2141, 2142, 2143, 2144, 2145, 2146, 2149, 2150, 2151, 2152, 2153, 2154, 2155, 2156, 2157, 2158, 2159, 2160, 2161, 2162, 2163, 2164, 2165, 2167, 2168, 2169, 2170, 2172, 2173, 2174, 2175, 2176, 2177, 2178, 2179, 2180, 2181, 2182, 2183, 2184, 2185, 2186, 2187, 2188, 2189, 2190, 2191, 2192, 2193, 2195, 2197, 2199, 2200, 2201, 2202, 2203, 2204, 2206, 2207, 2208, 2209, 2210, 2212, 2214, 2215, 2217, 2218, 2219, 2220, 2222, 2223, 2224, 2225, 2226, 2227, 2228, 2229, 2231, 2232, 2233, 2234, 2235, 2237, 2238, 2242, 2243, 2244, 2245, 2246, 2247, 2248, 2249, 2250, 2251, 2252, 2253, 2254, 2256, 2257, 2259, 2260, 2261, 2262, 2264, 2265, 2266, 2267, 2268, 2269, 2270, 2271, 2272, 2274, 2275, 2276, 2278, 2279, 2280, 2281, 2282, 2283, 2284, 2285, 2287, 2306, 2307, 2308, 2309, 2310, 2311, 2312, 2313, 2314, 3231, 2316, 2317, 2318, 2319, 2320, 2321, 2322, 2323, 2325, 2326, 2328, 2329, 2330, 2331, 2332, 2333, 2334, 2335, 2336, 2338, 2339, 2340, 2342, 2343, 2344, 2345, 2346, 2348, 2349, 2350, 2352, 2353, 2354, 2355, 2356, 2357, 2361, 2362, 2363, 2365, 2366, 2367, 2368, 2369, 2370, 2371, 2372, 2373, 2374, 2375, 2376, 2377, 2378, 2379, 2380, 2382, 2383, 2384, 2385, 2386, 2387, 2389, 2390, 2391, 2392, 2393, 2394, 2395, 2396, 2397, 2398, 2399, 2401, 2402, 2405, 2406, 2407, 2409, 2413, 2416, 2418, 2419, 2420, 2421, 2422, 2423, 2426, 2427, 2428, 2431, 2432, 2433, 2434, 2436, 2437, 2438, 2440, 2441, 2442, 2443, 2444, 2445, 2446, 2447, 2448, 2449, 2450, 2451, 2452, 2453, 2454, 2455, 2456, 2458, 2459, 2460, 2461, 2462, 2463, 2464, 2465, 2466, 2470, 2471, 2472, 2473, 2474, 2475, 2476, 2477, 2478, 2479, 2480, 2481, 2482, 2484, 2485, 2486, 2487, 2488, 2489, 2490, 2492, 2493, 2495, 2496, 2497, 2499, 2500, 2501, 2502, 2503, 2523, 2687, 3231, 2700, 2701, 2702, 2703, 2704, 2705, 2707, 2708, 2709, 2710, 2711, 2712, 2713, 2714, 2715, 2716, 2717, 2718, 2719, 2720, 2721, 2722, 2725, 2726, 2727, 2728, 2729, 2731, 2732, 2734, 2735, 2736, 2737, 2738, 2739, 2740, 2741, 2742, 2743, 2744, 2745, 2746, 2747, 2748, 2749, 2750, 2752, 2753, 2754, 2755, 2756, 2757, 2758, 2759, 2760, 2761, 2763, 2764, 2765, 2766, 2767, 2768, 2769, 2770, 2771, 2772, 2773, 2774, 2775, 2776, 2777, 2778, 2779, 2780, 2781, 2782, 2783, 2784, 2785, 2786, 2787, 2788, 2789, 2790, 2791, 2792, 2793, 2794, 2796, 2797, 2798, 2799, 2800, 2801, 2802, 2803, 2804, 2805, 2807, 2808, 2809, 2810, 2811, 2812, 2814, 2815, 2816, 2817, 2818, 2819, 2820, 2821, 2822, 2823, 2824, 2825, 2826, 2827, 2828, 2829, 2830, 2831, 2832, 2833, 2836, 2837, 2838, 2839, 2840, 2842, 2843, 2844, 2845, 2846, 2847, 2848, 2849, 2851, 2852, 2853, 2854, 2855, 2856, 2857, 2858, 2859, 2860, 2861, 2862, 2863, 2864, 2865, 2868, 2869, 2870, 2871, 2872, 2873, 2875, 2876, 2877, 2879, 2880, 2881, 2882, 2883, 2884, 2885, 2886, 2887, 2888, 2889, 2890, 2891, 2892, 2893, 2894, 2895, 2896, 2897, 2899, 2900, 2901, 2903, 2905, 2906, 2907, 2908, 2909, 2910, 2911, 2912, 2913, 2914, 2915, 2916, 2918, 2919, 2920, 2921, 2922, 2923, 2924, 2925, 2926, 2927, 2928, 2929, 2930, 2931, 2932, 2933, 2934, 2935, 2936, 2937, 2938, 2939, 2940, 2941, 2942, 2943, 2944, 2945, 2946, 2948, 2949, 2950, 2951, 2952, 2953, 2954, 2955, 2956, 2958, 2959, 2961, 2962, 2963, 2964, 2965, 2966, 2967, 2968, 2970, 2971, 2974, 2975, 2976, 2977, 2978, 2979, 2980, 2981, 2982, 2983, 2984, 2985, 2986, 2987, 2988, 2989, 2990, 2991, 2992, 2993, 2994, 2995, 2996, 2997, 2998, 3000, 3001, 3002, 3003, 3005, 3006, 3007, 3008, 3009, 3010, 3011, 3012, 3013, 3014, 3015, 3016, 3017, 3018, 3020, 3021, 3022, 3023, 3024, 3025, 3026, 3027, 3029, 3030, 3031, 3032, 3033, 3034, 3035, 3036, 3037, 3038, 3039, 3040, 3041, 3042, 3043, 3044, 3045, 3046, 3047, 3048, 3049, 3050, 3051, 3052, 3053, 3055, 3056, 3057, 3058, 3059, 3060, 3061, 3062, 3063, 3065, 3066, 3067, 3068, 3069, 3070, 3071, 3072, 3073, 3074, 3075, 3076, 3077, 3078, 3079, 3080, 3081, 3083, 3084, 3085, 3086, 3087, 3088, 3089, 3090, 3091, 3092, 3094, 3095, 3096, 3097, 3098, 3099, 3100, 3101, 3102, 3103, 3104, 3105, 3106, 3107, 3108, 3109, 3110, 3112, 3113, 3114, 3115, 3116, 3117, 3118, 3119, 3120, 3121, 3122, 3124, 3125, 3126, 3127, 3128, 3129, 3130, 3131, 3132, 3151, 3152, 3153, 3154, 3155, 3156, 3157, 3158, 3159, 3160, 3161, 3163, 3164, 3165, 3166, 3167, 3168, 3169, 3170, 3171, 3172, 3173, 3174, 3175, 3176, 3177, 3178, 3179, 3180, 3181, 3182, 3183, 3184, 3185, 3186, 3187, 3188, 3189, 3190, 3191, 3192, 3193, 3194, 3195, 3196, 3197, 3198, 3199, 3200, 3201, 3202, 3203, 3204, 3205, 3206, 3207, 3208, 3209, 3210, 3211, 3212, 3213, 3214, 3215, 3216, 3217, 3218, 3219, 3220, 3221, 3223, 3232, 3233, 3234, 3235, 3236, 3237, 3238, 3239, 3240, 3241, 3242, 3243, 3245, 3246, 3247, 3249, 3250, 3251, 3252, 3253, 3254, 3255, 3256, 3257, 3258, 3260, 3261, 3262, 3263, 3266, 3267, 3268, 3269, 3270, 3271, 3272, 3273, 3274, 3275, 3276, 3277, 3278, 3279, 3280, 3281, 3282, 3283, 3284, 3285, 3286, 3288, 3289, 3290, 3292, 3293, 3294, 3295, 3296, 3297, 3298, 3299, 3300, 3301, 3302, 3304, 3305, 3306, 3307, 3308, 3309, 3312, 3313, 3314, 3315, 3316, 3317, 3318, 3319, 3320, 3322, 3323, 3325, 3326, 3327, 3328, 3329, 3330, 3331, 3332, 3333, 3334, 3335, 3336, 3337, 3338, 3339, 3340, 3341, 3342, 3343, 3344, 3345, 3346, 3347, 3348, 3349, 3350, 3351, 3352, 3353, 3354, 3356, 3357, 3358, 3359, 3360, 3361, 3363, 3364, 3365, 3366, 3367, 3368, 3369, 3370, 3372, 3373, 3374, 3375, 3376, 3377, 3378, 3379, 3380, 3381, 3382, 3383, 3384, 3385, 3386, 3387, 3388, 3389, 3390, 3391, 3392, 3393, 3394, 3395, 3396, 3397, 3398, 3399, 3400, 3401, 3402, 3403, 3404, 3405, 3406, 3407, 3408, 3409, 3410, 3411, 3412, 3413, 3414, 3415, 3416, 3417, 3418, 3419, 3420, 3421, 3422, 3423, 3424, 3425, 3426, 3427, 3428, 3430, 3431, 3432, 3433, 3435, 3436, 3437, 3438, 3439, 3440, 3441, 3443, 3444, 3445, 3447, 3448, 3449, 3450, 3451, 3452, 3453, 3454, 3455, 3456, 3457, 3459, 3460, 3461, 3462, 3463, 3464, 3465, 3466, 3467, 3468, 3469, 3470, 3472, 3473, 3474, 3475, 3476, 3477, 3478, 3479, 3480, 3481, 3482, 3483, 3484, 3485, 3487, 3488, 3489, 3490, 3491, 3492, 3493, 3494, 3495, 3497, 3498, 3500, 3501, 3502, 3505, 3506, 3507, 3509, 3510, 3511, 3512, 3513, 3514, 3515, 3516, 3517, 3518, 3519, 3520, 3521, 3522, 3523, 3524, 3525, 3526, 3527, 3528, 3529, 3530, 3531, 3532, 3533, 3534, 3535, 3536, 3537, 3538, 3540, 3541, 3542, 3543, 3544, 3545, 3546, 3547, 3548, 3550, 3551, 3552, 3553, 3554, 3555, 3556, 3557, 3559, 3560, 3561, 3562, 3563, 3564, 3565, 3566, 3567, 3568, 3569, 3570, 3571, 3572, 3573, 3574, 3575, 3576, 3578, 3579, 3580, 3581, 3582, 3583, 3584, 3585, 3586, 3587, 3588, 3589, 3590, 3591, 3592, 3594, 3595, 3596, 3597, 3599, 3600, 3602, 3604, 3605, 3607, 3608, 3609, 3610, 3612, 3613, 3614, 3615, 3616, 3617, 3618, 3619, 3620, 3621, 3622, 3623, 3624, 3625, 3626, 3627, 3628, 3629, 3630, 3631, 3632, 3633, 3634, 3635, 3636, 3637, 3638, 3639, 3640, 3641, 3643, 3644, 3645, 3646, 3647, 3648, 3649, 3650, 3651, 3652, 3654, 3656, 3657, 3658, 3659, 3660, 3661, 3662, 3663, 3664, 3665, 3666, 3667, 3668, 3669, 3671, 3672, 3674, 3675, 3676, 3677, 3678, 3679, 3680, 3681, 3683, 3684, 3685, 3686, 3687, 3688, 3689, 3691, 3692, 3693, 3695, 3696, 3697, 3698, 3699, 3700, 3701, 3702, 3703, 3704, 3705, 3706, 3708, 3709, 3710, 3711, 3712, 3713, 3714, 3715, 3717, 3718, 3719, 3720, 3721, 3722, 3723, 3724, 3725, 3726, 3728, 3729, 3730, 3731, 3732, 3733, 3734, 3735, 3738, 3739, 3740, 3741, 3742, 3743, 3745, 3746, 3747, 3748, 3749, 3750, 3751, 3752, 3754, 3755, 3756, 3757, 3758, 3759, 3760, 3761, 3762, 3763, 3764, 3766, 3767, 3768, 3769, 3770, 3771, 3772, 3773, 3774, 3775, 3776, 3777, 3778, 3779, 3780, 3781, 3782, 3783, 3784, 3785, 3786, 3787, 3788, 3789, 4463, 3792, 3793, 3794, 3795, 3796, 3797, 3800, 3801, 3802, 3803, 3804, 3805, 3806, 3807, 3808, 3809, 3810, 3811, 3812, 3813, 3814, 3815, 3816, 3817, 3818, 3819, 3820, 3821, 3822, 3823, 3824, 3825, 3826, 3827, 3828, 3829, 3830, 3831, 3832, 3833, 3834, 3835, 3837, 3838, 3839, 3840, 3841, 3842, 3843, 3844, 3845, 3846, 3847, 3848, 3849, 3851, 3852, 3853, 3854, 3855, 3856, 3857, 3858, 3859, 3860, 3861, 3862, 3863, 3864, 3865, 3866, 3867, 3868, 3869, 3870, 3871, 3873, 3874, 3875, 3876, 3877, 3878, 3879, 3880, 3882, 3883, 3884, 3886, 3887, 3888, 3889, 3890, 3891, 3892, 3893, 3894, 3895, 3896, 3897, 3898, 3899, 3900, 3901, 3902, 3903, 3904, 3905, 3906, 3907, 3908, 3909, 3910, 3911, 3912, 3913, 3914, 3917, 3918, 3919, 3920, 3921, 3923, 3924, 3925, 3926, 3927, 3928, 3929, 3930, 3932, 3933, 3934, 3935, 3936, 3937, 3939, 3940, 3941, 3942, 3943, 3944, 3945, 3946, 3947, 3948, 3949, 3950, 3951, 3952, 3953, 3954, 3955, 3956, 3957, 3958, 3959, 3960, 3961, 3962, 3963, 3964, 3965, 3966, 3967, 3968, 3970, 3971, 3972, 3973, 3974, 3975, 3976, 3977, 3978, 3979, 3980, 3981, 3982, 3983, 3984, 3985, 3986, 3987, 3988, 3989, 3990, 3991, 3992, 3993, 3994, 3995, 3996, 3997, 3998, 3999, 4000, 4001, 4002, 4003, 4004, 4005, 4006, 4007, 4008, 4009, 4010, 4012, 4013, 4014, 4015, 4016, 4018, 4019, 4020, 4021, 4022, 4023, 4024, 4025, 4026, 4027, 4028, 4029, 4030, 4031, 4032, 4033, 4034, 4035, 4036, 4037, 4038, 4039, 4040, 4041, 4042, 4043, 4044, 4045, 4046, 4047, 4048, 4049, 4050, 4051, 4052, 4053, 4054, 4055, 4057, 4058, 4060, 4061, 4062, 4063, 4064, 4065, 4066, 4067, 4069, 4070, 4071, 4072, 4073, 4074, 4075, 4086, 4087, 4088, 4089, 4091, 4092, 4093, 4094, 4095, 4096, 4097, 4098, 4099, 4100, 4101, 4102, 4103, 4104, 4105, 4107, 4108, 4109, 4110, 4111, 4113, 4114, 4115, 4116, 4118, 4119, 4120, 4121, 4122, 4123, 4124, 4125, 4126, 4127, 4128, 4129, 4130, 4131, 4132, 4133, 4134, 4135, 4136, 4137, 4138, 4139, 4140, 4141, 4142, 4143, 4146, 4147, 4148, 4149, 4150, 4151, 4152, 4153, 4154, 4155, 4156, 4157, 4177, 4178, 4183, 4184, 4185, 4186, 4187, 4188, 4189, 4190, 4191, 4192, 4193, 4194, 4195, 4196, 4197, 4198, 4199, 4200, 4202, 4203, 4204, 4205, 4206, 4207, 4208, 4209, 4210, 4211, 4212, 4213, 4214, 4215, 4216, 4217, 4218, 4219, 4220, 4222, 4223, 4224, 4225, 4226, 4227, 4228, 4229, 4230, 4231, 4232, 4233, 4234, 4236, 4237, 4238, 4239, 4240, 4241, 4242, 4243, 4244, 4245, 4246, 4247, 4248, 4249, 4250, 4251, 4252, 4253, 4254, 4255, 4256, 4257, 4258, 4259, 4260, 4261, 4262, 4263, 4264, 4265, 4266, 4267, 4268, 4269, 4270, 4271, 4272, 4273, 4274, 4275, 4276, 4277, 4278, 4279, 4280, 4281, 4282, 4283, 4284, 4286, 4287, 4289, 4290, 4291, 4292, 4293, 4294, 4295, 4296, 4297, 4298, 4299, 4300, 4301, 4302, 4303, 4304, 4306, 4307, 4308, 4309, 4310, 4311, 4312, 4313, 4314, 4315, 4316, 4317, 4318, 4319, 4320, 4321, 4322, 4324, 4325, 4326, 4327, 4328, 4329, 4330, 4331, 4332, 4334, 4335, 4337, 4338, 4339, 4340, 4341, 4342, 4343, 4344, 4345, 4346, 4347, 4348, 4349, 4350, 4351, 4352, 4354, 4355, 4356, 4357, 4358, 4359, 4360, 4361, 4362, 4363, 4364, 4365, 4366, 4367, 4368, 4369, 4370, 4371, 4372, 4373, 4374, 4375, 4376, 4377, 4378, 4379, 4380, 4381, 4382, 4383, 4384, 4385, 4386, 4387, 4388, 4389, 4390, 4391, 4392, 4393, 4394, 4395, 4396, 4397, 4399, 4400, 4401, 4402, 4403, 4404, 4405, 4406, 4407, 4408, 4410, 4411, 4412, 4413, 4414, 4415, 4416, 4418, 4419, 4420, 4422, 4424, 4425, 4426, 4427, 4428, 4430, 4431, 4432, 4433, 4434, 4436, 4437, 4438, 4439, 4440, 4441, 4442, 4443, 4444, 4445, 4446, 4447, 4449, 4451, 4452, 4453, 4454, 4455, 4456, 4457, 4458, 4459, 4460, 4461, 4462, 4463, 4464, 4465, 4466, 4467, 4468, 4470, 4471, 4473, 4474, 4475, 4477, 4478, 4479, 4480, 4481, 4483, 4484, 4485, 4486, 4487, 4489, 4490, 4491, 4492, 4493, 4494, 4495, 4496, 4497, 4498, 4499, 4500, 4501, 4502, 4503, 4504, 4505, 4506, 4507, 4508, 4509, 4510, 4511, 4512, 4514, 4515, 4516, 4517, 4519, 4520, 4521, 4522, 4523, 4524, 4525, 4526, 4527, 4528, 4529, 4530, 4531, 4532, 4533, 4534, 4535, 4536, 4537, 4538, 4539, 4540, 4541, 4542, 4543, 4545, 4546, 4547, 4548, 4549, 4550, 4551, 4552, 4553, 4554, 4555, 4556, 4557, 4558, 4561, 4562, 4563, 4564, 4565, 4566, 4567, 4568, 4570, 4572, 4573, 4574, 4575, 4576, 4577, 4579, 4580, 4581, 4582, 4583, 4584, 4585, 4586, 4587, 4588, 4589, 4590, 4591, 4592, 4593, 4594, 4595, 4596, 4597, 4598, 4599, 4600, 4601, 4602, 4603, 4604, 4605, 4606, 4608, 4609, 4610, 4611, 4612, 4613, 4614, 4615, 4616, 4617, 4618, 4619, 4620, 4621, 4623, 4624, 4625, 4626, 4627, 4628, 4629, 4630, 4632, 4633, 4634, 4635, 4636, 4637, 4638, 4639, 4640, 4641, 4644, 4645, 4646, 4649, 4651, 4652, 4653, 4654, 4655, 4656, 4657, 4659, 4660, 4661, 4662, 4663, 4664, 4665, 4666, 4667, 4668, 4670, 4671, 4673, 4674, 4675, 4676, 4677, 4678, 4679, 4680, 4681, 4683, 4684, 4685, 4686, 4690, 4691, 4692, 4693, 4694, 4695, 4696, 4697, 4698, 4699, 4700, 4701, 4702, 4703, 4705, 4706, 4708, 4709, 4710, 4711, 4712, 4713, 4714, 4715, 4716, 4717, 4719, 4720, 4721, 4722, 4723, 4724, 4725, 4726, 4728, 4729, 4730, 4731, 4732, 4733, 4734, 4735, 4736, 4737, 4738, 4739, 4740, 4741, 4742, 4743, 4744, 4745, 4746, 4747, 4748, 4749, 4750, 4751, 4752, 4753, 4755, 4756, 4757, 4758, 4759, 4760, 4761, 4762, 4763, 4764, 4765, 4766, 4768, 4769, 4770, 4771, 4772, 4773, 4774, 4775, 4776, 4777, 4778, 4779, 4780, 4781, 4782, 4783, 4784, 4785, 4787, 4788, 4789, 4790, 4791, 4793, 4794, 4795, 4796, 4798, 4799, 4800, 4801, 4802, 4803, 4805, 4806, 4807, 4808, 4809, 4810, 4811, 4813, 4815, 4816, 4817, 4818, 4819, 4820, 4821, 4822, 4824, 4825, 4826, 4827, 4828, 4829, 4831, 4832, 4834, 4835, 4836, 4837, 4839, 4840, 4841, 4842, 4843, 4844, 4845, 4846, 4847, 4850, 4851, 4852, 4853, 4854, 4855, 4856, 4857, 4858, 4860, 4861, 4863, 4864, 4865, 4866, 4867, 4868, 4869, 4870, 4871, 4873, 4874, 4875, and 4876.

In a third aspect, the present invention relates to an isolated biologically active substance encoded by a polynucleotide that hybridizes, as described above, under very low stringency conditions, preferably low stringency conditions, more preferably medium stringency conditions, more preferably medium-high stringency conditions, even more preferably high stringency conditions, and most preferably very high stringency conditions with a polynucleotide selected from the group consisting of (i) the polynucleotides of SEQ ID NOs: 2-4876, and subsequences thereof, and (ii) full-length complementary strands thereof. A subsequence of any of SEQ ID NOs: 2-4876 may be at least 100 nucleotides or preferably at least 200 nucleotides. Moreover, the subsequence may encode a fragment, *e.g.*, a fragment that has biological activity.

In a fourth aspect, the present invention relates to an artificial variant comprising a substitution, deletion, and/or insertion of one or more (several) amino acids of an amino acid sequence selected from the group consisting of SEQ ID NOs: 4877-9751 and fragments thereof retaining biological activity, as described above.

**Nucleic Acid Constructs**

The present invention also relates to nucleic acid constructs comprising an isolated polynucleotide or isolated polynucleotides (*e.g.*, operon) of the present invention operably linked to one or more (several) control sequences that direct the expression of the coding sequence in a suitable host cell under conditions compatible with the control sequences.

An isolated polynucleotide(s) of the present invention may be manipulated in a variety of ways to provide for production of a biologically active substance encoded directly or indirectly by the polynucleotide(s). Manipulation of the nucleotide sequence prior to its insertion into a vector may be desirable or necessary depending on the expression vector. The techniques for modifying nucleotide sequences utilizing recombinant DNA methods are well known in the art.

The control sequence may be an appropriate promoter sequence, a nucleotide sequence that is recognized by a host cell for expression of the polynucleotide(s) encoding the biologically active substance. The promoter sequence contains transcriptional control sequences that mediate the expression of the biologically active substance. The promoter may be any nucleotide sequence that shows transcriptional activity in the host cell of choice including mutant, truncated, and hybrid promoters, and may be obtained from genes encoding extracellular or intracellular polypeptides or biologically active substances either homologous or heterologous to the host cell.

Examples of suitable promoters for directing the transcription of the nucleic acid constructs of the present invention, especially in a bacterial host cell, are the promoters obtained from the *E. coli lac* operon, *Streptomyces coelicolor* agarase gene (*dagA*), *Bacillus subtilis* levansucrase gene (*sacB*), *Bacillus licheniformis* alpha-amylase gene *(amyL), Bacillus stearothermophilus* maltogenic amylase gene *(amyM), Bacillus amyloliquefaciens* alpha-amylase gene (*amyQ*), *Bacillus licheniformis* penicillinase gene *(penP), Bacillus subtilis xylA* and *xylB* genes, and prokaryotic beta-lactamase gene (Villa-Kamaroff et al., 1978, Proceedings of the National Academy of Sciences USA 75: 3727-3731), as well as the *tac* promoter (DeBoer et al., 1983, Proceedings of the National Academy of Sciences USA 80: 21-25). Further promoters are described in "Useful proteins from recombinant bacteria" in Scientific American, 1980, 242: 74-94; and in Sambrook *et al.,* 1989, *supra.*

The control sequence may also be a suitable transcription terminator sequence, a sequence recognized by a host cell to terminate transcription. The terminator sequence is operably linked to the 3' terminus of the gene encoding the biologically active substance. Any terminator that is functional in the host cell of choice may be used in the present invention.

The control sequence may also be a signal peptide coding region that codes for an amino acid sequence linked to the amino terminus of a polypeptide and directs the encoded polypeptide into the cell's secretory pathway. The 5' end of the coding sequence of the nucleotide sequence may inherently contain a signal peptide coding region naturally linked in translation reading frame with the segment of the coding region that encodes the secreted polypeptide. Alternatively, the 5' end of the coding sequence may contain a signal peptide coding region that is foreign to the coding sequence. The foreign signal peptide coding region may be required where the coding sequence does not naturally contain a signal peptide coding region. Alternatively, the foreign signal peptide coding region may simply replace the natural signal peptide coding region in order to enhance secretion of the polypeptide. However, any signal peptide coding region that directs the expressed polypeptide into the secretory pathway of a host cell of choice may be used in the present invention.

Effective signal peptide coding regions for bacterial host cells are the signal peptide coding regions obtained from the genes for *Bacillus* NCIB 11837 maltogenic amylase, *Bacillus stearothermophilus* alpha-amylase, *Bacillus licheniformis* subtilisin, *Bacillus licheniformis* beta-lactamase, *Bacillus stearothermophilus* neutral proteases *(nprT, nprS, nprM),* and *Bacillus subtilis prsA.* Further signal peptides are described by Simonen and Palva, 1993, Microbiological Reviews 57: 109-137.

The control sequence may also be a propeptide coding region that codes for an amino acid sequence positioned at the amino terminus of a polypeptide. The resultant polypeptide is known as a proenzyme or propolypeptide (or a zymogen in some cases). A propolypeptide is generally inactive and can be converted to a mature active polypeptide by catalytic or autocatalytic cleavage of the propeptide from the propolypeptide. The propeptide coding region may be obtained from the genes for *Bacillus subtilis* alkaline protease (*aprE*) and *Bacillus subtilis* neutral protease (*nprT).*

Where both signal peptide and propeptide regions are present at the amino terminus of a polypeptide, the propeptide region is positioned next to the amino terminus of a polypeptide and the signal peptide region is positioned next to the amino terminus of the propeptide region.

It may also be desirable to add regulatory sequences that allow the regulation of the expression of a biologically active substance relative to the growth of the host cell. Examples of regulatory systems are those that cause the expression of the gene to be turned on or off in response to a chemical or physical stimulus, including the presence of a regulatory compound. Regulatory systems in prokaryotic systems include the *lac, tac,* and *trp* operator systems. Other examples of regulatory sequences are those that allow for gene amplification. In eukaryotic systems, these include the dihydrofolate reductase gene that is amplified in the presence of methotrexate, and the metallothionein genes that are amplified with heavy metals. In these cases, the nucleotide sequence encoding the biologically active substance would be operably linked with the regulatory sequence.

**Expression Vectors**

The present invention also relates to recombinant expression vectors comprising an isolated polynucleotide of the present invention, a promoter, and transcriptional and translational stop signals. The various nucleic acid and control sequences described above may be joined together to produce a recombinant expression vector that may include one or more (several) convenient restriction sites to allow for insertion or substitution of the nucleotide sequence encoding the polypeptide at such sites. Alternatively, a polynucleotide of the present invention may be expressed by inserting the nucleotide sequence or a nucleic acid construct comprising the sequence into an appropriate vector for expression. In creating the expression vector, the coding sequence is located in the vector so that the coding sequence is operably linked with the appropriate control sequences for expression.

The recombinant expression vector may be any vector (*e.g.*, a plasmid or virus) that can be conveniently subjected to recombinant DNA procedures and can bring about the expression of a polynucleotide of the present invention. The choice of the vector will typically depend on the compatibility of the vector with the host cell into which the vector is to be introduced. The vectors may be linear or closed circular plasmids.

The vector may be an autonomously replicating vector, i.e., a vector that exists as an extrachromosomal entity, the replication of which is independent of chromosomal replication, *e.g.*, a plasmid, an extrachromosomal element, a minichromosome, or an artificial chromosome. The vector may contain any means for assuring self-replication. Alternatively, the vector may be one that, when introduced into the host cell, is integrated into the genome and replicated together with the chromosome(s) into which it has been integrated. Furthermore, a single vector or plasmid or two or more vectors or plasmids that together contain the total DNA to be introduced into the genome of the host cell, or a transposon may be used.

The vectors of the present invention preferably contain one or more (several) selectable markers that permit easy selection of transformed cells. A selectable marker is a gene the product of which provides for biocide or viral resistance, resistance to heavy metals, prototrophy to auxotrophs, and the like.

Examples of bacterial selectable markers are the *dal* genes from *Bacillus subtilis* or *Bacillus licheniformis,* or markers that confer antibiotic resistance such as ampicillin, kanamycin, chloramphenicol or tetracycline resistance.

The vectors of the present invention preferably contain an element(s) that permits integration of the vector into the host cell's genome or autonomous replication of the vector in the cell independent of the genome.

For integration into the host cell genome, the vector may rely on portions of the sequence of the gene or any other element of the vector for integration of the vector into the genome by homologous or nonhomologous recombination. Alternatively, the vector may contain additional nucleotide sequences for directing integration by homologous recombination into the genome of the host cell. The additional nucleotide sequences enable the vector to be integrated into the host cell genome at a precise location(s) in the chromosome(s). To increase the likelihood of integration at a precise location, the integrational elements should preferably contain a sufficient number of nucleotides, such as 100 to 10,000 base pairs, preferably 400 to 10,000 base pairs, and most preferably 800 to 10,000 base pairs, which are highly homologous with the corresponding target sequence to enhance the probability of homologous recombination. The integrational elements may be any sequence that is homologous with the target sequence in the genome of the host cell. Furthermore, the integrational elements may be non-encoding or encoding nucleotide sequences. On the other hand, the vector may be integrated into the genome of the host cell by non-homologous recombination.

For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" is defined herein as a sequence that enables a plasmid or vector to replicate *in vivo.* Examples of bacterial origins of replication are the origins of replication of plasmids pBR322, pUC19, pACYC177, and pACYC184 permitting replication in E. *coli,* and pUB110, pE194, pTA1060, and pAMß1 permitting replication in *Bacillus.*

More than one copy of a polynucleotide of the present invention may be inserted into the host cell to increase production of the polynucleotide product. An increase in the copy number of the polynucleotide can be obtained by integrating at least one additional copy of the sequence into the host cell genome or by including an amplifiable selectable marker gene with a polynucleotide of the present invention where cells containing amplified copies of the selectable marker gene, and thereby additional copies of the polynucleotide of the present invention, can be selected for by cultivating the cells in the presence of the appropriate selectable agent.

The procedures used to ligate the elements described above to construct the recombinant expression vectors of the present invention are well known to one skilled in the art (see, *e.g.*, Sambrook *et al., 1989, supra).*

**Host Cells**

The present invention also relates to recombinant host cells, comprising an isolated polynucleotide of the present invention, where the host cells are advantageously used in the recombinant production of a biologically active substance encoded by the polynucleotide. A vector comprising a polynucleotide of the present invention is introduced into a host cell so that the vector is maintained as a chromosomal integrant or as a self-replicating extra-chromosomal vector as described earlier. The term "host cell" encompasses any progeny of a parent cell that is not identical to the parent cell due to mutations that occur during replication. The choice of a host cell will to a large extent depend upon the polynucleotide encoding the biologically active substance and its source.

The host cell may be any unicellular microorganism, *e.g.*, a prokaryote, or a non-unicellular microorganism, *e.g.*, a eukaryote.

The bacterial host cell may be any Gram positive bacterium or a Gram negative bacterium. Gram positive bacteria include, but not limited to, *Bacillus, Streptococcus, Streptomyces, Staphylococcus, Enterococcus, Lactobacillus, Lactococcus, Clostridium, Geobacillus,* and *Oceanobacillus.* Gram negative bacteria include, but not limited to, *E. coli, Pseudomonas, Salmonella, Campylobacter, Helicobacter, Flavobacterium, Fusobacterium, Ilyobacter, Neisseria,* and *Ureaplasma.*

The bacterial host cell may be any *Bacillus* cell. *Bacillus* cells useful in the practice of the present invention include, but are not limited to, *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* and *Bacillus thuringiensis* cells.

In a preferred aspect, the bacterial host cell is a *Bacillus amyloliquefaciens, Bacillus lentus, Bacillus licheniformis, Bacillus stearothermophilus* or *Bacillus subtilis* cell. In a more preferred aspect, the bacterial host cell is a *Bacillus amyloliquefaciens* cell. In another more preferred aspect, the bacterial host cell is a *Bacillus clausii* cell. In another more preferred aspect, the bacterial host cell is a *Bacillus licheniformis* cell. In another more preferred aspect, the bacterial host cell is a *Bacillus subtilis* cell.

The bacterial host cell may also be any *Streptococcus* cell. *Streptococcus* cells useful in the practice of the present invention include, but are not limited to, *Streptococcus equisimilis, Streptococcus pyogenes, Streptococcus uberis,* and *Streptococcus equi* subsp. *Zooepidemicus.*

In a preferred aspect, the bacterial host cell is a *Streptococcus equisimilis* cell. In another preferred aspect, the bacterial host cell is a *Streptococcus pyogenes* cell. In another preferred aspect, the bacterial host cell is a *Streptococcus uberis* cell. In another preferred aspect, the bacterial host cell is a *Streptococcus equi* subsp. *Zooepidemicus* cell.

The bacterial host cell may also be any *Streptomyces* cell. *Streptomyces* cells useful in the practice of the present invention include, but are not limited to, *Streptomyces achromogenes, Streptomyces avermitilis, Streptomyces coelicolor, Streptomyces griseus,* and *Streptomyces lividans.*

In a preferred aspect, the bacterial host cell is a *Streptomyces achromogenes* cell. In another preferred aspect, the bacterial host cell is a *Streptomyces avermitilis* cell. In another preferred aspect, the bacterial host cell is a *Streptomyces coelicolor* cell. In another preferred aspect, the bacterial host cell is a *Streptomyces griseus* cell. In another preferred aspect, the bacterial host cell is a *Streptomyces lividans* cell.

The introduction of DNA into a *Bacillus* cell may, for instance, be effected by protoplast transformation (see, *e.g.*, Chang and Cohen, 1979, Molecular General Genetics 168: 111-115), by using competent cells (see, *e.g.*, Young and Spizizen, 1961, Journal of Bacteriology 81: 823-829, or Dubnau and Davidoff-Abelson, 1971, Journal of Molecular Biology 56: 209-221), by electroporation (see, *e.g.*, Shigekawa and Dower, 1988, Biotechniques 6: 742-751), or by conjugation (see, *e.g.*, Koehler and Thorne, 1987, Journal of Bacteriology 169: 5271-5278). The introduction of DNA into an *E coli* cell may, for instance, be effected by protoplast transformation (see, *e.g.*, Hanahan, 1983, J. Mol. Biol. 166: 557-580) or electroporation (see, *e.g.*, Dower et al., 1988, Nucleic Acids Res. 16: 6127-6145). The introduction of DNA into a *Streptomyces* cell may, for instance, be effected by protoplast transformation and electroporation (see, *e.g.*, Gong et al., 2004, Folia Microbiol. (Praha) 49: 399-405), by conjugation (see, *e.g.*, Mazodier et al., 1989, J. Bacteriol. 171: 3583-3585), or by transduction (see, *e.g.*, Burke et al., 2001, Proc. Natl. Acad. Sci. USA 98:6289-6294). The introduction of DNA into a *Pseudomonas* cell may, for instance, be effected by electroporation (see, *e.g.*, Choi et al., 2006, J. Microbiol. Methods 64: 391-397) or by conjugation (see, *e.g.*, Pinedo and Smets, 2005, Appl. Environ. Microbiol. 71: 51-57). The introduction of DNA into a *Streptococcus* cell may, for instance, be effected by natural competence (see, *e.g.*, Perry and Kuramitsu, 1981, Infect. Immun. 32: 1295-1297), by protoplast transformation (see, *e.g.*, Catt and Jollick, 1991, Microbios. 68: 189-2070, by electroporation (see, *e.g.*, Buckley et al., 1999, Appl. Environ. Microbiol. 65: 3800-3804) or by conjugation (see, *e.g.*, Clewell, 1981, Microbiol. Rev. 45: 409-436). However, any method known in the for introducing DNA into a host cell can be used.

**Methods of Production**

The present invention also relates to methods for producing a biologically active substance of the present invention comprising (a) cultivating a strain, which in its wild-type form is capable of producing the biologically active substance, under conditions conducive for production of the biologically active substance; and (b) recovering the biologically active substance. Preferably, the strain is of the genus *Bacillus,* and more preferably *Bacillus licheniformis.*

The present invention also relates to methods for producing a biologically active substance of the present invention comprising (a) cultivating a host cell under conditions conducive for production of the biologically active substance; and (b) recovering the biologically active substance.

The present invention also relates to methods for producing a biologically active substance of the present invention comprising (a) cultivating a host cell under conditions conducive for production of the biologically active substance, wherein the host cell comprises a mutant polynucleotide comprising at least one mutation in the coding region of any of SEQ ID NOs: 2-4876, wherein the mutant polynucleotide encodes a biologically active substance that consists of SEQ ID NOs: 4877-9751, respectively, and (b) recovering the biologically active substance.

In the production methods of the present invention, the cells are cultivated in a nutrient medium suitable for production of the biologically active substance using methods known in the art. For example, the cell may be cultivated by shake flask cultivation, and small-scale or large-scale fermentation (including continuous, batch, fed-batch, or solid state fermentations) in laboratory or industrial fermentors performed in a suitable medium and under conditions allowing the biologically active substance to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (*e.g.*, in catalogues of the American Type Culture Collection). If the biologically active substance is secreted into the nutrient medium, the biologically active substance can be recovered directly from the medium. If the biologically active substance is not secreted, it can be recovered from cell lysates.

The biologically active substances may be detected using methods known in the art that are specific for the polypeptides. These detection methods may include use of specific antibodies, formation of an enzyme product, or disappearance of an enzyme substrate. For example, an enzyme assay may be used to determine the activity of an enzyme. See, for example, Enzyme Nomenclature, Academic Press, Inc., New York, 2007.

The resulting biologically active substances may be recovered by methods known in the art. For example, the biologically active substances may be recovered from the nutrient medium by conventional procedures including, but not limited to, centrifugation, filtration, extraction, spray-drying, evaporation, or precipitation.

The biologically active substances of the present invention may be purified by a variety of procedures known in the art including, but not limited to, chromatography (*e.g.*, ion exchange, affinity, hydrophobic, chromatofocusing, and size exclusion), electrophoretic procedures (*e.g.*, preparative isoelectric focusing), differential solubility (*e.g.*, ammonium sulfate precipitation), SDS-PAGE, or extraction (see, *e.g.,* Protein Purification, J.-C. Janson and Lars Ryden, editors, VCH Publishers, New York, 1989).

**Plants**

The present invention also relates to a transgenic plant, plant part, or plant cell, which has been transformed with a polynucleotide encoding a biologically active substance of the present invention so as to express and produce the biologically active substance in recoverable quantities. The biologically active substance may be recovered from the plant or plant part. Alternatively, the plant or plant part containing the recombinant biologically active substance may be used as such for improving the quality of a food or feed, *e.g.*, improving nutritional value, palatability, and rheological properties, or to destroy an antinutritive factor.

The transgenic plant can be dicotyledonous (a dicot) or monocotyledonous (a monocot). Examples of monocot plants are grasses, such as meadow grass (blue grass, Poa), forage grass such as festuca, lolium, temperate grass, such as Agrostis, and cereals, *e.g.*, wheat, oats, rye, barley, rice, sorghum, and maize (corn).

Examples of dicot plants are tobacco, legumes, such as lupins, potato, sugar beet, pea, bean and soybean, and cruciferous plants (family Brassicaceae), such as cauliflower, rape seed, and the closely related model organism *Arabidopsis thaliana.*

Examples of plant parts are stem, callus, leaves, root, fruits, seeds, and tubers as well as the individual tissues comprising these parts, *e.g.*, epidermis, mesophyll, parenchyme, vascular tissues, meristems. In the present context, also specific plant cell compartments, such as chloroplast, apoplast, mitochondria, vacuole, peroxisomes and cytoplasm are considered to be a plant part. Furthermore, any plant cell, whatever the tissue origin, is considered to be a plant part. Likewise, plant parts such as specific tissues and cells isolated to facilitate the utilisation of the invention are also considered plant parts e.g. embryos, endosperms, aleurone and seeds coats.

Also included within the scope of the present invention are the progeny of such plants, plant parts, and plant cells.

The transgenic plant or plant cell expressing a biologically active substance of the present invention may be constructed in accordance with methods known in the art. Briefly, the plant or plant cell is constructed by incorporating one or more (several) expression constructs encoding a biologically active substance of the present invention into the plant host genome and propagating the resulting modified plant or plant cell into a transgenic plant or plant cell.

The expression construct is conveniently a nucleic acid construct that comprises a polynucleotide encoding a biologically active substance of the present invention operably linked with appropriate regulatory sequences required for expression of the nucleotide sequence in the plant or plant part of choice. Furthermore, the expression construct may comprise a selectable marker useful for identifying host cells into which the expression construct has been integrated and DNA sequences necessary for introduction of the construct into the plant in question (the latter depends on the DNA introduction method to be used).

The choice of regulatory sequences, such as promoter and terminator sequences and optionally signal or transit sequences is determined, for example, on the basis of when, where, and how the biologically active substance is desired to be expressed. For instance, the expression of the polynucleotide encoding a biologically active substance of the present invention may be constitutive or inducible, or may be developmental, stage or tissue specific, and the gene product may be targeted to a specific tissue or plant part such as seeds or leaves. Regulatory sequences are, for example, described by Tague et al., 1988, Plant Physiology 86: 506.

For constitutive expression the 35S-CaMV, the maize ubiquitin 1 and the rice actin 1 promoter may be used (Franck et al., 1980. Cell 21: 285-294, Christensen AH, Sharrock RA and Quail, 1992, Plant Mo. Biol. 18: 675-689.; Zhang W, McElroy D. and Wu R., 1991, Plant Cell 3: 1155-1165). Organ-specific promoters may be, for example, a promoter from storage sink tissues such as seeds, potato tubers, and fruits (Edwards and Coruzzi, 1990, Ann. Rev. Genet. 24: 275-303), or from metabolic sink tissues such as meristems (Ito et al., 1994, Plant Mol. Biol. 24: 863-878), a seed specific promoter such as the glutelin, prolamin, globulin, or albumin promoter from rice (Wu et al., 1998, Plant and Cell Physiology 39: 885-889), a *Vicia faba* promoter from the legumin B4 and the unknown seed protein gene from *Vicia* faba (Conrad et al., 1998, Journal of Plant Physiology 152: 708-711), a promoter from a seed oil body protein (Chen et al., 1998, Plant and Cell Physiology 39: 935-941), the storage protein napA promoter from *Brassica napus,* or any other seed specific promoter known in the art, *e.g.*, as described in WO 91/14772. Furthermore, the promoter may be a leaf specific promoter such as the *rbcs* promoter from rice or tomato (Kyozuka et al., 1993, Plant Physiology 102: 991-1000, the chlorella virus adenine methyltransferase gene promoter (Mitra and Higgins, 1994, Plant Molecular Biology 26: 85-93), or the *aldP* gene promoter from rice (Kagaya et al., 1995, Molecular and General Genetics 248: 668-674), or a wound inducible promoter such as the potato pin2 promoter (Xu et al., 1993, Plant Molecular Biology 22: 573-588). Likewise, the promoter may inducible by abiotic treatments such as temperature, drought or alterations in salinity or induced by exogenously applied substances that activate the promoter, *e.g.*, ethanol, oestrogens, plant hormones like ethylene, abscisic acid and gibberellic acid and heavy metals.

A promoter enhancer element may also be used to achieve higher expression of the enzyme in the plant. For instance, the promoter enhancer element may be an intron that is placed between the promoter and the nucleotide sequence encoding a biologically active substance of the present invention. For instance, Xu *et al.,* 1993, *supra* disclose the use of the first intron of the rice actin 1 gene to enhance expression.

The selectable marker gene and any other parts of the expression construct may be chosen from those available in the art.

The nucleic acid construct is incorporated into the plant genome according to conventional techniques known in the art, including Agrobacterium-mediated transformation, virus-mediated transformation, microinjection, particle bombardment, biolistic transformation, and electroporation (Gasser et al., 1990, Science 244: 1293; Potrykus, 1990, Bio/Technology 8: 535; Shimamoto et al., 1989, Nature 338: 274).

Presently, *Agrobacterium tumefaciens*-mediated gene transfer is the method of choice for generating transgenic dicots (for a review, see Hooykas and Schilperoort, 1992, Plant Molecular Biology 19: 15-38). However it can also be used for transforming monocots, although other transformation methods are generally preferred for these plants. Presently, the method of choice for generating transgenic monocots is particle bombardment (microscopic gold or tungsten particles coated with the transforming DNA) of embryonic calli or developing embryos (Christou, 1992, Plant Journal 2: 275-281; Shimamoto, 1994, Current Opinion Biotechnology 5: 158-162; Vasil et al., 1992, Bio/Technology 10: 667-674). An alternative method for transformation of monocots is based on protoplast transformation as described by Omirulleh et al., 1993, Plant Molecular Biology 21: 415-428.

Following transformation, the transformants having incorporated therein the expression construct are selected and regenerated into whole plants according to methods well-known in the art. Often the transformation procedure is designed for the selective elimination of selection genes either during regeneration or in the following generations by using, for example, co-transformation with two separate T-DNA constructs or site specific excision of the selection gene by a specific recombinase.

The present invention also relates to methods for producing a biologically active substance of the present invention comprising (a) cultivating a transgenic plant or a plant cell comprising a polynucleotide encoding a biologically active substance of the present invention under conditions conducive for production of the biologically active substance; and (b) recovering the biologically active substance.

## Removal or Reduction of Biologically Active Substance

The present invention also relates to methods for producing a mutant of a parent cell, which comprises disrupting or deleting all or a portion of a polynucleotide encoding a biologically active substance of the present invention, which results in the mutant cell producing less of the biologically active substance than the parent cell when cultivated under the same conditions.

The mutant cell may be constructed by reducing or eliminating expression of a gene encoding or regulatory synthesis of a biologically active substance of the present invention using methods well known in the art, for example, insertions, disruptions, replacements, or deletions. The gene to be modified or inactivated may be, for example, the coding region or a part thereof essential for activity, or a regulatory element of the gene required for the expression of the coding region. An example of such a regulatory or control sequence may be a promoter sequence or a functional part thereof, i.e., a part that is sufficient for affecting expression of the gene. Other control sequences for possible modification include, but are not limited to, a leader, propeptide sequence, signal peptide sequence, transcription terminator, and transcriptional activator.

Modification or inactivation of the gene may be performed by subjecting the parent cell to mutagenesis and selecting for mutant cells in which expression of the gene has been reduced or eliminated. The mutagenesis, which may be specific or random, may be performed, for example, by use of a suitable physical or chemical mutagenizing agent, by use of a suitable oligonucleotide, or by subjecting the DNA sequence to PCR generated mutagenesis. Furthermore, the mutagenesis may be performed by use of any combination of these mutagenizing agents.

Examples of a physical or chemical mutagenizing agent suitable for the present purpose include ultraviolet (UV) irradiation, hydroxylamine, N-methyl-N'-nitro-N-nitrosoguanidine (MNNG), O-methyl hydroxylamine, nitrous acid, ethyl methane sulphonate (EMS), sodium bisulphite, formic acid, and nucleotide analogues.

When such agents are used, the mutagenesis is typically performed by incubating the parent cell to be mutagenized in the presence of the mutagenizing agent of choice under suitable conditions, and screening and/or selecting for mutant cells exhibiting reduced or no expression of the gene.

Modification or inactivation of the nucleotide sequence may be accomplished by introduction, substitution, or removal of one or more (several) nucleotides in the gene or a regulatory element required for the transcription or translation thereof. For example, nucleotides may be inserted or removed so as to result in the introduction of a stop codon, the removal of the start codon, or a change in the open reading frame. Such modification or inactivation may be accomplished by site-directed mutagenesis or PCR generated mutagenesis in accordance with methods known in the art. Although, in principle, the modification may be performed in *vivo, i.e.,* directly on the cell expressing the nucleotide sequence to be modified, it is preferred that the modification be performed *in vitro* as exemplified below.

An example of a convenient way to eliminate or reduce expression of a nucleotide sequence by a cell of choice is based on techniques of gene replacement, gene deletion, or gene disruption. For example, in the gene disruption method, a nucleic acid sequence corresponding to the endogenous nucleotide sequence is mutagenized *in vitro* to produce a defective nucleic acid sequence that is then transformed into the parent cell to produce a defective gene. By homologous recombination, the defective nucleic acid sequence replaces the endogenous nucleotide sequence. It may be desirable that the defective nucleotide sequence also encodes a marker that may be used for selection of transformants in which the nucleotide sequence has been modified or destroyed. In a particularly preferred aspect, the nucleotide sequence is disrupted with a selectable marker such as those described herein.

Alternatively, modification or inactivation of the nucleotide sequence may be performed by established antisense or RNA interference (RNAi) techniques using a sequence complementary to the nucleotide sequence. More specifically, expression of the nucleotide sequence by a cell may be reduced or eliminated by introducing a sequence complementary to the nucleic acid sequence of the gene that may be transcribed in the cell and is capable of hybridizing to the mRNA produced in the cell. Under conditions allowing the complementary anti-sense nucleotide sequence to hybridize to the mRNA, the amount of protein translated is thus reduced or eliminated.

The present invention further relates to a mutant cell of a parent cell that comprises a disruption or deletion of a nucleotide sequence encoding the biologically active substance or a control sequence thereof, which results in the mutant cell producing less of the biologically active substance than the parent cell.

The biologically active substance-deficient mutant cells so created are particularly useful as host cells for the expression of homologous and/or heterologous substances, such as polypeptides. Therefore, the present invention further relates to methods for producing a homologous or heterologous substance comprising (a) cultivating the mutant cell under conditions conducive for production of the substance; and (b) recovering the substance. The term "heterologous substances" is defined herein as substances that are not native to the host cell, a native substance in which modifications have been made to alter the native sequence, or a native substance whose expression is quantitatively altered as a result of a manipulation of the host cell by recombinant DNA techniques.

In a further aspect, the present invention relates to a method for producing a protein product essentially free of a biologically active substance by fermentation of a cell that produces both a biologically active substance of the present invention as well as the protein product of interest by adding an effective amount of an agent capable of inhibiting activity of the biologically active substance to the fermentation broth before, during, or after the fermentation has been completed, recovering the product of interest from the fermentation broth, and optionally subjecting the recovered product to further purification.

In accordance with this aspect of the invention, it is possible to remove at least 60%, preferably at least 75%, more preferably at least 85%, still more preferably at least 95%, and most preferably at least 99% of the biologically active substance. Complete removal of biologically active substance may be obtained by use of this method.

The methods used for cultivation and purification of the product of interest may be performed by methods known in the art.

The methods of the present invention for producing an essentially biologically active substance-free product is of particular interest in the production of prokaryotic polypeptides, in particular bacterial proteins such as enzymes. The enzyme may be selected from, *e.g.*, an amylolytic enzyme, lipolytic enzyme, proteolytic enzyme, cellulytic enzyme, oxidoreductase, or plant cell-wall degrading enzyme. Examples of such enzymes include an aminopeptidase, amylase, amyloglucosidase, carbohydrase, carboxypeptidase, catalase, cellulase, chitinase, cutinase, cyclodextrin glycosyltransferase, deoxyribonuclease, esterase, galactosidase, beta-galactosidase, glucoamylase, glucose oxidase, glucosidase, haloperoxidase, hemicellulase, invertase, isomerase, laccase, ligase, lipase, lyase, mannosidase, oxidase, pectinolytic enzyme, peroxidase, phytase, phenoloxidase, polyphenoloxidase, proteolytic enzyme, ribonuclease, transferase, transglutaminase, or xylanase. The biologically active substance-deficient cells may also be used to express heterologous proteins of pharmaceutical interest such as hormones, growth factors, receptors, polymers, *e.g.*, hyaluronic acid and elastin, and the like.

It will be understood that the term "prokaryotic polypeptides" includes not only native polypeptides, but also those polypeptides, *e.g.*, enzymes, which have been modified by amino acid substitutions, deletions or additions, or other such modifications to enhance activity, thermostability, pH tolerance and the like.

In a further aspect, the present invention relates to a product of a protein or substance essentially free of a biologically active substance of the invention, produced by a method of the present invention.

## Methods of Inhibiting Expression of a Polypeptide

The present invention also relates to methods of inhibiting the expression of a biological substance in a cell, comprising administering to the cell or expressing in the cell a double-stranded RNA (dsRNA) molecule, wherein the dsRNA comprises a subsequence of a polynucleotide of the present invention. In a preferred aspect, the dsRNA is about 15, 16, 17, 18, 19, 20, 21, 22, 23, 24, 25 or more duplex nucleotides in length.

The dsRNA is preferably a small interfering RNA (siRNA) or a micro RNA (miRNA). In a preferred aspect, the dsRNA is small interfering RNA (siRNAs) for inhibiting transcription. In another preferred aspect, the dsRNA is micro RNA (miRNAs) for inhibiting translation.

The present invention also relates to such double-stranded RNA (dsRNA) molecules, comprising a portion of the coding sequence of any of SEQ ID NOs: 2-4876 for inhibiting expression of a biological substance in a cell. While the present invention is not limited by any particular mechanism of action, the dsRNA can enter a cell and cause the degradation of a single-stranded RNA (ssRNA) of similar or identical sequences, including endogenous mRNAs. When a cell is exposed to dsRNA, mRNA from the homologous gene is selectively degraded by a process called RNA interference (RNAi).

The dsRNAs of the present invention can be used in gene-silencing therapeutics. In one aspect, the invention provides methods to selectively degrade RNA using the dsRNAis of the present invention. The process may be practiced *in vitro,* ex *vivo* or *in vivo.* In one aspect, the dsRNA molecules can be used to generate a loss-of-function mutation in a cell, an organ or an animal. Methods for making and using dsRNA molecules to selectively degrade RNA are well known in the art, see, for example, U.S. Patent No. 6,506,559; U.S. Patent No. 6,511,824; U.S. Patent No. 6,515,109; and U.S. Patent No. 6,489,127.

**Compositions**

The present invention also relates to compositions comprising a biologically active substance of the present invention. Preferably, the compositions are enriched in the biologically active substance. The term "enriched" indicates that the biologically active substance of the composition has been increased, *e.g.*, with an enrichment factor of 1.1.

The composition may comprise a biologically active substance of the invention as the major component, *e.g.*, a mono-component composition. Alternatively, the composition may comprise multiple biologically active substances, for example, multiple enzymes, such as an aminopeptidase, amylase, carbohydrase, carboxypeptidase, catalase, cellulase, chitinase, cutinase, cyclodextrin glycosyltransferase, deoxyribonuclease, esterase, alpha-galactosidase, beta-galactosidase, glucoamylase, alpha-glucosidase, beta-glucosidase, haloperoxidase, invertase, laccase, lipase, mannosidase, oxidase, pectinolytic enzyme, peptidoglutaminase, peroxidase, phytase, polyphenoloxidase, proteolytic enzyme, ribonuclease, transglutaminase, or xylanase.

The compositions may be prepared in accordance with methods known in the art and may be in the form of a liquid or a dry composition. For instance, the composition may be in the form of a granulate or a microgranulate. The biologically active substance to be included in the composition may be stabilized in accordance with methods known in the art.

**Methods for Using the *Bacillus licheniformis* Chromosome**

The present invention also relates to methods for using the *Bacillus licheniformis* chromosome.

The chromosome of *Bacillus licheniformis* serves as a reservoir of useful genes/proteins that have environmental, energy, health, and industrial applications (*e.g.*, enzymes, antibiotics, biochemicals). A clear extension of this is that the newly discovered molecules can be used as starting points for further improvements via well-established gene shuffling, directed evolution, and protein engineering methods. Additionally, regions or motifs (*e.g.*, signal peptides, active sites, substrate-binding regions) from the newly discovered molecules may be employed to derive novel chimeras with industrially advantageous properties.

The 5' and 3' untranslated regions that are located upstream and downstream, respectively, of the chromosomal genes contain promoters, terminators, and other regulatory elements that control transcription and translation of the adjacent coding DNA sequences (CDSs). These elements may be used to construct novel vectors for efficient expression of homologous and heterologous genes in *Bacillus licheniformis* and other *Bacillus* species.

The chromosomal DNA sequence may be used for selecting integration sites for stable inheritance and expression of inserted vector constructs. These constructs may be targeted to specific chromosomal locations using homologous recombination between vector and chromosomal DNA molecules.

The genes encoded in the chromosome may be used for monitoring global gene expression during the life cycle of the organism or during industrial fermentations (*e.g.*, implemented on DNA microarrays). By monitoring global gene expression, for example, improved processes for industrial fermentation can be implemented with greater efficiency and economy.

The genes of *Bacillus licheniformis* SJ1904 may be employed to improve industrial fermentation strains via selective breeding, conjugative mating, bacteriophage-mediated transduction, and DNA-mediated transformation. In addition, the chromosome of *Bacillus licheniformis* SJ1904 may be useful, in whole or in part, to construct new microbial cell factories via synthetic biology approaches (Danchin, A. 2004. The bag or the spindle: the cell factory at the time of systems' biology. Microb. Cell Fact. 3: 13).

The chromosome is useful in comparative evolutionary and ecological studies. For example, dozens of *Bacillus licheniformis* isolates can be readily compared on a global scale by hybridization of their genomic DNAs to a microarray fabricated from the reference strain presented herein (so-called comparative genomic hybridization). Using this method, one can compare various isolates to look for similarities/differences among geographical and environmental niches or among biocontrol strains versus saprophytic isolates.

The chromosome sequence may be used to construct the metabolic blueprint for *Bacillus licheniformis* that includes all catabolic and anabolic pathways, signaling pathways, regulatory networks, growth substrates, biochemical intermediates, end products, electron donors/acceptors and others. In doing so, it is possible to modify the metabolic machinery of the organism by deleting unwanted pathways and/or adding enzymes/pathways from other organisms to generate useful chemicals and intermediates.

The pathways and components that contribute to production of extracellular and surface proteins in *Bacillus licheniformis* can be extracted from the chromosomal sequence. This affords opportunities for improved production of extracellular proteins by genetic manipulation of the secretion machinery.

The chromosome data allows deduction of the essential genes for *Bacillus licheniformis* (either by comparison to related bacteria such as *Bacillus subtilis* or by systematic gene-by-gene knock outs). Thus it has become possible to design custom-made strains that contain only the genes that are essential for production of specific proteins or metabolites (so-called cell factory concept).

The chromosome data may be used to construct interspecies hybrids between *Bacillus licheniformis* and other bacteria. Venter et al., 2003, Proc. Nat. Acad. Sci. USA 100, 15440-15445 have shown that it is possible to construct an entire virus genome from smaller DNA segments. Thus, segments of the *Bacillus licheniformis* chromosome may be employed to derive novel chromosomal segments or even entire chimeric chromosomes for specific applications.

In a preferred aspect, methods for using the *Bacillus licheniformis* chromosome include host improvement, *e.g.*, secretion of a protein or metabolite, genome shuffling, construction of new genomes, metabolic engineering and pathway reconstruction, carrier for heterologous expression vectors, microarrays as described herein, identification of polypeptides in proteomics analyses, and comparative genomics with other *Bacillus* species or related organisms.

**Methods for Isolating Genes**

The present invention also relates to methods for isolating a polynucleotide encoding a biologically active substance from a microbial strain. The method comprises first the addition of a mixture of first labeled nucleic acid probes, isolated from a microbial strain cultured on medium without an inducing substrate, and a mixture of second labeled nucleic acid probes, isolated from the microbial strain cultured on medium with the inducing substrate, to an array of *Bacillus licheniformis* polynucleotides selected from the group consisting of the polynucleotides of SEQ ID NOs: 2-4876, full-length complementary strands of SEQ ID NOs: 2-4876, and fragments of SEQ ID NOs: 2-4876, under conditions where the labeled nucleic acid probes hybridize to complementary sequences of the *Bacillus licheniformis* polynucleotides on the array. The first nucleic acid probes are labeled with a first reporter and the second nucleic acid probes are labeled with a second reporter. The array is then examined under conditions wherein the relative expression of the genes of the microbial strain is determined by the observed hybridization reporter signal of each spot on the array in which (i) the *Bacillus licheniformis* polynucleotides on the array that hybridize to the first nucleic acid probes produce a distinct first hybridization reporter signal or to the second nucleic acid probes produce a distinct second hybridization reporter signal, and (ii) the *Bacillus licheniformis* polynucleotides on the array that hybridize to both the first and second nucleic acid probes produce a distinct combined hybridization reporter signal. The probe is then sequenced to isolate from the microbial strain the corresponding gene that encodes an enzyme that degrades or converts the substrate.

**Enzymes.** The gene of interest may encode any enzyme including an oxidoreductase, transferase, hydrolase, lyase, isomerase, or ligase. In a preferred aspect, the enzyme is an acylase, alpha-glucosidase, amidase, aminopeptidase, amylase, carbohydrase, carboxypeptidase, catalase, cellulase, chitinase, cutinase, cyclodextrin glycosyltransferase, deoxyribonuclease, dextrinase, endoglucanase, esterase, galactanase, alpha-galactosidase, beta-galactosidase, glucoamylase, glucanase, glucocerebrosidase, alpha-glucosidase, beta-glucosidase, hemicellulase, invertase, laccase, lignase, lipase, lysin, mannosidase, mutanase, oxidase, pectinolytic enzyme, peroxidase, phosphatase, phospholipase, phytase, polyphenoloxidase, proteolytic enzyme, pullulanase, ribonuclease, transglutaminase, urokinase, or xylanase.

**Inducing Substrate.** The inducing substrate may be any substrate that is subject to the action of an enzyme, i.e., that degrades or converts the substrate. In a preferred aspect, the inducing substrate is lignin or a lignin-containing material. In a more preferred aspect, the lignin-containing material is lignocellulose. In another preferred aspect, the inducing substrate is cellulose. In another preferred aspect, the inducing substrate is hemicellulose. In another preferred aspect, the inducing substrate is pectin. In another preferred aspect, the inducing substrate is a lipid. In another preferred aspect, the inducing substrate is phospholipid. In another preferred aspect, the inducing substrate is phytic acid. In another preferred aspect, the inducing substrate is protein. In another preferred aspect, the inducing substrate is a starch. In another preferred aspect, the inducing substrate is a medium that is low in nutrients such as amino acids, carbon, nitrogen, phosphate, or iron.

In a more preferred aspect, the protein substrate is blood, casein, egg, gelatin, gluten, milk protein, or soy protein. In another more preferred aspect, the lignin-containing material is hardwood thermomechanical pulp. In another more preferred aspect, the lignocellulose is corn stover. In another more preferred aspect, the lignocellulose is white poplar. In another more preferred aspect, the lignocellulose is rice straw. In another more preferred aspect, the lignocellulose is switch grass.

**Microbial Strains.** In the methods of the present invention, the microbial strain may be any microbial strain. The strain is cultured on a suitable nutrient medium with and without a substrate of interest. The strain cultured on medium without the substrate is used as a reference for identifying differences in expression of the same or similar complement of genes in the strain cultured on medium with substrate. The strain may be a wild-type, mutant, or recombinant strain.

In the methods of the present invention, the microbial strain is preferably a bacterium. In a more preferred aspect, the bacterium is a *Bacillus, Pseudomonas, Streptococcus,* or *Streptomyces* strain or E. *coli.*

The *Bacillus* strain may be any *Bacillus* strain. In a preferred aspect, the *Bacillus* strain is *Bacillus alkalophilus, Bacillus amyloliquefaciens, Bacillus brevis, Bacillus cereus, Bacillus circulans, Bacillus clausii, Bacillus coagulans, Bacillus fastidiosus, Bacillus firmus, Bacillus lautus, Bacillus lentus, Bacillus licheniformis, Bacillus macerans, Bacillus megaterium, Bacillus methanolicus, Bacillus pumilus, Bacillus sphaericus, Bacillus stearothermophilus, Bacillus subtilis,* or *Bacillus thuringiensis.* It will be understood that the term *"Bacillus"* also encompasses relatives of *Bacillus* such as *Paenibacillus, Oceanobacillus,* and the like.

The *Pseudomonas* strain may be any *Pseudomonas* strain. In a preferred aspect, the *Pseudomonas* strain is *Pseudomonas acidovorans, Pseudomonas aeruginosa, Pseudomonas alcaligenes, Pseudomonas anguilliseptica, Pseudomonas abtimicrobica, Pseudomonas aurantiaca, Pseudomonas aureofaciens, Pseudomonas beijerinckii, Pseudomonas boreopolis, Pseudomonas chlororaphis, Pseudomonas citronellolis, Pseudomonas cocovenenans, Pseudomonas diminuta, Pseudomonas doudoroffii, Pseudomonas echinoides, Pseudomonas elongata, Pseudomonas fluorescens, Pseudomonas fragi, Pseudomonas halophobica, Pseudomonas huttiensis, Pseudomonas indigofera, Pseudomonas lanceolata, Pseudomonas lemoignei, Pseudomonas lundensis, Pseudomonas mendocina, Pseudomonas mephitica, Pseudomonas mucidolens, Pseudomonas oleovorans, Pseudomonas phenazinium, Pseudomonas pictorium, Pseudomonas putida, Pseudomonas resinovorans, Pseudomonas saccharophila, Pseudomonas stanieri, Pseudomonas stutzeri, Pseudomonas taetrolens,* or *Pseudomonas vesicularis.*

The *Streptococcus* strain may be any *Streptococcus* strain. In a preferred aspect, the *Streptococcus* strain is a *Streptococcus equisimilis* cell. In another preferred aspect, the *Streptococcus* strain is a *Streptococcus pyogenes* cell. In another preferred aspect, the *Streptococcus* strain is a *Streptococcus uberis* cell. In another preferred aspect, the *Streptococcus* strain is a *Streptococcus equi* subsp. *Zooepidemicus* cell.

The *Streptomyces* strain may be any *Streptomyces* strain. In a preferred aspect, the *Streptomyces* strain is a *Streptomyces achromogenes* cell. In another preferred aspect, the *Streptomyces* strain is a *Streptomyces avermitilis* cell. In another preferred aspect, the *Streptomyces* strain is a *Streptomyces coelicolor* cell. In another preferred aspect, the *Streptomyces* strain is a *Streptomyces griseus* cell. In a preferred aspect, the *Streptomyces* strain is *Streptomyces lividans.* In another preferred aspect, the *Streptomyces* strain is *Streptomyces murinus.*

**Microarrays.** The term "an array of *Bacillus licheniformis* polynucleotides" is defined herein as a linear or two-dimensional array of preferably discrete elements of an array of *Bacillus licheniformis* polynucleotides selected from the group consisting of SEQ ID NOs: 2-4876, full-length complementary strands of SEQ ID NOs: 2-4876, and fragments of SEQ ID NOs: 2-4876 (*e.g.*, synthetic oligonucleotides of, for example, 20-60 nucleotides), wherein each discrete element has a finite area, formed on the surface of a solid support. It is understood herein that the term *"Bacillus licheniformis* polynucleotides" encompasses the polynucleotides of SEQ ID NOs: 2-4876, full-length complementary strands of SEQ ID NOs: 2-4876, and fragments of SEQ ID NOs: 2-4876.

The term "microarray" is defined herein as an array of *Bacillus licheniformis* polynucleotide elements having a density of discrete of *Bacillus licheniformis* polynucleotide elements of at least about $100/cm^2$, and preferably at least about $1000/cm^2$. The *Bacillus licheniformis* polynucleotide elements in a microarray have typical dimensions, *e.g.*, diameters, in the range of between about 10 to about 250 $\mu$m, preferably in the range of between about 10 to about 200 $\mu$m, more preferably in the range of between about 20 to about 150 $\mu$m, even more preferably in the range of between about 20 to about 100 $\mu$m, most preferably in the range of between about 50 to about 100 $\mu$m, and even most preferably in the range of between about 80 to about 100 $\mu$m, and are separated from other polynucleotide elements in the microarray by about the same distance.

Methods and instruments for forming microarrays on the surface of a solid support are well known in the art. See, for example, U.S. Patent No. 5,807,522; U.S. Patent No. 5,700,637; and U.S. Patent No. 5,770,151. The instrument may be an automated device such as described in U.S. Patent No. 5,807,522.

The term "a substrate containing an array of *Bacillus licheniformis* polynucleotides" is defined herein as a solid support having deposited on the surface of the support one or more (several) of a plurality of *Bacillus licheniformis* polynucleotides, as described herein, for use in detecting binding of labeled nucleic acids to the *Bacillus licheniformis* polynucleotides.

The substrate may, in one aspect, be a glass support (*e.g.*, glass slide) having a hydrophilic or hydrophobic coating on the surface of the support, and an array of distinct random nucleic acid fragments bound to the coating, where each distinct random nucleic acid fragment is disposed at a separate, defined position.

Each microarray in the substrate preferably contains at least $10^3$ distinct *Bacillus licheniformis* in a surface area of less than about 5 or 6 $cm^2$. Each distinct *Bacillus licheniformis* polynucleotide (i) is disposed at a separate, defined position on the array, (ii) has a length of at least 50 bp, and (iii) is present in a defined amount between about 0.1 femtomoles and 100 nanomoles or higher if necessary.

For a hydrophilic coating, the glass slide is coated by placing a film of a polycationic polymer with a uniform thickness on the surface of the slide and drying the film to form a dried coating. The amount of polycationic polymer added should be sufficient to form at least a monolayer of polymers on the glass surface. The polymer film is bound to the surface via electrostatic binding between negative silyl-OH groups on the surface and charged cationic groups in the polymers. Such polycationic polymers include, but are not limited to, polylysine and polyarginine.

Another coating strategy employs reactive aldehydes to couple DNA to the slides (Schena et al., 1996, Proceedings of the National Academy of Science USA 93: 10614-10619; Heller at al., 1997, Proceedings of the National Academy of Science USA 94: 2150-2155).

Alternatively, the surface may have a relatively hydrophobic character, i.e., one that causes aqueous medium deposited on the surface to bead. A variety of known hydrophobic polymers, such as polystyrene, polypropylene, or polyethylene, have desirable hydrophobic properties, as do glass and a variety of lubricant or other hydrophobic films that may be applied to the support surface. A support surface is "hydrophobic" if an aqueous droplet applied to the surface does not spread out substantially beyond the area size of the applied droplet, wherein the surface acts to prevent spreading of the droplet applied to the surface by hydrophobic interaction with the droplet.

In another aspect, the substrate may be a multi-cell substrate where each cell contains a microarray of *Bacillus licheniformis* and preferably an identical microarray, formed on a porous surface. For example, a 96-cell array may typically have array dimensions between about 12 and 244 mm in width and 8 and 400 mm in length, with the cells in the array having width and length dimension of 1/12 and 1/8 the array width and length dimensions, respectively, i.e., between about 1 and 20 in width and 1 and 50 mm in length.

The solid support may include a water-impermeable backing such as a glass slide or rigid polymer sheet, or other non-porous material. Formed on the surface of the backing is a water-permeable film, which is formed of porous material. Such porous materials include, but are not limited to, nitrocellulose membrane nylon, polypropylene, and polyvinylidene difluoride (PVDF) polymer. The thickness of the film is preferably between about 10 and 1000 $\mu$m. The film may be applied to the backing by spraying or coating, or by applying a preformed membrane to the backing.

Alternatively, the solid support may be simply a filter composed of nitrocellulose, nylon, polypropylene, or polyvinylidene difluoride (PVDF) polymer, or, for that matter, any material suitable for use.

The film surface may be partitioned into a desirable array of cells by water-impermeable grid lines typically at a distance of about 100 to 2000 $\mu$m above the film surface. The grid lines can be formed on the surface of the film by laying down an uncured flowable resin or elastomer solution in an array grid, allowing the material to infiltrate the porous film down to the backing, and then curing the grid lines to form the cell-array substrate.

The barrier material of the grid lines may be a flowable silicone, wax-based material, thermoset material (*e.g.*, epoxy), or any other useful material. The grid lines may be applied to the solid support using a narrow syringe, printing techniques, heat-seal stamping, or any other useful method known in the art.

Each well preferably contains a microarray of distinct *Bacillus licheniformis* polynucleotides. "Distinct *Bacillus licheniformis* polynucleotides" as applied to the polynucleotides forming a microarray is defined herein as an array member that is distinct from other array members on the basis of a different *Bacillus licheniformis* polynucleotide sequence or oligo sequence thereof, and/or different concentrations of the same or distinct *Bacillus licheniformis* polynucleotides and/or different mixtures of distinct *Bacillus licheniformis* polynucleotides or different-concentrations of *Bacillus licheniformis* polynucleotides. Thus an array of "distinct *Bacillus licheniformis* polynucleotides" may be an array containing, as its members, (i) distinct *Bacillus licheniformis* genes that may have a defined amount in each member, (ii) different, graded concentrations of a specific *Bacillus licheniformis* polynucleotide, and/or (iii) different-composition mixtures of two or more distinct *Bacillus licheniformis* polynucleotides.

It will be understood, however, that in the methods of the present invention, any type of substrate known in the art may be used.

The delivery of a known amount of a selected *Bacillus licheniformis* polynucleotide to a specific position on the support surface is preferably performed with a dispensing device equipped with one or more (several) tips for insuring reproducible deposition and location of the *Bacillus licheniformis* polynucleotides and for preparing multiple arrays. Any dispensing device known in the art may be used in the methods of the present invention. See, for example, U.S. Patent No. 5,807,522.

For liquid-dispensing on a hydrophilic surface, the liquid will have less of a tendency to bead, and the dispensed volume will be more sensitive to the total dwell time of the dispenser tip in the immediate vicinity of the support surface.

For liquid-dispensing on a hydrophobic surface, flow of fluid from the tip onto the support surface will continue from the dispenser onto the support surface until it forms a liquid bead. At a given bead size, i.e., volume, the tendency of liquid to flow onto the surface will be balanced by the hydrophobic surface interaction of the bead with the support surface, which acts to limit the total bead area on the surface, and by the surface tension of the droplet, which tends toward a given bead curvature. At this point, a given bead volume will have formed, and continued contact of the dispenser tip with the bead, as the dispenser tip is being withdrawn, will have little or no effect on bead volume.

The desired deposition volume, i.e., bead volume, formed is preferably in the range 2 pl (picoliters) to 2 nl (nanoliters), although volumes as high as 100 nl or more may be dispensed. It will be appreciated that the selected dispensed volume will depend on (i) the "footprint" of the dispenser tip(s), i.e., the size of the area spanned by the tip(s), (ii) the hydrophobicity of the support surface, and (iii) the time of contact with and rate of withdrawal of the tip(s) from the support surface. In addition, bead size may be reduced by increasing the viscosity of the medium, effectively reducing the flow time of liquid from the dispensing device onto the support surface. The drop size may be further constrained by depositing the drop in a hydrophilic region surrounded by a hydrophobic grid pattern on the support surface.

At a given tip size, bead volume can be reduced in a controlled fashion by increasing surface hydrophobicity, reducing time of contact of the tip with the surface, increasing rate of movement of the tip away from the surface, and/or increasing the viscosity of the medium. Once these parameters are fixed, a selected deposition volume in the desired picoliter to nanoliter range can be achieved in a repeatable fashion.

After depositing a liquid droplet of a *Bacillus licheniformis* polynucleotide sample at one selected location on a support, the tip may be moved to a corresponding position on a second support, the *Bacillus licheniformis* polynucleotide sample is deposited at that position, and this process is repeated until the random nucleic acid fragment sample has been deposited at a selected position on a plurality of supports.

This deposition process may then be repeated with another random nucleic acid fragment sample at another microarray position on each of the supports.

The diameter of each *Bacillus licheniformis* polynucleotide region is preferably between about 20-200 $\mu$m. The spacing between each region and its closest (non-diagonal) neighbor, measured from center-to-center, is preferably in the range of about 20-400 $\mu$m. Thus, for example, an array having a center-to-center spacing of about 250 $\mu$m contains about 40 regions/cm or 1,600 regions/cm$^2$. After formation of the array, the support is treated to evaporate the liquid of the droplet forming each region, to leave a desired array of dried, relatively flat *Bacillus licheniformis* polynucleotide or oligo thereof regions. This drying may be done by heating or under vacuum. The DNA can also be UV-crosslinked to the polymer coating.

**Nucleic Acid Probes.** In the methods of the present invention, the strains are cultivated in a nutrient medium with and without a substrate using methods well known in the art for isolation of nucleic acids to be used as probes. For example, the strains may be cultivated by shake flask cultivation, small-scale or large-scale fermentation (including continuous, batch, fed-batch, or solid state fermentations) in laboratory or industrial fermentors performed in a suitable medium. The cultivation takes place in a suitable nutrient medium comprising carbon and nitrogen sources and inorganic salts, using procedures known in the art. Suitable media are available from commercial suppliers or may be prepared according to published compositions (*e.g.*, in catalogues of the American Type Culture Collection).

The nucleic acid probes from the microbial strains cultured on medium with and without substrate may be any nucleic acid including genomic DNA, cDNA, and RNA, and may be isolated using standard methods known in the art.

The populations of isolated nucleic acid probes may be labeled with detection reporters such as colorimetric, radioactive for example, $^{32}$P, $^{33}$P, or $^{35}$S), fluorescent reporters, or other reporters using methods known in the art (Chen et al., 1998, Genomics 51: 313-324; DeRisi et al., 1997, Science 278: 680-686; U.S. Patent No. 5,770,367).

In a preferred aspect, the probes are labeled with fluorescent reporters. For example, the DNA probes may be labeled during reverse transcription from the respective RNA pools by incorporation of fluorophores as dye-labeled nucleotides (DeRisi *et al.,* 1997, *supra), e.g.,* Cy5-labeled deoxyuridine triphosphate, or the isolated cDNAs may be directly labeled with different fluorescent functional groups. Fluorescent-labeled nucleotides include, but are not limited to, fluorescein conjugated nucleotide analogs (green fluorescence), lissamine nucleotide analogs (red fluorescence). Fluorescent functional groups include, but are not limited to, Cy3 (a green fluorescent dye) and Cy5 (red fluorescent dye).

**Array Hybridization.** The labeled nucleic acids from the two strains cultivated with and without substrate are then added to an array of *Bacillus licheniformis* polynucleotides under conditions where the nucleic acid pools from the two strains hybridize to complementary sequences of the *Bacillus licheniformis* polynucleotides on the array. For purposes of the present invention, hybridization indicates that the labeled nucleic acids from the two strains hybridize to the *Bacillus licheniformis* polynucleotides under very low to very high stringency conditions.

A small volume of the labeled nucleic acids mixture is loaded onto the substrate. The solution will spread to cover the entire microarray. In the case of a multi-cell substrate, one or more (several) solutions are loaded into each cell that stop at the barrier elements.

For nucleic acid probes of at least about 100 nucleotides in length, miroarray hybridization conditions described by Eisen and Brown , 1999, Methods of Enzymology 303: 179-205, may be used. Hybridization is conducted under a cover slip at 65°C in 3X SSC for 4-16 hours followed by post-hybridization at room temperature after removal of the cover slip in 2X SSC, 0.1% SDS by washing the array two or three times in the solution, followed by successive washes in 1X SSC for 2 minutes and 0.2X SSC wash for two or more minutes.

Conventional conditions of very low to very high stringency conditions may also be used. Very low to very high stringency conditions are defined as prehybridization and hybridization at 42°C in 5X SSPE, 0.3% SDS, 200 $\mu$g/ml sheared and denatured salmon sperm DNA, and either 25% formamide for very low and low stringencies, 35% formamide for medium and medium-high stringencies, or 50% formamide for high and very high stringencies, following standard Southern blotting procedures.

The carrier material is finally washed three times each for 15 minutes using 2 x SSC, 0.2% SDS preferably at least at 45°C (very low stringency), more preferably at least at 50°C (low stringency), more preferably at least at 55°C (medium stringency), more preferably at least at 60°C (medium-high stringency), even more preferably at least at 65°C (high stringency), and most preferably at least at 70°C (very high stringency).

For shorter nucleic acid probes that are less than 50 nucleotides, microarray hybridization conditions described by Kane et al., 2000, Nucleic Acids Research 28: 4552-4557, may be used. Hybridization is conducted under a supported coverslip at 42°C for 16-18 hours at high humidity in 50% formamide, 4.1X Denhardt's solution, 4.4X SSC, and 100 μg/ml of herring sperm DNA. Arrays are washed after removal of the coverslip in 4X SSC by immersion into 1X SSC, 0.1% SDS for 10 minutes, 0.1X SSC, 0.1% SDS twice for 10 minutes, and 0.1X SSC twice for 10 minutes.

For shorter nucleic acid probes that are about 50 nucleotides to about 100 nucleotides in length, conventional stringency conditions may be used. Such stringency conditions are defined as prehybridization, hybridization, and washing post-hybridization at 5°C to 10°C below the calculated $T_m$ using the calculation according to Bolton and McCarthy (1962, Proceedings of the National Academy of Sciences USA 48:1390) in 0.9 M NaCl, 0.09 M Tris-HCl pH 7.6, 6 mM EDTA, 0.5% NP-40, 1X Denhardt's solution, 1 mM sodium pyrophosphate, 1 mM sodium monobasic phosphate, 0.1 mM ATP, and 0.2 mg of yeast RNA per ml following standard Southern blotting procedures.

The carrier material is finally washed once in 6X SSC plus 0.1% SDS for 15 minutes and twice each for 15 minutes using 6X SSC at 5°C to 10°C below the calculated $T_m$.

The choice of hybridization conditions will depend on the degree of homology between the *Bacillus licheniformis* polynucleotides and the nucleic acid probes obtained from the strain cultured with and without inducing substrate. For example, where the nucleic acid probes and the *Bacillus licheniformis* polynucleotides are obtained from identical strains, high stringency conditions may be most suitable. Where the strains are from a genus or species different from which the *Bacillus licheniformis* polynucleotides were obtained, low or medium stringency conditions may be more suitable.

In a preferred aspect, the hybridization is conducted under low stringency conditions. In a more preferred aspect, the hybridization is conducted under medium stringency conditions. In a most preferred aspect, the hybridization is conducted under high stringency conditions.

The entire solid support is then reacted with detection reagents if needed and analyzed using standard colorimetric, radioactive, or fluorescent detection means. All processing and detection steps are performed simultaneously to all of the microarrays on the solid support ensuring uniform assay conditions for all of the microarrays on the solid support.

**Detection.** The most common detection method is laser-induced fluorescence detection using confocal optics (Cheung et al., 1998, Nat. Genet. 18: 225-230). The array is examined under fluorescence excitation conditions such that (i) the *Bacillus licheniformis* polynucleotides on the array that hybridize to the first nucleic acid probes obtained from the strain cultured without inducing substrate and to the second nucleic acid probes obtained from the strain cultured with inducing substrate produce a distinct first fluorescence emission color and a distinct second fluorescence emission color, respectively, and (ii) the *Bacillus licheniformis* polynucleotides on the array that hybridize to substantially equal numbers of nucleic acid probes obtained from the strain cultured without inducing substrate and from the strain cultured with inducing substrate produce a distinct combined fluorescence emission color; wherein the relative expression of the genes in the strains can be determined by the observed fluorescence emission color of each spot on the array.

The fluorescence excitation conditions are based on the selection of the fluorescence reporters. For example, Cy3 and Cy5 reporters are detected with solid state lasers operating at 532 nm and 632 nm, respectively.

However, other methods of detection well known in the art may be used such as standard photometric, colorimetric, or radioactive detection means, as described earlier.

**Data Analysis.** The data obtained from the scanned image may then be analyzed using any of the commercially available image analysis software. The software preferably identifies array elements, subtracts backgrounds, deconvolutes multi-color images, flags or removes artifacts, verifies that controls have performed properly, and normalizes the signals (Chen et al., 1997, Journal of Biomedical Optics 2: 364-374).

Several computational methods have been described for the analysis and interpretation of microarray-based expression profiles including cluster analysis (Eisen et al., 1998, Proc. Nat. Acad. Sci. USA 95: 14863-14868), parametric ordering of genes (Spellman et al., 1998, Mol. Biol. Cell 9: 3273-3297), and supervised clustering methods based on representative hand-picked or computer-generated expression profiles (Chu et al., 1998. Science 282: 699-705). Preferred methods for evaluating the results of the microarrays employ statistical analysis to determine the significance of the differences in expression levels. In the methods of the present invention, the difference in the detected expression level is at least about 10% or greater, preferably at least about 20% or greater, more preferably at least about 50% or greater, even more preferably at least about 75% or greater; and most preferably at least about 100% or greater.

One such preferred system is the Significance Analysis of Microarrays (SAM) (Tusher et al., 2001, Proc. Natl. Acad. Sci. USA 98: 5116-5121). Statistical analysis allows the determination of significantly altered expression of levels of about 50% or even less. The PAM (or predictive analysis for microarrays) represents another approach for analyzing the results of the microarrays (Tibshirani et al., 2002, Proc. Natl. Acad. Sci. USA 99: 6567-6572).

Cluster algorithms may also be used to analyze microarray expression data. From the analysis of the expression profiles it is possible to identify co-regulated genes that perform common metabolic or biosynthetic functions. Hierarchical clustering has been employed in the analysis of microarray expression data in order to place genes into clusters based on sharing similar patterns of expression (Eisen *et al.,* 1998, *supra).* This method yields a graphical display that resembles a kind of phylogenetic tree where the relatedness of the expression behavior of each gene to every other gene is depicted by branch lengths. The programs Cluster and TreeView, both written by Michael Eisen (Eisen et al., 1998 Proc. Nat. Acad. Sci. USA 95: 14863-14868) are freely available. Genespring is a commercial program available for such analysis (Silicon Genetics, Redwood City, CA).

Self-organizing maps (SOMs), a non-hierarchical method, have also been used to analyze microarray expression data (Tamayo et al., 1999, Proc. Natl. Acad. Sci. USA 96: 2907-2912). This method involves selecting a geometry of nodes, where the number of nodes defines the number of clusters. Then, the number of genes analyzed and the number of experimental conditions that were used to provide the expression values of these genes are subjected to an iterative process (20,000 - 50,000 iterations) that maps the nodes and data points into multidimensional gene expression space. After the identification of significantly regulated genes, the expression level of each gene is normalized across experiments. As a result, the expression profile of the genome is highlighted in a manner that is relatively independent of each gene's expression magnitude. Software for the "GENECLUSTER" SOM program for microarray expression analysis can be obtained from the Whitehead/MIT Center for Genome Research. SOMs can also be constructed using the GeneSpring software package.

**Isolation of Genes.** Probes containing genes or portions thereof identified to be induced by the present of substrate in the medium are characterized by determining the sequence of the probe. Based on the sequence, the gene can then be isolated using methods well known in the art.

The techniques used to isolate or clone a gene include isolation from genomic DNA, preparation from cDNA, or a combination thereof. The cloning of the gene from such genomic DNA can be effected, *e.g.*, by using the well known polymerase chain reaction (PCR) or antibody screening of expression libraries to detect cloned DNA fragments with shared structural features. See, *e.g.*, Innis et al., 1990, PCR: A Guide to Methods and Application, Academic Press, New York. Other nucleic acid amplification procedures such as ligase chain reaction (LCR), ligated activated transcription (LAT) and nucleic acid sequence-based amplification (NASBA) may be used. The gene may be cloned from the strain of interest, or another or related organism and thus, for example, may be a species variant of the gene.

**Methods for Monitoring Differential Expression of a Plurality of Genes**

The present invention also relates to methods for monitoring differential expression of a plurality of genes in a first bacterial cell relative to expression of the same genes in one or more (several) second bacterial cells, comprising:

(a) adding a mixture of detection reporter-labeled nucleic acids isolated from the bacterial cells to a substrate containing an array of *Bacillus licheniformis* polynucleotides selected from the group consisting of SEQ ID NOs: 2-4876, full-length complementary strands of SEQ ID NOs: 2-4876, and fragments of SEQ ID NOs: 2-4876, under conditions where the detection reporter-labeled nucleic acids hybridize to complementary sequences of the *Bacillus licheniformis* polynucleotides on the array, wherein the nucleic acids from the first bacterial cell and the one or more (several) second bacterial cells are labeled with a first detection reporter and one or more (several) different second detection reporters, respectively; and

(b) examining the array under conditions wherein the relative expression of the genes in the bacterial cells is determined by the observed detection signal of each spot on the array in which (i) the *Bacillus licheniformis* polynucleotides on the array that hybridize to the nucleic acids obtained from either the first or the one or more (several) second bacterial cells produce a distinct first detection signal or one or more (several) second detection signals, respectively, and (ii) the *Bacillus licheniformis* polynucleotides on the array that hybridize to the nucleic acids obtained from both the first and one or more (several) second bacterial produce a distinct combined detection signal.

The methods of the present invention may be used to monitor global expression of a plurality of genes from a *Bacillus* cell, discover new genes, identify possible functions of unknown open reading frames, and monitor gene copy number variation and stability. For example, the global view of changes in expression of genes may be used to provide a picture of the way in which *Bacillus* cells adapt to changes in culture conditions, environmental stress, or other physiological provocation. Other possibilities for monitoring global expression include spore morphogenesis, recombination, metabolic or catabolic pathway engineering.

The methods of the present invention are particularly advantageous since one spot on an array equals one gene or open reading frame because extensive follow-up characterization is unnecessary since sequence information is available, and the *Bacillus licheniformis* microarrays can be organized based on function of the gene products.

**Bacterial Cells.** In the methods of the present invention, the two or more *Bacillus* cells may be any *Bacillus* cell where one of the cells is used as a reference for identifying differences in expression of the same or similar complement of genes in the other cell(s). In one aspect, the two or more cells are the same cell. For example, they may be compared under different growth conditions, *e.g.*, oxygen limitation, nutrition, and/or physiology. In another aspect, one or more (several) cells are mutants of the reference cell. For example, the mutant(s) may have a different phenotype. In a further aspect, the two or more cells are of different species (*e.g.*, *Bacillus clausii* and *Bacillus subtilis*). In another further aspect, the two or more cells are of different genera. In an even further aspect, one or more (several) cells are transformants of the reference cell, wherein the one or more (several) transformants exhibit a different property. For example, the transformants may have an improved phenotype relative to the reference cell and/or one of the other transformants. The term "phenotype" is defined herein as an observable or outward characteristic of a cell determined by its genotype and modulated by its environment. Such improved phenotypes may include, but are not limited to, improved secretion or production of a protein or compound, reduced or no secretion or production of a protein or compound, improved or reduced expression of a gene, desirable morphology, an altered growth rate under desired conditions, relief of over-expression mediated growth inhibition, or tolerance to low oxygen conditions.

The *Bacillus* cells may be any *Bacillus* cells, but preferably *Bacillus alkalophilus, Bacillus amyloliquefaciens, Bacillus brevis, Bacillus cereus, Bacillus circulans, Bacillus clausii, Bacillus coagulans, Bacillus fastidiosus, Bacillus firmus, Bacillus lautus, Bacillus lentus, Bacillus licheniformis, Bacillus macerans, Bacillus megaterium, Bacillus methanolicus, Bacillus pumilus, Bacillus sphaericus, Bacillus stearothermophilus, Bacillus subtilis,* or *Bacillus thuringiensis* cells.

In a preferred aspect, the *Bacillus* cells are *Bacillus alkalophilus* cells. In another preferred aspect, the *Bacillus* cells are *Bacillus amyloliquefaciens* cells. In another preferred aspect, the *Bacillus* cells are *Bacillus brevis* cells. In another preferred aspect, the *Bacillus* cells are *Bacillus cereus* cells In another preferred aspect, the *Bacillus* cells are *Bacillus circulans* cells. In another preferred aspect, the *Bacillus* cells are *Bacillus clausii* cells. In another preferred aspect, the *Bacillus* cells are *Bacillus coagulans* cells. In another preferred aspect, the *Bacillus* cells are *Bacillus fastidiosus* cells. In another preferred aspect, the *Bacillus* cells are *Bacillus firmus* cells. In another preferred aspect, the *Bacillus* cells are *Bacillus lautus* cells. In another preferred aspect, the *Bacillus* cells are *Bacillus lentus* cells. In another preferred aspect, the *Bacillus* cells are *Bacillus licheniformis* cells. In another preferred aspect, the *Bacillus* cells are *Bacillus macerans* cells. In another preferred aspect, the *Bacillus* cells are *Bacillus megaterium* cells. In another preferred aspect, the *Bacillus* cells are *Bacillus methanolicus* cells. In another preferred aspect, the *Bacillus* cells are *Bacillus pumilus* cells. In n another preferred aspect, the *Bacillus* cells are *Bacillus sphaericus* cells. In another preferred aspect, the *Bacillus* cells are *Bacillus stearothermophilus* cells. In another preferred aspect, the *Bacillus* cells are *Bacillus subtilis* cells. In another preferred aspect, the *Bacillus* cells are *Bacillus thuringiensis* cells.

In a more preferred aspect, the *Bacillus* cells are *Bacillus licheniformis* cells. In a most preferred aspect, the *Bacillus licheniformis* cells are *Bacillus licheniformis* SJ 1904 cells.

In another more preferred aspect, the *Bacillus* cells are *Bacillus clausii* cells. In another most preferred aspect, the *Bacillus clausii* cells are *Bacillus clausii* NCIB 10309 cells.

It will be understood that the term *"Bacillus"* also encompasses relatives of *Bacillus* such as *Paenibacillus, Oceanobacillus,* and the like.

In the methods of the present invention, the cells are cultivated in a nutrient medium suitable for growth using methods well known in the art for isolation of the nucleic acids to be used as probes. For example, the cells may be cultivated by shake flask cultivation, small-scale or large-scale fermentation (including continuous, batch, fed-batch, or solid state fermentations) in laboratory or industrial fermentors performed in a suitable medium. The cultivation takes place in a suitable nutrient medium comprising carbon and nitrogen sources and inorganic salts, using procedures known in the art. Suitable media are available from commercial suppliers or may be prepared according to published compositions (*e.g.*, in catalogues of the American Type Culture Collection).

**Nucleic Acid Probes.** The nucleic acid probes from the two or more *Bacillus* cells may be any nucleic acid including genomic DNA, cDNA, and RNA, and may be isolated using standard methods known in the art, as described herein. The populations of isolated nucleic acid probes may be labeled with colorimetric, radioactive, fluorescent reporters, or other reporters using methods described herein.

In a preferred aspect, the probes are labeled with fluorescent reporters, *e.g.*, Cy3 (a green fluorescent dye) and Cy5 (red fluorescent dye), as described herein.

**Array Hybridization.** The labeled nucleic acids from the two or more *Bacillus* cells are then added to a substrate containing an array of *Bacillus licheniformis* polynucleotides under conditions, as described herein, where the nucleic acid pools from the two or more *Bacillus* cells hybridize to complementary sequences of the *Bacillus licheniformis* polynucleotides on the array.

**Detection and Data Analysis.** The same methods as described herein are used for detection and data analysis.

**Computer Readable Media and Computer-Based Systems**

The *Bacillus licheniformis* chromosome and its polynucleotides (genes) described herein may be "provided" in a variety of media to facilitate their use. The term "provided" refers to a manufacture comprising an array of *Bacillus licheniformis* polynucleotides. Such manufactures provide the *Bacillus licheniformis* polynucleotides in a form that allows one skilled in the art to examine the manufacture using means not directly applicable to examining the chromosome or a subset thereof as it exists in nature or in purified form.

Thus, the present invention also relates to such a manufacture in the form of a computer readable medium comprising an array of *Bacillus licheniformis* polynucleotides selected from the group consisting of SEQ ID NOs: 2-4876, full-length complementary strands of SEQ ID NOs: 2-4876, and fragments of SEQ ID NOs: 2-4876.

In one application of this aspect, the *Bacillus licheniformis* polynucleotides of the present invention can be recorded on computer readable media. The term "computer readable media" is defined herein as any medium that can be read and accessed by a computer. Such computer readable media include, but are not limited to, magnetic storage media, *e.g.*, floppy discs, hard disc storage medium, and magnetic tape; optical storage media, *e.g.*, CD-ROM, DVD; electrical storage media, *e.g.*, RAM and ROM; and hybrids of these categories, *e.g.*, magnetic/optical storage media. One skilled in the art can readily appreciate how any of the presently known computer readable media can be used to create a manufacture comprising computer readable medium having recorded thereon a nucleotide sequence of the present invention. Likewise, it will be clear to those of skill how additional computer readable media that may be developed also can be used to create analogous manufactures having recorded thereon a nucleotide sequence of the present invention.

As used herein, "recorded" refers to a process for storing information on computer readable medium. One skilled in the art can readily adopt any of the presently known methods for recording information on computer readable medium to generate manufactures comprising the nucleotide sequence information of the present invention.

A variety of data storage structures are available for creating a computer readable medium having recorded thereon a nucleotide sequence of the present invention. The choice of the data storage structure will generally be based on the means chosen to access the stored information. In addition, a variety of data processor programs and formats can be used to store the nucleotide sequence information of the present invention on computer readable medium. The sequence information can be represented in a word processing text file, formatted in commercially-available software such as WordPerfect and Microsoft Word, or represented in the form of an ASCII file, stored in a database application, such as DB2, Sybase, Oracle, or the like. A skilled artisan can readily adapt any number of data-processor structuring formats (*e.g.*, text file or database) in order to obtain computer readable medium having recorded thereon the nucleotide sequence information of the present invention.

Various computer software programs are publicly available that allow a skilled artisan to access sequence information provided in a computer readable medium. Thus, by providing in computer readable form an array of *Bacillus licheniformis* polynucleotides selected from the group consisting of SEQ ID NOs: 2-4876, full-length complementary strands of SEQ ID NOs: 2-4876, and fragments of SEQ ID NOs: 2-4876, enables one skilled in the art to routinely access the provided sequence information for a wide variety of purposes.

Software utilizing the BLAST (Altschul *et al.,* 1990, *supra),* BLAZE (Brutlag et al., 1993, Comp. Chem. 17: 203-207), GENEMARK (Lukashin and Borodovsky, 1998, Nucleic Acids Research 26: 1107-1115), GENSCAN (Burge and Karlin, 1997, Journal of Molecular Biology 268: 78-94), GLIMMER (Salzberg et al., 1998, Nucleic Acids Research 26: 544-548), and GRAIL (Xu et al., 1994, Comput. Appl. Biosci. 10: 613-623) search algorithms may be used to identify open reading frames (ORFs) within a genome of interest, which contain homology to ORFs or proteins from both *Bacillus licheniformis* and *Bacillus clausii* and from other organisms. Among the ORFs discussed herein are protein encoding fragments of the *Bacillus licheniformis* and *Bacillus clausii* genomes useful in producing commercially important proteins, such as enzymes used in fermentation reactions and in the production of commercially useful metabolites.

The present invention further provides systems, particularly computer-based systems, which contain the sequence information described herein. Such systems are designed to identify, among other things, genes and gene products - many of which could be products themselves or used to genetically modify an industrial expression host through increased or decreased expression of a specific gene sequence(s).

The term "a computer-based system" is herein defined as the hardware means, software means, and data storage means used to analyze the nucleotide sequence information of the present invention. The minimum hardware means of the computer-based systems of the present invention comprises a central processing unit (CPU), input means, output means, and data storage means. One skilled in the art can readily appreciate that any currently available computer-based system is suitable for use in the present invention.

As stated above, the computer-based systems of the present invention comprise a data storage means having stored therein a nucleotide sequence of the present invention and the necessary hardware means and software means for supporting and implementing a search means.

The term "data storage means" is defined herein as memory that can store nucleotide sequence information of the present invention, or a memory access means that can access manufactures having recorded thereon the nucleotide sequence information of the present invention.

The term "search means" refers is defined herein as one or more (several) programs that are implemented on the computer-based system to compare a target sequence or target structural motif with the sequence information stored within the data storage means. Search means are used to identify fragments or regions of the present genomic sequences that match a particular target sequence or target motif. A variety of known algorithms are disclosed publicly and a variety of commercially available software for conducting search means are and can be used in the computer-based systems of the present invention. Examples of such software includes, but is not limited to, MacPattern (Fuchs, 1991, Comput. Appl. Biosci. 7: 105-106), BLASTN and BLASTX National Center for Biotechnology Information (NCBI). One skilled in the art can readily recognize that any one of the available algorithms or implementing software packages for conducting homology searches can be adapted for use in the present computer-based systems.

The term "target sequence" is defined here as any DNA (genomic DNA, cDNA) or amino acid sequence of six or more nucleotides or two or more amino acids. One skilled in the art can readily recognize that the longer a target sequence is, the less likely a target sequence will be present as a random occurrence in the database. The most preferred sequence length of a target sequence is from about 10 to 100 amino acids or from about 30 to 300 nucleotide residues. However, it is well recognized that searches for commercially important fragments, such as sequence fragments involved in gene expression and protein processing, may be of shorter length.

The term "a target structural motif" or "target motif" is defined herein as any rationally selected sequence or combination of sequences in which the sequence(s) are chosen based on a three-dimensional configuration that is formed upon the folding of the target motif. There are a variety of target motifs known in the art. Protein target motifs include, but are not limited to, enzyme active sites and signal sequences, substrate and cofactor binding domains, transmembrane domains, and sites for post-translational modifications. Nucleic acid target motifs include, but are not limited to, promoter sequences, hairpin structures and inducible expression elements (protein binding sequences), repeats, palindromes, dyad symmetries, and transcription and translation start and stop sites.

A variety of structural formats for the input and output means can be used to input and output the information in the computer-based systems of the present invention. A preferred format for an output means ranks fragments of the *Bacillus licheniformis* or *Bacillus clausii* genomic sequences possessing varying degrees of homology to the target sequence or target motif. Such presentation provides one skilled in the art with a ranking of sequences that contain various amounts of the target sequence or target motif and identifies the degree of homology contained in the identified fragment.

A variety of comparing means can be used to compare a target sequence or target motif with the data storage means to identify sequence fragments of the *Bacillus licheniformis* and *Bacillus clausii* genomes. For example, implementing software that utilize the BLAST and BLAZE algorithms, described in Altschul *et al.,* 1990, *supra,* may be used to identify open reading frames within the *Bacillus licheniformis* or *Bacillus clausii* genome or the genomes of other organisms. A skilled artisan can readily recognize that any one of the publicly available homology search programs can be used as the search means for the computer-based systems of the present invention. Suitable proprietary systems that may be known to those of skill also may be employed in this regard.

**Codon Usage Table**

The present invention further relates to methods for preparing a synthetic gene, comprising (a) generating a codon usage table based on codons used in one or more (several) open reading frames or portions thereof of SEQ ID NO: 1, (b) constructing a synthetic gene or portion thereof that contains in place of one or more (several) native codons one or more (several) preferred codons from the codon usage table, and (c) recovering the synthetic gene. In a preferred aspect, the codon usage table is Table 5.

The *Bacillus licheniformis* chromosomal sequence of SEQ ID NO: 1 or portions thereof can be used to generate codon usage tables to design synthetic genes for their efficient heterologous expression in *Bacillus licheniformis* host cells. The codon usage tables can be based on (1) the codon used in all the open reading frames, (2) selected open reading frames, (3) fragments of the open reading frames, or (4) fragments of selected open reading frames. With a codon usage table, synthetic genes can be designed with only the most preferred codon for each amino acid; with a number of common codons for each amino acid; or with the same or a similar statistical average of codon usages found in the table of choice.

The synthetic gene can be constructed using any method such as site-directed mutagenesis or PCR generated mutagenesis in accordance with methods known in the art. Although, in principle, the modification may be performed *in vivo, i.e.,* directly on the cell expressing the nucleotide sequence to be modified, it is preferred that the modification is performed *in vitro.*

The synthetic gene can be further modified by operably linking the synthetic gene to one or more (several) control sequences that direct the expression of the coding sequence in a suitable host cell under conditions compatible with the control sequences using the methods described herein. Nucleic acid constructs, recombinant expression vectors, and recombinant host cells comprising the synthetic gene can also be prepared using the methods described herein.

The present invention also relates to methods for producing a polypeptide encoded by such a synthetic gene comprising (a) cultivating a host cell comprising the synthetic gene under conditions conducive for production of the polypeptide; and (b) recovering the polypeptide.

The present invention is further described by the following examples that should not be construed as limiting the scope of the invention.

## Examples

### Example 1: DNA sequencing and genome assembly

The genome of *Bacillus licheniformis* SJ1904 was sequenced by a combination of the whole genome shotgun method described by Wilson, R.K. and Mardis, E.R., 1997, In Genome Analysis: A Laboratory Manual, Vol. 1, eds. Birren, B., Green, E.D., Meyers, R.M., and Roskams, J. (Cold Spring Harbor Press, Cold Spring Harbor, NY), pp. 397-454, and by the highly parallel pyrosequencing method described by Margulies et al., 2005, Genome sequencing in microfabricated high-density picolitre reactors. Nature 437: 376-380.

Genomic DNA of *Bacillus licheniformis* SJ1904 was isolated using the following method: A single colony was used to inoculate 20 ml of LB broth (Davis, R.W., Botstein, D., and Roth, J.R. 1980, Advanced Bacterial Genetics, Cold Spring Harbor Press, Cold Spring Harbor, NY) in a sterile 125 ml Erlenmeyer flask. The culture was incubated at 37°C overnight with agitation at 240 rpm. The resulting cells were collected by centrifugation in a 45 ml screw-cap tube for 10 minutes at 6000 x g, and the cell pellet was resuspended in 5 ml of Tris-glucose buffer (50 mM Tris-HCl, pH 8.0, 50 mM glucose, 10 mM EDTA). Lysozyme was added to a final concentration of 50 $\mu$g/ml and the suspension was incubated in a 37°C water bath for 25 minutes. Next, 200 $\mu$l of 10% SDS was added and the tube was gently inverted several times. Five milliliters of a second detergent mixture (1% BRIJ®, 1% deoxycholate, 50 mM EDTA, pH 7.5) was added, and the tube was inverted several times while incubating for 20 minutes at room temperature. An equal volume of phenol: chloroform (1:1 v/v) was added and the tube was inverted gently at room temperature for 20-30 minutes. The tube was centrifuged for 20 minutes at 12,000 x g, 4°C. The top aqueous layer was carefully removed with a wide-bore pipette and placed in a clean 45 ml screw-cap tube. The phenol;chloroform extraction was repeated and 1/10 volume of 3 M sodium acetate pH 5.2 was added to the aqueous layer. Two volumes of cold ethanol were carefully layered on top and the DNA was spooled from the solution onto a sterile glass rod. Spooled DNA was carefully rinsed in 70% ethanol and resuspended in a suitable amount of TE buffer (10 mM Tris-HCl, pH 8.0, 1 mM EDTA).

Plasmid libraries were constructed using randomly-sheared and *Nhe*I-digested genomic DNA that was enriched for 2-3 kb fragments by preparative agarose gel electrophoresis (Berka, R.M., Schneider, P., Golightly, E.J., Brown, S.H., Madden, M., Brown, K.M., Halkier, T., Mondorf, K., and Xu, F., 1997, Appl. Environ. Microbiol. 63: 3151-3157). Approximately 24,000 random clones were sequenced using dye-terminator chemistry (Applied Biosystems, Foster City, CA) with ABI 377, ABI 3700, and ABI 3130XL automated sequencers yielding approximately 2X coverage of the genome. For highly parallel pyrosequencing pools of random DNA fragments were generated by shearing genomic DNA and isolating single DNA molecules by limiting dilution (Margulies *et al.,* 2005, *supra).* Overall sequencing coverage from this method was approximately 20X. High quality contigs were assembled from the raw pyrosequencing data using computer software described by Margulies *et al.* (2005).

A combination of methods was employed for gap closure including primer walking on selected clones, and PCR-amplified DNA fragments. Sequences from dye-terminator reactions were base-called using TraceTuner 2.0 (Paracel, Inc., Pasadena, CA) and assembled using Phrap (Gordon D., Abajian C., and Green P., 1998, Genome Res. 8: 195-202). Phrap, Crossmatch, and Consed were used for sequence finishing (Gordon D., Abajian C., and Green P., 1998, Genome Res. 8: 195-202).

### Example 2: Identification and annotation of open reading frames (ORFs)

Protein coding regions in the assembled genome sequence data were identified using Glimmer version 3.0 (Delcher, A,L., Harmon, D., Kasif, S., White, O. and Salzberg, S.L., 1999, Nucleic Acids Res. 27, 4636-4641), and post-processed using TiCO version 2.0 (Tech M, Morgenstern B, and Meinicke P., 2006, Nucleic Acids Res. 34 (Web Server issue): W588-90). Predicted proteins were compared to the non-redundant database Uniref100 (Bairoch A, Apweiler R, Wu CH, Barker WC, Boeckmann B, Ferro S, Gasteiger E, Huang H, Lopez R, Magrane M, Martin MJ, Natale DA, O'Donovan C, Redaschi N, Yeh LS. 2005. The Universal Protein Resource [UniProt]. Nucleic Acids Res. 33: D154-159) and the *Bacillus subtilis* genome (SubtilList) using BLASTP with an E-value threshold of 1x10$^{-5}$. InterProScan version 3.3 with database release 13.0 was used to predict function (Zdobnov, E.M. and Apweiler, R., 2001, Bioinformatics 17, 847-848). The InterPro analysis included comparison to Pfam (Bateman, A., Coin, L., Durbin, R., Finn, R.D., Hollich, V., Griffiths-Jones, S., Khanna, A., Sonnhammer, E.L. et al., 2004, Nucleic Acids Res. 32, D138-D141), TIGRfam (Haft, D.J., Selengut, J.D. and White, O., 2003, Nucleic Acids Res. 31: 371-373), Interpro (Apweiler, R., Attwood, T.K., Bairock, A., Bateman, A., Birney, E., Biswas, M., Bucher, P., Cerutti, L., Corpet, F., Croning, M.D., et al., 2001, Nucleic Acids Res. 29: 37-40), signal peptide prediction using SignalP version 3.0 (Nielsen, H., Engelbrecht, J., Brunak, S., and von Heijne, G., 1997, Protein Engineering 10: 1-6), and trans-membrane domain prediction using TMHMM version 2.0 (Krogh, A., Larsson, B., von Heijne, G. and Sonnhammer, E.L.L., 2000, J. Mol. Biol. 305, 567-580). These coding sequences (CDSs) were assigned to functional categories based on the Cluster of Orthologous Groups (COG) database (Tatusov, R.L., Natale, D.A., Garkavtsev, I.V., Tatusova, T.A., Shankavarum, U.T., Rao, B.S., Kiryutin, B., Galperin, M.Y., Federova, N.D., and Koonin, E.V. 2001. The COG database: new developments in phylogenetic classification of proteins from complete genomes. Nucleic Acids Res. 29: 22-28) with manual verification as described (Tatusov, R.L., Koonin, E.V. and Lipman, D.J., 1997, Science 278: 631-637; Koonin, E.V. and Galperin, M.Y., 2002, Sequence - Evolution - Function: Computational Approaches in Comparative Genomics (Kluwer, Boston)). Transfer RNA genes were identified using tRNAscan-SE version 1.21 (Lowe, T.M. and Eddy, S.R., 1997, Nucleic Acids Res. 25: 955-964).

### Example 3: General features of the *Bacillus licheniformis* SJ1904 genome

The genome of *Bacillus licheniformis* SJ1904 was determined to consist of a circular molecule of 4,345,159 bp with an average %G+C content of 46.7% (Table 2). No plasmids were found during the genome analysis, and none were found by agarose gel electrophoresis.

Using a combination of several gene-finding algorithms, 4875 protein coding CDSs and pseudogenes with an average size of 789 bp were identified. Among the predicted CDSs, 4225 (86%) have significant similarity ($E \leq 1.0E-05$) to proteins in Uniref100 (UniProt). The number of hypothetical and conserved hypothetical proteins in the *Bacillus licheniformis* SJ1904 genome with hits in the Uniref100 database was 1532. There are 650 CDSs that are unique to the genome of *Bacillus licheniformis* SJ1904 (no homologues found in the Uniref100 database). There are 72 tRNA genes representing all 20 amino acids and 7 rRNA operons.

The likely origin of replication was identified by homology to the corresponding region in the *Bacillus licheniformis* ATCC 14580 origin (Rey, M.W, Ramaiya, P, Nelson, B.A., Brody-Karpin, S.D., Zaretsky, E.J., Tang, M., Lopez de Leon, A., Xiang, H., Gusti, V., Clausen, I.G., Olsen, P.B., Rasmussen, M.D., Andersen, J.T., Jørgensen, P.L., Larsen, T.S., Sorokin, A., Bolotin, A., Lapidus, A., Galleron, N., Ehrlich, S.D., and Berka, R.M., 2004, Complete genome sequence of the industrial bacterium Bacillus licheniformis and comparisons with closely related Bacillus species. Genome Biol. 5: R77).

Extracellular proteins: In the *Bacillus licheniformis* genome, 625 of the 4875 gene models have signal peptides as forecasted by SignalP version 3.0 (Identification of prokaryotic and eukaryotic signal peptides and prediction of their cleavage sites. Henrik Nielsen, Jacob Engelbrecht, Søren Brunak and Gunnar von Heijne, 1997, Protein Engineering 10: 1-6) and/or PSORTb version 2.0 (J.L. Gardy, M.R. Laird, F. Chen, S. Rey, C.J. Walsh, M. Ester, and F.S.L. Brinkman (2005) PSORTb v.2.0: expanded prediction of bacterial protein subcellular localization and insights gained from comparative proteome analysis, Bioinformatics 21: 617-623). Of these, 304 either have no trans-membrane domain as predicted with TMHMM version 2.0 (A. Krogh, B. Larsson, G. von Heijne, and E.L.L. Sonnhammer, 2000, Journal of Molecular Biology 305: 567-580) or a possible transmembrane domain that overlaps a predicted signal peptide. Based on manual examination, 204 were determined to encode probable secreted proteins and enzymes. Of these 78 (38%) are hypothetical or conserved hypothetical proteins. The sequence ID numbers for each of these genes encoding likely extracellular proteins are listed in Table 4.

Genes found in *Bacillus licheniformis* SJ1904 that are not predicted in *Bacillus licheniformis* ATCC 14580: There are 673 gene models in the *Bacillus licheniformis* SJ1904 chromosome that were not predicted in the *Bacillus licheniformis* ATCC 14580 chromosome data (GENBANK® accession number CP000002). The vast majority of the *Bacillus licheniformis* SJ1904 specific gene models encode hypothetical proteins of unknown or unassigned function (see Table 3). Among those gene models that share similarity to protein sequences in the UniRef100 database, most encode functions that can be ascribed to bacteriophages, transposons, and other mobile genetic elements.

### Example 4: Codon usage table

The evolution of codon bias, the unequal usage of synonomous codons, is thought to be due to natural selection for the use of preferred codons that match the most abundant species of isoaccepting tRNAs, resulting in increased translational efficiency and accuracy. The practical applications for utilizing codon bias information include optimizing expression of heterologous and mutant genes (Jiang and Mannervik, 1999, Protein Expression and Purification 15: 92-98), site-directed mutagenesis to derive variant polypeptides from a given gene (Wong et al., 1995, J. Immunol. 154: 3351-3358; Kaji, H. et al., 1999, J. Biochem. 126: 769-775), design and synthesis of synthetic genes (Libertini and Di Donato, 1992, Protein Engineering 5: 821-825; Feng et al., 2000, Biochem. 39: 15399-15409), and fine-tuning or reducing of translation efficiency of specific genes by introduction of non-preferred codons (Crombie et al., 1992, J. Mol. Biol. 228: 7-12; Carlini and Stephan, 2003, Genetics 163: 239-243).

A codon usage table (Table 5) was generated from SEQ ID NO: 1 with Artemis, a software package created by the Wellcome Trust Sanger Institute (K. Rutherford, J. Parkhill, J. Crook, T. Horsnell, P. Rice, M-A. Rajandream and B. Barrell. 2000. Artemis: sequence visualisation and annotation. Bioinformatics 16: 944-945) on all the predicted protein-coding genes of the *Bacillus licheniformis* SJ1904 chromosome. Artemis read the coding sequences and calculated the codon frequency table shown in Table 5. The codon usage data presented in Table 5 comprises the collective frequencies of each codon among the 4875 protein-coding gene models. Some noteworthy observations include (a) CTA and AGT codons are used infrequently, and (b) in several instances where two codon alternatives exist (*e.g.*, Asp, Glu, Phe and Lys), codons with an A or T in the wobble position are preferred.

**Table 1. Predicted functions**

| SEQID | Gene Name & Product | Uniref100 Hit Description | Uniref100 Accession | Hit Organism |
|---|---|---|---|---|
| 2 | dnaA DnaA | Hypothetical protein dnaA [Bacillus licheniformis DSM 13] | UniRef100_Q65PM2 | Bacillus licheniformis DSM 13 |
| 3 | dnaN DNA polymerase III (beta subunit) | DnaN [Bacillus licheniformis DSM 13] | UniRef100_Q65PM1 | Bacillus licheniformis DSM 13 |
| 4 | yaaA conserved protein YaaA | YaaA [Bacillus licheniformis DSM 13] | UniRef100_Q65PM0 | Bacillus licheniformis DSM 13 |
| 5 | recF DNA repair RecF | Hypothetical protein recF [Bacillus licheniformis DSM 13] | UniRef100_Q65PL9 | Bacillus licheniformis DSM 13 |
| 6 | yaaB YaaB | Hypothetical protein yaaB [Bacillus licheniformis DSM 13] | UniRef100_Q65PL8 | Bacillus licheniformis DSM 13 |
| 7 | gyrB DNA gyrase (subunit B) | DNA gyrase [Bacillus licheniformis DSM 13] | UniRef100_Q65PL7 | Bacillus licheniformis DSM 13 |
| 8 | gyrA DNA gyrase (subunit A) | DNA gyrase subunit A [Bacillus licheniformis] | UniRef100_Q65PL6 | Bacillus licheniformis |
| 9 | yaaC conserved protein YaaC | YaaC [Bacillus licheniformis DSM 13] | UniRef100_Q65PL5 | Bacillus licheniformis DSM 13 |
| 10 | guaB inosine-monophosphate dehydrogenase | GuaB [Bacillus licheniformis DSM 13] | UniRef100_Q65PL4 | Bacillus licheniformis DSM 13 |
| 11 | dacA D-alanyl-D-alanine carboxypeptidase (penicillin-binding protein 5) | DacA [Bacillus licheniformis DSM 13] | UniRef100_Q65PL3 | Bacillus licheniformis DSM 13 |
| 12 | pdx1 Vitamin B6 biosynthesis protein | YaaD [Bacillus licheniformis DSM 13] | UniRef100_Q65PL2 | Bacillus licheniformis DSM 13 |
| 13 | pdxT SNO glutamine amidotransferase | YaaE [Bacillus licheniformis DSM 13] | UniRef100_Q65PL1 | Bacillus licheniformis DSM 13 |
| 14 | serS seryl-tRNA synthetase | SerS [Bacillus licheniformis DSM 13] | UniRef100_Q65PL0 | Bacillus licheniformis DSM 13 |
| 15 | glxK Glycerate kinase GlxK | Glycerate kinase [Bacillus licheniformis DSM 13] | UniRef100_Q630A8 | Bacillus licheniformis DSM 13 |
| 16 | BLP0015 Putative gluconate transporter | YojA [Bacillus licheniformis DSM 13] | UniRef100_Q65PK9 | Bacillus licheniformis DSM 13 |
| 17 | BLP00016 Putative sugar diacid recognition protein | YsfB [Bacillus licheniformis DSM 13] | UniRef100_Q65PK8 | Bacillus licheniformis DSM 13 |
| 18 | BLP00017 conserved Hypothetical Heavy metal transport_detoxification protein | Hypothetical protein [Bacillus licheniformis DSM 13] | UniRef100_Q65PK7 | Bacillus licheniformis DSM 13 |
| 19 | dck deoxyadenosine_deoxycytidine kinase | Dck [Bacillus licheniformis DSM 13] | UniRef100_Q65PK6 | Bacillus licheniformis DSM 13 |
| 20 | dgk deoxyguanosine kinase | Dgk [Bacillus licheniformis DSM 13] | UniRef100_Q65PK5 | Bacillus licheniformis DSM 13 |
| 21 | yaaH Glycoside hydrolase, family 18, YaaH | YdhD [Bacillus licheniformis DSM 13] | UniRef100_Q65PK4 | Bacillus licheniformis DSM 13 |
| 22 | yaaI putative Isochorismatase hydrolase YaaI | YaaI [Bacillus licheniformis DSM 13] | UniRef100_Q65PK3 | Bacillus licheniformis DSM 13 |
| 23 | yaaJ putative Cytidine_deoxycytidylate deaminase, zinc-binding region YaaJ | YaaJ [Bacillus licheniformis DSM 13] | UniRef100_Q65PK2 | Bacillus licheniformis DSM 13 |
| 24 | dnaX DNA polymerase III (gamma and tau subunits) | DnaX [Bacillus licheniformis DSM 13] | UniRef100_Q65PK1 | Bacillus licheniformis DSM 13 |
| 25 | yaaK conserved hypothetical YaaK | | | |
| 26 | recR DNA repair protein RecR | Hypothetical protein recR [Bacillus licheniformis DSM 13] | UniRef100_Q65PJ9 | Bacillus licheniformis DSM 13 |
| 27 | yaaL conserved hypothetical protein YaaL | Hypothetical protein yaaL [Bacillus licheniformis DSM 13] | UniRef100_Q65PJ8 | Bacillus licheniformis DSM 13 |
| 28 | bofA BofA | Hypothetical protein bofA [Bacillus licheniformis DSM 13] | UniRef100_Q65PJ7 | Bacillus licheniformis DSM 13 |
| 29 | csfB CsfB | Hypothetical protein csfB [Bacillus licheniformis DSM 13] | UniRef100_Q62ZY6 | Bacillus licheniformis DSM 13 |
| 30 | xpaC XpaC | Hypothetical protein xpaC [Bacillus licheniformis DSM 13] | UniRef100_Q65PJ6 | Bacillus licheniformis DSM 13 |
| 31 | yaaN putative Toxic anion resistance protein YaaN | YaaN [Bacillus licheniformis DSM 13] | UniRef100_Q65PJ5 | Bacillus licheniformis DSM 13 |

| | | | | |
|---|---|---|---|---|
| 32 | yaaO putative Orn_Lys_Arg decarboxylase, C-terminal YaaO | YaaO [Bacillus licheniformis DSM 13] | UniRef100_Q65PJ4 | Bacillus licheniformis DSM 13 |
| 33 | tmk thymidylate kinase | Tmk [Bacillus licheniformis DSM 13] | UniRef100_Q65PJ3 | Bacillus licheniformis DSM 13 |
| 34 | yaaQ YaaQ | Hypothetical protein yaaQ [Bacillus licheniformis DSM 13] | UniRef100_Q65PJ2 | Bacillus licheniformis DSM 13 |
| 35 | yaaR conserved hypothetical protein YaaR | YaaR [Bacillus licheniformis DSM 13] | UniRef100_Q65PJ1 | Bacillus licheniformis DSM 13 |
| 36 | holB DNA polymerase III (delta' subunit) | HolB [Bacillus licheniformis DSM 13] | UniRef100_Q65PJ0 | Bacillus licheniformis DSM 13 |
| 37 | yaaT putative signal peptidase II YaaT | YaaT [Bacillus licheniformis DSM 13] | UniRef100_Q65PI9 | Bacillus licheniformis DSM 13 |
| 38 | yabA DnaA and DnaN interacting protein containing domain DUF972- YabA | Hypothetical protein yabA [Bacillus licheniformis DSM 13] | UniRef100_Q65PI8 | Bacillus licheniformis DSM 13 |
| 39 | yabB conserved hypothetical protein containing SAM (and some other nucleotide) binding motif YabB | YabB (SAM (And some other nucleotide) binding motif) [Bacillus licheniformis DSM 13] | UniRef100_Q65PI7 | Bacillus licheniformis DSM 13 |
| 40 | yazA putative Excinuclease ABC, C subunit, N-terminal YazA | YazA [Bacillus licheniformis DSM 13] | UniRef100_Q65PI6 | Bacillus licheniformis DSM 13 |
| 41 | yabC putative methyltransferase YabC containing domain UPF0011 | YabC [Bacillus licheniformis DSM 13] | UniRef100_Q65PI5 | Bacillus licheniformis DSM 13 |
| 42 | abrB transcriptional regulator AbrB | AbrB [Bacillus licheniformis DSM 13] | UniRef100_Q65PI4 | Bacillus licheniformis DSM 13 |
| 43 | metS methionyl-tRNA synthetase MetS | MetS [Bacillus licheniformis DSM 13] | UniRef100_Q65PI3 | Bacillus licheniformis DSM 13 |
| 44 | yabD putative TatD-related deoxyribonuclease YabD | YabD [Bacillus licheniformis DSM 13] | UniRef100_Q65PI2 | Bacillus licheniformis DSM 13 |
| 45 | yabE conserved hypothetical containing domain DUF348 YabE | YabE [Bacillus licheniformis DSM 13] | UniRef100_Q65PI1 | Bacillus licheniformis DSM 13 |
| 46 | rnmV ribonuclease M5 | RnmV [Bacillus licheniformis DSM 13] | UniRef100_Q65PI0 | Bacillus licheniformis DSM 13 |
| 47 | ksgA dimethyladenosine transferase | KsgA [Bacillus licheniformis DSM 13] | UniRef100_Q65PH9 | Bacillus licheniformis DSM 13 |
| 48 | yabG spore coat assembly Peptidase U57, YabG | Peptidase U57, YabG [Bacillus licheniformis DSM 13] | UniRef100_Q65PH8 | Bacillus licheniformis DSM 13 |
| 49 | veg Veg | Hypothetical protein veg [Bacillus licheniformis DSM 13] | UniRef100_Q65PH7 | Bacillus licheniformis DSM 13 |
| 50 | BLP04682 hypothetical protein | | | |
| 51 | sspF small acid-soluble spore protein (alpha_beta-type SASP) | SspF [Bacillus licheniformis DSM 13] | UniRef100_Q65PH6 | Bacillus licheniformis DSM 13 |
| 52 | ispE 4-diphosphocytidyl-2-C-methyl-D-erythritol kinase | 4-diphosphocytidyl-2-C-methyl-D-erythritol kinase [Bacillus licheniformis DSM 13] | UniRef100_Q62ZW4 | Bacillus licheniformis DSM 13 |
| 53 | purR transcriptional regulator | PurR [Bacillus licheniformis DSM 13] | UniRef100_Q65PH4 | Bacillus licheniformis DSM 13 |
| 54 | yabJ putative regulator of purine operon, putative translation initiation inhibitor | YabJ [Bacillus licheniformis DSM 13] | UniRef100_Q65PH3 | Bacillus licheniformis DSM 13 |
| 55 | BLP00052 hypothetical protein | | | |
| 56 | spoVG Stage V sporulation protein G | Hypothetical protein spoVG [Bacillus licheniformis DSM 13] | UniRef100_Q65PH2 | Bacillus licheniformis DSM 13 |
| 57 | gcaD UDP-N-acetylglucosamine pyrophosphorylase | GcaD [Bacillus licheniformis DSM 13] | UniRef100_Q65PH1 | Bacillus licheniformis DSM 13 |
| 58 | prs phosphoribosylpyrophosphate synthetase | Prs [Bacillus licheniformis DSM 13] | UniRef100_Q65PH0 | Bacillus licheniformis DSM 13 |
| 59 | ctc general stress protein | Ctc [Bacillus licheniformis DSM 13] | UniRef100_Q65PG9 | Bacillus licheniformis DSM 13 |
| 60 | spoVC peptidyl-tRNA hydrolase | SpoVC [Bacillus licheniformis DSM 13] | UniRef100_Q65PG8 | Bacillus licheniformis DSM 13 |
| 61 | yabK conserved hypothetical YabK | Hypothetical protein yabK [Bacillus licheniformis DSM 13] | UniRef100_Q65PG7 | Bacillus licheniformis DSM 13 |
| 62 | mfd transcription-repair coupling factor | Mfd [Bacillus licheniformis DSM 13] | UniRef100_Q65PG6 | Bacillus licheniformis DSM 13 |
| 63 | spoVT transcriptional regulator | SpoVT [Bacillus licheniformis DSM 13] | UniRef100_Q65PG5 | Bacillus licheniformis DSM 13 |

| 64 | yabM putative Polysaccharide biosynthesis protein YabM | YabM [Bacillus licheniformis DSM 13] | UniRef100_Q65PG4 | Bacillus licheniformis DSM 13 |
|---|---|---|---|---|
| 65 | yabN putative phosphatase and methylase | YabN [Bacillus licheniformis DSM 13] | UniRef100_Q65PG3 | Bacillus licheniformis DSM 13 |
| 66 | yabO putative RNA-binding S4 protein YabO | YabO [Bacillus licheniformis DSM 13] | UniRef100_Q65PG2 | Bacillus licheniformis DSM 13 |
| 67 | yabP conserved hypothetical protein YabP | Hypothetical protein yabP [Bacillus licheniformis DSM 13] | UniRef100_Q65PG1 | Bacillus licheniformis DSM 13 |
| 68 | yabQ essential protein for formation of the spore cortex YabQ | Hypothetical protein yabQ [Bacillus licheniformis DSM 13] | UniRef100_Q65PG0 | Bacillus licheniformis DSM 13 |
| 69 | divIC cell-division initiation protein | DivIC [Bacillus licheniformis DSM 13] | UniRef100_Q65PF9 | Bacillus licheniformis DSM 13 |
| 70 | yabR putative Nucleic acid-binding OB-fold protein,contains S1 domain | YabR [Bacillus licheniformis DSM 13] | UniRef100_Q65PF8 | Bacillus licheniformis DSM 13 |
| 71 | spoIIE serine phosphatase | SpoIIE [Bacillus licheniformis DSM 13] | UniRef100_Q65PF7 | Bacillus licheniformis DSM 13 |
| 72 | yabS YabS | YabS [Bacillus licheniformis DSM 13] | UniRef100_Q65PF6 | Bacillus licheniformis DSM 13 |
| 73 | yabT putative serine_threonine-protein kinase | YabT [Bacillus licheniformis DSM 13] | UniRef100_Q65PF5 | Bacillus licheniformis DSM 13 |
| 74 | tilS tRNA(Ile)-lysidine synthetase | YacA [Bacillus licheniformis DSM 13] | UniRef100_Q65PF4 | Bacillus licheniformis DSM 13 |
| 75 | hprT hypoxanthine-guanine phosphoribosyltransferase | HprT [Bacillus licheniformis DSM 13] | UniRef100_Q65PF3 | Bacillus licheniformis DSM 13 |
| 76 | ftsH cell-division protein and general stress protein (class III heat-shock) | FtsH [Bacillus licheniformis DSM 13] | UniRef100_Q65PF2 | Bacillus licheniformis DSM 13 |
| 77 | BLP00074 hypothetical protein | | | |
| 78 | yacB conserved hypothetical protein YacB | Bordetella pertussis Bvg accessory factor [Bacillus licheniformis DSM 13] | UniRef100_Q62ZU0 | Bacillus licheniformis DSM 13 |
| 79 | hslO putative chaperone protein HslO | YacC [Bacillus licheniformis DSM 13] | UniRef100_Q65PF0 | Bacillus licheniformis DSM 13 |
| 80 | yacD putative PpiC-type peptidyl-prolyl cis-trans isomerase | YacD [Bacillus licheniformis DSM 13] | UniRef100_Q65PE9 | Bacillus licheniformis DSM 13 |
| 81 | cysK cysteine synthetase A | CysK [Bacillus licheniformis DSM 13] | UniRef100_Q65PE8 | Bacillus licheniformis DSM 13 |
| 82 | BLP04683 hypothetical protein | | | |
| 83 | pabB para-aminobenzoate synthase (subunit A) | PabB [Bacillus licheniformis DSM 13] | UniRef100_Q65PE7 | Bacillus licheniformis DSM 13 |
| 84 | pabA para-aminobenzoate synthase glutamine amidotransferase (subunit B) and anthranilate synthase (subunit II) | PabA (Para-aminobenzoate synthase glutamine amidotransferase (Subunit B) and anthranilate synthase) [Bacillus licheniformis DSM 13] | UniRef100_Q65PE6 | Bacillus licheniformis DSM 13 |
| 85 | pabC aminodeoxychorismate lyase | PabC [Bacillus licheniformis DSM 13] | UniRef100_Q65PE5 | Bacillus licheniformis DSM 13 |
| 86 | sul dihydropteroate synthase | Sul [Bacillus licheniformis DSM 13] | UniRef100_Q65PE4 | Bacillus licheniformis DSM 13 |
| 87 | folB dihydroneopterin aldolase | FolB [Bacillus licheniformis DSM 13] | UniRef100_Q65PE3 | Bacillus licheniformis DSM 13 |
| 88 | folK 7,8-dihydro-6-hydroxymethylpterin pyrophosphokinase | FolK [Bacillus licheniformis DSM 13] | UniRef100_Q65PE2 | Bacillus licheniformis DSM 13 |
| 89 | dus1 putative Dihydrouridine synthase TIM-barrel protein | YacF [Bacillus licheniformis DSM 13] | UniRef100_Q65PE1 | Bacillus licheniformis DSM 13 |
| 90 | lysS lysyl-tRNA synthetase | LysS [Bacillus licheniformis DSM 13] | UniRef100_Q65PE0 | Bacillus licheniformis DSM 13 |
| 91 | BLP00087 hypothetical protein | | | |
| 92 | ctsR transcriptional regulator | CtsR [Bacillus licheniformis DSM 13] | UniRef100_Q65PD9 | Bacillus licheniformis DSM 13 |
| 93 | mcsA McsA | Hypothetical protein mcsA [Bacillus licheniformis DSM 13] | UniRef100_Q65PD8 | Bacillus licheniformis DSM 13 |
| 94 | mcsB McsB | Hypothetical protein mcsB [Bacillus licheniformis DSM 13] | UniRef100_Q65PD7 | Bacillus licheniformis DSM 13 |

| 95 | clpC class III stress response-related ATPase | ClpC [Bacillus licheniformis DSM 13] | UniRef100_Q65PD6 | Bacillus licheniformis DSM 13 |
|---|---|---|---|---|
| 96 | radA DNA repair protein RadA | RadA [Bacillus licheniformis DSM 13] | UniRef100_Q65PD5 | Bacillus licheniformis DSM 13 |
| 97 | yacK putative DNA binding protein YacK | YacK (H+-transporting two-sector ATPase, delta (OSCP) subunit) [Bacillus licheniformis DSM 13] | UniRef100_Q65PD4 | Bacillus licheniformis DSM 13 |
| 98 | BLP00094 hypothetical protein | | | |
| 99 | yacL conserved hypothetical protein YacL | YacL [Bacillus licheniformis DSM 13] | UniRef100_Q65PD3 | Bacillus licheniformis DSM 13 |
| 100 | ispD 4-diphosphocytidyl-2C-methyl-D-erythritol synthase | YacM [Bacillus licheniformis DSM 13] | UniRef100_Q65PD2 | Bacillus licheniformis DSM 13 |
| 101 | ispF 2-C-methyl-D-erythritol 2,4-cyclodiphosphate synthase | YacN [Bacillus licheniformis DSM 13] | UniRef100_Q65PD1 | Bacillus licheniformis DSM 13 |
| 102 | gltX glutamyl-tRNA synthetase | GltX [Bacillus licheniformis DSM 13] | UniRef100_Q65PD0 | Bacillus licheniformis DSM 13 |
| 103 | BLP04684 hypothetical protein | | | |
| 104 | cysE serine acetyltransferase | CysE [Bacillus licheniformis DSM 13] | UniRef100_Q65PC9 | Bacillus licheniformis DSM 13 |
| 105 | cysS cysteinyl-tRNA synthetase | CysS [Bacillus licheniformis DSM 13] | UniRef100_Q65PC8 | Bacillus licheniformis DSM 13 |
| 106 | yazC putative Ribonuclease III, conserved hypothetical YazC protein | YazC [Bacillus licheniformis DSM 13] | UniRef100_Q65PC7 | Bacillus licheniformis DSM 13 |
| 107 | yacO putative tRNA_rRNA methyltransferase YacO | YacO [Bacillus licheniformis DSM 13] | UniRef100_Q65PC6 | Bacillus licheniformis DSM 13 |
| 108 | yacP conserved hypothetical protein YacP | Hypothetical protein yacP [Bacillus licheniformis DSM 13] | UniRef100_Q65PC5 | Bacillus licheniformis DSM 13 |
| 109 | sigH RNA polymerase sigma-30 factor (sigma-H) | RNA polymerase sigma-H factor [Bacillus licheniformis] | UniRef100_P02964 | Bacillus licheniformis |
| 110 | rpmGB 50S ribosomal protein L33 2 | RpmGB [Bacillus licheniformis DSM 13] | UniRef100_Q65PC3 | Bacillus licheniformis DSM 13 |
| 111 | secE preprotein translocase subunit | Preprotein translocase secE subunit [Bacillus licheniformis] | UniRef100_P38381 | Bacillus licheniformis |
| 112 | nusG transcription antitermination factor | NusG [Bacillus licheniformis DSM 13] | UniRef100_Q65PC1 | Bacillus licheniformis DSM 13 |
| 113 | rplK ribosomal protein L11 | RplK [Bacillus licheniformis DSM 13] | UniRef100_Q65PC0 | Bacillus licheniformis DSM 13 |
| 114 | rplA ribosomal protein L1 (BL1) | RplA [Bacillus licheniformis DSM 13] | UniRef100_Q65PB9 | Bacillus licheniformis DSM 13 |
| 115 | rplJ ribosomal protein L10 (BL5) | Ribosomal protein L10 [Bacillus licheniformis DSM 13] | UniRef100_Q62ZQ7 | Bacillus licheniformis DSM 13 |
| 116 | rplL ribosomal protein L12 (BL9) | Ribosomal protein L12 [Bacillus licheniformis DSM 13] | UniRef100_Q62ZQ6 | Bacillus licheniformis DSM 13 |
| 117 | ybxB hypothetical protein with SAM binding motif | YbxB (SAM (And some other nucleotide) binding motif) [Bacillus licheniformis DSM 13] | UniRef100_Q65PB6 | Bacillus licheniformis DSM 13 |
| 118 | rpoB RNA polymerase (beta subunit) | RNA polymerase beta subunit [Bacillus licheniformis] | UniRef100_Q65PB5 | Bacillus licheniformis |
| 119 | rpoC RNA polymerase (beta subunit) | RpoC [Bacillus licheniformis DSM 13] | UniRef100_Q65PB4 | Bacillus licheniformis DSM 13 |
| 120 | ybxF putative ribosomal protein L7AE family | YbxF [Bacillus licheniformis DSM 13] | UniRef100_Q65PB3 | Bacillus licheniformis DSM 13 |
| 121 | rpsL ribosomal protein S12 (BS12) | RpsL [Bacillus licheniformis DSM 13] | UniRef100_Q65PB2 | Bacillus licheniformis DSM 13 |
| 122 | rpsG ribosomal protein S7 (BS7) | RpsG [Bacillus licheniformis DSM 13] | UniRef100_Q65PB1 | Bacillus licheniformis DSM 13 |
| 123 | fusA elongation factor G | FusA [Bacillus licheniformis DSM 13] | UniRef100_Q65PB0 | Bacillus licheniformis DSM 13 |
| 124 | tufA elongation factor Tu | TufA [Bacillus licheniformis DSM 13] | UniRef100_Q65PA9 | Bacillus licheniformis DSM 13 |
| 125 | BLP04685 hypothetical protein | | | |
| 126 | rpsJ ribosomal protein RpsJ | 30S ribosomal protein S10 [Bacillus halodurans] | UniRef100_Q9Z9L5 | Bacillus halodurans |
| 127 | rplC ribosomal protein L3 (BL3) | RplC [Bacillus licheniformis DSM 13] | UniRef100_Q65PA7 | Bacillus licheniformis DSM 13 |

| 128 | rplD ribosomal protein L4 | RplD [Bacillus licheniformis DSM 13] | UniRef100_Q65PA6 | Bacillus licheniformis DSM 13 |
|---|---|---|---|---|
| 129 | rplW ribosomal protein L23 | RplW [Bacillus licheniformis DSM 13] | UniRef100_Q65PA5 | Bacillus licheniformis DSM 13 |
| 130 | rplB ribosomal protein L2 (BL2) | RplB [Bacillus licheniformis DSM 13] | UniRef100_Q65PA4 | Bacillus licheniformis DSM 13 |
| 131 | rpsS ribosomal protein RpsS | RpsS [Bacillus licheniformis DSM 13] | UniRef100_Q65PA3 | Bacillus licheniformis DSM 13 |
| 132 | rplV ribosomal protein RplV | RplV [Bacillus licheniformis DSM 13] | UniRef100_Q65PA2 | Bacillus licheniformis DSM 13 |
| 133 | rpsC ribosomal protein S3 (BS3) | RpsC [Bacillus licheniformis DSM 13] | UniRef100_Q65PA1 | Bacillus licheniformis DSM 13 |
| 134 | rplP ribosomal protein L16 | RplP [Bacillus licheniformis DSM 13] | UniRef100_Q65PA0 | Bacillus licheniformis DSM 13 |
| 135 | rpmC ribosomal protein L29 | | | |
| 136 | rpsQ ribosomal protein S17 (BS16) | RpsQ [Bacillus licheniformis DSM 13] | UniRef100_Q65P98 | Bacillus licheniformis DSM 13 |
| 137 | rplN ribosomal protein RplN | RplN [Bacillus licheniformis DSM 13] | UniRef100_Q65P97 | Bacillus licheniformis DSM 13 |
| 138 | rplX ribosomal protein L24 (BL23) (histone-like protein HPB12) | RplX [Bacillus licheniformis DSM 13] | UniRef100_Q65P96 | Bacillus licheniformis DSM 13 |
| 139 | rplE ribosomal protein L5 (BL6) | RplE [Bacillus licheniformis DSM 13] | UniRef100_Q65P95 | Bacillus licheniformis DSM 13 |
| 140 | rpsN 30S ribosomal protein S14 | RpsN [Bacillus licheniformis DSM 13] | UniRef100_Q65P94 | Bacillus licheniformis DSM 13 |
| 141 | rpsH ribosomal protein S8 (BS8) | RpsH [Bacillus licheniformis DSM 13] | UniRef100_Q65P93 | Bacillus licheniformis DSM 13 |
| 142 | rplF ribosomal protein L6 (BL8) | Ribosomal protein L6 [Bacillus licheniformis DSM 13] | UniRef100_Q62ZN1 | Bacillus licheniformis DSM 13 |
| 143 | rplR ribosomal protein L18 | Ribosomal protein L18 [Bacillus licheniformis DSM 13] | UniRef100_Q62ZN0 | Bacillus licheniformis DSM 13 |
| 144 | rpsE ribosomal protein S5 | RpsE [Bacillus licheniformis DSM 13] | UniRef100_Q65P90 | Bacillus licheniformis DSM 13 |
| 145 | rpmD ribosomal protein L30 (BL27) | RpmD [Bacillus licheniformis DSM 13] | UniRef100_Q65P89 | Bacillus licheniformis DSM 13 |
| 146 | rplO ribosomal protein L15 | RplO [Bacillus licheniformis DSM 13] | UniRef100_Q65P88 | Bacillus licheniformis DSM 13 |
| 147 | secY preprotein translocase subunit | SecY [Bacillus licheniformis DSM 13] | UniRef100_Q65P87 | Bacillus licheniformis DSM 13 |
| 148 | adk adenylate kinase | Adk [Bacillus licheniformis DSM 13] | UniRef100_Q65P86 | Bacillus licheniformis DSM 13 |
| 149 | map methionine aminopeptidase | Map [Bacillus licheniformis DSM 13] | UniRef100_Q65P85 | Bacillus licheniformis DSM 13 |
| 150 | BLP00139 hypothetical protein | Hypothetical protein [Bacillus licheniformis DSM 13] | UniRef100_Q65P84 | Bacillus licheniformis DSM 13 |
| 151 | infA Translation initiation factor IF-1 InfA | InfA [Bacillus licheniformis DSM 13] | UniRef100_Q65P83 | Bacillus licheniformis DSM 13 |
| 152 | rpmJ ribosomal protein L36 (ribosomal protein B) | 50S ribosomal protein L36 [Bacillus subtilis] | UniRef100_P20278 | Bacillus subtilis |
| 153 | rpsM Ribosomal protein S13 RpsM | RpsM [Bacillus licheniformis DSM 13] | UniRef100_Q65P81 | Bacillus licheniformis DSM 13 |
| 154 | rpsK ribosomal protein S11 (BS11) | RpsK [Bacillus licheniformis DSM 13] | UniRef100_Q65P80 | Bacillus licheniformis DSM 13 |
| 155 | rpoA RNA polymerase (alpha subunit) | RpoA [Bacillus licheniformis DSM 13] | UniRef100_Q65P79 | Bacillus licheniformis DSM 13 |
| 156 | rplQ ribosomal protein L17 (BL15) | RplQ [Bacillus licheniformis DSM 13] | UniRef100_Q65P78 | Bacillus licheniformis DSM 13 |
| 157 | cbiO ABC transporter | YbxA [Bacillus licheniformis DSM 13] | UniRef100_Q65P77 | Bacillus licheniformis DSM 13 |
| 158 | ybaE ABC transport system ATP-binding protein | YbaE [Bacillus licheniformis DSM 13] | UniRef100_Q65P76 | Bacillus licheniformis DSM 13 |
| 159 | ybaF Cobalt transport protein | YbaF [Bacillus licheniformis DSM 13] | UniRef100_Q65P75 | Bacillus licheniformis DSM 13 |
| 160 | truA pseudouridylate synthase I | TruA [Bacillus licheniformis DSM 13] | UniRef100_Q65P74 | Bacillus licheniformis DSM 13 |
| 161 | rplM ribosomal protein L13 | RplM [Bacillus licheniformis DSM 13] | UniRef100_Q65P73 | Bacillus licheniformis DSM 13 |
| 162 | rpsI ribosomal protein S9 | RpsI [Bacillus licheniformis DSM 13] | UniRef100_Q65P72 | Bacillus licheniformis DSM 13 |

| 163 | BLP00148 Lambda integrase-like protein(phage related) | Hypothetical protein [Bacillus licheniformis DSM 13] | UniRef100_Q65KT3 | Bacillus licheniformis DSM 13 |
|---|---|---|---|---|
| 164 | BLP04812 phage related protein | Hypothetical protein [Bacillus licheniformis DSM 13] | UniRef100_Q62W79 | Bacillus licheniformis DSM 13 |
| 165 | yonS conserved hypothetical phage protein YonS | Hypothetical protein [Bacillus licheniformis DSM 13] | UniRef100_Q65KT1 | Bacillus licheniformis DSM 13 |
| 166 | BLP00149 conserved hypothetical phage protein | Hypothetical protein [Bacillus licheniformis DSM 13] | UniRef100_Q65KT0 | Bacillus licheniformis DSM 13 |
| 167 | BLP00150 hypothetical protein | Hypothetical protein [Bacillus licheniformis DSM 13] | UniRef100_Q65KS9 | Bacillus licheniformis DSM 13 |
| 168 | yqaE probable transcriptional regulator (phage-related) | YqaE [Bacillus licheniformis DSM 13] | UniRef100_Q65KS8 | Bacillus licheniformis DSM 13 |
| 169 | BLP00152 Putative transcriptional regulator | Transcriptional regulator, Cro/CI family [Enterococcus faecalis] | UniRef100_Q832S7 | Enterococcus faecalis |
| 170 | BLP00153 hypothetical protein | Hypothetical protein [Bacillus licheniformis DSM 13] | UniRef100_Q65KS7 | Bacillus licheniformis DSM 13 |
| 171 | BLP00154 conserved hypothetical protein | Hypothetical protein [Bacillus licheniformis DSM 13] | UniRef100_Q65KS6 | Bacillus licheniformis DSM 13 |
| 172 | BLP00155 putative phage protein | 77ORF010 [Bacteriophage 77] | UniRef100_Q6R854 | Bacteriophage 77 |
| 173 | BLP00156 hypothetical protein | Hypothetical protein [Bacillus licheniformis DSM 13] | UniRef100_Q65KS4 | Bacillus licheniformis DSM 13 |
| 174 | BLP04687 hypothetical protein | | | |
| 175 | BLP04842 conserved hypothetical protein | Hypothetical protein [Bacillus licheniformis DSM 13] | UniRef100_Q62W68 | Bacillus licheniformis DSM 13 |
| 176 | BLP00157 phage related protein | Orf178 [Staphylococcus aureus temperate phage phiSLT] | UniRef100_Q9B0G4 | Staphylococcus aureus temperate phage phiSLT |
| 177 | BLP00158 conserved hypothetical phage protein | Hypothetical protein [Enterococcus faecalis] | UniRef100_Q833D2 | Enterococcus faecalis |
| 178 | BLP00159 conserved hypothetical phage protein | | | |
| 179 | BLP00160 hypothetical protein | | | |
| 180 | BLP04686 hypothetical protein | | | |
| 181 | BLP00161 hypothetical protein | | | |
| 182 | BLP00162 conserved hypothetical phage protein | Hypothetical protein [Bacillus anthracis] | UniRef100_Q81XU9 | Bacillus anthracis |
| 183 | BLP00163 hypothetical protein | DNA replication protein DnaC [Clostridium acetobutylicum] | UniRef100_Q97HS5 | Clostridium acetobutylicum |
| 184 | yqaO phage related protein YqaO | YqaO [Bacillus licheniformis DSM 13] | UniRef100_Q65KR7 | Bacillus licheniformis DSM 13 |
| 185 | BLP00166 conserved hypothetical protein | Hypothetical protein [Bacillus licheniformis DSM 13] | UniRef100_Q65KR6 | Bacillus licheniformis DSM 13 |
| 186 | BLP00167 conserved hypothetical protein | Hypothetical protein [Bacillus licheniformis DSM 13] | UniRef100_Q65KR5 | Bacillus licheniformis DSM 13 |
| 187 | BLP00168 hypothetical protein | | | |
| 188 | BLP00169 hypothetical protein | Hypothetical protein [Bacillus licheniformis DSM 13] | UniRef100_Q65KR2 | Bacillus licheniformis DSM 13 |
| 189 | BLP00170 phage related protein | Prophage Lp1 protein 30 [Lactobacillus plantarum] | UniRef100_Q88YU0 | Lactobacillus plantarum |
| 190 | yqaN phage related protein YqaN | YqaN [Bacillus licheniformis DSM 13] | UniRef100_Q65KQ9 | Bacillus licheniformis DSM 13 |
| 191 | BLP00172 hypothetical protein | Hypothetical protein [Bacillus licheniformis DSM 13] | UniRef100_Q62W56 | Bacillus licheniformis DSM 13 |
| 192 | BLP00173 hypothetical protein | | | |
| 193 | BLP00174 conserved Ssb-homolog protein | Ssb [Bacillus licheniformis DSM 13] | UniRef100_Q65CP4 | Bacillus licheniformis DSM 13 |
| 194 | BLP00175 hypothetical protein | | | |
| 195 | BLP00175 hypothetical protein | | | |
| 196 | BLP00177 hypothetical protein | | | |

| 197 | ssb single-strand DNA-binding protein | Single-strand DNA-binding protein [Bacillus licheniformis DSM 13] | UniRef100_Q62W55 | Bacillus licheniformis DSM 13 |
|---|---|---|---|---|
| 198 | BLP04690 hypothetical protein | | | |
| 199 | BLP00179 conserved hypothetical protein | Hypothetical protein [Bacillus licheniformis DSM 13] | UniRef100_Q65KQ7 | Bacillus licheniformis DSM 13 |
| 200 | BLP00180 hypothetical protein | Hypothetical protein [Bacillus licheniformis DSM 13] | UniRef100_Q65KQ6 | Bacillus licheniformis DSM 13 |
| 201 | BLP00181 conserved hypothetical protein | YqaQ [Bacillus licheniformis DSM 13] | UniRef100_Q65KQ3 | Bacillus licheniformis DSM 13 |
| 202 | BLP00182 phage related protein | Hypothetical protein [Bacillus licheniformis DSM 13] | UniRef100_Q65KQ1 | Bacillus licheniformis DSM 13 |
| 203 | BLP00183 hypothetical protein | | | |
| 204 | BLP00184 putative terminase small subunit protein | Putative terminase small subunit TerS [Bacteriophage Aaphi23] | UniRef100_Q7Y5U8 | Bacteriophage Aaphi23 |
| 205 | BLP00185 putative phage terminase, large subunit, PBSX family | UPI00003CC57A UniRef100 entry | UniRef100_UPI00003CC57A | |
| 206 | BLP00186 hypothetical protein | | | |
| 207 | BLP00187 putative Phage portal protein, A118 family | UPI00003CC5A4 UniRef100 entry | UniRef100_UPI00003CC5A4 | |
| 208 | BLP00189 putative Gp4-like protein | UPI00003CC514 UniRef100 entry | UniRef100_UPI00003CC514 | |
| 209 | BLP00190 hypothetical protein | | | |
| 210 | BLP00191 hypothetical protein | | | |
| 211 | BLP00192 conserved hypothetical phage protein | Pas24 [Actinoplanes phage phiAsp2] | UniRef100_Q6J807 | Actinoplanes phage phiAsp2 |
| 212 | BLP00193 conserved hypothetical phage-like protein | Hypothetical protein SPs0635 [Streptococcus pyogenes] | UniRef100_Q879B4 | Streptococcus pyogenes |
| 213 | BLP00194 conserved phage protein | Lin2390 protein [Listeria innocua] | UniRef100_Q928Z0 | Listeria innocua |
| 214 | BLP00195 conserved phage protein | | | |
| 215 | BLP00196 conserved phage protein | UPI00003CC51A UniRef100 entry | UniRef100_UPI00003CC51A | |
| 216 | BLP04843 conserved hypothetical phage protein | UPI00003CA3DF UniRef100 entry | UniRef100_UPI00003CA3DF | |
| 217 | BLP00197 hypothetical protein | | | |
| 218 | BLP00198 hypothetical protein | UPI00003CC51D UniRef100 entry | UniRef100_UPI00003CC51D | |
| 219 | BLP04691 hypothetical protein | | | |
| 220 | BLP00199 hypothetical protein | UPI00003CC51E UniRef100 entry | UniRef100_UPI00003CC51E | |
| 221 | BLP00200 hypothetical protein | UPI00003CC549 UniRef100 entry | UniRef100_UPI00003CC549 | |
| 222 | BLP00201 hypothetical protein | Hypothetical protein [Bacillus licheniformis DSM 13] | UniRef100_Q65KN3 | Bacillus licheniformis DSM 13 |
| 223 | BLP00203 hypothetical protein | UPI00003CC2A0 UniRef100 entry | UniRef100_UPI00003CC2A0 | |

| 224 | BLP00204 phage related protein | Hypothetical Phage minor structural protein, N-terminal [Bacillus licheniformis DSM 13] | UniRef100_Q65KN1 | Bacillus licheniformis DSM 13 |
|---|---|---|---|---|
| 225 | BLP00205 hypothetical protein | Complete nucleotide sequence [Bacteriophage SPP1] | UniRef100_O48465 | Bacteriophage SPP1 |
| 226 | BLP00206 hypothetical protein | | | |
| 227 | BLP00207 hypothetical protein | | | |
| 228 | BLP00208 hypothetical protein | | | |
| 229 | BLP00209 hypothetical protein | | | |
| 230 | BLP00210 hypothetical protein | | | |
| 231 | BLP00211 N-acetylmuramoyl-L-alanine amidase | Complete nucleotide sequence [Bacteriophage SPP1] | UniRef100_O48471 | Bacteriophage SPP1 |
| 232 | BLP00212 hypothetical protein | YizA [Bacillus licheniformis DSM 13] | UniRef100_Q65P71 | Bacillus licheniformis DSM 13 |
| 233 | ybaK conserved hypothetical protein YbaK | Hypothetical protein ybaK [Bacillus licheniformis DSM 13] | UniRef100_Q65P70 | Bacillus licheniformis DSM 13 |
| 234 | cwlD N-acetylmuramoyl-L-alanine amidase | CwlD [Bacillus licheniformis DSM 13] | UniRef100_Q65P69 | Bacillus licheniformis DSM 13 |
| 235 | mrp putative ATP-binding protein Mrp | YbaL [Bacillus licheniformis DSM 13] | UniRef100_Q65P68 | Bacillus licheniformis DSM 13 |
| 236 | gerD GerD | Hypothetical protein gerD [Bacillus licheniformis DSM 13] | UniRef100_Q65P67 | Bacillus licheniformis DSM 13 |
| 237 | BLP04692 hypothetical protein | | | |
| 238 | kbaA kinB signaling pathway activation protein KbaA | Hypothetical protein kbaA [Bacillus licheniformis DSM 13] | UniRef100_Q65P66 | Bacillus licheniformis DSM 13 |
| 239 | ybaN Polysaccharide deacetylase,Carbohydrate Esterase Family 4 | YbaN [Bacillus licheniformis DSM 13] | UniRef100_Q65P65 | Bacillus licheniformis DSM 13 |
| 240 | BLP00219 putative integrase | UPI00003CB8B4 UniRef100 entry | UniRef100_UPI00003CB8B4 | |
| 241 | BLP00220 hypothetical protein | | | |
| 242 | BLP00221 hypothetical protein | Hypothetical protein [Bacillus licheniformis DSM 13] | UniRef100_Q65KE2 | Bacillus licheniformis DSM 13 |
| 243 | BLP00222 hypothetical protein | | | |
| 244 | BLP04911 conserved hyopothetical protein | | | |
| 245 | BLP04912 hypothetical protein | | | |
| 246 | BLP04693 hypothetical protein | | | |
| 247 | BLP04913 hypothetical protein | | | |
| 248 | BLP00223 hypothetical protein | | | |
| 249 | BLP04914 hypothetical protein | | | |
| 250 | BLP00224 hypothetical protein | | | |
| 251 | BLP04915 hypothetical protein | | | |
| 252 | BLP00225 hypothetical protein | Hypothetical protein [Bacillus cereus ZK] | UniRef100_Q633V9 | Bacillus cereus ZK |
| 253 | BLP04916 hypothetical protein | | | |
| 254 | BLP00226 hypothetical protein | | | |
| 255 | BLP00227 hypothetical protein | | | |
| 256 | BLP04917 hypothetical protein | | | |
| 257 | BLP04918 hypothetical protein | | | |

| 258 | BLP00228 hypothetical protein | Transposase, IS256 family [Enterococcus faecalis] | UniRef100_Q833S8 | Enterococcus faecalis |
|---|---|---|---|---|
| 259 | BLP04694 hypothetical protein | Very-short-patch-repair endonuclease [Oceanobacillus iheyensis] | UniRef100_Q8EL90 | Oceanobacillus iheyensis |
| 260 | BLP00229 hypothetical protein | Hypothetical protein OB3334 [Oceanobacillus iheyensis] | UniRef100_Q8EL97 | Oceanobacillus iheyensis |
| 261 | BLP04813 conserved hypothetical | Hypothetical protein [Bacillus cereus ZK] | UniRef100_Q636X5 | Bacillus cereus ZK |
| 262 | BLP00230 Modification methylase, Cytosine-specific methyltransferase | Cytosine-specific DNA-methyltransferase [Oceanobacillus iheyensis] | UniRef100_Q8EL98 | Oceanobacillus iheyensis |
| 263 | BLP00231 hypothetical protein | | | |
| 264 | BLP00232 hypothetical protein | | | |
| 265 | BLP00233 hypothetical protein | | | |
| 266 | BLP04695 hypothetical protein | DNA-binding protein [Listeria monocytogenes] | UniRef100_Q723Z9 | Listeria monocytogenes |
| 267 | BLP00234 hypothetical protein | | | |
| 268 | pbpX penicillin-binding protein | PbpX [Bacillus licheniformis DSM 13] | UniRef100_Q65P64 | Bacillus licheniformis DSM 13 |
| 269 | BLP00236 hypothetical protein | Hypothetical protein [Bacillus licheniformis DSM 13] | UniRef100_Q65P63 | Bacillus licheniformis DSM 13 |
| 270 | ybaS Bile acid:sodium symporter | YbaS [Bacillus licheniformis DSM 13] | UniRef100_Q65P62 | Bacillus licheniformis DSM 13 |
| 271 | yxaJ conserved hypothetical protein YxaJ | YxaJ [Bacillus licheniformis DSM 13] | UniRef100_Q65P61 | Bacillus licheniformis DSM 13 |
| 272 | BLP00239 Phenazine biosynthesis PhzC_PhzF protein, Aminoacyl-tRNA synthetase, class I | Hypothetical protein [Bacillus licheniformis DSM 13] | UniRef100_Q65P60 | Bacillus licheniformis DSM 13 |
| 273 | ybbC conserved hypothetical protein YbbC | YbbC [Bacillus licheniformis DSM 13] | UniRef100_Q65P59 | Bacillus licheniformis DSM 13 |
| 274 | ybbD Glycoside hydrolase, family 3 | YbbD [Bacillus licheniformis DSM 13] | UniRef100_Q65P58 | Bacillus licheniformis DSM 13 |
| 275 | ybbE putative beta-lactamase YbbE | YbbE [Bacillus licheniformis DSM 13] | UniRef100_Q65P57 | Bacillus licheniformis DSM 13 |
| 276 | BLP00243 hypothetical protein | | | |
| 277 | ybbF Phosphotransferase system PTS, EIIB domain, Phosphotransferase system, EIIC | YbbF [Bacillus licheniformis DSM 13] | UniRef100_Q65P56 | Bacillus licheniformis DSM 13 |
| 278 | ybbH probable transcriptional regulator YbbH | YbbH [Bacillus licheniformis DSM 13] | UniRef100_Q65P55 | Bacillus licheniformis DSM 13 |
| 279 | ybbI putative sugar phosphate isomerase YbbI | YbbI [Bacillus licheniformis DSM 13] | UniRef100_Q65P54 | Bacillus licheniformis DSM 13 |
| 280 | ybbK conserved hypothetical protein YbbK | YbbK [Bacillus licheniformis DSM 13] | UniRef100_Q65P53 | Bacillus licheniformis DSM 13 |
| 281 | rocF arginase | Hypothetical protein [Bacillus licheniformis DSM 13] | UniRef100_Q65P52 | Bacillus licheniformis DSM 13 |
| 282 | sigW RNA polymerase ECF(extracytoplasmic function)-type sigma factor (sigma-W) | SigW (RNA polymerase ECF(Extracytoplasmic function)-type sigma factor) [Bacillus licheniformis DSM 13] | UniRef100_Q65P51 | Bacillus licheniformis DSM 13 |
| 283 | rsiW antisigma factor RsiW | Hypothetical protein ybbM [Bacillus licheniformis DSM 13] | UniRef100_Q65P50 | Bacillus licheniformis DSM 13 |
| 284 | ybbP conserved hypothetical containing domain DUF147 YbbP | Hypothetical protein ybbP [Bacillus licheniformis DSM 13] | UniRef100_Q62ZI8 | Bacillus licheniformis DSM 13 |
| 285 | ybbR conserved hypothetical protein YbbR | Hypothetical protein ybbR [Bacillus licheniformis DSM 13] | UniRef100_Q65P48 | Bacillus licheniformis DSM 13 |
| 286 | glmM putative Phosphoglucosamine mutase GlmM | YbbT [Bacillus licheniformis DSM 13] | UniRef100_Q65P47 | Bacillus licheniformis DSM 13 |
| 287 | BLP00254 hypothetical protein | | | |
| 288 | glmS L-glutamine-D-fructose-6-phosphate amidotransferase | GlmS [Bacillus licheniformis DSM 13] | UniRef100_Q65P46 | Bacillus licheniformis DSM 13 |

| 289 | BLP00256 hypothetical protein | Hypothetical protein [Bacillus licheniformis DSM 13] | UniRef100_Q65P45 | Bacillus licheniformis DSM 13 |
|---|---|---|---|---|
| 290 | BLP00257 hypothetical protein | Hypothetical protein [Bacillus licheniformis DSM 13] | UniRef100_Q65P44 | Bacillus licheniformis DSM 13 |
| 291 | BLP00258 ABC transporter,ATP_GTP-binding site motif A (P-loop) | Hypothetical protein [Bacillus licheniformis DSM 13] | UniRef100_Q65P43 | Bacillus licheniformis DSM 13 |
| 292 | BLP00259 conserved hypothetical protein | Hypothetical protein [Bacillus licheniformis DSM 13] | UniRef100_Q65P42 | Bacillus licheniformis DSM 13 |
| 293 | BLP00260 hypothetical protein | Hypothetical protein [Bacillus licheniformis DSM 13] | UniRef100_Q65P41 | Bacillus licheniformis DSM 13 |
| 294 | ybcL probable transporter | YbcL [Bacillus licheniformis DSM 13] | UniRef100_Q65P40 | Bacillus licheniformis DSM 13 |
| 295 | BLP00262 conserved hypothetical protein | Hypothetical protein [Bacillus licheniformis DSM 13] | UniRef100_Q65P39 | Bacillus licheniformis DSM 13 |
| 296 | yvcC ABC transporter | YvcC [Bacillus licheniformis DSM 13] | UniRef100_Q65P38 | Bacillus licheniformis DSM 13 |
| 297 | ywbO DSBA oxidoreductase YwbO | YwbO [Bacillus licheniformis DSM 13] | UniRef100_Q65P37 | Bacillus licheniformis DSM 13 |
| 298 | BLP04696 hypothetical protein | | | |
| 299 | BLP00265 hypothetical protein | Hypothetical protein [Bacillus licheniformis DSM 13] | UniRef100_Q65P36 | Bacillus licheniformis DSM 13 |
| 300 | BLP00266 conserved hypothetical protein | Hypothetical protein [Bacillus licheniformis DSM 13] | UniRef100_Q65P35 | Bacillus licheniformis DSM 13 |
| 301 | aadK aminoglycoside 6-adenylyltransferase | AadK [Bacillus licheniformis DSM 13] | UniRef100_Q65P34 | Bacillus licheniformis DSM 13 |
| 302 | BLP00268 conserved hypothetical kinase-like protein | Hypothetical protein [Bacillus licheniformis DSM 13] | UniRef100_Q65P33 | Bacillus licheniformis DSM 13 |
| 303 | yfnB HAD-superfamily hydrolase, subfamily IA, variant 1 YfnB | YfnB [Bacillus licheniformis DSM 13] | UniRef100_Q65P32 | Bacillus licheniformis DSM 13 |
| 304 | ybxG Amino acid permease YbxG | YbxG [Bacillus licheniformis DSM 13] | UniRef100_Q65P31 | Bacillus licheniformis DSM 13 |
| 305 | BLP00271 hypothetical protein | | | |
| 306 | BLP00272 hypothetical protein | | | |
| 307 | citM secondary transporter of divalent metal ions_citrate complexes | CitM [Bacillus licheniformis DSM 13] | UniRef100_Q65P30 | Bacillus licheniformis DSM 13 |
| 308 | BLP00274 hypothetical protein | | | |
| 309 | yflP YflP | YflP [Bacillus licheniformis DSM 13] | UniRef100_Q65P29 | Bacillus licheniformis DSM 13 |
| 310 | citT two-component response regulator | CitT [Bacillus licheniformis DSM 13] | UniRef100_Q65P28 | Bacillus licheniformis DSM 13 |
| 311 | citS two-component sensor histidine kinase | CitS [Bacillus licheniformis DSM 13] | UniRef100_Q65P27 | Bacillus licheniformis DSM 13 |
| 312 | cisL transcriptional activator of the cysJI operon | Hypothetical protein [Bacillus licheniformis DSM 13] | UniRef100_Q65P26 | Bacillus licheniformis DSM 13 |
| 313 | BLP00279 hypothetical protein | Hypothetical protein [Bacillus licheniformis DSM 13] | UniRef100_Q65P25 | Bacillus licheniformis DSM 13 |
| 314 | BLP00280 Sugar transporter superfamily | Hypothetical protein [Bacillus licheniformis DSM 13] | UniRef100_Q65P24 | Bacillus licheniformis DSM 13 |
| 315 | csgA sporulation-specific SASP protein | CsgA [Bacillus licheniformis DSM 13] | UniRef100_Q65P23 | Bacillus licheniformis DSM 13 |
| 316 | ybxH conserved hypothetical protein YbxH | Hypothetical protein ybxH [Bacillus licheniformis DSM 13] | UniRef100_Q65P22 | Bacillus licheniformis DSM 13 |
| 317 | BLP00283 hypothetical protein | | | |
| 318 | ybyB conserved hypothetical protein YbyB | Hypothetical protein ybyB [Bacillus licheniformis DSM 13] | UniRef100_Q62ZG0 | Bacillus licheniformis DSM 13 |
| 319 | yyaL conserved protein YyaL | YyaL [Bacillus licheniformis DSM 13] | UniRef100_Q65P20 | Bacillus licheniformis DSM 13 |
| 320 | ydeG Major facilitator superfamily | YdeG [Bacillus licheniformis DSM 13] | UniRef100_Q65P12 | Bacillus licheniformis DSM 13 |
| 321 | BLP00287 SAM (and some other nucleotide) binding motif,Generic methyltransferase | Hypothetical protein [Bacillus licheniformis DSM 13] | UniRef100_Q65P11 | Bacillus licheniformis DSM 13 |
| 322 | BLP00288 conserved hypothetical, putative epimerase | Hypothetical protein [Bacillus licheniformis DSM 13] | UniRef100_Q65P10 | Bacillus licheniformis DSM 13 |

| 323 | BLP04697 hypothetical protein | | | |
|---|---|---|---|---|
| 324 | BLP00289 hypothetical protein | Hypothetical protein [Bacillus licheniformis DSM 13] | UniRef100_Q65P09 | Bacillus licheniformis DSM 13 |
| 325 | BLP00290 Na_Pi cotransporter II-related protein | Hypothetical protein [Bacillus licheniformis DSM 13] | UniRef100_Q65P08 | Bacillus licheniformis DSM 13 |
| 326 | yubF conserved protein YubF | YubF [Bacillus licheniformis DSM 13] | UniRef100_Q65P07 | Bacillus licheniformis DSM 13 |
| 327 | BLP00292 hypothetical protein | Hypothetical protein [Bacillus licheniformis DSM 13] | UniRef100_Q65P06 | Bacillus licheniformis DSM 13 |
| 328 | BLP00293 hypothetical protein | YbfF [Bacillus licheniformis DSM 13] | UniRef100_Q65P05 | Bacillus licheniformis DSM 13 |
| 329 | BLP00294 hypothetical protein | Hypothetical protein [Bacillus licheniformis DSM 13] | UniRef100_Q65P04 | Bacillus licheniformis DSM 13 |
| 330 | BLP00295 hypothetical protein | Hypothetical protein [Bacillus licheniformis DSM 13] | UniRef100_Q65P03 | Bacillus licheniformis DSM 13 |
| 331 | ybfH conserved hypothetical membrane protein YbfH | Hypothetical protein ybfH [Bacillus licheniformis DSM 13] | UniRef100_Q65P02 | Bacillus licheniformis DSM 13 |
| 332 | ybfI conserved hypothetical protein YbfI | YbfI protein [Bacillus subtilis] | UniRef100_O31449 | Bacillus subtilis |
| 333 | BLP00298 hypothetical protein | Hypothetical protein [Bacillus licheniformis DSM 13] | UniRef100_Q65NZ9 | Bacillus licheniformis DSM 13 |
| 334 | BLP00299 conserved hypothetical DNA-binding protein | Hypothetical protein [Bacillus licheniformis DSM 13] | UniRef100_Q65NZ8 | Bacillus licheniformis DSM 13 |
| 335 | BLP00300 Hypothetical Bipartite response regulator, C-terminal effector | Hypothetical protein [Bacillus licheniformis DSM 13] | UniRef100_Q65NZ7 | Bacillus licheniformis DSM 13 |
| 336 | BLP00301 putative Iron-containing alcohol dehydrogenase | Hypothetical protein [Bacillus licheniformis DSM 13] | UniRef100_Q65NZ6 | Bacillus licheniformis DSM 13 |
| 337 | yvaQ putative transmembrane receptor taxis protein YvaQ | YvaQ [Bacillus licheniformis DSM 13] | UniRef100_Q65NZ5 | Bacillus licheniformis DSM 13 |
| 338 | BLP00303 putative ribose ABC transporter (ribose-binding protein) | Hypothetical protein [Bacillus licheniformis DSM 13] | UniRef100_Q65NZ4 | Bacillus licheniformis DSM 13 |
| 339 | BLP00304 putative lytic transglycosylase YomI | Hypothetical protein yomI [Bacillus licheniformis DSM 13] | UniRef100_Q65NZ3 | Bacillus licheniformis DSM 13 |
| 340 | BLP00305 hypothetical protein | Hypothetical protein [Bacillus licheniformis DSM 13] | UniRef100_Q65NZ2 | Bacillus licheniformis DSM 13 |
| 341 | BLP00306 putative soluble quinoprotein glucose dehydrogenase protein | Hypothetical protein [Bacillus licheniformis DSM 13] | UniRef100_Q65NZ1 | Bacillus licheniformis DSM 13 |
| 342 | ycbP conserved hypothetical protein YcbP | YcbP [Bacillus licheniformis DSM 13] | UniRef100_Q65NZ0 | Bacillus licheniformis DSM 13 |
| 343 | ybgF Amino acid permease YbgF | YbgF [Bacillus licheniformis DSM 13] | UniRef100_Q65NY9 | Bacillus licheniformis DSM 13 |
| 344 | ybgG Homocysteine S-methyltransferase YbgG | YbgG [Bacillus licheniformis DSM 13] | UniRef100_Q65NY8 | Bacillus licheniformis DSM 13 |
| 345 | BLP00310 Hypothetical YdjC-like protein | Hypothetical protein [Bacillus licheniformis DSM 13] | UniRef100_Q65NY7 | Bacillus licheniformis DSM 13 |
| 346 | BLP00311 phosphotransferase system (PTS) N-acetylglucosamine-specific enzyme IICB component | Hypothetical protein (Phosphotransferase system (PTS) N-acetylglucosamine-specific enzyme IICB component) [Bacillus licheniformis DSM 13] | UniRef100_Q65NY6 | Bacillus licheniformis DSM 13 |
| 347 | mtaA transcriptional regulator | Hypothetical protein [Bacillus licheniformis DSM 13] | UniRef100_Q65NY5 | Bacillus licheniformis DSM 13 |
| 348 | BLP00313 conserved hypothetical protein | Hypothetical protein [Bacillus licheniformis DSM 13] | UniRef100_Q65NY4 | Bacillus licheniformis DSM 13 |
| 349 | BLP00314 putative carbonic anhyrdase | Hypothetical protein [Bacillus licheniformis DSM 13] | UniRef100_Q65NY3 | Bacillus licheniformis DSM 13 |
| 350 | BLP00315 hypothetical protein | | | |
| 351 | yjgA conserved hypothetical protein YjgA | YjgA [Bacillus licheniformis DSM 13] | UniRef100_Q65NY2 | Bacillus licheniformis DSM 13 |
| 352 | BLP00317 Sodium:alanine symporter | Sodium:alanine symporter,Sodium:alanine symporter [Bacillus licheniformis DSM 13] | UniRef100_Q62ZC8 | Bacillus licheniformis DSM 13 |
| 353 | ybgJ Glutaminase YbgJ | YbgJ [Bacillus licheniformis DSM 13] | UniRef100_Q65NY1 | Bacillus licheniformis DSM 13 |

| | | | | |
|---|---|---|---|---|
| 354 | BLP00319 hypothetical protein | | | |
| 355 | ycbA Two component Histidine kinase YcbA | YcbA [Bacillus licheniformis DSM 13] | UniRef100_Q65NY0 | Bacillus licheniformis DSM 13 |
| 356 | glnL, ycbB Sensory transduction protein GlnL | YcbB [Bacillus licheniformis DSM 13] | UniRef100_Q65NX9 | Bacillus licheniformis DSM 13 |
| 357 | ynaD GCN5-related N-acetyltransferase | YnaD [Bacillus licheniformis DSM 13] | UniRef100_Q65NX8 | Bacillus licheniformis DSM 13 |
| 358 | blaRI Penicillin-binding protein, transpeptidase domain,Peptidase M56, BlaR1 | Regulatory protein blaR1 [Bacillus licheniformis DSM 13] | UniRef100_Q65NX7 | Bacillus licheniformis DSM 13 |
| 359 | blaI putative beta-lactamase repressor protein | Penicillinase repressor [Bacillus licheniformis DSM 13] | UniRef100_Q65NX6 | Bacillus licheniformis DSM 13 |
| 360 | BLP00325 hypothetical protein | | | |
| 361 | BLP00326 hypothetical protein | | | |
| 362 | penP beta-lactamase precursor | PenP [Bacillus licheniformis DSM 13] | UniRef100_Q65NX5 | Bacillus licheniformis DSM 13 |
| 363 | BLP00328 hypothetical protein | | | |
| 364 | phoD phosphodiesterase_alkaline phosphatase D | Phosphodiesterase/alkaline phosphatase D [Bacillus licheniformis DSM 13] | UniRef100_Q62ZC0 | Bacillus licheniformis DSM 13 |
| 365 | tatAD component of the twin-arginine pre-protein translocation pathway | TatAD [Bacillus licheniformis DSM 13] | UniRef100_Q65NX3 | Bacillus licheniformis DSM 13 |
| 366 | tatCD component of the twin-arginine pre-protein translocation pathway | TatCD [Bacillus licheniformis DSM 13] | UniRef100_Q65NX2 | Bacillus licheniformis DSM 13 |
| 367 | BLP00332 hypothetical protein | | | |
| 368 | kdgD Probable 5-dehydro-4-deoxyglucarate dehydratase KdgD | YcbC [Bacillus licheniformis DSM 13] | UniRef100_Q65NX1 | Bacillus licheniformis DSM 13 |
| 369 | ybcD Aldehyde dehydrogenase YbcD | YcbD [Bacillus licheniformis DSM 13] | UniRef100_Q65NX0 | Bacillus licheniformis DSM 13 |
| 370 | gudP D-galactonate transporter | YcbE [Bacillus licheniformis DSM 13] | UniRef100_Q65NW9 | Bacillus licheniformis DSM 13 |
| 371 | gudD Mandelate racemase_muconate lactonizing enzyme | YcbF [Bacillus licheniformis DSM 13] | UniRef100_Q65NW8 | Bacillus licheniformis DSM 13 |
| 372 | ycbG putative regulatory protein YcbG | YcbG [Bacillus licheniformis DSM 13] | UniRef100_Q65NW7 | Bacillus licheniformis DSM 13 |
| 373 | garD D-galactarate dehydratase_Altronate hydrolase, N-terminal,D-galactarate dehydratase_Altronate hydrolase, C-terminal | YcbH [Bacillus licheniformis DSM 13] | UniRef100_Q65NW6 | Bacillus licheniformis DSM 13 |
| 374 | ycbL YcbL | Hypothetical protein [Bacillus licheniformis DSM 13] | UniRef100_Q65NW5 | Bacillus licheniformis DSM 13 |
| 375 | ycbM putative sensor histidine kinase | HPr serine phosphorylation site [Bacillus licheniformis DSM 13] | UniRef100_Q62ZB0 | Bacillus licheniformis DSM 13 |
| 376 | ycbN ABC transporter YcbN | YcbN [Bacillus licheniformis DSM 13] | UniRef100_Q65NW2 | Bacillus licheniformis DSM 13 |
| 377 | ycbO conserved hypothetical protein YcbO | YcbO [Bacillus licheniformis DSM 13] | UniRef100_Q65NW1 | Bacillus licheniformis DSM 13 |
| 378 | ycbO2 conserved hypothetical protein YcbO | | | |
| 379 | yetN conserved hypothetical protein YetN | Hypothetical protein yetN [Bacillus licheniformis DSM 13] | UniRef100_Q65NV9 | Bacillus licheniformis DSM 13 |
| 380 | ybdO conserved hypothetical protein YbdO | YbdO [Bacillus licheniformis DSM 13] | UniRef100_Q65NV8 | Bacillus licheniformis DSM 13 |
| 381 | BLP00347 hypothetical protein | | | |
| 382 | ycbJ putative Macrolide 2'-phosphotransferase | YcbJ [Bacillus licheniformis DSM 13] | UniRef100_Q65NV7 | Bacillus licheniformis DSM 13 |
| 383 | ywhA putative transcriptional regulator | YwhA [Bacillus licheniformis DSM 13] | UniRef100_Q65NV6 | Bacillus licheniformis DSM 13 |
| 384 | ydaB AMP-dependent synthetase and ligase | YdaB [Bacillus licheniformis DSM 13] | UniRef100_Q65NV5 | Bacillus licheniformis DSM 13 |
| 385 | BLP00351 putative Proteinase inhibititor I4, serpin | Hypothetical protein [Bacillus licheniformis DSM 13] | UniRef100_Q65NV4 | Bacillus licheniformis DSM 13 |

| 386 | BLP00352 unassigned | YbdN [Bacillus licheniformis DSM 13] | UniRef100_Q65NV3 | Bacillus licheniformis DSM 13 |
|---|---|---|---|---|
| 387 | BLP00353 hypothetical protein | Hypothetical protein [Bacillus licheniformis DSM 13] | UniRef100_Q65NV2 | Bacillus licheniformis DSM 13 |
| 388 | mtaB transcriptional regulator (MerR family) | Mta [Bacillus licheniformis DSM 13] | UniRef100_Q65NV1 | Bacillus licheniformis DSM 13 |
| 389 | BLP00355 hypothetical protein | Hypothetical protein [Bacillus licheniformis DSM 13] | UniRef100_Q65NV0 | Bacillus licheniformis DSM 13 |
| 390 | BLP00356 putative DNA binding protein | Hypothetical protein [Bacillus licheniformis DSM 13] | UniRef100_Q65NU9 | Bacillus licheniformis DSM 13 |
| 391 | BLP00357 hypothetical protein | Hypothetical protein [Bacillus licheniformis DSM 13] | UniRef100_Q65NU8 | Bacillus licheniformis DSM 13 |
| 392 | rtpA inhibitor of TRAP, regulated by T-BOX (trp) sequence RtpA | YczA [Bacillus licheniformis DSM 13] | UniRef100_Q65NU7 | Bacillus licheniformis DSM 13 |
| 393 | ycbK hypothetical membrane protein YcbK | YcbK [Bacillus licheniformis DSM 13] | UniRef100_Q65NU6 | Bacillus licheniformis DSM 13 |
| 394 | yczC conserved membrane protein YczC | RDD [Bacillus licheniformis DSM 13] | UniRef100_Q62Z92 | Bacillus licheniformis DSM 13 |
| 395 | yccF conserved hypothetical protein YccF | YccF [Bacillus licheniformis DSM 13] | UniRef100_Q65NU4 | Bacillus licheniformis DSM 13 |
| 396 | spaF ABC transporter SpaF | Hypothetical protein [Bacillus licheniformis DSM 13] | UniRef100_Q65NU3 | Bacillus licheniformis DSM 13 |
| 397 | spaE SpaE | Hypothetical protein [Bacillus licheniformis DSM 13] | UniRef100_Q65NU2 | Bacillus licheniformis DSM 13 |
| 398 | spaG SpaG | Hypothetical protein [Bacillus licheniformis DSM 13] | UniRef100_Q65NU1 | Bacillus licheniformis DSM 13 |
| 399 | spaR SpaR | Hypothetical protein [Bacillus licheniformis DSM 13] | UniRef100_Q65NU0 | Bacillus licheniformis DSM 13 |
| 400 | spaK two-component sensor histidine kinase SpaK | Hypothetical protein [Bacillus licheniformis DSM 13] | UniRef100_Q65NT9 | Bacillus licheniformis DSM 13 |
| 401 | ytlI transcription regulatory protein YtlI | YtlI [Bacillus licheniformis DSM 13] | UniRef100_Q65NT8 | Bacillus licheniformis DSM 13 |
| 402 | yusQ 4-oxalocrotonate tautomerase YusQ | YusQ [Bacillus licheniformis DSM 13] | UniRef100_Q65NT7 | Bacillus licheniformis DSM 13 |
| 403 | yusR putative Short-chain dehydrogenase_reductase YusR | Hypothetical protein [Bacillus licheniformis DSM 13] | UniRef100_Q65NT6 | Bacillus licheniformis DSM 13 |
| 404 | BLP00370 putative methyltransferase | Hypothetical protein (Hypothetical SAM (And some other nucleotide) binding motif,Generic methyltransferase) [Bacillus licheniformis DSM 13] | UniRef100_Q65NT5 | Bacillus licheniformis DSM 13 |
| 405 | ycdA putative lipoprotein YcdA | YcdA [Bacillus licheniformis DSM 13] | UniRef100_Q65NT4 | Bacillus licheniformis DSM 13 |
| 406 | ycgA C4-dicarboxylate anaerobic carrier YcgA | YcgA [Bacillus licheniformis DSM 13] | UniRef100_Q65NT3 | Bacillus licheniformis DSM 13 |
| 407 | hipO Peptidase M20D, amidohydrolase | Hypothetical protein [Bacillus licheniformis DSM 13] | UniRef100_Q65NT2 | Bacillus licheniformis DSM 13 |
| 408 | yvbK GCN5-related N-acetyltransferase | YvbK [Bacillus licheniformis DSM 13] | UniRef100_Q65NT1 | Bacillus licheniformis DSM 13 |
| 409 | BLP00375 glutamyl-tRNA(Gln) amidotransferase (subunit A) | Hypothetical protein [Bacillus licheniformis DSM 13] | UniRef100_Q65NT0 | Bacillus licheniformis DSM 13 |
| 410 | BLP00376 neutral zinc metallopeptidase | Hypothetical protein [Bacillus licheniformis DSM 13] | UniRef100_Q65NS9 | Bacillus licheniformis DSM 13 |
| 411 | BLP00377 conserved membrane protein | Hypothetical protein [Bacillus licheniformis DSM 13] | UniRef100_Q65NS8 | Bacillus licheniformis DSM 13 |
| 412 | BLP00378 penicillin-binding protein | Hypothetical protein [Bacillus licheniformis DSM 13] | UniRef100_Q65NS7 | Bacillus licheniformis DSM 13 |
| 413 | ysfD 4Fe-4S ferredoxin, iron-sulfur binding domain | YsfD [Bacillus licheniformis DSM 13] | UniRef100_Q65NS6 | Bacillus licheniformis DSM 13 |
| 414 | ysfC Glycolate oxidase subunit GlcD | YsfC [Bacillus licheniformis DSM 13] | UniRef100_Q65NS5 | Bacillus licheniformis DSM 13 |
| 415 | BLP00381 hypothetical protein | | | |
| 416 | ydhM Phosphotransferase system, lactose_cellobiose-specific IIB subunit | Hypothetical protein [Bacillus licheniformis DSM 13] | UniRef100_Q65NS4 | Bacillus licheniformis DSM 13 |

| | | | | |
|---|---|---|---|---|
| 417 | BLP00383 phosphotransferase system (PTS) lichenan-specific enzyme IIA component | Hypothetical protein (Phosphotransferase system (PTS) lichenan- specific enzyme IIA component) [Bacillus licheniformis DSM 13] | UniRef100_Q65NS3 | Bacillus licheniformis DSM 13 |
| 418 | BLP00384 PTS lactose_cellobiose IIC component,PTS system cellobiose-specific IIC component | YwbA [Bacillus licheniformis DSM 13] | UniRef100_Q65NS2 | Bacillus licheniformis DSM 13 |
| 419 | BLP00385 Glycoside Hydrolase Family 4 | Hypothetical protein [Bacillus licheniformis DSM 13] | UniRef100_Q65NS1 | Bacillus licheniformis DSM 13 |
| 420 | BLP00386 transcriptional regulator,GntR family, conserved hypothetical protein | YurK [Bacillus licheniformis DSM 13] | UniRef100_Q65NS0 | Bacillus licheniformis DSM 13 |
| 421 | BLP00387 conserved hypothetical protein | Hypothetical protein [Bacillus licheniformis DSM 13] | UniRef100_Q65NR9 | Bacillus licheniformis DSM 13 |
| 422 | BLP00388 hypothetical protein | | | |
| 423 | yvbX conserved protein, Glycoside Hydrolase Family 18, YvbX | Hypothetical protein [Bacillus licheniformis DSM 13] | UniRef100_Q65NR8 | Bacillus licheniformis DSM 13 |
| 424 | BLP00390 Chitinase precursor, Glycoside Hydrolase Family 18 | Chitodextrinase [Bacillus licheniformis] | UniRef100_Q65NR7 | Bacillus licheniformis |
| 425 | mpr Glutamyl Endo peptidase | Glutamyl endopeptidase precursor [Bacillus licheniformis] | UniRef100_P80057 | Bacillus licheniformis |
| 426 | ycdF Glucose 1-dehydrogenase II YcdF | YcdF [Bacillus licheniformis DSM 13] | UniRef100_Q65NR5 | Bacillus licheniformis DSM 13 |
| 427 | BLP00393 conserved hypothetical protein | Hypothetical protein [Bacillus licheniformis DSM 13] | UniRef100_Q65NR4 | Bacillus licheniformis DSM 13 |
| 428 | ycdC conserved hypothetical protein YcdC | YcdC [Bacillus licheniformis DSM 13] | UniRef100_Q65NR3 | Bacillus licheniformis DSM 13 |
| 429 | BLP00395 Thiosulfate sulfurtransferase | Hypothetical protein [Bacillus licheniformis DSM 13] | UniRef100_Q65NR2 | Bacillus licheniformis DSM 13 |
| 430 | cwlJ cell wall hydrolase | Hypothetical protein cwlJ [Bacillus licheniformis DSM 13] | UniRef100_Q65NR1 | Bacillus licheniformis DSM 13 |
| 431 | yceB putative monooxygenase YceB | Luciferase-like [Bacillus licheniformis DSM 13] | UniRef100_Q62Z57 | Bacillus licheniformis DSM 13 |
| 432 | BLP00398 hypothetical protein | | | |
| 433 | yvcE putative peptidoglycan hydrolase, DL-endopeptidase II family | YvcE [Bacillus licheniformis DSM 13] | UniRef100_Q65NQ9 | Bacillus licheniformis DSM 13 |
| 434 | BLP00400 putative permease | Hypothetical protein [Bacillus licheniformis DSM 13] | UniRef100_Q65NQ8 | Bacillus licheniformis DSM 13 |
| 435 | BLP00401 ABC transporter | Hypothetical protein [Bacillus licheniformis DSM 13] | UniRef100_Q65NQ7 | Bacillus licheniformis DSM 13 |
| 436 | BLP00402 transcriptional regulator | Hypothetical protein [Bacillus licheniformis DSM 13] | UniRef100_Q65NQ6 | Bacillus licheniformis DSM 13 |
| 437 | BLP00403 hypothetical protein | Hypothetical protein [Bacillus licheniformis DSM 13] | UniRef100_Q65NQ5 | Bacillus licheniformis DSM 13 |
| 438 | BLP00404 hypothetical protein | Hypothetical protein [Bacillus licheniformis DSM 13] | UniRef100_Q65NQ4 | Bacillus licheniformis DSM 13 |
| 439 | BLP00405 transcriptional regulator | Hypothetical protein [Bacillus licheniformis DSM 13] | UniRef100_Q65NQ3 | Bacillus licheniformis DSM 13 |
| 440 | yceC putative stress response protein YceC | YceC [Bacillus licheniformis DSM 13] | UniRef100_Q65NQ2 | Bacillus licheniformis DSM 13 |
| 441 | yceD putative stress response protein YceD | YceD [Bacillus licheniformis DSM 13] | UniRef100_Q65NQ1 | Bacillus licheniformis DSM 13 |
| 442 | yceE putative stress response protein YceE | YceE [Bacillus licheniformis DSM 13] | UniRef100_Q65NQ0 | Bacillus licheniformis DSM 13 |
| 443 | yceF Integral membrane protein TerC family, YceF | YceF [Bacillus licheniformis DSM 13] | UniRef100_Q65NP9 | Bacillus licheniformis DSM 13 |
| 444 | BLP00410 Putative ATP_GTP-binding protein | Hypothetical protein [Bacillus licheniformis DSM 13] | UniRef100_Q65NP8 | Bacillus licheniformis DSM 13 |
| 445 | BLP00411 conserved hypothetical protein | Hypothetical protein [Bacillus licheniformis DSM 13] | UniRef100_Q65NP7 | Bacillus licheniformis DSM 13 |
| 446 | yceG conserved hypothetical protein YceG | YceG [Bacillus licheniformis DSM 13] | UniRef100_Q65NP6 | Bacillus licheniformis DSM 13 |
| 447 | yceH putative signal peptide binding protein YceH | YceH (Signal peptide binding (SRP54) M-domain) [Bacillus licheniformis DSM 13] | UniRef100_Q65NP5 | Bacillus licheniformis DSM 13 |

| | | | | |
|---|---|---|---|---|
| 448 | BLP00414 integral membrane protein | Hypothetical protein [Bacillus licheniformis DSM 13] | UniRef100_Q65NP4 | Bacillus licheniformis DSM 13 |
| 449 | BLP00415 conserved hypothetical protein | Hypothetical protein [Bacillus licheniformis DSM 13] | UniRef100_Q65NP3 | Bacillus licheniformis DSM 13 |
| 450 | mtaC transcriptional regulator | Hypothetical protein [Bacillus licheniformis DSM 13] | UniRef100_Q65NP2 | Bacillus licheniformis DSM 13 |
| 451 | nasA nitrate transporter | NasA [Bacillus licheniformis DSM 13] | UniRef100_Q65NP1 | Bacillus licheniformis DSM 13 |
| 452 | ldh L-lactate dehydrogenase | Ldh [Bacillus licheniformis DSM 13] | UniRef100_Q65NP0 | Bacillus licheniformis DSM 13 |
| 453 | lctP L-lactate permease | LctP [Bacillus licheniformis DSM 13] | UniRef100_Q65NN9 | Bacillus licheniformis DSM 13 |
| 454 | ycgF Lysine exporter protein YcgF | YcgF [Bacillus licheniformis DSM 13] | UniRef100_Q65NN8 | Bacillus licheniformis DSM 13 |
| 455 | ycgH Amino acid_polyamine transporter I YcgH | YcgH [Bacillus licheniformis DSM 13] | UniRef100_Q65NN7 | Bacillus licheniformis DSM 13 |
| 456 | nadE NH3-dependent NAD synthetase | NadE [Bacillus licheniformis DSM 13] | UniRef100_Q65NN6 | Bacillus licheniformis DSM 13 |
| 457 | aroK shikimate kinase | AroK [Bacillus licheniformis DSM 13] | UniRef100_Q65NN5 | Bacillus licheniformis DSM 13 |
| 458 | ycgL conserved hypothetical protein YcgL | YcgL [Bacillus licheniformis DSM 13] | UniRef100_Q65NN4 | Bacillus licheniformis DSM 13 |
| 459 | ycgM putative proline dehydrogenase YcgM | YcgM [Bacillus licheniformis DSM 13] | UniRef100_Q65NN3 | Bacillus licheniformis DSM 13 |
| 460 | ycgN delta-1-pyrroline-5-carboxylate dehydrogenase 2 YcgN | YcgN [Bacillus licheniformis DSM 13] | UniRef100_Q65NN2 | Bacillus licheniformis DSM 13 |
| 461 | ycgO Na _solute symporter YcgO | Na+/solute symporter [Bacillus licheniformis DSM 13] | UniRef100_Q62Z29 | Bacillus licheniformis DSM 13 |
| 462 | ycgP YcgP | YcgP [Bacillus licheniformis DSM 13] | UniRef100_Q65NN0 | Bacillus licheniformis DSM 13 |
| 463 | ycgQ YcgQ | YcgQ [Bacillus licheniformis DSM 13] | UniRef100_Q65NM9 | Bacillus licheniformis DSM 13 |
| 464 | ycgR putative permease YcgR | Predicted permease [Bacillus licheniformis DSM 13] | UniRef100_Q62Z26 | Bacillus licheniformis DSM 13 |
| 465 | cah cephalosporin C deacetylase, Carbohydrate Esterase Family 7 | Cah [Bacillus licheniformis DSM 13] | UniRef100_Q65NM7 | Bacillus licheniformis DSM 13 |
| 466 | BLP00432 putative transcriptional regulator | Hypothetical protein [Bacillus licheniformis DSM 13] | UniRef100_Q65NM6 | Bacillus licheniformis DSM 13 |
| 467 | BLP00433 conserved hypothetical protein | Hypothetical protein [Bacillus licheniformis DSM 13] | UniRef100_Q65NM5 | Bacillus licheniformis DSM 13 |
| 468 | BLP00434 hypothetical protein | Hypothetical protein [Bacillus licheniformis DSM 13] | UniRef100_Q65NM4 | Bacillus licheniformis DSM 13 |
| 469 | BLP00435 hypothetical protein | | | |
| 470 | yckA Amino acid ABC transporter, permease protein YckA | YckA [Bacillus licheniformis DSM 13] | UniRef100_Q65NM3 | Bacillus licheniformis DSM 13 |
| 471 | yckB putative extracellular solute-binding protein YckB | YckB [Bacillus licheniformis DSM 13] | UniRef100_Q65NM2 | Bacillus licheniformis DSM 13 |
| 472 | BLP00438 hypothetical protein | | | |
| 473 | BLP00439 NAD(P)H dehydrogenase | Hypothetical protein (NAD(P)H dehydrogenase) [Bacillus licheniformis DSM 13] | UniRef100_Q65NM1 | Bacillus licheniformis DSM 13 |
| 474 | BLP00440 ABC transporter | Hypothetical protein [Bacillus licheniformis DSM 13] | UniRef100_Q65NM0 | Bacillus licheniformis DSM 13 |
| 475 | yckE putative Glycosyl Hydrolase Family 1 YckE | YckE [Bacillus licheniformis DSM 13] | UniRef100_Q65NL9 | Bacillus licheniformis DSM 13 |
| 476 | nin Nin | Hypothetical protein nin [Bacillus licheniformis DSM 13] | UniRef100_Q62Z16 | Bacillus licheniformis DSM 13 |
| 477 | nucA nuclease NucA | NucA [Bacillus licheniformis DSM 13] | UniRef100_Q65NL7 | Bacillus licheniformis DSM 13 |
| 478 | BLP00444 hypothetical protein | | | |
| 479 | tlpC Methyl-accepting chemotaxis protein tlpC TlpC | | | |
| 480 | ycqG Endoplasmic reticulum targeting sequence YcqG | Hypothetical protein [Bacillus licheniformis DSM 13] | UniRef100_Q65NL2 | Bacillus licheniformis DSM 13 |
| 481 | BLP00447 Hydantoin utilization protein A | Hypothetical protein [Bacillus licheniformis DSM 13] | UniRef100_Q65NL1 | Bacillus licheniformis DSM 13 |

| | | | | |
|---|---|---|---|---|
| 482 | BLP00448 Hydantoin utilization protein B | Hypothetical protein [Bacillus licheniformis DSM 13] | UniRef100_Q65NL0 | Bacillus licheniformis DSM 13 |
| 483 | BLP00449 putative extracellular solute-binding protein | Extracellular solute-binding protein, family 1 [Bacillus licheniformis DSM 13] | UniRef100_Q62Z10 | Bacillus licheniformis DSM 13 |
| 484 | BLP00450 multiple sugar-binding transport ATP-binding protein | Hypothetical protein [Bacillus licheniformis DSM 13] | UniRef100_Q65NK8 | Bacillus licheniformis DSM 13 |
| 485 | BLP00451 Binding-protein-dependent transport systems inner membrane component | Hypothetical protein [Bacillus licheniformis DSM 13] | UniRef100_Q65NK7 | Bacillus licheniformis DSM 13 |
| 486 | BLP00452 Binding-protein-dependent transport systems inner membrane component | Hypothetical protein [Bacillus licheniformis DSM 13] | UniRef100_Q65NK6 | Bacillus licheniformis DSM 13 |
| 487 | BLP00453 hypothetical protein | | | |
| 488 | BLP04698 hypothetical protein | | | |
| 489 | lchAA lichenysin synthetase A | Lichenysin synthetase A [Bacillus licheniformis DSM 13] | UniRef100_Q65NK5 | Bacillus licheniformis DSM 13 |
| 490 | lchAB lichenysin synthetase B | Lichenysin synthetase B [Bacillus licheniformis DSM 13] | UniRef100_Q65NK4 | Bacillus licheniformis DSM 13 |
| 491 | comS ComS | | | |
| 492 | lchAC lichenysin synthetase C | Lichenysin synthetase C [Bacillus licheniformis DSM 13] | UniRef100_Q65NK3 | Bacillus licheniformis DSM 13 |
| 493 | lchAD thioesterase LchAD | Lichenysin synthetase D [Bacillus licheniformis DSM 13] | UniRef100_Q65NK2 | Bacillus licheniformis DSM 13 |
| 494 | ycxC conserved membrane protein YcxC | Hypothetical protein ycxC [Bacillus licheniformis DSM 13] | UniRef100_Q65NK1 | Bacillus licheniformis DSM 13 |
| 495 | ycxD probable transcriptional regulator YcxD | YcxD [Bacillus licheniformis DSM 13] | UniRef100_Q65NK0 | Bacillus licheniformis DSM 13 |
| 496 | sfp phosphopantetheinyl transferase | Hypothetical protein [Bacillus licheniformis DSM 13] | UniRef100_Q65NJ9 | Bacillus licheniformis DSM 13 |
| 497 | ybbA Putative carbohydrate esterase, Family 1, YbbA | Hypothetical protein [Bacillus licheniformis DSM 13] | UniRef100_Q65NJ8 | Bacillus licheniformis DSM 13 |
| 498 | BLP00462 hypothetical protein | Transcriptional activator, AraC family [Bacillus cereus] | UniRef100_Q73CS4 | Bacillus cereus |
| 499 | yczE YczE | Hypothetical protein yczE [Bacillus licheniformis DSM 13] | UniRef100_Q65NJ6 | Bacillus licheniformis DSM 13 |
| 500 | BLP04844 conserved hypothetical protein | Hypothetical protein [Bacillus licheniformis DSM 13] | UniRef100_Q65NJ5 | Bacillus licheniformis DSM 13 |
| 501 | BLP00465 ABC transporter | Hypothetical protein [Bacillus licheniformis DSM 13] | UniRef100_Q65NJ4 | Bacillus licheniformis DSM 13 |
| 502 | BLP00466 putative transcriptional regulator | Hypothetical protein [Bacillus licheniformis DSM 13] | UniRef100_Q65NJ3 | Bacillus licheniformis DSM 13 |
| 503 | BLP00467 hypothetical protein | Hypothetical protein [Bacillus licheniformis DSM 13] | UniRef100_Q62YZ2 | Bacillus licheniformis DSM 13 |
| 504 | BLP00469 Putative L-2,4-diaminobutyrate decarboxylase | Hypothetical protein [Bacillus licheniformis DSM 13] | UniRef100_Q65NJ2 | Bacillus licheniformis DSM 13 |
| 505 | BLP00470 methyl-accepting chemotaxis protein | Hypothetical protein [Bacillus licheniformis DSM 13] | UniRef100_Q65NJ1 | Bacillus licheniformis DSM 13 |
| 506 | yckI ABC transporter YckI | YckI [Bacillus licheniformis DSM 13] | UniRef100_Q65NJ0 | Bacillus licheniformis DSM 13 |
| 507 | yckJ ABC transporter permease protein YckJ | Amino acid ABC transporter, permease protein, 3-TM region, His/Glu/Gln/Arg/opine,Binding-protein-dependent transport systems inner membrane component [Bacillus licheniformis DSM 13] | UniRef100_Q62YY8 | Bacillus licheniformis DSM 13 |
| 508 | yckK putative extracellular solute-binding protein, family 3 YckK | YckK [Bacillus licheniformis DSM 13] | UniRef100_Q65NI8 | Bacillus licheniformis DSM 13 |
| 509 | rocR transcriptional activator of arginine utilization operons | RocR [Bacillus licheniformis DSM 13] | UniRef100_Q65NI7 | Bacillus licheniformis DSM 13 |
| 510 | rocD ornithine aminotransferase | RocD [Bacillus licheniformis DSM 13] | UniRef100_Q65NI6 | Bacillus licheniformis DSM 13 |
| 511 | rocE amino acid permease | RocE [Bacillus licheniformis DSM 13] | UniRef100_Q65NI5 | Bacillus licheniformis DSM 13 |

| | | | | |
|---|---|---|---|---|
| 512 | rocF arginase | RocF [Bacillus licheniformis DSM 13] | UniRef100_Q65NI4 | Bacillus licheniformis DSM 13 |
| 513 | yclA transcriptional regulator YclA | YclA [Bacillus licheniformis DSM 13] | UniRef100_Q65NI3 | Bacillus licheniformis DSM 13 |
| 514 | yclB 3-octaprenyl-4-hydroxybenzoate carboxy-lyase YclB | YclB [Bacillus licheniformis DSM 13] | UniRef100_Q65NI2 | Bacillus licheniformis DSM 13 |
| 515 | yclC Carboxylyase-related protein YclC | YclC [Bacillus licheniformis DSM 13] | UniRef100_Q65NI1 | Bacillus licheniformis DSM 13 |
| 516 | BLP00481 hypothetical protein | Hypothetical protein [Bacillus licheniformis DSM 13] | UniRef100_Q65NI0 | Bacillus licheniformis DSM 13 |
| 517 | BLP00482 hypothetical protein | Hypothetical protein [Bacillus licheniformis DSM 13] | UniRef100_Q65NH9 | Bacillus licheniformis DSM 13 |
| 518 | BLP00483 hypothetical protein | Hypothetical protein [Bacillus licheniformis DSM 13] | UniRef100_Q65NH8 | Bacillus licheniformis DSM 13 |
| 519 | BLP00484 hypothetical protein | Hypothetical protein [Bacillus licheniformis DSM 13] | UniRef100_Q65NH7 | Bacillus licheniformis DSM 13 |
| 520 | BLP00485 hypothetical protein | Hypothetical protein [Bacillus licheniformis DSM 13] | UniRef100_Q65NH6 | Bacillus licheniformis DSM 13 |
| 521 | BLP00486 hypothetical protein | | | |
| 522 | BLP00487 hypothetical protein | Hypothetical protein [Bacillus licheniformis DSM 13] | UniRef100_Q65NH5 | Bacillus licheniformis DSM 13 |
| 523 | BLP00488 conserved hypothetical protein | Hypothetical protein [Bacillus licheniformis DSM 13] | UniRef100_Q65NH4 | Bacillus licheniformis DSM 13 |
| 524 | BLP00489 hypothetical protein | | | |
| 525 | BLP00490 hypothetical protein | Hypothetical protein [Bacillus licheniformis DSM 13] | UniRef100_Q65NH3 | Bacillus licheniformis DSM 13 |
| 526 | yxiD putative transposase protein YxiD | Hypothetical protein [Bacillus licheniformis DSM 13] | UniRef100_Q65NH2 | Bacillus licheniformis DSM 13 |
| 527 | yxiC YxiC | Hypothetical protein yxiC [Bacillus licheniformis DSM 13] | UniRef100_Q62YX1 | Bacillus licheniformis DSM 13 |
| 528 | yxiB putative prefoldin | Hypothetical protein [Bacillus licheniformis DSM 13] | UniRef100_Q65NH0 | Bacillus licheniformis DSM 13 |
| 529 | BLP00494 ribose ABC transporter (ribose-binding protein) | Hypothetical protein [Bacillus licheniformis DSM 13] | UniRef100_Q65NG9 | Bacillus licheniformis DSM 13 |
| 530 | BLP00495 putative Histidine kinase | Hypothetical protein [Bacillus licheniformis DSM 13] | UniRef100_Q65NG8 | Bacillus licheniformis DSM 13 |
| 531 | BLP00496 transcriptional regulator | Hypothetical protein [Bacillus licheniformis DSM 13] | UniRef100_Q65NG7 | Bacillus licheniformis DSM 13 |
| 532 | BLP00497 ribose ABC transporter (ribose-binding protein) | Hypothetical protein [Bacillus licheniformis DSM 13] | UniRef100_Q65NG6 | Bacillus licheniformis DSM 13 |
| 533 | BLP00498 sugar ABC transporter (ATP-binding protein) | Hypothetical protein [Bacillus licheniformis DSM 13] | UniRef100_Q65NG5 | Bacillus licheniformis DSM 13 |
| 534 | BLP00499 sugar ABC transporter (permease) | Hypothetical protein [Bacillus licheniformis DSM 13] | UniRef100_Q65NG4 | Bacillus licheniformis DSM 13 |
| 535 | ybfB Major facilitator superfamily protein YbfB | YbfB [Bacillus licheniformis DSM 13] | UniRef100_Q65NG3 | Bacillus licheniformis DSM 13 |
| 536 | ybfA putative DNA binding protein YbfA | YbfA [Bacillus licheniformis DSM 13] | UniRef100_Q65NG2 | Bacillus licheniformis DSM 13 |
| 537 | BLP00502 hypothetical protein | | | |
| 538 | lacA2 Glycoside Hydrolase Family 42 | Hypothetical protein [Bacillus licheniformis DSM 13] | UniRef100_Q65NG1 | Bacillus licheniformis DSM 13 |
| 539 | phy phytase | PhyL [Bacillus licheniformis] | UniRef100_Q6DNH6 | Bacillus licheniformis |
| 540 | yclF putative oligo-peptide transporter YclF | YclF [Bacillus licheniformis DSM 13] | UniRef100_Q65NF9 | Bacillus licheniformis DSM 13 |
| 541 | BLP00506 conserved hypothetical protein | Hypothetical protein [Bacillus licheniformis DSM 13] | UniRef100_Q65NF8 | Bacillus licheniformis DSM 13 |
| 542 | yclG Pectin lyase-like protein | YclG [Bacillus licheniformis DSM 13] | UniRef100_Q65NF7 | Bacillus licheniformis DSM 13 |
| 543 | BLP00508 hypothetical protein | YczF [Bacillus licheniformis DSM 13] | UniRef100_Q65NF6 | Bacillus licheniformis DSM 13 |
| 544 | BLP00509 hypothetical protein | GerKA [Bacillus licheniformis DSM 13] | UniRef100_Q65NF5 | Bacillus licheniformis DSM 13 |
| 545 | gerKC GerKC | Hypothetical protein gerKC [Bacillus licheniformis DSM 13] | UniRef100_Q65NF4 | Bacillus licheniformis DSM 13 |

| 546 | gerKB GerKC | Hypothetical protein gerKB [Bacillus licheniformis DSM 13] | UniRef100_Q65NF3 | Bacillus licheniformis DSM 13 |
|---|---|---|---|---|
| 547 | mtaD transcriptional regulator (MerR family) | Hypothetical protein [Bacillus licheniformis DSM 13] | UniRef100_Q65NF2 | Bacillus licheniformis DSM 13 |
| 548 | yclH ABC transporter YclH | YclH [Bacillus licheniformis DSM 13] | UniRef100_Q65NF1 | Bacillus licheniformis DSM 13 |
| 549 | yclI hypothetical membrane protein YclI | YclI [Bacillus licheniformis DSM 13] | UniRef100_Q65NF0 | Bacillus licheniformis DSM 13 |
| 550 | yclJ hypothetical sensory transduction protein YclJ | YclJ [Bacillus licheniformis DSM 13] | UniRef100_Q65NE9 | Bacillus licheniformis DSM 13 |
| 551 | yclK Histidine kinase, homodimeric YclK | YclK [Bacillus licheniformis DSM 13] | UniRef100_Q65NE8 | Bacillus licheniformis DSM 13 |
| 552 | tlpA methyl-accepting chemotaxis protein | TlpA [Bacillus licheniformis DSM 13] | UniRef100_Q65NE7 | Bacillus licheniformis DSM 13 |
| 553 | BLP00518 hypothetical protein | | | |
| 554 | yclM Aspartate kinase YclM | YclM [Bacillus licheniformis DSM 13] | UniRef100_Q65NE6 | Bacillus licheniformis DSM 13 |
| 555 | yclN putative  transport system permease protein | YclN [Bacillus licheniformis DSM 13] | UniRef100_Q65NE5 | Bacillus licheniformis DSM 13 |
| 556 | yclO putative transport system permease protein | YclO [Bacillus licheniformis DSM 13] | UniRef100_Q65NE4 | Bacillus licheniformis DSM 13 |
| 557 | yclP ABC transporter | YclP [Bacillus licheniformis DSM 13] | UniRef100_Q65NE3 | Bacillus licheniformis DSM 13 |
| 558 | yclQ Periplasmic binding protein | YclQ [Bacillus licheniformis DSM 13] | UniRef100_Q65NE2 | Bacillus licheniformis DSM 13 |
| 559 | ycnB Drug resistance transporter YcnB | YcnB [Bacillus licheniformis DSM 13] | UniRef100_Q65NE1 | Bacillus licheniformis DSM 13 |
| 560 | ycnC probable transcriptional regulator YcnC | YcnC [Bacillus licheniformis DSM 13] | UniRef100_Q65NE0 | Bacillus licheniformis DSM 13 |
| 561 | BLP00526 hypothetical protein | Hypothetical protein [Bacillus licheniformis DSM 13] | UniRef100_Q65ND9 | Bacillus licheniformis DSM 13 |
| 562 | nfrA2 NADPH-linked nitro_flavin reductase | YcnD [Bacillus licheniformis DSM 13] | UniRef100_Q65ND8 | Bacillus licheniformis DSM 13 |
| 563 | ycnE Dimeric alpha-beta barrel | YcnE [Bacillus licheniformis DSM 13] | UniRef100_Q65ND7 | Bacillus licheniformis DSM 13 |
| 564 | yczG putative transcriptional regulator YczG | YczG [Bacillus licheniformis DSM 13] | UniRef100_Q65ND6 | Bacillus licheniformis DSM 13 |
| 565 | gabR transcriptional regulator GabR | GabR [Bacillus licheniformis DSM 13] | UniRef100_Q65ND5 | Bacillus licheniformis DSM 13 |
| 566 | gabT 4-aminobutyrate aminotransferase | GabT [Bacillus licheniformis DSM 13] | UniRef100_Q65ND4 | Bacillus licheniformis DSM 13 |
| 567 | BLP00532 Amino acid_polyamine transporter I | YhdG [Bacillus licheniformis DSM 13] | UniRef100_Q65ND3 | Bacillus licheniformis DSM 13 |
| 568 | gabD succinate-semialdehyde dehydrogenase | GabD [Bacillus licheniformis DSM 13] | UniRef100_Q65ND2 | Bacillus licheniformis DSM 13 |
| 569 | ywfM conserved hypothetical protein YwfM | YwfM [Bacillus licheniformis DSM 13] | UniRef100_Q65ND1 | Bacillus licheniformis DSM 13 |
| 570 | BLP04699 hypothetical protein | | | |
| 571 | BLP00534 hypothetical protein | Hypothetical protein [Bacillus licheniformis DSM 13] | UniRef100_Q65ND0 | Bacillus licheniformis DSM 13 |
| 572 | ycnI conserved membrane protein | YcnI [Bacillus licheniformis DSM 13] | UniRef100_Q65NC9 | Bacillus licheniformis DSM 13 |
| 573 | ycnJ putative copper export protein YcnJ | YcnJ [Bacillus licheniformis DSM 13] | UniRef100_Q65NC8 | Bacillus licheniformis DSM 13 |
| 574 | ycnK probable transcriptional regulator YcnK | YcnK [Bacillus licheniformis DSM 13] | UniRef100_Q65NC7 | Bacillus licheniformis DSM 13 |
| 575 | BLP00538 hypothetical protein | | | |
| 576 | nasB assimilatory nitrate reductase (electron transfer subunit) | NasB [Bacillus licheniformis DSM 13] | UniRef100_Q65NC6 | Bacillus licheniformis DSM 13 |
| 577 | nasC assimilatory nitrate reductase (catalytic subunit) | NasC [Bacillus licheniformis DSM 13] | UniRef100_Q65NC5 | Bacillus licheniformis DSM 13 |
| 578 | nasD assimilatory nitrite reductase (subunit) | NasD [Bacillus licheniformis DSM 13] | UniRef100_Q65NC4 | Bacillus licheniformis DSM 13 |
| 579 | nasE assimilatory nitrite reductase (subunit) | NasE [Bacillus licheniformis DSM 13] | UniRef100_Q65NC3 | Bacillus licheniformis DSM 13 |
| 580 | nasF uroporphyrin-III C-methyltransferase | NasF [Bacillus licheniformis DSM 13] | UniRef100_Q65NC2 | Bacillus licheniformis DSM 13 |

| 581 | BLP04700 hypothetical protein | | | |
|---|---|---|---|---|
| 582 | BLP00544 putative regulatory protein | YdhC [Bacillus licheniformis DSM 13] | UniRef100_Q65NC1 | Bacillus licheniformis DSM 13 |
| 583 | BLP00545 Sodium_sulphate symporter | Hypothetical protein [Bacillus licheniformis DSM 13] | UniRef100_Q65NC0 | Bacillus licheniformis DSM 13 |
| 584 | BLP00546 hypothetical protein | Hypothetical protein [Bacillus licheniformis DSM 13] | UniRef100_Q65NB9 | Bacillus licheniformis DSM 13 |
| 585 | BLP00547 conserved hypothetical protein | Hypothetical protein [Bacillus licheniformis DSM 13] | UniRef100_Q65NB7 | Bacillus licheniformis DSM 13 |
| 586 | BLP00548 transcriptional regulator | Hypothetical protein [Bacillus licheniformis DSM 13] | UniRef100_Q65NB6 | Bacillus licheniformis DSM 13 |
| 587 | BLP00549 putative sugar phosphotransferase | Hypothetical protein [Bacillus licheniformis DSM 13] | UniRef100_Q65NB5 | Bacillus licheniformis DSM 13 |
| 588 | BLP00550 putative sugar phosphotransferase system protein | Hypothetical protein [Bacillus licheniformis DSM 13] | UniRef100_Q65NB4 | Bacillus licheniformis DSM 13 |
| 589 | tktA transketolase | Hypothetical protein [Bacillus licheniformis DSM 13] | UniRef100_Q65NB3 | Bacillus licheniformis DSM 13 |
| 590 | BLP00552 1-deoxyxylulose-5-phosphate synthase | Hypothetical protein [Bacillus licheniformis DSM 13] | UniRef100_Q65NB2 | Bacillus licheniformis DSM 13 |
| 591 | ycsE putative hydrolase YcsE | YcsE [Bacillus licheniformis DSM 13] | UniRef100_Q65NB1 | Bacillus licheniformis DSM 13 |
| 592 | BLP00554 hypothetical protein | | | |
| 593 | ycsF putative lactam utilization protein YcsF | YcsF [Bacillus licheniformis DSM 13] | UniRef100_Q65NB0 | Bacillus licheniformis DSM 13 |
| 594 | ycsG putative branched chain amino acids transporter YcsG | Hypothetical protein ycsG [Bacillus licheniformis DSM 13] | UniRef100_Q65NA9 | Bacillus licheniformis DSM 13 |
| 595 | ycsI conserved protein YcsI | Hypothetical protein ycsI [Bacillus licheniformis DSM 13] | UniRef100_Q65NA8 | Bacillus licheniformis DSM 13 |
| 596 | kipI kinase inhibitor KipI | Hypothetical protein kipI [Bacillus licheniformis DSM 13] | UniRef100_Q65NA7 | Bacillus licheniformis DSM 13 |
| 597 | kipA KipA | Hypothetical protein kipA [Bacillus licheniformis DSM 13] | UniRef100_Q65NA6 | Bacillus licheniformis DSM 13 |
| 598 | kipR transcriptional regulator KipR | KipR [Bacillus licheniformis DSM 13] | UniRef100_Q65NA5 | Bacillus licheniformis DSM 13 |
| 599 | ycsK Lipolytic enzyme, G-D-S-L | YcsK [Bacillus licheniformis DSM 13] | UniRef100_Q65NA4 | Bacillus licheniformis DSM 13 |
| 600 | BLP00562 hypothetical protein | MtlA [Bacillus licheniformis DSM 13] | UniRef100_Q65NA3 | Bacillus licheniformis DSM 13 |
| 601 | mtlD mannitol-1-phosphate dehydrogenase | MtlD [Bacillus licheniformis DSM 13] | UniRef100_Q65NA1 | Bacillus licheniformis DSM 13 |
| 602 | mtlR transcriptional regulator MtlR | MtlR [Bacillus licheniformis DSM 13] | UniRef100_Q65NA0 | Bacillus licheniformis DSM 13 |
| 603 | ydaD putative Short-chain dehydrogenase_reductase YdaD | YdaD [Bacillus licheniformis DSM 13] | UniRef100_Q65N99 | Bacillus licheniformis DSM 13 |
| 604 | ydaE YdaE | YdaE [Bacillus licheniformis DSM 13] | UniRef100_Q65N98 | Bacillus licheniformis DSM 13 |
| 605 | BLP00567 hypothetical protein | Hypothetical protein [Bacillus licheniformis DSM 13] | UniRef100_Q65N97 | Bacillus licheniformis DSM 13 |
| 606 | ydaG FMN-binding split barrel domain protein YdaG | YdaG [Bacillus licheniformis DSM 13] | UniRef100_Q65N96 | Bacillus licheniformis DSM 13 |
| 607 | BLP00569 hypothetical protein | | | |
| 608 | ydaH conserved membrane protein YdaH | Hypothetical protein ydaH [Bacillus licheniformis DSM 13] | UniRef100_Q65N95 | Bacillus licheniformis DSM 13 |
| 609 | BLP00571 Cell envelope-related transcriptional attenuator | Hypothetical protein [Bacillus licheniformis DSM 13] | UniRef100_Q65N94 | Bacillus licheniformis DSM 13 |
| 610 | ydzA conserved membrane protein YdzA | Hypothetical protein ydzA [Bacillus licheniformis DSM 13] | UniRef100_Q65N93 | Bacillus licheniformis DSM 13 |
| 611 | BLP00573 hypothetical protein | Hypothetical protein [Bacillus licheniformis DSM 13] | UniRef100_Q65N92 | Bacillus licheniformis DSM 13 |
| 612 | lrpC transcriptional regulator (Lrp_AsnC family) | LrpC [Bacillus licheniformis DSM 13] | UniRef100_Q65N91 | Bacillus licheniformis DSM 13 |
| 613 | topB DNA topoisomerase III | TopB [Bacillus licheniformis DSM 13] | UniRef100_Q65N90 | Bacillus licheniformis DSM 13 |
| 614 | BLP00576 hypothetical protein | Hypothetical protein [Bacillus licheniformis DSM 13] | UniRef100_Q62YP3 | Bacillus licheniformis DSM 13 |

| 615 | BLP00577 hypothetical protein | Hypothetical protein [Bacillus licheniformis DSM 13] | UniRef100_Q62YP2 | Bacillus licheniformis DSM 13 |
|---|---|---|---|---|
| 616 | BLP04921 conserved hypothetical protein | Hypothetical protein [Bacillus licheniformis DSM 13] | UniRef100_Q62YP1 | Bacillus licheniformis DSM 13 |
| 617 | ydaO putative amino acid permease | YdaO [Bacillus licheniformis DSM 13] | UniRef100_Q65N89 | Bacillus licheniformis DSM 13 |
| 618 | BLP00579 hypothetical protein | | | |
| 619 | ydaP Pyruvate decarboxylase | YdaP [Bacillus licheniformis DSM 13] | UniRef100_Q65N88 | Bacillus licheniformis DSM 13 |
| 620 | BLP00581 putative chitin binding protein | Hypothetical protein [Bacillus licheniformis DSM 13] | UniRef100_Q65N87 | Bacillus licheniformis DSM 13 |
| 621 | BLP00582 hypothetical protein | | | |
| 622 | BLP00583 hypothetical protein | Hypothetical protein [Bacillus licheniformis DSM 13] | UniRef100_Q62YN4 | Bacillus licheniformis DSM 13 |
| 623 | ydaF GCN5-related N-acetyltransferase | YdaF [Bacillus licheniformis DSM 13] | UniRef100_Q65N83 | Bacillus licheniformis DSM 13 |
| 624 | mntH manganese transporter | MntH [Bacillus licheniformis DSM 13] | UniRef100_Q65N82 | Bacillus licheniformis DSM 13 |
| 625 | ydaS Transglycosylase-associated protein | YdaS [Bacillus licheniformis DSM 13] | UniRef100_Q65N81 | Bacillus licheniformis DSM 13 |
| 626 | ansB L-aspartase AnsB | AnsB [Bacillus licheniformis DSM 13] | UniRef100_Q65N80 | Bacillus licheniformis DSM 13 |
| 627 | BLP04701 hypothetical protein | | | |
| 628 | BLP00588 hypothetical protein | | | |
| 629 | yojK Glycosyl Transferase Family 1 | YojK [Bacillus licheniformis DSM 13] | UniRef100_Q65N79 | Bacillus licheniformis DSM 13 |
| 630 | ydaT conserved protein YdaT | Hypothetical protein ydaT [Bacillus licheniformis DSM 13] | UniRef100_Q65N78 | Bacillus licheniformis DSM 13 |
| 631 | BLP00591 hypothetical protein | | | |
| 632 | ydbA YdbA | YdbA [Bacillus licheniformis DSM 13] | UniRef100_Q65N77 | Bacillus licheniformis DSM 13 |
| 633 | BLP00593 Na _H antiporter | Hypothetical protein [Bacillus licheniformis DSM 13] | UniRef100_Q65N76 | Bacillus licheniformis DSM 13 |
| 634 | ydbB conserved protein YdbB | YdbB [Bacillus licheniformis DSM 13] | UniRef100_Q65N75 | Bacillus licheniformis DSM 13 |
| 635 | gsiB general stress protein | GsiB [Bacillus licheniformis DSM 13] | UniRef100_Q65N74 | Bacillus licheniformis DSM 13 |
| 636 | ydbI conserved membrane protein YdbI | Hypothetical protein ydbI [Bacillus licheniformis DSM 13] | UniRef100_Q65N73 | Bacillus licheniformis DSM 13 |
| 637 | BLP00597 proton_sodium-glutamate symport protein | Hypothetical protein [Bacillus licheniformis DSM 13] | UniRef100_Q65N72 | Bacillus licheniformis DSM 13 |
| 638 | ydbJ ABC transporter | YdbJ [Bacillus licheniformis DSM 13] | UniRef100_Q65N71 | Bacillus licheniformis DSM 13 |
| 639 | ydbK probable ABC transport system permease protein YdbK | Hypothetical protein ydbK [Bacillus licheniformis DSM 13] | UniRef100_Q62YM0 | Bacillus licheniformis DSM 13 |
| 640 | ydbL YdbL | Hypothetical protein ydbL [Bacillus licheniformis DSM 13] | UniRef100_Q65N69 | Bacillus licheniformis DSM 13 |
| 641 | ydbM putative Acyl-CoA dehydrogenase YdbM | YdbM [Bacillus licheniformis DSM 13] | UniRef100_Q65N68 | Bacillus licheniformis DSM 13 |
| 642 | ydbN small antisense RNA YdbN | Hypothetical protein ydbN [Bacillus licheniformis DSM 13] | UniRef100_Q65N67 | Bacillus licheniformis DSM 13 |
| 643 | BLP00603 hypothetical protein | Hypothetical protein [Bacillus licheniformis DSM 13] | UniRef100_Q62YL6 | Bacillus licheniformis DSM 13 |
| 644 | ydbP putative thiredoxin protein YdpP | YdbP [Bacillus licheniformis DSM 13] | UniRef100_Q65N66 | Bacillus licheniformis DSM 13 |
| 645 | ddl D-alanyl-D-alanine ligase A | Ddl [Bacillus licheniformis DSM 13] | UniRef100_Q65N65 | Bacillus licheniformis DSM 13 |
| 646 | murF UDP-N-acetylmuramoylalanyl-D-glutamyl-2,6-diaminopimelate-D-alanyl-D-alanine ligase | MurF [Bacillus licheniformis DSM 13] | UniRef100_Q65N64 | Bacillus licheniformis DSM 13 |
| 647 | BLP00607 Esterase_lipase_thioesterase | Hypothetical protein [Bacillus licheniformis DSM 13] | UniRef100_Q65N63 | Bacillus licheniformis DSM 13 |
| 648 | ydbR probable ATP-dependent RNA helicase YdbR | YdbR [Bacillus licheniformis DSM 13] | UniRef100_Q65N62 | Bacillus licheniformis DSM 13 |
| 649 | ydbS conserved membrane protein YdbS | YdbS [Bacillus licheniformis DSM 13] | UniRef100_Q65N61 | Bacillus licheniformis DSM 13 |

| 650 | ydbT conserved membrane protein | YdbT [Bacillus licheniformis DSM 13] | UniRef100_Q65N60 | Bacillus licheniformis DSM 13 |
|---|---|---|---|---|
| 651 | ydcA conserved membrane protein YdcA | YdcA [Bacillus licheniformis DSM 13] | UniRef100_Q65N59 | Bacillus licheniformis DSM 13 |
| 652 | ydcC conserved membrane protein YdcC | YdcC [Bacillus licheniformis DSM 13] | UniRef100_Q65N58 | Bacillus licheniformis DSM 13 |
| 653 | dal D-alanine racemase | Alr [Bacillus licheniformis DSM 13] | UniRef100_Q65N57 | Bacillus licheniformis DSM 13 |
| 654 | endoAI transcriptional inhibitor endoAI | YdcD [Bacillus licheniformis DSM 13] | UniRef100_Q65N56 | Bacillus licheniformis DSM 13 |
| 655 | endoA putative RNase | YdcE [Bacillus licheniformis DSM 13] | UniRef100_Q65N55 | Bacillus licheniformis DSM 13 |
| 656 | rsbR RbsR | Hypothetical protein rsbR [Bacillus licheniformis DSM 13] | UniRef100_Q65N54 | Bacillus licheniformis DSM 13 |
| 657 | rsbS antagonist of RsbT | RsbS [Bacillus licheniformis DSM 13] | UniRef100_Q65N53 | Bacillus licheniformis DSM 13 |
| 658 | rsbT switch protein_serine-threonine kinase | RsbT [Bacillus licheniformis DSM 13] | UniRef100_Q65N52 | Bacillus licheniformis DSM 13 |
| 659 | rsbU serine phosphatase | RsbU [Bacillus licheniformis DSM 13] | UniRef100_Q65N51 | Bacillus licheniformis DSM 13 |
| 660 | rsbV anti-anti-sigma factor (antagonist of RsbW) | RsbV [Bacillus licheniformis DSM 13] | UniRef100_Q65N50 | Bacillus licheniformis DSM 13 |
| 661 | rsbW switch protein_serine kinase and anti-sigma factor (inhibitory sigma-B binding protein) | RsbW [Bacillus licheniformis DSM 13] | UniRef100_Q65N49 | Bacillus licheniformis DSM 13 |
| 662 | sigB RNA polymerase sigma-37 factor (sigma-B) | SigB [Bacillus licheniformis DSM 13] | UniRef100_Q65N48 | Bacillus licheniformis DSM 13 |
| 663 | rsbX serine phosphatase | RsbX [Bacillus licheniformis DSM 13] | UniRef100_Q65N47 | Bacillus licheniformis DSM 13 |
| 664 | ydcI putative RNA binding protein containing S1 RNA binding domain,YdcI | RuvA domain 2-like,Nucleic acid-binding OB-fold [Bacillus licheniformis DSM 13] | UniRef100_Q62YJ5 | Bacillus licheniformis DSM 13 |
| 665 | BLP00625 hypothetical protein | TetR [Bacillus licheniformis DSM 13] | UniRef100_Q65N45 | Bacillus licheniformis DSM 13 |
| 666 | BLP04703 hypothetical protein | | | |
| 667 | ydgH conserved hypothetical protein YdgH | YdgH [Bacillus licheniformis DSM 13] | UniRef100_Q65N44 | Bacillus licheniformis DSM 13 |
| 668 | ydcK conserved protein YdcK | Hypothetical protein ydcK [Bacillus licheniformis DSM 13] | UniRef100_Q65N43 | Bacillus licheniformis DSM 13 |
| 669 | BLP00628 hypothetical protein | Hypothetical protein [Bacillus licheniformis DSM 13] | UniRef100_Q65N41 | Bacillus licheniformis DSM 13 |
| 670 | BLP00629 hypothetical protein | Hypothetical protein [Bacillus licheniformis DSM 13] | UniRef100_Q65N40 | Bacillus licheniformis DSM 13 |
| 671 | BLP00630 fatty acid desaturase | Hypothetical protein [Bacillus licheniformis DSM 13] | UniRef100_Q65N39 | Bacillus licheniformis DSM 13 |
| 672 | BLP00631 hypothetical protein | CspC [Bacillus licheniformis DSM 13] | UniRef100_Q65N38 | Bacillus licheniformis DSM 13 |
| 673 | cspC cold-shock protein | CspC [Bacillus licheniformis DSM 13] | UniRef100_Q65N37 | Bacillus licheniformis DSM 13 |
| 674 | BLP00633 hypothetical protein | GroES-like [Bacillus licheniformis DSM 13] | UniRef100_Q62YI5 | Bacillus licheniformis DSM 13 |
| 675 | yyaS conserved membrane protein YyaS | Hypothetical protein yyaS [Bacillus licheniformis DSM 13] | UniRef100_Q65N35 | Bacillus licheniformis DSM 13 |
| 676 | yybA negative regulator of yyaTS - YybA | YybA [Bacillus licheniformis DSM 13] | UniRef100_Q65N34 | Bacillus licheniformis DSM 13 |
| 677 | paiA transcriptional regulator PaiA | PaiA [Bacillus licheniformis DSM 13] | UniRef100_Q65N33 | Bacillus licheniformis DSM 13 |
| 678 | paiB transcriptional regulator | PaiB [Bacillus licheniformis DSM 13] | UniRef100_Q65N32 | Bacillus licheniformis DSM 13 |
| 679 | BLP00638 arginase | Hypothetical protein [Bacillus licheniformis DSM 13] | UniRef100_Q65N31 | Bacillus licheniformis DSM 13 |
| 680 | BLP00639 putative carboxy-lyase | Hypothetical protein [Bacillus licheniformis DSM 13] | UniRef100_Q65N30 | Bacillus licheniformis DSM 13 |
| 681 | ydeO conserved hypothetical protein YdeO | YdeO [Bacillus licheniformis DSM 13] | UniRef100_Q65N29 | Bacillus licheniformis DSM 13 |
| 682 | BLP00641 Lysine exporter protein | LysE [Bacillus licheniformis DSM 13] | UniRef100_Q65N28 | Bacillus licheniformis DSM 13 |
| 683 | BLP00642 putative regulatory protein | Hypothetical protein [Bacillus licheniformis DSM 13] | UniRef100_Q65N27 | Bacillus licheniformis DSM 13 |

| | | | | |
|---|---|---|---|---|
| 684 | BLP00643 hypothetical protein | Hypothetical protein [Bacillus licheniformis DSM 13] | UniRef100_Q65N26 | Bacillus licheniformis DSM 13 |
| 685 | BLP00645 Major facilitator superfamily, transporter | Hypothetical protein [Bacillus licheniformis DSM 13] | UniRef100_Q65N25 | Bacillus licheniformis DSM 13 |
| 686 | BLP00646 hypothetical protein | Hypothetical protein [Bacillus licheniformis DSM 13] | UniRef100_Q65N24 | Bacillus licheniformis DSM 13 |
| 687 | ydgF Amino acid permease YdgF | YdgF [Bacillus licheniformis DSM 13] | UniRef100_Q65N23 | Bacillus licheniformis DSM 13 |
| 688 | BLP04706 hypothetical protein | | | |
| 689 | BLP00648 hypothetical protein | Hypothetical protein [Bacillus licheniformis DSM 13] | UniRef100_Q65N22 | Bacillus licheniformis DSM 13 |
| 690 | BLP00649 hypothetical protein | Hypothetical protein [Bacillus licheniformis DSM 13] | UniRef100_Q65N21 | Bacillus licheniformis DSM 13 |
| 691 | BLP00650 hypothetical protein | | | |
| 692 | sigV RNA polymerase ECF(extracytoplasmic function)-type sigma factor (sigma-V) | SigV (RNA polymerase ECF(Extracytoplasmic function)-type sigma factor) [Bacillus licheniformis DSM 13] | UniRef100_Q65N20 | Bacillus licheniformis DSM 13 |
| 693 | yrhM YrhM | Hypothetical protein yrhM [Bacillus licheniformis DSM 13] | UniRef100_Q65N19 | Bacillus licheniformis DSM 13 |
| 694 | yrhL ABC transporter | YrhL [Bacillus licheniformis DSM 13] | UniRef100_Q65N18 | Bacillus licheniformis DSM 13 |
| 695 | ydgK putative Drug resistance transporter | YdgK [Bacillus licheniformis DSM 13] | UniRef100_Q65N17 | Bacillus licheniformis DSM 13 |
| 696 | ywpD two-component sensor histidine kinase | YwpD [Bacillus licheniformis DSM 13] | UniRef100_Q65N16 | Bacillus licheniformis DSM 13 |
| 697 | BLP00656 two-component response regulator | Hypothetical protein [Bacillus licheniformis DSM 13] | UniRef100_Q65N15 | Bacillus licheniformis DSM 13 |
| 698 | BLP00657 LPXTG-motif containing, cell wall anchor domain protein | Hypothetical Surface protein from Gram-positive cocci, anchor region [Bacillus licheniformis DSM 13] | UniRef100_Q65N14 | Bacillus licheniformis DSM 13 |
| 699 | BLP00658 hypothetical protein | | | |
| 700 | ywpE conserved protein YwpE | Hypothetical protein [Bacillus licheniformis DSM 13] | UniRef100_Q65N13 | Bacillus licheniformis DSM 13 |
| 701 | BLP00660 hypothetical protein | | | |
| 702 | BLP00661 hypothetical protein | | | |
| 703 | iolT major inositol transport protein iolT | YdjK [Bacillus licheniformis DSM 13] | UniRef100_Q65N12 | Bacillus licheniformis DSM 13 |
| 704 | thiL thiamine-monophosphate kinase | ThiL [Bacillus licheniformis DSM 13] | UniRef100_Q65N11 | Bacillus licheniformis DSM 13 |
| 705 | ydiB hypothetical conserved protein YdiB | YdiB [Bacillus licheniformis DSM 13] | UniRef100_Q65N10 | Bacillus licheniformis DSM 13 |
| 706 | ydiC Peptidase M22, glycoprotease YdiC | YdiC [Bacillus licheniformis DSM 13] | UniRef100_Q65N09 | Bacillus licheniformis DSM 13 |
| 707 | ydiD Ribosomal-protein-alanine acetyltransferase YdiD | YdiD [Bacillus licheniformis DSM 13] | UniRef100_Q65N08 | Bacillus licheniformis DSM 13 |
| 708 | gcp O-sialoglycoprotein endopeptidase | Gcp [Bacillus licheniformis DSM 13] | UniRef100_Q65N07 | Bacillus licheniformis DSM 13 |
| 709 | ydiF ABC transporter YdiF | YdiF [Bacillus licheniformis DSM 13] | UniRef100_Q65N06 | Bacillus licheniformis DSM 13 |
| 710 | BLP00669 hypothetical protein | | | |
| 711 | moaC Molybdopterin cofactor biosynthesis protein MoaC | YdiG [Bacillus licheniformis DSM 13] | UniRef100_Q65N05 | Bacillus licheniformis DSM 13 |
| 712 | rex redox regulator Rex | YdiH [Bacillus licheniformis DSM 13] | UniRef100_Q65N04 | Bacillus licheniformis DSM 13 |
| 713 | tatAY component of the twin-arginine pre-protein translocation pathway | TatAY [Bacillus licheniformis DSM 13] | UniRef100_Q65N03 | Bacillus licheniformis DSM 13 |
| 714 | tatCY component of the twin-arginine pre-protein translocation pathway | TatCY [Bacillus licheniformis DSM 13] | UniRef100_Q65N02 | Bacillus licheniformis DSM 13 |
| 715 | ydiK YdiK | YdiK [Bacillus licheniformis DSM 13] | UniRef100_Q65N01 | Bacillus licheniformis DSM 13 |
| 716 | ydiL Aldo_keto reductase | YdiL [Bacillus licheniformis DSM 13] | UniRef100_Q65N00 | Bacillus licheniformis DSM 13 |

| | | | | |
|---|---|---|---|---|
| 717 | groES class I heat-shock protein (chaperonin) | GroES [Bacillus licheniformis DSM 13] | UniRef100_Q65MZ9 | Bacillus licheniformis DSM 13 |
| 718 | groEL class I heat-shock protein (chaperonin) | GroEL [Bacillus licheniformis DSM 13] | UniRef100_Q65MZ8 | Bacillus licheniformis DSM 13 |
| 719 | BLP04707 hypothetical protein | | | |
| 720 | BLP04846 conserved hypothetical protein | Hypothetical Sugar transporter superfamily [Bacillus licheniformis DSM 13] | UniRef100_Q62YE4 | Bacillus licheniformis DSM 13 |
| 721 | BLP00678 hypothetical protein | Hypothetical protein [Bacillus licheniformis DSM 13] | UniRef100_Q62YE3 | Bacillus licheniformis DSM 13 |
| 722 | BLP00679 hypothetical protein | Hypothetical protein [Bacillus licheniformis DSM 13] | UniRef100_Q62YE2 | Bacillus licheniformis DSM 13 |
| 723 | BLP00680 hypothetical protein | Hypothetical protein [Bacillus licheniformis DSM 13] | UniRef100_Q62YE0 | Bacillus licheniformis DSM 13 |
| 724 | yoaR conserved protein YoaR | YoaR [Bacillus licheniformis DSM 13] | UniRef100_Q65MZ6 | Bacillus licheniformis DSM 13 |
| 725 | yfmQ conserved hypothetical protein YfmQ | YfmQ [Bacillus licheniformis DSM 13] | UniRef100_Q65MZ5 | Bacillus licheniformis DSM 13 |
| 726 | BLP00683 hypothetical protein | | | |
| 727 | BLP00684 hypothetical protein | Hypothetical protein [Bacillus licheniformis DSM 13] | UniRef100_Q65MZ4 | Bacillus licheniformis DSM 13 |
| 728 | yoqW YoqW | YoqW [Bacillus licheniformis DSM 13] | UniRef100_Q65MZ3 | Bacillus licheniformis DSM 13 |
| 729 | BLP00686 conserved hypothetical | Hypothetical protein [Bacillus licheniformis DSM 13] | UniRef100_Q65G40 | Bacillus licheniformis DSM 13 |
| 730 | BLP00687 putative regulatory protein | Hypothetical protein [Bacillus licheniformis DSM 13] | UniRef100_Q65MZ2 | Bacillus licheniformis DSM 13 |
| 731 | BLP00688 phosphoenolpyruvate synthase | Hypothetical protein [Bacillus licheniformis DSM 13] | UniRef100_Q65MZ1 | Bacillus licheniformis DSM 13 |
| 732 | yoaF YoaF | Hypothetical protein yoaF [Bacillus licheniformis DSM 13] | UniRef100_Q65MZ0 | Bacillus licheniformis DSM 13 |
| 733 | BLP00690 conserved hypothetical protein | Hypothetical protein [Bacillus licheniformis DSM 13] | UniRef100_Q65MY9 | Bacillus licheniformis DSM 13 |
| 734 | BLP00691 hypothetical protein | | | |
| 735 | dltE DltE | Hypothetical protein dltE [Bacillus licheniformis DSM 13] | UniRef100_Q65MY8 | Bacillus licheniformis DSM 13 |
| 736 | pnbA para-nitrobenzyl esterase (intracellular esterase B) | PnbA [Bacillus licheniformis DSM 13] | UniRef100_Q65MY7 | Bacillus licheniformis DSM 13 |
| 737 | BLP00694 inositol transport protein | Hypothetical protein [Bacillus licheniformis DSM 13] | UniRef100_Q65MY6 | Bacillus licheniformis DSM 13 |
| 738 | pspA phage shock protein A homolog | PspA [Bacillus licheniformis DSM 13] | UniRef100_Q65MY5 | Bacillus licheniformis DSM 13 |
| 739 | ydjG conserved protein YdjG | Hypothetical protein ydjG [Bacillus licheniformis DSM 13] | UniRef100_Q65MY4 | Bacillus licheniformis DSM 13 |
| 740 | ydjH conserved membrane protein YdjH | Hypothetical protein ydjH [Bacillus licheniformis DSM 13] | UniRef100_Q65MY3 | Bacillus licheniformis DSM 13 |
| 741 | ydjI conserved protein YdjI | Hypothetical protein ydjI [Bacillus licheniformis DSM 13] | UniRef100_Q65MY2 | Bacillus licheniformis DSM 13 |
| 742 | BLP00699 putative oxidoreductase | Hypothetical protein [Bacillus licheniformis DSM 13] | UniRef100_Q65MY1 | Bacillus licheniformis DSM 13 |
| 743 | yrhO putative cyclodextrin metabolism protein YrhO | YrhO [Bacillus licheniformis DSM 13] | UniRef100_Q65MY0 | Bacillus licheniformis DSM 13 |
| 744 | yrhP Lysine exporter protein YrhP | YrhP [Bacillus licheniformis DSM 13] | UniRef100_Q65G40 | Bacillus licheniformis DSM 13 |
| 745 | ykvA2 conserved membrane protein | Hypothetical protein [Bacillus licheniformis DSM 13] | UniRef100_Q65MX8 | Bacillus licheniformis DSM 13 |
| 746 | BLP00703 sporulation related protein Stage V protein E | Hypothetical protein [Bacillus licheniformis DSM 13] | UniRef100_Q65MX7 | Bacillus licheniformis DSM 13 |
| 747 | BLP00704 cell-division protein | Hypothetical protein [Bacillus licheniformis DSM 13] | UniRef100_Q65MX6 | Bacillus licheniformis DSM 13 |
| 748 | BLP00705 hypothetical protein | | | |
| 749 | BLP00706 conserved hypothetical protein | Hypothetical protein [Bacillus licheniformis DSM 13] | UniRef100_Q65MX5 | Bacillus licheniformis DSM 13 |
| 750 | BLP00707 hypothetical protein | Winged helix DNA-binding [Bacillus licheniformis DSM 13] | UniRef100_Q62YB6 | Bacillus licheniformis DSM 13 |

| 751 | BLP00708 hypothetical protein | | | |
|---|---|---|---|---|
| 752 | yjeAA conserved hypothetical protein | Hypothetical protein yjeAA [Bacillus licheniformis DSM 13] | UniRef100_Q65MX2 | Bacillus licheniformis DSM 13 |
| 753 | yjeA Putative Carbohydrate Esterase Family 4 protein | YjeA [Bacillus licheniformis DSM 13] | UniRef100_Q65MX1 | Bacillus licheniformis DSM 13 |
| 754 | amyL alpha amylase, Glycoside Hydrolase Family 13 | Alpha-amylase precursor [Bacillus licheniformis] | UniRef100_P06278 | Bacillus licheniformis |
| 755 | yvdE probable transcriptional regulator YvdE | YvdE [Bacillus licheniformis DSM 13] | UniRef100_Q65MW9 | Bacillus licheniformis DSM 13 |
| 756 | yvdF Glycoside Hydrolase Family 13 YvdF | Maltogenic alpha-amylase [Bacillus licheniformis DSM 13] | UniRef100_Q65MW8 | Bacillus licheniformis DSM 13 |
| 757 | mdxE maltodextrin transport system substrate-binding protein MdxE | YvdG [Bacillus licheniformis DSM 13] | UniRef100_Q65MW7 | Bacillus licheniformis DSM 13 |
| 758 | mdxF maltodextrin transport system permease protein MdxF | YvdH [Bacillus licheniformis DSM 13] | UniRef100_Q65MW6 | Bacillus licheniformis DSM 13 |
| 759 | mdxG maltodextrin transport system permease protein MdxG | YvdI [Bacillus licheniformis DSM 13] | UniRef100_Q65MW5 | Bacillus licheniformis DSM 13 |
| 760 | yvdJ conserved membrane protein YvdJ | Hypothetical protein yvdJ [Bacillus licheniformis DSM 13] | UniRef100_Q65MW4 | Bacillus licheniformis DSM 13 |
| 761 | yvdK Glycoside Hydrolase Family 65, YvdK | YvdK [Bacillus licheniformis DSM 13] | UniRef100_Q65MW3 | Bacillus licheniformis DSM 13 |
| 762 | malL Glycoside Hydrolase Family 13, MalL | MalL [Bacillus licheniformis DSM 13] | UniRef100_Q65MW2 | Bacillus licheniformis DSM 13 |
| 763 | pgcM beta-phosphoglucomutase and glucose-1-phosphate phosphodismutase | PgcM [Bacillus licheniformis DSM 13] | UniRef100_Q65MW1 | Bacillus licheniformis DSM 13 |
| 764 | BLP00721 hypothetical protein | | | |
| 765 | tyrZ tyrosyl-tRNA synthetase | TyrZ [Bacillus licheniformis DSM 13] | UniRef100_Q65MW0 | Bacillus licheniformis DSM 13 |
| 766 | ywaE probable transcriptional regulator YwaE | YwaE [Bacillus licheniformis DSM 13] | UniRef100_Q65MV9 | Bacillus licheniformis DSM 13 |
| 767 | BLP00724 hypothetical protein | Hypothetical protein [Bacillus licheniformis DSM 13] | UniRef100_Q65MV8 | Bacillus licheniformis DSM 13 |
| 768 | BLP00725 hypothetical protein | Hypothetical protein [Bacillus licheniformis DSM 13] | UniRef100_Q65MV7 | Bacillus licheniformis DSM 13 |
| 769 | ydjM YdjM | Rare lipoprotein A [Bacillus licheniformis DSM 13] | UniRef100_Q62Y99 | Bacillus licheniformis DSM 13 |
| 770 | ydjN YdjN | YdjN [Bacillus licheniformis DSM 13] | UniRef100_Q65MV5 | Bacillus licheniformis DSM 13 |
| 771 | yeaA conserved protein YeaA | Hypothetical protein yeaA [Bacillus licheniformis DSM 13] | UniRef100_Q65MV4 | Bacillus licheniformis DSM 13 |
| 772 | BLP00730 Small nuclear-like ribonucleoprotein | Hypothetical protein [Bacillus licheniformis DSM 13] | UniRef100_Q65MV3 | Bacillus licheniformis DSM 13 |
| 773 | BLP00731 conserved hypothetical protein | Hypothetical protein [Bacillus licheniformis DSM 13] | UniRef100_Q65MV2 | Bacillus licheniformis DSM 13 |
| 774 | BLP00732 hypothetical protein | | | |
| 775 | sipS type I signal peptidase | SipS [Bacillus licheniformis DSM 13] | UniRef100_Q65MV1 | Bacillus licheniformis DSM 13 |
| 776 | BLP00734 putative methyltransferase | Hypothetical protein [Bacillus licheniformis DSM 13] | UniRef100_Q65MV0 | Bacillus licheniformis DSM 13 |
| 777 | BLP00735 hypothetical protein | | | |
| 778 | yhfK conserved protein YhfK | Hypothetical protein yhfK [Bacillus licheniformis DSM 13] | UniRef100_Q65MU9 | Bacillus licheniformis DSM 13 |
| 779 | ydeE probable transcriptional regulator YdeE | Hypothetical protein [Bacillus licheniformis DSM 13] | UniRef100_Q65MU8 | Bacillus licheniformis DSM 13 |
| 780 | cotA spore coat protein (outer) | CotA [Bacillus licheniformis DSM 13] | UniRef100_Q65MU7 | Bacillus licheniformis DSM 13 |
| 781 | BLP00739 Acyltransferase 3 | Hypothetical protein [Bacillus licheniformis DSM 13] | UniRef100_Q65MU6 | Bacillus licheniformis DSM 13 |
| 782 | BLP00740 hypothetical protein | Hypothetical protein [Bacillus licheniformis DSM 13] | UniRef100_Q65MU5 | Bacillus licheniformis DSM 13 |
| 783 | yeaB Cation efflux protein | YeaB [Bacillus licheniformis DSM 13] | UniRef100_Q65MU4 | Bacillus licheniformis DSM 13 |
| 784 | yeaC YeaC | YeaC [Bacillus licheniformis DSM 13] | UniRef100_Q65MU3 | Bacillus licheniformis DSM 13 |

| 785 | yeaD conserved protein YeaD | Hypothetical protein yeaD [Bacillus licheniformis DSM 13] | UniRef100_Q65MU2 | Bacillus licheniformis DSM 13 |
|---|---|---|---|---|
| 786 | yebA transglutaminase-like enzymes, putative cysteine protease YebA | YebA [Bacillus licheniformis DSM 13] | UniRef100_Q65MU1 | Bacillus licheniformis DSM 13 |
| 787 | guaA GMP synthetase | GuaA [Bacillus licheniformis DSM 13] | UniRef100_Q65MU0 | Bacillus licheniformis DSM 13 |
| 788 | pbuG hypoxanthine_guanine permease | PbuG [Bacillus licheniformis DSM 13] | UniRef100_Q65MT9 | Bacillus licheniformis DSM 13 |
| 789 | BLP00747 hypothetical protein | | | |
| 790 | yebC YbeC | YebC [Bacillus licheniformis DSM 13] | UniRef100_Q65MT8 | Bacillus licheniformis DSM 13 |
| 791 | BLP00749 hypothetical protein | | | |
| 792 | yebE conserved membrane protein YebE | Hypothetical protein yebE [Bacillus licheniformis DSM 13] | UniRef100_Q65MT6 | Bacillus licheniformis DSM 13 |
| 793 | yebG conserved protein YebG | Hypothetical protein yebG [Bacillus licheniformis DSM 13] | UniRef100_Q65MT5 | Bacillus licheniformis DSM 13 |
| 794 | purE phosphoribosylaminoimidazole carboxylase I | PurE [Bacillus licheniformis DSM 13] | UniRef100_Q65MT4 | Bacillus licheniformis DSM 13 |
| 795 | purK phosphoribosylaminoimidazole carboxylase II | PurK [Bacillus licheniformis DSM 13] | UniRef100_Q65MT3 | Bacillus licheniformis DSM 13 |
| 796 | purB adenylosuccinate lyase | PurB [Bacillus licheniformis DSM 13] | UniRef100_Q65MT2 | Bacillus licheniformis DSM 13 |
| 797 | purC phosphoribosylaminoimidazole succinocarboxamide synthetase | PurC [Bacillus licheniformis DSM 13] | UniRef100_Q65MT1 | Bacillus licheniformis DSM 13 |
| 798 | purS PurS | Hypothetical protein purS [Bacillus licheniformis DSM 13] | UniRef100_Q65MT0 | Bacillus licheniformis DSM 13 |
| 799 | purQ phosphoribosylformylglycinamidine synthetase I | PurQ [Bacillus licheniformis DSM 13] | UniRef100_Q65MS9 | Bacillus licheniformis DSM 13 |
| 800 | purL phosphoribosylformylglycinamidine synthetase II | PurL [Bacillus licheniformis DSM 13] | UniRef100_Q65MS8 | Bacillus licheniformis DSM 13 |
| 801 | purF glutamine phosphoribosylpyrophosphate amidotransferase | PurF [Bacillus licheniformis DSM 13] | UniRef100_Q65MS7 | Bacillus licheniformis DSM 13 |
| 802 | purM phosphoribosylaminoimidazole synthetase | PurM [Bacillus licheniformis DSM 13] | UniRef100_Q65MS6 | Bacillus licheniformis DSM 13 |
| 803 | purN phosphoribosylglycinamide formyltransferase | PurN [Bacillus licheniformis DSM 13] | UniRef100_Q65MS5 | Bacillus licheniformis DSM 13 |
| 804 | purH phosphoribosylaminoimidazole carboxy formyl formyltransferase and inosine-monophosphate cyclohydrolase | PurH [Bacillus licheniformis DSM 13] | UniRef100_Q65MS4 | Bacillus licheniformis DSM 13 |
| 805 | purD phosphoribosylglycinamide synthetase | PurD [Bacillus licheniformis DSM 13] | UniRef100_Q65MS3 | Bacillus licheniformis DSM 13 |
| 806 | BLP00764 hypothetical protein | Homeodomain-like [Bacillus licheniformis DSM 13] | UniRef100_Q62Y67 | Bacillus licheniformis DSM 13 |
| 807 | yjiB Cytochrome P450 | YjiB [Bacillus licheniformis DSM 13] | UniRef100_Q65MS1 | Bacillus licheniformis DSM 13 |
| 808 | BLP04847 conserved hypothetical protein | Hypothetical protein [Bacillus licheniformis DSM 13] | UniRef100_Q62Y65 | Bacillus licheniformis DSM 13 |
| 809 | yybP YybP | YybP [Bacillus licheniformis DSM 13] | UniRef100_Q65MS0 | Bacillus licheniformis DSM 13 |
| 810 | BLP00767 hypothetical protein | Hypothetical protein [Bacillus licheniformis DSM 13] | UniRef100_Q65MR9 | Bacillus licheniformis DSM 13 |
| 811 | BLP00768 hypothetical protein | Hypothetical protein [Bacillus licheniformis DSM 13] | UniRef100_Q65MR8 | Bacillus licheniformis DSM 13 |
| 812 | yerA putative Metallo-dependent hydrolase | YerA [Bacillus licheniformis DSM 13] | UniRef100_Q65MR7 | Bacillus licheniformis DSM 13 |
| 813 | yerB conserved protein YerB | Hypothetical protein yerB [Bacillus licheniformis DSM 13] | UniRef100_Q65MR6 | Bacillus licheniformis DSM 13 |
| 814 | yerC conserved protein YerC | YerC [Bacillus licheniformis DSM 13] | UniRef100_Q65MR5 | Bacillus licheniformis DSM 13 |
| 815 | pcrB PcrB | PcrB [Bacillus licheniformis DSM 13] | UniRef100_Q65MR4 | Bacillus licheniformis DSM 13 |
| 816 | pcrA ATP-dependent DNA helicase | PcrA [Bacillus licheniformis DSM 13] | UniRef100_Q65MR3 | Bacillus licheniformis DSM 13 |
| 817 | ligA DNA ligase | LigA [Bacillus licheniformis DSM 13] | UniRef100_Q65MR2 | Bacillus licheniformis DSM 13 |
| 818 | yerH YerH | Hypothetical protein yerH [Bacillus licheniformis DSM 13] | UniRef100_Q65MR1 | Bacillus licheniformis DSM 13 |

| 819 | BLP00776 hypothetical protein | Hypothetical protein [Bacillus licheniformis DSM 13] | UniRef100_Q65MR0 | Bacillus licheniformis DSM 13 |
|---|---|---|---|---|
| 820 | BLP00777 hypothetical protein | Hypothetical protein [Bacillus licheniformis DSM 13] | UniRef100_Q65MQ9 | Bacillus licheniformis DSM 13 |
| 821 | BLP00778 hypothetical protein | Hypothetical protein [Bacillus licheniformis DSM 13] | UniRef100_Q65MQ8 | Bacillus licheniformis DSM 13 |
| 822 | BLP00779 hypothetical protein | Hypothetical protein [Bacillus licheniformis DSM 13] | UniRef100_Q65MQ7 | Bacillus licheniformis DSM 13 |
| 823 | BLP00780 zin-ion binding putative hydrolase | Hypothetical protein [Bacillus licheniformis DSM 13] | UniRef100_Q65MQ6 | Bacillus licheniformis DSM 13 |
| 824 | BLP00781 hypothetical protein | Hypothetical protein [Bacillus licheniformis DSM 13] | UniRef100_Q65MQ5 | Bacillus licheniformis DSM 13 |
| 825 | BLP00782 putative phosphopentomutase | Hypothetical protein [Bacillus licheniformis DSM 13] | UniRef100_Q65MQ4 | Bacillus licheniformis DSM 13 |
| 826 | BLP00783 putative alanine racemase | Hypothetical protein [Bacillus licheniformis DSM 13] | UniRef100_Q65MQ3 | Bacillus licheniformis DSM 13 |
| 827 | BLP04708 hypothetical protein | | | |
| 828 | nagB N-acetylglucosamine-6-phosphate isomerase | NagB [Bacillus licheniformis DSM 13] | UniRef100_Q65MQ2 | Bacillus licheniformis DSM 13 |
| 829 | BLP00785 hypothetical protein | | | |
| 830 | sapB SapB | Hypothetical protein sapB [Bacillus licheniformis DSM 13] | UniRef100_Q65MQ1 | Bacillus licheniformis DSM 13 |
| 831 | BLP00787 hypothetical protein | | | |
| 832 | opuE proline transporter | OpuE [Bacillus licheniformis DSM 13] | UniRef100_Q65MQ0 | Bacillus licheniformis DSM 13 |
| 833 | BLP00789 hypothetical protein | | | |
| 834 | gatC glutamyl-tRNA(Gln) amidotransferase (subunit C) | GatC (Glutamyl-tRNA(Gln) amidotransferase) [Bacillus licheniformis DSM 13] | UniRef100_Q65MP9 | Bacillus licheniformis DSM 13 |
| 835 | gatA glutamyl-tRNA(Gln) amidotransferase (subunit A) | GatA (Glutamyl-tRNA(Gln) amidotransferase) [Bacillus licheniformis DSM 13] | UniRef100_Q65MP8 | Bacillus licheniformis DSM 13 |
| 836 | gatB glutamyl-tRNA(Gln) amidotransferase (subunit B) | GatB [Bacillus licheniformis DSM 13] | UniRef100_Q65MP7 | Bacillus licheniformis DSM 13 |
| 837 | BLP00793 conserved hypothetical protein | Hypothetical protein [Bacillus licheniformis DSM 13] | UniRef100_Q65MP6 | Bacillus licheniformis DSM 13 |
| 838 | BLP00794 hypothetical protein | Hypothetical protein [Bacillus licheniformis DSM 13] | UniRef100_Q65MP5 | Bacillus licheniformis DSM 13 |
| 839 | ydhT Glutamine amidotransferase, class-II | YdhT [Bacillus licheniformis DSM 13] | UniRef100_Q65MP4 | Bacillus licheniformis DSM 13 |
| 840 | yerO putative transcriptional regulator YerO | YerO [Bacillus licheniformis DSM 13] | UniRef100_Q65MP3 | Bacillus licheniformis DSM 13 |
| 841 | swrC swarming and motility protein SwrC | YerP [Bacillus licheniformis DSM 13] | UniRef100_Q65MP2 | Bacillus licheniformis DSM 13 |
| 842 | BLP00798 conserved hypothetical protein | Hypothetical protein [Bacillus licheniformis DSM 13] | UniRef100_Q65MP1 | Bacillus licheniformis DSM 13 |
| 843 | yjcK putative ribosomal-protein-alanine N-acetyltransferase YjcK | YjcK [Bacillus licheniformis DSM 13] | UniRef100_Q65MP0 | Bacillus licheniformis DSM 13 |
| 844 | BLP00800 putative inosine-uridine preferring nucleoside hydrolase | Hypothetical protein [Bacillus licheniformis DSM 13] | UniRef100_Q65MN9 | Bacillus licheniformis DSM 13 |
| 845 | yerQ putative kinase related to diacylglycerol kinase YerQ | Hypothetical protein yerQ [Bacillus licheniformis DSM 13] | UniRef100_Q65MN8 | Bacillus licheniformis DSM 13 |
| 846 | yefA putative RNA methyltransferase YefA | YefA (TRNA (Uracil-5-)-methyltransferase/TrmA) [Bacillus licheniformis DSM 13] | UniRef100_Q65MN7 | Bacillus licheniformis DSM 13 |
| 847 | BLP00803 hypothetical protein | Hypothetical protein [Bacillus cereus] | UniRef100_Q73EH0 | Bacillus cereus |
| 848 | BLP00805 HNH endonuclease domain protein | HNH endonuclease domain protein [Bacillus cereus] | UniRef100_Q73EG9 | Bacillus cereus |
| 849 | CDS_00859 hypothetical protein | S-adenosylmethionine synthetase [Bacillus cereus] | UniRef100_Q73EG7 | Bacillus cereus |
| 850 | BLP00806 putative S-adenosylmethionine synthetase | S-adenosylmethionine synthetase [Bacillus cereus] | UniRef100_Q73EG7 | Bacillus cereus |

| | | | | |
|---|---|---|---|---|
| 851 | BLP00807 DNA methylase | DNA methylase, family protein [Bacillus cereus] | UniRef100_Q73EG6 | Bacillus cereus |
| 852 | BLP00808 cytosine methyltransferase | DNA-cytosine methyltransferase [Bacillus cereus] | UniRef100_Q73EG5 | Bacillus cereus |
| 853 | rapK response regulator aspartate phosphatase | RapK [Bacillus licheniformis DSM 13] | UniRef100_Q65MM8 | Bacillus licheniformis DSM 13 |
| 854 | phrK regulator of the activity of RapK phosphatase | Regulator of the activity of phosphatase RapK [Bacillus licheniformis DSM 13] | UniRef100_Q62Y21 | Bacillus licheniformis DSM 13 |
| 855 | BLP04709 hypothetical protein | | | |
| 856 | BLP00811 putative SAM methyltransferase | Hypothetical protein (Hypothetical SAM (And some other nucleotide) binding motif) [Bacillus licheniformis DSM 13] | UniRef100_Q65MM6 | Bacillus licheniformis DSM 13 |
| 857 | BLP00812 hypothetical protein | | | |
| 858 | yeeI hypothetical protein YeeI | YeeI [Bacillus licheniformis DSM 13] | UniRef100_Q65MM5 | Bacillus licheniformis DSM 13 |
| 859 | BLP00814 Putative HTH-type transcriptional regulator | Hypothetical protein [Bacillus licheniformis DSM 13] | UniRef100_Q65MM4 | Bacillus licheniformis DSM 13 |
| 860 | BLP00815 hypothetical protein | Hypothetical protein [Bacillus licheniformis DSM 13] | UniRef100_Q65MM3 | Bacillus licheniformis DSM 13 |
| 861 | yfmT Aldehyde dehydrogenase YfmT | YfmT [Bacillus licheniformis DSM 13] | UniRef100_Q65MM2 | Bacillus licheniformis DSM 13 |
| 862 | yfmS putative chemotaxis sensory transducer YfmS | YfmS [Bacillus licheniformis DSM 13] | UniRef100_Q65MM1 | Bacillus licheniformis DSM 13 |
| 863 | BLP00818 Sodium_sulphate symporter | Hypothetical protein [Bacillus licheniformis DSM 13] | UniRef100_Q65MM0 | Bacillus licheniformis DSM 13 |
| 864 | yfmR ABC transporter YfmR | YfmR [Bacillus licheniformis DSM 13] | UniRef100_Q65ML9 | Bacillus licheniformis DSM 13 |
| 865 | BLP00820 hypothetical protein | Hypothetical protein [Bacillus licheniformis DSM 13] | UniRef100_Q65ML8 | Bacillus licheniformis DSM 13 |
| 866 | yciA conserved hypothetical protein YciA | YciA [Bacillus licheniformis DSM 13] | UniRef100_Q65ML7 | Bacillus licheniformis DSM 13 |
| 867 | BLP00822 NAD-binding site protein | Hypothetical protein [Bacillus licheniformis DSM 13] | UniRef100_Q65ML6 | Bacillus licheniformis DSM 13 |
| 868 | BLP00823 GTP binding protein | Hypothetical protein [Bacillus licheniformis DSM 13] | UniRef100_Q65ML5 | Bacillus licheniformis DSM 13 |
| 869 | yciC Cobalamin synthesis protein_P47K | YciC [Bacillus licheniformis DSM 13] | UniRef100_Q65ML4 | Bacillus licheniformis DSM 13 |
| 870 | bioW 6-carboxyhexanoate-CoA ligase | BioW [Bacillus licheniformis DSM 13] | UniRef100_Q65ML3 | Bacillus licheniformis DSM 13 |
| 871 | bioA adenosylmethionine-8-amino-7-oxononanoate aminotransferase | BioA [Bacillus licheniformis DSM 13] | UniRef100_Q65ML2 | Bacillus licheniformis DSM 13 |
| 872 | bioF 8-amino-7-oxononanoate synthase | BioF [Bacillus licheniformis DSM 13] | UniRef100_Q65ML1 | Bacillus licheniformis DSM 13 |
| 873 | bioD dethiobiotin synthetase | BioD [Bacillus licheniformis DSM 13] | UniRef100_Q65ML0 | Bacillus licheniformis DSM 13 |
| 874 | bioB biotin synthetase | BioB [Bacillus licheniformis DSM 13] | UniRef100_Q65MK9 | Bacillus licheniformis DSM 13 |
| 875 | bioI cytochrome P450 enzyme | BioI [Bacillus licheniformis DSM 13] | UniRef100_Q65MK8 | Bacillus licheniformis DSM 13 |
| 876 | BLP00831 cation transporter | Hypothetical protein (Hypothetical Cation (Not K+) channel, TM region,Cation channel, non-ligand gated) [Bacillus licheniformis DSM 13] | UniRef100_Q65MK7 | Bacillus licheniformis DSM 13 |
| 877 | ymfP Putative DNA binding protein YmfP | YfmP [Bacillus licheniformis DSM 13] | UniRef100_Q65MK6 | Bacillus licheniformis DSM 13 |
| 878 | yfmO putative multidrug efflux protein | YfmO [Bacillus licheniformis DSM 13] | UniRef100_Q65MK5 | Bacillus licheniformis DSM 13 |
| 879 | yfmM ABC transporter | YfmM [Bacillus licheniformis DSM 13] | UniRef100_Q65MK4 | Bacillus licheniformis DSM 13 |
| 880 | yfmL putative ATP binding helicase | YfmL [Bacillus licheniformis DSM 13] | UniRef100_Q65MK3 | Bacillus licheniformis DSM 13 |
| 881 | yfmJ putative oxidoreductase | YfmJ [Bacillus licheniformis DSM 13] | UniRef100_Q65MK2 | Bacillus licheniformis DSM 13 |
| 882 | BLP00837 hypothetical protein | YfmB [Bacillus licheniformis DSM 13] | UniRef100_Q65MK1 | Bacillus licheniformis DSM 13 |

| 883 | yflT conserved hypothetical protein YflT | YflT [Bacillus licheniformis DSM 13] | UniRef100_Q65MK0 | Bacillus licheniformis DSM 13 |
|---|---|---|---|---|
| 884 | yflS putative 2-oxoglutarate_malate translocator | YflS [Bacillus licheniformis DSM 13] | UniRef100_Q65MJ9 | Bacillus licheniformis DSM 13 |
| 885 | BLP00840 Putative Type IIc bacteriocin lichenein | | | |
| 886 | BLP00841 ABC transporter, ATPase subunit | ABC transporter ATP-binding protein [Clostridium tetani] | UniRef100_Q893J7 | Clostridium tetani |
| 887 | BLP00842 ABC transporter, permease subunit | ABC transporter-associated permease [Clostridium tetani] | UniRef100_Q899Z8 | Clostridium tetani |
| 888 | BLP00843 hypothetical protein | Hypothetical protein [Bacillus licheniformis DSM 13] | UniRef100_Q65MJ8 | Bacillus licheniformis DSM 13 |
| 889 | BLP00844 Beta-lactamase-like protein | Hypothetical protein [Bacillus licheniformis DSM 13] | UniRef100_Q65MJ7 | Bacillus licheniformis DSM 13 |
| 890 | yflN Beta-lactamase-like protein YflN | YflN [Bacillus licheniformis DSM 13] | UniRef100_Q65MJ6 | Bacillus licheniformis DSM 13 |
| 891 | BLP00846 putative carboxypeptidase | Hypothetical protein [Bacillus licheniformis DSM 13] | UniRef100_Q65MJ5 | Bacillus licheniformis DSM 13 |
| 892 | nos Nitric oxide synthase Nos | YflM [Bacillus licheniformis DSM 13] | UniRef100_Q65MJ4 | Bacillus licheniformis DSM 13 |
| 893 | sdpI conserved membrane protein SdpI | Hypothetical protein yvaZ [Bacillus licheniformis DSM 13] | UniRef100_Q65MJ3 | Bacillus licheniformis DSM 13 |
| 894 | sdpR Transcriptional regulator SdpR | Winged helix DNA-binding [Bacillus licheniformis DSM 13] | UniRef100_Q62XY4 | Bacillus licheniformis DSM 13 |
| 895 | yflL Acylphosphatase YflL | YflL [Bacillus licheniformis DSM 13] | UniRef100_Q65MJ1 | Bacillus licheniformis DSM 13 |
| 896 | yflK conserved hypothetical protein with MOSC domain YflK | YflK [Bacillus licheniformis DSM 13] | UniRef100_Q65MJ0 | Bacillus licheniformis DSM 13 |
| 897 | yflJ hypothetical protein | YflJ [Bacillus licheniformis DSM 13] | UniRef100_Q65MI9 | Bacillus licheniformis DSM 13 |
| 898 | yflI conserved hypothetical protein YflI | YflI [Bacillus licheniformis DSM 13] | UniRef100_Q65MI8 | Bacillus licheniformis DSM 13 |
| 899 | yflG Peptidase M24A, methionine aminopeptidase, subfamily 1 | YflG [Bacillus licheniformis DSM 13] | UniRef100_Q65MI7 | Bacillus licheniformis DSM 13 |
| 900 | yflE Sulfatase YflE | YflE [Bacillus licheniformis DSM 13] | UniRef100_Q65MI6 | Bacillus licheniformis DSM 13 |
| 901 | BLP00856 hypothetical protein | | | |
| 902 | yflB conserved hypothetical protein YflB | YflB [Bacillus licheniformis DSM 13] | UniRef100_Q65MI5 | Bacillus licheniformis DSM 13 |
| 903 | yflA Sodium:alanine symporter YflA | YflA [Bacillus licheniformis DSM 13] | UniRef100_Q65MI4 | Bacillus licheniformis DSM 13 |
| 904 | treP phosphotransferase system (PTS) trehalose-specific enzyme IIBC component | TreP (Phosphotransferase system (PTS) trehalose-specific enzyme IIBC component) [Bacillus licheniformis DSM 13] | UniRef100_Q65MI3 | Bacillus licheniformis DSM 13 |
| 905 | treA Glycoside Hydrolase Family 13 | TreA [Bacillus licheniformis DSM 13] | UniRef100_Q65MI2 | Bacillus licheniformis DSM 13 |
| 906 | treR transcriptional regulator (GntR family) | TreR [Bacillus licheniformis DSM 13] | UniRef100_Q65MI1 | Bacillus licheniformis DSM 13 |
| 907 | BLP00862 putative transferase hexapeptide repeat containing protein | Hypothetical protein [Bacillus licheniformis DSM 13] | UniRef100_Q65MI0 | Bacillus licheniformis DSM 13 |
| 908 | BLP00863 hypothetical protein | Hypothetical protein [Bacillus licheniformis DSM 13] | UniRef100_Q65MH9 | Bacillus licheniformis DSM 13 |
| 909 | BLP00864 hypothetical protein | | | |
| 910 | BLP00865 Hypothetical protein | Hypothetical protein [Bacillus licheniformis DSM 13] | UniRef100_Q65MH9 | Bacillus licheniformis DSM 13 |
| 911 | BLP00866 Hypothetical protein | Hypothetical protein [Bacillus licheniformis DSM 13] | UniRef100_Q65MH8 | Bacillus licheniformis DSM 13 |
| 912 | spsC spore coat biosynthesis protein | Hypothetical protein spsCA [Bacillus licheniformis DSM 13] | UniRef100_Q65MH7 | Bacillus licheniformis DSM 13 |
| 913 | BLP00868 putative transferase hexapeptide repeat containing protein | Hypothetical protein [Bacillus licheniformis DSM 13] | UniRef100_Q65MH6 | Bacillus licheniformis DSM 13 |
| 914 | BLP00869 NAD-dependent epimerase_dehydratase | YtcB [Bacillus licheniformis DSM 13] | UniRef100_Q65MH5 | Bacillus licheniformis DSM 13 |
| 915 | BLP00870 putative transferase, conserved hypothetical protein | Hypothetical protein [Bacillus licheniformis DSM 13] | UniRef100_Q65MH4 | Bacillus licheniformis DSM 13 |

| 916 | BLP00871 putative glycosyl transferase family 2_4 protein | Hypothetical protein [Bacillus licheniformis DSM 13] | UniRef100_Q65MH3 | Bacillus licheniformis DSM 13 |
|---|---|---|---|---|
| 917 | BLP00872 TPR-like, putative glycosyl transferase family 2 protein | Hypothetical protein [Bacillus licheniformis DSM 13] | UniRef100_Q65MH2 | Bacillus licheniformis DSM 13 |
| 918 | spsC spore coat biosynthesis protein | SpsC [Bacillus licheniformis DSM 13] | UniRef100_Q65MH1 | Bacillus licheniformis DSM 13 |
| 919 | BLP00874 putative Oxidoreductase | YrbE [Bacillus licheniformis DSM 13] | UniRef100_Q65MH0 | Bacillus licheniformis DSM 13 |
| 920 | BLP00875 UDP-glucose 6-dehydrogenase | YtcA [Bacillus licheniformis DSM 13] | UniRef100_Q65MG9 | Bacillus licheniformis DSM 13 |
| 921 | BLP00876 conserved hypothetical protein | Hypothetical protein [Bacillus licheniformis DSM 13] | UniRef100_Q65MG8 | Bacillus licheniformis DSM 13 |
| 922 | ydeP conserved hypothetical DNA binding protein YdeP | YdeP [Bacillus licheniformis DSM 13] | UniRef100_Q65MG7 | Bacillus licheniformis DSM 13 |
| 923 | nfsB NADH-dependent nitro_flavin oxidoreductase | YfkO [Bacillus licheniformis DSM 13] | UniRef100_Q65MG6 | Bacillus licheniformis DSM 13 |
| 924 | yfkN putative nucleotidase YfkN | YfkN [Bacillus licheniformis DSM 13] | UniRef100_Q65MG5 | Bacillus licheniformis DSM 13 |
| 925 | yfkM general stress protein YfkM | YfkM [Bacillus licheniformis DSM 13] | UniRef100_Q65MG4 | Bacillus licheniformis DSM 13 |
| 926 | yfkK conserved protein YfkK | Hypothetical protein yfkK [Bacillus licheniformis DSM 13] | UniRef100_Q65MG3 | Bacillus licheniformis DSM 13 |
| 927 | BLP00882 putative Sodium:aminoacid symporter | Hypothetical protein [Bacillus licheniformis DSM 13] | UniRef100_Q65MG2 | Bacillus licheniformis DSM 13 |
| 928 | yfkJ protein-tyrosine phosphatase YfkJ | Low molecular weight phosphotyrosine protein phosphatase [Bacillus licheniformis DSM 13] | UniRef100_Q62XV3 | Bacillus licheniformis DSM 13 |
| 929 | yfkI YfkI | Hypothetical protein yfkI [Bacillus licheniformis DSM 13] | UniRef100_Q65MG0 | Bacillus licheniformis DSM 13 |
| 930 | yfkH Possible RNase YfkH | YfkH [Bacillus licheniformis DSM 13] | UniRef100_Q65MF9 | Bacillus licheniformis DSM 13 |
| 931 | yfkF putative transcriptional regulator YfkF | YfkF [Bacillus licheniformis DSM 13] | UniRef100_Q65MF8 | Bacillus licheniformis DSM 13 |
| 932 | BLP00887 putative stress response protein | Hypothetical protein [Bacillus licheniformis DSM 13] | UniRef100_Q65MF7 | Bacillus licheniformis DSM 13 |
| 933 | yfkE H _Ca2 exchanger YfkE | YfkE [Bacillus licheniformis DSM 13] | UniRef100_Q65MF6 | Bacillus licheniformis DSM 13 |
| 934 | yfkD conserved protein YfkD | Hypothetical protein yfkD [Bacillus licheniformis DSM 13] | UniRef100_Q65MF5 | Bacillus licheniformis DSM 13 |
| 935 | yfkA conserved protein YfkA | Hypothetical protein [Bacillus licheniformis DSM 13] | UniRef100_Q65MF4 | Bacillus licheniformis DSM 13 |
| 936 | yfjT conserved protein YfjT | Hypothetical protein yfjT [Bacillus licheniformis DSM 13] | UniRef100_Q65MF3 | Bacillus licheniformis DSM 13 |
| 937 | yfjS putative Carbohydrate Esterase Family 4 YfjS | YfjS [Bacillus licheniformis DSM 13] | UniRef100_Q65MF2 | Bacillus licheniformis DSM 13 |
| 938 | BLP00893 putative iron-binding oxidoreductase | Hypothetical protein [Bacillus licheniformis DSM 13] | UniRef100_Q65MF1 | Bacillus licheniformis DSM 13 |
| 939 | BLP00894 hypothetical protein | Hypothetical protein [Bacillus licheniformis DSM 13] | UniRef100_Q65MF0 | Bacillus licheniformis DSM 13 |
| 940 | corA Magnesium and cobalt transport protein CorA | YfjQ [Bacillus licheniformis DSM 13] | UniRef100_Q65ME9 | Bacillus licheniformis DSM 13 |
| 941 | yfjP putative DNA glycosylase | YfjP [Bacillus licheniformis DSM 13] | UniRef100_Q65ME8 | Bacillus licheniformis DSM 13 |
| 942 | yfjO tRNA (uracil-5-)-methyltransferase | YfjO [Bacillus licheniformis DSM 13] | UniRef100_Q65ME7 | Bacillus licheniformis DSM 13 |
| 943 | yfjM conserved protein YfjM | YfjM [Bacillus licheniformis DSM 13] | UniRef100_Q65ME6 | Bacillus licheniformis DSM 13 |
| 944 | BLP00900 conserved hypothetical protein | Hypothetical protein [Bacillus licheniformis DSM 13] | UniRef100_Q65ME5 | Bacillus licheniformis DSM 13 |
| 945 | BLP00901 hypothetical protein | Hypothetical protein [Bacillus licheniformis DSM 13] | UniRef100_Q65ME4 | Bacillus licheniformis DSM 13 |
| 946 | yfjL putative transporter YfjL | Hypothetical protein [Bacillus licheniformis DSM 13] | UniRef100_Q65ME3 | Bacillus licheniformis DSM 13 |
| 947 | yvkB probable transcriptional regulator | YvkB [Bacillus licheniformis DSM 13] | UniRef100_Q65ME2 | Bacillus licheniformis DSM 13 |
| 948 | ydhE UDP-glycosyltransferase,Glycosyl Transferase Family 1, YdhE | YdhE [Bacillus licheniformis DSM 13] | UniRef100_Q65ME1 | Bacillus licheniformis DSM 13 |
| 949 | BLP00905 hypothetical protein | YckD [Bacillus licheniformis DSM 13] | UniRef100_Q65ME0 | Bacillus licheniformis DSM 13 |

| 950 | ycel Sugar transporter superfamily Ycel | Ycel [Bacillus licheniformis DSM 13] | UniRef100_Q65MD9 | Bacillus licheniformis DSM 13 |
|---|---|---|---|---|
| 951 | sacX negative regulatory protein of SacY | SacX [Bacillus licheniformis DSM 13] | UniRef100_Q65MD8 | Bacillus licheniformis DSM 13 |
| 952 | sacY transcriptional antiterminator | SacY [Bacillus licheniformis DSM 13] | UniRef100_Q65MD7 | Bacillus licheniformis DSM 13 |
| 953 | ybcF Carbonic anhydrase YbcF | YbcF [Bacillus licheniformis DSM 13] | UniRef100_Q65MD6 | Bacillus licheniformis DSM 13 |
| 954 | ybcD conserved hypothetical protein YbcD | YbcD [Bacillus licheniformis DSM 13] | UniRef100_Q65MD5 | Bacillus licheniformis DSM 13 |
| 955 | ndhF NADH dehydrogenase (subunit 5) | NdhF [Bacillus licheniformis DSM 13] | UniRef100_Q65MD4 | Bacillus licheniformis DSM 13 |
| 956 | ybcI conserved protein YbcI | YbcI [Bacillus licheniformis DSM 13] | UniRef100_Q65MD3 | Bacillus licheniformis DSM 13 |
| 957 | BLP00913 hypothetical protein | | | |
| 958 | BLP00914 putative intracellular protease | Hypothetical protein [Bacillus licheniformis DSM 13] | UniRef100_Q65MD2 | Bacillus licheniformis DSM 13 |
| 959 | acoA acetoin dehydrogenase E1 component (TPP-dependent alpha subunit) | AcoA [Bacillus licheniformis DSM 13] | UniRef100_Q65MD1 | Bacillus licheniformis DSM 13 |
| 960 | acoB acetoin dehydrogenase E1 component (TPP-dependent beta subunit) | AcoB [Bacillus licheniformis DSM 13] | UniRef100_Q65MD0 | Bacillus licheniformis DSM 13 |
| 961 | acoC acetoin dehydrogenase E2 component (dihydrolipoamide acetyltransferase) | AcoC [Bacillus licheniformis DSM 13] | UniRef100_Q65MC9 | Bacillus licheniformis DSM 13 |
| 962 | acoL acetoin dehydrogenase E3 component (dihydrolipoamide dehydrogenase) | AcoL [Bacillus licheniformis DSM 13] | UniRef100_Q65MC8 | Bacillus licheniformis DSM 13 |
| 963 | acoR transcriptional regulator | AcoR [Bacillus licheniformis DSM 13] | UniRef100_Q65MC7 | Bacillus licheniformis DSM 13 |
| 964 | yfjF conserved membrane protein YfjF | Hypothetical protein yfjF [Bacillus licheniformis DSM 13] | UniRef100_Q62XR9 | Bacillus licheniformis DSM 13 |
| 965 | glvA Glycoside Hydrolase,Family 4, GlvA | MalA [Bacillus licheniformis DSM 13] | UniRef100_Q65MC5 | Bacillus licheniformis DSM 13 |
| 966 | glvR ATP-dependent helicase, DEAD-box | YfiA [Bacillus licheniformis DSM 13] | UniRef100_Q65MC4 | Bacillus licheniformis DSM 13 |
| 967 | malP phosphotransferase system (PTS) maltose-specific enzyme IICB component | Phosphotransferase system (PTS) maltose-specific enzyme IICB component [Bacillus licheniformis DSM 13] | UniRef100_Q62XR6 | Bacillus licheniformis DSM 13 |
| 968 | BLP00924 putative SAM methyltransferase | Hypothetical protein (Hypothetical SAM (And some other nucleotide) binding motif) [Bacillus licheniformis DSM 13] | UniRef100_Q65MC2 | Bacillus licheniformis DSM 13 |
| 969 | BLP00925 hypothetical protein | Hypothetical protein [Bacillus licheniformis DSM 13] | UniRef100_Q65MC1 | Bacillus licheniformis DSM 13 |
| 970 | yvaP possible transcriptional regulator YvaP | YodB [Bacillus licheniformis DSM 13] | UniRef100_Q65MC0 | Bacillus licheniformis DSM 13 |
| 971 | yfiD conserved membrane protein YfiD | YfiD [Bacillus licheniformis DSM 13] | UniRef100_Q65MB9 | Bacillus licheniformis DSM 13 |
| 972 | yfiE Glyoxalase I YfiE | YfiE [Bacillus licheniformis DSM 13] | UniRef100_Q65MB8 | Bacillus licheniformis DSM 13 |
| 973 | xynBA Glycoside Hydrolase Family 43 | Hypothetical protein [Bacillus licheniformis DSM 13] | UniRef100_Q65MB7 | Bacillus licheniformis DSM 13 |
| 974 | xynBB Glycoside Hydrolase Family 43 | XynB [Bacillus licheniformis DSM 13] | UniRef100_Q65MB6 | Bacillus licheniformis DSM 13 |
| 975 | yfiF probable transcriptional regulator YfiF | YfiF [Bacillus licheniformis DSM 13] | UniRef100_Q65MB5 | Bacillus licheniformis DSM 13 |
| 976 | BLP00932 putative extracellular solute-binding protein, family 1 | Hypothetical protein [Bacillus licheniformis DSM 13] | UniRef100_Q65MB4 | Bacillus licheniformis DSM 13 |
| 977 | BLP00933 hypothetical protein | | | |
| 978 | ydeQ NAD(P)H dehydrogenase (quinone) | YdeQ (NAD(P)H dehydrogenase) [Bacillus licheniformis DSM 13] | UniRef100_Q65MB3 | Bacillus licheniformis DSM 13 |
| 979 | yjhB hypothetical protein, putative hydrolase | YjhB [Bacillus licheniformis DSM 13] | UniRef100_Q65MB2 | Bacillus licheniformis DSM 13 |
| 980 | BLP00936 hypothetical protein | Hypothetical protein yfiT [Bacillus licheniformis DSM 13] | UniRef100_Q65MB1 | Bacillus licheniformis DSM 13 |

| 981 | yfiX conserved membrane protein YfiX | Hypothetical protein yfiX [Bacillus licheniformis DSM 13] | UniRef100_Q65MA9 | Bacillus licheniformis DSM 13 |
|---|---|---|---|---|
| 982 | BLP00938 hypothetical protein | | | |
| 983 | yfhB Phenazine biosynthesis-like protein | YfhB [Bacillus licheniformis DSM 13] | UniRef100_Q65MA8 | Bacillus licheniformis DSM 13 |
| 984 | yfhC Nitroreductase | Hypothetical protein [Bacillus licheniformis DSM 13] | UniRef100_Q65MA7 | Bacillus licheniformis DSM 13 |
| 985 | yfhD conserved hypothetical YfhD | Hypothetical protein yfhD [Bacillus licheniformis DSM 13] | UniRef100_Q65MA6 | Bacillus licheniformis DSM 13 |
| 986 | BLP00942 hypothetical protein | Hypothetical protein [Bacillus licheniformis DSM 13] | UniRef100_Q62XP8 | Bacillus licheniformis DSM 13 |
| 987 | BLP00943 putative acetyl transferase | Hypothetical protein [Bacillus licheniformis DSM 13] | UniRef100_Q65MA5 | Bacillus licheniformis DSM 13 |
| 988 | yfhF Conserved hypothetical protein, YfcH | YfhF [Bacillus licheniformis DSM 13] | UniRef100_Q65MA4 | Bacillus licheniformis DSM 13 |
| 989 | recX conserved protein RecX | YfhG [Bacillus licheniformis DSM 13] | UniRef100_Q65MA3 | Bacillus licheniformis DSM 13 |
| 990 | yfhH conserved protein YfhH | Hypothetical protein yfhH [Bacillus licheniformis DSM 13] | UniRef100_Q65MA2 | Bacillus licheniformis DSM 13 |
| 991 | BLP00947 hypothetical protein | | | |
| 992 | sspK small acid-soluble spore protein | SspK [Bacillus licheniformis DSM 13] | UniRef100_Q65MA1 | Bacillus licheniformis DSM 13 |
| 993 | yfhJ conserved protein YfhJ | Hypothetical protein yfhJ [Bacillus licheniformis DSM 13] | UniRef100_Q65MA0 | Bacillus licheniformis DSM 13 |
| 994 | BLP00950 hypothetical protein | | | |
| 995 | csbB stress response protein, Glycosyl Transferase Family 2 | CsbB [Bacillus licheniformis DSM 13] | UniRef100_Q65M99 | Bacillus licheniformis DSM 13 |
| 996 | BLP00952 putative transporter | Hypothetical protein [Bacillus licheniformis DSM 13] | UniRef100_Q65M98 | Bacillus licheniformis DSM 13 |
| 997 | yfhO conserved membrane protein YfhO | Hypothetical protein yfhO [Bacillus licheniformis DSM 13] | UniRef100_Q65M97 | Bacillus licheniformis DSM 13 |
| 998 | yfhP Predicted membrane-bound metal-dependent hydrolase | YfhP [Bacillus licheniformis DSM 13] | UniRef100_Q65M96 | Bacillus licheniformis DSM 13 |
| 999 | yfhQ putative A_G-specific adenine glycosylase YfhQ | YfhQ [Bacillus licheniformis DSM 13] | UniRef100_Q65M95 | Bacillus licheniformis DSM 13 |
| 1000 | yfhS conserved protein YhfS | Hypothetical protein yfhS [Bacillus licheniformis DSM 13] | UniRef100_Q65M94 | Bacillus licheniformis DSM 13 |
| 1001 | fabL enoyl-acyl carrier protein reductase | FabL [Bacillus licheniformis DSM 13] | UniRef100_Q65M93 | Bacillus licheniformis DSM 13 |
| 1002 | sspE small acid-soluble spore protein (gamma-type SASP) | Hypothetical protein [Bacillus licheniformis DSM 13] | UniRef100_Q65M92 | Bacillus licheniformis DSM 13 |
| 1003 | ygaB conserved hypothetical protein YgaB | YgaB [Bacillus licheniformis DSM 13] | UniRef100_Q65M91 | Bacillus licheniformis DSM 13 |
| 1004 | ygaC conserved hypothetical protein YgaC | YgaC [Bacillus licheniformis DSM 13] | UniRef100_Q65M90 | Bacillus licheniformis DSM 13 |
| 1005 | ygaD ABC transporter YgaD | YgaD [Bacillus licheniformis DSM 13] | UniRef100_Q65M89 | Bacillus licheniformis DSM 13 |
| 1006 | appDC oligopeptide ABC transporter (ATP-binding protein) | Hypothetical protein [Bacillus licheniformis DSM 13] | UniRef100_Q65M88 | Bacillus licheniformis DSM 13 |
| 1007 | appFC oligopeptide ABC transporter (ATP-binding protein) | Hypothetical protein [Bacillus licheniformis DSM 13] | UniRef100_Q65M87 | Bacillus licheniformis DSM 13 |
| 1008 | appAC oligopeptide ABC transporter (binding protein) | Hypothetical protein [Bacillus licheniformis DSM 13] | UniRef100_Q65M86 | Bacillus licheniformis DSM 13 |
| 1009 | appBC oligopeptide ABC transporter (permease) | Hypothetical protein [Bacillus licheniformis DSM 13] | UniRef100_Q65M85 | Bacillus licheniformis DSM 13 |
| 1010 | appCC oligopeptide ABC transporter (permease) | Hypothetical protein [Bacillus licheniformis DSM 13] | UniRef100_Q65M84 | Bacillus licheniformis DSM 13 |
| 1011 | ygaE conserved hypothetical protein YgaE | YgaE [Bacillus licheniformis DSM 13] | UniRef100_Q65M83 | Bacillus licheniformis DSM 13 |
| 1012 | gsaB glutamate-1-semialdehyde aminotransferase | GsaB [Bacillus licheniformis DSM 13] | UniRef100_Q65M82 | Bacillus licheniformis DSM 13 |
| 1013 | ygaF YgaF | YgaF [Bacillus licheniformis DSM 13] | UniRef100_Q65M81 | Bacillus licheniformis DSM 13 |
| 1014 | perR transcriptional regulator (Fur family) | PerR [Bacillus licheniformis DSM 13] | UniRef100_Q65M80 | Bacillus licheniformis DSM 13 |

| 1015 | BLP00970 hypothetical protein | | | |
|---|---|---|---|---|
| 1016 | ygzB conserved hypothetical protein ygzB | YgzB [Bacillus licheniformis DSM 13] | UniRef100_Q65M79 | Bacillus licheniformis DSM 13 |
| 1017 | ygxA conserved protein YgxA | Hypothetical protein ygxA [Bacillus licheniformis DSM 13] | UniRef100_Q65M78 | Bacillus licheniformis DSM 13 |
| 1018 | BLP00973 hypothetical protein | | | |
| 1019 | BLP00974 response regulator aspartate phosphatase | Hypothetical protein [Bacillus licheniformis DSM 13] | UniRef100_Q65M77 | Bacillus licheniformis DSM 13 |
| 1020 | BLP00975 hypothetical protein | Hypothetical protein [Bacillus licheniformis DSM 13] | UniRef100_Q65M75 | Bacillus licheniformis DSM 13 |
| 1021 | BLP00976 hypothetical protein | Hypothetical protein [Bacillus licheniformis DSM 13] | UniRef100_Q62XL7 | Bacillus licheniformis DSM 13 |
| 1022 | BLP00977 conserved hypothetical protein | Hypothetical protein [Bacillus licheniformis DSM 13] | UniRef100_Q65M74 | Bacillus licheniformis DSM 13 |
| 1023 | BLP00978 conserved hypothetical phagelike protein | Hypothetical protein [Bacillus licheniformis DSM 13] | UniRef100_Q62XL6 | Bacillus licheniformis DSM 13 |
| 1024 | BLP00979 hypothetical phage-like protein | Hypothetical protein [Bacillus licheniformis DSM 13] | UniRef100_Q62XL5 | Bacillus licheniformis DSM 13 |
| 1025 | BLP00980 N-acetylmuramoyl-L-alanine amidase | Hypothetical protein [Bacillus licheniformis DSM 13] | UniRef100_Q65M73 | Bacillus licheniformis DSM 13 |
| 1026 | BLP04848 conserved hypothetical protein | Hypothetical protein [Bacillus licheniformis DSM 13] | UniRef100_Q65M72 | Bacillus licheniformis DSM 13 |
| 1027 | BLP00981 transposase protein | Hypothetical protein [Bacillus licheniformis DSM 13] | UniRef100_Q65M71 | Bacillus licheniformis DSM 13 |
| 1028 | BLP04849 conserved hypothetical protein | Hypothetical protein [Bacillus licheniformis DSM 13] | UniRef100_Q62XL1 | Bacillus licheniformis DSM 13 |
| 1029 | BLP00982 hypothetical protein | Hypothetical protein [Bacillus licheniformis DSM 13] | UniRef100_Q65M70 | Bacillus licheniformis DSM 13 |
| 1030 | BLP00983 putative transposase | Hypothetical protein [Bacillus licheniformis DSM 13] | UniRef100_Q65M71 | Bacillus licheniformis DSM 13 |
| 1031 | rapE response regulator aspartate phosphatase | RapE [Bacillus licheniformis DSM 13] | UniRef100_Q65M68 | Bacillus licheniformis DSM 13 |
| 1032 | BLP00985 hypothetical protein | | | |
| 1033 | thrSA threonyl-tRNA synthetase | Hypothetical protein [Bacillus licheniformis DSM 13] | UniRef100_Q65M67 | Bacillus licheniformis DSM 13 |
| 1034 | BLP00987 hypothetical protein | BH3865 protein [Bacillus halodurans] | UniRef100_Q9K667 | Bacillus halodurans |
| 1035 | aroC 3-dehydroquinate dehydratase | AroC [Bacillus licheniformis DSM 13] | UniRef100_Q65M66 | Bacillus licheniformis DSM 13 |
| 1036 | thiC ThiC | ThiC [Bacillus licheniformis DSM 13] | UniRef100_Q65M65 | Bacillus licheniformis DSM 13 |
| 1037 | BLP00990 hypothetical protein | | | |
| 1038 | ssuB aliphatic sulfonate ABC transporter (binding protein) | SsuB [Bacillus licheniformis DSM 13] | UniRef100_Q65M64 | Bacillus licheniformis DSM 13 |
| 1039 | ssuA aliphatic sulfonate ABC transporter (binding lipoprotein) | SsuA [Bacillus licheniformis DSM 13] | UniRef100_Q65M63 | Bacillus licheniformis DSM 13 |
| 1040 | ssuC aliphatic sulfonate ABC transporter (permease) | SsuC [Bacillus licheniformis DSM 13] | UniRef100_Q65M62 | Bacillus licheniformis DSM 13 |
| 1041 | ssuD aliphatic sulfonate monooxygenase | SsuD [Bacillus licheniformis DSM 13] | UniRef100_Q65M61 | Bacillus licheniformis DSM 13 |
| 1042 | ygaO conserved hypothetical protein YgaO | YgaO [Bacillus licheniformis DSM 13] | UniRef100_Q65M60 | Bacillus licheniformis DSM 13 |
| 1043 | yazB putative DNA binding protein YazB | YazB [Bacillus licheniformis DSM 13] | UniRef100_Q65M59 | Bacillus licheniformis DSM 13 |
| 1044 | BLP00997 conserved hypothetical protein | Hypothetical protein [Bacillus licheniformis DSM 13] | UniRef100_Q65M58 | Bacillus licheniformis DSM 13 |
| 1045 | BLP00998 ABC transporter | Hypothetical protein [Bacillus licheniformis DSM 13] | UniRef100_Q65M57 | Bacillus licheniformis DSM 13 |
| 1046 | BLP00999 hypothetical protein | Hypothetical protein [Bacillus licheniformis DSM 13] | UniRef100_Q65M56 | Bacillus licheniformis DSM 13 |
| 1047 | BLP01000 putative metallopeptidase | Hypothetical protein [Bacillus licheniformis DSM 13] | UniRef100_Q65M55 | Bacillus licheniformis DSM 13 |
| 1048 | BLP01001 hypothetical protein with conserved domain | Hypothetical protein [Bacillus licheniformis DSM 13] | UniRef100_Q65M54 | Bacillus licheniformis DSM 13 |
| 1049 | rpsNB 30S ribosomal protein S14-2 | YhzA [Bacillus licheniformis DSM 13] | UniRef100_Q65M53 | Bacillus licheniformis DSM 13 |

| 1050 | BLP01003 conserved hypothetical protein | YhzB [Bacillus licheniformis DSM 13] | UniRef100_Q65M52 | Bacillus licheniformis DSM 13 |
|---|---|---|---|---|
| 1051 | yhbA 4Fe-4S cluster binding putative ferredoxin | YhbA [Bacillus licheniformis DSM 13] | UniRef100_Q65M51 | Bacillus licheniformis DSM 13 |
| 1052 | yhbB conserved hypothetical protein yhbB | YhbB [Bacillus licheniformis DSM 13] | UniRef100_Q65M50 | Bacillus licheniformis DSM 13 |
| 1053 | cspR rRNA methylase homolog | CspR [Bacillus licheniformis DSM 13] | UniRef100_Q65M49 | Bacillus licheniformis DSM 13 |
| 1054 | yhbD Putative DNA binding protein YhbD | Putative DNA binding [Bacillus licheniformis DSM 13] | UniRef100_Q62XI6 | Bacillus licheniformis DSM 13 |
| 1055 | yhbE putative cytosolic protein YhbE | YhbE [Bacillus licheniformis DSM 13] | UniRef100_Q65M47 | Bacillus licheniformis DSM 13 |
| 1056 | yhbF conserved hypothetical protein YhbF | YhbF [Bacillus licheniformis DSM 13] | UniRef100_Q65M46 | Bacillus licheniformis DSM 13 |
| 1057 | BLP01010 hypothetical protein | Hypothetical protein [Bacillus licheniformis DSM 13] | UniRef100_Q65M45 | Bacillus licheniformis DSM 13 |
| 1058 | BLP01011 hypothetical protein | | | |
| 1059 | prkA serine protein kinase | PrkA [Bacillus licheniformis DSM 13] | UniRef100_Q65M44 | Bacillus licheniformis DSM 13 |
| 1060 | yhbH putative stress response protein YhbH | YhbH [Bacillus licheniformis DSM 13] | UniRef100_Q65M43 | Bacillus licheniformis DSM 13 |
| 1061 | yhbJ putative transporter YhbJ | YhbJ [Bacillus licheniformis DSM 13] | UniRef100_Q65M42 | Bacillus licheniformis DSM 13 |
| 1062 | yhcA Drug resistance transporter YhcA | YhcA [Bacillus licheniformis DSM 13] | UniRef100_Q65M41 | Bacillus licheniformis DSM 13 |
| 1063 | yhcB putative Flavodoxin_nitric oxide synthase YhcB | YhcB [Bacillus licheniformis DSM 13] | UniRef100_Q65M40 | Bacillus licheniformis DSM 13 |
| 1064 | yhcC conserved hypothetical protein YhcC | Hypothetical protein [Bacillus licheniformis DSM 13] | UniRef100_Q65M39 | Bacillus licheniformis DSM 13 |
| 1065 | yhcG ABC transporter YhcG | Hypothetical protein [Bacillus licheniformis DSM 13] | UniRef100_Q62XH6 | Bacillus licheniformis DSM 13 |
| 1066 | yhcH ABC transporter YhcH | YhcH [Bacillus licheniformis DSM 13] | UniRef100_Q65M37 | Bacillus licheniformis DSM 13 |
| 1067 | yhcI putative permease YhcI | YhcI [Bacillus licheniformis DSM 13] | UniRef100_Q65M36 | Bacillus licheniformis DSM 13 |
| 1068 | cspB major cold-shock protein | CspB [Bacillus licheniformis DSM 13] | UniRef100_Q65M35 | Bacillus licheniformis DSM 13 |
| 1069 | BLP01023 ABC transporter | Hypothetical protein [Bacillus licheniformis DSM 13] | UniRef100_Q65M34 | Bacillus licheniformis DSM 13 |
| 1070 | BLP01024 Binding-protein-dependent transport systems inner membrane component | Hypothetical protein [Bacillus licheniformis DSM 13] | UniRef100_Q65M33 | Bacillus licheniformis DSM 13 |
| 1071 | yhcJ ABC transport system protein YhcJ | YhcJ [Bacillus licheniformis DSM 13] | UniRef100_Q65M32 | Bacillus licheniformis DSM 13 |
| 1072 | yhcL Sodium:dicarboxylate symporter YhcL | YhcL [Bacillus licheniformis DSM 13] | UniRef100_Q65M31 | Bacillus licheniformis DSM 13 |
| 1073 | yhcM conserved hypothetical protein YhcM | Hypothetical protein [Bacillus licheniformis DSM 13] | UniRef100_Q65M30 | Bacillus licheniformis DSM 13 |
| 1074 | BLP01028 putative amine dehydrogenase | Hypothetical protein [Bacillus licheniformis DSM 13] | UniRef100_Q65M29 | Bacillus licheniformis DSM 13 |
| 1075 | yhcN conserved hypothetical protein YhcN | YhcN [Bacillus licheniformis DSM 13] | UniRef100_Q65M28 | Bacillus licheniformis DSM 13 |
| 1076 | yhcP conserved hypothetical protein YhcP | YhcP [Bacillus licheniformis DSM 13] | UniRef100_Q65M27 | Bacillus licheniformis DSM 13 |
| 1077 | yhcQ conserved hypothetical protein YhcQ | YhcQ [Bacillus licheniformis DSM 13] | UniRef100_Q65M26 | Bacillus licheniformis DSM 13 |
| 1078 | BLP01032 hypothetical protein | | | |
| 1079 | kinEA two-component sensor histidine kinase | Hypothetical protein [Bacillus licheniformis DSM 13] | UniRef100_Q65M25 | Bacillus licheniformis DSM 13 |
| 1080 | BLP01034 hypothetical protein | | | |
| 1081 | yhcR Nucleic acid-binding hydrolase YhcR | YhcR [Bacillus licheniformis DSM 13] | UniRef100_Q65M24 | Bacillus licheniformis DSM 13 |
| 1082 | yhcS putative transferase YhcS | YhcS [Bacillus licheniformis DSM 13] | UniRef100_Q65M23 | Bacillus licheniformis DSM 13 |
| 1083 | yhcT putative Pseudouridine synthase YhcT | YhcT [Bacillus licheniformis DSM 13] | UniRef100_Q65M22 | Bacillus licheniformis DSM 13 |
| 1084 | yhcU conserved hypothetical protein YhcU | YhcU [Bacillus licheniformis DSM 13] | UniRef100_Q65M21 | Bacillus licheniformis DSM 13 |

| 1085 | yhcV ABC transporter YhcV | YhcV [Bacillus licheniformis DSM 13] | UniRef100_Q65M20 | Bacillus licheniformis DSM 13 |
|---|---|---|---|---|
| 1086 | yhcW HAD-superfamily hydrolase YhcW | YhcW [Bacillus licheniformis DSM 13] | UniRef100_Q65M19 | Bacillus licheniformis DSM 13 |
| 1087 | yhcX conserved hypothetical protein YhcX | YhcX [Bacillus licheniformis DSM 13] | UniRef100_Q65M18 | Bacillus licheniformis DSM 13 |
| 1088 | BLP04710 hypothetical protein | | | |
| 1089 | BLP01042 ABC transporter | Hypothetical protein [Bacillus licheniformis DSM 13] | UniRef100_Q65M17 | Bacillus licheniformis DSM 13 |
| 1090 | yrhG Formate_nitrite transporter | YrhG [Bacillus licheniformis DSM 13] | UniRef100_Q65M16 | Bacillus licheniformis DSM 13 |
| 1091 | BLP01044 hypothetical protein | | | |
| 1092 | BLP01045 putative permease | Hypothetical protein [Bacillus licheniformis DSM 13] | UniRef100_Q65M15 | Bacillus licheniformis DSM 13 |
| 1093 | yogA alcohol dehydrogenase YogA | YogA [Bacillus licheniformis DSM 13] | UniRef100_Q65M14 | Bacillus licheniformis DSM 13 |
| 1094 | glpP transcription antiterminator | GlpP [Bacillus licheniformis DSM 13] | UniRef100_Q65M13 | Bacillus licheniformis DSM 13 |
| 1095 | glpF glycerol uptake facilitator | GlpF [Bacillus licheniformis DSM 13] | UniRef100_Q65M12 | Bacillus licheniformis DSM 13 |
| 1096 | glpK glycerol kinase | GlpK [Bacillus licheniformis DSM 13] | UniRef100_Q65M11 | Bacillus licheniformis DSM 13 |
| 1097 | glpD glycerol-3-phosphate dehydrogenase | GlpD [Bacillus licheniformis DSM 13] | UniRef100_Q65M10 | Bacillus licheniformis DSM 13 |
| 1098 | BLP01051 hypothetical protein | | | |
| 1099 | pgcA Alpha Phosphoglucomutase PgcA | YhxB [Bacillus licheniformis DSM 13] | UniRef100_Q65M09 | Bacillus licheniformis DSM 13 |
| 1100 | BLP01053 hypothetical protein | Hypothetical protein [Bacillus licheniformis DSM 13] | UniRef100_Q65M08 | Bacillus licheniformis DSM 13 |
| 1101 | BLP01054 putative transcriptional regulator | Hypothetical protein [Bacillus licheniformis DSM 13] | UniRef100_Q65M07 | Bacillus licheniformis DSM 13 |
| 1102 | yhcY ATP-binding region protein | ATP-binding region, ATPase-like,GAF [Bacillus licheniformis DSM 13] | UniRef100_Q62XE3 | Bacillus licheniformis DSM 13 |
| 1103 | yhcZ putative transcriptional regulator YhcZ | YhcZ [Bacillus licheniformis DSM 13] | UniRef100_Q65M05 | Bacillus licheniformis DSM 13 |
| 1104 | yhdA putative NADPH-dependent FMN reductase YhdA | NADPH-dependent FMN reductase [Bacillus licheniformis DSM 13] | UniRef100_Q62XE1 | Bacillus licheniformis DSM 13 |
| 1105 | yhdB conserved hypothetical protein YhdB | YhdB [Bacillus licheniformis DSM 13] | UniRef100_Q65M03 | Bacillus licheniformis DSM 13 |
| 1106 | yhdC conserved hypothetical protein | YhdC [Bacillus licheniformis DSM 13] | UniRef100_Q65M02 | Bacillus licheniformis DSM 13 |
| 1107 | yhdE conserved hypothetical protein YhdE | YhdE [Bacillus licheniformis DSM 13] | UniRef100_Q65M01 | Bacillus licheniformis DSM 13 |
| 1108 | BLP04711 hypothetical protein | | | |
| 1109 | hmp flavohemoglobin | Hmp [Bacillus licheniformis DSM 13] | UniRef100_Q65M00 | Bacillus licheniformis DSM 13 |
| 1110 | spoVR Stage V sporulation protein R, spoVR | Hypothetical protein spoVR [Bacillus licheniformis DSM 13] | UniRef100_Q65LZ9 | Bacillus licheniformis DSM 13 |
| 1111 | BLP01063 hypothetical protein | | | |
| 1112 | BLP04712 hypothetical protein | | | |
| 1113 | lytE cell wall hydrolase phosphatase-associated protein | LytE [Bacillus licheniformis DSM 13] | UniRef100_Q65LZ8 | Bacillus licheniformis DSM 13 |
| 1114 | citR transcriptional regulator CitR | CitR [Bacillus licheniformis DSM 13] | UniRef100_Q65LZ7 | Bacillus licheniformis DSM 13 |
| 1115 | citA citrate synthase I | CitA [Bacillus licheniformis DSM 13] | UniRef100_Q65LZ6 | Bacillus licheniformis DSM 13 |
| 1116 | BLP01067 putative glucose dehydrogenase | Hypothetical Soluble quinoprotein glucose dehydrogenase [Bacillus licheniformis DSM 13] | UniRef100_Q65LZ5 | Bacillus licheniformis DSM 13 |
| 1117 | yhdF Short-chain dehydrogenase_reductase SDR YhdF | YhdF [Bacillus licheniformis DSM 13] | UniRef100_Q65LZ4 | Bacillus licheniformis DSM 13 |

| 1118 | BLP01069 hypothetical protein | | | |
|------|-------------------------------|---|---|---|
| 1119 | yhdH putative Sodium:neurotransmitter symporter YhdH | YhdH [Bacillus licheniformis DSM 13] | UniRef100_Q65LZ3 | Bacillus licheniformis DSM 13 |
| 1120 | BLP01071 putative aminoacid decarboxylase | Hypothetical protein [Bacillus licheniformis DSM 13] | UniRef100_Q65LZ2 | Bacillus licheniformis DSM 13 |
| 1121 | ydeE putative transcriptional regulator YdeE | YdeE [Bacillus licheniformis DSM 13] | UniRef100_Q65LZ1 | Bacillus licheniformis DSM 13 |
| 1122 | ydeL conserved hypothetical YdeL | YdeL [Bacillus licheniformis DSM 13] | UniRef100_Q65LZ0 | Bacillus licheniformis DSM 13 |
| 1123 | yhdJ GCN5-related N-acetyltransferase | YhdJ [Bacillus licheniformis DSM 13] | UniRef100_Q65LY9 | Bacillus licheniformis DSM 13 |
| 1124 | yhdK conserved hypothetical protein | YhdK [Bacillus licheniformis DSM 13] | UniRef100_Q65LY8 | Bacillus licheniformis DSM 13 |
| 1125 | yhdL conserved hypothetical protein | YhdL [Bacillus licheniformis DSM 13] | UniRef100_Q65LY7 | Bacillus licheniformis DSM 13 |
| 1126 | sigM RNA polymerase ECF(extracytoplasmic function)-type sigma factor (sigma-M) | SigM (RNA polymerase ECF(Extracytoplasmic function)-type sigma factor) [Bacillus licheniformis DSM 13] | UniRef100_Q65LY6 | Bacillus licheniformis DSM 13 |
| 1127 | yrkC conserved hypothetical protein | Hypothetical protein [Bacillus licheniformis DSM 13] | UniRef100_Q65LY5 | Bacillus licheniformis DSM 13 |
| 1128 | BLP01079 hypothetical protein | | | |
| 1129 | plsC 1-acylglycerol-3-phosphate O-acyltransferase | YhdO [Bacillus licheniformis DSM 13] | UniRef100_Q65LY4 | Bacillus licheniformis DSM 13 |
| 1130 | BLP01081 hypothetical protein | | | |
| 1131 | BLP01082 conserved hypothetical protein | Hypothetical protein [Bacillus licheniformis DSM 13] | UniRef100_Q65LY3 | Bacillus licheniformis DSM 13 |
| 1132 | BLP01083 conserved hypothetical protein | Hypothetical protein [Bacillus licheniformis DSM 13] | UniRef100_Q65LY2 | Bacillus licheniformis DSM 13 |
| 1133 | BLP01084 putative transcriptional regulator | Hypothetical protein [Bacillus licheniformis DSM 13] | UniRef100_Q65LY1 | Bacillus licheniformis DSM 13 |
| 1134 | yhdP CBS domain, conserved hypothetical protein YhdP | YhdP [Bacillus licheniformis DSM 13] | UniRef100_Q65LY0 | Bacillus licheniformis DSM 13 |
| 1135 | cueR transcriptional activator | YhdQ [Bacillus licheniformis DSM 13] | UniRef100_Q65LX9 | Bacillus licheniformis DSM 13 |
| 1136 | yhdT CBS domain conserved hypothetical protein YhdT | YhdT [Bacillus licheniformis DSM 13] | UniRef100_Q65LX8 | Bacillus licheniformis DSM 13 |
| 1137 | BLP01088 hypothetical protein | Hypothetical protein [Bacillus licheniformis DSM 13] | UniRef100_Q65LX7 | Bacillus licheniformis DSM 13 |
| 1138 | BLP01089 hypothetical protein | Hypothetical protein [Bacillus licheniformis DSM 13] | UniRef100_Q65LX6 | Bacillus licheniformis DSM 13 |
| 1139 | BLP01090 N-acetylmuramoyl-L-alanine amidase | Hypothetical protein [Bacillus licheniformis DSM 13] | UniRef100_Q65LX5 | Bacillus licheniformis DSM 13 |
| 1140 | crcB1 integral membrane protein possibly involved in chromosome condensation | YhdU [Bacillus licheniformis DSM 13] | UniRef100_Q65LX4 | Bacillus licheniformis DSM 13 |
| 1141 | crcB2 Protein secE_sec61-gamma protein | Hypothetical protein [Bacillus licheniformis DSM 13] | UniRef100_Q65LX3 | Bacillus licheniformis DSM 13 |
| 1142 | yhdW Glycerophosphoryl diester phosphodiesterase YhdW | YhdW [Bacillus licheniformis DSM 13] | UniRef100_Q65LX2 | Bacillus licheniformis DSM 13 |
| 1143 | yhdX conserved hypothetical YhdX | YhdX [Bacillus licheniformis DSM 13] | UniRef100_Q65LX1 | Bacillus licheniformis DSM 13 |
| 1144 | yhdY putative mechanosensitive ion channel protein, conserved hypothetical YhdY | YhdY [Bacillus licheniformis DSM 13] | UniRef100_Q65LX0 | Bacillus licheniformis DSM 13 |
| 1145 | npdA NdpA | YhdZ [Bacillus licheniformis DSM 13] | UniRef100_Q65LW9 | Bacillus licheniformis DSM 13 |
| 1146 | BLP01097 N-acetyltransferase | Hypothetical protein [Bacillus licheniformis DSM 13] | UniRef100_Q65LW8 | Bacillus licheniformis DSM 13 |
| 1147 | BLP01098 Polysaccharide deacetylase, Carbohydrate Esterase Family 4 | YheN [Bacillus licheniformis DSM 13] | UniRef100_Q65LW7 | Bacillus licheniformis DSM 13 |
| 1148 | BLP01099 hypothetical protein | | | |
| 1149 | dat D-alanine aminotransferase | Dat [Bacillus licheniformis DSM 13] | UniRef100_Q65LW6 | Bacillus licheniformis DSM 13 |

| 1150 | nhaC Na _H  antiporter | NhaC [Bacillus licheniformis DSM 13] | UniRef100_Q65LW5 | Bacillus licheniformis DSM 13 |
|------|------------------------|--------------------------------------|------------------|-------------------------------|
| 1151 | BLP01102 hypothetical protein | | | |
| 1152 | yoxA conserved hypothetical protein YoxA | YoxA [Bacillus licheniformis DSM 13] | UniRef100_Q65LW4 | Bacillus licheniformis DSM 13 |
| 1153 | BLP01105 hypothetical protein | | | |
| 1154 | BLP01106 hypothetical protein | Hypothetical protein [Bacillus thuringiensis] | UniRef100_Q6HKJ9 | Bacillus thuringiensis |
| 1155 | ydhH hypothetical protein | YdhH [Bacillus licheniformis DSM 13] | UniRef100_Q65LW3 | Bacillus licheniformis DSM 13 |
| 1156 | BLP01108 conserved hypothetical protein | Hypothetical protein [Bacillus licheniformis DSM 13] | UniRef100_Q65LW2 | Bacillus licheniformis DSM 13 |
| 1157 | BLP01109 hypothetical protein | Hypothetical protein [Bacillus licheniformis DSM 13] | UniRef100_Q65LW1 | Bacillus licheniformis DSM 13 |
| 1158 | BLP01110 hypothetical protein | | | |
| 1159 | nhaX stress response protein, NhaX | Hypothetical protein nhaX [Bacillus licheniformis DSM 13] | UniRef100_Q65LW0 | Bacillus licheniformis DSM 13 |
| 1160 | BLP01112 hypothetical protein | | | |
| 1161 | yheI ABC transporter YheI | YheI [Bacillus licheniformis DSM 13] | UniRef100_Q65LV9 | Bacillus licheniformis DSM 13 |
| 1162 | yheH ABC transporter YheH | YheH [Bacillus licheniformis DSM 13] | UniRef100_Q65LV8 | Bacillus licheniformis DSM 13 |
| 1163 | yheG conserved hypothetical protein | Hypothetical protein [Bacillus licheniformis DSM 13] | UniRef100_Q62X94 | Bacillus licheniformis DSM 13 |
| 1164 | BLP01116 hypothetical protein | | | |
| 1165 | sspB small acid-soluble spore protein (beta-type) | SspB [Bacillus licheniformis DSM 13] | UniRef100_Q65LV6 | Bacillus licheniformis DSM 13 |
| 1166 | BLP01118 hypothetical protein | Hypothetical protein [Bacillus licheniformis DSM 13] | UniRef100_Q65LV5 | Bacillus licheniformis DSM 13 |
| 1167 | msmX multiple sugar-binding transport ATP-binding protein | MsmX [Bacillus licheniformis DSM 13] | UniRef100_Q65LV4 | Bacillus licheniformis DSM 13 |
| 1168 | BLP01120 hypothetical protein with helix-turn-helix domain | YxkF [Bacillus licheniformis DSM 13] | UniRef100_Q65LV3 | Bacillus licheniformis DSM 13 |
| 1169 | yheE conserved hypothetical protein YheE | YheE [Bacillus licheniformis DSM 13] | UniRef100_Q65LV2 | Bacillus licheniformis DSM 13 |
| 1170 | BLP01122 hypothetical protein | | | |
| 1171 | yheD conserved hypothetical protein YheD | YheD [Bacillus licheniformis DSM 13] | UniRef100_Q65LV1 | Bacillus licheniformis DSM 13 |
| 1172 | yheC conserved hypothetical protein YheC | YheC [Bacillus licheniformis DSM 13] | UniRef100_Q65LV0 | Bacillus licheniformis DSM 13 |
| 1173 | yheB conserved hypothetical protein YheB | Hypothetical protein [Bacillus licheniformis DSM 13] | UniRef100_Q62X86 | Bacillus licheniformis DSM 13 |
| 1174 | yheA conserved hypothetical protein YheA | YheA [Bacillus licheniformis DSM 13] | UniRef100_Q65LU8 | Bacillus licheniformis DSM 13 |
| 1175 | BLP01127 hypothetical protein | | | |
| 1176 | BLP04814 unassigned | Hypothetical protein [Bacillus licheniformis DSM 13] | UniRef100_Q65LU7 | Bacillus licheniformis DSM 13 |
| 1177 | BLP01128 hypothetical protein | | | |
| 1178 | yhaX HAD-superfamily hydrolase protein YhaX | YhaX [Bacillus licheniformis DSM 13] | UniRef100_Q65LU6 | Bacillus licheniformis DSM 13 |
| 1179 | hemZ coproporphyrinogen III oxidase | Coproporphyrinogen III oxidase [Bacillus licheniformis DSM 13] | UniRef100_Q62X83 | Bacillus licheniformis DSM 13 |
| 1180 | BLP01131 hypothetical protein | Hypothetical protein yhaU [Bacillus subtilis] | UniRef100_O07536 | Bacillus subtilis |
| 1181 | BLP01132 hypothetical protein | Hypothetical protein yhaT [Bacillus subtilis] | UniRef100_O07535 | Bacillus subtilis |
| 1182 | yhaR Enoyl-CoA hydratase_isomerase YhaR | YhaR [Bacillus licheniformis DSM 13] | UniRef100_Q65LU4 | Bacillus licheniformis DSM 13 |
| 1183 | BLP01134 hypothetical protein | | | |
| 1184 | BLP01135 conserved hypothetical protein | Hypothetical protein [Bacillus licheniformis DSM 13] | UniRef100_Q65LU2 | Bacillus licheniformis DSM 13 |

| 1185 | BLP01136 hypothetical protein | Hypothetical protein [Bacillus licheniformis DSM 13] | UniRef100_Q65LU1 | Bacillus licheniformis DSM 13 |
|---|---|---|---|---|
| 1186 | yhaQ ABC transporter YhaQ | YhaQ [Bacillus licheniformis DSM 13] | UniRef100_Q65LU0 | Bacillus licheniformis DSM 13 |
| 1187 | yhaP conserved hypothetical protein YhaP | YhaP [Bacillus licheniformis DSM 13] | UniRef100_Q65LT9 | Bacillus licheniformis DSM 13 |
| 1188 | yhaO conserved hypothetical protein YhaO | YhaO [Bacillus licheniformis DSM 13] | UniRef100_Q65LT8 | Bacillus licheniformis DSM 13 |
| 1189 | yhaN conserved hypothetical protein YhaN | YhaN [Bacillus licheniformis DSM 13] | UniRef100_Q65LT7 | Bacillus licheniformis DSM 13 |
| 1190 | yhaM Nucleic acid-binding protein YhaM | YhaM [Bacillus licheniformis DSM 13] | UniRef100_Q65LT6 | Bacillus licheniformis DSM 13 |
| 1191 | yhaL conserved hypothetical protein YhaL | YhaL [Bacillus licheniformis DSM 13] | UniRef100_Q65LT5 | Bacillus licheniformis DSM 13 |
| 1192 | prsA molecular chaperone PrsA | PrsA [Bacillus licheniformis DSM 13] | UniRef100_Q65LT4 | Bacillus licheniformis DSM 13 |
| 1193 | BLP04850 conserved hypothetical protein | | | |
| 1194 | BLP01144 conserved hypothetical protein | Hypothetical protein [Bacillus licheniformis DSM 13] | UniRef100_Q65LT1 | Bacillus licheniformis DSM 13 |
| 1195 | yhaK conserved hypothetical protein YhaK | Hypothetical protein [Bacillus licheniformis DSM 13] | UniRef100_Q65LT0 | Bacillus licheniformis DSM 13 |
| 1196 | yhaI conserved hypothetical protein YhaI | YhaI [Bacillus licheniformis DSM 13] | UniRef100_Q65LS9 | Bacillus licheniformis DSM 13 |
| 1197 | hpr transcriptional regulator Hpr | Hpr [Bacillus licheniformis DSM 13] | UniRef100_Q65LS8 | Bacillus licheniformis DSM 13 |
| 1198 | yhaH conserved hypothetical protein YhaH | YhaH [Bacillus licheniformis DSM 13] | UniRef100_Q65LS7 | Bacillus licheniformis DSM 13 |
| 1199 | BLP01149 conserved hypothetical protein | Hypothetical protein [Bacillus licheniformis DSM 13] | UniRef100_Q65LS6 | Bacillus licheniformis DSM 13 |
| 1200 | trpP possible transmembrane protein involved in tryptophan transport | YhaG [Bacillus licheniformis DSM 13] | UniRef100_Q65LS5 | Bacillus licheniformis DSM 13 |
| 1201 | serC phosphoserine aminotransferase | SerC [Bacillus licheniformis DSM 13] | UniRef100_Q65LS4 | Bacillus licheniformis DSM 13 |
| 1202 | hit cell-cycle regulation protein- Hit | Hit [Bacillus licheniformis DSM 13] | UniRef100_Q65LS3 | Bacillus licheniformis DSM 13 |
| 1203 | BLP01153 hypothetical protein | Hypothetical protein [Bacillus licheniformis DSM 13] | UniRef100_Q65LS2 | Bacillus licheniformis DSM 13 |
| 1204 | ecsA ABC transporter (ATP-binding protein) | EcsA [Bacillus licheniformis DSM 13] | UniRef100_Q65LS1 | Bacillus licheniformis DSM 13 |
| 1205 | ecsB ABC transporter (membrane protein) | EcsB [Bacillus licheniformis DSM 13] | UniRef100_Q65LS0 | Bacillus licheniformis DSM 13 |
| 1206 | ecsC EscC | Hypothetical protein ecsC [Bacillus licheniformis DSM 13] | UniRef100_Q65LR9 | Bacillus licheniformis DSM 13 |
| 1207 | yhaA putative amidohydrolase YhaA | YhaA [Bacillus licheniformis DSM 13] | UniRef100_Q65LR8 | Bacillus licheniformis DSM 13 |
| 1208 | BLP04713 hypothetical protein | | | |
| 1209 | yhfA conserved hypothetical protein YhfA | YhfA [Bacillus licheniformis DSM 13] | UniRef100_Q65LR7 | Bacillus licheniformis DSM 13 |
| 1210 | yhgC conserved hypothetical protein YhgC | YhgC [Bacillus licheniformis DSM 13] | UniRef100_Q65LR6 | Bacillus licheniformis DSM 13 |
| 1211 | pbpF penicillin-binding protein,putative glycosyl transferase family 51 | PbpF [Bacillus licheniformis DSM 13] | UniRef100_Q65LR5 | Bacillus licheniformis DSM 13 |
| 1212 | hemE uroporphyrinogen III decarboxylase | HemE [Bacillus licheniformis DSM 13] | UniRef100_Q65LR4 | Bacillus licheniformis DSM 13 |
| 1213 | hemH ferrochelatase | HemH [Bacillus licheniformis DSM 13] | UniRef100_Q65LR3 | Bacillus licheniformis DSM 13 |
| 1214 | hemY protoporphyrinogen IX and coproporphyrinogen III oxidase | HemY [Bacillus licheniformis DSM 13] | UniRef100_Q65LR2 | Bacillus licheniformis DSM 13 |
| 1215 | BLP01164 hypothetical protein | | | |
| 1216 | yhgD putative transcriptional regulator | YhgD [Bacillus licheniformis DSM 13] | UniRef100_Q65LR1 | Bacillus licheniformis DSM 13 |
| 1217 | yhgE conserved hypothetical protein YhgE | YhgE [Bacillus licheniformis DSM 13] | UniRef100_Q65LR0 | Bacillus licheniformis DSM 13 |
| 1218 | fabHB beta-ketoacyl-acyl carrier protein synthase III | FabHB [Bacillus licheniformis DSM 13] | UniRef100_Q65LQ9 | Bacillus licheniformis DSM 13 |
| 1219 | yhfE putative glucanase_aminopeptidase YhfE | YhfE [Bacillus licheniformis DSM 13] | UniRef100_Q65LQ8 | Bacillus licheniformis DSM 13 |

| 1220 | BLP01169 hypothetical protein | | | |
|---|---|---|---|---|
| 1221 | gltT proton_sodium-glutamate symport protein | GltT [Bacillus licheniformis DSM 13] | UniRef100_Q65LQ6 | Bacillus licheniformis DSM 13 |
| 1222 | BLP04815 conserved hypothetical YfhH | Hypothetical protein yhfH [Bacillus subtilis] | UniRef100_O07606 | Bacillus subtilis |
| 1223 | yhfI putative hydrolase. conserved hypothetical protein | YhfI [Bacillus licheniformis DSM 13] | UniRef100_Q65LQ5 | Bacillus licheniformis DSM 13 |
| 1224 | yhfJ putative Lipoyltransferase and lipoate-protein ligase | YhfJ [Bacillus licheniformis DSM 13] | UniRef100_Q65LQ4 | Bacillus licheniformis DSM 13 |
| 1225 | yhfL AMP-dependent synthetase and ligase | YhfL [Bacillus licheniformis DSM 13] | UniRef100_Q65LQ3 | Bacillus licheniformis DSM 13 |
| 1226 | yhfM conserved hypothetical protein | Hypothetical protein [Bacillus licheniformis DSM 13] | UniRef100_Q65LQ2 | Bacillus licheniformis DSM 13 |
| 1227 | BLP01175 putative transcriptional regulator | Hypothetical protein [Bacillus licheniformis DSM 13] | UniRef100_Q65LQ1 | Bacillus licheniformis DSM 13 |
| 1228 | azlC putative branched chain aminoacid transporter | Hypothetical protein azlC [Bacillus licheniformis DSM 13] | UniRef100_Q65LQ0 | Bacillus licheniformis DSM 13 |
| 1229 | BLP01177 conserved hypothetical protein | Hypothetical protein [Bacillus licheniformis DSM 13] | UniRef100_Q65LP9 | Bacillus licheniformis DSM 13 |
| 1230 | yhfN putative metallopeptidase YhfN | YhfN [Bacillus licheniformis DSM 13] | UniRef100_Q65LP8 | Bacillus licheniformis DSM 13 |
| 1231 | kerA KerA | Subtilisin Carlsberg precursor [Bacillus licheniformis] | UniRef100_P00780 | Bacillus licheniformis |
| 1232 | ydeD putative membrane protein, transporter YdeD | YdeD [Bacillus licheniformis DSM 13] | UniRef100_Q65LP6 | Bacillus licheniformis DSM 13 |
| 1233 | yhfQ putative transferase | YhfQ [Bacillus licheniformis DSM 13] | UniRef100_Q65LP5 | Bacillus licheniformis DSM 13 |
| 1234 | yfmD putative transport system permease protein YfmD | YfmD [Bacillus licheniformis DSM 13] | UniRef100_Q65LP4 | Bacillus licheniformis DSM 13 |
| 1235 | yfmE conserved hypothetical YfmE | YfmE [Bacillus licheniformis DSM 13] | UniRef100_Q65LP3 | Bacillus licheniformis DSM 13 |
| 1236 | yhfR putative Phosphoglycerate_bisphosphoglycerate mutase YhfR | YhfR [Bacillus licheniformis DSM 13] | UniRef100_Q65LP2 | Bacillus licheniformis DSM 13 |
| 1237 | BLP01185 hypothetical protein | | | |
| 1238 | hemAT haem-based aerotactic transducer | HemAT [Bacillus licheniformis DSM 13] | UniRef100_Q65LP1 | Bacillus licheniformis DSM 13 |
| 1239 | yhfW putative oxidoreductase YhfW | YhfW [Bacillus licheniformis DSM 13] | UniRef100_Q65LP0 | Bacillus licheniformis DSM 13 |
| 1240 | yhxC Short-chain dehydrogenase_reductase SDR YhxC | YhxC [Bacillus licheniformis DSM 13] | UniRef100_Q65LN9 | Bacillus licheniformis DSM 13 |
| 1241 | yhzC conserved hypothetical protein YhzC | YhzC [Bacillus licheniformis DSM 13] | UniRef100_Q65LN8 | Bacillus licheniformis DSM 13 |
| 1242 | comK competence transcription factor ComK | ComK [Bacillus licheniformis DSM 13] | UniRef100_Q65LN7 | Bacillus licheniformis DSM 13 |
| 1243 | yhjD conserved hypothetical protein YhjD | YhjD [Bacillus licheniformis DSM 13] | UniRef100_Q65LN6 | Bacillus licheniformis DSM 13 |
| 1244 | BLP04714 hypothetical protein | | | |
| 1245 | yhjE conserved hypothetical protein YhjE | YhjE [Bacillus licheniformis DSM 13] | UniRef100_Q65LN5 | Bacillus licheniformis DSM 13 |
| 1246 | sipV type I signal peptidase | SipV [Bacillus licheniformis DSM 13] | UniRef100_Q65LN4 | Bacillus licheniformis DSM 13 |
| 1247 | epr extracellular serine protease | Epr [Bacillus licheniformis DSM 13] | UniRef100_Q65LN3 | Bacillus licheniformis DSM 13 |
| 1248 | BLP01195 Major facilitator superfamily , putative transporter | Hypothetical protein [Bacillus licheniformis DSM 13] | UniRef100_Q65LN2 | Bacillus licheniformis DSM 13 |
| 1249 | BLP01196 transcriptional regulator | Hypothetical protein [Bacillus licheniformis DSM 13] | UniRef100_Q65LN1 | Bacillus licheniformis DSM 13 |
| 1250 | BLP01197 Putative hydantoin utilization protein | Hypothetical protein [Bacillus licheniformis DSM 13] | UniRef100_Q65LM9 | Bacillus licheniformis DSM 13 |
| 1251 | BLP01198 conserved hypothetical protein | Hypothetical protein [Bacillus licheniformis DSM 13] | UniRef100_Q65LM8 | Bacillus licheniformis DSM 13 |
| 1252 | BLP01199 Ureidoglycolate dehydrogenase | Hypothetical protein [Bacillus licheniformis DSM 13] | UniRef100_Q65LM7 | Bacillus licheniformis DSM 13 |
| 1253 | yjmC putative ureidoglycolate dehydrogenase YjmC | YjmC [Bacillus licheniformis DSM 13] | UniRef100_Q65LM6 | Bacillus licheniformis DSM 13 |

| 1254 | BLP01201 putative oxidoreductase | Hypothetical protein [Bacillus licheniformis DSM 13] | UniRef100_Q65LM5 | Bacillus licheniformis DSM 13 |
|---|---|---|---|---|
| 1255 | ylbE conserved hypothetical protein | Hypothetical protein [Bacillus licheniformis DSM 13] | UniRef100_Q65LM4 | Bacillus licheniformis DSM 13 |
| 1256 | BLP01203 conserved hypothetical protein | Hypothetical protein [Bacillus licheniformis DSM 13] | UniRef100_Q65LM3 | Bacillus licheniformis DSM 13 |
| 1257 | BLP01204 carbamate kinase | Hypothetical protein [Bacillus licheniformis DSM 13] | UniRef100_Q65LM2 | Bacillus licheniformis DSM 13 |
| 1258 | BLP01205 putative transporter,membrane protein | Hypothetical protein [Bacillus licheniformis DSM 13] | UniRef100_Q65LM1 | Bacillus licheniformis DSM 13 |
| 1259 | BLP01206 conserved hypothetical protein | Hypothetical protein [Bacillus licheniformis DSM 13] | UniRef100_Q65LM0 | Bacillus licheniformis DSM 13 |
| 1260 | rapG response regulator aspartate phosphatase | RapG [Bacillus licheniformis DSM 13] | UniRef100_Q65LL9 | Bacillus licheniformis DSM 13 |
| 1261 | phrG regulator of the activity of phosphatase RapG - PhrG | Regulator of the activity of phosphatase RapG [Bacillus licheniformis DSM 13] | UniRef100_Q62X10 | Bacillus licheniformis DSM 13 |
| 1262 | BLP01209 hypothetical protein | | | |
| 1263 | BLP04715 hypothetical protein | | | |
| 1264 | BLP04716 conserved hypothetical protein | Hypothetical protein [Bacillus licheniformis DSM 13] | UniRef100_Q65LL8 | Bacillus licheniformis DSM 13 |
| 1265 | BLP01210 hypothetical protein | | | |
| 1266 | BLP01211 hypothetical protein | | | |
| 1267 | msmR transcriptional regulator MsmR | MsmR [Bacillus licheniformis DSM 13] | UniRef100_Q65LL7 | Bacillus licheniformis DSM 13 |
| 1268 | msmE multiple sugar-binding protein MsmE | MsmE [Bacillus licheniformis DSM 13] | UniRef100_Q65LL6 | Bacillus licheniformis DSM 13 |
| 1269 | amyD sugar transporter AmyD | Hypothetical protein amyD [Bacillus licheniformis DSM 13] | UniRef100_Q65LL5 | Bacillus licheniformis DSM 13 |
| 1270 | amyC maltose transport protein AmyC | AmyC [Bacillus licheniformis DSM 13] | UniRef100_Q65LL4 | Bacillus licheniformis DSM 13 |
| 1271 | melA alpha-D-galactoside galactohydrolase, Glycoside Hydrolase Family 4,MelA | MelA [Bacillus licheniformis DSM 13] | UniRef100_Q65LL3 | Bacillus licheniformis DSM 13 |
| 1272 | yhjN conserved hypothetical protein | YhjN [Bacillus licheniformis DSM 13] | UniRef100_Q65LL2 | Bacillus licheniformis DSM 13 |
| 1273 | cotJA CotJA | CotJA [Bacillus licheniformis DSM 13] | UniRef100_Q65LL1 | Bacillus licheniformis DSM 13 |
| 1274 | cotJB CotJB | Hypothetical protein cotJB [Bacillus licheniformis DSM 13] | UniRef100_Q65LL0 | Bacillus licheniformis DSM 13 |
| 1275 | cotJC CotJC | Hypothetical protein cotJC [Bacillus licheniformis DSM 13] | UniRef100_Q65LK9 | Bacillus licheniformis DSM 13 |
| 1276 | ynglA long-chain fatty-acid-CoA ligase | Hypothetical protein [Bacillus licheniformis DSM 13] | UniRef100_Q65LK8 | Bacillus licheniformis DSM 13 |
| 1277 | yhjO conserved hypothetical protein YhjO | Hypothetical protein yhjO [Bacillus subtilis] | UniRef100_O07569 | Bacillus subtilis |
| 1278 | yhjP YhjP | Hypothetical protein yhjP [Bacillus subtilis] | UniRef100_O07570 | Bacillus subtilis |
| 1279 | BLP01224 conserved hypothetical protein | Hypothetical protein [Bacillus licheniformis DSM 13] | UniRef100_Q65LK6 | Bacillus licheniformis DSM 13 |
| 1280 | BLP01225 two-component sensor histidine kinase | Hypothetical protein [Bacillus licheniformis DSM 13] | UniRef100_Q65LK5 | Bacillus licheniformis DSM 13 |
| 1281 | BLP01226 Two-component response regulator | Hypothetical protein [Bacillus licheniformis DSM 13] | UniRef100_Q65LK4 | Bacillus licheniformis DSM 13 |
| 1282 | yhjR conserved hypothetical protein YhjR | Ferritin/ribonucleotide reductase-like [Bacillus licheniformis DSM 13] | UniRef100_Q62WZ4 | Bacillus licheniformis DSM 13 |
| 1283 | yvgL molybdate transport system substrate-binding protein | YvgL [Bacillus licheniformis DSM 13] | UniRef100_Q65LK2 | Bacillus licheniformis DSM 13 |
| 1284 | yvgM molybdate transport system permease protein | YvgM [Bacillus licheniformis DSM 13] | UniRef100_Q65LK1 | Bacillus licheniformis DSM 13 |
| 1285 | addB ATP-dependent deoxyribonuclease (subunit B) | AddB [Bacillus licheniformis DSM 13] | UniRef100_Q65LK0 | Bacillus licheniformis DSM 13 |
| 1286 | addA ATP-dependent deoxyribonuclease (subunit A) | AddA [Bacillus licheniformis DSM 13] | UniRef100_Q65LJ9 | Bacillus licheniformis DSM 13 |

| 1287 | sbcD exonuclease SbcD | Exonuclease SbcD homolog [Bacillus licheniformis DSM 13] | UniRef100_Q65LJ8 | Bacillus licheniformis DSM 13 |
|------|-----------------------|--------------------------------------------------------|------------------|-------------------------------|
| 1288 | sbcC ABC transporter, exonuclease SbcC | YirY (RNA-binding region RNP-1 (RNA recognition motif),ABC transporter) [Bacillus licheniformis DSM 13] | UniRef100_Q65LJ7 | Bacillus licheniformis DSM 13 |
| 1289 | gerPF spore germination protein | GerPF [Bacillus licheniformis DSM 13] | UniRef100_Q65LJ6 | Bacillus licheniformis DSM 13 |
| 1290 | gerPE spore germination protein | GerPE [Bacillus licheniformis DSM 13] | UniRef100_Q65LJ5 | Bacillus licheniformis DSM 13 |
| 1291 | gerPD sporulation protein GerPD | GerPD [Bacillus licheniformis DSM 13] | UniRef100_Q65LJ4 | Bacillus licheniformis DSM 13 |
| 1292 | BLP01236 hypothetical protein | | | |
| 1293 | gerPC spore germination protein | GerPC [Bacillus licheniformis DSM 13] | UniRef100_Q65LJ3 | Bacillus licheniformis DSM 13 |
| 1294 | gerPB spore germination protein | GerPB [Bacillus licheniformis DSM 13] | UniRef100_Q65LJ2 | Bacillus licheniformis DSM 13 |
| 1295 | gerPA spore germination protein | GerPA [Bacillus licheniformis DSM 13] | UniRef100_Q65LJ1 | Bacillus licheniformis DSM 13 |
| 1296 | yitR YitR | Hypothetical protein yitR [Bacillus licheniformis DSM 13] | UniRef100_Q65LJ0 | Bacillus licheniformis DSM 13 |
| 1297 | yisI YisI | Hypothetical protein yisI [Bacillus licheniformis DSM 13] | UniRef100_Q62WY0 | Bacillus licheniformis DSM 13 |
| 1298 | cotH spore coat protein (inner) | CotH [Bacillus licheniformis DSM 13] | UniRef100_Q65LI8 | Bacillus licheniformis DSM 13 |
| 1299 | BLP01243 hypothetical protein | Hypothetical protein [Bacillus licheniformis DSM 13] | UniRef100_Q65LI7 | Bacillus licheniformis DSM 13 |
| 1300 | yisK Fumarylacetoacetate (FAA) hydrolase YisK | YisK (Fumarylacetoacetate (FAA) hydrolase) [Bacillus licheniformis DSM 13] | UniRef100_Q65LI6 | Bacillus licheniformis DSM 13 |
| 1301 | engCA GTP-binding protein | Hypothetical protein engCA [Bacillus licheniformis DSM 13] | UniRef100_Q65LI5 | Bacillus licheniformis DSM 13 |
| 1302 | BLP01246 hypothetical protein | | | |
| 1303 | yisL conserved membrane protein | YisL [Bacillus licheniformis DSM 13] | UniRef100_Q65LI4 | Bacillus licheniformis DSM 13 |
| 1304 | yisN YisN | Hypothetical protein yisN [Bacillus licheniformis DSM 13] | UniRef100_Q65LI3 | Bacillus licheniformis DSM 13 |
| 1305 | asnO asparagine synthetase | AsnO [Bacillus licheniformis DSM 13] | UniRef100_Q65LI2 | Bacillus licheniformis DSM 13 |
| 1306 | BLP01250 ammonium transporter | Hypothetical protein [Bacillus licheniformis DSM 13] | UniRef100_Q65LI1 | Bacillus licheniformis DSM 13 |
| 1307 | nrgBA nitrogen-regulated PII-like protein | Hypothetical protein [Bacillus licheniformis DSM 13] | UniRef100_Q65LI0 | Bacillus licheniformis DSM 13 |
| 1308 | yisQ probable transporter YisQ | YisQ [Bacillus licheniformis DSM 13] | UniRef100_Q65LH9 | Bacillus licheniformis DSM 13 |
| 1309 | yisR probable transcriptional regulator | YisR [Bacillus licheniformis DSM 13] | UniRef100_Q65LH8 | Bacillus licheniformis DSM 13 |
| 1310 | BLP01254 N-acetyltransferase | YokL [Bacillus licheniformis DSM 13] | UniRef100_Q65LH7 | Bacillus licheniformis DSM 13 |
| 1311 | degA transcriptional regulator | DegA [Bacillus licheniformis DSM 13] | UniRef100_Q65LH6 | Bacillus licheniformis DSM 13 |
| 1312 | yisS putative oxidoreductase | YisS [Bacillus licheniformis DSM 13] | UniRef100_Q65LH5 | Bacillus licheniformis DSM 13 |
| 1313 | BLP01257 hypothetical protein | Spore germination protein GerHC [Bacillus anthracis] | UniRef100_Q81KL1 | Bacillus anthracis |
| 1314 | BLP01258 hypothetical protein | Spore germination protein GerHB [Bacillus anthracis] | UniRef100_Q81KL2 | Bacillus anthracis |
| 1315 | BLP01259 hypothetical protein | | | |
| 1316 | BLP01260 hypothetical protein | | | |
| 1317 | BLP01261 hypothetical protein | Spore germination protein [Oceanobacillus iheyensis] | UniRef100_Q8CUL1 | Oceanobacillus iheyensis |
| 1318 | BLP01262 hypothetical protein | YisU [Bacillus subtilis] | UniRef100_O06730 | Bacillus subtilis |
| 1319 | yisW putative transcriptional regulator | YisV protein [Bacillus subtilis] | UniRef100_Q796Q6 | Bacillus subtilis |
| 1320 | BLP01265 hypothetical protein | | | |

| 1321 | BLP01266 4-aminobutyrate aminotransferase | Hypothetical protein [Bacillus licheniformis DSM 13] | UniRef100_Q65LH3 | Bacillus licheniformis DSM 13 |
|---|---|---|---|---|
| 1322 | BLP01267 putative L-2,4-diaminobutyrate decarboxylase | Hypothetical protein [Bacillus licheniformis DSM 13] | UniRef100_Q65LH2 | Bacillus licheniformis DSM 13 |
| 1323 | BLP01268 putative Siderophore biosynthesis protein | Hypothetical protein [Bacillus licheniformis DSM 13] | UniRef100_Q65LH1 | Bacillus licheniformis DSM 13 |
| 1324 | BLP01269 Siderophore biosynthesis protein | Hypothetical protein [Bacillus licheniformis DSM 13] | UniRef100_Q65LH0 | Bacillus licheniformis DSM 13 |
| 1325 | BLP01270 Siderophore biosynthesis protein | Hypothetical protein [Bacillus licheniformis DSM 13] | UniRef100_Q65LG9 | Bacillus licheniformis DSM 13 |
| 1326 | BLP01271 Siderophore biosynthesis protein | Hypothetical protein [Bacillus licheniformis DSM 13] | UniRef100_Q65LG8 | Bacillus licheniformis DSM 13 |
| 1327 | yitI probable acetyltransferase | YitI [Bacillus licheniformis DSM 13] | UniRef100_Q65LG7 | Bacillus licheniformis DSM 13 |
| 1328 | BLP01273 Short-chain dehydrogenase_reductase SDR | Hypothetical protein [Bacillus licheniformis DSM 13] | UniRef100_Q65LG6 | Bacillus licheniformis DSM 13 |
| 1329 | BLP01274 hypothetical DNA-binding protein | Hypothetical protein [Bacillus licheniformis DSM 13] | UniRef100_Q65LG5 | Bacillus licheniformis DSM 13 |
| 1330 | metH putative 5-methyltetrahydrofolate--homocysteine methyltransferase MetH | Hypothetical protein (EC 2.1.1.13) (Cobalamin-dependent methionine synthase, B12-binding-like,Cobalamin (B12)-binding) [Bacillus licheniformis DSM 13] | UniRef100_Q65LG4 | Bacillus licheniformis DSM 13 |
| 1331 | yitJ Homocysteine S-methyltransferase | YitJ [Bacillus licheniformis DSM 13] | UniRef100_Q65LG3 | Bacillus licheniformis DSM 13 |
| 1332 | yitK conserved protein YitK | Hypothetical protein yitK [Bacillus licheniformis DSM 13] | UniRef100_Q65LG2 | Bacillus licheniformis DSM 13 |
| 1333 | yitL Nucleic acid-binding protein YitL | Nucleic acid-binding OB-fold [Bacillus licheniformis DSM 13] | UniRef100_Q62WV2 | Bacillus licheniformis DSM 13 |
| 1334 | BLP01279 hypothetical protein | | | |
| 1335 | BLP01280 hypothetical protein | Hypothetical protein [Bacillus licheniformis DSM 13] | UniRef100_Q65LG0 | Bacillus licheniformis DSM 13 |
| 1336 | yitS conserved protein YitS | YitS [Bacillus licheniformis DSM 13] | UniRef100_Q65LF9 | Bacillus licheniformis DSM 13 |
| 1337 | yitT conserved protein YitT | Hypothetical protein yitT [Bacillus licheniformis DSM 13] | UniRef100_Q65LF8 | Bacillus licheniformis DSM 13 |
| 1338 | ipi intracellular proteinase inhibitor Ipi | Ipi [Bacillus licheniformis DSM 13] | UniRef100_Q65LF7 | Bacillus licheniformis DSM 13 |
| 1339 | guaC GMP reductase | GuaC [Bacillus licheniformis DSM 13] | UniRef100_Q65LF6 | Bacillus licheniformis DSM 13 |
| 1340 | BLP01285 hypothetical protein | Hypothetical protein [Bacillus licheniformis DSM 13] | UniRef100_Q65LF5 | Bacillus licheniformis DSM 13 |
| 1341 | BLP01286 hypothetical protein | Hypothetical protein [Bacillus licheniformis DSM 13] | UniRef100_Q62WU4 | Bacillus licheniformis DSM 13 |
| 1342 | yitU putative hydrolase YitU | YitU [Bacillus licheniformis DSM 13] | UniRef100_Q65LF3 | Bacillus licheniformis DSM 13 |
| 1343 | yitV conserved protein,putative esterase YitV | YitV [Bacillus licheniformis DSM 13] | UniRef100_Q65LF2 | Bacillus licheniformis DSM 13 |
| 1344 | yitW N-6 Adenine-specific DNA methylase YitW | YitW [Bacillus licheniformis DSM 13] | UniRef100_Q65LF1 | Bacillus licheniformis DSM 13 |
| 1345 | BLP01290 hypothetical protein | | | |
| 1346 | argC N-acetylglutamate gamma-semialdehyde dehydrogenase | ArgC [Bacillus licheniformis DSM 13] | UniRef100_Q65LF0 | Bacillus licheniformis DSM 13 |
| 1347 | argJ ornithine acetyltransferase and amino-acid acetyltransferase | ArgJ [Bacillus licheniformis DSM 13] | UniRef100_Q65LE9 | Bacillus licheniformis DSM 13 |
| 1348 | argB N-acetylglutamate 5-phosphotransferase | ArgB [Bacillus licheniformis DSM 13] | UniRef100_Q65LE8 | Bacillus licheniformis DSM 13 |
| 1349 | argD N-acetylornithine aminotransferase | ArgD [Bacillus licheniformis DSM 13] | UniRef100_Q65LE7 | Bacillus licheniformis DSM 13 |
| 1350 | carA carbamoyl-phosphate transferase-arginine (subunit A) | CarA [Bacillus licheniformis DSM 13] | UniRef100_Q65LE6 | Bacillus licheniformis DSM 13 |
| 1351 | carB carbamoyl-phosphate transferase-arginine (subunit B) | CarB [Bacillus licheniformis DSM 13] | UniRef100_Q65LE5 | Bacillus licheniformis DSM 13 |
| 1352 | argF ornithine carbamoyltransferase | ArgF [Bacillus licheniformis DSM 13] | UniRef100_Q65LE4 | Bacillus licheniformis DSM 13 |

| 1353 | upkA probable undecaprenol kinase UpkA | Hypothetical protein [Bacillus licheniformis DSM 13] | UniRef100_Q65LE3 | Bacillus licheniformis DSM 13 |
|---|---|---|---|---|
| 1354 | yjzC conserved hypothetical protein YjzC | YjzC [Bacillus licheniformis DSM 13] | UniRef100_Q65LE2 | Bacillus licheniformis DSM 13 |
| 1355 | BLP01300 hypothetical protein | | | |
| 1356 | yjaU conserved hypothetical protein YjaU | YjaU [Bacillus licheniformis DSM 13] | UniRef100_Q65LE0 | Bacillus licheniformis DSM 13 |
| 1357 | yjaV conserved hypothetical protein YjaV | YjaV [Bacillus licheniformis DSM 13] | UniRef100_Q65LD9 | Bacillus licheniformis DSM 13 |
| 1358 | med transcriptional activator protein Med | Hypothetical protein med [Bacillus licheniformis DSM 13] | UniRef100_Q65LD8 | Bacillus licheniformis DSM 13 |
| 1359 | comZ ComZ | Hypothetical protein comZ [Bacillus licheniformis DSM 13] | UniRef100_Q62WS7 | Bacillus licheniformis DSM 13 |
| 1360 | yjzB conserved hypothetical protein yjzB | YjzB [Bacillus licheniformis DSM 13] | UniRef100_Q65LD6 | Bacillus licheniformis DSM 13 |
| 1361 | fabHA beta-ketoacyl-acyl carrier protein synthase III | FabHA [Bacillus licheniformis DSM 13] | UniRef100_Q65LD5 | Bacillus licheniformis DSM 13 |
| 1362 | fabF beta-ketoacyl-acyl carrier protein synthase II | FabF [Bacillus licheniformis DSM 13] | UniRef100_Q65LD4 | Bacillus licheniformis DSM 13 |
| 1363 | yjaZ conserved hypothetical protein YjaZ | YjaZ [Bacillus licheniformis DSM 13] | UniRef100_Q65LD3 | Bacillus licheniformis DSM 13 |
| 1364 | appD oligopeptide ABC transporter (ATP-binding protein) | AppD [Bacillus licheniformis DSM 13] | UniRef100_Q65LD2 | Bacillus licheniformis DSM 13 |
| 1365 | appF oligopeptide ABC transporter (ATP-binding protein) | AppF [Bacillus licheniformis DSM 13] | UniRef100_Q65LD1 | Bacillus licheniformis DSM 13 |
| 1366 | appA oligopeptide ABC transporter (oligopeptide-binding protein) | AppA [Bacillus licheniformis DSM 13] | UniRef100_Q65LD0 | Bacillus licheniformis DSM 13 |
| 1367 | appB oligopeptide ABC transporter (permease) | AppB [Bacillus licheniformis DSM 13] | UniRef100_Q65LC9 | Bacillus licheniformis DSM 13 |
| 1368 | appC oligopeptide ABC transporter (permease) | AppC [Bacillus licheniformis DSM 13] | UniRef100_Q65LC8 | Bacillus licheniformis DSM 13 |
| 1369 | BLP01314 Major facilitator superfamily | Hypothetical protein [Bacillus licheniformis DSM 13] | UniRef100_Q65LC7 | Bacillus licheniformis DSM 13 |
| 1370 | BLP01315 hypothetical protein | | | |
| 1371 | yjbA conserved protein YjbA | YjbA [Bacillus licheniformis DSM 13] | UniRef100_Q65LC6 | Bacillus licheniformis DSM 13 |
| 1372 | trpS tryptophanyl-tRNA synthetase | TrpS [Bacillus licheniformis DSM 13] | UniRef100_Q65LC5 | Bacillus licheniformis DSM 13 |
| 1373 | BLP01318 hypothetical protein | | | |
| 1374 | BLP01319 hypothetical protein | | | |
| 1375 | oppA oligopeptide ABC transporter (binding protein) | OppA [Bacillus licheniformis DSM 13] | UniRef100_Q65LC4 | Bacillus licheniformis DSM 13 |
| 1376 | oppB oligopeptide ABC transporter (permease) | OppB [Bacillus licheniformis DSM 13] | UniRef100_Q65LC3 | Bacillus licheniformis DSM 13 |
| 1377 | oppC oligopeptide ABC transporter (permease) | OppC [Bacillus licheniformis DSM 13] | UniRef100_Q65LC2 | Bacillus licheniformis DSM 13 |
| 1378 | oppD oligopeptide ABC transporter (ATP-binding protein) | OppD [Bacillus licheniformis DSM 13] | UniRef100_Q65LC1 | Bacillus licheniformis DSM 13 |
| 1379 | oppF oligopeptide ABC transporter (ATP-binding protein) | OppF [Bacillus licheniformis DSM 13] | UniRef100_Q65LC0 | Bacillus licheniformis DSM 13 |
| 1380 | yjbC GCN5-related N-acetyltransferase | YjbC [Bacillus licheniformis DSM 13] | UniRef100_Q65LB9 | Bacillus licheniformis DSM 13 |
| 1381 | spx glutaredoxin family protein Spx | YjbD [Bacillus licheniformis DSM 13] | UniRef100_Q65LB8 | Bacillus licheniformis DSM 13 |
| 1382 | yjbE conserved membrane protein YjbE | Integral membrane protein TerC family [Bacillus licheniformis DSM 13] | UniRef100_Q62WQ7 | Bacillus licheniformis DSM 13 |
| 1383 | mecA Adaptor protein MecA | Hypothetical protein mecA [Bacillus licheniformis DSM 13] | UniRef100_Q65LB7 | Bacillus licheniformis DSM 13 |
| 1384 | BLP01329 conserved hypothetical protein | Hypothetical protein [Bacillus licheniformis DSM 13] | UniRef100_Q65LB6 | Bacillus licheniformis DSM 13 |
| 1385 | BLP01330 conserved hypothetical protein | Hypothetical protein [Bacillus licheniformis DSM 13] | UniRef100_Q65LB5 | Bacillus licheniformis DSM 13 |

| 1386 | BLP01331 conserved hypothetical protein | Hypothetical protein [Bacillus licheniformis DSM 13] | UniRef100_Q65LB4 | Bacillus licheniformis DSM 13 |
|---|---|---|---|---|
| 1387 | BLP01332 two-component response regulator | Hypothetical protein [Bacillus licheniformis DSM 13] | UniRef100_Q65LB3 | Bacillus licheniformis DSM 13 |
| 1388 | BLP01333 two-component sensor kinase | Hypothetical protein [Bacillus licheniformis DSM 13] | UniRef100_Q65LB2 | Bacillus licheniformis DSM 13 |
| 1389 | yjbF conserved protein YjbF | YjbF [Bacillus licheniformis DSM 13] | UniRef100_Q65LB1 | Bacillus licheniformis DSM 13 |
| 1390 | yjbG oligoendopeptidase F,Pz peptidase | YjbG [Bacillus licheniformis DSM 13] | UniRef100_Q65LB0 | Bacillus licheniformis DSM 13 |
| 1391 | BLP01336 hypothetical protein | Hypothetical protein [Bacillus licheniformis DSM 13] | UniRef100_Q65LA9 | Bacillus licheniformis DSM 13 |
| 1392 | yjbH conserved protein YjbH | Hypothetical protein yjbH [Bacillus licheniformis DSM 13] | UniRef100_Q65LA8 | Bacillus licheniformis DSM 13 |
| 1393 | yjbI truncated form of bacterial hemoglobin  YjbI | YjbI [Bacillus licheniformis DSM 13] | UniRef100_Q65LA7 | Bacillus licheniformis DSM 13 |
| 1394 | BLP01339 hypothetical protein | YjbJ [Bacillus licheniformis DSM 13] | UniRef100_Q65LA6 | Bacillus licheniformis DSM 13 |
| 1395 | yjbK conserved protein YjbK | YjbK [Bacillus licheniformis DSM 13] | UniRef100_Q65LA5 | Bacillus licheniformis DSM 13 |
| 1396 | yjbL conserved protein YjbL | Hypothetical protein yjbL [Bacillus licheniformis DSM 13] | UniRef100_Q65LA4 | Bacillus licheniformis DSM 13 |
| 1397 | yjbM conserved protein YjbM | YjbM [Bacillus licheniformis DSM 13] | UniRef100_Q65LA3 | Bacillus licheniformis DSM 13 |
| 1398 | ppnK1 probable inorganic polyphosphate_ATP-NAD kinase | YjbN [Bacillus licheniformis DSM 13] | UniRef100_Q65LA2 | Bacillus licheniformis DSM 13 |
| 1399 | yjbO Pseudouridine synthase YjbO | Pseudouridine synthase, RluD [Bacillus licheniformis DSM 13] | UniRef100_Q62WP0 | Bacillus licheniformis DSM 13 |
| 1400 | yjbP Ser_Thr protein phosphatase YjbP | YjbP [Bacillus licheniformis DSM 13] | UniRef100_Q65LA0 | Bacillus licheniformis DSM 13 |
| 1401 | BLP04717 hypothetical protein | | | |
| 1402 | yjbQ cation transporter YjbQ | YjbQ [Bacillus licheniformis DSM 13] | UniRef100_Q65L99 | Bacillus licheniformis DSM 13 |
| 1403 | tenA transcriptional regulator | TenA [Bacillus licheniformis DSM 13] | UniRef100_Q65L98 | Bacillus licheniformis DSM 13 |
| 1404 | tenI transcriptional regulator | TenI [Bacillus licheniformis DSM 13] | UniRef100_Q65L97 | Bacillus licheniformis DSM 13 |
| 1405 | goxB glycine oxidase | GoxB [Bacillus licheniformis DSM 13] | UniRef100_Q65L96 | Bacillus licheniformis DSM 13 |
| 1406 | thiS ThiS | Hypothetical protein thiS [Bacillus licheniformis DSM 13] | UniRef100_Q65L95 | Bacillus licheniformis DSM 13 |
| 1407 | thiG ThiG | Hypothetical protein thiG [Bacillus licheniformis DSM 13] | UniRef100_Q65L94 | Bacillus licheniformis DSM 13 |
| 1408 | thiF ThiF | Hypothetical protein thiF [Bacillus licheniformis DSM 13] | UniRef100_Q65L93 | Bacillus licheniformis DSM 13 |
| 1409 | thiDA Phosphomethylpyrimidine kinase | YjbV [Bacillus licheniformis DSM 13] | UniRef100_Q65L92 | Bacillus licheniformis DSM 13 |
| 1410 | fabI enoyl-acyl carrier protein reductase | FabI [Bacillus licheniformis DSM 13] | UniRef100_Q65L91 | Bacillus licheniformis DSM 13 |
| 1411 | BLP01355 hypothetical protein | | | |
| 1412 | cotO CotO | Hypothetical protein yjbX [Bacillus licheniformis DSM 13] | UniRef100_Q65L90 | Bacillus licheniformis DSM 13 |
| 1413 | cotZ spore coat protein (insoluble fraction) | CotZ [Bacillus licheniformis DSM 13] | UniRef100_Q65L89 | Bacillus licheniformis DSM 13 |
| 1414 | cotY spore coat protein (insoluble fraction) | CotY [Bacillus licheniformis DSM 13] | UniRef100_Q65L88 | Bacillus licheniformis DSM 13 |
| 1415 | cotX spore coat protein (insoluble fraction) | Spore coat protein [Bacillus licheniformis DSM 13] | UniRef100_Q62WM6 | Bacillus licheniformis DSM 13 |
| 1416 | cotW spore coat protein (insoluble fraction) | Hypothetical protein [Bacillus licheniformis DSM 13] | UniRef100_Q65L86 | Bacillus licheniformis DSM 13 |
| 1417 | cotV spore coat protein (insoluble fraction) | Spore coat protein [Bacillus licheniformis DSM 13] | UniRef100_Q62WM4 | Bacillus licheniformis DSM 13 |
| 1418 | yjcA YjcA | Hypothetical protein yjcA [Bacillus licheniformis DSM 13] | UniRef100_Q62WM3 | Bacillus licheniformis DSM 13 |
| 1419 | BLP04718 hypothetical protein | Hypothetical protein [Bacillus licheniformis DSM 13] | UniRef100_Q65L84 | Bacillus licheniformis DSM 13 |
| 1420 | BLP01364 hypothetical protein | Hypothetical protein [Bacillus licheniformis DSM 13] | UniRef100_Q62WM2 | Bacillus licheniformis DSM 13 |

| 1421 | BLP01365 hypothetical protein | | | |
|---|---|---|---|---|
| 1422 | yjcC YjcC | Hypothetical protein yjcC [Bacillus licheniformis DSM 13] | UniRef100_Q65L82 | Bacillus licheniformis DSM 13 |
| 1423 | yjcD putative ATP-dependent DNA helicase YjcD | YjcD [Bacillus licheniformis DSM 13] | UniRef100_Q65L81 | Bacillus licheniformis DSM 13 |
| 1424 | BLP01368 hypothetical protein | | | |
| 1425 | yngC conserved membrane protein YngC | YngC [Bacillus licheniformis DSM 13] | UniRef100_Q65L79 | Bacillus licheniformis DSM 13 |
| 1426 | BLP01370 putative UDP-glucose 4-epimerase | Hypothetical protein [Bacillus licheniformis DSM 13] | UniRef100_Q65L78 | Bacillus licheniformis DSM 13 |
| 1427 | yngB UTP--glucose-1-phosphate uridylyltransferase YngB | YngB [Bacillus licheniformis DSM 13] | UniRef100_Q65L77 | Bacillus licheniformis DSM 13 |
| 1428 | yngA conserved membrane protein YngA | YngA [Bacillus licheniformis DSM 13] | UniRef100_Q65L76 | Bacillus licheniformis DSM 13 |
| 1429 | yjcF probable acetyltransferase YjcF | YjcF [Bacillus licheniformis DSM 13] | UniRef100_Q65L75 | Bacillus licheniformis DSM 13 |
| 1430 | yjcG conserved protein YjcG | YjcG [Bacillus licheniformis DSM 13] | UniRef100_Q65L74 | Bacillus licheniformis DSM 13 |
| 1431 | yjcH conserved protein,putative carbohydrate esterase family 1, YjcH | YjcH [Bacillus licheniformis DSM 13] | UniRef100_Q65L73 | Bacillus licheniformis DSM 13 |
| 1432 | BLP04816 conserved hypothetical protein | Hypothetical protein [Bacillus licheniformis DSM 13] | UniRef100_Q65L72 | Bacillus licheniformis DSM 13 |
| 1433 | BLP01376 hypothetical protein | | | |
| 1434 | BLP01377 conserved hypothetical protein | Hypothetical protein [Bacillus licheniformis DSM 13] | UniRef100_Q65L71 | Bacillus licheniformis DSM 13 |
| 1435 | BLP01378 hypothetical DNA-binding protein | Hypothetical protein [Bacillus licheniformis DSM 13] | UniRef100_Q65L70 | Bacillus licheniformis DSM 13 |
| 1436 | yjcI Cys_Met metabolism pyridoxal-phosphate-dependent enzymes | YjcI [Bacillus licheniformis DSM 13] | UniRef100_Q65L69 | Bacillus licheniformis DSM 13 |
| 1437 | yjcJ putative Cystathionine beta-lyase | YjcJ [Bacillus licheniformis DSM 13] | UniRef100_Q65L68 | Bacillus licheniformis DSM 13 |
| 1438 | yjcL conserved membrane protein YjcL | Hypothetical protein yjcL [Bacillus licheniformis DSM 13] | UniRef100_Q65L67 | Bacillus licheniformis DSM 13 |
| 1439 | BLP01382 hypothetical DNA-binding protein,putative transcriptional regulator | Hypothetical protein [Bacillus licheniformis DSM 13] | UniRef100_Q65L66 | Bacillus licheniformis DSM 13 |
| 1440 | BLP01383 conserved hypothetical protein | Hypothetical protein [Bacillus licheniformis DSM 13] | UniRef100_Q65L65 | Bacillus licheniformis DSM 13 |
| 1441 | pbpE penicillin-binding protein 4 | PbpE [Bacillus licheniformis DSM 13] | UniRef100_Q65L64 | Bacillus licheniformis DSM 13 |
| 1442 | BLP04719 hypothetical protein | | | |
| 1443 | abnA Glycoside Hydrolase Family 43 | AbnA [Bacillus licheniformis DSM 13] | UniRef100_Q65L63 | Bacillus licheniformis DSM 13 |
| 1444 | BLP01386 hypothetical protein | Hypothetical protein [Bacillus licheniformis DSM 13] | UniRef100_Q65L62 | Bacillus licheniformis DSM 13 |
| 1445 | maa maltose O-acetyltransferase | Maa [Bacillus licheniformis DSM 13] | UniRef100_Q65L61 | Bacillus licheniformis DSM 13 |
| 1446 | BLP01388 hypothetical protein | Hypothetical protein [Bacillus licheniformis DSM 13] | UniRef100_Q65L60 | Bacillus licheniformis DSM 13 |
| 1447 | cisLA transcriptional activator of the cysJI operon | YwfK [Bacillus licheniformis DSM 13] | UniRef100_Q65L59 | Bacillus licheniformis DSM 13 |
| 1448 | BLP01390 hypothetical protein | | | |
| 1449 | cysI sulfite reductase (NADPH) flavoprotein alpha-component CysI | YvgR [Bacillus licheniformis DSM 13] | UniRef100_Q65L58 | Bacillus licheniformis DSM 13 |
| 1450 | cysJ sulfite reductase (NADPH) hemoprotein beta-component CysJ | YvgQ [Bacillus licheniformis DSM 13] | UniRef100_Q65L57 | Bacillus licheniformis DSM 13 |
| 1451 | BLP01393 hypothetical protein | Putative HTH-type transcriptional regulator yoaU [Bacillus subtilis] | UniRef100_O34701 | Bacillus subtilis |
| 1452 | yoaV conserved membrane protein YoaV | Hypothetical protein yoaV [Bacillus licheniformis DSM 13] | UniRef100_Q65L54 | Bacillus licheniformis DSM 13 |
| 1453 | yyaH putative Glyoxalase family protein | YyaH [Bacillus licheniformis DSM 13] | UniRef100_Q65L53 | Bacillus licheniformis DSM 13 |

| 1454 | yoeB conserved hypothetical protein YoeB | YoeB [Bacillus licheniformis DSM 13] | UniRef100_Q65L52 | Bacillus licheniformis DSM 13 |
|------|------|------|------|------|
| 1455 | BLP04720 hypothetical protein | | | |
| 1456 | yocH Peptidoglycan-binding protein | Hypothetical protein [Bacillus licheniformis DSM 13] | UniRef100_Q65L51 | Bacillus licheniformis DSM 13 |
| 1457 | BLP01398 putative transporter, drug-export protein | Hypothetical protein [Bacillus licheniformis DSM 13] | UniRef100_Q65L50 | Bacillus licheniformis DSM 13 |
| 1458 | yxaF putative transscriptional regulator HTH type YxaF | YxaF [Bacillus licheniformis DSM 13] | UniRef100_Q65L49 | Bacillus licheniformis DSM 13 |
| 1459 | BLP01400 hypothetical protein | Hypothetical protein [Bacillus licheniformis DSM 13] | UniRef100_Q65L48 | Bacillus licheniformis DSM 13 |
| 1460 | BLP01401 hypothetical protein | Hypothetical protein [Bacillus licheniformis DSM 13] | UniRef100_Q65L47 | Bacillus licheniformis DSM 13 |
| 1461 | BLP01402 putative transposase | YeeF [Bacillus licheniformis DSM 13] | UniRef100_Q65L45 | Bacillus licheniformis DSM 13 |
| 1462 | yjqB hypothetical protein | YjqB [Bacillus licheniformis DSM 13] | UniRef100_Q65L44 | Bacillus licheniformis DSM 13 |
| 1463 | xkdA phage related gene | Hypothetical protein xkDa [Bacillus licheniformis DSM 13] | UniRef100_Q65L43 | Bacillus licheniformis DSM 13 |
| 1464 | xre transcriptional regulator | Xre [Bacillus licheniformis DSM 13] | UniRef100_Q65L42 | Bacillus licheniformis DSM 13 |
| 1465 | BLP04817 conserved hypothetical protein | Hypothetical protein [Bacillus licheniformis DSM 13] | UniRef100_Q65L41 | Bacillus licheniformis DSM 13 |
| 1466 | BLP01406 hypothetical protein | | | |
| 1467 | BLP01407 hypothetical protein | Hypothetical protein [Bacillus licheniformis DSM 13] | UniRef100_Q65L40 | Bacillus licheniformis DSM 13 |
| 1468 | xkdB phage related protein | Hypothetical protein xkdB [Bacillus licheniformis DSM 13] | UniRef100_Q65L39 | Bacillus licheniformis DSM 13 |
| 1469 | xkdC phage related protein | Hypothetical protein xkdC [Bacillus licheniformis DSM 13] | UniRef100_Q65L38 | Bacillus licheniformis DSM 13 |
| 1470 | BLP01410 conserved hypothetical | Hypothetical protein [Bacillus licheniformis DSM 13] | UniRef100_Q65L37 | Bacillus licheniformis DSM 13 |
| 1471 | xkdD phage related protein | Hypothetical protein xkdD [Bacillus licheniformis DSM 13] | UniRef100_Q65L36 | Bacillus licheniformis DSM 13 |
| 1472 | xtrA phage related protein | Hypothetical protein xtrA [Bacillus licheniformis DSM 13] | UniRef100_Q65L35 | Bacillus licheniformis DSM 13 |
| 1473 | xpf putative phage RNA polymerase sigma factor-like protein | Xpf [Bacillus licheniformis DSM 13] | UniRef100_Q65L34 | Bacillus licheniformis DSM 13 |
| 1474 | xtmA putative phage terminase (small subunit) | XtmA [Bacillus licheniformis DSM 13] | UniRef100_Q65L33 | Bacillus licheniformis DSM 13 |
| 1475 | xtmB putative phage terminase (large subunit) | XtmB [Bacillus licheniformis DSM 13] | UniRef100_Q65L32 | Bacillus licheniformis DSM 13 |
| 1476 | xkdE phage related protein | Hypothetical protein xkdE [Bacillus licheniformis DSM 13] | UniRef100_Q65L31 | Bacillus licheniformis DSM 13 |
| 1477 | xkdF phage related protein | Hypothetical protein xkdF [Bacillus licheniformis DSM 13] | UniRef100_Q65L30 | Bacillus licheniformis DSM 13 |
| 1478 | xkdG phage related protein | Hypothetical protein xkdG [Bacillus licheniformis DSM 13] | UniRef100_Q65L29 | Bacillus licheniformis DSM 13 |
| 1479 | yqbG hypothetical phage related protein YqbG | YqbG [Bacillus licheniformis DSM 13] | UniRef100_Q65L28 | Bacillus licheniformis DSM 13 |
| 1480 | xkdH phage related protein XkdH | YqbH [Bacillus licheniformis DSM 13] | UniRef100_Q65L27 | Bacillus licheniformis DSM 13 |
| 1481 | xkdI hypothetical phage related protein | Hypothetical protein xkdI [Bacillus licheniformis DSM 13] | UniRef100_Q65L26 | Bacillus licheniformis DSM 13 |
| 1482 | xkdJ phage related protein | Hypothetical protein xkdJ [Bacillus licheniformis DSM 13] | UniRef100_Q65L25 | Bacillus licheniformis DSM 13 |
| 1483 | BLP01423 conserved hypothetical protein | Hypothetical protein [Bacillus licheniformis DSM 13] | UniRef100_Q65L24 | Bacillus licheniformis DSM 13 |
| 1484 | xkdK hypothetical phage related protein | Hypothetical protein xkdK [Bacillus licheniformis DSM 13] | UniRef100_Q65L23 | Bacillus licheniformis DSM 13 |
| 1485 | xkdM hypothetical phage related protein | Hypothetical protein xkdM [Bacillus licheniformis DSM 13] | UniRef100_Q65L22 | Bacillus licheniformis DSM 13 |
| 1486 | xkdN phage related protein XkdN | XkdN1 [Bacillus licheniformis DSM 13] | UniRef100_Q65L21 | Bacillus licheniformis DSM 13 |
| 1487 | xkdO hypothetical phage related protein | Hypothetical protein xkdO [Bacillus licheniformis DSM 13] | UniRef100_Q65L19 | Bacillus licheniformis DSM 13 |
| 1488 | xkdP hypothetical phage related protein | XkdP [Bacillus licheniformis DSM 13] | UniRef100_Q65L18 | Bacillus licheniformis DSM 13 |

| 1489 | xkdQ hypothetical phage related protein | YqbQ [Bacillus licheniformis DSM 13] | UniRef100_Q65L17 | Bacillus licheniformis DSM 13 |
|---|---|---|---|---|
| 1490 | xkdR hypothetical phage related protein | Hypothetical protein [Bacillus licheniformis DSM 13] | UniRef100_Q65L16 | Bacillus licheniformis DSM 13 |
| 1491 | xkdS hypothetical phage related protein | Hypothetical protein xkdS [Bacillus licheniformis DSM 13] | UniRef100_Q65L15 | Bacillus licheniformis DSM 13 |
| 1492 | xkdT hypothetical phage related protein | YqbT [Bacillus licheniformis DSM 13] | UniRef100_Q65L14 | Bacillus licheniformis DSM 13 |
| 1493 | xkdU hypothetical phage related protein | Hypothetical protein xkdU [Bacillus licheniformis DSM 13] | UniRef100_Q65L13 | Bacillus licheniformis DSM 13 |
| 1494 | BLP01435 hypothetical phage related protein | Hypothetical protein [Bacillus licheniformis DSM 13] | UniRef100_Q65L12 | Bacillus licheniformis DSM 13 |
| 1495 | xkdV hypothetical phage related protein | Hypothetical protein [Bacillus licheniformis DSM 13] | UniRef100_Q65L10 | Bacillus licheniformis DSM 13 |
| 1496 | xkdW phage related protein XkdW | YomQ [Bacillus licheniformis DSM 13] | UniRef100_Q65EU0 | Bacillus licheniformis DSM 13 |
| 1497 | xkdX phage related protein XkdX | YomP [Bacillus licheniformis DSM 13] | UniRef100_Q65KE9 | Bacillus licheniformis DSM 13 |
| 1498 | BLP01440 hypothetical phage related protein | Hypothetical protein xkdV [Bacillus licheniformis DSM 13] | UniRef100_Q65L11 | Bacillus licheniformis DSM 13 |
| 1499 | xhlA phage related protein | XpaL1 homologous protein [Bacillus licheniformis] | UniRef100_P70924 | Bacillus licheniformis |
| 1500 | xpaL2 holin | XpaL2 homologous protein [Bacillus licheniformis] | UniRef100_P70925 | Bacillus licheniformis |
| 1501 | BLP01443 hypothetical protein | ORF [Bacillus licheniformis] | UniRef100_P70926 | Bacillus licheniformis |
| 1502 | cwlL N-acetylmuramoyl-L-alanine amidase | Hypothetical protein [Bacillus licheniformis DSM 13] | UniRef100_Q65L07 | Bacillus licheniformis DSM 13 |
| 1503 | BLP01445 hypothetical DNA-binding protein, putative transcriptional regulator | Hypothetical protein L4 [Bacillus licheniformis] | UniRef100_Q65L06 | Bacillus licheniformis |
| 1504 | yjmD Zinc-containing alcohol dehydrogenase YjmD | YjmD [Bacillus licheniformis DSM 13] | UniRef100_Q65L05 | Bacillus licheniformis DSM 13 |
| 1505 | uxuA D-mannonate hydrolase | UxuA [Bacillus licheniformis DSM 13] | UniRef100_Q65L04 | Bacillus licheniformis DSM 13 |
| 1506 | yjmF Short-chain dehydrogenase_reductase YjmF | YjmF [Bacillus licheniformis DSM 13] | UniRef100_Q65L03 | Bacillus licheniformis DSM 13 |
| 1507 | BLP01449 putative Zn binding dehydrogenase | Hypothetical protein [Bacillus licheniformis DSM 13] | UniRef100_Q65L02 | Bacillus licheniformis DSM 13 |
| 1508 | exuT hexuronate transporter | ExuT [Bacillus licheniformis DSM 13] | UniRef100_Q65L01 | Bacillus licheniformis DSM 13 |
| 1509 | BLP01451 hypothetical protein | | | |
| 1510 | spoIISB Stage II sporulation protein SB SpoIISB | Hypothetical protein spoIISB [Bacillus licheniformis DSM 13] | UniRef100_Q65L00 | Bacillus licheniformis DSM 13 |
| 1511 | spoIISA Stage II sporulation protein SA SpoIISA | Hypothetical protein spoIISA [Bacillus licheniformis DSM 13] | UniRef100_Q65KZ9 | Bacillus licheniformis DSM 13 |
| 1512 | pit low-affinity inorganic phosphate transporter | Pit [Bacillus licheniformis DSM 13] | UniRef100_Q65KZ8 | Bacillus licheniformis DSM 13 |
| 1513 | ykaA putative gamma glutamyl tranferase YkaA | YkaA [Bacillus licheniformis DSM 13] | UniRef100_Q65KZ7 | Bacillus licheniformis DSM 13 |
| 1514 | ggt gamma-glutamyltranspeptidase | Ggt [Bacillus licheniformis DSM 13] | UniRef100_Q65KZ6 | Bacillus licheniformis DSM 13 |
| 1515 | yesL conserved membrane protein YesL | Hypothetical protein yesL [Bacillus licheniformis DSM 13] | UniRef100_Q65KZ5 | Bacillus licheniformis DSM 13 |
| 1516 | yesM two-component sensor histidine kinase YesM | YesM [Bacillus licheniformis DSM 13] | UniRef100_Q65KZ4 | Bacillus licheniformis DSM 13 |
| 1517 | yesN two-component response regulator YesN | YesN [Bacillus licheniformis DSM 13] | UniRef100_Q65KZ3 | Bacillus licheniformis DSM 13 |
| 1518 | yesO putative transport system substrate-binding protein YesO | YesO [Bacillus licheniformis DSM 13] | UniRef100_Q65KZ2 | Bacillus licheniformis DSM 13 |
| 1519 | yesP putatice transporter permease protein YesP | YesP [Bacillus licheniformis DSM 13] | UniRef100_Q65KZ1 | Bacillus licheniformis DSM 13 |
| 1520 | yesQ probable transporter permease protein YesQ | YesQ [Bacillus licheniformis DSM 13] | UniRef100_Q65KZ0 | Bacillus licheniformis DSM 13 |
| 1521 | yesR conserved protein YesR | YesR [Bacillus licheniformis DSM 13] | UniRef100_Q65KY9 | Bacillus licheniformis DSM 13 |
| 1522 | yesS probable transcriptional regulator | YesS [Bacillus licheniformis DSM 13] | UniRef100_Q65KY8 | Bacillus licheniformis DSM 13 |
| 1523 | yesT putative carbohydrate esterase, Family 12, YesT | YesT [Bacillus licheniformis DSM 13] | UniRef100_Q65KY7 | Bacillus licheniformis DSM 13 |

| 1524 | yesU YesU | YesU [Bacillus licheniformis DSM 13] | UniRef100_Q65KY6 | Bacillus licheniformis DSM 13 |
|---|---|---|---|---|
| 1525 | yesV conserved membrane protein YesV | Hypothetical protein yesV [Bacillus licheniformis DSM 13] | UniRef100_Q65KY5 | Bacillus licheniformis DSM 13 |
| 1526 | yesW putative polysaccharide lyase family 11 protein | YesW [Bacillus licheniformis DSM 13] | UniRef100_Q65KY4 | Bacillus licheniformis DSM 13 |
| 1527 | BLP01469 hypothetical protein | | | |
| 1528 | BLP01470 hypothetical protein | Hypothetical protein [Bacillus licheniformis DSM 13] | UniRef100_Q65KY3 | Bacillus licheniformis DSM 13 |
| 1529 | yesT putative carbohydrate esterase Family 12 | Hypothetical protein [Bacillus licheniformis DSM 13] | UniRef100_Q65KY2 | Bacillus licheniformis DSM 13 |
| 1530 | yesX Polysaccharide lyase Family 11 | YesX [Bacillus licheniformis DSM 13] | UniRef100_Q65KY1 | Bacillus licheniformis DSM 13 |
| 1531 | BLP01473 putative oxidoreductase | YccK [Bacillus licheniformis DSM 13] | UniRef100_Q65KY0 | Bacillus licheniformis DSM 13 |
| 1532 | yesY carbohydrate esterase, Family 12 YesY | YesY [Bacillus licheniformis DSM 13] | UniRef100_Q65KX9 | Bacillus licheniformis DSM 13 |
| 1533 | yesZ Glycoside hydrolase, family 42 | YesZ [Bacillus licheniformis DSM 13] | UniRef100_Q65KX8 | Bacillus licheniformis DSM 13 |
| 1534 | yetA conserved hypothetical protein | YetA [Bacillus licheniformis DSM 13] | UniRef100_Q65KX7 | Bacillus licheniformis DSM 13 |
| 1535 | lplA lipoprotein | LplA [Bacillus licheniformis DSM 13] | UniRef100_Q65KX6 | Bacillus licheniformis DSM 13 |
| 1536 | lplB transmembrane lipoprotein | LplB [Bacillus licheniformis DSM 13] | UniRef100_Q65KX5 | Bacillus licheniformis DSM 13 |
| 1537 | lplC transmembrane lipoprotein | LplC [Bacillus licheniformis DSM 13] | UniRef100_Q65KX4 | Bacillus licheniformis DSM 13 |
| 1538 | ykbA putative Amino acid_polyamine permease | YkbA [Bacillus licheniformis DSM 13] | UniRef100_Q65KX3 | Bacillus licheniformis DSM 13 |
| 1539 | BLP01481 hypothetical protein | | | |
| 1540 | yckA2 Glyoxalase_Bleomycin resistance protein_dioxygenase domain superfamily protein YckA2 | YkcA [Bacillus licheniformis DSM 13] | UniRef100_Q65KX2 | Bacillus licheniformis DSM 13 |
| 1541 | BLP01483 conserved hypothetical protein | Hypothetical protein [Bacillus licheniformis DSM 13] | UniRef100_Q65KX1 | Bacillus licheniformis DSM 13 |
| 1542 | htrA serine protease HtrA | HtrA [Bacillus licheniformis DSM 13] | UniRef100_Q65KX0 | Bacillus licheniformis DSM 13 |
| 1543 | proG pyrroline-5-carboxylate reductase | ProG [Bacillus licheniformis DSM 13] | UniRef100_Q65KW9 | Bacillus licheniformis DSM 13 |
| 1544 | dppA D-alanyl-aminopeptidase | DppA [Bacillus licheniformis DSM 13] | UniRef100_Q65KW8 | Bacillus licheniformis DSM 13 |
| 1545 | dppB dipeptide ABC transporter (permease) | Dipeptide ABC transporter [Bacillus licheniformis DSM 13] | UniRef100_Q62WB4 | Bacillus licheniformis DSM 13 |
| 1546 | dppC dipeptide ABC transporter (permease) | DppC [Bacillus licheniformis DSM 13] | UniRef100_Q65KW6 | Bacillus licheniformis DSM 13 |
| 1547 | dppD dipeptide ABC transporter (ATP-binding protein) | DppD [Bacillus licheniformis DSM 13] | UniRef100_Q65KW5 | Bacillus licheniformis DSM 13 |
| 1548 | dppE dipeptide ABC transporter (dipeptide-binding protein) | DppE [Bacillus licheniformis DSM 13] | UniRef100_Q65KW4 | Bacillus licheniformis DSM 13 |
| 1549 | yfkA conserved hypothetical protein YfkA | YkfA [Bacillus licheniformis DSM 13] | UniRef100_Q65KW3 | Bacillus licheniformis DSM 13 |
| 1550 | yfkB putative muconate cycloisomerase YfkB | YkfB [Bacillus licheniformis DSM 13] | UniRef100_Q65KW2 | Bacillus licheniformis DSM 13 |
| 1551 | yfkC putative hydrolase YfkC | YkfC [Bacillus licheniformis DSM 13] | UniRef100_Q65KW1 | Bacillus licheniformis DSM 13 |
| 1552 | ykfD Oligopeptide_dipeptide ABC transporter YkfD | YkfD [Bacillus licheniformis DSM 13] | UniRef100_Q65KW0 | Bacillus licheniformis DSM 13 |
| 1553 | ykgA putative transferase, conserved hypothetical protein YkgA | YkgA [Bacillus licheniformis DSM 13] | UniRef100_Q65KV9 | Bacillus licheniformis DSM 13 |
| 1554 | ykhA Putative acyl-CoA thioester hydrolase YkhA | YkhA [Bacillus licheniformis DSM 13] | UniRef100_Q65KV8 | Bacillus licheniformis DSM 13 |
| 1555 | ykjA conserved hypothetical protein YkjA | YkjA [Bacillus licheniformis DSM 13] | UniRef100_Q65KV7 | Bacillus licheniformis DSM 13 |
| 1556 | BLP04721 hypothetical protein | | | |
| 1557 | pelI pectate lyase family 1,PelI | Pel [Bacillus licheniformis DSM 13] | UniRef100_Q65KV6 | Bacillus licheniformis DSM 13 |

| 1558 | BLP01499 putative transcriptional regulator | Hypothetical protein [Bacillus licheniformis DSM 13] | UniRef100_Q65KV5 | Bacillus licheniformis DSM 13 |
|---|---|---|---|---|
| 1559 | BLP01500 hypothetical protein | Hypothetical protein [Bacillus licheniformis DSM 13] | UniRef100_Q65KV4 | Bacillus licheniformis DSM 13 |
| 1560 | BLP01501 putative DNA binding protein | Hypothetical protein [Bacillus licheniformis DSM 13] | UniRef100_Q65KV3 | Bacillus licheniformis DSM 13 |
| 1561 | ykkA conserved hypothetical protein YkkA | YkkA [Bacillus licheniformis DSM 13] | UniRef100_Q65KV2 | Bacillus licheniformis DSM 13 |
| 1562 | ykkC putative membrane protein,probable transporter | YkkC [Bacillus licheniformis DSM 13] | UniRef100_Q65KV1 | Bacillus licheniformis DSM 13 |
| 1563 | ykkD conserved hypothetical protein YkkD | YkkD [Bacillus licheniformis DSM 13] | UniRef100_Q65KV0 | Bacillus licheniformis DSM 13 |
| 1564 | purU Formyltetrahydrofolate deformylase | YkkE [Bacillus licheniformis DSM 13] | UniRef100_Q65KU9 | Bacillus licheniformis DSM 13 |
| 1565 | BLP01506 hypothetical protein | | | |
| 1566 | proB glutamate 5-kinase ProB | ProB [Bacillus licheniformis DSM 13] | UniRef100_Q65KU8 | Bacillus licheniformis DSM 13 |
| 1567 | proA gamma-glutamyl phosphate reductase ProA | ProA [Bacillus licheniformis DSM 13] | UniRef100_Q65KU7 | Bacillus licheniformis DSM 13 |
| 1568 | ohrA organic hydroperoxide resistance protein OhrA | YklA [Bacillus licheniformis DSM 13] | UniRef100_Q65KU6 | Bacillus licheniformis DSM 13 |
| 1569 | ohrR repressor of ohrA - OhrR | YkmA [Bacillus licheniformis DSM 13] | UniRef100_Q65KU5 | Bacillus licheniformis DSM 13 |
| 1570 | BLP01511 hypothetical protein | | | |
| 1571 | ohrB organic hydroperoxide resistance protein, sigmaB regulon | YkzA [Bacillus licheniformis DSM 13] | UniRef100_Q65KU4 | Bacillus licheniformis DSM 13 |
| 1572 | BLP01513 hypothetical protein | Hypothetical protein [Bacillus licheniformis DSM 13] | UniRef100_Q65KU3 | Bacillus licheniformis DSM 13 |
| 1573 | guaD guanine deaminase | GuaD [Bacillus licheniformis DSM 13] | UniRef100_Q65KU2 | Bacillus licheniformis DSM 13 |
| 1574 | BLP01515 putative phosphatase | Hypothetical protein [Bacillus licheniformis DSM 13] | UniRef100_Q65KU1 | Bacillus licheniformis DSM 13 |
| 1575 | BLP01516 hypothetical protein | Hypothetical protein [Bacillus licheniformis DSM 13] | UniRef100_Q65KU0 | Bacillus licheniformis DSM 13 |
| 1576 | BLP01517 hypothetical protein | Hypothetical protein [Bacillus licheniformis DSM 13] | UniRef100_Q65KT9 | Bacillus licheniformis DSM 13 |
| 1577 | metE methionine synthase | MetE [Bacillus licheniformis DSM 13] | UniRef100_Q65KT8 | Bacillus licheniformis DSM 13 |
| 1578 | ispA intracellular serine protease | IspA [Bacillus licheniformis DSM 13] | UniRef100_Q65KT7 | Bacillus licheniformis DSM 13 |
| 1579 | ykzD conserved hypothetical protein YkzD | YkzD [Bacillus licheniformis DSM 13] | UniRef100_Q65KT6 | Bacillus licheniformis DSM 13 |
| 1580 | ykoK Divalent cation transporter | YkoK2 [Bacillus licheniformis DSM 13] | UniRef100_Q65KT5 | Bacillus licheniformis DSM 13 |
| 1581 | BLP01522 hypothetical protein | Hypothetical protein [Bacillus licheniformis DSM 13] | UniRef100_Q65KT4 | Bacillus licheniformis DSM 13 |
| 1582 | tnrA transcriptional regulator | TnrA [Bacillus licheniformis DSM 13] | UniRef100_Q65KM3 | Bacillus licheniformis DSM 13 |
| 1583 | ykzB conserved hypothetical protein YkzB | YkzB [Bacillus licheniformis DSM 13] | UniRef100_Q65KM2 | Bacillus licheniformis DSM 13 |
| 1584 | ykoL conserved hypothetical protein YkoL | Hypothetical protein [Bacillus licheniformis DSM 13] | UniRef100_Q62W20 | Bacillus licheniformis DSM 13 |
| 1585 | ykoM hypothetical DNA-binding protein YkoM | YkoM [Bacillus licheniformis DSM 13] | UniRef100_Q65KM0 | Bacillus licheniformis DSM 13 |
| 1586 | ykoU ATP-dependent DNA ligase YkoU | YkoU [Bacillus licheniformis DSM 13] | UniRef100_Q65KL9 | Bacillus licheniformis DSM 13 |
| 1587 | ykoV Cytochrome c heme-binding site protein YkoV | YkoV [Bacillus licheniformis DSM 13] | UniRef100_Q65KL8 | Bacillus licheniformis DSM 13 |
| 1588 | ykoW GGDEF, sensory domain containing protein YkoW | YkoW [Bacillus licheniformis DSM 13] | UniRef100_Q65KL7 | Bacillus licheniformis DSM 13 |
| 1589 | BLP01530 hypothetical protein | | | |
| 1590 | ykoX DedA family protein YkoX | YkoX [Bacillus licheniformis DSM 13] | UniRef100_Q65KL6 | Bacillus licheniformis DSM 13 |
| 1591 | ykoY Integral membrane protein YkoY | YkoY [Bacillus licheniformis DSM 13] | UniRef100_Q65KL5 | Bacillus licheniformis DSM 13 |
| 1592 | sigI RNA polymerase sigma factor | SigI [Bacillus licheniformis DSM 13] | UniRef100_Q65KL4 | Bacillus licheniformis DSM 13 |

| 1593 | ykrI conserved hypothetical protein YkrI | YkrI [Bacillus licheniformis DSM 13] | UniRef100_Q65KL3 | Bacillus licheniformis DSM 13 |
|---|---|---|---|---|
| 1594 | sspD small acid-soluble spore protein | SspD [Bacillus licheniformis DSM 13] | UniRef100_Q65KL2 | Bacillus licheniformis DSM 13 |
| 1595 | ykrK conserved hypothetical protein YkrK | YkrK [Bacillus licheniformis DSM 13] | UniRef100_Q65KL1 | Bacillus licheniformis DSM 13 |
| 1596 | htpX putative protease_metalloendopeptidase | YkrL [Bacillus licheniformis DSM 13] | UniRef100_Q65KL0 | Bacillus licheniformis DSM 13 |
| 1597 | ktrD Cation transporter | YkrM [Bacillus licheniformis DSM 13] | UniRef100_Q65KK9 | Bacillus licheniformis DSM 13 |
| 1598 | pbpC penicillin-binding protein 3 | PbpC [Bacillus licheniformis DSM 13] | UniRef100_Q65KK8 | Bacillus licheniformis DSM 13 |
| 1599 | BLP01540 conserved hypothetical protein | Hypothetical protein [Bacillus licheniformis DSM 13] | UniRef100_Q65KK7 | Bacillus licheniformis DSM 13 |
| 1600 | ykzE conserved hypothetical protein YkzE | YkzE [Bacillus licheniformis DSM 13] | UniRef100_Q65KK6 | Bacillus licheniformis DSM 13 |
| 1601 | ykrP putative Acyltransferase 3 YkrP | YkrP [Bacillus licheniformis DSM 13] | UniRef100_Q65KK5 | Bacillus licheniformis DSM 13 |
| 1602 | kinE two-component sensor histidine kinase | KinE [Bacillus licheniformis DSM 13] | UniRef100_Q65KK4 | Bacillus licheniformis DSM 13 |
| 1603 | ogt O6-methylguanine DNA alkyltransferase | Ogt [Bacillus licheniformis DSM 13] | UniRef100_Q65KK3 | Bacillus licheniformis DSM 13 |
| 1604 | BLP01545 hypothetical protein | Hypothetical protein [Bacillus licheniformis DSM 13] | UniRef100_Q62W01 | Bacillus licheniformis DSM 13 |
| 1605 | mtnS methylthioribose-1-phosphate isomerase | YkrS [Bacillus licheniformis DSM 13] | UniRef100_Q65KK2 | Bacillus licheniformis DSM 13 |
| 1606 | mtnK methylthioribose kinase | YkrT [Bacillus licheniformis DSM 13] | UniRef100_Q65KK1 | Bacillus licheniformis DSM 13 |
| 1607 | mtnU methylthioribose recycling protein | YkrU [Bacillus licheniformis DSM 13] | UniRef100_Q65KK0 | Bacillus licheniformis DSM 13 |
| 1608 | mtnV Aminotransferase, class I and II | YkrV [Bacillus licheniformis DSM 13] | UniRef100_Q65KJ9 | Bacillus licheniformis DSM 13 |
| 1609 | mtnW 2,3-diketo-5-methylthiopentyl-1-phosphate enolase | YkrW [Bacillus licheniformis DSM 13] | UniRef100_Q65KJ8 | Bacillus licheniformis DSM 13 |
| 1610 | ykrY putative  Methylthioribulose-1-phosphate dehydratase YkrY | YkrY (HAD-superfamily hydrolase, subfamily IB (PSPase-like),HAD- superfamily subfamily IB hydrolase, hypothetical 1) [Bacillus licheniformis DSM 13] | UniRef100_Q65KJ7 | Bacillus licheniformis DSM 13 |
| 1611 | mtnZ 1,2-dihydroxy-3-keto-5-methylthiopentene dioxygenase | YkrZ [Bacillus licheniformis DSM 13] | UniRef100_Q65KJ6 | Bacillus licheniformis DSM 13 |
| 1612 | ykvA conserved hypothetical protein YkvA | YkvA [Bacillus licheniformis DSM 13] | UniRef100_Q65KJ4 | Bacillus licheniformis DSM 13 |
| 1613 | spo0E negative sporulation regulatory phosphatase | Spo0E [Bacillus licheniformis DSM 13] | UniRef100_Q65KJ3 | Bacillus licheniformis DSM 13 |
| 1614 | kinD two-component sensor histidine kinase | KinD [Bacillus licheniformis DSM 13] | UniRef100_Q65KJ2 | Bacillus licheniformis DSM 13 |
| 1615 | ykvE putative DNA-binding protein YkvE | YkvE [Bacillus licheniformis DSM 13] | UniRef100_Q65KJ1 | Bacillus licheniformis DSM 13 |
| 1616 | motB motility protein B | MotB [Bacillus licheniformis DSM 13] | UniRef100_Q65KJ0 | Bacillus licheniformis DSM 13 |
| 1617 | motA motility protein A | MotA [Bacillus licheniformis DSM 13] | UniRef100_Q65KI9 | Bacillus licheniformis DSM 13 |
| 1618 | clpE ATP-dependent Clp protease-like (class III stress gene) ClpE | ClpE [Bacillus licheniformis DSM 13] | UniRef100_Q65KI8 | Bacillus licheniformis DSM 13 |
| 1619 | BLP01561 hypothetical protein | | | |
| 1620 | ykvI Amino acid_polyamine transporter II YkvI | YkvI [Bacillus licheniformis DSM 13] | UniRef100_Q65KI7 | Bacillus licheniformis DSM 13 |
| 1621 | queC Queuosine (Q) synthesis protein QueC | YkvJ [Bacillus licheniformis DSM 13] | UniRef100_Q65KI6 | Bacillus licheniformis DSM 13 |
| 1622 | queD 6-pyruvoyl tetrahydropterin synthase QueD | YkvK [Bacillus licheniformis DSM 13] | UniRef100_Q65KI5 | Bacillus licheniformis DSM 13 |
| 1623 | queE QueE | YkvL [Bacillus licheniformis DSM 13] | UniRef100_Q65KI4 | Bacillus licheniformis DSM 13 |
| 1624 | queF Queuosine biosynthesis protein QueF | YkvM [Bacillus licheniformis DSM 13] | UniRef100_Q65KI3 | Bacillus licheniformis DSM 13 |
| 1625 | BLP01567 putative Lambda integrase-like protein | Hypothetical protein [Bacillus licheniformis DSM 13] | UniRef100_Q65KI2 | Bacillus licheniformis DSM 13 |

| 1626 | BLP01568 putative integrase_recombinase | Hypothetical protein [Bacillus licheniformis DSM 13] | UniRef100_Q65KI1 | Bacillus licheniformis DSM 13 |
|---|---|---|---|---|
| 1627 | BLP01569 conserved hyopothetical protein | Hypothetical protein [Bacillus licheniformis DSM 13] | UniRef100_Q65KH9 | Bacillus licheniformis DSM 13 |
| 1628 | BLP01570 conserved hypothetical protein | Hypothetical protein [Bacillus licheniformis DSM 13] | UniRef100_Q65KH8 | Bacillus licheniformis DSM 13 |
| 1629 | BLP01571 conserved hypothetical protein | Hypothetical protein [Bacillus licheniformis DSM 13] | UniRef100_Q65KC9 | Bacillus licheniformis DSM 13 |
| 1630 | BLP04818 conserved hypothetical protein | Hypothetical protein [Bacillus licheniformis DSM 13] | UniRef100_Q65KH6 | Bacillus licheniformis DSM 13 |
| 1631 | BLP01572 hypothetical protein | | | |
| 1632 | BLP01573 conserved hypothetical protein | Hypothetical protein [Bacillus licheniformis DSM 13] | UniRef100_Q62VX4 | Bacillus licheniformis DSM 13 |
| 1633 | BLP01574 hypothetical protein | Hypothetical protein [Bacillus licheniformis DSM 13] | UniRef100_Q65KH3 | Bacillus licheniformis DSM 13 |
| 1634 | BLP01575 hypothetical protein | | | |
| 1635 | BLP01576 hypothetical protein | | | |
| 1636 | BLP04851 conserved hypothetical protein | | | |
| 1637 | BLP01577 putative transposase | Hypothetical protein [Bacillus licheniformis DSM 13] | UniRef100_Q65EU5 | Bacillus licheniformis DSM 13 |
| 1638 | BLP04852 hypothetical protein | | | |
| 1639 | BLP01578 hypothetical protein | | | |
| 1640 | BLP04853 hypothetical protein | | | |
| 1641 | BLP04722 hypothetical protein | | | |
| 1642 | BLP01579 hypothetical protein | | | |
| 1643 | BLP04854 hypothetical protein | | | |
| 1644 | BLP04855 hypothetical protein | | | |
| 1645 | BLP04724 hypothetical protein | BH0955 protein [Bacillus halodurans] | UniRef100_Q9KEA1 | Bacillus halodurans |
| 1646 | BLP01580 hypothetical protein | | | |
| 1647 | BLP01581 hypothetical protein | | | |
| 1648 | BLP01582 conserved hypothetical protein | Hypothetical protein [Bacillus licheniformis DSM 13] | UniRef100_Q65KG9 | Bacillus licheniformis DSM 13 |
| 1649 | BLP04856 hypothetical protein | | | |
| 1650 | BLP01583 hypothetical protein | | | |
| 1651 | BLP01584 hypothetical protein | Hypothetical protein CTC01098 [Clostridium tetani] | UniRef100_Q896B0 | Clostridium tetani |
| 1652 | BLP04857 hypothetical protein | | | |
| 1653 | BLP01585 hypothetical protein | | | |
| 1654 | BLP01586 hypothetical protein | | | |
| 1655 | BLP04725 hypothetical protein | | | |
| 1656 | BLP01587 phage related, putative terminase large sub-unit | Prophage LambdaBa02, terminase, large subunit, putative [Bacillus anthracis] | UniRef100_Q81W88 | Bacillus anthracis |
| 1657 | BLP01589 integrase like protein | | | |
| 1658 | BLP01590 phage protein | Phage protein [Bacillus cereus] | UniRef100_Q81ES3 | Bacillus cereus |
| 1659 | BLP01591 phage protein | Phage protein [Bacillus cereus] | UniRef100_Q81ES4 | Bacillus cereus |
| 1660 | BLP01592 phage portal like protein | | | |

| 1661 | BLP01593 phage head protein | Lin2392 protein [Listeria innocua] | UniRef100_Q928Y8 | Listeria innocua |
|---|---|---|---|---|
| 1662 | BLP01594 phage protein | | | |
| 1663 | BLP01595 phage protein minor capsid-like | | | |
| 1664 | BLP01596 phage protein | Phage protein [Bacillus cereus] | UniRef100_Q81ES0 | Bacillus cereus |
| 1665 | BLP01597 hypothetical protein | | | |
| 1666 | BLP01598 hypothetical protein | | | |
| 1667 | BLP04858 hypothetical protein | | | |
| 1668 | BLP01599 hypothetical protein | | | |
| 1669 | BLP01600 hypothetical protein | | | |
| 1670 | BLP01601 phage protein | ORF36 [Bacteriophage phi-105] | UniRef100_Q9ZXE7 | Bacteriophage phi-105 |
| 1671 | BLP01602 phage related protein | Hypothetical protein [Bacillus licheniformis DSM 13] | UniRef100_Q65KF4 | Bacillus licheniformis DSM 13 |
| 1672 | BLP01603 phage related protein | Hypothetical Phage minor structural protein, N-terminal [Bacillus licheniformis DSM 13] | UniRef100_Q65KF3 | Bacillus licheniformis DSM 13 |
| 1673 | BLP01605 phage related protein | Hypothetical protein [Bacillus licheniformis DSM 13] | UniRef100_Q65KF2 | Bacillus licheniformis DSM 13 |
| 1674 | BLP01606 phage related protein | PBSX [Bacillus licheniformis DSM 13] | UniRef100_Q65KF1 | Bacillus licheniformis DSM 13 |
| 1675 | BLP01607 hypothetical phage like protein | YomQ [Bacillus licheniformis DSM 13] | UniRef100_Q65EU0 | Bacillus licheniformis DSM 13 |
| 1676 | BLP01608 hypothetical protein | | | |
| 1677 | BLP01609 hypothetical protein | | | |
| 1678 | xlyB N-acetylmuramoyl-L-alanine amidase | N-acetylmuramoyl-L-alanine amidase xlyB precursor [Bacillus subtilis] | UniRef100_O34391 | Bacillus subtilis |
| 1679 | BLP01611 putative holin phage-like protein | Holin protein [Bacillus halodurans] | UniRef100_Q9KE89 | Bacillus halodurans |
| 1680 | ydcL Lambda integrase-like, N-terminal, DNA breaking-rejoining enzyme, catalytic core | Hypothetical protein [Bacillus licheniformis DSM 13] | UniRef100_Q65KE0 | Bacillus licheniformis DSM 13 |
| 1681 | BLP01613 hypothetical protein | YkvM protein [Bacillus subtilis] | UniRef100_O31678 | Bacillus subtilis |
| 1682 | BLP01614 hypothetical protein | | | |
| 1683 | rapH Response regulator aspartate phosphatase H | Response regulator aspartate phosphatase H [Bacillus subtilis] | UniRef100_P40771 | Bacillus subtilis |
| 1684 | BLP01616 hypothetical protein | | | |
| 1685 | yoaT conserved membrane protein YoaT | Hypothetical protein yoaT [Bacillus licheniformis DSM 13] | UniRef100_Q65KD5 | Bacillus licheniformis DSM 13 |
| 1686 | yozG probable transcriptional regulator YozG | YozG [Bacillus licheniformis DSM 13] | UniRef100_Q65KD4 | Bacillus licheniformis DSM 13 |
| 1687 | yoaS YoaS | Hypothetical protein yoaS [Bacillus licheniformis DSM 13] | UniRef100_Q65KD3 | Bacillus licheniformis DSM 13 |
| 1688 | BLP04726 hypothetical protein | | | |
| 1689 | BLP01620 hypothetical protein | Hypothetical protein [Bacillus licheniformis DSM 13] | UniRef100_Q65KD2 | Bacillus licheniformis DSM 13 |
| 1690 | BLP01621 hypothetical protein | Hypothetical protein [Bacillus licheniformis DSM 13] | UniRef100_Q65KD0 | Bacillus licheniformis DSM 13 |
| 1691 | BLP01622 hypothetical protein | Hypothetical protein [Bacillus licheniformis DSM 13] | UniRef100_Q65KC9 | Bacillus licheniformis DSM 13 |
| 1692 | BLP01623 hypothetical protein | | | |
| 1693 | BLP01624 hypothetical protein | Hypothetical protein [Bacillus licheniformis DSM 13] | UniRef100_Q65KC8 | Bacillus licheniformis DSM 13 |
| 1694 | BLP01625 hypothetical protein | | | |

| 1695 | ykvS conserved hypothetical protein YkvS | Hypothetical protein [Bacillus licheniformis DSM 13] | UniRef100_Q65KC7 | Bacillus licheniformis DSM 13 |
|------|------|------|------|------|
| 1696 | BLP01627 hypothetical protein | Hypothetical protein [Bacillus licheniformis DSM 13] | UniRef100_Q65KC6 | Bacillus licheniformis DSM 13 |
| 1697 | BLP01628 hypothetical protein | | | |
| 1698 | ykvT putative Cell wall hydrolase YkvT | YkvT [Bacillus licheniformis DSM 13] | UniRef100_Q65KC5 | Bacillus licheniformis DSM 13 |
| 1699 | ykvU Polysaccharide biosynthesis protein YkvU | YkvU [Bacillus licheniformis DSM 13] | UniRef100_Q65KC4 | Bacillus licheniformis DSM 13 |
| 1700 | ykvV Thioredoxin type domain, electron transporter protein YkvV | YkvV [Bacillus licheniformis DSM 13] | UniRef100_Q65KC3 | Bacillus licheniformis DSM 13 |
| 1701 | zosA Heavy metal-(Cd_Co_Hg_Pb_Zn)-translocating P-type ATPase - ZosA | YkvW [Bacillus licheniformis DSM 13] | UniRef100_Q65KC2 | Bacillus licheniformis DSM 13 |
| 1702 | ykvY Peptidase M24,putative metallopetidase YkvY | YkvY [Bacillus licheniformis DSM 13] | UniRef100_Q65KC1 | Bacillus licheniformis DSM 13 |
| 1703 | BLP01634 hypothetical protein | | | |
| 1704 | BLP01635 hypothetical protein | | | |
| 1705 | BLP01636 conserved hypothetical protein | Hypothetical Necrosis inducing [Bacillus licheniformis DSM 13] | UniRef100_Q62VS2 | Bacillus licheniformis DSM 13 |
| 1706 | ykvZ Peptidylprolyl isomerase YkvZ | YkvZ [Bacillus licheniformis DSM 13] | UniRef100_Q65KB9 | Bacillus licheniformis DSM 13 |
| 1707 | glcT transcriptional antiterminator | GlcT [Bacillus licheniformis DSM 13] | UniRef100_Q65KB8 | Bacillus licheniformis DSM 13 |
| 1708 | ptsG phosphotransferase system (PTS) glucose-specific enzyme IICBA component | PtsG [Bacillus licheniformis DSM 13] | UniRef100_Q65KB7 | Bacillus licheniformis DSM 13 |
| 1709 | ptsH histidine-containing phosphocarrier protein of the phosphotransferase system (PTS) (HPr protein) | PtsH [Bacillus licheniformis DSM 13] | UniRef100_Q65KB6 | Bacillus licheniformis DSM 13 |
| 1710 | ptsI phosphotransferase system (PTS) enzyme I | PtsI (Phosphotransferase system (PTS) enzyme I) [Bacillus licheniformis DSM 13] | UniRef100_Q65KB5 | Bacillus licheniformis DSM 13 |
| 1711 | splA transcriptional regulator | SplA [Bacillus licheniformis DSM 13] | UniRef100_Q65KB4 | Bacillus licheniformis DSM 13 |
| 1712 | splB spore photoproduct (thymine dimer) lyase | SplB [Bacillus licheniformis DSM 13] | UniRef100_Q65KB3 | Bacillus licheniformis DSM 13 |
| 1713 | ykwB GCN5-related N-acetyltransferase YkwB | YkwB [Bacillus licheniformis DSM 13] | UniRef100_Q65KB2 | Bacillus licheniformis DSM 13 |
| 1714 | mcpC methyl-accepting chemotaxis protein | McpC [Bacillus licheniformis DSM 13] | UniRef100_Q65KB1 | Bacillus licheniformis DSM 13 |
| 1715 | BLP01646 beta-ketoacyl-acyl carrier protein reductase | Hypothetical protein [Bacillus licheniformis DSM 13] | UniRef100_Q65KB0 | Bacillus licheniformis DSM 13 |
| 1716 | ykwC 3-hydroxyisobutyrate dehydrogenase YkwC | YkwC [Bacillus licheniformis DSM 13] | UniRef100_Q65KA9 | Bacillus licheniformis DSM 13 |
| 1717 | ykwD Allergen V5_Tpx-1 related YkwD | YkwD [Bacillus licheniformis DSM 13] | UniRef100_Q65KA8 | Bacillus licheniformis DSM 13 |
| 1718 | ykuA Penicillin-binding Protein dimerisation domain YkuA | YkuA [Bacillus licheniformis DSM 13] | UniRef100_Q65KA7 | Bacillus licheniformis DSM 13 |
| 1719 | BLP01650 hypothetical protein | KinA [Bacillus licheniformis DSM 13] | UniRef100_Q65KA6 | Bacillus licheniformis DSM 13 |
| 1720 | patA putative aspartate transaminase | PatA [Bacillus licheniformis DSM 13] | UniRef100_Q65KA5 | Bacillus licheniformis DSM 13 |
| 1721 | BLP01652 hypothetical protein | Hypothetical protein [Bacillus licheniformis DSM 13] | UniRef100_Q62VQ6 | Bacillus licheniformis DSM 13 |
| 1722 | yxaI conserved hypothetical protein YxaI | YxaI [Bacillus licheniformis DSM 13] | UniRef100_Q65KA4 | Bacillus licheniformis DSM 13 |
| 1723 | yxiO MFS Sugar transporter superfamily protein | YxiO [Bacillus licheniformis DSM 13] | UniRef100_Q65KA3 | Bacillus licheniformis DSM 13 |
| 1724 | cheV CheV | Hypothetical protein cheV [Bacillus licheniformis DSM 13] | UniRef100_Q65KA2 | Bacillus licheniformis DSM 13 |
| 1725 | ykyB hypothetical protein YkyB | YkyB [Bacillus licheniformis DSM 13] | UniRef100_Q65KA1 | Bacillus licheniformis DSM 13 |
| 1726 | ykuC Drug antiporter protein YkuC | YkuC [Bacillus licheniformis DSM 13] | UniRef100_Q65KA0 | Bacillus licheniformis DSM 13 |

| 1727 | ykuD Peptidoglycan-binding protein YkuD | YkuD [Bacillus licheniformis DSM 13] | UniRef100_Q65K99 | Bacillus licheniformis DSM 13 |
|---|---|---|---|---|
| 1728 | BLP01660 hypothetical protein | Hypothetical protein [Bacillus licheniformis DSM 13] | UniRef100_Q65K98 | Bacillus licheniformis DSM 13 |
| 1729 | ykuE Hypothetical metallophosphoesterase YkuE | YkuE [Bacillus licheniformis DSM 13] | UniRef100_Q65K97 | Bacillus licheniformis DSM 13 |
| 1730 | ykuF Short-chain dehydrogenase_reductase YkuF | YkuF [Bacillus licheniformis DSM 13] | UniRef100_Q65K96 | Bacillus licheniformis DSM 13 |
| 1731 | ykuI YkuI | YkuI [Bacillus licheniformis DSM 13] | UniRef100_Q65K95 | Bacillus licheniformis DSM 13 |
| 1732 | BLP01663 hypothetical protein | | | |
| 1733 | BLP01664 hypothetical protein | Hypothetical protein [Bacillus licheniformis DSM 13] | UniRef100_Q65K94 | Bacillus licheniformis DSM 13 |
| 1734 | BLP01665 hypothetical protein | | | |
| 1735 | ykuJ YkuJ | YkuJ [Bacillus licheniformis DSM 13] | UniRef100_Q65K93 | Bacillus licheniformis DSM 13 |
| 1736 | BLP01667 hypothetical protein | Hypothetical protein [Bacillus anthracis] | UniRef100_Q81MP7 | Bacillus anthracis |
| 1737 | ykuK conserved hypothetical protein YkuK | YkuK [Bacillus licheniformis DSM 13] | UniRef100_Q65K92 | Bacillus licheniformis DSM 13 |
| 1738 | ykzF conserved hypothetical protein YkzF | YkzF [Bacillus licheniformis DSM 13] | UniRef100_Q65K91 | Bacillus licheniformis DSM 13 |
| 1739 | ykuL CBS domain protein YkuL | YkuL [Bacillus licheniformis DSM 13] | UniRef100_Q65K90 | Bacillus licheniformis DSM 13 |
| 1740 | ccpC transcriptional regulator | CcpC [Bacillus licheniformis DSM 13] | UniRef100_Q65K89 | Bacillus licheniformis DSM 13 |
| 1741 | ykuN Flavodoxin, short chain YkuN | YkuN [Bacillus licheniformis DSM 13] | UniRef100_Q65K88 | Bacillus licheniformis DSM 13 |
| 1742 | ykuO Glycoside hydrolase YkuO | YkuO [Bacillus licheniformis DSM 13] | UniRef100_Q65K87 | Bacillus licheniformis DSM 13 |
| 1743 | ykuP Flavodoxin, short chain YkuP | YkuP [Bacillus licheniformis DSM 13] | UniRef100_Q65K86 | Bacillus licheniformis DSM 13 |
| 1744 | ykuQ Tetrahydrodipicolinate succinylase | YkuQ [Bacillus licheniformis DSM 13] | UniRef100_Q65K85 | Bacillus licheniformis DSM 13 |
| 1745 | ykuR Peptidase M20D, amidohydrolase YkuR | YkuR [Bacillus licheniformis DSM 13] | UniRef100_Q65K84 | Bacillus licheniformis DSM 13 |
| 1746 | ykuS YkuS | YkuS [Bacillus licheniformis DSM 13] | UniRef100_Q65K83 | Bacillus licheniformis DSM 13 |
| 1747 | ykuU Alkyl hydroperoxide reductase | YkuU [Bacillus licheniformis DSM 13] | UniRef100_Q65K82 | Bacillus licheniformis DSM 13 |
| 1748 | ykuV Thioredoxin domain protein YkuV | YkuV [Bacillus licheniformis DSM 13] | UniRef100_Q65K81 | Bacillus licheniformis DSM 13 |
| 1749 | rok Rok | Hypothetical protein rok [Bacillus licheniformis DSM 13] | UniRef100_Q65K80 | Bacillus licheniformis DSM 13 |
| 1750 | yknT sigma-E controlled sporulation protein | YknT [Bacillus licheniformis DSM 13] | UniRef100_Q65K79 | Bacillus licheniformis DSM 13 |
| 1751 | mobA molybdopterin-guanine dinucleotide biosynthesis protein A | MobA [Bacillus licheniformis DSM 13] | UniRef100_Q65K78 | Bacillus licheniformis DSM 13 |
| 1752 | moeB molybdopterin biosynthesis protein | MoeB [Bacillus licheniformis DSM 13] | UniRef100_Q65K77 | Bacillus licheniformis DSM 13 |
| 1753 | moeA molybdopterin biosynthesis protein | MoeA [Bacillus licheniformis DSM 13] | UniRef100_Q65K76 | Bacillus licheniformis DSM 13 |
| 1754 | mobB molybdopterin-guanine dinucleotide biosynthesis protein B | MobB [Bacillus licheniformis DSM 13] | UniRef100_Q65K75 | Bacillus licheniformis DSM 13 |
| 1755 | moaE molybdopterin converting factor (subunit 2) | MoaE [Bacillus licheniformis DSM 13] | UniRef100_Q65K74 | Bacillus licheniformis DSM 13 |
| 1756 | moaD molybdopterin converting factor (subunit 1) MoaD | MoaD [Bacillus licheniformis DSM 13] | UniRef100_Q65K73 | Bacillus licheniformis DSM 13 |
| 1757 | BLP01687 conserved hypothetical protein | Hypothetical protein [Bacillus licheniformis DSM 13] | UniRef100_Q65K72 | Bacillus licheniformis DSM 13 |
| 1758 | yknU ABC transporter YknU | YknU [Bacillus licheniformis DSM 13] | UniRef100_Q65K71 | Bacillus licheniformis DSM 13 |
| 1759 | yknV ABC transporter YknV | YknV [Bacillus licheniformis DSM 13] | UniRef100_Q65K70 | Bacillus licheniformis DSM 13 |
| 1760 | yknW conserved hypothetical protein YknW | YknW [Bacillus licheniformis DSM 13] | UniRef100_Q65K69 | Bacillus licheniformis DSM 13 |
| 1761 | yknX putative secretion protein, protein transporter | YknX [Bacillus licheniformis DSM 13] | UniRef100_Q65K68 | Bacillus licheniformis DSM 13 |

| | | YknX | | | |
|---|---|---|---|---|---|
| | 1762 | yknY ABC transporter YknY | YknY [Bacillus licheniformis DSM 13] | UniRef100_Q65K67 | Bacillus licheniformis DSM 13 |
| | 1763 | yknZ putative ABC transporter permease YknZ | YknZ [Bacillus licheniformis DSM 13] | UniRef100_Q65K66 | Bacillus licheniformis DSM 13 |
| | 1764 | fruR transcriptional regulator (DeoR family) | FruR [Bacillus licheniformis DSM 13] | UniRef100_Q65K65 | Bacillus licheniformis DSM 13 |
| | 1765 | fruK fructose-1-phosphate kinase | FruK [Bacillus licheniformis DSM 13] | UniRef100_Q65K64 | Bacillus licheniformis DSM 13 |
| | 1766 | fruA Phosphotransferase system (PTS) fructose-specific enzyme IIABC component FruA | FruA (Phosphotransferase system (PTS) fructose-specific enzyme IIABC component) [Bacillus licheniformis DSM 13] | UniRef100_Q65K63 | Bacillus licheniformis DSM 13 |
| | 1767 | sipT type I signal peptidase | Signal peptidase I [Bacillus licheniformis] | UniRef100_P42668 | Bacillus licheniformis |
| | 1768 | ykoA conserved hypothetical protein YkoA | YkoA [Bacillus licheniformis DSM 13] | UniRef100_Q65K61 | Bacillus licheniformis DSM 13 |
| | 1769 | BLP01699 hypothetical protein | Hypothetical protein [Bacillus licheniformis DSM 13] | UniRef100_Q65K60 | Bacillus licheniformis DSM 13 |
| | 1770 | ykpA ABC transporter YkpA | YkpA [Bacillus licheniformis DSM 13] | UniRef100_Q65K59 | Bacillus licheniformis DSM 13 |
| | 1771 | BLP01701 conserved hypothetical protein | Hypothetical protein [Bacillus licheniformis DSM 13] | UniRef100_Q65K58 | Bacillus licheniformis DSM 13 |
| | 1772 | ampS aminopeptidase | AmpS [Bacillus licheniformis DSM 13] | UniRef100_Q65K57 | Bacillus licheniformis DSM 13 |
| | 1773 | ykpC conserved hypothetical protein YkpC | Hypothetical protein [Bacillus licheniformis DSM 13] | UniRef100_Q62VK8 | Bacillus licheniformis DSM 13 |
| | 1774 | mreBH cell-shape determining protein | MreBH [Bacillus licheniformis DSM 13] | UniRef100_Q65K55 | Bacillus licheniformis DSM 13 |
| | 1775 | BLP01705 hypothetical protein | | | |
| | 1776 | abhB transcriptional regulator of transition state genes (AbrB-like) | Abh [Bacillus licheniformis DSM 13] | UniRef100_Q65K54 | Bacillus licheniformis DSM 13 |
| | 1777 | kinC two-component sensor histidine kinase | KinC [Bacillus licheniformis DSM 13] | UniRef100_Q65K53 | Bacillus licheniformis DSM 13 |
| | 1778 | ktrC NAD-binding site | YkqB [Bacillus licheniformis DSM 13] | UniRef100_Q65K52 | Bacillus licheniformis DSM 13 |
| | 1779 | BLP01709 hypothetical protein | | | |
| | 1780 | adeC adenine deaminase | AdeC [Bacillus licheniformis DSM 13] | UniRef100_Q65K51 | Bacillus licheniformis DSM 13 |
| | 1781 | ykqC essential RNase J1_J2, metallo-beta-lactamase superfamily protein YkqC | YkqC [Bacillus licheniformis DSM 13] | UniRef100_Q65K50 | Bacillus licheniformis DSM 13 |
| | 1782 | ykzG conserved hypothetical protein YkzG | YkzG [Bacillus licheniformis DSM 13] | UniRef100_Q65K49 | Bacillus licheniformis DSM 13 |
| | 1783 | BLP04727 hypothetical protein | | | |
| | 1784 | ykrA putative HAD hydrolase YkrA | YkrA [Bacillus licheniformis DSM 13] | UniRef100_Q65K48 | Bacillus licheniformis DSM 13 |
| | 1785 | defB main formylmethionine deformylase | YkrB [Bacillus licheniformis DSM 13] | UniRef100_Q65K47 | Bacillus licheniformis DSM 13 |
| | 1786 | BLP01715 conserved hypothetical protein | | | |
| | 1787 | ykyA putative transcription factor, conserved hypothetical YkyA | Helix-turn-helix, Fis-type, Helix-turn-helix, Fis-type [Bacillus licheniformis DSM 13] | UniRef100_Q62VJ7 | Bacillus licheniformis DSM 13 |
| | 1788 | pdhA pyruvate dehydrogenase (E1 alpha subunit) | PdhA [Bacillus licheniformis DSM 13] | UniRef100_Q65K44 | Bacillus licheniformis DSM 13 |
| | 1789 | pdhB pyruvate dehydrogenase (E1 beta subunit) | PdhB [Bacillus licheniformis DSM 13] | UniRef100_Q65K43 | Bacillus licheniformis DSM 13 |
| | 1790 | pdhC pyruvate dehydrogenase (dihydrolipoamide acetyltransferase E2 subunit) | PdhC [Bacillus licheniformis DSM 13] | UniRef100_Q65K42 | Bacillus licheniformis DSM 13 |
| | 1791 | pdhD dihydrolipoamide dehydrogenase E3 subunit of both pyruvate dehydrogenase and 2-oxoglutarate dehydrogenase complexes | PdhD [Bacillus licheniformis DSM 13] | UniRef100_Q65K41 | Bacillus licheniformis DSM 13 |
| | 1792 | BLP01721 hypothetical protein | | | |

| 1793 | BLP01722 conserved hypothetical protein | Hypothetical protein [Bacillus licheniformis DSM 13] | UniRef100_Q62VI9 | Bacillus licheniformis DSM 13 |
|---|---|---|---|---|
| 1794 | speA arginine decarboxylase | SpeA [Bacillus licheniformis DSM 13] | UniRef100_Q65K37 | Bacillus licheniformis DSM 13 |
| 1795 | BLP01724 hypothetical protein | | | |
| 1796 | yktA conserved hypothetical protein YktA | YktA [Bacillus licheniformis DSM 13] | UniRef100_Q65K36 | Bacillus licheniformis DSM 13 |
| 1797 | yktB conserved hypothetical protein YktB | YktB [Bacillus licheniformis DSM 13] | UniRef100_Q65K35 | Bacillus licheniformis DSM 13 |
| 1798 | ykzI conserved hypothetical YkzI | | | |
| 1799 | suhB Inositol monophosphatase SuhB | YktC [Bacillus licheniformis DSM 13] | UniRef100_Q65K33 | Bacillus licheniformis DSM 13 |
| 1800 | ykzC GCN5-related N-acetyltransferase YkzC | YkzC [Bacillus licheniformis DSM 13] | UniRef100_Q65K32 | Bacillus licheniformis DSM 13 |
| 1801 | ylaA conserved hypothetical protein YlaA | YlaA [Bacillus licheniformis DSM 13] | UniRef100_Q65K31 | Bacillus licheniformis DSM 13 |
| 1802 | ylaB conserved hypothetical YlaB | YlaB [Bacillus licheniformis DSM 13] | UniRef100_Q65K30 | Bacillus licheniformis DSM 13 |
| 1803 | ylaC ECF sigma factor YlaC | YlaC [Bacillus licheniformis DSM 13] | UniRef100_Q65K29 | Bacillus licheniformis DSM 13 |
| 1804 | ylaD antisigma factor for YlaC | YlaD [Bacillus licheniformis DSM 13] | UniRef100_Q65K28 | Bacillus licheniformis DSM 13 |
| 1805 | ylaF conserved hypothetical protein YlaF | YlaF [Bacillus licheniformis DSM 13] | UniRef100_Q65K27 | Bacillus licheniformis DSM 13 |
| 1806 | typA GTP-binding protein TypA | YlaG [Bacillus licheniformis DSM 13] | UniRef100_Q65K26 | Bacillus licheniformis DSM 13 |
| 1807 | ylaH conserved hypothetical protein YlaH | YlaH [Bacillus licheniformis DSM 13] | UniRef100_Q65K25 | Bacillus licheniformis DSM 13 |
| 1808 | yhjH conserved hypothetical DNA-binding protein YhjH | YhjH [Bacillus licheniformis DSM 13] | UniRef100_Q65K24 | Bacillus licheniformis DSM 13 |
| 1809 | yhjG putative Flavoprotein monooxygenase YhjG | YhjG [Bacillus licheniformis DSM 13] | UniRef100_Q65K23 | Bacillus licheniformis DSM 13 |
| 1810 | ylaI YlaI | YlaI [Bacillus licheniformis DSM 13] | UniRef100_Q65K22 | Bacillus licheniformis DSM 13 |
| 1811 | ylaJ conserved hypothetical protein YlaJ | YlaJ [Bacillus licheniformis DSM 13] | UniRef100_Q65K21 | Bacillus licheniformis DSM 13 |
| 1812 | ylaK Nucleotide binding protein YlaK | YlaK [Bacillus licheniformis DSM 13] | UniRef100_Q65K20 | Bacillus licheniformis DSM 13 |
| 1813 | ylaL conserved hypothetical protein YlaL | YlaL [Bacillus licheniformis DSM 13] | UniRef100_Q65K19 | Bacillus licheniformis DSM 13 |
| 1814 | ylaM Glutaminase YlaM | YlaM [Bacillus licheniformis DSM 13] | UniRef100_Q65K18 | Bacillus licheniformis DSM 13 |
| 1815 | ylaN YlaN | YlaN [Bacillus licheniformis DSM 13] | UniRef100_Q65K17 | Bacillus licheniformis DSM 13 |
| 1816 | BLP01743 hypothetical protein | | | |
| 1817 | ftsW cell-division protein | FtsW [Bacillus licheniformis DSM 13] | UniRef100_Q65K16 | Bacillus licheniformis DSM 13 |
| 1818 | pycA pyruvate carboxylase | PycA [Bacillus licheniformis DSM 13] | UniRef100_Q65K15 | Bacillus licheniformis DSM 13 |
| 1819 | ctaA heme-containing membrane protein CtaA | CtaA [Bacillus licheniformis DSM 13] | UniRef100_Q65K14 | Bacillus licheniformis DSM 13 |
| 1820 | ctaB cytochrome caa3 oxydase assembly factor | CtaB [Bacillus licheniformis DSM 13] | UniRef100_Q65K13 | Bacillus licheniformis DSM 13 |
| 1821 | ctaC cytochrome caa3 oxidase (subunit II) | CtaC [Bacillus licheniformis DSM 13] | UniRef100_Q65K12 | Bacillus licheniformis DSM 13 |
| 1822 | ctaD cytochrome caa3 oxidase (subunit I) | CtaD [Bacillus licheniformis DSM 13] | UniRef100_Q65K11 | Bacillus licheniformis DSM 13 |
| 1823 | ctaE cytochrome caa3 oxidase (subunit III) | CtaE [Bacillus licheniformis DSM 13] | UniRef100_Q65K10 | Bacillus licheniformis DSM 13 |
| 1824 | ctaF cytochrome caa3 oxidase (subunit IV) | CtaF [Bacillus licheniformis DSM 13] | UniRef100_Q65K09 | Bacillus licheniformis DSM 13 |
| 1825 | ctaG CtaG | Hypothetical protein ctaG [Bacillus licheniformis DSM 13] | UniRef100_Q65K08 | Bacillus licheniformis DSM 13 |
| 1826 | ylbA conserved hypothetical protein YlbA | YlbA [Bacillus licheniformis DSM 13] | UniRef100_Q65K07 | Bacillus licheniformis DSM 13 |
| 1827 | ylbB YlbB | CBS domain [Bacillus licheniformis DSM 13] | UniRef100_Q62VF7 | Bacillus licheniformis DSM 13 |
| 1828 | ylbC conserved hypothetical protein YlbC | YlbC [Bacillus licheniformis DSM 13] | UniRef100_Q65K05 | Bacillus licheniformis DSM 13 |

| 1829 | ylbD conserved hypothetical protein YlbD | YlbD [Bacillus licheniformis DSM 13] | UniRef100_Q65K04 | Bacillus licheniformis DSM 13 |
|---|---|---|---|---|
| 1830 | ylbE conserved hypothetical protein YlbE | YlbE [Bacillus licheniformis DSM 13] | UniRef100_Q65K03 | Bacillus licheniformis DSM 13 |
| 1831 | BLP01758 hypothetical protein | | | |
| 1832 | ylbF YlbF | YlbF [Bacillus licheniformis DSM 13] | UniRef100_Q65K02 | Bacillus licheniformis DSM 13 |
| 1833 | ylbG conserved hypothetical protein YlbG | YlbG [Bacillus licheniformis DSM 13] | UniRef100_Q65K01 | Bacillus licheniformis DSM 13 |
| 1834 | ylbH Adenine-specific DNA methylase YlbH | YlbH [Bacillus licheniformis DSM 13] | UniRef100_Q65K00 | Bacillus licheniformis DSM 13 |
| 1835 | coaD Coenzyme A biosynthesis protein,Cytidyltransferase-related domain | Ylbl [Bacillus licheniformis DSM 13] | UniRef100_Q65JZ9 | Bacillus licheniformis DSM 13 |
| 1836 | BLP01763 hypothetical protein | | | |
| 1837 | ylbJ YlbJ | Hypothetical protein [Bacillus licheniformis DSM 13] | UniRef100_Q62VE9 | Bacillus licheniformis DSM 13 |
| 1838 | BLP01765 hypothetical protein | | | |
| 1839 | ylbL YlbL | YlbL [Bacillus licheniformis DSM 13] | UniRef100_Q65JZ7 | Bacillus licheniformis DSM 13 |
| 1840 | ylbM conserved hypothetical protein | YlbM [Bacillus licheniformis DSM 13] | UniRef100_Q65JZ6 | Bacillus licheniformis DSM 13 |
| 1841 | ylbN conserved hypothetical protein | YlbN [Bacillus licheniformis DSM 13] | UniRef100_Q65JZ5 | Bacillus licheniformis DSM 13 |
| 1842 | rpmF ribosomal protein L32 | RpmF [Bacillus licheniformis DSM 13] | UniRef100_Q65JZ4 | Bacillus licheniformis DSM 13 |
| 1843 | ylbO DNA-binding domain protein YlbO | YlbO [Bacillus licheniformis DSM 13] | UniRef100_Q65JZ3 | Bacillus licheniformis DSM 13 |
| 1844 | ylbP GCN5-related N-acetyltransferase YlbP | YlbP [Bacillus licheniformis DSM 13] | UniRef100_Q65JZ2 | Bacillus licheniformis DSM 13 |
| 1845 | panE 2-dehydropantoate 2-reductase | YlbQ [Bacillus licheniformis DSM 13] | UniRef100_Q65JZ1 | Bacillus licheniformis DSM 13 |
| 1846 | yllA conserved hypothetical protein YllA | YllA [Bacillus licheniformis DSM 13] | UniRef100_Q65JZ0 | Bacillus licheniformis DSM 13 |
| 1847 | mraZ conserved protein MraZ | Hypothetical protein mraZ [Bacillus licheniformis DSM 13] | UniRef100_Q62VE0 | Bacillus licheniformis DSM 13 |
| 1848 | mraW putative methyltransferase | YlxA [Bacillus licheniformis DSM 13] | UniRef100_Q65JY8 | Bacillus licheniformis DSM 13 |
| 1849 | ftsL cell-division protein | FtsL [Bacillus licheniformis DSM 13] | UniRef100_Q65JY7 | Bacillus licheniformis DSM 13 |
| 1850 | pbpB penicillin-binding protein 2B | PbpB [Bacillus licheniformis DSM 13] | UniRef100_Q65JY6 | Bacillus licheniformis DSM 13 |
| 1851 | BLP01777 hypothetical protein | | | |
| 1852 | spoVD penicillin-binding protein | SpoVD [Bacillus licheniformis DSM 13] | UniRef100_Q65JY5 | Bacillus licheniformis DSM 13 |
| 1853 | BLP01779 hypothetical protein | | | |
| 1854 | murE UDP-N-acetylmuramoylalanyl-D-glutamate-2,6-diaminopimelate ligase | MurE [Bacillus licheniformis DSM 13] | UniRef100_Q65JY4 | Bacillus licheniformis DSM 13 |
| 1855 | mraY phospho-N-acetylmuramoyl-pentapeptidetransferase | MraY [Bacillus licheniformis DSM 13] | UniRef100_Q65JY3 | Bacillus licheniformis DSM 13 |
| 1856 | murD UDP-N-acetylmuramoylalanyl-D-glutamate ligase | MurD [Bacillus licheniformis DSM 13] | UniRef100_Q65JY2 | Bacillus licheniformis DSM 13 |
| 1857 | spoVE SpoVE | Hypothetical protein spoVE [Bacillus licheniformis DSM 13] | UniRef100_Q65JY1 | Bacillus licheniformis DSM 13 |
| 1858 | murG Glycosyl transferase Family 28 | MurG (UDP-N-acetylglucosamine-N-acetylmuramyl-(Pentapeptide)pyrophosphoryl- undecaprenol N-acetylglucosamine transferase) [Bacillus licheniformis DSM 13] | UniRef100_Q65JY0 | Bacillus licheniformis DSM 13 |
| 1859 | murB UDP-N-acetylenolpyruvoylglucosamine reductase | MurB [Bacillus licheniformis DSM 13] | UniRef100_Q65JX9 | Bacillus licheniformis DSM 13 |
| 1860 | divIB cell-division initiation protein | DivIB [Bacillus licheniformis DSM 13] | UniRef100_Q65JX8 | Bacillus licheniformis DSM 13 |

| 1861 | ylxW conserved hypothetical protein YlxW | YlxW [Bacillus licheniformis DSM 13] | UniRef100_Q65JX7 | Bacillus licheniformis DSM 13 |
|------|------------------------------------------|--------------------------------------|------------------|------------------------------|
| 1862 | ylxX conserved hypothetical protein YlxX | YlxX [Bacillus licheniformis DSM 13] | UniRef100_Q65JX6 | Bacillus licheniformis DSM 13 |
| 1863 | sbp small basic protein | Sbp [Bacillus licheniformis DSM 13] | UniRef100_Q65JX5 | Bacillus licheniformis DSM 13 |
| 1864 | ftsA cell-division protein | FtsA [Bacillus licheniformis DSM 13] | UniRef100_Q65JX4 | Bacillus licheniformis DSM 13 |
| 1865 | ftsZ cell-division initiation protein | FtsZ [Bacillus licheniformis DSM 13] | UniRef100_Q65JX3 | Bacillus licheniformis DSM 13 |
| 1866 | BLP01792 hypothetical protein | | | |
| 1867 | bprA bacillopeptidase F | Hypothetical protein [Bacillus licheniformis DSM 13] | UniRef100_Q65JX2 | Bacillus licheniformis DSM 13 |
| 1868 | bprB bacillopeptidase F | Bpr [Bacillus licheniformis DSM 13] | UniRef100_Q65JX1 | Bacillus licheniformis DSM 13 |
| 1869 | spoIIGA protease SpoIIGA | SpoIIGA [Bacillus licheniformis DSM 13] | UniRef100_Q65JX0 | Bacillus licheniformis DSM 13 |
| 1870 | sigE RNA polymerase sporulation-specific sigma-29 factor (sigma-E) | SigE [Bacillus licheniformis DSM 13] | UniRef100_Q65JW9 | Bacillus licheniformis DSM 13 |
| 1871 | sigG RNA polymerase sporulation-specific sigma factor (sigma-G) | SigG [Bacillus licheniformis DSM 13] | UniRef100_Q65JW8 | Bacillus licheniformis DSM 13 |
| 1872 | ylmA ABC transporter YlmA | YlmA [Bacillus licheniformis DSM 13] | UniRef100_Q65JW7 | Bacillus licheniformis DSM 13 |
| 1873 | BLP01799 conserved hypothetical protein | YxjB [Bacillus licheniformis DSM 13] | UniRef100_Q65JW5 | Bacillus licheniformis DSM 13 |
| 1874 | ylmC YlmC | YlmC [Bacillus licheniformis DSM 13] | UniRef100_Q65JW4 | Bacillus licheniformis DSM 13 |
| 1875 | ylmD conserved hypothetical protein YlmD | YlmD [Bacillus licheniformis DSM 13] | UniRef100_Q65JW3 | Bacillus licheniformis DSM 13 |
| 1876 | ylmE putative carboxypeptidase YlmE | YlmE [Bacillus licheniformis DSM 13] | UniRef100_Q65JW2 | Bacillus licheniformis DSM 13 |
| 1877 | ylmF divisiome protein YlmF | YlmF [Bacillus licheniformis DSM 13] | UniRef100_Q65JW1 | Bacillus licheniformis DSM 13 |
| 1878 | ylmG conserved hypothetical protein | YlmG [Bacillus licheniformis DSM 13] | UniRef100_Q65JW0 | Bacillus licheniformis DSM 13 |
| 1879 | ylmH RNA-binding protein YlmH | YlmH [Bacillus licheniformis DSM 13] | UniRef100_Q65JV9 | Bacillus licheniformis DSM 13 |
| 1880 | divIVA cell-division initiation protein | DivIVA [Bacillus licheniformis DSM 13] | UniRef100_Q65JV8 | Bacillus licheniformis DSM 13 |
| 1881 | ileS isoleucyl-tRNA synthetase | IleS [Bacillus licheniformis DSM 13] | UniRef100_Q65JV7 | Bacillus licheniformis DSM 13 |
| 1882 | ylyA conserved hypothetical protein YlyA | YlyA [Bacillus licheniformis DSM 13] | UniRef100_Q65JV6 | Bacillus licheniformis DSM 13 |
| 1883 | BLP04927 hypothetical protein | Hypothetical protein [Bacillus licheniformis DSM 13] | UniRef100_Q65JV5 | Bacillus licheniformis DSM 13 |
| 1884 | lspA signal peptidase II LspA | LspA [Bacillus licheniformis DSM 13] | UniRef100_Q65JV4 | Bacillus licheniformis DSM 13 |
| 1885 | ylyB putative RNA pseudouridine synthase YlyB | YlyB [Bacillus licheniformis DSM 13] | UniRef100_Q65JV3 | Bacillus licheniformis DSM 13 |
| 1886 | pyrR transcriptional attenuator and uracil phosphoribosyltransferase activity (minor) | PyrR [Bacillus licheniformis DSM 13] | UniRef100_Q65JV2 | Bacillus licheniformis DSM 13 |
| 1887 | BLP01812 hypothetical protein | | | |
| 1888 | pyrP uracil permease | PyrP [Bacillus licheniformis DSM 13] | UniRef100_Q65JV1 | Bacillus licheniformis DSM 13 |
| 1889 | pyrB aspartate carbamoyltransferase | PyrB [Bacillus licheniformis DSM 13] | UniRef100_Q65JV0 | Bacillus licheniformis DSM 13 |
| 1890 | pyrC dihydroorotase | PyrC [Bacillus licheniformis DSM 13] | UniRef100_Q65JU9 | Bacillus licheniformis DSM 13 |
| 1891 | pyrAA carbamoyl-phosphate synthetase (glutaminase subunit) | PyrAA [Bacillus licheniformis DSM 13] | UniRef100_Q65JU8 | Bacillus licheniformis DSM 13 |
| 1892 | pyrAB carbamoyl-phosphate synthetase (catalytic subunit) | PyrAB [Bacillus licheniformis DSM 13] | UniRef100_Q65JU7 | Bacillus licheniformis DSM 13 |
| 1893 | pyrK dihydroorotate dehydrogenase (electron transfer subunit) | PyrK [Bacillus licheniformis DSM 13] | UniRef100_Q65JU6 | Bacillus licheniformis DSM 13 |

| 1894 | pyrD dihydroorotate dehydrogenase (catalytic subunit) | PyrD [Bacillus licheniformis DSM 13] | UniRef100_Q65JU5 | Bacillus licheniformis DSM 13 |
|---|---|---|---|---|
| 1895 | pyrF orotidine 5'-phosphate decarboxylase | PyrF [Bacillus licheniformis DSM 13] | UniRef100_Q65JU4 | Bacillus licheniformis DSM 13 |
| 1896 | pyrE orotate phosphoribosyltransferase | PyrE [Bacillus licheniformis DSM 13] | UniRef100_Q65JU3 | Bacillus licheniformis DSM 13 |
| 1897 | BLP01822 conserved hypothetical protein | Hypothetical protein [Bacillus licheniformis DSM 13] | UniRef100_Q65JU2 | Bacillus licheniformis DSM 13 |
| 1898 | cysH phosphoadenosine phosphosulfate | Hypothetical protein [Bacillus licheniformis DSM 13] | UniRef100_Q65JU1 | Bacillus licheniformis DSM 13 |
| 1899 | cysPA sulfate permease | CysP [Bacillus licheniformis DSM 13] | UniRef100_Q65JU0 | Bacillus licheniformis DSM 13 |
| 1900 | sat sulfate adenylyltransferase | Sat [Bacillus licheniformis DSM 13] | UniRef100_Q65JT9 | Bacillus licheniformis DSM 13 |
| 1901 | cysC adenylylsulfate kinase | CysC [Bacillus licheniformis DSM 13] | UniRef100_Q65JT8 | Bacillus licheniformis DSM 13 |
| 1902 | cysG Uroporphyrin-III C-methyltransferase, C-terminal | YlnD [Bacillus licheniformis DSM 13] | UniRef100_Q65JT7 | Bacillus licheniformis DSM 13 |
| 1903 | sirB Cobalamin (vitamin B12) biosynthesis CbiX protein | YlnE (Cobalamin (Vitamin B12) biosynthesis CbiX protein) [Bacillus licheniformis DSM 13] | UniRef100_Q65JT6 | Bacillus licheniformis DSM 13 |
| 1904 | sirA Siroheme synthase SirA | YlnF [Bacillus licheniformis DSM 13] | UniRef100_Q65JT5 | Bacillus licheniformis DSM 13 |
| 1905 | yloA putative fibronectin binding protein YloA | YloA [Bacillus licheniformis DSM 13] | UniRef100_Q65JT4 | Bacillus licheniformis DSM 13 |
| 1906 | yloB ATPase, E1-E2 type protein YloB | YloB [Bacillus licheniformis DSM 13] | UniRef100_Q65JT3 | Bacillus licheniformis DSM 13 |
| 1907 | yloC Conserved hypothetical protein YloC | YloC [Bacillus licheniformis DSM 13] | UniRef100_Q65JT2 | Bacillus licheniformis DSM 13 |
| 1908 | ylzA conserved hypothetical protein YlzA | YlzA [Bacillus licheniformis DSM 13] | UniRef100_Q65JT1 | Bacillus licheniformis DSM 13 |
| 1909 | gmk guanylate kinase | Guanylate kinase [Bacillus licheniformis DSM 13] | UniRef100_Q62V85 | Bacillus licheniformis DSM 13 |
| 1910 | rpoZ RNA polymerase, omega subunit | YloH [Bacillus licheniformis DSM 13] | UniRef100_Q65JS9 | Bacillus licheniformis DSM 13 |
| 1911 | ylol DNA_pantothenate metabolism flavoprotein Ylol | Ylol [Bacillus licheniformis DSM 13] | UniRef100_Q65JS8 | Bacillus licheniformis DSM 13 |
| 1912 | priA primosomal replication factor Y (primosomal protein N') | PriA [Bacillus licheniformis DSM 13] | UniRef100_Q65JS7 | Bacillus licheniformis DSM 13 |
| 1913 | def polypeptide deformylase | Def [Bacillus licheniformis DSM 13] | UniRef100_Q65JS6 | Bacillus licheniformis DSM 13 |
| 1914 | fmt methionyl-tRNA formyltransferase Fmt | Fmt [Bacillus licheniformis DSM 13] | UniRef100_Q65JS5 | Bacillus licheniformis DSM 13 |
| 1915 | rsmB rRNA SAM-dependent methyltransferase RmsB | Fmu, rRNA SAM-dependent methyltransferase [Bacillus licheniformis DSM 13] | UniRef100_Q62V79 | Bacillus licheniformis DSM 13 |
| 1916 | yloN Conserved hypothetical protein YloN | YloN [Bacillus licheniformis DSM 13] | UniRef100_Q65JS3 | Bacillus licheniformis DSM 13 |
| 1917 | prpC protein phosphatase | PrpC [Bacillus licheniformis DSM 13] | UniRef100_Q65JS2 | Bacillus licheniformis DSM 13 |
| 1918 | prkC protein kinase PrkC | PrkC [Bacillus licheniformis DSM 13] | UniRef100_Q65JS1 | Bacillus licheniformis DSM 13 |
| 1919 | engC GTP binding protein EngC | YloQ [Bacillus licheniformis DSM 13] | UniRef100_Q65JS0 | Bacillus licheniformis DSM 13 |
| 1920 | rpe ribulose-5-phosphate 3-epimerase Rpe | Rpe [Bacillus licheniformis DSM 13] | UniRef100_Q65JR9 | Bacillus licheniformis DSM 13 |
| 1921 | yloS Thiamine pyrophosphokinase YloS | YloS [Bacillus licheniformis DSM 13] | UniRef100_Q65JR8 | Bacillus licheniformis DSM 13 |
| 1922 | spoVM SpoVM | Stage V sporulation protein M [Bacillus subtilis] | UniRef100_P37817 | Bacillus subtilis |
| 1923 | rpmB 50S ribosomal protein L28 RpmB | RpmB [Bacillus licheniformis DSM 13] | UniRef100_Q65JR6 | Bacillus licheniformis DSM 13 |
| 1924 | yloU highly conserved hypothetical protein YloU | YloU [Bacillus licheniformis DSM 13] | UniRef100_Q65JR5 | Bacillus licheniformis DSM 13 |
| 1925 | yloV glycerone kinase protein YloV | Dak phosphatase domain [Bacillus licheniformis DSM 13] | UniRef100_Q62V69 | Bacillus licheniformis DSM 13 |
| 1926 | sdaAB L-serine dehydratase (beta chain) | SdaAB [Bacillus licheniformis DSM 13] | UniRef100_Q65JR3 | Bacillus licheniformis DSM 13 |
| 1927 | sdaAA L-serine dehydratase (alpha chain) | SdaAA [Bacillus licheniformis DSM 13] | UniRef100_Q65JR2 | Bacillus licheniformis DSM 13 |

| 1928 | recG ATP-dependent DNA helicase | RecG [Bacillus licheniformis DSM 13] | UniRef100_Q65JR1 | Bacillus licheniformis DSM 13 |
|---|---|---|---|---|
| 1929 | fapR Transcription factor FapR | YlpC [Bacillus licheniformis DSM 13] | UniRef100_Q65JR0 | Bacillus licheniformis DSM 13 |
| 1930 | plsX phospholipid biosythesis protein PlsX | Hypothetical protein plsX [Bacillus licheniformis DSM 13] | UniRef100_Q65JQ9 | Bacillus licheniformis DSM 13 |
| 1931 | fabD malonyl CoA-acyl carrier protein transacylase | FabD [Bacillus licheniformis DSM 13] | UniRef100_Q65JQ8 | Bacillus licheniformis DSM 13 |
| 1932 | fabG beta-ketoacyl-acyl carrier protein reductase | FabG [Bacillus licheniformis DSM 13] | UniRef100_Q65JQ7 | Bacillus licheniformis DSM 13 |
| 1933 | acpA acyl carrier protein | AcpA [Bacillus licheniformis DSM 13] | UniRef100_Q65JQ6 | Bacillus licheniformis DSM 13 |
| 1934 | rnc ribonuclease III | Rnc [Bacillus licheniformis DSM 13] | UniRef100_Q65JQ5 | Bacillus licheniformis DSM 13 |
| 1935 | smc chromosome segregation SMC protein homolg | Smc [Bacillus licheniformis DSM 13] | UniRef100_Q65JQ4 | Bacillus licheniformis DSM 13 |
| 1936 | ftsY signal recognition particle (docking protein) | FtsY [Bacillus licheniformis DSM 13] | UniRef100_Q65JQ3 | Bacillus licheniformis DSM 13 |
| 1937 | ylxM Bipartite response regulator, C-terminal effector YlxM | YlxM [Bacillus licheniformis DSM 13] | UniRef100_Q65JQ2 | Bacillus licheniformis DSM 13 |
| 1938 | ffh signal recognition particle-like (SRP) component | Ffh (Signal recognition particle-like (SRP) component) [Bacillus licheniformis DSM 13] | UniRef100_Q65JQ1 | Bacillus licheniformis DSM 13 |
| 1939 | rpsP ribosomal protein S16 (BS17) | RpsP [Bacillus licheniformis DSM 13] | UniRef100_Q65JQ0 | Bacillus licheniformis DSM 13 |
| 1940 | ylqC conserved hypothetical protein YlqC | KH domain, prokaryotic type [Bacillus licheniformis DSM 13] | UniRef100_Q62V54 | Bacillus licheniformis DSM 13 |
| 1941 | ylqD conserved hypothetical protein YlqD | YlqD [Bacillus licheniformis DSM 13] | UniRef100_Q65JP8 | Bacillus licheniformis DSM 13 |
| 1942 | rimM 16S rRNA processing protein | RimM [Bacillus licheniformis DSM 13] | UniRef100_Q65JP7 | Bacillus licheniformis DSM 13 |
| 1943 | trmD tRNA methyltransferase | TrmD [Bacillus licheniformis DSM 13] | UniRef100_Q65JP6 | Bacillus licheniformis DSM 13 |
| 1944 | BLP04728 hypothetical protein | | | |
| 1945 | rplS ribosomal protein L19 | RplS [Bacillus licheniformis DSM 13] | UniRef100_Q65JP5 | Bacillus licheniformis DSM 13 |
| 1946 | BLP04729 hypothetical protein | | | |
| 1947 | ylqF GTP-binding domain protein YlqF | YlqF [Bacillus licheniformis DSM 13] | UniRef100_Q65JP4 | Bacillus licheniformis DSM 13 |
| 1948 | rnhB ribonuclease HII | RnhB [Bacillus licheniformis DSM 13] | UniRef100_Q65JP3 | Bacillus licheniformis DSM 13 |
| 1949 | ylqG conserved hypothetical protein YlqG | Hypothetical protein [Bacillus licheniformis DSM 13] | UniRef100_Q62V47 | Bacillus licheniformis DSM 13 |
| 1950 | ylqH conserved hypothetical protein YlqH | YlqH [Bacillus licheniformis DSM 13] | UniRef100_Q65JP1 | Bacillus licheniformis DSM 13 |
| 1951 | sucC succinyl-CoA synthetase (beta subunit) | SucC [Bacillus licheniformis DSM 13] | UniRef100_Q65JP0 | Bacillus licheniformis DSM 13 |
| 1952 | sucD succinyl-CoA synthetase (alpha subunit) | SucD [Bacillus licheniformis DSM 13] | UniRef100_Q65JN9 | Bacillus licheniformis DSM 13 |
| 1953 | smf DNA processing Smf protein homolog | Smf [Bacillus licheniformis DSM 13] | UniRef100_Q65JN8 | Bacillus licheniformis DSM 13 |
| 1954 | topA DNA topoisomerase I | TopA [Bacillus licheniformis DSM 13] | UniRef100_Q65JN7 | Bacillus licheniformis DSM 13 |
| 1955 | gid glucose-inhibited division protein | Gid [Bacillus licheniformis DSM 13] | UniRef100_Q65JN6 | Bacillus licheniformis DSM 13 |
| 1956 | xerC site-specific integrase_recombinase | CodV [Bacillus licheniformis DSM 13] | UniRef100_Q65JN5 | Bacillus licheniformis DSM 13 |
| 1957 | clpQ two-component ATP-dependent protease (N-terminal serine protease) | ClpQ [Bacillus licheniformis DSM 13] | UniRef100_Q65JN4 | Bacillus licheniformis DSM 13 |
| 1958 | clpY two-component ATP-dependent protease (N-terminal serine protease) | ClpY [Bacillus licheniformis DSM 13] | UniRef100_Q65JN3 | Bacillus licheniformis DSM 13 |
| 1959 | codY transcriptional regulator | CodY [Bacillus licheniformis DSM 13] | UniRef100_Q65JN2 | Bacillus licheniformis DSM 13 |
| 1960 | flgB flagellar basal-body rod protein | FlgB [Bacillus licheniformis DSM 13] | UniRef100_Q65JN1 | Bacillus licheniformis DSM 13 |
| 1961 | flgC flagellar basal-body rod protein | FlgC [Bacillus licheniformis DSM 13] | UniRef100_Q65JN0 | Bacillus licheniformis DSM 13 |

| 1962 | fliE flagellar hook-basal body protein | FliE [Bacillus licheniformis DSM 13] | UniRef100_Q65JM9 | Bacillus licheniformis DSM 13 |
|---|---|---|---|---|
| 1963 | fliF flagellar basal-body M-ring protein | FliF [Bacillus licheniformis DSM 13] | UniRef100_Q65JM8 | Bacillus licheniformis DSM 13 |
| 1964 | fliG flagellar motor switch protein | FliG [Bacillus licheniformis DSM 13] | UniRef100_Q65JM7 | Bacillus licheniformis DSM 13 |
| 1965 | fliH flagellar assembly protein | FliH [Bacillus licheniformis DSM 13] | UniRef100_Q65JM6 | Bacillus licheniformis DSM 13 |
| 1966 | fliI flagellar-specific ATP synthase | FliI [Bacillus licheniformis DSM 13] | UniRef100_Q65JM5 | Bacillus licheniformis DSM 13 |
| 1967 | fliJ flagellar protein | FliJ [Bacillus licheniformis DSM 13] | UniRef100_Q65JM4 | Bacillus licheniformis DSM 13 |
| 1968 | ylxF Protein kinase PKN_PRK1, effector, Flagellar motor switch protein FliG-like YlxF | YlxF [Bacillus licheniformis DSM 13] | UniRef100_Q65JM3 | Bacillus licheniformis DSM 13 |
| 1969 | fliK Flagellar protein FliK | Hypothetical protein fliK [Bacillus licheniformis DSM 13] | UniRef100_Q65JM2 | Bacillus licheniformis DSM 13 |
| 1970 | ylxG Flagellar hook capping protein YlxG | YlxG [Bacillus licheniformis DSM 13] | UniRef100_Q65JM1 | Bacillus licheniformis DSM 13 |
| 1971 | flgE flagellar hook protein | FlgE [Bacillus licheniformis DSM 13] | UniRef100_Q65JM0 | Bacillus licheniformis DSM 13 |
| 1972 | BLP01894 hypothetical protein | Hypothetical protein [Bacillus licheniformis DSM 13] | UniRef100_Q65JL9 | Bacillus licheniformis DSM 13 |
| 1973 | fliL flagellar protein | FliL [Bacillus licheniformis DSM 13] | UniRef100_Q65JL8 | Bacillus licheniformis DSM 13 |
| 1974 | fliM flagellar motor switch protein | FliM [Bacillus licheniformis DSM 13] | UniRef100_Q65JL7 | Bacillus licheniformis DSM 13 |
| 1975 | fliY flagellar motor switch protein | FliY [Bacillus licheniformis DSM 13] | UniRef100_Q65JL6 | Bacillus licheniformis DSM 13 |
| 1976 | cheY two-component response regulator | CheY [Bacillus licheniformis DSM 13] | UniRef100_Q65JL5 | Bacillus licheniformis DSM 13 |
| 1977 | fliZ flagellar protein | FliZ [Bacillus licheniformis DSM 13] | UniRef100_Q65JL4 | Bacillus licheniformis DSM 13 |
| 1978 | fliP flagellar protein | FliP [Bacillus licheniformis DSM 13] | UniRef100_Q65JL3 | Bacillus licheniformis DSM 13 |
| 1979 | fliQ Flagellar biosynthetic protein FliQ | FliQ [Bacillus licheniformis DSM 13] | UniRef100_Q65JL2 | Bacillus licheniformis DSM 13 |
| 1980 | fliR flagellar protein | FliR [Bacillus licheniformis DSM 13] | UniRef100_Q65JL1 | Bacillus licheniformis DSM 13 |
| 1981 | flhB flagella-associated protein | FlhB [Bacillus licheniformis DSM 13] | UniRef100_Q65JL0 | Bacillus licheniformis DSM 13 |
| 1982 | flhA flagella-associated protein | FlhA [Bacillus licheniformis DSM 13] | UniRef100_Q65JK9 | Bacillus licheniformis DSM 13 |
| 1983 | flhF flagella-associated protein | FlhF [Bacillus licheniformis DSM 13] | UniRef100_Q65JK8 | Bacillus licheniformis DSM 13 |
| 1984 | ylxH conserved hypothetical ATPas YlxH | YlxH [Bacillus licheniformis DSM 13] | UniRef100_Q65JK7 | Bacillus licheniformis DSM 13 |
| 1985 | cheB methyl-accepting chemotaxis proteins (MCP)-glutamate methylesterase and two-component response regulator-like protein CheB | CheB (Methyl-accepting chemotaxis proteins (MCP)-glutamate methylesterase and two-component response regulator-like) [Bacillus licheniformis DSM 13] | UniRef100_Q65JK6 | Bacillus licheniformis DSM 13 |
| 1986 | cheA two-component sensor histidine kinase CheA | CheA [Bacillus licheniformis DSM 13] | UniRef100_Q65JK5 | Bacillus licheniformis DSM 13 |
| 1987 | cheW CheW chemotaxis protein | Hypothetical protein cheW [Bacillus licheniformis DSM 13] | UniRef100_Q65JK4 | Bacillus licheniformis DSM 13 |
| 1988 | cheC CheC chemotaxis protein | Hypothetical protein cheC [Bacillus licheniformis DSM 13] | UniRef100_Q65JK3 | Bacillus licheniformis DSM 13 |
| 1989 | cheD CheD chemotaxis protein | Hypothetical protein cheD [Bacillus licheniformis DSM 13] | UniRef100_Q65JK2 | Bacillus licheniformis DSM 13 |
| 1990 | sigD RNA polymerase sigma-28 factor (sigma-D) | SigD [Bacillus licheniformis DSM 13] | UniRef100_Q65JK1 | Bacillus licheniformis DSM 13 |
| 1991 | ylxL conserved hypothetical protein YlxL | YlxL [Bacillus licheniformis DSM 13] | UniRef100_Q65JK0 | Bacillus licheniformis DSM 13 |
| 1992 | rpsB ribosomal protein S2 | RpsB [Bacillus licheniformis DSM 13] | UniRef100_Q65JJ9 | Bacillus licheniformis DSM 13 |
| 1993 | tsf elongation factor Ts | Translation elongation factor Ts [Bacillus cereus] | UniRef100_Q65JJ8 | Bacillus cereus |
| 1994 | pyrH uridylate kinase | PyrH [Bacillus licheniformis DSM 13] | UniRef100_Q65JJ7 | Bacillus licheniformis DSM 13 |

| 1995 | frr ribosome recycling factor | Frr [Bacillus licheniformis DSM 13] | UniRef100_Q65JJ6 | Bacillus licheniformis DSM 13 |
|---|---|---|---|---|
| 1996 | uppS undecaprenyl pyrophosphate synthetase | UppS [Bacillus licheniformis DSM 13] | UniRef100_Q65JJ5 | Bacillus licheniformis DSM 13 |
| 1997 | cdsA phosphatidate cytidylyltransferase (CDP-diglyceride synthase) | CdsA [Bacillus licheniformis DSM 13] | UniRef100_Q65JJ4 | Bacillus licheniformis DSM 13 |
| 1998 | dxr 1-deoxy-D-xylulose-5-phosphate reductoisomerase | Dxr [Bacillus licheniformis DSM 13] | UniRef100_Q65JJ3 | Bacillus licheniformis DSM 13 |
| 1999 | yluC intramembrane zinc metallopeptidase YluC | YluC [Bacillus licheniformis DSM 13] | UniRef100_Q65JJ2 | Bacillus licheniformis DSM 13 |
| 2000 | proS prolyl-tRNA synthetase | ProS [Bacillus licheniformis DSM 13] | UniRef100_Q65JJ1 | Bacillus licheniformis DSM 13 |
| 2001 | polC DNA polymerase III (alpha subunit) | PolC [Bacillus licheniformis DSM 13] | UniRef100_Q65JJ0 | Bacillus licheniformis DSM 13 |
| 2002 | BLP01924 hypothetical protein | | | |
| 2003 | celA Glycoside Hydrolase Family 9 | Hypothetical protein [Bacillus licheniformis DSM 13] | UniRef100_Q65JI9 | Bacillus licheniformis DSM 13 |
| 2004 | celB Glycoside Hydrolase Family 48 | Hypothetical protein [Bacillus licheniformis DSM 13] | UniRef100_Q65JI8 | Bacillus licheniformis DSM 13 |
| 2005 | celC Glycoside Hydrolase Family 5 | Hypothetical protein [Bacillus licheniformis DSM 13] | UniRef100_Q65JI7 | Bacillus licheniformis DSM 13 |
| 2006 | celD Glycoside Hydrolase family 5 | Hypothetical protein [Bacillus licheniformis DSM 13] | UniRef100_Q65JI6 | Bacillus licheniformis DSM 13 |
| 2007 | BLP01929 hypothetical protein | | | |
| 2008 | BLP01930 conserved hypothetical protein | YlxS [Bacillus licheniformis DSM 13] | UniRef100_Q65JI5 | Bacillus licheniformis DSM 13 |
| 2009 | nusA NusA | Hypothetical protein nusA [Bacillus licheniformis DSM 13] | UniRef100_Q65JI4 | Bacillus licheniformis DSM 13 |
| 2010 | ylxR conserved hypothetical protein YlxR | YlxR [Bacillus licheniformis DSM 13] | UniRef100_Q65JI3 | Bacillus licheniformis DSM 13 |
| 2011 | BLP01932 Ribosomal protein | YlxQ [Bacillus licheniformis DSM 13] | UniRef100_Q65JI2 | Bacillus licheniformis DSM 13 |
| 2012 | infB initiation factor IF-2 | InfB [Bacillus licheniformis DSM 13] | UniRef100_Q65JI1 | Bacillus licheniformis DSM 13 |
| 2013 | BLP01934 hypothetical protein | YlxP [Bacillus licheniformis DSM 13] | UniRef100_Q65JI0 | Bacillus licheniformis DSM 13 |
| 2014 | rbfA ribosome-binding factor A | RbfA [Bacillus licheniformis DSM 13] | UniRef100_Q65JH9 | Bacillus licheniformis DSM 13 |
| 2015 | truB tRNA pseudouridine 55 synthase | TruB [Bacillus licheniformis DSM 13] | UniRef100_Q65JH8 | Bacillus licheniformis DSM 13 |
| 2016 | ribC riboflavin kinase and FAD synthase | RibC [Bacillus licheniformis DSM 13] | UniRef100_Q65JH7 | Bacillus licheniformis DSM 13 |
| 2017 | rpsO 30S ribosomal protein S15. RpsO | RpsO [Bacillus licheniformis DSM 13] | UniRef100_Q65JH6 | Bacillus licheniformis DSM 13 |
| 2018 | pnpA polynucleotide phosphorylase (PNPase) | PnpA [Bacillus licheniformis DSM 13] | UniRef100_Q65JH5 | Bacillus licheniformis DSM 13 |
| 2019 | ylxY Carbohydrate Esterase Family 4 protein, putative polysaccharide deacetylase YlxY | Arginine repressor [Bacillus licheniformis DSM 13] | UniRef100_Q62UX9 | Bacillus licheniformis DSM 13 |
| 2020 | mlpA peptidase | MlpA [Bacillus licheniformis DSM 13] | UniRef100_Q65JH3 | Bacillus licheniformis DSM 13 |
| 2021 | ymxH hypothetical protein YmxH | YmxH [Bacillus licheniformis DSM 13] | UniRef100_Q65JH2 | Bacillus licheniformis DSM 13 |
| 2022 | BLP04730 hypothetical protein | | | |
| 2023 | spoVFA dipicolinate synthase subunit A | SpoVFA [Bacillus licheniformis DSM 13] | UniRef100_Q65JH1 | Bacillus licheniformis DSM 13 |
| 2024 | spoVFB dipicolinate synthase subunit B | SpoVFB [Bacillus licheniformis DSM 13] | UniRef100_Q65JH0 | Bacillus licheniformis DSM 13 |
| 2025 | asd aspartate-semialdehyde dehydrogenase | Asd [Bacillus licheniformis DSM 13] | UniRef100_Q65JG9 | Bacillus licheniformis DSM 13 |
| 2026 | dapG aspartokinase I (alpha and beta subunits) | DapG [Bacillus licheniformis DSM 13] | UniRef100_Q65JG8 | Bacillus licheniformis DSM 13 |
| 2027 | dapA dihydrodipicolinate synthase | Dihydrodipicolinate synthase [Bacillus licheniformis DSM 13] | UniRef100_Q62UX2 | Bacillus licheniformis DSM 13 |
| 2028 | ymfA putative RNase,metallo beta-lactamase superfamily protein,YmfA | YmfA [Bacillus licheniformis DSM 13] | UniRef100_Q65JG6 | Bacillus licheniformis DSM 13 |

| | | | | |
|---|---|---|---|---|
| 2029 | tepA translocation-enhancing protein | TepA [Bacillus licheniformis DSM 13] | UniRef100_Q65JG5 | Bacillus licheniformis DSM 13 |
| 2030 | BLP01949 conserved hypothetical protein | Hypothetical protein [Bacillus licheniformis DSM 13] | UniRef100_Q65JG4 | Bacillus licheniformis DSM 13 |
| 2031 | spoIIIE DNA translocase SpoIIIE | SpoIIIE [Bacillus licheniformis DSM 13] | UniRef100_Q65JG3 | Bacillus licheniformis DSM 13 |
| 2032 | ymfC transcriptional regulator YmfC | YmfC [Bacillus licheniformis DSM 13] | UniRef100_Q65JG2 | Bacillus licheniformis DSM 13 |
| 2033 | BLP01952 putative transporter | Hypothetical protein [Bacillus licheniformis DSM 13] | UniRef100_Q65JG1 | Bacillus licheniformis DSM 13 |
| 2034 | BLP01953 putative peptidase | Hypothetical protein [Bacillus licheniformis DSM 13] | UniRef100_Q65JG0 | Bacillus licheniformis DSM 13 |
| 2035 | ymfH putative metalloendopeptidase YmfH | YmfH [Bacillus licheniformis DSM 13] | UniRef100_Q65JF9 | Bacillus licheniformis DSM 13 |
| 2036 | ymfI putative oxidoreductase | YmfI [Bacillus licheniformis DSM 13] | UniRef100_Q65JF8 | Bacillus licheniformis DSM 13 |
| 2037 | ymfJ conserved hypothetical protein YmfJ | YmfJ [Bacillus licheniformis DSM 13] | UniRef100_Q65JF7 | Bacillus licheniformis DSM 13 |
| 2038 | BLP01957 hypothetical protein | | | |
| 2039 | ymfK hypothetical protein YmfK | Hypothetical protein [Bacillus licheniformis DSM 13] | UniRef100_Q65JF6 | Bacillus licheniformis DSM 13 |
| 2040 | ymfM putative DNA binding protein, transcriptional regulator YmfM | Hypothetical protein [Bacillus licheniformis DSM 13] | UniRef100_Q65JF5 | Bacillus licheniformis DSM 13 |
| 2041 | pgsA phosphatidylglycerophosphate synthase | PgsA [Bacillus licheniformis DSM 13] | UniRef100_Q65JF4 | Bacillus licheniformis DSM 13 |
| 2042 | cinA competence-damage inducible protein | CinA [Bacillus licheniformis DSM 13] | UniRef100_Q65JF3 | Bacillus licheniformis DSM 13 |
| 2043 | recA multifunctional SOS repair regulator | RecA protein [Bacillus licheniformis] | UniRef100_P62211 | Bacillus licheniformis |
| 2044 | BLP01963 hypothetical protein | | | |
| 2045 | ymdA conserved hypothetical protein YmdA | YmdA [Bacillus licheniformis DSM 13] | UniRef100_Q65JF1 | Bacillus licheniformis DSM 13 |
| 2046 | ymdB Conserved hypothetical protein YmdB | YmdB [Bacillus licheniformis DSM 13] | UniRef100_Q65JF0 | Bacillus licheniformis DSM 13 |
| 2047 | spoVS SpoVS | Hypothetical protein spoVS [Bacillus licheniformis DSM 13] | UniRef100_Q65JE9 | Bacillus licheniformis DSM 13 |
| 2048 | BLP01967 hypothetical protein | | | |
| 2049 | BLP01968 putative dipeptidase family protein | Hypothetical protein [Bacillus licheniformis DSM 13] | UniRef100_Q65JE8 | Bacillus licheniformis DSM 13 |
| 2050 | tdh threonine 3-dehydrogenase | Tdh [Bacillus licheniformis DSM 13] | UniRef100_Q65JE7 | Bacillus licheniformis DSM 13 |
| 2051 | kbl 2-amino-3-ketobutyrate CoA ligase (glycine acetyl transferase) | Kbl [Bacillus licheniformis DSM 13] | UniRef100_Q65JE6 | Bacillus licheniformis DSM 13 |
| 2052 | ymcB conserved hypothetical protein YmcB | YmcB [Bacillus licheniformis DSM 13] | UniRef100_Q65JE5 | Bacillus licheniformis DSM 13 |
| 2053 | ymcA conserved hypothetical protein YmcA | YmcA [Bacillus licheniformis DSM 13] | UniRef100_Q65JE4 | Bacillus licheniformis DSM 13 |
| 2054 | cotE morphogenic protein | CotE [Bacillus licheniformis DSM 13] | UniRef100_Q65JE3 | Bacillus licheniformis DSM 13 |
| 2055 | mutS MutS | MutS [Bacillus licheniformis DSM 13] | UniRef100_Q65JE2 | Bacillus licheniformis DSM 13 |
| 2056 | mutL MutL | Hypothetical protein mutL [Bacillus licheniformis DSM 13] | UniRef100_Q65JE1 | Bacillus licheniformis DSM 13 |
| 2057 | yjcS conserved protein YjcS | YjcS [Bacillus licheniformis DSM 13] | UniRef100_Q65JE0 | Bacillus licheniformis DSM 13 |
| 2058 | BLP01977 hypothetical protein | Hypothetical protein [Bacillus licheniformis DSM 13] | UniRef100_Q65JD9 | Bacillus licheniformis DSM 13 |
| 2059 | BLP04931 hypothetical protein | Hypothetical protein [Bacillus licheniformis DSM 13] | UniRef100_Q62UU2 | Bacillus licheniformis DSM 13 |
| 2060 | BLP01978 hypothetical protein | Hypothetical protein [Bacillus licheniformis DSM 13] | UniRef100_Q65JD8 | Bacillus licheniformis DSM 13 |
| 2061 | BLP01979 Cobalamin synthesis protein_P47K family protein | Hypothetical protein [Bacillus licheniformis DSM 13] | UniRef100_Q65JD7 | Bacillus licheniformis DSM 13 |
| 2062 | BLP01980 hypothetical protein | NADH-ubiquinone oxidoreductase 51 kDa subunit [Bacillus licheniformis DSM 13] | UniRef100_Q65JD6 | Bacillus licheniformis DSM 13 |

| 2063 | BLP01981 GCN5-related N-acetyltransferase | Hypothetical protein [Bacillus licheniformis DSM 13] | UniRef100_Q65JD5 | Bacillus licheniformis DSM 13 |
|---|---|---|---|---|
| 2064 | BLP01982 hypothetical protein | Hypothetical protein [Bacillus licheniformis DSM 13] | UniRef100_Q65JD4 | Bacillus licheniformis DSM 13 |
| 2065 | BLP01983 conserved hypothetical protein | | | |
| 2066 | yobN YobN | Putative L-amino acid oxidase [Bacillus subtilis] | UniRef100_O34363 | Bacillus subtilis |
| 2067 | BLP01985 hypothetical protein | | | |
| 2068 | yoaK conserved membrane protein YoaK | YoaK [Bacillus licheniformis DSM 13] | UniRef100_Q65JD1 | Bacillus licheniformis DSM 13 |
| 2069 | BLP01987 pH regulator, solute hydrogen antiporter | Hypothetical protein [Bacillus licheniformis DSM 13] | UniRef100_Q65JD0 | Bacillus licheniformis DSM 13 |
| 2070 | BLP01988 hypothetical protein | Tetracycline resistance protein [Bacillus subtilis] | UniRef100_P23054 | Bacillus subtilis |
| 2071 | BLP01989 conserved hypothetical protein | Hypothetical protein [Bacillus licheniformis DSM 13] | UniRef100_Q65JC6 | Bacillus licheniformis DSM 13 |
| 2072 | BLP01990 hypothetical protein | | | |
| 2073 | BLP01991 conserved hypothetical protein | | | |
| 2074 | yqeD conserved hypothetical protein YqeD | YqeD [Bacillus licheniformis DSM 13] | UniRef100_Q65JC4 | Bacillus licheniformis DSM 13 |
| 2075 | BLP01993 penicillin-binding protein | Hypothetical protein [Bacillus licheniformis DSM 13] | UniRef100_Q65JC3 | Bacillus licheniformis DSM 13 |
| 2076 | BLP04731 hypothetical protein | | | |
| 2077 | yjiC Glycosyl transferase Family 1 | YjiC [Bacillus licheniformis DSM 13] | UniRef100_Q65JC2 | Bacillus licheniformis DSM 13 |
| 2078 | yveA Amino acid_polyamine transporter I | YveA [Bacillus licheniformis DSM 13] | UniRef100_Q65JC1 | Bacillus licheniformis DSM 13 |
| 2079 | yraD YraD | Hypothetical protein yraD [Bacillus licheniformis DSM 13] | UniRef100_Q65JC0 | Bacillus licheniformis DSM 13 |
| 2080 | yraE YraE | Hypothetical protein yraG [Bacillus licheniformis DSM 13] | UniRef100_Q62US7 | Bacillus licheniformis DSM 13 |
| 2081 | ydhD Glycoside hydrolase, family 18 | Hypothetical protein [Bacillus licheniformis DSM 13] | UniRef100_Q65JB8 | Bacillus licheniformis DSM 13 |
| 2082 | yezE probable transcriptional regulator (TetR family) | YezE [Bacillus licheniformis DSM 13] | UniRef100_Q65JB7 | Bacillus licheniformis DSM 13 |
| 2083 | BLP02000 hypothetical protein | | | |
| 2084 | yesE conserved protein YesE | Hypothetical protein yesE [Bacillus licheniformis DSM 13] | UniRef100_Q65JB6 | Bacillus licheniformis DSM 13 |
| 2085 | yesF conserved protein YesF | Hypothetical protein yesF [Bacillus licheniformis DSM 13] | UniRef100_Q65JB5 | Bacillus licheniformis DSM 13 |
| 2086 | BLP02003 conserved hypothetical protein | Hypothetical protein [Bacillus licheniformis DSM 13] | UniRef100_Q65JB4 | Bacillus licheniformis DSM 13 |
| 2087 | ymaD conserved protein YmaD | YmaD [Bacillus licheniformis DSM 13] | UniRef100_Q65JB3 | Bacillus licheniformis DSM 13 |
| 2088 | ebrB multidrug resistance protein EbrB | EbrB [Bacillus licheniformis DSM 13] | UniRef100_Q65JB2 | Bacillus licheniformis DSM 13 |
| 2089 | ebrA multidrug resistance protein EbrA | EbrA [Bacillus licheniformis DSM 13] | UniRef100_Q65JB1 | Bacillus licheniformis DSM 13 |
| 2090 | BLP02007 hypothetical protein | Hypothetical protein [Bacillus licheniformis DSM 13] | UniRef100_Q62UR8 | Bacillus licheniformis DSM 13 |
| 2091 | ymaF conserved hypothetical protein YmaF | Hypothetical protein [Bacillus licheniformis DSM 13] | UniRef100_Q62UR7 | Bacillus licheniformis DSM 13 |
| 2092 | miaA tRNA isopentenylpyrophosphate transferase MiaA | MiaA [Bacillus licheniformis DSM 13] | UniRef100_Q65JA9 | Bacillus licheniformis DSM 13 |
| 2093 | hfq host factor-1 protein Hfq | YmaH [Bacillus licheniformis DSM 13] | UniRef100_Q65JA8 | Bacillus licheniformis DSM 13 |
| 2094 | BLP04732 hypothetical protein | | | |
| 2095 | ymzA conserved hypothetical protein YmzA | YmzA [Bacillus licheniformis DSM 13] | UniRef100_Q65JA7 | Bacillus licheniformis DSM 13 |
| 2096 | nrdI NrdI | YmaA [Bacillus licheniformis DSM 13] | UniRef100_Q65JA6 | Bacillus licheniformis DSM 13 |
| 2097 | nrdE NrdE Ribonucleoside-diphosphate reductase alpha subunit | NrdE [Bacillus licheniformis DSM 13] | UniRef100_Q65JA5 | Bacillus licheniformis DSM 13 |

| 2098 | nrdF NrdF ribonucleoside-diphosphate reductase beta subunit | NrdF [Bacillus licheniformis DSM 13] | UniRef100_Q65JA4 | Bacillus licheniformis DSM 13 |
|------|---|---|---|---|
| 2099 | ymaB conserved protein YmaB | Hypothetical protein ymaB [Bacillus licheniformis DSM 13] | UniRef100_Q65JA3 | Bacillus licheniformis DSM 13 |
| 2100 | BLP02016 putative hydrolase | Hypothetical protein [Bacillus licheniformis DSM 13] | UniRef100_Q65JA2 | Bacillus licheniformis DSM 13 |
| 2101 | nifU Fe-S cluster formation protein | Hypothetical protein [Bacillus licheniformis DSM 13] | UniRef100_Q65JA1 | Bacillus licheniformis DSM 13 |
| 2102 | BLP02018 putative transcriptional regulator | Transcriptional regulator [Bacillus licheniformis DSM 13] | UniRef100_Q62UQ7 | Bacillus licheniformis DSM 13 |
| 2103 | ydhC probable transcriptional regulator YdhC | Hypothetical protein [Bacillus licheniformis DSM 13] | UniRef100_Q65J99 | Bacillus licheniformis DSM 13 |
| 2104 | yjlA conserved membrane protein YjlA | Hypothetical protein yjlA [Bacillus licheniformis DSM 13] | UniRef100_Q65J98 | Bacillus licheniformis DSM 13 |
| 2105 | cwlC N-acetylmuramoyl-L-alanine amidase | CwlC [Bacillus licheniformis DSM 13] | UniRef100_Q65J97 | Bacillus licheniformis DSM 13 |
| 2106 | BLP02022 hypothetical protein | | | |
| 2107 | BLP02023 conserved hypothetical membrane protein | Hypothetical protein [Bacillus licheniformis DSM 13] | UniRef100_Q65J96 | Bacillus licheniformis DSM 13 |
| 2108 | BLP02024 conserved hypothetical protein | Hypothetical protein [Bacillus licheniformis DSM 13] | UniRef100_Q65J95 | Bacillus licheniformis DSM 13 |
| 2109 | BLP04932 conserved hypothetical protein | Hypothetical protein [Bacillus licheniformis DSM 13] | UniRef100_Q65J94 | Bacillus licheniformis DSM 13 |
| 2110 | BLP02025 hypothetical protein | Hypothetical protein [Bacillus licheniformis DSM 13] | UniRef100_Q62UQ1 | Bacillus licheniformis DSM 13 |
| 2111 | BLP02026 hypothetical protein | Hypothetical protein [Bacillus licheniformis DSM 13] | UniRef100_Q65J93 | Bacillus licheniformis DSM 13 |
| 2112 | ydgC possible transcriptional regulator YdgC | Hypothetical protein [Bacillus licheniformis DSM 13] | UniRef100_Q65J92 | Bacillus licheniformis DSM 13 |
| 2113 | BLP02028 hypothetical protein | Hypothetical protein [Bacillus licheniformis DSM 13] | UniRef100_Q65J91 | Bacillus licheniformis DSM 13 |
| 2114 | pucI Permease for cytosine_purines, uracil, thiamine, allantoin | Hypothetical protein [Bacillus licheniformis DSM 13] | UniRef100_Q65J90 | Bacillus licheniformis DSM 13 |
| 2115 | BLP02030 Hydantoin utilization protein A | Hypothetical protein [Bacillus licheniformis DSM 13] | UniRef100_Q65J89 | Bacillus licheniformis DSM 13 |
| 2116 | BLP02031 N-methylhydantoinase B | Hypothetical protein [Bacillus licheniformis DSM 13] | UniRef100_Q65J88 | Bacillus licheniformis DSM 13 |
| 2117 | BLP02032 Hydantoin Utilization protein B | Hypothetical protein [Bacillus licheniformis DSM 13] | UniRef100_Q65J87 | Bacillus licheniformis DSM 13 |
| 2118 | BLP02033 hypothetical protein | Hypothetical protein [Bacillus licheniformis DSM 13] | UniRef100_Q65J86 | Bacillus licheniformis DSM 13 |
| 2119 | BLP02034 hypothetical protein | | | |
| 2120 | BLP02035 hypothetical protein | | | |
| 2121 | BLP02036 hypothetical protein | | | |
| 2122 | BLP02037 conserved hypothetical protein | Hypothetical protein [Bacillus licheniformis DSM 13] | UniRef100_Q65J85 | Bacillus licheniformis DSM 13 |
| 2123 | BLP04933 hypothetical protein | Hypothetical protein [Bacillus licheniformis DSM 13] | UniRef100_Q62UP1 | Bacillus licheniformis DSM 13 |
| 2124 | BLP02038 hypothetical protein | | | |
| 2125 | BLP02039 hypothetical protein | Hypothetical protein [Bacillus licheniformis DSM 13] | UniRef100_Q62UP0 | Bacillus licheniformis DSM 13 |
| 2126 | spoVK SpoVK | Hypothetical protein spoVK [Bacillus licheniformis DSM 13] | UniRef100_Q65J83 | Bacillus licheniformis DSM 13 |
| 2127 | ynbA GTP-binding protein YnbA | YnbA [Bacillus licheniformis DSM 13] | UniRef100_Q65J82 | Bacillus licheniformis DSM 13 |
| 2128 | ynbB conserved hypothetical protein YnbB | YnbB [Bacillus licheniformis DSM 13] | UniRef100_Q65J81 | Bacillus licheniformis DSM 13 |
| 2129 | glnR transcriptional regulator | GlnR [Bacillus licheniformis DSM 13] | UniRef100_Q65J80 | Bacillus licheniformis DSM 13 |
| 2130 | glnA glutamine synthetase | GlnA [Bacillus licheniformis DSM 13] | UniRef100_Q65J79 | Bacillus licheniformis DSM 13 |
| 2131 | BLP02045 conserved hypothetical protein | Hypothetical protein [Bacillus licheniformis DSM 13] | UniRef100_Q65J78 | Bacillus licheniformis DSM 13 |
| 2132 | BLP04819 hypothetical protein | Hypothetical protein [Bacillus licheniformis DSM 13] | UniRef100_Q62UN4 | Bacillus licheniformis DSM 13 |

| 2133 | BLP02046 hypothetical protein | Hypothetical protein [Bacillus licheniformis DSM 13] | UniRef100_Q65J77 | Bacillus licheniformis DSM 13 |
|---|---|---|---|---|
| 2134 | BLP02047 hypothetical protein | Hypothetical protein [Bacillus licheniformis DSM 13] | UniRef100_Q65J76 | Bacillus licheniformis DSM 13 |
| 2135 | BLP04862 conserved hypothetical protein | Hypothetical protein [Bacillus licheniformis DSM 13] | UniRef100_Q65J75 | Bacillus licheniformis DSM 13 |
| 2136 | BLP04863 conserved putative histidine triad (HIT) protein | Hypothetical protein [Bacillus licheniformis DSM 13] | UniRef100_Q65J74 | Bacillus licheniformis DSM 13 |
| 2137 | BLP04820 hypothetical protein | Hypothetical protein [Bacillus licheniformis DSM 13] | UniRef100_Q65J73 | Bacillus licheniformis DSM 13 |
| 2138 | ydjC YdjC | Hypothetical protein [Bacillus licheniformis DSM 13] | UniRef100_Q65J72 | Bacillus licheniformis DSM 13 |
| 2139 | BLP02049 hypothetical protein | Hypothetical protein [Bacillus licheniformis DSM 13] | UniRef100_Q65J71 | Bacillus licheniformis DSM 13 |
| 2140 | BLP02050 hypothetical protein | Hypothetical protein [Bacillus licheniformis DSM 13] | UniRef100_Q65J70 | Bacillus licheniformis DSM 13 |
| 2141 | BLP02051 hypothetical protein | Hypothetical protein [Bacillus licheniformis DSM 13] | UniRef100_Q65J69 | Bacillus licheniformis DSM 13 |
| 2142 | yoaO YoaO | YoaO [Bacillus licheniformis DSM 13] | UniRef100_Q65J68 | Bacillus licheniformis DSM 13 |
| 2143 | BLP02053 hypothetical protein | Hypothetical protein [Bacillus licheniformis DSM 13] | UniRef100_Q65J67 | Bacillus licheniformis DSM 13 |
| 2144 | BLP02054 conserved hypothetical | Hypothetical protein [Bacillus licheniformis DSM 13] | UniRef100_Q65J66 | Bacillus licheniformis DSM 13 |
| 2145 | BLP04934 hypothetical protein | Hypothetical protein [Bacillus licheniformis DSM 13] | UniRef100_Q65J65 | Bacillus licheniformis DSM 13 |
| 2146 | BLP02055 hypothetical phage-related protein | Hypothetical protein [Bacillus licheniformis DSM 13] | UniRef100_Q65J64 | Bacillus licheniformis DSM 13 |
| 2147 | BLP02056 hypothetical protein | | | |
| 2148 | BLP02057 hypothetical protein | | | |
| 2149 | yvdT probable transcriptional regulator YvdT | YvdT [Bacillus licheniformis DSM 13] | UniRef100_Q65J63 | Bacillus licheniformis DSM 13 |
| 2150 | yvdS SMR-type multidrug efflux transporter | YvdS [Bacillus licheniformis DSM 13] | UniRef100_Q65J62 | Bacillus licheniformis DSM 13 |
| 2151 | yvdR SMR-type multidrug efflux transporter | YvdR [Bacillus licheniformis DSM 13] | UniRef100_Q65J61 | Bacillus licheniformis DSM 13 |
| 2152 | BLP02061 GCN5-related N-acetyltransferase | Hypothetical protein [Bacillus licheniformis DSM 13] | UniRef100_Q65J60 | Bacillus licheniformis DSM 13 |
| 2153 | BLP02062 conserved hypothetical protein | Hypothetical protein [Bacillus licheniformis DSM 13] | UniRef100_Q65J59 | Bacillus licheniformis DSM 13 |
| 2154 | yoaW YoaW | Hypothetical protein yoaW [Bacillus licheniformis DSM 13] | UniRef100_Q65J58 | Bacillus licheniformis DSM 13 |
| 2155 | BLP02064 hypothetical protein | | | |
| 2156 | ydbDA putative catalase YdbDA | Hypothetical protein [Bacillus licheniformis DSM 13] | UniRef100_Q65J56 | Bacillus licheniformis DSM 13 |
| 2157 | BLP02066 conservd hypothetical protein | Hypothetical protein [Bacillus licheniformis DSM 13] | UniRef100_Q65J55 | Bacillus licheniformis DSM 13 |
| 2158 | BLP02067 GCN5-related N-acetyltransferase | Hypothetical protein [Bacillus licheniformis DSM 13] | UniRef100_Q65J54 | Bacillus licheniformis DSM 13 |
| 2159 | BLP02068 conserved hypothetical protein | Hypothetical protein [Bacillus licheniformis DSM 13] | UniRef100_Q65J53 | Bacillus licheniformis DSM 13 |
| 2160 | BLP02069 putative transcriptional regulator | Hypothetical protein [Bacillus licheniformis DSM 13] | UniRef100_Q65J52 | Bacillus licheniformis DSM 13 |
| 2161 | BLP02070 putative transcriptional regulator | Hypothetical protein [Bacillus licheniformis DSM 13] | UniRef100_Q65J51 | Bacillus licheniformis DSM 13 |
| 2162 | ydfN putative oxidoreductase YdfN | YdfN [Bacillus licheniformis DSM 13] | UniRef100_Q65J50 | Bacillus licheniformis DSM 13 |
| 2163 | ydfO Glyoxalase domain protein YdfO | YdfO [Bacillus licheniformis DSM 13] | UniRef100_Q65J49 | Bacillus licheniformis DSM 13 |
| 2164 | ywrF conserved protein YwrF | YwrF [Bacillus licheniformis DSM 13] | UniRef100_Q65J48 | Bacillus licheniformis DSM 13 |
| 2165 | wrbA Trp repressor binding protein, putative | Hypothetical protein [Bacillus licheniformis DSM 13] | UniRef100_Q65J47 | Bacillus licheniformis DSM 13 |
| 2166 | BLP02075 hypothetical protein | | | |
| 2167 | BLP02076 phage-like protein | Hypothetical protein [Bacillus licheniformis DSM 13] | UniRef100_Q65J46 | Bacillus licheniformis DSM 13 |

| 2168 | BLP02077 conserved ypothetical protein | Hypothetical protein [Bacillus licheniformis DSM 13] | UniRef100_Q65J45 | Bacillus licheniformis DSM 13 |
|---|---|---|---|---|
| 2169 | thyA Thymidylate synthase A | Thymidylate synthase A [Bacillus licheniformis] | UniRef100_Q59212 | Bacillus licheniformis |
| 2170 | nucB nuclease | NucB [Bacillus licheniformis DSM 13] | UniRef100_Q65J43 | Bacillus licheniformis DSM 13 |
| 2171 | BLP02080 hypothetical protein | | | |
| 2172 | lexA transcriptional regulator | LexA [Bacillus licheniformis DSM 13] | UniRef100_Q65J42 | Bacillus licheniformis DSM 13 |
| 2173 | yneA Peptidoglycan-binding protein YneA | YneA [Bacillus licheniformis DSM 13] | UniRef100_Q65J41 | Bacillus licheniformis DSM 13 |
| 2174 | yneB Resolvase, N-terminal domain - putative recombinase YneB | YneB [Bacillus licheniformis DSM 13] | UniRef100_Q65J40 | Bacillus licheniformis DSM 13 |
| 2175 | ynzC conserved protein YnzC | Hypothetical protein ynzC [Bacillus licheniformis DSM 13] | UniRef100_Q65J39 | Bacillus licheniformis DSM 13 |
| 2176 | tkt transketolase | Tkt [Bacillus licheniformis DSM 13] | UniRef100_Q65J38 | Bacillus licheniformis DSM 13 |
| 2177 | yneE conserved hypothetical protein YneE | YneE [Bacillus licheniformis DSM 13] | UniRef100_Q65J37 | Bacillus licheniformis DSM 13 |
| 2178 | yneF conserved hypothetical protein YneF | YneF [Bacillus licheniformis DSM 13] | UniRef100_Q65J36 | Bacillus licheniformis DSM 13 |
| 2179 | ynzD YnzD | YnzD [Bacillus licheniformis DSM 13] | UniRef100_Q65J35 | Bacillus licheniformis DSM 13 |
| 2180 | ccdA integral membrane protein cytochrome biogenesis protein CcdA | CcdA [Bacillus licheniformis DSM 13] | UniRef100_Q65J34 | Bacillus licheniformis DSM 13 |
| 2181 | ccdB putative two-component response regulator | YneI [Bacillus licheniformis DSM 13] | UniRef100_Q65J33 | Bacillus licheniformis DSM 13 |
| 2182 | ccdC conserved membrane protein CcdC | YneJ [Bacillus licheniformis DSM 13] | UniRef100_Q65J32 | Bacillus licheniformis DSM 13 |
| 2183 | yneK conserved protein YneK | Hypothetical protein yneK [Bacillus licheniformis DSM 13] | UniRef100_Q65J31 | Bacillus licheniformis DSM 13 |
| 2184 | cotM spore coat protein (outer) | CotM [Bacillus licheniformis DSM 13] | UniRef100_Q65J30 | Bacillus licheniformis DSM 13 |
| 2185 | sspP small acid-soluble spore protein | SspP [Bacillus licheniformis DSM 13] | UniRef100_Q65J29 | Bacillus licheniformis DSM 13 |
| 2186 | sspO small acid-soluble spore protein | SspO [Bacillus licheniformis DSM 13] | UniRef100_Q65J28 | Bacillus licheniformis DSM 13 |
| 2187 | citB aconitate hydratase (aconitase) | CitB [Bacillus licheniformis DSM 13] | UniRef100_Q65J27 | Bacillus licheniformis DSM 13 |
| 2188 | yneN putative thiol:disulfide interchange protein YneN | YneN [Bacillus licheniformis DSM 13] | UniRef100_Q65J26 | Bacillus licheniformis DSM 13 |
| 2189 | BLP02098 hypothetical protein | Hypothetical protein [Bacillus licheniformis DSM 13] | UniRef100_Q65J25 | Bacillus licheniformis DSM 13 |
| 2190 | sspN small acid-soluble spore protein | SspN [Bacillus licheniformis DSM 13] | UniRef100_Q65J24 | Bacillus licheniformis DSM 13 |
| 2191 | tlp small acid-soluble spore protein (thioredoxin-like protein) | Tlp [Bacillus licheniformis DSM 13] | UniRef100_Q65J23 | Bacillus licheniformis DSM 13 |
| 2192 | yneP putative thioesterase YneP | YneP [Bacillus licheniformis DSM 13] | UniRef100_Q65J22 | Bacillus licheniformis DSM 13 |
| 2193 | yneQ conserved protein YneQ | Hypothetical protein yneQ [Bacillus licheniformis DSM 13] | UniRef100_Q65J21 | Bacillus licheniformis DSM 13 |
| 2194 | BLP02103 hypothetical protein | | | |
| 2195 | BLP02104 conserved hypothetical protein | Hypothetical protein [Bacillus licheniformis DSM 13] | UniRef100_Q65J20 | Bacillus licheniformis DSM 13 |
| 2196 | BLP04733 hypothetical protein | | | |
| 2197 | BLP02105 conserved hypothetical membrane protein | Hypothetical protein [Bacillus licheniformis DSM 13] | UniRef100_Q65J19 | Bacillus licheniformis DSM 13 |
| 2198 | BLP02106 hypothetical protein | | | |
| 2199 | yneR conserved protein YneR | YneR [Bacillus licheniformis DSM 13] | UniRef100_Q65J18 | Bacillus licheniformis DSM 13 |
| 2200 | yneS conserved membrane protein YneS | Hypothetical protein yneS [Bacillus licheniformis DSM 13] | UniRef100_Q65J17 | Bacillus licheniformis DSM 13 |
| 2201 | yneT conserved protein YneT | YneT [Bacillus licheniformis DSM 13] | UniRef100_Q65J16 | Bacillus licheniformis DSM 13 |
| 2202 | parE subunit of DNA topoisomerase IV | ParE [Bacillus licheniformis DSM 13] | UniRef100_Q65J15 | Bacillus licheniformis DSM 13 |

| 2203 | parC subunit of DNA topoisomerase IV | ParC [Bacillus licheniformis DSM 13] | UniRef100_Q65J14 | Bacillus licheniformis DSM 13 |
|---|---|---|---|---|
| 2204 | BLP04734 hypothetical protein | | | |
| 2205 | BLP04735 hypothetical protein | | | |
| 2206 | araR transcriptional regulator (LacI family) AraR | AraR [Bacillus licheniformis DSM 13] | UniRef100_Q65J13 | Bacillus licheniformis DSM 13 |
| 2207 | BLP02113 Sugar kinase, possible xylulose kinase | Hypothetical protein [Bacillus licheniformis DSM 13] | UniRef100_Q65J12 | Bacillus licheniformis DSM 13 |
| 2208 | araDA L-ribulose-5-phosphate 4-epimerase | Hypothetical protein [Bacillus licheniformis DSM 13] | UniRef100_Q65J11 | Bacillus licheniformis DSM 13 |
| 2209 | BLP02115 L-arabinose isomerase | Hypothetical protein [Bacillus licheniformis DSM 13] | UniRef100_Q65J10 | Bacillus licheniformis DSM 13 |
| 2210 | BLP02116 Arabinose transport protein | Hypothetical protein [Bacillus licheniformis DSM 13] | UniRef100_Q65J09 | Bacillus licheniformis DSM 13 |
| 2211 | BLP02117 hypothetical protein | | | |
| 2212 | BLP02118 putative beta-ketoacyl-acyl carrier protein reductase | Hypothetical protein [Bacillus licheniformis DSM 13] | UniRef100_Q65J08 | Bacillus licheniformis DSM 13 |
| 2213 | BLP02019 conserved hypothetical protein | Hypothetical protein [Bacillus licheniformis DSM 13] | UniRef100_Q65J07 | Bacillus licheniformis DSM 13 |
| 2214 | ynfC YfnC | Hypothetical protein ynfC [Bacillus licheniformis DSM 13] | UniRef100_Q65J06 | Bacillus licheniformis DSM 13 |
| 2215 | alsT amino acid carrier protein | AlsT [Bacillus licheniformis DSM 13] | UniRef100_Q65J05 | Bacillus licheniformis DSM 13 |
| 2216 | BLP02122 conserved hypothetical protein | Hypothetical protein [Bacillus licheniformis DSM 13] | UniRef100_Q65J04 | Bacillus licheniformis DSM 13 |
| 2217 | narI nitrate reductase (gamma subunit) | NarI [Bacillus licheniformis DSM 13] | UniRef100_Q65J03 | Bacillus licheniformis DSM 13 |
| 2218 | narJ nitrate reductase (protein J) | NarJ [Bacillus licheniformis DSM 13] | UniRef100_Q65J02 | Bacillus licheniformis DSM 13 |
| 2219 | narH nitrate reductase (beta subunit) | Nitrate reductase beta-subunit [Bacillus licheniformis] | UniRef100_Q65J01 | Bacillus licheniformis |
| 2220 | narG nitrate reductase (alpha subunit) | NarG [Bacillus licheniformis DSM 13] | UniRef100_Q65J00 | Bacillus licheniformis DSM 13 |
| 2221 | BLP02127 hypothetical protein | | | |
| 2222 | BLP02129 conserved hypothetical protein | Hypothetical protein [Bacillus licheniformis DSM 13] | UniRef100_Q65IZ9 | Bacillus licheniformis DSM 13 |
| 2223 | BLP02130 hypothetical protein | Hypothetical protein [Bacillus licheniformis DSM 13] | UniRef100_Q65IZ8 | Bacillus licheniformis DSM 13 |
| 2224 | BLP02131 Coenzyme PQQ synthesis protein, putative | MoaA / nifB / pqqE family [Bacillus licheniformis DSM 13] | UniRef100_Q65IZ7 | Bacillus licheniformis DSM 13 |
| 2225 | BLP02132 hypothetical protein | | | |
| 2226 | arfM probable transcriptional regulator | ArfM [Bacillus licheniformis DSM 13] | UniRef100_Q65IZ6 | Bacillus licheniformis DSM 13 |
| 2227 | ywiC conserved membrane protein, putative carboxy peptidase YwiC | YwiC [Bacillus licheniformis DSM 13] | UniRef100_Q65IZ5 | Bacillus licheniformis DSM 13 |
| 2228 | fnr transcriptional regulator Fnr | FNR protein [Bacillus licheniformis] | UniRef100_O86128 | Bacillus licheniformis |
| 2229 | narK nitrite extrusion protein | NarK [Bacillus licheniformis DSM 13] | UniRef100_Q65IZ3 | Bacillus licheniformis DSM 13 |
| 2230 | BLP04736 hypothetical protein | | | |
| 2231 | BLP02137 conserved hypothetical protein | Hypothetical protein [Bacillus licheniformis DSM 13] | UniRef100_Q65IZ2 | Bacillus licheniformis DSM 13 |
| 2232 | BLP02138 transcriptional regulator Fnr_Crp family | Hypothetical protein [Bacillus licheniformis DSM 13] | UniRef100_Q65IZ1 | Bacillus licheniformis DSM 13 |
| 2233 | BLP02139 Nitric oxide reductase cytochrome b subunit | Hypothetical protein [Bacillus licheniformis DSM 13] | UniRef100_Q65IZ0 | Bacillus licheniformis DSM 13 |
| 2234 | scdA putative ScdA | Hypothetical protein [Bacillus licheniformis DSM 13] | UniRef100_Q65IY9 | Bacillus licheniformis DSM 13 |
| 2235 | fenH FenH | Hypothetical protein yngL [Bacillus licheniformis DSM 13] | UniRef100_Q65IY8 | Bacillus licheniformis DSM 13 |
| 2236 | BLP02142 hypothetical protein | | | |
| 2237 | bglC endo-1,4-beta-glucanase, Glycoside hydrolase | Cel5A [Bacillus licheniformis] | UniRef100_Q7X3S6 | Bacillus licheniformis |

| | | | | |
|---|---|---|---|---|
| | Family 5 | | | |
| 2238 | ynfE putative acetyl transferase YfnE | Hypothetical protein ynfE [Bacillus licheniformis DSM 13] | UniRef100_Q62UE5 | Bacillus licheniformis DSM 13 |
| 2239 | ykkB GCN5-related N-acetyltransferase YkkB | YkkB [Bacillus licheniformis DSM 13] | UniRef100_Q65IY5 | Bacillus licheniformis DSM 13 |
| 2240 | BLP02146 putative transcriptional regulator | | | |
| 2241 | BLP02148 putative transporter | Quinolone resistence NorA protein, major facilitator family transporter [Bacillus thuringiensis] | UniRef100_Q6HN29 | Bacillus thuringiensis |
| 2242 | ywoF Polysaccharide Lyase Family 9,YwoF | YwoF [Bacillus licheniformis DSM 13] | UniRef100_Q65IY0 | Bacillus licheniformis DSM 13 |
| 2243 | brnQ branched-chain amino acid transporter | BrnQ [Bacillus licheniformis DSM 13] | UniRef100_Q65IX9 | Bacillus licheniformis DSM 13 |
| 2244 | adhA NADP-dependent alcohol dehydrogenase | AdhA [Bacillus licheniformis DSM 13] | UniRef100_Q65IX8 | Bacillus licheniformis DSM 13 |
| 2245 | yraB Putative DNA binding | Hypothetical protein [Bacillus licheniformis DSM 13] | UniRef100_Q65IX7 | Bacillus licheniformis DSM 13 |
| 2246 | BLP02153 PTS phosophotransferase | Hypothetical protein [Bacillus licheniformis DSM 13] | UniRef100_Q65IX6 | Bacillus licheniformis DSM 13 |
| 2247 | BLP02154 conserved hypothetical protein | Hypothetical protein [Bacillus licheniformis DSM 13] | UniRef100_Q65IX5 | Bacillus licheniformis DSM 13 |
| 2248 | BLP02155 hypothetical protein | Hypothetical protein [Bacillus licheniformis DSM 13] | UniRef100_Q65IX4 | Bacillus licheniformis DSM 13 |
| 2249 | BLP02156 Enoyl-CoA hydratase_isomerase | Hypothetical protein [Bacillus licheniformis DSM 13] | UniRef100_Q65IX3 | Bacillus licheniformis DSM 13 |
| 2250 | BLP02157 Enoyl-CoA hydratase_isomerase | Hypothetical protein [Bacillus licheniformis DSM 13] | UniRef100_Q65IX2 | Bacillus licheniformis DSM 13 |
| 2251 | mmsA methylmalonate-semialdehyde dehydrogenase MmsA | MmsA [Bacillus licheniformis DSM 13] | UniRef100_Q65IX1 | Bacillus licheniformis DSM 13 |
| 2252 | ykwC 3-hydroxyisobutyrate dehydrogenase | Hypothetical protein [Bacillus licheniformis DSM 13] | UniRef100_Q65IX0 | Bacillus licheniformis DSM 13 |
| 2253 | BLP02160 Acyl-CoA dehydrogenase | Hypothetical protein [Bacillus licheniformis DSM 13] | UniRef100_Q65IW9 | Bacillus licheniformis DSM 13 |
| 2254 | BLP02161 hypothetical protein | | | |
| 2255 | BLP02162 hypothetical protein | | | |
| 2256 | manA mannose-6-phosphate isomerase | ManA [Bacillus licheniformis DSM 13] | UniRef100_Q65IW8 | Bacillus licheniformis DSM 13 |
| 2257 | manP phosphotransferase system (PTS) mannose-specific enzyme IIBCA component | ManP t (Phosphotransferase system (PTS) mannose-specific enzyme IIBCA component) [Bacillus licheniformis DSM 13] | UniRef100_Q65IW7 | Bacillus licheniformis DSM 13 |
| 2258 | BLP04737 hypothetical protein | | | |
| 2259 | BLP04935 hypothetical protein | Hypothetical protein [Bacillus licheniformis DSM 13] | UniRef100_Q65IW6 | Bacillus licheniformis DSM 13 |
| 2260 | BLP02165 hypothetical protein | Hypothetical protein [Bacillus licheniformis DSM 13] | UniRef100_Q62UC5 | Bacillus licheniformis DSM 13 |
| 2261 | BLP04864 conserved hypothetical protein | Hypothetical protein [Bacillus licheniformis DSM 13] | UniRef100_Q62UC4 | Bacillus licheniformis DSM 13 |
| 2262 | manR transcriptional regulator | ManR [Bacillus licheniformis DSM 13] | UniRef100_Q65IW5 | Bacillus licheniformis DSM 13 |
| 2263 | BLP04738 hypothetical protein | | | |
| 2264 | BLP02167 conserved hypothetical protein | Hypothetical protein [Bacillus licheniformis DSM 13] | UniRef100_Q65IW4 | Bacillus licheniformis DSM 13 |
| 2265 | BLP02168 ABC transporter | Hypothetical protein [Bacillus licheniformis DSM 13] | UniRef100_Q65IW3 | Bacillus licheniformis DSM 13 |
| 2266 | BLP02169 putative transcriptional regulator, GntR family | Hypothetical protein [Bacillus licheniformis DSM 13] | UniRef100_Q65IW2 | Bacillus licheniformis DSM 13 |
| 2267 | BLP02170 Short-chain dehydrogenase_reductase SDR | Hypothetical protein [Bacillus licheniformis DSM 13] | UniRef100_Q65IW1 | Bacillus licheniformis DSM 13 |
| 2268 | BLP02171 mannonate dehydratase | D-mannonate hydrolase [Bacillus licheniformis DSM 13] | UniRef100_Q62UB8 | Bacillus licheniformis DSM 13 |
| 2269 | BLP02172 C4-dicarboxylate transport system (Permease large) | Hypothetical protein [Bacillus licheniformis DSM 13] | UniRef100_Q65IV8 | Bacillus licheniformis DSM 13 |

| 2270 | BLP02173 putative C4-dicarboxylate transport system permease small | Hypothetical protein [Bacillus licheniformis DSM 13] | UniRef100_Q65IV7 | Bacillus licheniformis DSM 13 |
|---|---|---|---|---|
| 2271 | dctB possible C4-dicarboxylate binding protein | DctB [Bacillus licheniformis DSM 13] | UniRef100_Q65IV6 | Bacillus licheniformis DSM 13 |
| 2272 | BLP02175 transcriptional regulator | Hypothetical protein [Bacillus licheniformis DSM 13] | UniRef100_Q65IV5 | Bacillus licheniformis DSM 13 |
| 2273 | BLP04739 hypothetical protein | | | |
| 2274 | arsC arsenate reductase | ArsC [Bacillus licheniformis DSM 13] | UniRef100_Q65IV4 | Bacillus licheniformis DSM 13 |
| 2275 | ydfA Arsenical pump membrane protein | YdfA [Bacillus licheniformis DSM 13] | UniRef100_Q65IV3 | Bacillus licheniformis DSM 13 |
| 2276 | ydeT hypothetical DNA-binding protein YdeT | YdeT [Bacillus licheniformis DSM 13] | UniRef100_Q65IV2 | Bacillus licheniformis DSM 13 |
| 2277 | BLP02179 hypothetical protein | | | |
| 2278 | ydeI conserved hypothetical protein YdeI | YdeI [Bacillus licheniformis DSM 13] | UniRef100_Q65IV1 | Bacillus licheniformis DSM 13 |
| 2279 | BLP02181 hypothetical protein | Hypothetical Endonuclease/exonuclease/phosphatase [Bacillus licheniformis DSM 13] | UniRef100_Q62UA8 | Bacillus licheniformis DSM 13 |
| 2280 | glcU hexose transporter GlcU | Hypothetical protein glcU [Bacillus licheniformis DSM 13] | UniRef100_Q65IU9 | Bacillus licheniformis DSM 13 |
| 2281 | yngK conserved protein YngK | Hypothetical protein yngK [Bacillus licheniformis DSM 13] | UniRef100_Q65IU8 | Bacillus licheniformis DSM 13 |
| 2282 | BLP02184 hypothetical protein | | | |
| 2283 | yngD conserved protein YngD | Hypothetical protein yngD [Bacillus licheniformis DSM 13] | UniRef100_Q65IU7 | Bacillus licheniformis DSM 13 |
| 2284 | BLP02186 putative Pyruvate formate-lyase-activating enzyme | Hypothetical protein [Bacillus licheniformis DSM 13] | UniRef100_Q65IU6 | Bacillus licheniformis DSM 13 |
| 2285 | pfl putative formate C-acetyltransferase Pfl | Hypothetical protein [Bacillus licheniformis DSM 13] | UniRef100_Q65IU5 | Bacillus licheniformis DSM 13 |
| 2286 | BLP04740 hypothetical protein | | | |
| 2287 | dacC penicillin-binding protein (D-alanyl-D-alanine carboxypeptidase) | DacC [Bacillus licheniformis DSM 13] | UniRef100_Q65IU4 | Bacillus licheniformis DSM 13 |
| 2288 | BLP04865 conserved hypothetical protein | YetF protein [Bacillus subtilis] | UniRef100_O31533 | Bacillus subtilis |
| 2289 | BLP02189 putative cardiolipin synthetase | Cardiolipin synthetase [Bacillus pseudofirmus] | UniRef100_O66043 | Bacillus pseudofirmus |
| 2290 | BLP02190 conserved hypothetical | Hypothetical Membrane Associated Protein [Bacillus cereus] | UniRef100_Q813S1 | Bacillus cereus |
| 2291 | BLP02191 conserved hypothetical protein | Hypothetical protein [Bacillus cereus] | UniRef100_Q72XX0 | Bacillus cereus |
| 2292 | BLP04866 conserved hypothetical protein | Hypothetical protein [Bacillus cereus] | UniRef100_Q815J7 | Bacillus cereus |
| 2293 | BLP02192 SpoVA-like protein | Stage V sporulation protein AE [Oceanobacillus iheyensis] | UniRef100_Q8ERC6 | Oceanobacillus iheyensis |
| 2294 | BLP02193 SpoVAD-like protein | Stage V sporulation protein AD [Bacillus anthracis] | UniRef100_Q81X67 | Bacillus anthracis |
| 2295 | BLP02194 SpoVAC-like protein | Stage V sporulation protein AC [Bacillus cereus] | UniRef100_Q72XX4 | Bacillus cereus |
| 2296 | BLP02195 conserved hypothetical protein | Lipoprotein, putative [Bacillus cereus] | UniRef100_Q72XX5 | Bacillus cereus |
| 2297 | BLP04867 highly conserved hypothetical protein | Hypothetical protein OB1383 [Oceanobacillus iheyensis] | UniRef100_Q8ERC2 | Oceanobacillus iheyensis |
| 2298 | BLP04868 conserved hypothetical, putative oxidoreductase | UPI000032B913 UniRef100 entry | UniRef100_UPI000032B913 | |
| 2299 | BLP02196 manganese catalase | Mn catalase [Bacillus halodurans] | UniRef100_Q9KDZ2 | Bacillus halodurans |
| 2300 | BLP04821 conserved hypothetical | BH2251 protein [Bacillus halodurans] | UniRef100_Q9KAN6 | Bacillus halodurans |
| 2301 | BLP02197 hypothetical protein | UPI00003CB598 UniRef100 entry | UniRef100_UPI00003CB598 | |

| 2302 | BLP02199 N-acetylmuramoyl-L-alanine amidase | Sporulation specific N-acetylmuramoyl-L-alanine amidase [Oceanobacillus iheyensis] | UniRef100_Q8CX69 | Oceanobacillus iheyensis |
|---|---|---|---|---|
| 2303 | BLP04869 hypothetical protein | | | |
| 2304 | BLP02200 resolvase | Transposon Tn1546 resolvase [Enterococcus faecium] | UniRef100_Q06237 | Enterococcus faecium |
| 2305 | BLP02201 putative transposase | Transposase for transposon Tn1546 [Enterococcus faecium] | UniRef100_Q06238 | Enterococcus faecium |
| 2306 | yjlD Aminotransferase, class IV YjlD | YjlD [Bacillus licheniformis DSM 13] | UniRef100_Q65IU3 | Bacillus licheniformis DSM 13 |
| 2307 | yjlC conserved hypothetical protein YjlC | YjlC [Bacillus licheniformis DSM 13] | UniRef100_Q65IU2 | Bacillus licheniformis DSM 13 |
| 2308 | BLP02207 hypothetical protein | Hypothetical protein [Bacillus licheniformis DSM 13] | UniRef100_Q62U99 | Bacillus licheniformis DSM 13 |
| 2309 | yoeD DNA binding domain, excisionase family protein YoeD | YoeD [Bacillus licheniformis DSM 13] | UniRef100_Q65IU1 | Bacillus licheniformis DSM 13 |
| 2310 | ykzH conserved hypothetical protein YkzH | YkzH [Bacillus licheniformis DSM 13] | UniRef100_Q65IU0 | Bacillus licheniformis DSM 13 |
| 2311 | BLP02210 acetyl-CoA synthetase | Hypothetical protein [Bacillus licheniformis DSM 13] | UniRef100_Q65IT9 | Bacillus licheniformis DSM 13 |
| 2312 | yngE Carboxyl transferase | YngE [Bacillus licheniformis DSM 13] | UniRef100_Q65IT8 | Bacillus licheniformis DSM 13 |
| 2313 | yngF Enoyl-CoA hydratase_isomerase YngF | YngF [Bacillus licheniformis DSM 13] | UniRef100_Q65IT7 | Bacillus licheniformis DSM 13 |
| 2314 | yngG putative hydroxymethylglutaryl-CoA lyase | YngG [Bacillus licheniformis DSM 13] | UniRef100_Q65IT6 | Bacillus licheniformis DSM 13 |
| 2315 | | Hypothetical protein [Bacillus licheniformis DSM 13] | UniRef100_Q65IT5 | Bacillus licheniformis DSM 13 |
| 2316 | yngH Carbamoyl-phosphate synthase L chain, ATP binding | YngH [Bacillus licheniformis DSM 13] | UniRef100_Q65IT4 | Bacillus licheniformis DSM 13 |
| 2317 | yngI AMP-dependent synthetase and ligase | YngI [Bacillus licheniformis DSM 13] | UniRef100_Q65IT3 | Bacillus licheniformis DSM 13 |
| 2318 | yngJ Acyl-CoA dehydrogenase | YngJ [Bacillus licheniformis DSM 13] | UniRef100_Q65IT2 | Bacillus licheniformis DSM 13 |
| 2319 | yrkL putative NAD(P)H oxidoreductase | YrkL (NAD(P)H dehydrogenase) [Bacillus licheniformis DSM 13] | UniRef100_Q65IT1 | Bacillus licheniformis DSM 13 |
| 2320 | ydzF putative transcriptional regulator YdzF | | | |
| 2321 | proAA gamma-glutamyl phosphate reductase | Hypothetical protein [Bacillus licheniformis DSM 13] | UniRef100_Q65IS9 | Bacillus licheniformis DSM 13 |
| 2322 | proJ glutamate 5-kinase | ProJ [Bacillus licheniformis DSM 13] | UniRef100_Q65IS8 | Bacillus licheniformis DSM 13 |
| 2323 | proH pyrroline-5-carboxylate reductase | ProH [Bacillus licheniformis DSM 13] | UniRef100_Q65IS7 | Bacillus licheniformis DSM 13 |
| 2324 | BLP02222 hypothetical protein | | | |
| 2325 | BLP02223 hypothetical protein | | | |
| 2326 | yetF conserved membrane protein YetF | Hypothetical protein yetF [Bacillus licheniformis DSM 13] | UniRef100_Q65IS6 | Bacillus licheniformis DSM 13 |
| 2327 | BLP02225 hypothetical protein | | | |
| 2328 | cysP sulfate permease | Hypothetical protein [Bacillus licheniformis DSM 13] | UniRef100_Q65IS5 | Bacillus licheniformis DSM 13 |
| 2329 | yitB Phosphoadenosine phosphosulfate reductase CysH-type | YitB [Bacillus licheniformis DSM 13] | UniRef100_Q65IS4 | Bacillus licheniformis DSM 13 |
| 2330 | yitD 2R-phospho-3-sulfolactate synthase, ComA | YitD ((2R)-phospho-3-sulfolactate synthase, ComA) [Bacillus licheniformis DSM 13] | UniRef100_Q65IS3 | Bacillus licheniformis DSM 13 |
| 2331 | yitE conserved membrane protein YitE | Hypothetical protein yitE [Bacillus licheniformis DSM 13] | UniRef100_Q65IS2 | Bacillus licheniformis DSM 13 |
| 2332 | yitF YitF | YitF [Bacillus licheniformis DSM 13] | UniRef100_Q65IS1 | Bacillus licheniformis DSM 13 |
| 2333 | yitG Major facilitator superfamily YitG | YitG [Bacillus licheniformis DSM 13] | UniRef100_Q65IS0 | Bacillus licheniformis DSM 13 |
| 2334 | ykoT Glycosyl transferase, family 2 YkoT | YkoT [Bacillus licheniformis DSM 13] | UniRef100_Q65IR9 | Bacillus licheniformis DSM 13 |

| 2335 | ykoR hypothetical protein | YkoS [Bacillus licheniformis DSM 13] | UniRef100_Q65IR8 | Bacillus licheniformis DSM 13 |
|---|---|---|---|---|
| 2336 | BLP02234 hypothetical protein | | | |
| 2337 | BLP02235 hypothetical protein | | | |
| 2338 | gltB glutamate synthase (small subunit) | GltB [Bacillus licheniformis DSM 13] | UniRef100_Q65IR7 | Bacillus licheniformis DSM 13 |
| 2339 | gltA glutamate synthase (large subunit) | GltA [Bacillus licheniformis DSM 13] | UniRef100_Q65IR6 | Bacillus licheniformis DSM 13 |
| 2340 | gltC transcriptional regulator | GltC [Bacillus licheniformis DSM 13] | UniRef100_Q65IR5 | Bacillus licheniformis DSM 13 |
| 2341 | BLP02239 hypothetical protein | | | |
| 2342 | BLP02240 putative electron transporter | Hypothetical protein [Bacillus licheniformis DSM 13] | UniRef100_Q65IR4 | Bacillus licheniformis DSM 13 |
| 2343 | BLP02241 hypothetical protein | Hypothetical protein [Bacillus licheniformis DSM 13] | UniRef100_Q65IR3 | Bacillus licheniformis DSM 13 |
| 2344 | BLP02242 putative transcriptional regulator | Hypothetical protein [Bacillus licheniformis DSM 13] | UniRef100_Q65IR2 | Bacillus licheniformis DSM 13 |
| 2345 | yfiB ABC transporter | YfiB [Bacillus licheniformis DSM 13] | UniRef100_Q65IR1 | Bacillus licheniformis DSM 13 |
| 2346 | yfiC ABC transporter | YfiC [Bacillus licheniformis DSM 13] | UniRef100_Q65IR0 | Bacillus licheniformis DSM 13 |
| 2347 | BLP02245 hypothetical protein | | | |
| 2348 | BLP02246 Cytochrome P450 protein | Hypothetical protein [Bacillus licheniformis DSM 13] | UniRef100_Q65IQ9 | Bacillus licheniformis DSM 13 |
| 2349 | yoxD Short-chain dehydrogenase_reductase SDR YoxD | YoxD [Bacillus licheniformis DSM 13] | UniRef100_Q65IQ8 | Bacillus licheniformis DSM 13 |
| 2350 | ppsI phosphoenolpyruvate synthase | Pps [Bacillus licheniformis DSM 13] | UniRef100_Q65IQ7 | Bacillus licheniformis DSM 13 |
| 2351 | BLP02249 hypothetical protein | | | |
| 2352 | lmrB drug-export protein | LmrB [Bacillus licheniformis DSM 13] | UniRef100_Q65IQ6 | Bacillus licheniformis DSM 13 |
| 2353 | yoaE putative oxidoreductase | YoaE [Bacillus licheniformis DSM 13] | UniRef100_Q65IQ5 | Bacillus licheniformis DSM 13 |
| 2354 | BLP02252 hypothetical protein | | | |
| 2355 | ycgE hypothetical DNA-binding protein | YcgE [Bacillus licheniformis DSM 13] | UniRef100_Q65IQ4 | Bacillus licheniformis DSM 13 |
| 2356 | mdr putative Drug resistance transporter, EmrB_QacA family, Mdr | Drug resistance transporter, EmrB/QacA family [Bacillus cereus] | UniRef100_Q736I5 | Bacillus cereus |
| 2357 | yndE Spore germination protein YndE | YndE [Bacillus licheniformis DSM 13] | UniRef100_Q65IQ2 | Bacillus licheniformis DSM 13 |
| 2358 | yndF putative spore germination protein YndF | YndF [Bacillus licheniformis DSM 13] | UniRef100_Q65IQ1 | Bacillus licheniformis DSM 13 |
| 2359 | BLP02257 Spore germination protein | Hypothetical protein [Bacillus licheniformis DSM 13] | UniRef100_Q65IQ0 | Bacillus licheniformis DSM 13 |
| 2360 | BLP02258 Spore germination protein | Hypothetical protein [Bacillus licheniformis DSM 13] | UniRef100_Q65IP9 | Bacillus licheniformis DSM 13 |
| 2361 | yndD putative spore germination protein YndD | YndD [Bacillus licheniformis DSM 13] | UniRef100_Q65IP8 | Bacillus licheniformis DSM 13 |
| 2362 | BLP02260 hypothetical protein | | | |
| 2363 | expZ ATP-binding transport protein | ExpZ [Bacillus licheniformis DSM 13] | UniRef100_Q65IP7 | Bacillus licheniformis DSM 13 |
| 2364 | BLP02262 hypothetical protein | | | |
| 2365 | tlpB methyl-accepting chemotaxis protein TlpB | TlpB [Bacillus licheniformis DSM 13] | UniRef100_Q65IP6 | Bacillus licheniformis DSM 13 |
| 2366 | csaA molecular chaperone | CsaA [Bacillus licheniformis DSM 13] | UniRef100_Q65IP5 | Bacillus licheniformis DSM 13 |
| 2367 | aprX alkaline serine protease | AprX [Bacillus licheniformis DSM 13] | UniRef100_Q65IP4 | Bacillus licheniformis DSM 13 |
| 2368 | ynzE YnzE | Hypothetical protein ynzE [Bacillus licheniformis DSM 13] | UniRef100_Q65IP3 | Bacillus licheniformis DSM 13 |
| 2369 | BLP02267 putative transcriptional regulator | Hypothetical protein [Bacillus licheniformis DSM 13] | UniRef100_Q65IP2 | Bacillus licheniformis DSM 13 |

| 2370 | yvmB possible transcriptional regulator YvmB | YvmB [Bacillus licheniformis DSM 13] | UniRef100_Q65IP1 | Bacillus licheniformis DSM 13 |
|------|------|------|------|------|
| 2371 | BLP02269 putative hydrolase | Hypothetical protein [Bacillus licheniformis DSM 13] | UniRef100_Q65IP0 | Bacillus licheniformis DSM 13 |
| 2372 | BLP02270 conserved hypothetical protein | Hypothetical protein [Bacillus licheniformis DSM 13] | UniRef100_Q65IN9 | Bacillus licheniformis DSM 13 |
| 2373 | yndJ conserved hypothetical protein YndJ | YndJ [Bacillus licheniformis DSM 13] | UniRef100_Q65IN8 | Bacillus licheniformis DSM 13 |
| 2374 | yndH conserved hypothetical protein YndH | YndH [Bacillus licheniformis DSM 13] | UniRef100_Q65IN7 | Bacillus licheniformis DSM 13 |
| 2375 | yndG conserved hypothetical protein YndG | YndG [Bacillus licheniformis DSM 13] | UniRef100_Q65IN6 | Bacillus licheniformis DSM 13 |
| 2376 | yobS putative transcriptional regulator YobS | YobS [Bacillus licheniformis DSM 13] | UniRef100_Q65IN5 | Bacillus licheniformis DSM 13 |
| 2377 | yobT Beta-lactamase-like protein YobT | YobT [Bacillus licheniformis DSM 13] | UniRef100_Q65IN4 | Bacillus licheniformis DSM 13 |
| 2378 | yobU regulatory protein YobU | YobU [Bacillus licheniformis DSM 13] | UniRef100_Q65IN3 | Bacillus licheniformis DSM 13 |
| 2379 | BLP02277 conserved hypothetical protein | Hypothetical protein [Bacillus licheniformis DSM 13] | UniRef100_Q65IN2 | Bacillus licheniformis DSM 13 |
| 2380 | yobV putative transcriptional regulator YobV | YobV [Bacillus licheniformis DSM 13] | UniRef100_Q65IN1 | Bacillus licheniformis DSM 13 |
| 2381 | BLP02279 hypothetical protein | | | |
| 2382 | csk22 sigma-K-controlled sporulation membrane protein | Hypothetical protein csk22 [Bacillus licheniformis DSM 13] | UniRef100_Q62U37 | Bacillus licheniformis DSM 13 |
| 2383 | yozA Transcriptional regulator, ArsR family, YozA | YozA [Bacillus licheniformis DSM 13] | UniRef100_Q65IM9 | Bacillus licheniformis DSM 13 |
| 2384 | ymaE putative Metallo-beta-lactamase family protein YmaE | YmaE [Bacillus licheniformis DSM 13] | UniRef100_Q65IM8 | Bacillus licheniformis DSM 13 |
| 2385 | BLP02283 hypothetical protein | Hypothetical protein [Bacillus licheniformis DSM 13] | UniRef100_Q62U34 | Bacillus licheniformis DSM 13 |
| 2386 | yfnA Amino acid_polyamine transporter I | YfnA [Bacillus licheniformis DSM 13] | UniRef100_Q65IM7 | Bacillus licheniformis DSM 13 |
| 2387 | yocA putative Glycoside Hydrolase Family 23 YocA | YocA [Bacillus licheniformis DSM 13] | UniRef100_Q65IM6 | Bacillus licheniformis DSM 13 |
| 2388 | BLP04741 hypothetical protein | | | |
| 2389 | BLP02286 Capsular polysaccharide biosynthesis protein E | Hypothetical protein [Bacillus licheniformis DSM 13] | UniRef100_Q65IM5 | Bacillus licheniformis DSM 13 |
| 2390 | BLP02287 hypothetical protein | Hypothetical protein [Bacillus licheniformis DSM 13] | UniRef100_Q65IM4 | Bacillus licheniformis DSM 13 |
| 2391 | BLP02288 hypothetical protein | Hypothetical protein [Bacillus licheniformis DSM 13] | UniRef100_Q65IM3 | Bacillus licheniformis DSM 13 |
| 2392 | BLP02289 putative glycosyl transferase Family 4 | Hypothetical protein [Bacillus licheniformis DSM 13] | UniRef100_Q65IM2 | Bacillus licheniformis DSM 13 |
| 2393 | yozB conserved hypothetical protein YozB | YozB [Bacillus licheniformis DSM 13] | UniRef100_Q65IM1 | Bacillus licheniformis DSM 13 |
| 2394 | BLP02291 conserved hypothetical protein | Hypothetical protein [Bacillus licheniformis DSM 13] | UniRef100_Q62U26 | Bacillus licheniformis DSM 13 |
| 2395 | yocC conserved hypothetical protein YocC | YocC [Bacillus licheniformis DSM 13] | UniRef100_Q65IL9 | Bacillus licheniformis DSM 13 |
| 2396 | BLP02293 Na _solute symporter | Na+/solute symporter [Bacillus licheniformis DSM 13] | UniRef100_Q62U24 | Bacillus licheniformis DSM 13 |
| 2397 | yocH conserved hypothetical protein YocH | YocH [Bacillus licheniformis DSM 13] | UniRef100_Q65IL7 | Bacillus licheniformis DSM 13 |
| 2398 | yocI ATP-dependent DNA helicase YocI | YocI [Bacillus licheniformis DSM 13] | UniRef100_Q65IL6 | Bacillus licheniformis DSM 13 |
| 2399 | BLP02296 conserved hypothetical protein | Hypothetical protein [Bacillus licheniformis DSM 13] | UniRef100_Q65IL5 | Bacillus licheniformis DSM 13 |
| 2400 | BLP02297 hypothetical protein | | | |
| 2401 | yjfB conserved hypothetical protein | YjfB [Bacillus licheniformis DSM 13] | UniRef100_Q65IL4 | Bacillus licheniformis DSM 13 |
| 2402 | yyaQ conserved protein YyaQ | Hypothetical protein yyaQ [Bacillus licheniformis DSM 13] | UniRef100_Q65IL3 | Bacillus licheniformis DSM 13 |
| 2403 | BLP04743 Adenine methyltransferase | | | |
| 2404 | BLP02299 hypothetical protein | Lin1262 protein [Listeria innocua] | UniRef100_Q92CC6 | Listeria innocua |

| 2405 | BLP02300 conserved hypothetical protein | Hypothetical protein [Bacillus licheniformis DSM 13] | UniRef100_Q65IL2 | Bacillus licheniformis DSM 13 |
|---|---|---|---|---|
| 2406 | yjgD hypothetical protein | YjgD [Bacillus licheniformis DSM 13] | UniRef100_Q65IL1 | Bacillus licheniformis DSM 13 |
| 2407 | yjgC iron-sulfur binding domain containing protein | | | |
| 2408 | BLP02303 hypothetical protein | | | |
| 2409 | BLP02304 hypothetical protein | Hypothetical protein [Bacillus licheniformis DSM 13] | UniRef100_Q65IK9 | Bacillus licheniformis DSM 13 |
| 2410 | BLP02305 hypothetical protein | | | |
| 2411 | BLP04870 hypothetical protein | | | |
| 2412 | BLP02306 conserved hypothetical protein | Hypothetical protein [Bacillus licheniformis DSM 13] | UniRef100_Q65IK8 | Bacillus licheniformis DSM 13 |
| 2413 | ypfA conserved hypothetical protein YpfA | YpfA [Bacillus licheniformis DSM 13] | UniRef100_Q65IK7 | Bacillus licheniformis DSM 13 |
| 2414 | BLP04744 hypothetical protein | | | |
| 2415 | BLP02308 hypothetical protein | | | |
| 2416 | BLP04871 conserved hypothetical protein | Hypothetical protein [Bacillus licheniformis DSM 13] | UniRef100_Q62U12 | Bacillus licheniformis DSM 13 |
| 2417 | BLP02309 partial transposase protein | | | |
| 2418 | BLP02310 conserved hypothetical protein | Hypothetical protein [Bacillus licheniformis DSM 13] | UniRef100_Q65IK6 | Bacillus licheniformis DSM 13 |
| 2419 | BLP02311 hypothetical protein | Prefoldin [Bacillus licheniformis DSM 13] | UniRef100_Q62U10 | Bacillus licheniformis DSM 13 |
| 2420 | BLP02312 hypothetical protein | Regulator of the activity of phosphatase RapE [Bacillus licheniformis DSM 13] | UniRef100_Q62U09 | Bacillus licheniformis DSM 13 |
| 2421 | BLP02313 hypothetical protein | Hypothetical protein [Bacillus licheniformis DSM 13] | UniRef100_Q62U08 | Bacillus licheniformis DSM 13 |
| 2422 | yozQ conserved hypothetical protein YozQ | Hypothetical protein [Bacillus licheniformis DSM 13] | UniRef100_Q62U07 | Bacillus licheniformis DSM 13 |
| 2423 | BLP02315 Spore germination B3 GerAC like, C-terminal | YndF [Bacillus licheniformis DSM 13] | UniRef100_Q65IQ1 | Bacillus licheniformis DSM 13 |
| 2424 | BLP02316 hypothetical protein | | | |
| 2425 | BLP04745 hypothetical protein | | | |
| 2426 | BLP02317 hypothetical protein | Hypothetical protein [Bacillus licheniformis DSM 13] | UniRef100_Q62U05 | Bacillus licheniformis DSM 13 |
| 2427 | BLP02318 conserved hypothetical protein | Hypothetical protein [Bacillus licheniformis DSM 13] | UniRef100_Q65IK1 | Bacillus licheniformis DSM 13 |
| 2428 | BLP02319 putative amidohydrolase like protein | Hypothetical protein [Bacillus licheniformis DSM 13] | UniRef100_Q65IK0 | Bacillus licheniformis DSM 13 |
| 2429 | BLP04746 spore coat protein (inner) | CotD [Bacillus licheniformis DSM 13] | UniRef100_Q65IJ9 | Bacillus licheniformis DSM 13 |
| 2430 | BLP02320 hypothetical protein | Hypothetical protein [Bacillus licheniformis DSM 13] | UniRef100_Q65IJ8 | Bacillus licheniformis DSM 13 |
| 2431 | yisY Esterase_lipase_thioesterase family protein YisY | YisY [Bacillus licheniformis DSM 13] | UniRef100_Q65IJ7 | Bacillus licheniformis DSM 13 |
| 2432 | BLP02322 hypothetical protein | YoqH [Bacillus licheniformis DSM 13] | UniRef100_Q65IJ6 | Bacillus licheniformis DSM 13 |
| 2433 | BLP04747 spore coat protein (inner) | Spore coat protein [Bacillus licheniformis DSM 13] | UniRef100_Q62TZ9 | Bacillus licheniformis DSM 13 |
| 2434 | BLP02323 Lysine exporter protein | Hypothetical protein [Bacillus licheniformis DSM 13] | UniRef100_Q65IJ5 | Bacillus licheniformis DSM 13 |
| 2435 | BLP04748 hypothetical protein | | | |
| 2436 | BLP02324 30S ribosomal protein S14 | Hypothetical protein [Bacillus licheniformis DSM 13] | UniRef100_Q65IJ4 | Bacillus licheniformis DSM 13 |
| 2437 | mutT mutator protein | MutT [Bacillus licheniformis DSM 13] | UniRef100_Q65IJ3 | Bacillus licheniformis DSM 13 |
| 2438 | ppsII phosphoenolpyruvate synthase | Hypothetical protein [Bacillus licheniformis DSM 13] | UniRef100_Q65IJ2 | Bacillus licheniformis DSM 13 |
| 2439 | BLP04749 hypothetical protein | | | |

| 2440 | BLP02327 putative transcriptional regulator TetR family | TetR [Bacillus licheniformis DSM 13] | UniRef100_Q65IJ1 | Bacillus licheniformis DSM 13 |
|---|---|---|---|---|
| 2441 | BLP02328 hypothetical protein | Hypothetical protein [Bacillus licheniformis DSM 13] | UniRef100_Q62TZ3 | Bacillus licheniformis DSM 13 |
| 2442 | yndM conserved hypothetical protein YndM | YndM [Bacillus licheniformis DSM 13] | UniRef100_Q65IJ0 | Bacillus licheniformis DSM 13 |
| 2443 | yisT conserved protein YisT | DinB [Bacillus licheniformis DSM 13] | UniRef100_Q65II9 | Bacillus licheniformis DSM 13 |
| 2444 | yocJ Putative acyl carrier protein phosphodiesterase, NAD(P)H dehydrogenase YocJ | YocJ (NAD(P)H dehydrogenase) [Bacillus licheniformis DSM 13] | UniRef100_Q65II8 | Bacillus licheniformis DSM 13 |
| 2445 | yocK general stress protein homolog, Zn-finger protein YocK | YocK [Bacillus licheniformis DSM 13] | UniRef100_Q65II7 | Bacillus licheniformis DSM 13 |
| 2446 | BLP02333 hypothetical protein | | | |
| 2447 | yozO conserved hypothetical protein YozO | YozO [Bacillus licheniformis DSM 13] | UniRef100_Q65II6 | Bacillus licheniformis DSM 13 |
| 2448 | yozC conserved protein YozC | YozC [Bacillus licheniformis DSM 13] | UniRef100_Q65II5 | Bacillus licheniformis DSM 13 |
| 2449 | BLP04750 hypothetical protein | | | |
| 2450 | dhaS aldehyde dehydrogenase | DhaS [Bacillus licheniformis DSM 13] | UniRef100_Q65II4 | Bacillus licheniformis DSM 13 |
| 2451 | yjbB conserved membrane protein YjbB, putative transporter | YjbB [Bacillus licheniformis DSM 13] | UniRef100_Q65II3 | Bacillus licheniformis DSM 13 |
| 2452 | BLP02339 GCN5-related N-acetyltransferase | Hypothetical protein [Bacillus licheniformis DSM 13] | UniRef100_Q65II2 | Bacillus licheniformis DSM 13 |
| 2453 | BLP02340 putative deaminase | Hypothetical protein [Bacillus licheniformis DSM 13] | UniRef100_Q65II1 | Bacillus licheniformis DSM 13 |
| 2454 | sqhC SqhC | Squalene-hopene cyclase [Bacillus licheniformis DSM 13] | UniRef100_Q62TY2 | Bacillus licheniformis DSM 13 |
| 2455 | sodF superoxide dismutase | SodF [Bacillus licheniformis DSM 13] | UniRef100_Q65IH9 | Bacillus licheniformis DSM 13 |
| 2456 | yvgO conserved protein YvgO | Hypothetical protein yvgO [Bacillus licheniformis DSM 13] | UniRef100_Q65IH8 | Bacillus licheniformis DSM 13 |
| 2457 | BLP02344 hypothetical protein | | | |
| 2458 | yocR sodium-dependent transporter | YocR [Bacillus licheniformis DSM 13] | UniRef100_Q65IH7 | Bacillus licheniformis DSM 13 |
| 2459 | yocS sodium ion transporter YocS | YocS [Bacillus licheniformis DSM 13] | UniRef100_Q65IH6 | Bacillus licheniformis DSM 13 |
| 2460 | odhB 2-oxoglutarate dehydrogenase complex (dihydrolipoamide transsuccinylase, E2 subunit) | OdhB [Bacillus licheniformis DSM 13] | UniRef100_Q65IH5 | Bacillus licheniformis DSM 13 |
| 2461 | odhA 2-oxoglutarate dehydrogenase (E1 subunit) | OdhA [Bacillus licheniformis DSM 13] | UniRef100_Q65IH4 | Bacillus licheniformis DSM 13 |
| 2462 | yojO von Willebrand factor, type A domain containing protein YojO | YojO [Bacillus licheniformis DSM 13] | UniRef100_Q65IH3 | Bacillus licheniformis DSM 13 |
| 2463 | yojN putative nitric oxide reductase YojN | YojN [Bacillus licheniformis DSM 13] | UniRef100_Q65IH2 | Bacillus licheniformis DSM 13 |
| 2464 | BLP02351 hypothetical protein | | | |
| 2465 | yojM Copper_Zinc superoxide dismutase YojM | YojM [Bacillus licheniformis DSM 13] | UniRef100_Q65IH1 | Bacillus licheniformis DSM 13 |
| 2466 | yojL gamma-D-glutamate-meso-diaminopimelate muropeptidase YojL | YojL [Bacillus licheniformis DSM 13] | UniRef100_Q65IH0 | Bacillus licheniformis DSM 13 |
| 2467 | BLP02354 hypothetical protein | | | |
| 2468 | BLP04751 hypothetical protein | | | |
| 2469 | BLP04752 hypothetical protein | | | |
| 2470 | norM putative multidrug resistance protein | YojI [Bacillus licheniformis DSM 13] | UniRef100_Q65IG9 | Bacillus licheniformis DSM 13 |
| 2471 | BLP02356 conserved protein, Carbohydrate Esterase Family 14 | Hypothetical protein [Bacillus licheniformis DSM 13] | UniRef100_Q65IG8 | Bacillus licheniformis DSM 13 |

| 2472 | BLP02357 hypothetical protein | YojF [Bacillus licheniformis DSM 13] | UniRef100_Q65IG7 | Bacillus licheniformis DSM 13 |
|---|---|---|---|---|
| 2473 | yojE conserved hypothetical protein YojE | Hypothetical protein [Bacillus licheniformis DSM 13] | UniRef100_Q65IG6 | Bacillus licheniformis DSM 13 |
| 2474 | yojD putative chloramphenicol sensitive protein YojD | YojE [Bacillus licheniformis DSM 13] | UniRef100_Q65IG5 | Bacillus licheniformis DSM 13 |
| 2475 | BLP02360 hypothetical protein | YozR [Bacillus licheniformis DSM 13] | UniRef100_Q65IG4 | Bacillus licheniformis DSM 13 |
| 2476 | BLP02361 hypothetical protein | | | |
| 2477 | yoaJ YoaJ | YoaJ [Bacillus licheniformis DSM 13] | UniRef100_Q65IG3 | Bacillus licheniformis DSM 13 |
| 2478 | yojB conserved hypothetical protein YojB | Hypothetical protein [Bacillus licheniformis DSM 13] | UniRef100_Q65IG2 | Bacillus licheniformis DSM 13 |
| 2479 | BLP02364 Short-chain dehydrogenase_reductase protein | Hypothetical protein [Bacillus licheniformis DSM 13] | UniRef100_Q65IG1 | Bacillus licheniformis DSM 13 |
| 2480 | yodB hypothetical DNA-binding protein YodB | Winged helix DNA-binding [Bacillus licheniformis DSM 13] | UniRef100_Q62TW2 | Bacillus licheniformis DSM 13 |
| 2481 | yodC putative Nitroreductase YodC | YodC [Bacillus licheniformis DSM 13] | UniRef100_Q65IF9 | Bacillus licheniformis DSM 13 |
| 2482 | yodD Phospholipase_carboxylesterase family protein | YodD [Bacillus licheniformis DSM 13] | UniRef100_Q65IF8 | Bacillus licheniformis DSM 13 |
| 2483 | BLP02368 hypothetical protein | | | |
| 2484 | BLP02369 conserved hypothetical protein | YhjC [Bacillus licheniformis DSM 13] | UniRef100_Q65IF7 | Bacillus licheniformis DSM 13 |
| 2485 | yodF Na _solute symporter YodF | YodF [Bacillus licheniformis DSM 13] | UniRef100_Q65IF6 | Bacillus licheniformis DSM 13 |
| 2486 | ctpA carboxy-terminal processing protease | CtpA [Bacillus licheniformis DSM 13] | UniRef100_Q65IF3 | Bacillus licheniformis DSM 13 |
| 2487 | yodH SAM dependent methyltransferase YodH | YodH (SAM (And some other nucleotide) binding motif) [Bacillus licheniformis DSM 13] | UniRef100_Q65IF2 | Bacillus licheniformis DSM 13 |
| 2488 | yodI conserved hypothetical protein YodI | YodI [Bacillus licheniformis DSM 13] | UniRef100_Q65IF1 | Bacillus licheniformis DSM 13 |
| 2489 | yodJ putative carboxypeptidase | YodJ [Bacillus licheniformis DSM 13] | UniRef100_Q65IF0 | Bacillus licheniformis DSM 13 |
| 2490 | deoD purine nucleoside phosphorylase | DeoD [Bacillus licheniformis DSM 13] | UniRef100_Q65IE9 | Bacillus licheniformis DSM 13 |
| 2491 | BLP02376 hypothetical protein | | | |
| 2492 | BLP02377 Predicted permease_membrane protein | Hypothetical protein [Bacillus licheniformis DSM 13] | UniRef100_Q65IE8 | Bacillus licheniformis DSM 13 |
| 2493 | BLP02378 conserved hypothetical protein | Hypothetical protein [Bacillus licheniformis DSM 13] | UniRef100_Q65IE7 | Bacillus licheniformis DSM 13 |
| 2494 | BLP02379 conserved hypothetical protein | Hypothetical protein [Bacillus licheniformis DSM 13] | UniRef100_Q62TU8 | Bacillus licheniformis DSM 13 |
| 2495 | yodL conserved hypothetical protein YodL | YodL [Bacillus licheniformis DSM 13] | UniRef100_Q65IE6 | Bacillus licheniformis DSM 13 |
| 2496 | yodM putative acid phosphatase YodM | YodM [Bacillus licheniformis DSM 13] | UniRef100_Q65IE5 | Bacillus licheniformis DSM 13 |
| 2497 | yozD conserved hypothetical protein YozD | YozD [Bacillus licheniformis DSM 13] | UniRef100_Q65IE4 | Bacillus licheniformis DSM 13 |
| 2498 | BLP02383 hypothetical protein | | | |
| 2499 | yodN conserved hypothetical protein YodN | YodN [Bacillus licheniformis DSM 13] | UniRef100_Q65IE3 | Bacillus licheniformis DSM 13 |
| 2500 | BLP02385 hypothetical protein | | | |
| 2501 | yozE conserved hypothetical YozE | Hypothetical protein [Bacillus licheniformis DSM 13] | UniRef100_Q65IE2 | Bacillus licheniformis DSM 13 |
| 2502 | yokU hypothetical protein | Hypothetical protein [Bacillus licheniformis DSM 13] | UniRef100_Q65IE1 | Bacillus licheniformis DSM 13 |
| 2503 | kamA lysine 2,3-aminomutase | KamA [Bacillus licheniformis DSM 13] | UniRef100_Q65IE0 | Bacillus licheniformis DSM 13 |
| 2504 | yodV, yokI phage protein | Hypothetical protein yotM [Bacteriophage SPBc2] | UniRef100_O64195 | Bacteriophage SPBc2 |
| 2505 | yotK phage related sp-beta protein YotK | | | |

| 2506 | BLP02390 hypothetical phage protein | UPI00002F65FC UniRef100 entry | UniRef100_UPI00002F65FC | |
| 2507 | BLP02391 hypothetical phage protein | | | |
| 2508 | BLP02392 hypothetical phage protein | | | |
| 2509 | yotH phage protein YotH | Hypothetical protein yotH [Bacteriophage SPBc2] | UniRef100_O64190 | Bacteriophage SPBc2 |
| 2510 | BLP04754 hypothetical phage protein | | | |
| 2511 | yotB phage protein YotB | Hypothetical protein yotB [Bacteriophage SPBc2] | UniRef100_O64184 | Bacteriophage SPBc2 |
| 2512 | BLP02395 hypothetical protein | | | |
| 2513 | BLP04873 Small acid-soluble spore protein | | | |
| 2514 | BLP02396 hypothetical protein | | | |
| 2515 | BLP02397 hypothetical protein | | | |
| 2516 | yosX phage protein | Hypothetical protein yosX from SPbetac2 prophage [Bacillus subtilis] | UniRef100_O34646 | Bacillus subtilis |
| 2517 | BLP02399 putative Thymidylate synthase | Thymidylate synthase [Streptococcus pneumoniae] | UniRef100_P67050 | Streptococcus pneumoniae |
| 2518 | yosV YosV | Hypothetical protein yosV [Bacteriophage SPBc2] | UniRef100_O64180 | Bacteriophage SPBc2 |
| 2519 | BLP02401 hypothetical protein | | | |
| 2520 | yncF phage protein | YncF protein [Bacillus subtilis] | UniRef100_O31801 | Bacillus subtilis |
| 2521 | BLP02403 hypothetical protein | | | |
| 2522 | yosR thioredoxin phage sp-beta YosR | Thioredoxin [Bacteriophage SPBc2] | UniRef100_O64176 | Bacteriophage SPBc2 |
| 2523 | BLP02405 ribonucleoside-diphosphate reductase (minor subunit) | Ribonucleoside-diphosphate reductase beta chain [Bacillus subtilis] | UniRef100_P50621 | Bacillus subtilis |
| 2524 | BLP02406 DNA-methyltransferase (Cytosine-specific) | DNA-methyltransferase [Clostridium acetobutylicum] | UniRef100_Q97JQ1 | Clostridium acetobutylicum |
| 2525 | BLP02407 hypothetical protein | | | |
| 2526 | BLP02408 HNH endonuclease | Endonuclease [Bacteroides thetaiotaomicron] | UniRef100_Q8A660 | Bacteroides thetaiotaomicron |
| 2527 | BLP04874 nrdI-like phage protein | Phage-derived nrdI protein [Bacillus subtilis] | UniRef100_O31876 | Bacillus subtilis |
| 2528 | yosL YosL | Hypothetical protein yosL [Bacteriophage SPBc2] | UniRef100_O64171 | Bacteriophage SPBc2 |
| 2529 | BLP02412 hypothetical protein | | | |
| 2530 | yosI conserved hypothetical YosI | Hypothetical protein yosI [Bacteriophage SPBc2] | UniRef100_O64168 | Bacteriophage SPBc2 |
| 2531 | yosH phage like conserved hyopothetical YosH | Hypothetical protein yosH [Bacteriophage SPBc2] | UniRef100_O64167 | Bacteriophage SPBc2 |
| 2532 | BLP02415 conserved hypothetical protein | UPI0000313F1C UniRef100 entry | UniRef100_UPI0000313F1C | |
| 2533 | BLP04755 hypothetical protein | | | |
| 2534 | BLP02416 hypothetical protein | | | |
| 2535 | BLP02417 phage hypothetical protein | | | |
| 2536 | BLP02418 phage hypothetical protein | | | |
| 2537 | BLP02419 hypothetical protein | | | |
| 2538 | yorZ YorZ | Hypothetical protein yorZ [Bacteriophage SPBc2] | UniRef100_O64159 | Bacteriophage SPBc2 |

| 2539 | yorY YorY | Hypothetical protein yorY [Bacteriophage SPBc2] | UniRef100_O64158 | Bacteriophage SPBc2 |
|---|---|---|---|---|
| 2540 | BLP04875 hypothetical protein | | | |
| 2541 | BLP02422 conserved hyopothetical | | | |
| 2542 | yorQ YorQ | Hypothetical protein yorQ [Bacteriophage SPBc2] | UniRef100_O64151 | Bacteriophage SPBc2 |
| 2543 | BLP02424 hypothetical protein | | | |
| 2544 | yorP YorP | Hypothetical protein yorP [Bacteriophage SPBc2] | UniRef100_O64150 | Bacteriophage SPBc2 |
| 2545 | yorM sp beta phage related protein YorM | YorM protein [Bacteriophage SPBc2] | UniRef100_O64147 | Bacteriophage SPBc2 |
| 2546 | yorL YorL | Putative DNA polymerase [Bacteriophage SPBc2] | UniRef100_O64146 | Bacteriophage SPBc2 |
| 2547 | BLP02427 putative HNH endonuclease | UPI00003CC2BC UniRef100 entry | UniRef100_UPI00003CC2BC | |
| 2548 | yorK Putative replicative DNA helicase | Putative ss-DNA-specific endonuclease [Bacteriophage SPBc2] | UniRef100_O64145 | Bacteriophage SPBc2 |
| 2549 | yorJ spbeta like phage protein YorJ | Hypothetical protein yorJ [Bacteriophage SPBc2] | UniRef100_O64144 | Bacteriophage SPBc2 |
| 2550 | yorI Putative replicative DNA helicase | Putative replicative DNA helicase [Bacteriophage SPBc2] | UniRef100_O64143 | Bacteriophage SPBc2 |
| 2551 | yorH YorH | Hypothetical protein yorH [Bacteriophage SPBc2] | UniRef100_O64142 | Bacteriophage SPBc2 |
| 2552 | yorG ATP_GTP binding phage protein YorG | ATP/GTP binding protein [Bacteriophage SPBc2] | UniRef100_O64141 | Bacteriophage SPBc2 |
| 2553 | yorF conserved hypothetical phage protein YorF | Hypothetical protein yorF [Bacteriophage SPBc2] | UniRef100_O64140 | Bacteriophage SPBc2 |
| 2554 | yorE conserved hypothetical protein YorE | Hypothetical protein yorE [Bacteriophage SPBc2] | UniRef100_O64139 | Bacteriophage SPBc2 |
| 2555 | BLP04758 hypothetical protein | | | |
| 2556 | BLP02435 hypothetical protein | Precursor polypeptide (AA -37 to 1647) precursor [unidentified bacterium] | UniRef100_Q03658 | unidentified bacterium |
| 2557 | BLP02436 hypothetical protein | | | |
| 2558 | BLP04877 hypothetical protein | | | |
| 2559 | BLP02437 hypothetical protein | | | |
| 2560 | BLP04759 hypothetical protein | | | |
| 2561 | BLP02438 hypothetical protein | | | |
| 2562 | BLP02439 hypothetical protein | | | |
| 2563 | BLP02440 phage related protein | Hypothetical protein [Bacillus licheniformis DSM 13] | UniRef100_Q65KR1 | Bacillus licheniformis DSM 13 |
| 2564 | BLP02441 hypothetical protein | | | |
| 2565 | yoqX conserved hypothetical phage protein YoqX | Hypothetical protein yoqX [Bacteriophage SPBc2] | UniRef100_O64132 | Bacteriophage SPBc2 |
| 2566 | yoqV DNA ligase,phage related YoqV | YoqV protein [Bacteriophage SPBc2] | UniRef100_O64130 | Bacteriophage SPBc2 |
| 2567 | BLP02444 hypothetical protein | | | |
| 2568 | BLP02445 hypothetical protein | | | |
| 2569 | BLP02446 hypothetical protein | | | |
| 2570 | BLP02447 hypothetical protein | | | |
| 2571 | BLP04760 hypothetical protein | | | |
| 2572 | BLP04878 hypothetical protein | | | |

| | | | | |
|---|---|---|---|---|
| 2573 | BLP02448 hypothetical protein | | | |
| 2574 | yoqJ phage YoqJ protein | YoqJ protein [Bacteriophage SPBc2] | UniRef100_O64119 | Bacteriophage SPBc2 |
| 2575 | BLP02450 hypothetical protein | | | |
| 2576 | BLP02451 hypothetical protein | | | |
| 2577 | BLP02452 hypothetical protein | | | |
| 2578 | BLP02453 putative ribonuclease | Exodeoxyribonuclease V [Agrobacterium tumefaciens] | UniRef100_Q8UDU3 | Agrobacterium tumefaciens |
| 2579 | BLP02454 hypothetical protein | | | |
| 2580 | BLP02455 hypothetical protein | | | |
| 2581 | BLP04761 hypothetical protein | | | |
| 2582 | yopR YopR | Hypothetical protein yopR [Bacteriophage SPBc2] | UniRef100_O64101 | Bacteriophage SPBc2 |
| 2583 | yopQ YopQ | Hypothetical protein yopQ [Bacteriophage SPBc2] | UniRef100_O64100 | Bacteriophage SPBc2 |
| 2584 | yopP integrase family phage protein YopP | Hypothetical protein yopP [Bacteriophage SPBc2] | UniRef100_O64099 | Bacteriophage SPBc2 |
| 2585 | yopN SPBc2-like prophage-derived hypothetical protein YopN | | | |
| 2586 | yopM Hypothetical protein YopM | | | |
| 2587 | BLP02461 hypothetical protein | | | |
| 2588 | yopK conserved hypothetical protein YopK | Hypothetical protein yopK [Bacteriophage SPBc2] | UniRef100_O64094 | Bacteriophage SPBc2 |
| 2589 | BLP04879 conserved hypothetical protein | | | |
| 2590 | BLP02463 hypothetical protein | | | |
| 2591 | BLP02464 conserved hypothetical protein | MafB-related protein [Bacillus cereus] | UniRef100_Q81BC9 | Bacillus cereus |
| 2592 | BLP02465 hypothetical protein | | | |
| 2593 | BLP02466 Putative subtilase family protease | ParA [Bacillus subtilis] | UniRef100_Q45510 | Bacillus subtilis |
| 2594 | BLP04880 hypothetical protein | | | |
| 2595 | BLP02467 conserved hypothetical protein | | | |
| 2596 | BLP02468 conserved hypothetical protein | Hypothetical protein [Rhodopirellula baltica] | UniRef100_Q7UT52 | Rhodopirellula baltica |
| 2597 | BLP02469 hypothetical protein | | | |
| 2598 | BLP02471 hypothetical protein | | | |
| 2599 | BLP02472 hypothetical protein | | | |
| 2600 | BLP02473 hypothetical protein | | | |
| 2601 | BLP02474 hypothetical protein | | | |
| 2602 | BLP02475 hypothetical protein | | | |
| 2603 | BLP02476 hypothetical protein | | | |
| 2604 | BLP02477 conserved hypothetical protein | Abi-alpha protein [Staphylococcus epidermidis] | UniRef100_Q8CQJ5 | Staphylococcus epidermidis |
| 2605 | BLP02478 hypothetical protein | | | |
| 2606 | BLP02479 conserved hypothetical protein | UPI00002B934B UniRef100 entry | UniRef100_UPI00002B934B | |
| 2607 | BLP02476 hypothetical protein | | | |

| 2608 | BLP02476 hypothetical protein | | | |
|---|---|---|---|---|
| 2609 | BLP02482 hypothetical protein | | | |
| 2610 | BLP02483 hypothetical protein | | | |
| 2611 | BLP02483 hypothetical protein | | | |
| 2612 | BLP02485 hypothetical protein | | | |
| 2613 | yopB conserved phage hypothetical protein YopB | Hypothetical protein yopB [Bacteriophage SPBc2] | UniRef100_O64085 | Bacteriophage SPBc2 |
| 2614 | BLP02487 hypothetical protein | | | |
| 2615 | BLP02488 hypothetical protein | | | |
| 2616 | BLP02489 hypothetical protein | | | |
| 2617 | BLP04881 hypothetical protein | | | |
| 2618 | BLP04882 hypothetical protein | | | |
| 2619 | BLP02490 hypothetical protein | | | |
| 2620 | BLP02491 hypothetical protein | | | |
| 2621 | BLP02492 hypothetical protein | | | |
| 2622 | BLP02493 hypothetical protein | | | |
| 2623 | BLP02494 hypothetical protein | | | |
| 2624 | BLP02495 hypothetical protein | | | |
| 2625 | BLP02496 hypothetical protein | | | |
| 2626 | BLP02497 hypothetical protein | | | |
| 2627 | yonV hypothetical phage protein YonV | Hypothetical protein yonV [Bacteriophage SPBc2] | UniRef100_O64082 | Bacteriophage SPBc2 |
| 2628 | BLP02499 Putative GIY-YIG endonuclease | Hypothetical protein yonV [Bacteriophage SPBc2] | UniRef100_O64082 | Bacteriophage SPBc2 |
| 2629 | BLP04762 hypothetical protein | | | |
| 2630 | BLP04763 hypothetical protein | | | |
| 2631 | BLP04883 conserved hypothetical phage protein | Orf45 [Bacteriophage SPP1] | UniRef100_Q38074 | Bacteriophage SPP1 |
| 2632 | BLP04884 conserved hypothetical protein | YqgA [Bacillus subtilis] | UniRef100_Q71N51 | Bacillus subtilis |
| 2633 | BLP04885 hypothetical protein | | | |
| 2634 | BLP02500 hypothetical protein | | | |
| 2635 | BLP02501 conserved phage like protein | Hypothetical protein yonO [Bacteriophage SPBc2] | UniRef100_O64076 | Bacteriophage SPBc2 |
| 2636 | BLP02502 conserved phage related protein | | | |
| 2637 | BLP02503 DNA binding protein | Hbs (Non-specific DNA-binding protein HBsu signal recognition particle-like (SRP) component) [Bacillus licheniformis DSM 13] | UniRef100_Q65I20 | Bacillus licheniformis DSM 13 |
| 2638 | BLP02504 hypothetical protein | | | |
| 2639 | BLP02505 conserved hypothetical protein | Hypothetical protein [Bacillus cereus] | UniRef100_Q72XD0 | Bacillus cereus |
| 2640 | yonK YonK | Hypothetical protein yonK [Bacteriophage SPBc2] | UniRef100_O64074 | Bacteriophage SPBc2 |
| 2641 | yonJ phage protein YonJ | Hypothetical protein yonJ [Bacteriophage SPBc2] | UniRef100_O64073 | Bacteriophage SPBc2 |
| 2642 | BLP02508 hypothetical protein | | | |

| 2643 | BLP02509 hypothetical protein | | | |
|---|---|---|---|---|
| 2644 | BLP02510 hypothetical protein | | | |
| 2645 | BLP04886 hypothetical protein | Hypothetical protein [Bacillus thuringiensis] | UniRef100_Q6HM48 | Bacillus thuringiensis |
| 2646 | BLP02511 hypothetical protein | | | |
| 2647 | yonG YonG | Hypothetical protein yonG [Bacteriophage SPBc2] | UniRef100_O64070 | Bacteriophage SPBc2 |
| 2648 | yonF YonF | Hypothetical protein yonF [Bacteriophage SPBc2] | UniRef100_O64069 | Bacteriophage SPBc2 |
| 2649 | yonE YonE | Hypothetical protein yonE [Bacteriophage SPBc2] | UniRef100_O64068 | Bacteriophage SPBc2 |
| 2650 | yonD YonD | Hypothetical protein yonD [Bacteriophage SPBc2] | UniRef100_O64067 | Bacteriophage SPBc2 |
| 2651 | yonC YonC | Hypothetical protein yonC [Bacteriophage SPBc2] | UniRef100_O64066 | Bacteriophage SPBc2 |
| 2652 | yonB YonB | Hypothetical protein yonB [Bacteriophage SPBc2] | UniRef100_O64065 | Bacteriophage SPBc2 |
| 2653 | yonA YonA | Hypothetical protein yonA [Bacteriophage SPBc2] | UniRef100_O64064 | Bacteriophage SPBc2 |
| 2654 | yomZ YomZ | Hypothetical protein yomZ [Bacteriophage SPBc2] | UniRef100_O64063 | Bacteriophage SPBc2 |
| 2655 | yomY YomY | Hypothetical protein yomY [Bacteriophage SPBc2] | UniRef100_O64062 | Bacteriophage SPBc2 |
| 2656 | yomX YomX | Hypothetical protein yomX [Bacteriophage SPBc2] | UniRef100_O64061 | Bacteriophage SPBc2 |
| 2657 | yomW YomW | Hypothetical protein yomW [Bacteriophage SPBc2] | UniRef100_O64060 | Bacteriophage SPBc2 |
| 2658 | yomV YomV | Hypothetical protein yomV [Bacteriophage SPBc2] | UniRef100_O64059 | Bacteriophage SPBc2 |
| 2659 | yomU YomU | Hypothetical protein yomU [Bacteriophage SPBc2] | UniRef100_O64058 | Bacteriophage SPBc2 |
| 2660 | BLP02524 hypothetical phagelike protein | Hypothetical protein [Bacillus licheniformis DSM 13] | UniRef100_Q65ES8 | Bacillus licheniformis DSM 13 |
| 2661 | yomS YomS | YomS protein [Bacteriophage SPBc2] | UniRef100_O64055 | Bacteriophage SPBc2 |
| 2662 | BLP02526 conserved hypothetical phagelike protein | Hypothetical protein [Bacillus licheniformis DSM 13] | UniRef100_Q65ET9 | Bacillus licheniformis DSM 13 |
| 2663 | yomQ conserved hypothetical protein YomQ | YomQ [Bacillus licheniformis DSM 13] | UniRef100_Q65EU0 | Bacillus licheniformis DSM 13 |
| 2664 | yomO conserved hypothetical phage protein YomO | Hypothetical protein yomO [Bacteriophage SPBc2] | UniRef100_O64051 | Bacteriophage SPBc2 |
| 2665 | yomN conserved hypothetical protein YomN | Hypothetical protein [Bacteriophage SPBc2] | UniRef100_O64050 | Bacteriophage SPBc2 |
| 2666 | yomM conserved hypothetical phage protein YomM | Hypothetical protein yomM [Bacteriophage SPBc2] | UniRef100_O64049 | Bacteriophage SPBc2 |
| 2667 | BLP02529 hypothetical protein | | | |
| 2668 | BLP02530 conserved hypothetical protein | Hypothetical protein [Bacillus licheniformis DSM 13] | UniRef100_Q65J73 | Bacillus licheniformis DSM 13 |
| 2669 | BLP04889 conserved hypothetical protein | Hypothetical protein yjcP [Bacillus subtilis] | UniRef100_O31638 | Bacillus subtilis |
| 2670 | yomK conserved hypothetical phage protein YomK | Hypothetical protein yomK [Bacteriophage SPBc2] | UniRef100_O64047 | Bacteriophage SPBc2 |
| 2671 | BLP02532 hypothetical protein | | | |
| 2672 | BLP02533 conserved hypothetical phage protein | Phage protein [Bacillus cereus] | UniRef100_Q81D00 | Bacillus cereus |
| 2673 | yomJ YomJ | D protein [Bacteriophage phi-3T] | UniRef100_Q37974 | Bacteriophage phi-3T |
| 2674 | BLP02535 hypothetical protein | | | |
| 2675 | BLP04890 hypothetical protein | | | |
| 2676 | BLP02536 conserved hypothetical protein | Hypothetical protein Lin1255/Lin1742 [Listeria innocua] | UniRef100_Q926A7 | Listeria innocua |
| 2677 | BLP02537 hypothetical protein | | | |
| 2678 | yomI YomI | Putative transglycosylase [Bacteriophage SPBc2] | UniRef100_O64046 | Bacteriophage SPBc2 |

| 2679 | yomH conserved phage protein YomH | Hypothetical protein yomH [Bacteriophage SPBc2] | UniRef100_O64045 | Bacteriophage SPBc2 |
|---|---|---|---|---|
| 2680 | yomG conserved phage protein YomG | YomG [Bacteriophage SPBc2] | UniRef100_O64044 | Bacteriophage SPBc2 |
| 2681 | yomF conserved phage protein YomF | Hypothetical protein yomF [Bacteriophage SPBc2] | UniRef100_O64043 | Bacteriophage SPBc2 |
| 2682 | BLP02543 conserved hypothetical phage protein | Hypothetical protein [Bacillus licheniformis DSM 13] | UniRef100_Q65ET8 | Bacillus licheniformis DSM 13 |
| 2683 | blyAB N-acetylmuramoyl-L-alanine amidase | N-acetylmuramoyl-L-alanine amidase blyA [Bacillus subtilis] | UniRef100_O31982 | Bacillus subtilis |
| 2684 | BLP02545 hypothetical protein | | | |
| 2685 | BLP04764 putative holin | Holin [Staphylococcus aureus phage phi 13] | UniRef100_Q8SDK0 | Staphylococcus aureus phage phi 13 |
| 2686 | BLP02546 hypothetical protein | | | |
| 2687 | BLP02547 putative response regulator aspartate phosphatase | Hypothetical protein [Bacillus licheniformis DSM 13] | UniRef100_Q65FD0 | Bacillus licheniformis DSM 13 |
| 2688 | BLP02548 Putative macrolide-efflux determinant | Putative macrolide-efflux determinant, YvqJ [Bacillus subtilis] | UniRef100_O32203 | Bacillus subtilis |
| 2689 | BLP02549 hypothetical protein | | | |
| 2690 | BLP02550 conserved hypothetical protein | Hypothetical protein [Bacillus licheniformis DSM 13] | UniRef100_Q65J66 | Bacillus licheniformis DSM 13 |
| 2691 | BLP02551 conserved hypothetical protein | Hypothetical protein yobK [Bacillus subtilis] | UniRef100_O34596 | Bacillus subtilis |
| 2692 | yobK conserved hypothetical protein YobK | Hypothetical protein yobK [Bacillus subtilis] | UniRef100_O34596 | Bacillus subtilis |
| 2693 | BLP04892 conserved hypothetical | TopI homolog [Bacillus subtilis] | UniRef100_O34330 | Bacillus subtilis |
| 2694 | BLP02552 conserved hypothetical protein | Hypothetical protein [Bacillus licheniformis DSM 13] | UniRef100_Q65L46 | Bacillus licheniformis DSM 13 |
| 2695 | BLP02553 putative transposase | YeeF [Bacillus licheniformis DSM 13] | UniRef100_Q65L45 | Bacillus licheniformis DSM 13 |
| 2696 | yokH YokH | Hypothetical protein yokH [Bacteriophage SPBc2] | UniRef100_O64022 | Bacteriophage SPBc2 |
| 2697 | BLP02555 conserved hypothetical protein | | | |
| 2698 | BLP02557 similar to SpoIVCA protein | UPI00003CC102 UniRef100 entry | UniRef100_UPI00003CC102 | |
| 2699 | | KamA [Bacillus licheniformis DSM 13] | UniRef100_Q65IE0 | Bacillus licheniformis DSM 13 |
| 2700 | yodP GCN5-related N-acetyltransferase | YodP [Bacillus licheniformis DSM 13] | UniRef100_Q65ID9 | Bacillus licheniformis DSM 13 |
| 2701 | yodQ Acetylornithine deacetylase YodQ | YodQ [Bacillus licheniformis DSM 13] | UniRef100_Q65ID8 | Bacillus licheniformis DSM 13 |
| 2702 | yodR Coenzyme A transferase YodR | YodR [Bacillus licheniformis DSM 13] | UniRef100_Q65ID7 | Bacillus licheniformis DSM 13 |
| 2703 | yodS Acetate CoA-transferase, subunit A | YodS [Bacillus licheniformis DSM 13] | UniRef100_Q65ID6 | Bacillus licheniformis DSM 13 |
| 2704 | yodT Aminotransferase class-III protein YodT | YodT (Aminotransferase class-III,Blue (Type 1) copper domain) [Bacillus licheniformis DSM 13] | UniRef100_Q65ID5 | Bacillus licheniformis DSM 13 |
| 2705 | BLP02564 Major facilitator superfamily protein | Hypothetical protein [Bacillus licheniformis DSM 13] | UniRef100_Q65ID4 | Bacillus licheniformis DSM 13 |
| 2706 | BLP02565 conserved hypothetical protein | | | |
| 2707 | cgeE CgeE | Hypothetical protein cgeE [Bacillus licheniformis DSM 13] | UniRef100_Q65ID3 | Bacillus licheniformis DSM 13 |
| 2708 | BLP02567 hypothetical protein | | | |
| 2709 | msrB conserved protein MsrB | YppQ [Bacillus licheniformis DSM 13] | UniRef100_Q65ID2 | Bacillus licheniformis DSM 13 |
| 2710 | msrA peptidyl methionine sulfoxide reductase | MsrA [Bacillus licheniformis DSM 13] | UniRef100_Q65ID1 | Bacillus licheniformis DSM 13 |
| 2711 | ypoP hypothetical DNA-binding protein YpoP | YpoP [Bacillus licheniformis DSM 13] | UniRef100_Q65ID0 | Bacillus licheniformis DSM 13 |

| 2712 | BLP02571 conserved hypothetical protein | Hypothetical protein [Bacillus licheniformis DSM 13] | UniRef100_Q65IC9 | Bacillus licheniformis DSM 13 |
|---|---|---|---|---|
| 2713 | yhcK GGDEF-domain containing protein | YhcK [Bacillus licheniformis DSM 13] | UniRef100_Q65IC8 | Bacillus licheniformis DSM 13 |
| 2714 | ypnP putative Multi antimicrobial extrusion protein YpnP | YpnP [Bacillus licheniformis DSM 13] | UniRef100_Q65IC7 | Bacillus licheniformis DSM 13 |
| 2715 | BLP02574 hypothetical protein | Hypothetical protein [Bacillus licheniformis DSM 13] | UniRef100_Q65IC6 | Bacillus licheniformis DSM 13 |
| 2716 | ypmT YpmT | YpmT [Bacillus licheniformis DSM 13] | UniRef100_Q65IC5 | Bacillus licheniformis DSM 13 |
| 2717 | BLP02576 hypothetical protein | | | |
| 2718 | ypmS conserved protein YpmS | Hypothetical protein ypmS [Bacillus licheniformis DSM 13] | UniRef100_Q65IC4 | Bacillus licheniformis DSM 13 |
| 2719 | ypmR conserved protein YpmR | YpmR [Bacillus licheniformis DSM 13] | UniRef100_Q65IC3 | Bacillus licheniformis DSM 13 |
| 2720 | BLP04765 hypothetical protein | | | |
| 2721 | ypmQ conserved hypothetical YpmQ | YpmQ [Bacillus licheniformis DSM 13] | UniRef100_Q65IC2 | Bacillus licheniformis DSM 13 |
| 2722 | degVA DegVA | Hypothetical protein [Bacillus licheniformis DSM 13] | UniRef100_Q65IC1 | Bacillus licheniformis DSM 13 |
| 2723 | BLP02581 hypothetical protein | | | |
| 2724 | ypmP conserved protein YpmP | Hypothetical protein ypmP [Bacillus licheniformis DSM 13] | UniRef100_Q65IC0 | Bacillus licheniformis DSM 13 |
| 2725 | ilvA threonine dehydratase | IlvA [Bacillus licheniformis DSM 13] | UniRef100_Q65IB9 | Bacillus licheniformis DSM 13 |
| 2726 | yplP putative sigma L dependent transcriptional regulator YplP | YplP [Bacillus licheniformis DSM 13] | UniRef100_Q65IB8 | Bacillus licheniformis DSM 13 |
| 2727 | yplQ putative membrane protein YplQ | YplQ [Bacillus licheniformis DSM 13] | UniRef100_Q65IB7 | Bacillus licheniformis DSM 13 |
| 2728 | ypkP conserved hypothetical protein ypkP | YpkP [Bacillus licheniformis DSM 13] | UniRef100_Q65IB6 | Bacillus licheniformis DSM 13 |
| 2729 | dfrA dihydrofolate reductase | DfrA [Bacillus licheniformis DSM 13] | UniRef100_Q65IB5 | Bacillus licheniformis DSM 13 |
| 2730 | BLP02588 hypothetical protein | | | |
| 2731 | ypjQ YpjQ | YpjQ [Bacillus licheniformis DSM 13] | UniRef100_Q65IB4 | Bacillus licheniformis DSM 13 |
| 2732 | ypjP conserved hypothetical protein YpjP | YpjP [Bacillus licheniformis DSM 13] | UniRef100_Q65IB3 | Bacillus licheniformis DSM 13 |
| 2733 | BLP04893 hypothetical protein | | | |
| 2734 | ypiP putative methyltransferase | YpiP [Bacillus licheniformis DSM 13] | UniRef100_Q65IB2 | Bacillus licheniformis DSM 13 |
| 2735 | yphP conserved hypothetical protein YphP | YphP [Bacillus licheniformis DSM 13] | UniRef100_Q65IB1 | Bacillus licheniformis DSM 13 |
| 2736 | ilvD dihydroxy-acid dehydratase | IlvD [Bacillus licheniformis DSM 13] | UniRef100_Q65IB0 | Bacillus licheniformis DSM 13 |
| 2737 | BLP02594 Rhodopsin-like GPCR superfamily protein | YpgR [Bacillus licheniformis DSM 13] | UniRef100_Q65IA9 | Bacillus licheniformis DSM 13 |
| 2738 | BLP02595 Metal-dependent phosphohydrolase, HD domain protein | YpgQ [Bacillus licheniformis DSM 13] | UniRef100_Q65IA8 | Bacillus licheniformis DSM 13 |
| 2739 | bsaA glutathione peroxidase | BsaA [Bacillus licheniformis DSM 13] | UniRef100_Q65IA7 | Bacillus licheniformis DSM 13 |
| 2740 | BLP02597 Formate tetrahydrofolate ligase | Hypothetical Formate-tetrahydrofolate ligase, FTHFS,Formate-tetrahydrofolate ligase, FTHFS [Bacillus licheniformis DSM 13] | UniRef100_Q62TQ7 | Bacillus licheniformis DSM 13 |
| 2741 | metA homoserine O-succinyltransferase | MetA [Bacillus licheniformis DSM 13] | UniRef100_Q65IA5 | Bacillus licheniformis DSM 13 |
| 2742 | ugtP putative Glycosyl Tranferase Family 28 | UgtP [Bacillus licheniformis DSM 13] | UniRef100_Q65IA4 | Bacillus licheniformis DSM 13 |
| 2743 | BLP02600 conserved hypothetical protein | Hypothetical protein [Bacillus licheniformis DSM 13] | UniRef100_Q65IA3 | Bacillus licheniformis DSM 13 |
| 2744 | cspD cold-shock protein | CspD [Bacillus licheniformis DSM 13] | UniRef100_Q65IA2 | Bacillus licheniformis DSM 13 |
| 2745 | degR DegR | Hypothetical protein degR [Bacillus licheniformis DSM 13] | UniRef100_Q65IA1 | Bacillus licheniformis DSM 13 |

| 2746 | ypzA conserved hypothetical protein YpzA | Hypothetical protein [Bacillus licheniformis DSM 13] | UniRef100_Q62TQ1 | Bacillus licheniformis DSM 13 |
|---|---|---|---|---|
| 2747 | ypeQ conserved hypothetical protein YpeQ | Hypothetical protein [Bacillus licheniformis DSM 13] | UniRef100_Q62TQ0 | Bacillus licheniformis DSM 13 |
| 2748 | yqeP conserved putative sugar transporter YqeP | YpeP [Bacillus licheniformis DSM 13] | UniRef100_Q65I98 | Bacillus licheniformis DSM 13 |
| 2749 | ypdP conserved protein YpdP | Hypothetical protein ypdP [Bacillus licheniformis DSM 13] | UniRef100_Q65I97 | Bacillus licheniformis DSM 13 |
| 2750 | rnhA putative ribonuclease H | YpdQ [Bacillus licheniformis DSM 13] | UniRef100_Q65I96 | Bacillus licheniformis DSM 13 |
| 2751 | sspL Small, acid-soluble spore protein L (SASP L) SspL | SspL [Bacillus licheniformis DSM 13] | UniRef100_Q65I95 | Bacillus licheniformis DSM 13 |
| 2752 | ypcP putative 5'-3' exonuclease | YpcP [Bacillus licheniformis DSM 13] | UniRef100_Q65I94 | Bacillus licheniformis DSM 13 |
| 2753 | BLP02610 conserved hypothetical protein | Hypothetical protein [Bacillus licheniformis DSM 13] | UniRef100_Q65I93 | Bacillus licheniformis DSM 13 |
| 2754 | ypbS conserved protein YpbS | Hypothetical protein ypbS [Bacillus licheniformis DSM 13] | UniRef100_Q65I92 | Bacillus licheniformis DSM 13 |
| 2755 | ypbR conserved protein YpbR | Hypothetical protein ypbR [Bacillus licheniformis DSM 13] | UniRef100_Q65I91 | Bacillus licheniformis DSM 13 |
| 2756 | BLP02613 hypothetical protein | Hypothetical protein [Bacillus licheniformis DSM 13] | UniRef100_Q62TP2 | Bacillus licheniformis DSM 13 |
| 2757 | ypbQ Isoprenylcysteine carboxyl methyltransferase | YpbQ [Bacillus licheniformis DSM 13] | UniRef100_Q65I90 | Bacillus licheniformis DSM 13 |
| 2758 | bcsA naringenin-chalcone synthase | BcsA [Bacillus licheniformis DSM 13] | UniRef100_Q65I89 | Bacillus licheniformis DSM 13 |
| 2759 | yokL putative N-acetyltransferase | Hypothetical protein [Bacillus licheniformis DSM 13] | UniRef100_Q65I88 | Bacillus licheniformis DSM 13 |
| 2760 | pbuX xanthine permease | PbuX [Bacillus licheniformis DSM 13] | UniRef100_Q65I87 | Bacillus licheniformis DSM 13 |
| 2761 | xpt xanthine phosphoribosyltransferase | Xpt [Bacillus licheniformis DSM 13] | UniRef100_Q65I86 | Bacillus licheniformis DSM 13 |
| 2762 | BLP02619 hypothetical protein | | | |
| 2763 | ypwA putative metallocarboxypeptidase YpwA | YpwA [Bacillus licheniformis DSM 13] | UniRef100_Q65I85 | Bacillus licheniformis DSM 13 |
| 2764 | ypvA Probable ATP-dependent helicase | Helicase c2 [Bacillus licheniformis DSM 13] | UniRef100_Q62TN5 | Bacillus licheniformis DSM 13 |
| 2765 | BLP02622 hypothetical protein | Hypothetical protein [Bacillus licheniformis DSM 13] | UniRef100_Q65I83 | Bacillus licheniformis DSM 13 |
| 2766 | BLP02623 conserved hypothetical protein | Hypothetical protein [Bacillus licheniformis DSM 13] | UniRef100_Q65I82 | Bacillus licheniformis DSM 13 |
| 2767 | ypsC Putative RNA methylase | YpsC [Bacillus licheniformis DSM 13] | UniRef100_Q65I81 | Bacillus licheniformis DSM 13 |
| 2768 | ypsB conserved hypothetical protein YpsB | YpsB [Bacillus licheniformis DSM 13] | UniRef100_Q65I80 | Bacillus licheniformis DSM 13 |
| 2769 | ypsA conserved hypothetical protein YpsA | YpsA [Bacillus licheniformis DSM 13] | UniRef100_Q65I79 | Bacillus licheniformis DSM 13 |
| 2770 | cotD spore coat protein (inner) | CotD [Bacillus licheniformis DSM 13] | UniRef100_Q65I78 | Bacillus licheniformis DSM 13 |
| 2771 | BLP04894 conserved hypothetical protein | | | |
| 2772 | yprB conserved hypothetical protein YprB | YprB [Bacillus licheniformis DSM 13] | UniRef100_Q65I77 | Bacillus licheniformis DSM 13 |
| 2773 | yprA putative ATP dependent helicase YprA | YprA [Bacillus licheniformis DSM 13] | UniRef100_Q65I76 | Bacillus licheniformis DSM 13 |
| 2774 | ypqE Putative PTS system IIA component YpqE | YpqE [Bacillus licheniformis DSM 13] | UniRef100_Q65I75 | Bacillus licheniformis DSM 13 |
| 2775 | ypqA conserved hypothetical protein YpqA | YpqA [Bacillus licheniformis DSM 13] | UniRef100_Q65I74 | Bacillus licheniformis DSM 13 |
| 2776 | ypqG conserved hypothetical protein YpqG | Hypothetical protein [Bacillus licheniformis DSM 13] | UniRef100_Q65I73 | Bacillus licheniformis DSM 13 |
| 2777 | yppF conserved hypothetical protein YppF | YppF [Bacillus licheniformis DSM 13] | UniRef100_Q65I72 | Bacillus licheniformis DSM 13 |
| 2778 | yppE conserved hypothetical protein YppE | Hypothetical protein [Bacillus licheniformis DSM 13] | UniRef100_Q62TM1 | Bacillus licheniformis DSM 13 |
| 2779 | yppD conserved hypothetical protein YppD | Hypothetical protein [Bacillus licheniformis DSM 13] | UniRef100_Q65I70 | Bacillus licheniformis DSM 13 |
| 2780 | sspM small acid-soluble spore protein | Small acid-soluble spore protein [Bacillus licheniformis DSM 13] | UniRef100_Q62TL9 | Bacillus licheniformis DSM 13 |

| 2781 | yppC conserved hypothetical protein YppC | YppC [Bacillus licheniformis DSM 13] | UniRef100_Q65I69 | Bacillus licheniformis DSM 13 |
|---|---|---|---|---|
| 2782 | recU RecU | Hypothetical protein recU [Bacillus licheniformis DSM 13] | UniRef100_Q65I68 | Bacillus licheniformis DSM 13 |
| 2783 | ponA penicillin-binding proteins,Glycosyl Transferase Family 51 | PonA [Bacillus licheniformis DSM 13] | UniRef100_Q65I67 | Bacillus licheniformis DSM 13 |
| 2784 | ypoC conserved hypothetical protein YpoC | YpoC [Bacillus licheniformis DSM 13] | UniRef100_Q65I66 | Bacillus licheniformis DSM 13 |
| 2785 | nth endonuclease III | Nth [Bacillus licheniformis DSM 13] | UniRef100_Q65I65 | Bacillus licheniformis DSM 13 |
| 2786 | dnaD DnaD | Hypothetical protein dnaD [Bacillus licheniformis DSM 13] | UniRef100_Q65I64 | Bacillus licheniformis DSM 13 |
| 2787 | asnS asparaginyl-tRNA synthetase | AsnS [Bacillus licheniformis DSM 13] | UniRef100_Q65I63 | Bacillus licheniformis DSM 13 |
| 2788 | aspB aspartate aminotransferase | AspB [Bacillus licheniformis DSM 13] | UniRef100_Q65I62 | Bacillus licheniformis DSM 13 |
| 2789 | ypmB conserved protein YpmB | Hypothetical protein ypmB [Bacillus licheniformis DSM 13] | UniRef100_Q65I61 | Bacillus licheniformis DSM 13 |
| 2790 | ypmA conserved protein YpmA | Hypothetical protein ypmA [Bacillus licheniformis DSM 13] | UniRef100_Q65I60 | Bacillus licheniformis DSM 13 |
| 2791 | dinG ATP-dependent helicase | DinG [Bacillus licheniformis DSM 13] | UniRef100_Q65I59 | Bacillus licheniformis DSM 13 |
| 2792 | panD aspartate 1-decarboxylase | PanD [Bacillus licheniformis DSM 13] | UniRef100_Q65I58 | Bacillus licheniformis DSM 13 |
| 2793 | panC pantothenate synthetase | PanC [Bacillus licheniformis DSM 13] | UniRef100_Q65I57 | Bacillus licheniformis DSM 13 |
| 2794 | panB ketopantoate hydroxymethyltransferase | PanB [Bacillus licheniformis DSM 13] | UniRef100_Q65I56 | Bacillus licheniformis DSM 13 |
| 2795 | BLP02651 hypothetical protein | | | |
| 2796 | birA transcriptional regulator and biotin acetyl-CoA-carboxylase synthetase | BirA [Bacillus licheniformis DSM 13] | UniRef100_Q65I55 | Bacillus licheniformis DSM 13 |
| 2797 | cca tRNA nucleotidyltransferase | Cca [Bacillus licheniformis DSM 13] | UniRef100_Q65I54 | Bacillus licheniformis DSM 13 |
| 2798 | ypjH Glycosyl transferase, Family 4, YpjH | YpjH [Bacillus licheniformis DSM 13] | UniRef100_Q65I53 | Bacillus licheniformis DSM 13 |
| 2799 | ypjG putative carbohydrate esterase Family 14 YpjG | LmbE-like protein [Bacillus licheniformis DSM 13] | UniRef100_Q62TK1 | Bacillus licheniformis DSM 13 |
| 2800 | mgsA methylglyoxal synthase | MgsA [Bacillus licheniformis DSM 13] | UniRef100_Q65I51 | Bacillus licheniformis DSM 13 |
| 2801 | dapB dihydrodipicolinate reductase | DapB [Bacillus licheniformis DSM 13] | UniRef100_Q65I50 | Bacillus licheniformis DSM 13 |
| 2802 | ypjD conserved hypothetical protein YpjD | YpjD [Bacillus licheniformis DSM 13] | UniRef100_Q65I49 | Bacillus licheniformis DSM 13 |
| 2803 | ypjC conserved hypothetical protein | YpjC [Bacillus licheniformis DSM 13] | UniRef100_Q65I48 | Bacillus licheniformis DSM 13 |
| 2804 | ypjB conserved hypothetical protein YpjB | YpjB [Bacillus licheniformis DSM 13] | UniRef100_Q65I47 | Bacillus licheniformis DSM 13 |
| 2805 | ypjA conserved hypothetical protein YpjA | Hypothetical protein [Bacillus licheniformis DSM 13] | UniRef100_Q62TJ5 | Bacillus licheniformis DSM 13 |
| 2806 | BLP02662 hypothetical protein | | | |
| 2807 | qcrC menaquinol:cytochrome c oxidoreductase (cytochrome b_c subunit) | QcrC [Bacillus licheniformis DSM 13] | UniRef100_Q65I45 | Bacillus licheniformis DSM 13 |
| 2808 | qcrB menaquinol:cytochrome c oxidoreductase (cytochrome b subunit) | QcrB [Bacillus licheniformis DSM 13] | UniRef100_Q65I44 | Bacillus licheniformis DSM 13 |
| 2809 | qcrA menaquinol:cytochrome c oxidoreductase (iron-sulfur subunit) | QcrA [Bacillus licheniformis DSM 13] | UniRef100_Q65I43 | Bacillus licheniformis DSM 13 |
| 2810 | ypiF conserved hypothetical protein YpiF | YpiF [Bacillus licheniformis DSM 13] | UniRef100_Q65I42 | Bacillus licheniformis DSM 13 |
| 2811 | ypiB conserved hypothetical protein | YpiB [Bacillus licheniformis DSM 13] | UniRef100_Q65I41 | Bacillus licheniformis DSM 13 |
| 2812 | ypiA TPR-repeat containing conserved hypothetical protein | YpiA [Bacillus licheniformis DSM 13] | UniRef100_Q65I40 | Bacillus licheniformis DSM 13 |
| 2813 | BLP02669 hypothetical protein | | | |

| 2814 | aroE 5-enolpyruvoylshikimate-3-phosphate synthase | AroE [Bacillus licheniformis DSM 13] | UniRef100_Q65I39 | Bacillus licheniformis DSM 13 |
|---|---|---|---|---|
| 2815 | tyrA prephenate dehydrogenase | TyrA [Bacillus licheniformis DSM 13] | UniRef100_Q65I38 | Bacillus licheniformis DSM 13 |
| 2816 | hisC histidinol-phosphate aminotransferase and tyrosine_phenylalanine aminotransferase | HisC [Bacillus licheniformis DSM 13] | UniRef100_Q65I37 | Bacillus licheniformis DSM 13 |
| 2817 | trpA tryptophan synthase (alpha subunit) | TrpA [Bacillus licheniformis DSM 13] | UniRef100_Q65I36 | Bacillus licheniformis DSM 13 |
| 2818 | trpB tryptophan synthase (beta subunit) | TrpB [Bacillus licheniformis DSM 13] | UniRef100_Q65I35 | Bacillus licheniformis DSM 13 |
| 2819 | trpF phosphoribosyl anthranilate isomerase | TrpF [Bacillus licheniformis DSM 13] | UniRef100_Q65I34 | Bacillus licheniformis DSM 13 |
| 2820 | trpC indol-3-glycerol phosphate synthase | TrpC [Bacillus licheniformis DSM 13] | UniRef100_Q65I33 | Bacillus licheniformis DSM 13 |
| 2821 | trpD anthranilate phosphoribosyltransferase | TrpD [Bacillus licheniformis DSM 13] | UniRef100_Q65I32 | Bacillus licheniformis DSM 13 |
| 2822 | trpE anthranilate synthase | TrpE [Bacillus licheniformis DSM 13] | UniRef100_Q65I31 | Bacillus licheniformis DSM 13 |
| 2823 | aroH chorismate mutase (isozymes 1 and 2) | AroH [Bacillus licheniformis DSM 13] | UniRef100_Q65I30 | Bacillus licheniformis DSM 13 |
| 2824 | aroB 3-dehydroquinate synthase | AroB [Bacillus licheniformis DSM 13] | UniRef100_Q65I29 | Bacillus licheniformis DSM 13 |
| 2825 | aroF chorismate synthase | AroF [Bacillus licheniformis DSM 13] | UniRef100_Q65I28 | Bacillus licheniformis DSM 13 |
| 2826 | cheR methyl-accepting chemotaxis proteins (MCPs) methyltransferase | CheR (Methyl-accepting chemotaxis proteins (MCPs) methyltransferase) [Bacillus licheniformis DSM 13] | UniRef100_Q65I27 | Bacillus licheniformis DSM 13 |
| 2827 | ndk nucleoside diphosphate kinase | Ndk [Bacillus licheniformis DSM 13] | UniRef100_Q65I26 | Bacillus licheniformis DSM 13 |
| 2828 | hepT heptaprenyl diphosphate synthase component II | HepT [Bacillus licheniformis DSM 13] | UniRef100_Q65I25 | Bacillus licheniformis DSM 13 |
| 2829 | menH methyltransferase | MenH [Bacillus licheniformis DSM 13] | UniRef100_Q65I24 | Bacillus licheniformis DSM 13 |
| 2830 | hepS heptaprenyl diphosphate synthase component I | HepS [Bacillus licheniformis DSM 13] | UniRef100_Q65I23 | Bacillus licheniformis DSM 13 |
| 2831 | mtrB tryptophan operon RNA-binding attenuation protein (TRAP) | MtrB [Bacillus licheniformis DSM 13] | UniRef100_Q65I22 | Bacillus licheniformis DSM 13 |
| 2832 | mtrA GTP cyclohydrolase I | MtrA [Bacillus licheniformis DSM 13] | UniRef100_Q65I21 | Bacillus licheniformis DSM 13 |
| 2833 | hbs non-specific DNA-binding protein HBsu signal recognition particle-like (SRP) component | Hbs (Non-specific DNA-binding protein HBsu signal recognition particle-like (SRP) component) [Bacillus licheniformis DSM 13] | UniRef100_Q65I20 | Bacillus licheniformis DSM 13 |
| 2834 | BLP04766 hypothetical protein | | | |
| 2835 | BLP02690 hypothetical protein | | | |
| 2836 | spoIVA Sporulation Stage IV protein A SpoIVA | Hypothetical protein spoIVA [Bacillus licheniformis DSM 13] | UniRef100_Q65I19 | Bacillus licheniformis DSM 13 |
| 2837 | yphF conserved hypothetical protein YphF | YphF [Bacillus licheniformis DSM 13] | UniRef100_Q65I18 | Bacillus licheniformis DSM 13 |
| 2838 | yphE conserved hypothetical protein YphE | YphE [Bacillus licheniformis DSM 13] | UniRef100_Q65I17 | Bacillus licheniformis DSM 13 |
| 2839 | gpsA NAD(P)H-dependent glycerol-3-phosphate dehydrogenase | GpsA (NAD(P)H-dependent glycerol-3-phosphate dehydrogenase) [Bacillus licheniformis DSM 13] | UniRef100_Q65I16 | Bacillus licheniformis DSM 13 |
| 2840 | engA GTP-binding protein essential for cell growth | YphC [Bacillus licheniformis DSM 13] | UniRef100_Q65I15 | Bacillus licheniformis DSM 13 |
| 2841 | BLP02696 hypothetical protein | | | |
| 2842 | BLP02697 conserved hypothetical protein | Hypothetical protein [Bacillus licheniformis DSM 13] | UniRef100_Q65I14 | Bacillus licheniformis DSM 13 |
| 2843 | seaA hypothetical protein | Hypothetical protein seaA [Bacillus licheniformis DSM 13] | UniRef100_Q65I13 | Bacillus licheniformis DSM 13 |
| 2844 | yphA conserved hypothetical protein YphA | YphA [Bacillus licheniformis DSM 13] | UniRef100_Q65I12 | Bacillus licheniformis DSM 13 |
| 2845 | BLP02700 hypothetical protein | Hypothetical protein [Bacillus licheniformis DSM 13] | UniRef100_Q65I11 | Bacillus licheniformis DSM 13 |

| 2846 | fni FMN_related compound-binding protein | FMN/related compound-binding core [Bacillus licheniformis DSM 13] | UniRef100_Q62TF8 | Bacillus licheniformis DSM 13 |
|------|------|------|------|------|
| 2847 | ypfD 30S ribosomal protein S1 homolog, Nucleic acid-binding protein YpfD | YpfD [Bacillus licheniformis DSM 13] | UniRef100_Q65I09 | Bacillus licheniformis DSM 13 |
| 2848 | cmk cytidylate kinase | Cmk [Bacillus licheniformis DSM 13] | UniRef100_Q65I08 | Bacillus licheniformis DSM 13 |
| 2849 | ypfD conserved hypothetical protein YpfD | YpfB [Bacillus licheniformis DSM 13] | UniRef100_Q65I07 | Bacillus licheniformis DSM 13 |
| 2850 | BLP02705 hypothetical protein | | | |
| 2851 | ybeB putative metallopeptidase YpeB | YpeB [Bacillus licheniformis DSM 13] | UniRef100_Q65I06 | Bacillus licheniformis DSM 13 |
| 2852 | sleB spore cortex-lytic enzyme | SleB [Bacillus licheniformis DSM 13] | UniRef100_Q65I05 | Bacillus licheniformis DSM 13 |
| 2853 | ypdC hypothetical protein | YpdC [Bacillus licheniformis DSM 13] | UniRef100_Q65I04 | Bacillus licheniformis DSM 13 |
| 2854 | BLP02710 L-asparaginase | Hypothetical protein [Bacillus licheniformis DSM 13] | UniRef100_Q65I03 | Bacillus licheniformis DSM 13 |
| 2855 | ypdA thioredoxin oxidoreductase YpdA | YpdA [Bacillus licheniformis DSM 13] | UniRef100_Q65I02 | Bacillus licheniformis DSM 13 |
| 2856 | gudB glutamate dehydrogenase | GudB [Bacillus licheniformis DSM 13] | UniRef100_Q65I01 | Bacillus licheniformis DSM 13 |
| 2857 | mecB Negative regulator of genetic competence | YpbH [Bacillus licheniformis DSM 13] | UniRef100_Q65I00 | Bacillus licheniformis DSM 13 |
| 2858 | ypbG putative hydrolase YpbG | YpbG [Bacillus licheniformis DSM 13] | UniRef100_Q65HZ9 | Bacillus licheniformis DSM 13 |
| 2859 | ypbF conserved hypothetical protein YpbF | YpbF [Bacillus licheniformis DSM 13] | UniRef100_Q65HZ8 | Bacillus licheniformis DSM 13 |
| 2860 | ypbE Peptidoglycan-binding protein YpbE | YpbE [Bacillus licheniformis DSM 13] | UniRef100_Q65HZ7 | Bacillus licheniformis DSM 13 |
| 2861 | ypbD conserved hypothetical protein YpbD | YpbD [Bacillus licheniformis DSM 13] | UniRef100_Q65HZ6 | Bacillus licheniformis DSM 13 |
| 2862 | recQ ATP-dependent DNA helicase RecQ | RecQ [Bacillus licheniformis DSM 13] | UniRef100_Q65HZ5 | Bacillus licheniformis DSM 13 |
| 2863 | ypbB Bipartite response regulator, C-terminal effector YpbB | YpbB [Bacillus licheniformis DSM 13] | UniRef100_Q65HZ4 | Bacillus licheniformis DSM 13 |
| 2864 | fer ferredoxin | Fer [Bacillus licheniformis DSM 13] | UniRef100_Q65HZ3 | Bacillus licheniformis DSM 13 |
| 2865 | BLP02721 putative riboflavin uptake protein YpaA | YpaA [Bacillus licheniformis DSM 13] | UniRef100_Q65HZ2 | Bacillus licheniformis DSM 13 |
| 2866 | BLP02722 hypothetical protein | | | |
| 2867 | BLP02723 hypothetical protein | | | |
| 2868 | serA phosphoglycerate dehydrogenase SerA | SerA [Bacillus licheniformis DSM 13] | UniRef100_Q65HZ1 | Bacillus licheniformis DSM 13 |
| 2869 | BLP02725 putative peptidogycan hydrolase | Hypothetical protein [Bacillus licheniformis DSM 13] | UniRef100_Q65HZ0 | Bacillus licheniformis DSM 13 |
| 2870 | rsiX RsiX | Hypothetical protein rsiX [Bacillus licheniformis DSM 13] | UniRef100_Q65HY9 | Bacillus licheniformis DSM 13 |
| 2871 | sigX RNA polymerase ECF(extractoplasmic function)-type sigma factor (sigma-X) | SigX (RNA polymerase ECF(Extracytoplasmic function)-type sigma factor) [Bacillus licheniformis DSM 13] | UniRef100_Q65HY8 | Bacillus licheniformis DSM 13 |
| 2872 | BLP02728 transcriptional regulator | Membrane-bound protein [Bacillus licheniformis DSM 13] | UniRef100_Q62TD6 | Bacillus licheniformis DSM 13 |
| 2873 | yheN Carbohydrate Esterase Family 4,YheN | Hypothetical protein [Bacillus licheniformis DSM 13] | UniRef100_Q65HY6 | Bacillus licheniformis DSM 13 |
| 2874 | BLP02730 hypothetical protein | | | |
| 2875 | BLP02731 conserved hypothetical protein | Hypothetical protein [Bacillus licheniformis DSM 13] | UniRef100_Q65HY5 | Bacillus licheniformis DSM 13 |
| 2876 | yclK two-component sensor histidine kinase YclK | Hypothetical protein [Bacillus licheniformis DSM 13] | UniRef100_Q65HY4 | Bacillus licheniformis DSM 13 |
| 2877 | yclJ two-component response regulator YclJ | Hypothetical protein [Bacillus licheniformis DSM 13] | UniRef100_Q65HY3 | Bacillus licheniformis DSM 13 |
| 2878 | BLP04767 hypothetical protein | | | |
| 2879 | resE two-component sensor histidine kinase ResE | ResE1 [Bacillus licheniformis DSM 13] | UniRef100_Q65HY2 | Bacillus licheniformis DSM 13 |

| 2880 | resD two-component response regulator ResD | ResD [Bacillus licheniformis DSM 13] | UniRef100_Q65HY1 | Bacillus licheniformis DSM 13 |
|---|---|---|---|---|
| 2881 | resC ResC | Hypothetical protein resC [Bacillus licheniformis DSM 13] | UniRef100_Q65HY0 | Bacillus licheniformis DSM 13 |
| 2882 | resB ResB | ResB [Bacillus licheniformis DSM 13] | UniRef100_Q65HX9 | Bacillus licheniformis DSM 13 |
| 2883 | resA essential cytochrome c biogenesis protein | | | |
| 2884 | rluB pseudouridine synthase | Pseudouridine synthase [Bacillus licheniformis DSM 13] | UniRef100_Q62TC6 | Bacillus licheniformis DSM 13 |
| 2885 | spmB spore maturation protein | SpmB [Bacillus licheniformis DSM 13] | UniRef100_Q65HX6 | Bacillus licheniformis DSM 13 |
| 2886 | spmA spore maturation protein | SpmA [Bacillus licheniformis DSM 13] | UniRef100_Q65HX5 | Bacillus licheniformis DSM 13 |
| 2887 | dacB D-alanyl-D-alanine carboxypeptidase (penicillin-binding protein 5*) | DacB [Bacillus licheniformis DSM 13] | UniRef100_Q65HX4 | Bacillus licheniformis DSM 13 |
| 2888 | ypuI conserved hypothetical protein YpuI | YpuI [Bacillus licheniformis DSM 13] | UniRef100_Q65HX3 | Bacillus licheniformis DSM 13 |
| 2889 | scpB SMC interacting protein | YpuH [Bacillus licheniformis DSM 13] | UniRef100_Q65HX2 | Bacillus licheniformis DSM 13 |
| 2890 | scpA Segregation and condensation protein A ScpA | YpuG [Bacillus licheniformis DSM 13] | UniRef100_Q65HX1 | Bacillus licheniformis DSM 13 |
| 2891 | BLP04937 conserved hypothetical protein | | | |
| 2892 | ypuF conserved hypothetical protein YpuF | YpuF [Bacillus licheniformis DSM 13] | UniRef100_Q65HW9 | Bacillus licheniformis DSM 13 |
| 2893 | ribT reductase | RibT [Bacillus licheniformis DSM 13] | UniRef100_Q65HW8 | Bacillus licheniformis DSM 13 |
| 2894 | ribH riboflavin synthase (beta subunit) | RibH [Bacillus licheniformis DSM 13] | UniRef100_Q65HW7 | Bacillus licheniformis DSM 13 |
| 2895 | ribA GTP cyclohydrolase II and 3,4-dihydroxy-2-butanone 4-phosphate synthase | RibA [Bacillus licheniformis DSM 13] | UniRef100_Q65HW6 | Bacillus licheniformis DSM 13 |
| 2896 | ribE riboflavin synthase (alpha subunit) | RibE [Bacillus licheniformis DSM 13] | UniRef100_Q65HW5 | Bacillus licheniformis DSM 13 |
| 2897 | ribD riboflavin-specific deaminase | RibD [Bacillus licheniformis DSM 13] | UniRef100_Q65HW4 | Bacillus licheniformis DSM 13 |
| 2898 | BLP02752 hypothetical protein | | | |
| 2899 | ypuD conserved hypothetical protein YpuD | YpuD [Bacillus licheniformis DSM 13] | UniRef100_Q65HW3 | Bacillus licheniformis DSM 13 |
| 2900 | BLP02754 putative phosphatase | Hypothetical protein [Bacillus licheniformis DSM 13] | UniRef100_Q65HW2 | Bacillus licheniformis DSM 13 |
| 2901 | BLP02755 Stress response homolog protein | Hypothetical protein [Bacillus licheniformis DSM 13] | UniRef100_Q65HW1 | Bacillus licheniformis DSM 13 |
| 2902 | BLP02756 conserved hypothetical protein | Hypothetical protein [Bacillus licheniformis DSM 13] | UniRef100_Q65HW0 | Bacillus licheniformis DSM 13 |
| 2903 | rapA response regulator aspartate phosphatase | RapA [Bacillus licheniformis DSM 13] | UniRef100_Q65HV9 | Bacillus licheniformis DSM 13 |
| 2904 | BLP02758 hypothetical protein | | | |
| 2905 | ppiB peptidyl-prolyl isomerase | PpiB [Bacillus licheniformis DSM 13] | UniRef100_Q65HV8 | Bacillus licheniformis DSM 13 |
| 2906 | ypuA conserved hypothetical protein YpuA | YpuA [Bacillus licheniformis DSM 13] | UniRef100_Q65HV7 | Bacillus licheniformis DSM 13 |
| 2907 | yndL conserved hypothetical protein YndL | YndL [Bacillus licheniformis DSM 13] | UniRef100_Q65HV4 | Bacillus licheniformis DSM 13 |
| 2908 | lysA diaminopimelate decarboxylase | LysA [Bacillus licheniformis DSM 13] | UniRef100_Q65HV3 | Bacillus licheniformis DSM 13 |
| 2909 | BLP02764 conserved hypothetical protein | Hypothetical protein [Bacillus licheniformis DSM 13] | UniRef100_Q65HV2 | Bacillus licheniformis DSM 13 |
| 2910 | spoVAF SpoVAF | Hypothetical protein spoVAF [Bacillus licheniformis DSM 13] | UniRef100_Q65HV1 | Bacillus licheniformis DSM 13 |
| 2911 | spoVAE1 Stage V sporulation protein SpoVAE1 | SpoVAE1 [Bacillus licheniformis DSM 13] | UniRef100_Q65HV0 | Bacillus licheniformis DSM 13 |
| 2912 | spoVAE2 Stage V sporulation protein SpoVAE2 | SpoVAE2 [Bacillus licheniformis DSM 13] | UniRef100_Q65HU9 | Bacillus licheniformis DSM 13 |
| 2913 | spoVAD Stage V sporulation protein SpoVAD | Hypothetical protein spoVAD [Bacillus licheniformis DSM 13] | UniRef100_Q65HU8 | Bacillus licheniformis DSM 13 |
| 2914 | spoVAC SpoVAC | SpoVAC [Bacillus licheniformis DSM 13] | UniRef100_Q65HU7 | Bacillus licheniformis DSM 13 |

| 2915 | spoVAB SpoVAB | Hypothetical protein spoVAB [Bacillus licheniformis DSM 13] | UniRef100_Q65HU6 | Bacillus licheniformis DSM 13 |
|---|---|---|---|---|
| 2916 | spoVAA SpoVAA | Stage V sporulation protein AA [Bacillus licheniformis] | UniRef100_Q65HU5 | Bacillus licheniformis |
| 2917 | BLP02771 hypothetical protein | | | |
| 2918 | sigF RNA polymerase sporulation-specific sigma factor (sigma-F) | SigF [Bacillus licheniformis DSM 13] | UniRef100_Q65HU4 | Bacillus licheniformis DSM 13 |
| 2919 | spoIIAB anti-sigma factor (antagonist of sigma-F) and serine kinase | Anti-sigma F factor [Bacillus licheniformis] | UniRef100_P26778 | Bacillus licheniformis |
| 2920 | spoIIAA anti-anti-sigma factor (antagonist of SpoIIAB) | SpoIIAA [Bacillus licheniformis DSM 13] | UniRef100_Q65HU2 | Bacillus licheniformis DSM 13 |
| 2921 | dacF penicilin binding protein (putative D-alanyl-D-alanine carboxypeptidase) | Penicilin binding protein [Bacillus licheniformis DSM 13] | UniRef100_Q62T91 | Bacillus licheniformis DSM 13 |
| 2922 | punA purine nucleoside phosphorylase | PunA [Bacillus licheniformis DSM 13] | UniRef100_Q65HU0 | Bacillus licheniformis DSM 13 |
| 2923 | drm phosphopentomutase | Drm [Bacillus licheniformis DSM 13] | UniRef100_Q65HT9 | Bacillus licheniformis DSM 13 |
| 2924 | ripX site-specific integrase_recombinase | RipX [Bacillus licheniformis DSM 13] | UniRef100_Q65HT8 | Bacillus licheniformis DSM 13 |
| 2925 | BLP02779 conserved hypothetical protein | Hypothetical protein [Bacillus licheniformis DSM 13] | UniRef100_Q65HT7 | Bacillus licheniformis DSM 13 |
| 2926 | fur transcriptional regulator | Fur family [Bacillus licheniformis DSM 13] | UniRef100_Q65HT6 | Bacillus licheniformis DSM 13 |
| 2927 | spoIIM SpoIIM | Hypothetical protein spoIIM [Bacillus licheniformis DSM 13] | UniRef100_Q65HT5 | Bacillus licheniformis DSM 13 |
| 2928 | BLP02782 PTS lactose_cellobiose IIC component | YdhO [Bacillus licheniformis DSM 13] | UniRef100_Q65HT4 | Bacillus licheniformis DSM 13 |
| 2929 | BLP02783 Phosphotransferase system, lactose_cellobiose-specific IIB subunit | Hypothetical protein [Bacillus licheniformis DSM 13] | UniRef100_Q65HT3 | Bacillus licheniformis DSM 13 |
| 2930 | BLP02784 putative allantoin permease | Probable allantoin permease [Bacillus subtilis] | UniRef100_P94575 | Bacillus subtilis |
| 2931 | BLP02785 GCN5-related N-acetyltransferase | Hypothetical protein [Bacillus licheniformis DSM 13] | UniRef100_Q65HT0 | Bacillus licheniformis DSM 13 |
| 2932 | yqkK conserved hypothetical protein yqkK | Hypothetical protein yqkK [Bacillus subtilis] | UniRef100_P54573 | Bacillus subtilis |
| 2933 | yybD probable acetyltransferase YybD | GCN5-related N-acetyltransferase [Bacillus licheniformis DSM 13] | UniRef100_Q62T81 | Bacillus licheniformis DSM 13 |
| 2934 | yqxK conserved protein YqxK | Hypothetical protein yqxK [Bacillus licheniformis DSM 13] | UniRef100_Q65HS8 | Bacillus licheniformis DSM 13 |
| 2935 | nudF ADP-ribose pyrophosphatase | NudF [Bacillus licheniformis DSM 13] | UniRef100_Q65HS7 | Bacillus licheniformis DSM 13 |
| 2936 | BLP02789 conserved hypothetical protein | Hypothetical protein [Bacillus licheniformis DSM 13] | UniRef100_Q65HS6 | Bacillus licheniformis DSM 13 |
| 2937 | ydgC conserved hypothetical protein YdgC | YdgC [Bacillus licheniformis DSM 13] | UniRef100_Q65HS5 | Bacillus licheniformis DSM 13 |
| 2938 | ydgD conserved hypothetical protein YdgD | YdgD [Bacillus licheniformis DSM 13] | UniRef100_Q65HS4 | Bacillus licheniformis DSM 13 |
| 2939 | yqkF Aldo_keto reductase YqkF | YqkF [Bacillus licheniformis DSM 13] | UniRef100_Q65HS3 | Bacillus licheniformis DSM 13 |
| 2940 | yqkE conserved hypothetical protein YqkE | YqkE [Bacillus licheniformis DSM 13] | UniRef100_Q65HS1 | Bacillus licheniformis DSM 13 |
| 2941 | yqkD conserved protein YqkD | YqkD [Bacillus licheniformis DSM 13] | UniRef100_Q65HS0 | Bacillus licheniformis DSM 13 |
| 2942 | yqkC YqkC | Hypothetical protein yqkC [Bacillus licheniformis DSM 13] | UniRef100_Q65HR9 | Bacillus licheniformis DSM 13 |
| 2943 | yqkB conserved protein YqkB | Hypothetical protein yqkB [Bacillus licheniformis DSM 13] | UniRef100_Q65HR8 | Bacillus licheniformis DSM 13 |
| 2944 | BLP02797 conserved hypothetical protein | Hypothetical protein [Bacillus licheniformis DSM 13] | UniRef100_Q65HR7 | Bacillus licheniformis DSM 13 |
| 2945 | yqkA probable acetyltransferase YqkA | Hypothetical protein yqkA [Bacillus licheniformis DSM 13] | UniRef100_Q65HR6 | Bacillus licheniformis DSM 13 |
| 2946 | yqjZ conserved protein YqjZ | YqjZ [Bacillus licheniformis DSM 13] | UniRef100_Q65HR5 | Bacillus licheniformis DSM 13 |
| 2947 | BLP04769 hypothetical protein | | | |
| 2948 | lip lipase | Lip [Bacillus licheniformis DSM 13] | UniRef100_Q65HR4 | Bacillus licheniformis DSM 13 |

| 2949 | BLP02801 conserved hypothetical protein | Hypothetical protein [Bacillus licheniformis DSM 13] | UniRef100_Q62T66 | Bacillus licheniformis DSM 13 |
|---|---|---|---|---|
| 2950 | appAA oligopeptide ABC transporter (oligopeptide-binding protein) | Hypothetical protein [Bacillus licheniformis DSM 13] | UniRef100_Q65HR3 | Bacillus licheniformis DSM 13 |
| 2951 | appBA oligopeptide ABC transporter (permease) | Hypothetical protein [Bacillus licheniformis DSM 13] | UniRef100_Q65HR2 | Bacillus licheniformis DSM 13 |
| 2952 | appCA oligopeptide ABC transporter (permease) | Hypothetical protein [Bacillus licheniformis DSM 13] | UniRef100_Q65HR1 | Bacillus licheniformis DSM 13 |
| 2953 | appDA ABC transporter (ATP-binding protein) | Hypothetical protein [Bacillus licheniformis DSM 13] | UniRef100_Q65HR0 | Bacillus licheniformis DSM 13 |
| 2954 | appFA oligopeptide ABC transporter (ATP-binding protein) | Hypothetical protein [Bacillus licheniformis DSM 13] | UniRef100_Q65HQ9 | Bacillus licheniformis DSM 13 |
| 2955 | BLP02807 putative multidrug extrusion protein | Hypothetical protein [Bacillus licheniformis DSM 13] | UniRef100_Q65HQ8 | Bacillus licheniformis DSM 13 |
| 2956 | BLP02808 Fibronectin-binding protein, putative | Hypothetical protein [Bacillus licheniformis DSM 13] | UniRef100_Q65HQ7 | Bacillus licheniformis DSM 13 |
| 2957 | BLP04939 hypothetical protein | Hypothetical protein [Bacillus licheniformis DSM 13] | UniRef100_Q65HQ6 | Bacillus licheniformis DSM 13 |
| 2958 | yolD conserved hypothetical protein YolD | YolD [Bacillus licheniformis DSM 13] | UniRef100_Q65HQ5 | Bacillus licheniformis DSM 13 |
| 2959 | uvrX UV-damage repair protein UvrX | UvrX [Bacillus licheniformis DSM 13] | UniRef100_Q65HQ4 | Bacillus licheniformis DSM 13 |
| 2960 | BLP02811 hypothetical protein | | | |
| 2961 | yqzH YqzH | Hypothetical protein yqzH [Bacillus licheniformis DSM 13] | UniRef100_Q65HQ3 | Bacillus licheniformis DSM 13 |
| 2962 | yqjV Sugar transporter superfamily YqjV | YqjV [Bacillus licheniformis DSM 13] | UniRef100_Q65HQ2 | Bacillus licheniformis DSM 13 |
| 2963 | yqjT conserved protein YqjT | YqjT [Bacillus licheniformis DSM 13] | UniRef100_Q65HQ1 | Bacillus licheniformis DSM 13 |
| 2964 | coaA pantothenate kinase | CoaA [Bacillus licheniformis DSM 13] | UniRef100_Q65HQ0 | Bacillus licheniformis DSM 13 |
| 2965 | lacG conserved LacG | Hypothetical protein [Bacillus licheniformis DSM 13] | UniRef100_Q65HP9 | Bacillus licheniformis DSM 13 |
| 2966 | lacF ABC transporter,ATP_GTP-binding site motif A (P-loop) | Hypothetical protein [Bacillus licheniformis DSM 13] | UniRef100_Q65HP8 | Bacillus licheniformis DSM 13 |
| 2967 | lacA3 LacA3 | Hypothetical protein [Bacillus licheniformis DSM 13] | UniRef100_Q65HP7 | Bacillus licheniformis DSM 13 |
| 2968 | BLP02819 transcriptional regulator, lacI family protein | Hypothetical protein [Bacillus licheniformis DSM 13] | UniRef100_Q65HP6 | Bacillus licheniformis DSM 13 |
| 2969 | BLP02820 hypothetical protein | | | |
| 2970 | BLP02821 conserved hypothetical protein | Hypothetical protein [Bacillus licheniformis DSM 13] | UniRef100_Q65HP5 | Bacillus licheniformis DSM 13 |
| 2971 | ymaC conserved hypothetical protein YmaC | YmaC [Bacillus licheniformis DSM 13] | UniRef100_Q65HP4 | Bacillus licheniformis DSM 13 |
| 2972 | BLP04770 hypothetical protein | | | |
| 2973 | BLP02823 hypothetical protein | | | |
| 2974 | yqjQ Short-chain dehydrogenase_reductase YqjQ | YqjQ [Bacillus licheniformis DSM 13] | UniRef100_Q65HP3 | Bacillus licheniformis DSM 13 |
| 2975 | yqjP conserved protein YqjP | YqjP [Bacillus licheniformis DSM 13] | UniRef100_Q65HP2 | Bacillus licheniformis DSM 13 |
| 2976 | proI pyrroline-5-carboxylate reductase | ProI [Bacillus licheniformis DSM 13] | UniRef100_Q65HP1 | Bacillus licheniformis DSM 13 |
| 2977 | yqjN putative amino acid degradation protein | YqjN [Bacillus licheniformis DSM 13] | UniRef100_Q65HP0 | Bacillus licheniformis DSM 13 |
| 2978 | yqjM NADH:flavin oxidoreductase_NADH oxidase | YqjM [Bacillus licheniformis DSM 13] | UniRef100_Q65HN9 | Bacillus licheniformis DSM 13 |
| 2979 | yqjL YqjL | YqjL [Bacillus licheniformis DSM 13] | UniRef100_Q65HN8 | Bacillus licheniformis DSM 13 |
| 2980 | rpmGAA possible ribosomal protein L33 | Hypothetical protein [Bacillus licheniformis DSM 13] | UniRef100_Q65HN7 | Bacillus licheniformis DSM 13 |
| 2981 | rnz RNase Z responsible for the maturation of the 3' end of tRNA | YqjK [Bacillus licheniformis DSM 13] | UniRef100_Q65HN6 | Bacillus licheniformis DSM 13 |
| 2982 | zwf glucose-6-phosphate 1-dehydrogenase | Zwf [Bacillus licheniformis DSM 13] | UniRef100_Q65HN5 | Bacillus licheniformis DSM 13 |

| 2983 | BLP02833 conserved hypothetical protein | Hypothetical protein [Bacillus licheniformis DSM 13] | UniRef100_Q65HN4 | Bacillus licheniformis DSM 13 |
|------|------|------|------|------|
| 2984 | BLP02834 conserved hypothetical protein | RocC [Bacillus licheniformis DSM 13] | UniRef100_Q65HN3 | Bacillus licheniformis DSM 13 |
| 2985 | BLP02835 conserved hypothetical protein | Hypothetical protein [Bacillus licheniformis DSM 13] | UniRef100_Q65HN2 | Bacillus licheniformis DSM 13 |
| 2986 | ydhQ transcriptional regulator YdhQ | YdhQ [Bacillus licheniformis DSM 13] | UniRef100_Q65HN1 | Bacillus licheniformis DSM 13 |
| 2987 | ydhP Glycoside hydrolase, family 1 YdhP | YdhP [Bacillus licheniformis DSM 13] | UniRef100_Q65HN0 | Bacillus licheniformis DSM 13 |
| 2988 | ydhO PTS system, cellobiose-specific enzyme IIC, YdhO | Putative cellobiose-specific enzyme IIC [Bacillus pumilus] | UniRef100_Q8KP28 | Bacillus pumilus |
| 2989 | ydhN Phosphotransferase system PTS, lactose_cellobiose-specific IIA subunit YdhN | Phosphotransferase system PTS, lactose/cellobiose-specific IIA subunit [Bacillus licheniformis DSM 13] | UniRef100_Q62T30 | Bacillus licheniformis DSM 13 |
| 2990 | ydhM Phosphotransferase system, lactose_cellobiose-specific IIB subunit YdhM | YdhM [Bacillus licheniformis DSM 13] | UniRef100_Q65HM6 | Bacillus licheniformis DSM 13 |
| 2991 | phoB alkaline phosphatase III | Alkaline phosphatase I [Bacillus licheniformis] | UniRef100_Q9F2A5 | Bacillus licheniformis |
| 2992 | gdh glucose 1-dehydrogenase | Glucose dehydrogenase [Bacillus licheniformis] | UniRef100_Q9F2A6 | Bacillus licheniformis |
| 2993 | yqjI 6-phosphogluconate dehydrogenase, decarboxylating YqjI | YqjI [Bacillus licheniformis DSM 13] | UniRef100_Q65HM3 | Bacillus licheniformis DSM 13 |
| 2994 | dinB1 UMUC-like DNA-repair protein DinB1 | YqjH [Bacillus licheniformis DSM 13] | UniRef100_Q65HM2 | Bacillus licheniformis DSM 13 |
| 2995 | yqzJ YqzJ | Hypothetical protein yqzJ [Bacillus licheniformis DSM 13] | UniRef100_Q65HM1 | Bacillus licheniformis DSM 13 |
| 2996 | oxaA2 translocase for membrane proteins OxaA2 | YqjG [Bacillus licheniformis DSM 13] | UniRef100_Q65HM0 | Bacillus licheniformis DSM 13 |
| 2997 | yqjE peptidase T YqjE | YqjE [Bacillus licheniformis DSM 13] | UniRef100_Q65HL9 | Bacillus licheniformis DSM 13 |
| 2998 | yqjD Carboxyl transferase YqjD | YqjD [Bacillus licheniformis DSM 13] | UniRef100_Q65HL8 | Bacillus licheniformis DSM 13 |
| 2999 | BLP02851 hypothetical protein | | | |
| 3000 | yqjA conserved membrane protein YqjA | Hypothetical protein yqjA [Bacillus licheniformis DSM 13] | UniRef100_Q65HL7 | Bacillus licheniformis DSM 13 |
| 3001 | artM ABC transporter | YqiZ [Bacillus licheniformis DSM 13] | UniRef100_Q65HL6 | Bacillus licheniformis DSM 13 |
| 3002 | artQ high affinity arginine transport system permease protein | YqiY [Bacillus licheniformis DSM 13] | UniRef100_Q65HL5 | Bacillus licheniformis DSM 13 |
| 3003 | yqiX Probable amino-acid ABC transporter extracellular-binding protein YqiX | Probable amino-acid ABC transporter extracellular binding protein yqiX precursor [Bacillus subtilis] | UniRef100_P54535 | Bacillus subtilis |
| 3004 | BLP02857 hypothetical protein | | | |
| 3005 | yqiW YqiW | YqiW [Bacillus licheniformis DSM 13] | UniRef100_Q65HL2 | Bacillus licheniformis DSM 13 |
| 3006 | bmrU multidrug resistance protein | BmrU [Bacillus licheniformis DSM 13] | UniRef100_Q65HL1 | Bacillus licheniformis DSM 13 |
| 3007 | bkdB branched-chain alpha-keto acid dehydrogenase E2 subunit (lipoamide acyltransferase) | BkdB [Bacillus licheniformis DSM 13] | UniRef100_Q65HL0 | Bacillus licheniformis DSM 13 |
| 3008 | bkdAB branched-chain alpha-keto acid dehydrogenase E1 subunit (2-oxoisovalerate dehydrogenase beta subunit) | BkdAB [Bacillus licheniformis DSM 13] | UniRef100_Q65HK9 | Bacillus licheniformis DSM 13 |
| 3009 | bkdAA branched-chain alpha-keto acid dehydrogenase E1 subunit (2-oxoisovalerate dehydrogenase alpha subunit) | BkdAA [Bacillus licheniformis DSM 13] | UniRef100_Q65HK8 | Bacillus licheniformis DSM 13 |
| 3010 | bfmBC branched-chain alpha-keto acid dehydrogenase E3 subunit (dihydrolipoamide dehydrogenase) | LpdV [Bacillus licheniformis DSM 13] | UniRef100_Q65HK7 | Bacillus licheniformis DSM 13 |
| 3011 | buk branched-chain fatty-acid kinase (butyrate kinase) | Buk [Bacillus licheniformis DSM 13] | UniRef100_Q65HK6 | Bacillus licheniformis DSM 13 |

| 3012 | bcd leucine dehydrogenase | Leucine dehydrogenase [Bacillus licheniformis] | UniRef100_Q53560 | Bacillus licheniformis |
|---|---|---|---|---|
| 3013 | ptb phosphate butyryltransferase | Ptb [Bacillus licheniformis DSM 13] | UniRef100_Q65HK4 | Bacillus licheniformis DSM 13 |
| 3014 | bkdR transcriptional regulator (sigma-L-dependent) | BkdR [Bacillus licheniformis DSM 13] | UniRef100_Q65HK3 | Bacillus licheniformis DSM 13 |
| 3015 | yqzF conserved protein YqzF | Hypothetical protein yqzF [Bacillus licheniformis DSM 13] | UniRef100_Q65HK2 | Bacillus licheniformis DSM 13 |
| 3016 | yqiK Glycerophosphoryl diester phosphodiesterase YqiK | YqiK [Bacillus licheniformis DSM 13] | UniRef100_Q65HK1 | Bacillus licheniformis DSM 13 |
| 3017 | yqiI N-acetylmuramoyl-L-alanine amidase autolysin YqiI | YqiI [Bacillus licheniformis DSM 13] | UniRef100_Q65HK0 | Bacillus licheniformis DSM 13 |
| 3018 | yqiH YqiH | | | |
| 3019 | BLP02873 hypothetical protein | | | |
| 3020 | cysEA serine acetyltransferase | Hypothetical protein [Bacillus licheniformis DSM 13] | UniRef100_Q65HJ8 | Bacillus licheniformis DSM 13 |
| 3021 | BLP04895 conserved hypothetical protein | Hypothetical protein [Bacillus licheniformis DSM 13] | UniRef100_Q62T02 | Bacillus licheniformis DSM 13 |
| 3022 | spo0A two-component response regulator | Spo0A protein [Bacillus subtilis] | UniRef100_Q65HJ7 | Bacillus subtilis |
| 3023 | spoIVB serine peptidase SpoIVB | Serine peptidase of the SA clan [Bacillus licheniformis DSM 13] | UniRef100_Q62T00 | Bacillus licheniformis DSM 13 |
| 3024 | recN RecN | Hypothetical protein recN [Bacillus licheniformis DSM 13] | UniRef100_Q65HJ5 | Bacillus licheniformis DSM 13 |
| 3025 | ahrC transcriptional regulator | Arginine repressor [Bacillus licheniformis] | UniRef100_O86130 | Bacillus licheniformis |
| 3026 | yqxC putative hemolysin A YqxC | YqxC [Bacillus licheniformis DSM 13] | UniRef100_Q65HJ3 | Bacillus licheniformis DSM 13 |
| 3027 | dxs 1-deoxyxylulose-5-phosphate synthase | Dxs [Bacillus licheniformis DSM 13] | UniRef100_Q65HJ2 | Bacillus licheniformis DSM 13 |
| 3028 | BLP02882 hypothetical protein | | | |
| 3029 | ispAB Polyprenyl synthetase,Polyprenyl synthetase | YqiD [Bacillus licheniformis DSM 13] | UniRef100_Q65HJ1 | Bacillus licheniformis DSM 13 |
| 3030 | xseB Exonuclease VII, small subunit | YqiC [Bacillus licheniformis DSM 13] | UniRef100_Q65HJ0 | Bacillus licheniformis DSM 13 |
| 3031 | xseA Exonuclease VII, large subunit | YqiB [Bacillus licheniformis DSM 13] | UniRef100_Q65HI9 | Bacillus licheniformis DSM 13 |
| 3032 | folD methylenetetrahydrofolate dehydrogenase and methenyltetrahydrofolate cyclohydrolase | FolD [Bacillus licheniformis DSM 13] | UniRef100_Q65HI8 | Bacillus licheniformis DSM 13 |
| 3033 | nusB NusB | Hypothetical protein nusB [Bacillus licheniformis DSM 13] | UniRef100_Q65HI7 | Bacillus licheniformis DSM 13 |
| 3034 | yqhY conserved protein YqhY | Hypothetical protein yqhY [Bacillus licheniformis DSM 13] | UniRef100_Q65HI6 | Bacillus licheniformis DSM 13 |
| 3035 | accC acetyl-CoA carboxylase subunit (biotin carboxylase subunit) | Acetyl-CoA carboxylase subunit [Bacillus licheniformis DSM 13] | UniRef100_Q62SY9 | Bacillus licheniformis DSM 13 |
| 3036 | accB acetyl-CoA carboxylase subunit (biotin carboxyl carrier subunit) | AccB [Bacillus licheniformis DSM 13] | UniRef100_Q65HI4 | Bacillus licheniformis DSM 13 |
| 3037 | spoIIIAH SpoIIIAH | Hypothetical protein spoIIIAH [Bacillus licheniformis DSM 13] | UniRef100_Q65HI3 | Bacillus licheniformis DSM 13 |
| 3038 | spoIIIAG SpoIIIAG | Hypothetical protein spoIIIAG [Bacillus licheniformis DSM 13] | UniRef100_Q65HI2 | Bacillus licheniformis DSM 13 |
| 3039 | spoIIIAF SpoIIIAF | Hypothetical protein spoIIIAF [Bacillus licheniformis DSM 13] | UniRef100_Q65HI1 | Bacillus licheniformis DSM 13 |
| 3040 | spoIIIAE SpoIIIAE | Hypothetical protein spoIIIAE [Bacillus licheniformis DSM 13] | UniRef100_Q65HI0 | Bacillus licheniformis DSM 13 |
| 3041 | spoIIIAD SpoIIIAD | Hypothetical protein spoIIIAD [Bacillus licheniformis DSM 13] | UniRef100_Q65HH9 | Bacillus licheniformis DSM 13 |
| 3042 | spoIIIAC SpoIIIAC | Hypothetical protein spoIIIAC [Bacillus licheniformis DSM 13] | UniRef100_Q65HH8 | Bacillus licheniformis DSM 13 |
| 3043 | spoIIIAB SpoIIIAB | Hypothetical protein spoIIIAB [Bacillus licheniformis DSM 13] | UniRef100_Q65HH7 | Bacillus licheniformis DSM 13 |
| 3044 | spoIIIAA SpoIIIAA | SpoIIIAA [Bacillus licheniformis DSM 13] | UniRef100_Q65HH6 | Bacillus licheniformis DSM 13 |

| 3045 | yqhV conserved protein YqhV | Hypothetical protein yqhV [Bacillus licheniformis DSM 13] | UniRef100_Q65HH5 | Bacillus licheniformis DSM 13 |
|---|---|---|---|---|
| 3046 | BLP02900 hypothetical protein | | | |
| 3047 | efp elongation factor P | Efp [Bacillus licheniformis DSM 13] | UniRef100_Q65HH4 | Bacillus licheniformis DSM 13 |
| 3048 | yqhT Peptidase M24B, X-Pro dipeptidase YqhT | YqhT [Bacillus licheniformis DSM 13] | UniRef100_Q65HH3 | Bacillus licheniformis DSM 13 |
| 3049 | yqhS 3-dehydroquinate dehydratase YqhS | YqhS [Bacillus licheniformis DSM 13] | UniRef100_Q65HH2 | Bacillus licheniformis DSM 13 |
| 3050 | yqhR conserved membrane protein YqhR | Hypothetical protein yqhR [Bacillus licheniformis DSM 13] | UniRef100_Q65HH1 | Bacillus licheniformis DSM 13 |
| 3051 | yqhQ conserved membrane protein YqhQ | Hypothetical protein yqhQ [Bacillus licheniformis DSM 13] | UniRef100_Q65HH0 | Bacillus licheniformis DSM 13 |
| 3052 | yqhP conserved membrane protein YqhP | Hypothetical protein yqhP [Bacillus licheniformis DSM 13] | UniRef100_Q65HG9 | Bacillus licheniformis DSM 13 |
| 3053 | yqhO conserved protein YqhO | YqhO [Bacillus licheniformis DSM 13] | UniRef100_Q65HG8 | Bacillus licheniformis DSM 13 |
| 3054 | BLP02908 hypothetical protein | | | |
| 3055 | mntR transcriptional regulator | MntR [Bacillus licheniformis DSM 13] | UniRef100_Q65HG7 | Bacillus licheniformis DSM 13 |
| 3056 | yqhM putative lipoate protein ligase YqhM | YqhM [Bacillus licheniformis DSM 13] | UniRef100_Q65HG6 | Bacillus licheniformis DSM 13 |
| 3057 | yqhL conserved protein YqhL | YqhL [Bacillus licheniformis DSM 13] | UniRef100_Q65HG5 | Bacillus licheniformis DSM 13 |
| 3058 | opuAC glycine betaine ABC transporter (glycine betaine-binding protein) | OpuAC [Bacillus licheniformis DSM 13] | UniRef100_Q65HG4 | Bacillus licheniformis DSM 13 |
| 3059 | opuAB glycine betaine ABC transporter (permease) | OpuAB [Bacillus licheniformis DSM 13] | UniRef100_Q65HG3 | Bacillus licheniformis DSM 13 |
| 3060 | opuAA glycine betaine ABC transporter (ATP-binding protein) | OpuAA [Bacillus licheniformis DSM 13] | UniRef100_Q65HG2 | Bacillus licheniformis DSM 13 |
| 3061 | gcvPB glycine decarboxylase (subunit 2) (glycine cleavage system protein P) | GcvPB [Bacillus licheniformis DSM 13] | UniRef100_Q65HG1 | Bacillus licheniformis DSM 13 |
| 3062 | gcvPA glycine decarboxylase (subunit 1) (glycine cleavage system protein P) | GcvPA [Bacillus licheniformis DSM 13] | UniRef100_Q65HG0 | Bacillus licheniformis DSM 13 |
| 3063 | gcvT aminomethyltransferase (glycine cleavage system protein T) | GcvT [Bacillus licheniformis DSM 13] | UniRef100_Q65HF9 | Bacillus licheniformis DSM 13 |
| 3064 | BLP02918 conserved hypothetical protein | Hypothetical protein [Bacillus licheniformis DSM 13] | UniRef100_Q65HF8 | Bacillus licheniformis DSM 13 |
| 3065 | yqhH DEAD_DEAH box helicase YqhH | YqhH [Bacillus licheniformis DSM 13] | UniRef100_Q65HF7 | Bacillus licheniformis DSM 13 |
| 3066 | yqhG conserved protein YqhG | Hypothetical protein yqhG [Bacillus licheniformis DSM 13] | UniRef100_Q65HF6 | Bacillus licheniformis DSM 13 |
| 3067 | sinI antagonist of SinR | SinI [Bacillus licheniformis DSM 13] | UniRef100_Q65HF5 | Bacillus licheniformis DSM 13 |
| 3068 | sinR transcriptional regulator | SinR [Bacillus licheniformis DSM 13] | UniRef100_Q65HF4 | Bacillus licheniformis DSM 13 |
| 3069 | BLP02923 hypothetical protein | | | |
| 3070 | tasA translocation-dependent antimicrobial spore component | TasA [Bacillus licheniformis DSM 13] | UniRef100_Q65HF3 | Bacillus licheniformis DSM 13 |
| 3071 | sipW type I signal peptidase | SipW [Bacillus licheniformis DSM 13] | UniRef100_Q65HF2 | Bacillus licheniformis DSM 13 |
| 3072 | yqxM secreted biofilm formation protein YqxM | Hypothetical protein yqxM [Bacillus licheniformis DSM 13] | UniRef100_Q65HF1 | Bacillus licheniformis DSM 13 |
| 3073 | yqzG YqzG | Hypothetical protein yqzG [Bacillus licheniformis DSM 13] | UniRef100_Q65HF0 | Bacillus licheniformis DSM 13 |
| 3074 | yqzE YqzE | Hypothetical protein yqzE [Bacillus licheniformis DSM 13] | UniRef100_Q65HE9 | Bacillus licheniformis DSM 13 |
| 3075 | comGG probably part of the DNA transport machinery ComGG | ComGG [Bacillus licheniformis DSM 13] | UniRef100_Q65HE8 | Bacillus licheniformis DSM 13 |
| 3076 | comGF probably part of the DNA transport machinery ComGF | ComGF [Bacillus licheniformis DSM 13] | UniRef100_Q65HE7 | Bacillus licheniformis DSM 13 |
| 3077 | comGE probably part of the DNA transport machinery | Late competence protein ComGE [Bacillus licheniformis] | UniRef100_Q8VQ69 | Bacillus licheniformis |

| | ComGE | | | |
|---|---|---|---|---|
| 3078 | comGD probably part of the DNA transport machinery ComGD | Late competence protein ComGD [Bacillus licheniformis] | UniRef100_Q8VQ70 | Bacillus licheniformis |
| 3079 | comGC ComGC | ComG operon protein 3 homolog precursor [Bacillus licheniformis] | UniRef100_Q8VQ71 | Bacillus licheniformis |
| 3080 | comGB probably part of the DNA transport machinery ComGB | ComGB [Bacillus licheniformis DSM 13] | UniRef100_Q65HE3 | Bacillus licheniformis DSM 13 |
| 3081 | comGA ComGA | Late competence protein ComGA [Bacillus licheniformis] | UniRef100_Q8VQ73 | Bacillus licheniformis |
| 3082 | BLP02936 hypothetical protein | | | |
| 3083 | yqhA putative sulphate transporter YqhA | YqhA [Bacillus licheniformis DSM 13] | UniRef100_Q65HE1 | Bacillus licheniformis DSM 13 |
| 3084 | yqgZ conserved protein YqgZ | Hypothetical protein yqgZ [Bacillus licheniformis DSM 13] | UniRef100_Q65HE0 | Bacillus licheniformis DSM 13 |
| 3085 | yqgY conserved protein YqgY | Hypothetical protein yqgY [Bacillus licheniformis DSM 13] | UniRef100_Q65HD9 | Bacillus licheniformis DSM 13 |
| 3086 | yqgX conserved protein YqgX | YqgX [Bacillus licheniformis DSM 13] | UniRef100_Q65HD8 | Bacillus licheniformis DSM 13 |
| 3087 | BLP02941 hypothetical protein | | | |
| 3088 | yqgW conserved protein YqgW | Hypothetical protein yqgW [Bacillus licheniformis DSM 13] | UniRef100_Q62SU2 | Bacillus licheniformis DSM 13 |
| 3089 | yqgV conserved protein YqgV | Hypothetical protein yqgV [Bacillus licheniformis DSM 13] | UniRef100_Q65HD6 | Bacillus licheniformis DSM 13 |
| 3090 | yqgU conserved protein YqgU | YqgU [Bacillus licheniformis DSM 13] | UniRef100_Q65HD5 | Bacillus licheniformis DSM 13 |
| 3091 | yqgT Peptidase M14, carboxypeptidase A | YqgT [Bacillus licheniformis DSM 13] | UniRef100_Q65HD4 | Bacillus licheniformis DSM 13 |
| 3092 | fhuD ferrichrome ABC transporter (ferrichrome-binding protein) | FhuD [Bacillus licheniformis DSM 13] | UniRef100_Q65HD3 | Bacillus licheniformis DSM 13 |
| 3093 | BLP02947 hypothetical protein | | | |
| 3094 | yqgS Sulfatase | YqgS [Bacillus licheniformis DSM 13] | UniRef100_Q65HD2 | Bacillus licheniformis DSM 13 |
| 3095 | glcK glucose kinase | GlcK [Bacillus licheniformis DSM 13] | UniRef100_Q65HD1 | Bacillus licheniformis DSM 13 |
| 3096 | yqgQ YqgQ | Hypothetical protein yqgQ [Bacillus licheniformis DSM 13] | UniRef100_Q65HD0 | Bacillus licheniformis DSM 13 |
| 3097 | spoVAFA SpoVAFA | Hypothetical protein spoVAFA [Bacillus licheniformis DSM 13] | UniRef100_Q65HC9 | Bacillus licheniformis DSM 13 |
| 3098 | gluP TPR-like,TPR repeat | YqgP [Bacillus licheniformis DSM 13] | UniRef100_Q65HC8 | Bacillus licheniformis DSM 13 |
| 3099 | yqgO YqgO | Hypothetical protein yqgO [Bacillus licheniformis DSM 13] | UniRef100_Q65HC7 | Bacillus licheniformis DSM 13 |
| 3100 | yqgN putative 5-formyltetrahydrofolate cyclo-ligase | YqgN [Bacillus licheniformis DSM 13] | UniRef100_Q65HC6 | Bacillus licheniformis DSM 13 |
| 3101 | yqgM Glycosyl transferase, Family 4 YqgM | YqgM [Bacillus licheniformis DSM 13] | UniRef100_Q65HC4 | Bacillus licheniformis DSM 13 |
| 3102 | yqgL YqgL | Hypothetical protein yqgL [Bacillus licheniformis DSM 13] | UniRef100_Q65HC3 | Bacillus licheniformis DSM 13 |
| 3103 | yqzD conserved protein YqzD | Homeodomain-like [Bacillus licheniformis DSM 13] | UniRef100_Q62SS7 | Bacillus licheniformis DSM 13 |
| 3104 | yqzC conserved protein YqzC | Hypothetical protein yqzC [Bacillus licheniformis DSM 13] | UniRef100_Q65HC1 | Bacillus licheniformis DSM 13 |
| 3105 | pstBB phosphate ABC transporter (ATP-binding protein) | PstBB [Bacillus licheniformis DSM 13] | UniRef100_Q65HC0 | Bacillus licheniformis DSM 13 |
| 3106 | pstBA phosphate ABC transporter (ATP-binding protein) | PstBA [Bacillus licheniformis DSM 13] | UniRef100_Q65HB9 | Bacillus licheniformis DSM 13 |
| 3107 | pstA phosphate ABC transporter (permease) | PstA [Bacillus licheniformis DSM 13] | UniRef100_Q65HB8 | Bacillus licheniformis DSM 13 |
| 3108 | pstC phosphate ABC transporter (permease) | PstC [Bacillus licheniformis DSM 13] | UniRef100_Q65HB7 | Bacillus licheniformis DSM 13 |
| 3109 | pstS phosphate ABC transporter (binding protein) | PstS [Bacillus licheniformis DSM 13] | UniRef100_Q65HB6 | Bacillus licheniformis DSM 13 |

| 3110 | pbpA penicillin-binding protein 2A | PbpA [Bacillus licheniformis DSM 13] | UniRef100_Q65HB5 | Bacillus licheniformis DSM 13 |
|------|-----------------------------------|-------------------------------------|------------------|------------------------------|
| 3111 | yqgE Sugar transporter superfamily | YqgE [Bacillus licheniformis DSM 13] | UniRef100_Q65HB4 | Bacillus licheniformis DSM 13 |
| 3112 | BLP02965 hypothetical protein | | | |
| 3113 | sodA superoxide dismutase | SodA [Bacillus licheniformis DSM 13] | UniRef100_Q65HB3 | Bacillus licheniformis DSM 13 |
| 3114 | yqgC conserved membrane protein YqgC | Hypothetical protein yqgC [Bacillus licheniformis DSM 13] | UniRef100_Q65HB2 | Bacillus licheniformis DSM 13 |
| 3115 | yqgB conserved membrane protein YqgB | YqgB [Bacillus licheniformis DSM 13] | UniRef100_Q65HB1 | Bacillus licheniformis DSM 13 |
| 3116 | yqfZ conserved protein YqfZ | Hypothetical protein yqfZ [Bacillus licheniformis DSM 13] | UniRef100_Q62SR5 | Bacillus licheniformis DSM 13 |
| 3117 | ispG IspG protein | YqfY [Bacillus licheniformis DSM 13] | UniRef100_Q65HA9 | Bacillus licheniformis DSM 13 |
| 3118 | yqfX conserved hypothetical protein YqfX | YqfX [Bacillus licheniformis DSM 13] | UniRef100_Q65HA8 | Bacillus licheniformis DSM 13 |
| 3119 | yqfW putative nucleotidease YqfW | YqfW [Bacillus licheniformis DSM 13] | UniRef100_Q65HA7 | Bacillus licheniformis DSM 13 |
| 3120 | zur transcriptional regulator | Zur [Bacillus licheniformis DSM 13] | UniRef100_Q65HA6 | Bacillus licheniformis DSM 13 |
| 3121 | yceA transcriptional regulator YceA | YceA [Bacillus licheniformis DSM 13] | UniRef100_Q65HA5 | Bacillus licheniformis DSM 13 |
| 3122 | BLP02975 ABC transporter | Hypothetical protein [Bacillus licheniformis DSM 13] | UniRef100_Q65HA4 | Bacillus licheniformis DSM 13 |
| 3123 | BLP02976 conserved hypothetical protein | Hypothetical protein [Bacillus licheniformis DSM 13] | UniRef100_Q65HA3 | Bacillus licheniformis DSM 13 |
| 3124 | yqfU conserved hypothetical protein YqfU | YqfU [Bacillus licheniformis DSM 13] | UniRef100_Q65HA2 | Bacillus licheniformis DSM 13 |
| 3125 | yqfT conserved hypothetical protein YqfT | YqfT [Bacillus licheniformis DSM 13] | UniRef100_Q65HA1 | Bacillus licheniformis DSM 13 |
| 3126 | nfo type IV apurinic_apyrimidinic endonuclease | YqfS [Bacillus licheniformis DSM 13] | UniRef100_Q65HA0 | Bacillus licheniformis DSM 13 |
| 3127 | yqfR DEAD_DEAH box ATP dependent helicase YqfR | YqfR [Bacillus licheniformis DSM 13] | UniRef100_Q65H99 | Bacillus licheniformis DSM 13 |
| 3128 | yqfQ Tubulin YqfQ | YqfQ [Bacillus licheniformis DSM 13] | UniRef100_Q65H98 | Bacillus licheniformis DSM 13 |
| 3129 | ispH isopentenyl diphosphate biosynthesis protein IspH | YqfP [Bacillus licheniformis DSM 13] | UniRef100_Q65H97 | Bacillus licheniformis DSM 13 |
| 3130 | yqfO conserved hypothetical protein YqfO | YqfO [Bacillus licheniformis DSM 13] | UniRef100_Q65H96 | Bacillus licheniformis DSM 13 |
| 3131 | yqfN conserved hypothetical protein YqfN | YqfN [Bacillus licheniformis DSM 13] | UniRef100_Q65H95 | Bacillus licheniformis DSM 13 |
| 3132 | BLP02986 Endonuclease V | YwqL [Bacillus licheniformis DSM 13] | UniRef100_Q65H93 | Bacillus licheniformis DSM 13 |
| 3133 | BLP04896 conserved hypothetical membrane associated protein | YetF protein [Bacillus subtilis] | UniRef100_O31533 | Bacillus subtilis |
| 3134 | BLP02987 putative cardiolipin synthetase | Cardiolipin synthetase [Bacillus pseudofirmus] | UniRef100_O66043 | Bacillus pseudofirmus |
| 3135 | BLP02988 conserved hypothetical protein | Hypothetical Membrane Associated Protein [Bacillus cereus] | UniRef100_Q813S1 | Bacillus cereus |
| 3136 | BLP02989 conserved hypothetical | Hypothetical protein [Bacillus cereus] | UniRef100_Q72XX0 | Bacillus cereus |
| 3137 | BLP04897 conserved hypothetical protein | Hypothetical protein [Bacillus cereus] | UniRef100_Q815J7 | Bacillus cereus |
| 3138 | BLP02990 Stage V sporulation protein AD homolog | Stage V sporulation protein AE [Oceanobacillus iheyensis] | UniRef100_Q8ERC6 | Oceanobacillus iheyensis |
| 3139 | spoVAD SpoVAD | Stage V sporulation protein AD [Bacillus anthracis] | UniRef100_Q81X67 | Bacillus anthracis |
| 3140 | spoVAC2 Stage V sporulation protein AC homolog | Stage V sporulation protein AC [Bacillus cereus] | UniRef100_Q72XX4 | Bacillus cereus |
| 3141 | BLP02993 conserved hypothetical | Lipoprotein, putative [Bacillus cereus] | UniRef100_Q72XX5 | Bacillus cereus |
| 3142 | BLP04898 conserved hypothetical protein | Hypothetical protein OB1383 [Oceanobacillus iheyensis] | UniRef100_Q8ERC2 | Oceanobacillus iheyensis |
| 3143 | BLP02993 manganese catalase | Mn catalase [Bacillus halodurans] | UniRef100_Q9KDZ2 | Bacillus halodurans |
| 3144 | BLP04824 conserved hypothetical protein | BH2251 protein [Bacillus halodurans] | UniRef100_Q9KAN6 | Bacillus halodurans |

| 3145 | gerKC putative spore germination protein | Spore germination protein XC precursor [Bacillus anthracis] | UniRef100_Q9ZFB3 | Bacillus anthracis |
|---|---|---|---|---|
| 3146 | gerKA partial protein | Spore germination protein XA [Bacillus cereus] | UniRef100_Q736Z7 | Bacillus cereus |
| 3147 | BLP04899 conserved hypotical protein | Sporulation specific N-acetylmuramoyl-L-alanine amidase [Oceanobacillus iheyensis] | UniRef100_Q8CX69 | Oceanobacillus iheyensis |
| 3148 | BLP02998 hypothetical protein | | | |
| 3149 | BLP02999 putative resolvase | Transposon Tn1546 resolvase [Enterococcus faecium] | UniRef100_Q06237 | Enterococcus faecium |
| 3150 | BLP03000 putative transposase | Transposase for transposon Tn1546 [Enterococcus faecium] | UniRef100_Q06238 | Enterococcus faecium |
| 3151 | BLP03001 conserved hypothetical protein | Hypothetical protein [Bacillus licheniformis DSM 13] | UniRef100_Q65H92 | Bacillus licheniformis DSM 13 |
| 3152 | BLP03002 conserved hypothetical protein longer than others do SJ1904C07D8 BANe | Hypothetical protein [Bacillus licheniformis DSM 13] | UniRef100_Q62SP9 | Bacillus licheniformis DSM 13 |
| 3153 | BLP03003 conserved hypothetical protein | Hypothetical protein [Bacillus licheniformis DSM 13] | UniRef100_Q65H90 | Bacillus licheniformis DSM 13 |
| 3154 | BLP04900 conserved hypothetical protein | Hypothetical protein ywqJ [Bacillus licheniformis DSM 13] | UniRef100_Q65H89 | Bacillus licheniformis DSM 13 |
| 3155 | BLP03004 conserved hypothetical protein | Hypothetical protein [Bacillus licheniformis DSM 13] | UniRef100_Q65H88 | Bacillus licheniformis DSM 13 |
| 3156 | BLP03005 conserved hypothetical protein | Hypothetical protein [Bacillus licheniformis DSM 13] | UniRef100_Q65H87 | Bacillus licheniformis DSM 13 |
| 3157 | BLP03006 conserved hypothetical protein | Hypothetical protein [Bacillus licheniformis DSM 13] | UniRef100_Q65H86 | Bacillus licheniformis DSM 13 |
| 3158 | ywqJA YwqJA | YwqJ [Bacillus licheniformis DSM 13] | UniRef100_Q65H85 | Bacillus licheniformis DSM 13 |
| 3159 | ywqI YwqI | Hypothetical protein ywqI [Bacillus licheniformis DSM 13] | UniRef100_Q65H84 | Bacillus licheniformis DSM 13 |
| 3160 | ywqH YwqH | Hypothetical protein ywqH [Bacillus licheniformis DSM 13] | UniRef100_Q62SP1 | Bacillus licheniformis DSM 13 |
| 3161 | cccA cytochrome c550 | CccA [Bacillus licheniformis DSM 13] | UniRef100_Q65H82 | Bacillus licheniformis DSM 13 |
| 3162 | BLP03011 hypothetical protein | | | |
| 3163 | sigA RNA polymerase major sigma-43 factor (sigma-A) | SigA [Bacillus licheniformis DSM 13] | UniRef100_Q65H81 | Bacillus licheniformis DSM 13 |
| 3164 | dnaG DNA primase | DnaG [Bacillus licheniformis DSM 13] | UniRef100_Q65H80 | Bacillus licheniformis DSM 13 |
| 3165 | yqxD conserved protein YqxD | Hypothetical protein yqxD [Bacillus licheniformis DSM 13] | UniRef100_Q65H79 | Bacillus licheniformis DSM 13 |
| 3166 | yqfL putative transcriptional regulator YqfL | YqfL [Bacillus licheniformis DSM 13] | UniRef100_Q65H78 | Bacillus licheniformis DSM 13 |
| 3167 | ccpN putative transcriptional repressor CcpN | YqzB [Bacillus licheniformis DSM 13] | UniRef100_Q65H77 | Bacillus licheniformis DSM 13 |
| 3168 | glyS glycyl-tRNA synthetase (beta subunit) | GlyS [Bacillus licheniformis DSM 13] | UniRef100_Q65H76 | Bacillus licheniformis DSM 13 |
| 3169 | glyQ glycyl-tRNA synthetase (alpha subunit) | GlyQ [Bacillus licheniformis DSM 13] | UniRef100_Q65H75 | Bacillus licheniformis DSM 13 |
| 3170 | recO RecO | Hypothetical protein recO [Bacillus licheniformis DSM 13] | UniRef100_Q65H74 | Bacillus licheniformis DSM 13 |
| 3171 | BLP03021 hypothetical protein | Hypothetical protein [Bacillus licheniformis DSM 13] | UniRef100_Q62SN1 | Bacillus licheniformis DSM 13 |
| 3172 | bex GTP-binding protein | Era [Bacillus licheniformis DSM 13] | UniRef100_Q65H73 | Bacillus licheniformis DSM 13 |
| 3173 | cdd cytidine_deoxycytidine deaminase | Cdd [Bacillus licheniformis DSM 13] | UniRef100_Q65H72 | Bacillus licheniformis DSM 13 |
| 3174 | dgkA diacylglycerol kinase | DgkA [Bacillus licheniformis DSM 13] | UniRef100_Q65H71 | Bacillus licheniformis DSM 13 |
| 3175 | BLP03025 Conserved hypothetical protein | YqfG [Bacillus licheniformis DSM 13] | UniRef100_Q65H70 | Bacillus licheniformis DSM 13 |
| 3176 | BLP03026 HDIG-domain containing protein | YqfF [Bacillus licheniformis DSM 13] | UniRef100_Q65H69 | Bacillus licheniformis DSM 13 |
| 3177 | phoH phosphate starvation-induced protein | PhoH [Bacillus licheniformis DSM 13] | UniRef100_Q65H68 | Bacillus licheniformis DSM 13 |

| 3178 | yqfD Putative stage IV sporulation YqfD | Putative stage IV sporulation YqfD [Bacillus licheniformis DSM 13] | UniRef100_Q65H67 | Bacillus licheniformis DSM 13 |
|---|---|---|---|---|
| 3179 | yqfC conserved hypothetical protein YqfC | YqfC [Bacillus licheniformis DSM 13] | UniRef100_Q65H66 | Bacillus licheniformis DSM 13 |
| 3180 | yqfB conserved hypothetical protein YqfB | YqfB [Bacillus licheniformis DSM 13] | UniRef100_Q65H65 | Bacillus licheniformis DSM 13 |
| 3181 | yqfA conserved hypothetical protein YqfA | YqfA [Bacillus licheniformis DSM 13] | UniRef100_Q65H64 | Bacillus licheniformis DSM 13 |
| 3182 | yqeZ intermembrane protease YqeZ | YqeZ [Bacillus licheniformis DSM 13] | UniRef100_Q65H63 | Bacillus licheniformis DSM 13 |
| 3183 | yqeY conserved hypothetical protein YqeY | YqeY [Bacillus licheniformis DSM 13] | UniRef100_Q65H62 | Bacillus licheniformis DSM 13 |
| 3184 | rpsU ribosomal protein S21 | RpsU [Bacillus licheniformis DSM 13] | UniRef100_Q65H61 | Bacillus licheniformis DSM 13 |
| 3185 | yqeW Na_Pi cotransporter II-related | Na/Pi cotransporter II-related [Bacillus licheniformis DSM 13] | UniRef100_Q62SL7 | Bacillus licheniformis DSM 13 |
| 3186 | BLP03036 deoxyribose-phosphate aldolase | Hypothetical protein [Bacillus licheniformis DSM 13] | UniRef100_Q65H59 | Bacillus licheniformis DSM 13 |
| 3187 | yqeV conserved hypothetical protein YqeV | YqeV [Bacillus licheniformis DSM 13] | UniRef100_Q65H58 | Bacillus licheniformis DSM 13 |
| 3188 | BLP03038 Conserved hypothetical protein | YqeU [Bacillus licheniformis DSM 13] | UniRef100_Q65H57 | Bacillus licheniformis DSM 13 |
| 3189 | prmA Ribosomal protein L11 methyltransferase | YqeT [Bacillus licheniformis DSM 13] | UniRef100_Q65H56 | Bacillus licheniformis DSM 13 |
| 3190 | dnaJ heat-shock protein | DnaJ [Bacillus licheniformis DSM 13] | UniRef100_Q65H55 | Bacillus licheniformis DSM 13 |
| 3191 | dnaK class I heat-shock protein (molecular chaperone) | DnaK [Bacillus licheniformis DSM 13] | UniRef100_Q65H54 | Bacillus licheniformis DSM 13 |
| 3192 | grpE heat-shock protein | GrpE [Bacillus licheniformis DSM 13] | UniRef100_Q65H53 | Bacillus licheniformis DSM 13 |
| 3193 | hrcA transcriptional regulator | HrcA [Bacillus licheniformis DSM 13] | UniRef100_Q65H52 | Bacillus licheniformis DSM 13 |
| 3194 | hemN coproporphyrinogen III oxidase | HemN [Bacillus licheniformis DSM 13] | UniRef100_Q65H51 | Bacillus licheniformis DSM 13 |
| 3195 | lepA GTP-binding protein | LepA [Bacillus licheniformis DSM 13] | UniRef100_Q65H50 | Bacillus licheniformis DSM 13 |
| 3196 | yqxA YqxA | Hypothetical protein yqxA [Bacillus licheniformis DSM 13] | UniRef100_Q65H49 | Bacillus licheniformis DSM 13 |
| 3197 | spoIIP Stage II sporulation protein P SpoIIP | SpoIIP [Bacillus licheniformis DSM 13] | UniRef100_Q65H48 | Bacillus licheniformis DSM 13 |
| 3198 | gpr spore protease | Gpr [Bacillus licheniformis DSM 13] | UniRef100_Q65H47 | Bacillus licheniformis DSM 13 |
| 3199 | rpsT ribosomal protein S20 | RpsT [Bacillus licheniformis DSM 13] | UniRef100_Q65H46 | Bacillus licheniformis DSM 13 |
| 3200 | holA DNA polymerase III clamp loader subunit, C-terminal | Hypothetical protein yqeN [Bacillus licheniformis] | UniRef100_Q65H45 | Bacillus licheniformis |
| 3201 | BLP03051 hypothetical protein | Hypothetical protein [Bacillus licheniformis DSM 13] | UniRef100_Q65H44 | Bacillus licheniformis DSM 13 |
| 3202 | comEC ComEC | Late competence protein ComEC [Bacillus licheniformis] | UniRef100_Q8VQ75 | Bacillus licheniformis |
| 3203 | comEB ComEB | Late competence protein ComEB [Bacillus licheniformis] | UniRef100_Q8VQ76 | Bacillus licheniformis |
| 3204 | comEA ComEA | Late competence protein ComEA [Bacillus licheniformis] | UniRef100_Q8VQ77 | Bacillus licheniformis |
| 3205 | comER ComER | Hypothetical protein comER [Bacillus licheniformis] | UniRef100_Q65H40 | Bacillus licheniformis |
| 3206 | yqeM SAM dependent methyltransferase YqeM | YqeM (SAM (And some other nucleotide) binding motif) [Bacillus licheniformis DSM 13] | UniRef100_Q65H39 | Bacillus licheniformis DSM 13 |
| 3207 | yqeL lojap-related protein YqeL | YqeL [Bacillus licheniformis DSM 13] | UniRef100_Q65H38 | Bacillus licheniformis DSM 13 |
| 3208 | yqeK Conserved hypothetical protein YqeK | YqeK [Bacillus licheniformis DSM 13] | UniRef100_Q65H37 | Bacillus licheniformis DSM 13 |
| 3209 | nadD nicotinate-nucleotide adenylyltransferase NadD | YqeJ [Bacillus licheniformis DSM 13] | UniRef100_Q65H36 | Bacillus licheniformis DSM 13 |
| 3210 | yqeI conserved hypothetical protein YqeI | YqeI [Bacillus licheniformis DSM 13] | UniRef100_Q65H35 | Bacillus licheniformis DSM 13 |
| 3211 | aroD shikimate 5-dehydrogenase | AroD [Bacillus licheniformis DSM 13] | UniRef100_Q65H34 | Bacillus licheniformis DSM 13 |

| 3212 | yqeH essential GTPase | YqeH [Bacillus licheniformis DSM 13] | UniRef100_Q65H33 | Bacillus licheniformis DSM 13 |
|---|---|---|---|---|
| 3213 | yqeG HAD superfamily (subfamily IIIA) phosphatase YqeG | YqeG (HAD superfamily (Subfamily IIIA) phosphatase,HAD-superfamily hydrolase, subfamily IIIA) [Bacillus licheniformis DSM 13] | UniRef100_Q65H32 | Bacillus licheniformis DSM 13 |
| 3214 | sda Sda | Hypothetical protein sda [Bacillus licheniformis DSM 13] | UniRef100_Q65H31 | Bacillus licheniformis DSM 13 |
| 3215 | BLP04771 conserved hypothetical protein | | | |
| 3216 | yqeF conserved hypothetical YqeF | Lipolytic enzyme, G-D-S-L [Bacillus licheniformis DSM 13] | UniRef100_Q62SI5 | Bacillus licheniformis DSM 13 |
| 3217 | ydfB Rhodopsin-like protein YdfB | YdfB [Bacillus licheniformis DSM 13] | UniRef100_Q65H29 | Bacillus licheniformis DSM 13 |
| 3218 | yrhF conserved protein YrhF | YrhF [Bacillus licheniformis DSM 13] | UniRef100_Q65H28 | Bacillus licheniformis DSM 13 |
| 3219 | yrhE Formate dehydrogenase, alpha subunit YrhE | YrhE [Bacillus licheniformis DSM 13] | UniRef100_Q65H27 | Bacillus licheniformis DSM 13 |
| 3220 | yrhD conserved protein YrhD | Hypothetical protein yrhD [Bacillus licheniformis DSM 13] | UniRef100_Q65H26 | Bacillus licheniformis DSM 13 |
| 3221 | BLP03070 conserved hypothetical protein | Hypothetical protein [Bacillus licheniformis DSM 13] | UniRef100_Q62SI0 | Bacillus licheniformis DSM 13 |
| 3222 | BLP03071 hypothetical protein | | | |
| 3223 | BLP03072 sigK RNA polymerase sporulation-specific sigma factor | SpoIIIC [Bacillus licheniformis DSM 13] | UniRef100_Q65H24 | Bacillus licheniformis DSM 13 |
| 3224 | BLP03075 ABC transport protein | ABC transporter, ATP-binding protein; possible subtilin transporter [Bacillus thuringiensis] | UniRef100_Q6HGQ9 | Bacillus thuringiensis |
| 3225 | BLP04901 conserved hypothetical protein | UPI00003163AD UniRef100 entry | UniRef100_UPI00003163AD | |
| 3226 | BLP04902 hypothetical protein | | | |
| 3227 | BLP03076 hypothetical protein | | | |
| 3228 | BLP03077 SpoIP homolog protein | Hypothetical protein ORF1 [Bacillus megaterium] | UniRef100_O87702 | Bacillus megaterium |
| 3229 | BLP03078 hypothetical protein | | | |
| 3230 | | | | |
| 3231 | | | | |
| 3232 | BLP03079 spoIIIC truncated SpoIIIC | SpoIIIC [Bacillus licheniformis DSM 13] | UniRef100_Q65H24 | Bacillus licheniformis DSM 13 |
| 3233 | BLP03080 conserved hypothetical protein | Hypothetical protein [Bacillus licheniformis DSM 13] | UniRef100_Q65H23 | Bacillus licheniformis DSM 13 |
| 3234 | BLP03081 GCN5-related N-acetyltransferase | Hypothetical protein [Bacillus licheniformis DSM 13] | UniRef100_Q65H22 | Bacillus licheniformis DSM 13 |
| 3235 | BLP03082 conserved hypothetical protein | Hypothetical protein [Bacillus licheniformis DSM 13] | UniRef100_Q62SH6 | Bacillus licheniformis DSM 13 |
| 3236 | BLP03083 partial SpoIIIC | Hypothetical protein [Bacillus licheniformis DSM 13] | UniRef100_Q65H21 | Bacillus licheniformis DSM 13 |
| 3237 | BLP03085 alanyl-tRNA synthetase | Hypothetical protein [Bacillus licheniformis DSM 13] | UniRef100_Q65H19 | Bacillus licheniformis DSM 13 |
| 3238 | BLP03086 putative alanine racemase | Hypothetical protein [Bacillus licheniformis DSM 13] | UniRef100_Q65H18 | Bacillus licheniformis DSM 13 |
| 3239 | yabJB putative translation initiation inhibitor | Hypothetical protein [Bacillus licheniformis DSM 13] | UniRef100_Q65H17 | Bacillus licheniformis DSM 13 |
| 3240 | ansZA putative Asparaginase_glutaminase | YccC1 [Bacillus licheniformis DSM 13] | UniRef100_Q65H16 | Bacillus licheniformis DSM 13 |
| 3241 | thrCA threonine synthase | Hypothetical protein [Bacillus licheniformis DSM 13] | UniRef100_Q65H14 | Bacillus licheniformis DSM 13 |
| 3242 | yabJA putative Endoribonuclease L-PSP | Hypothetical protein [Bacillus licheniformis DSM 13] | UniRef100_Q65H13 | Bacillus licheniformis DSM 13 |
| 3243 | BLP03091 hypothetical DNA binding protein | DctR [Bacillus licheniformis DSM 13] | UniRef100_Q65H12 | Bacillus licheniformis DSM 13 |

| 3244 | BLP03092 hypothetical protein | | | |
|---|---|---|---|---|
| 3245 | spoIIICA RNA polymerase sporulation-specific sigma factor (sigma-K) (C-terminal half) | Hypothetical protein [Bacillus licheniformis DSM 13] | UniRef100_Q65H11 | Bacillus licheniformis DSM 13 |
| 3246 | BLP03095 putative Flavoredoxin | Hypothetical protein [Bacillus licheniformis DSM 13] | UniRef100_Q65H10 | Bacillus licheniformis DSM 13 |
| 3247 | bmrR transcriptional regulator | BmrR [Bacillus licheniformis DSM 13] | UniRef100_Q65H09 | Bacillus licheniformis DSM 13 |
| 3248 | BLP03097 hypothetical protein | | | |
| 3249 | BLP03098 putative Hydroxyacylglutathione hydrolase | Hypothetical protein [Bacillus licheniformis DSM 13] | UniRef100_Q65H08 | Bacillus licheniformis DSM 13 |
| 3250 | BLP03099 putatice Sulfide-quinone reductase | Hypothetical protein [Bacillus licheniformis DSM 13] | UniRef100_Q65H07 | Bacillus licheniformis DSM 13 |
| 3251 | BLP03100 two-component response regulator | Hypothetical protein [Bacillus licheniformis DSM 13] | UniRef100_Q65H06 | Bacillus licheniformis DSM 13 |
| 3252 | BLP03101 two-component sensor histidine kinase | Hypothetical protein [Bacillus licheniformis DSM 13] | UniRef100_Q65H05 | Bacillus licheniformis DSM 13 |
| 3253 | ydfQ Cytochrome c  heme-binding protein YdfQ | YdfQ [Bacillus licheniformis DSM 13] | UniRef100_Q65H04 | Bacillus licheniformis DSM 13 |
| 3254 | yrkJ conserved membrane protein YrkJ | Hypothetical protein yrkJ [Bacillus licheniformis DSM 13] | UniRef100_Q65H03 | Bacillus licheniformis DSM 13 |
| 3255 | yrkI conserved protein YrkI | YrkI [Bacillus licheniformis DSM 13] | UniRef100_Q65H02 | Bacillus licheniformis DSM 13 |
| 3256 | yrkH conserved protein YrkH | YrkH [Bacillus licheniformis DSM 13] | UniRef100_Q65H01 | Bacillus licheniformis DSM 13 |
| 3257 | yrkF YrkF | YrkF [Bacillus licheniformis DSM 13] | UniRef100_Q65H00 | Bacillus licheniformis DSM 13 |
| 3258 | yrkE YrkE | Hypothetical protein yrkE [Bacillus licheniformis DSM 13] | UniRef100_Q65GZ9 | Bacillus licheniformis DSM 13 |
| 3259 | BLP03108 hypothetical protein | | | |
| 3260 | yrkD conserved protein YrkD | Hypothetical protein yrkD [Bacillus licheniformis DSM 13] | UniRef100_Q65GZ8 | Bacillus licheniformis DSM 13 |
| 3261 | BLP03110 hypothetical protein | | | |
| 3262 | BLP03111 SAM dependent methyltransferase | Hypothetical protein (SAM (And some other nucleotide) binding motif, Generic methyltransferase) [Bacillus licheniformis DSM 13] | UniRef100_Q65GZ7 | Bacillus licheniformis DSM 13 |
| 3263 | BLP03112 conserved hypothetical protein | Hypothetical protein [Bacillus licheniformis DSM 13] | UniRef100_Q65GZ6 | Bacillus licheniformis DSM 13 |
| 3264 | BLP03113 conserved hypothetical protein | Hypothetical protein [Bacillus licheniformis DSM 13] | UniRef100_Q65GZ5 | Bacillus licheniformis DSM 13 |
| 3265 | BLP03114 putative transport system permease protein | Hypothetical protein [Bacillus licheniformis DSM 13] | UniRef100_Q65GZ3 | Bacillus licheniformis DSM 13 |
| 3266 | BLP03116 putative carbohydrate esterase family 6 protein | Hypothetical protein [Bacillus licheniformis DSM 13] | UniRef100_Q65GZ2 | Bacillus licheniformis DSM 13 |
| 3267 | ytjAA conserved protein YtjAA | Hypothetical protein ytjAA [Bacillus licheniformis DSM 13] | UniRef100_Q65GZ1 | Bacillus licheniformis DSM 13 |
| 3268 | BLP03118 delta-aminolevulinic acid dehydratase (porphobilinogen synthase) | Hypothetical protein [Bacillus licheniformis DSM 13] | UniRef100_Q65GZ0 | Bacillus licheniformis DSM 13 |
| 3269 | hxlB 6-phospho-3-hexuloisomerase HxlB | HxlB [Bacillus licheniformis DSM 13] | UniRef100_Q65GY9 | Bacillus licheniformis DSM 13 |
| 3270 | hxlA 3-hexulose-6-phosphate synthase HxlA | HxlA [Bacillus licheniformis DSM 13] | UniRef100_Q65GY8 | Bacillus licheniformis DSM 13 |
| 3271 | hxlR DNA-binding protein, transcriptional regulator HxlR | Hypothetical protein hxlR [Bacillus licheniformis DSM 13] | UniRef100_Q65GY7 | Bacillus licheniformis DSM 13 |
| 3272 | des fatty acid desaturase | Des [Bacillus licheniformis DSM 13] | UniRef100_Q65GY6 | Bacillus licheniformis DSM 13 |
| 3273 | desK, yocF DesK two-component system sensor kinase | YocF [Bacillus licheniformis DSM 13] | UniRef100_Q65GY5 | Bacillus licheniformis DSM 13 |
| 3274 | desR two-component response regulator responsible for cold induction of the des gene | YocG [Bacillus licheniformis DSM 13] | UniRef100_Q65GY4 | Bacillus licheniformis DSM 13 |

| 3275 | ycgT FAD-dependent pyridine nucleotide-disulphide oxidoreductase YcgT | YcgT [Bacillus licheniformis DSM 13] | UniRef100_Q65GY3 | Bacillus licheniformis DSM 13 |
|---|---|---|---|---|
| 3276 | appAB oligopeptide ABC transporter (oligopeptide-binding protein) | Hypothetical protein [Bacillus licheniformis DSM 13] | UniRef100_Q65GY2 | Bacillus licheniformis DSM 13 |
| 3277 | appBB oligopeptide ABC transporter (permease) | Hypothetical protein [Bacillus licheniformis DSM 13] | UniRef100_Q65GY1 | Bacillus licheniformis DSM 13 |
| 3278 | appCB oligopeptide ABC transporter (permease) | Hypothetical protein [Bacillus licheniformis DSM 13] | UniRef100_Q65GY0 | Bacillus licheniformis DSM 13 |
| 3279 | appDB dipeptide ABC transporter (ATP-binding protein) | Hypothetical protein [Bacillus licheniformis DSM 13] | UniRef100_Q65GX9 | Bacillus licheniformis DSM 13 |
| 3280 | appFB oligopeptide ABC transporter (ATP-binding protein) | Hypothetical protein [Bacillus licheniformis DSM 13] | UniRef100_Q65GX8 | Bacillus licheniformis DSM 13 |
| 3281 | ydfL Putative DNA binding protein YdfL | YdfL [Bacillus licheniformis DSM 13] | UniRef100_Q65GX7 | Bacillus licheniformis DSM 13 |
| 3282 | matE Multi antimicrobial extrusion protein MatE | Hypothetical protein [Bacillus licheniformis DSM 13] | UniRef100_Q65GX6 | Bacillus licheniformis DSM 13 |
| 3283 | BLP03133 hypothetical protein | Hypothetical protein [Bacillus licheniformis DSM 13] | UniRef100_Q65GX5 | Bacillus licheniformis DSM 13 |
| 3284 | BLP03134 3-oxoacyl-[acyl-carrier-protein] reductase | Hypothetical protein [Bacillus licheniformis DSM 13] | UniRef100_Q65GX4 | Bacillus licheniformis DSM 13 |
| 3285 | BLP03135 putative phosphoesterase | Hypothetical protein [Bacillus licheniformis DSM 13] | UniRef100_Q65GX3 | Bacillus licheniformis DSM 13 |
| 3286 | BLP03136 Esterase_lipase_thioesterase family protein | Hypothetical protein [Bacillus licheniformis DSM 13] | UniRef100_Q65GX2 | Bacillus licheniformis DSM 13 |
| 3287 | BLP03137 hypothetical protein | | | |
| 3288 | cypA cytochrome P450-like enzyme | CypA [Bacillus licheniformis DSM 13] | UniRef100_Q65GX1 | Bacillus licheniformis DSM 13 |
| 3289 | ytnM conserved membrane protein YtnM | Hypothetical protein ytnM [Bacillus licheniformis DSM 13] | UniRef100_Q65GX0 | Bacillus licheniformis DSM 13 |
| 3290 | yndA conserved hypothetical protein | YndA [Bacillus licheniformis DSM 13] | UniRef100_Q65GW9 | Bacillus licheniformis DSM 13 |
| 3291 | BLP04772 hypothetical protein | | | |
| 3292 | yvaG putative 3-oxoacyl- acyl-carrier protein reductase YvaG | YvaG [Bacillus licheniformis DSM 13] | UniRef100_Q65GW8 | Bacillus licheniformis DSM 13 |
| 3293 | sacC Glycoside Hydrolase Family 32 | SacC [Bacillus licheniformis DSM 13] | UniRef100_Q65GW7 | Bacillus licheniformis DSM 13 |
| 3294 | levG phosphotransferase system (PTS) fructose-specific enzyme IID component | LevG (Phosphotransferase system (PTS) fructose-specific enzyme IID component) [Bacillus licheniformis DSM 13] | UniRef100_Q65GW6 | Bacillus licheniformis DSM 13 |
| 3295 | levF phosphotransferase system (PTS) fructose-specific enzyme IIC component | LevF (Phosphotransferase system (PTS) fructose-specific enzyme IIC component) [Bacillus licheniformis DSM 13] | UniRef100_Q65GW5 | Bacillus licheniformis DSM 13 |
| 3296 | levE phosphotransferase system (PTS) fructose-specific enzyme IIB component | LevE (Phosphotransferase system (PTS) fructose-specific enzyme IIB component) [Bacillus licheniformis DSM 13] | UniRef100_Q65GW4 | Bacillus licheniformis DSM 13 |
| 3297 | levD phosphotransferase system (PTS) fructose-specific enzyme IIA component | LevD (Phosphotransferase system (PTS) fructose-specific enzyme IIA component) [Bacillus licheniformis DSM 13] | UniRef100_Q65GW3 | Bacillus licheniformis DSM 13 |
| 3298 | levR transcriptional regulator (NifA_NtrC family) | LevR [Bacillus licheniformis DSM 13] | UniRef100_Q65GW2 | Bacillus licheniformis DSM 13 |
| 3299 | BLP03148 hypothetical protein | | | |
| 3300 | BLP03149 conserved hypothetical protein | Hypothetical protein [Bacillus licheniformis DSM 13] | UniRef100_Q65GW1 | Bacillus licheniformis DSM 13 |
| 3301 | yrhK YrhK | Hypothetical protein yrhK [Bacillus licheniformis DSM 13] | UniRef100_Q65GW0 | Bacillus licheniformis DSM 13 |
| 3302 | cat chloramphenicol O-acetyltransferase | Hypothetical protein [Bacillus licheniformis DSM 13] | UniRef100_Q65GV9 | Bacillus licheniformis DSM 13 |
| 3303 | BLP04773 hypothetical protein | | | |
| 3304 | adaA methylphosphotriester-DNA alkyltransferase and transcriptional regulator (AraC_XylS family) | AdaA [Bacillus licheniformis DSM 13] | UniRef100_Q65GV8 | Bacillus licheniformis DSM 13 |

| 3305 | adaB O6-methylguanine-DNA methyltransferase | AdaB [Bacillus licheniformis DSM 13] | UniRef100_Q65GV7 | Bacillus licheniformis DSM 13 |
|---|---|---|---|---|
| 3306 | ytbE Aldo_keto reductase YtbE | YtbE [Bacillus licheniformis DSM 13] | UniRef100_Q65GV6 | Bacillus licheniformis DSM 13 |
| 3307 | ytbD PTS system protein YtbD | YtbD [Bacillus licheniformis DSM 13] | UniRef100_Q65GV5 | Bacillus licheniformis DSM 13 |
| 3308 | ytcD possible transcriptional regulator YtcD | YtcD [Bacillus licheniformis DSM 13] | UniRef100_Q65GV4 | Bacillus licheniformis DSM 13 |
| 3309 | BLP03157 putative serine-type peptidase | Hypothetical protein [Bacillus licheniformis DSM 13] | UniRef100_Q65GV3 | Bacillus licheniformis DSM 13 |
| 3310 | BLP03158 hypothetical protein | | | |
| 3311 | BLP03159 hypothetical protein | | | |
| 3312 | yjjA Putative Uroporphyrinogen III synthase YjjA | YjjA [Bacillus licheniformis DSM 13] | UniRef100_Q65GV2 | Bacillus licheniformis DSM 13 |
| 3313 | BLP03161 conserved hypothetical protein | Hypothetical protein [Bacillus licheniformis DSM 13] | UniRef100_Q65GV1 | Bacillus licheniformis DSM 13 |
| 3314 | BLP03162 Periplasmic iron-binding protein | Hypothetical protein [Bacillus licheniformis DSM 13] | UniRef100_Q65GV0 | Bacillus licheniformis DSM 13 |
| 3315 | yybE transcriptional regulator YbbE | YybE [Bacillus licheniformis DSM 13] | UniRef100_Q65GU9 | Bacillus licheniformis DSM 13 |
| 3316 | yybF Sugar transporter superfamily protein YybF | YybF [Bacillus licheniformis DSM 13] | UniRef100_Q65GU8 | Bacillus licheniformis DSM 13 |
| 3317 | BLP03165 conserved hypothetical protein | Hypothetical protein [Bacillus licheniformis DSM 13] | UniRef100_Q65GU7 | Bacillus licheniformis DSM 13 |
| 3318 | cypE cytochrome P450 _ NADPH-ferrihemoprotein reductase | YrhJ [Bacillus licheniformis DSM 13] | UniRef100_Q65GU6 | Bacillus licheniformis DSM 13 |
| 3319 | yrhI transcriptional regulator | YrhI [Bacillus licheniformis DSM 13] | UniRef100_Q65GU5 | Bacillus licheniformis DSM 13 |
| 3320 | wprA cell wall-associated protease | Hypothetical protein [Bacillus licheniformis DSM 13] | UniRef100_Q65GU4 | Bacillus licheniformis DSM 13 |
| 3321 | BLP03169 hypothetical protein | | | |
| 3322 | yrhH putative SAM dependent methyltransferase YrrH | Hypothetical protein (SAM (And some other nucleotide) binding motif) [Bacillus licheniformis DSM 13] | UniRef100_Q65GU3 | Bacillus licheniformis DSM 13 |
| 3323 | BLP03171 conserved hypothetical protein | | | |
| 3324 | BLP03172 hypothetical protein | | | |
| 3325 | yrzI YrzI | Hypothetical protein yrzI [Bacillus licheniformis DSM 13] | UniRef100_Q62SA2 | Bacillus licheniformis DSM 13 |
| 3326 | yrhC YrhC | YrhC [Bacillus licheniformis DSM 13] | UniRef100_Q65GU2 | Bacillus licheniformis DSM 13 |
| 3327 | yrhB cystathionine gamma-lyase YrhB | YrhB [Bacillus licheniformis DSM 13] | UniRef100_Q65GU1 | Bacillus licheniformis DSM 13 |
| 3328 | yrhA putative cysteine synthase YrhA | YrhA [Bacillus licheniformis DSM 13] | UniRef100_Q65GU0 | Bacillus licheniformis DSM 13 |
| 3329 | mtn methylthioadenosine nucleosidase | Mtn [Bacillus licheniformis DSM 13] | UniRef100_Q65GT9 | Bacillus licheniformis DSM 13 |
| 3330 | yrrT probable S-adenosylmethionine-dependent methyltransferase YrrT | YrrT (SAM (And some other nucleotide) binding motif,Generic methyltransferase) [Bacillus licheniformis DSM 13] | UniRef100_Q65GT8 | Bacillus licheniformis DSM 13 |
| 3331 | yrzA conserved protein YrzA | Hypothetical protein yrzA [Bacillus licheniformis DSM 13] | UniRef100_Q62S96 | Bacillus licheniformis DSM 13 |
| 3332 | yrrS conserved protein YrrS | YrrS [Bacillus licheniformis DSM 13] | UniRef100_Q65GT6 | Bacillus licheniformis DSM 13 |
| 3333 | BLP03181 hypothetical protein | | | |
| 3334 | yrrR Penicillin-binding protein YrrR | YrrR [Bacillus licheniformis DSM 13] | UniRef100_Q65GT5 | Bacillus licheniformis DSM 13 |
| 3335 | greA transcription elongation factor | GreA [Bacillus licheniformis DSM 13] | UniRef100_Q65GT4 | Bacillus licheniformis DSM 13 |
| 3336 | udk uridine kinase | Udk [Bacillus licheniformis DSM 13] | UniRef100_Q65GT3 | Bacillus licheniformis DSM 13 |
| 3337 | yrrO putative protease YrrO | YrrO [Bacillus licheniformis DSM 13] | UniRef100_Q65GT2 | Bacillus licheniformis DSM 13 |
| 3338 | yrrN Calcium-binding protein YrrN | YrrN [Bacillus licheniformis DSM 13] | UniRef100_Q65GT1 | Bacillus licheniformis DSM 13 |

| 3339 | yrrM SAM-dependent methyltransferase YrrM | YrrM (SAM (And some other nucleotide) binding motif) [Bacillus licheniformis DSM 13] | UniRef100_Q65GT0 | Bacillus licheniformis DSM 13 |
|---|---|---|---|---|
| 3340 | yrrL conserved protein YrrL | Hypothetical protein yrrL [Bacillus licheniformis DSM 13] | UniRef100_Q65GS9 | Bacillus licheniformis DSM 13 |
| 3341 | yrzB conserved protein YrzB | Hypothetical protein yrzB [Bacillus licheniformis DSM 13] | UniRef100_Q62S87 | Bacillus licheniformis DSM 13 |
| 3342 | yrrK conserved protein YrrK | Hypothetical protein yrrK [Bacillus licheniformis DSM 13] | UniRef100_Q65GS7 | Bacillus licheniformis DSM 13 |
| 3343 | yrzL conserved protein YrzL | Hypothetical protein yrzL [Bacillus licheniformis DSM 13] | UniRef100_Q65GS6 | Bacillus licheniformis DSM 13 |
| 3344 | alaS alanyl-tRNA synthetase | AlaS [Bacillus licheniformis DSM 13] | UniRef100_Q65GS5 | Bacillus licheniformis DSM 13 |
| 3345 | yrrI conserved protein YrrI | Hypothetical protein yrrI [Bacillus licheniformis DSM 13] | UniRef100_Q65GS4 | Bacillus licheniformis DSM 13 |
| 3346 | BLP03195 conserved hypothetical protein | Hypothetical protein [Bacillus licheniformis DSM 13] | UniRef100_Q65GS3 | Bacillus licheniformis DSM 13 |
| 3347 | yrrD Photosynthetic reaction centre, H-chain, cytoplasmic | YrrD [Bacillus licheniformis DSM 13] | UniRef100_Q65GS2 | Bacillus licheniformis DSM 13 |
| 3348 | yrrC putative exodeoxyribonuclease V alpha subunit YrrC | Helicase RecD/TraA [Bacillus licheniformis DSM 13] | UniRef100_Q62S80 | Bacillus licheniformis DSM 13 |
| 3349 | yrrB conserved protein YrrB | YrrB [Bacillus licheniformis DSM 13] | UniRef100_Q65GS0 | Bacillus licheniformis DSM 13 |
| 3350 | trmU tRNA (5-methylaminomethyl-2-thiouridylate) methyltransferase | TrmU (TRNA (5-methylaminomethyl-2-thiouridylate) methyltransferase) [Bacillus licheniformis DSM 13] | UniRef100_Q65GR9 | Bacillus licheniformis DSM 13 |
| 3351 | yrvO Aminotransferase, class V | YrvO [Bacillus licheniformis DSM 13] | UniRef100_Q65GR8 | Bacillus licheniformis DSM 13 |
| 3352 | yrzC negative regulator of riboflavin kinase YrzC | Hypothetical protein yrzC [Bacillus licheniformis DSM 13] | UniRef100_Q65GR7 | Bacillus licheniformis DSM 13 |
| 3353 | yrvN Replication factor C conserved domain | YrvN [Bacillus licheniformis DSM 13] | UniRef100_Q65GR6 | Bacillus licheniformis DSM 13 |
| 3354 | yrvM Molybdenum cofactor biosynthesis | YrvM [Bacillus licheniformis DSM 13] | UniRef100_Q65GR5 | Bacillus licheniformis DSM 13 |
| 3355 | BLP03204 hypothetical protein | | | |
| 3356 | aspS aspartyl-tRNA synthetase | AspS [Bacillus licheniformis DSM 13] | UniRef100_Q65GR4 | Bacillus licheniformis DSM 13 |
| 3357 | hisS histidyl-tRNA synthetase | HisS [Bacillus licheniformis DSM 13] | UniRef100_Q65GR3 | Bacillus licheniformis DSM 13 |
| 3358 | yrzK YrzK | Hypothetical protein yrzK [Bacillus licheniformis DSM 13] | UniRef100_Q62S71 | Bacillus licheniformis DSM 13 |
| 3359 | yrvJ N-acetylmuramoyl-L-alanine amidase YrvJ | YrvJ1 [Bacillus licheniformis DSM 13] | UniRef100_Q65GR1 | Bacillus licheniformis DSM 13 |
| 3360 | yrvI D-tyrosyl-tRNA(Tyr) deacylase | YrvI (D-tyrosyl-tRNA(Tyr) deacylase) [Bacillus licheniformis DSM 13] | UniRef100_Q65GR0 | Bacillus licheniformis DSM 13 |
| 3361 | relA GTP pyrophosphokinase | RelA [Bacillus licheniformis DSM 13] | UniRef100_Q65GQ9 | Bacillus licheniformis DSM 13 |
| 3362 | BLP03211 hypothetical protein | | | |
| 3363 | apt adenine phosphoribosyltransferase | Apt [Bacillus licheniformis DSM 13] | UniRef100_Q65GQ8 | Bacillus licheniformis DSM 13 |
| 3364 | recJ RecJ exonuclease | YrvE [Bacillus licheniformis DSM 13] | UniRef100_Q65GQ7 | Bacillus licheniformis DSM 13 |
| 3365 | yrvD conserved protein YrvD | Hypothetical protein yrvD [Bacillus licheniformis DSM 13] | UniRef100_Q65GQ6 | Bacillus licheniformis DSM 13 |
| 3366 | secDF protein-export membrane protein SecDF | SecDF [Bacillus licheniformis DSM 13] | UniRef100_Q65GQ5 | Bacillus licheniformis DSM 13 |
| 3367 | yrzD conserved protein YrzD | Hypothetical protein yrzD [Bacillus licheniformis DSM 13] | UniRef100_Q65GQ4 | Bacillus licheniformis DSM 13 |
| 3368 | spoVB SpoVB | SpoVB [Bacillus licheniformis DSM 13] | UniRef100_Q65GQ3 | Bacillus licheniformis DSM 13 |
| 3369 | yrbG conserved membrane protein YrbG | Hypothetical protein yrbG [Bacillus licheniformis DSM 13] | UniRef100_Q65GQ2 | Bacillus licheniformis DSM 13 |
| 3370 | yrzE conserved membrane protein YrzE | Hypothetical protein yrzE [Bacillus licheniformis DSM 13] | UniRef100_Q65GQ1 | Bacillus licheniformis DSM 13 |
| 3371 | BLP04774 hypothetical protein | | | |

| 3372 | yrbF preprotein translocase subunit YrbF | YrbF [Bacillus licheniformis DSM 13] | UniRef100_Q65GQ0 | Bacillus licheniformis DSM 13 |
|---|---|---|---|---|
| 3373 | tgt tRNA-guanine transglycosylase | Tgt [Bacillus licheniformis DSM 13] | UniRef100_Q65GP9 | Bacillus licheniformis DSM 13 |
| 3374 | queA S-adenosylmethionine tRNA ribosyltransferase | QueA [Bacillus licheniformis DSM 13] | UniRef100_Q65GP8 | Bacillus licheniformis DSM 13 |
| 3375 | BLP03223 conserved hypothetical protein | Hypothetical protein [Bacillus licheniformis DSM 13] | UniRef100_Q65GP7 | Bacillus licheniformis DSM 13 |
| 3376 | ruvB Holliday junction DNA helicase | RuvB [Bacillus licheniformis DSM 13] | UniRef100_Q65GP6 | Bacillus licheniformis DSM 13 |
| 3377 | ruvA Holliday junction DNA helicase | RuvA [Bacillus licheniformis DSM 13] | UniRef100_Q65GP5 | Bacillus licheniformis DSM 13 |
| 3378 | bofC BofC | Hypothetical protein bofC [Bacillus licheniformis DSM 13] | UniRef100_Q65GP4 | Bacillus licheniformis DSM 13 |
| 3379 | yrzF probable serine_threonine-protein kinase YrzF | YrzF [Bacillus licheniformis DSM 13] | UniRef100_Q65GP3 | Bacillus licheniformis DSM 13 |
| 3380 | BLP03228 conserved hypothetical protein | Hypothetical protein [Bacillus licheniformis DSM 13] | UniRef100_Q62S51 | Bacillus licheniformis DSM 13 |
| 3381 | sspH small acid-soluble spore protein | SspH [Bacillus licheniformis DSM 13] | UniRef100_Q65GP2 | Bacillus licheniformis DSM 13 |
| 3382 | yjoA conserved hypothetical protein YjoA | YjoA [Bacillus licheniformis DSM 13] | UniRef100_Q65GP1 | Bacillus licheniformis DSM 13 |
| 3383 | ymaC phage-related protein YmaC | Hypothetical protein ymaC [Bacillus licheniformis DSM 13] | UniRef100_Q65GP0 | Bacillus licheniformis DSM 13 |
| 3384 | BLP03232 conserved hypothetical protein | Hypothetical protein [Bacillus licheniformis DSM 13] | UniRef100_Q65GN9 | Bacillus licheniformis DSM 13 |
| 3385 | BLP03233 conserved hypothetical protein | Hypothetical protein [Bacillus licheniformis DSM 13] | UniRef100_Q65GN8 | Bacillus licheniformis DSM 13 |
| 3386 | yrbC conserved protein YrbC | Hypothetical protein yrbC [Bacillus licheniformis DSM 13] | UniRef100_Q65GN7 | Bacillus licheniformis DSM 13 |
| 3387 | coxA spore cortex protein | CoxA [Bacillus licheniformis DSM 13] | UniRef100_Q65GN6 | Bacillus licheniformis DSM 13 |
| 3388 | safA morphogenetic protein associated with SpoVID | SafA [Bacillus licheniformis DSM 13] | UniRef100_Q65GN5 | Bacillus licheniformis DSM 13 |
| 3389 | nadA quinolinate synthetase | NadA [Bacillus licheniformis DSM 13] | UniRef100_Q65GN4 | Bacillus licheniformis DSM 13 |
| 3390 | nadC nicotinate-nucleotide pyrophosphorylase | NadC [Bacillus licheniformis DSM 13] | UniRef100_Q65GN3 | Bacillus licheniformis DSM 13 |
| 3391 | nadB L-aspartate oxidase | NadB [Bacillus licheniformis DSM 13] | UniRef100_Q65GN2 | Bacillus licheniformis DSM 13 |
| 3392 | nifS NifS | Hypothetical protein nifS [Bacillus licheniformis DSM 13] | UniRef100_Q65GN1 | Bacillus licheniformis DSM 13 |
| 3393 | nadR putative regulator of de-novo NAD biosythesis NadR | Winged helix DNA-binding [Bacillus licheniformis DSM 13] | UniRef100_Q62S38 | Bacillus licheniformis DSM 13 |
| 3394 | pheA prephenate dehydratase | PheA [Bacillus licheniformis DSM 13] | UniRef100_Q65GM9 | Bacillus licheniformis DSM 13 |
| 3395 | pheB chorismate mutase | PheB [Bacillus licheniformis DSM 13] | UniRef100_Q65GM8 | Bacillus licheniformis DSM 13 |
| 3396 | obg GTPase Obg | Obg [Bacillus licheniformis DSM 13] | UniRef100_Q65GM7 | Bacillus licheniformis DSM 13 |
| 3397 | spo0B sporulation initiation phosphotransferase | Spo0B [Bacillus licheniformis DSM 13] | UniRef100_Q65GM6 | Bacillus licheniformis DSM 13 |
| 3398 | rpmA ribosomal protein L27 (BL24) | RpmA [Bacillus licheniformis DSM 13] | UniRef100_Q65GM5 | Bacillus licheniformis DSM 13 |
| 3399 | ysxB hypothetical protein | YsxB [Bacillus licheniformis DSM 13] | UniRef100_Q65GM4 | Bacillus licheniformis DSM 13 |
| 3400 | rplU ribosomal protein L21 (BL20) | RplU [Bacillus licheniformis DSM 13] | UniRef100_Q65GM3 | Bacillus licheniformis DSM 13 |
| 3401 | spoIVFB membrane metalloprotease | SpoIVFB [Bacillus licheniformis DSM 13] | UniRef100_Q65GM2 | Bacillus licheniformis DSM 13 |
| 3402 | spoIVFA SpoIVFA | Hypothetical protein spoIVFA [Bacillus licheniformis DSM 13] | UniRef100_Q65GM1 | Bacillus licheniformis DSM 13 |
| 3403 | yndB conserved protein YndB | Hypothetical protein yndB [Bacillus licheniformis DSM 13] | UniRef100_Q65GM0 | Bacillus licheniformis DSM 13 |
| 3404 | BLP03252 hypothetical DNA binding protein | Hypothetical protein [Bacillus licheniformis DSM 13] | UniRef100_Q65GL9 | Bacillus licheniformis DSM 13 |
| 3405 | minD ATPase activator of MinC | MinD [Bacillus licheniformis DSM 13] | UniRef100_Q65GL8 | Bacillus licheniformis DSM 13 |
| 3406 | minC MinC | MinC [Bacillus licheniformis DSM 13] | UniRef100_Q65GL7 | Bacillus licheniformis DSM 13 |

| 3407 | mreD cell-shape determining protein | MreD [Bacillus licheniformis DSM 13] | UniRef100_Q65GL6 | Bacillus licheniformis DSM 13 |
|---|---|---|---|---|
| 3408 | mreC cell-shape determining protein | MreC [Bacillus licheniformis DSM 13] | UniRef100_Q65GL5 | Bacillus licheniformis DSM 13 |
| 3409 | mreB cell-shape determining protein | MreB [Bacillus licheniformis DSM 13] | UniRef100_Q65GL4 | Bacillus licheniformis DSM 13 |
| 3410 | radC DNA repair protein | RadC [Bacillus licheniformis DSM 13] | UniRef100_Q65GL3 | Bacillus licheniformis DSM 13 |
| 3411 | maf Maf | Hypothetical protein maf [Bacillus licheniformis DSM 13] | UniRef100_Q65GL2 | Bacillus licheniformis DSM 13 |
| 3412 | spoIIB SpoIIB | Endospore development protein SpoIIB [Bacillus licheniformis] | UniRef100_Q65GL1 | Bacillus licheniformis |
| 3413 | comC DNA-binding protein | Late competence protein ComC [Bacillus licheniformis] | UniRef100_Q62S18 | Bacillus licheniformis |
| 3414 | folC folyl-polyglutamate synthetase | FolC [Bacillus licheniformis DSM 13] | UniRef100_Q65GK9 | Bacillus licheniformis DSM 13 |
| 3415 | BLP03264 conserved hypothetical protein | Hypothetical protein [Bacillus licheniformis DSM 13] | UniRef100_Q65GK7 | Bacillus licheniformis DSM 13 |
| 3416 | ysxE conserved protein YsxE | YsxE [Bacillus licheniformis DSM 13] | UniRef100_Q65GK6 | Bacillus licheniformis DSM 13 |
| 3417 | spoVID SpoVID | Hypothetical protein spoVID [Bacillus licheniformis DSM 13] | UniRef100_Q65GK5 | Bacillus licheniformis DSM 13 |
| 3418 | hemL glutamate-1-semialdehyde 2,1-aminotransferase | HemL [Bacillus licheniformis DSM 13] | UniRef100_Q65GK4 | Bacillus licheniformis DSM 13 |
| 3419 | hemB delta-aminolevulinic acid dehydratase (porphobilinogen synthase) | HemB [Bacillus licheniformis DSM 13] | UniRef100_Q65GK3 | Bacillus licheniformis DSM 13 |
| 3420 | hemD uroporphyrinogen III cosynthase | HemD [Bacillus licheniformis DSM 13] | UniRef100_Q65GK2 | Bacillus licheniformis DSM 13 |
| 3421 | hemC porphobilinogen deaminase (hydroxymethylbilane synthase) | HemC [Bacillus licheniformis DSM 13] | UniRef100_Q65GK1 | Bacillus licheniformis DSM 13 |
| 3422 | hemX membrane-bound protein | HemX [Bacillus licheniformis DSM 13] | UniRef100_Q65GK0 | Bacillus licheniformis DSM 13 |
| 3423 | hemA glutamyl-tRNA reductase | HemA [Bacillus licheniformis DSM 13] | UniRef100_Q65GJ9 | Bacillus licheniformis DSM 13 |
| 3424 | BLP04775 hypothetical protein | | | |
| 3425 | ysxD YsxD | YsxD [Bacillus licheniformis DSM 13] | UniRef100_Q65GJ8 | Bacillus licheniformis DSM 13 |
| 3426 | engB GTP-binding domain,Small GTP-binding protein domain | YsxC [Bacillus licheniformis DSM 13] | UniRef100_Q65GJ7 | Bacillus licheniformis DSM 13 |
| 3427 | lonA class III heat-shock ATP-dependent Lon protease | LonA [Bacillus licheniformis DSM 13] | UniRef100_Q65GJ6 | Bacillus licheniformis DSM 13 |
| 3428 | lonB Lon-like ATP-dependent protease | LonB [Bacillus licheniformis DSM 13] | UniRef100_Q65GJ5 | Bacillus licheniformis DSM 13 |
| 3429 | BLP03277 hypothetical protein | | | |
| 3430 | clpX ATP-dependent Clp protease ATP-binding subunit (class III heat-shock protein) | ClpX [Bacillus licheniformis DSM 13] | UniRef100_Q65GJ4 | Bacillus licheniformis DSM 13 |
| 3431 | BLP03279 hypothetical protein | | | |
| 3432 | tig trigger factor (prolyl isomerase) | Tig [Bacillus licheniformis DSM 13] | UniRef100_Q65GJ3 | Bacillus licheniformis DSM 13 |
| 3433 | ysoA conserved protein YsoA | YsoA [Bacillus licheniformis DSM 13] | UniRef100_Q65GJ2 | Bacillus licheniformis DSM 13 |
| 3434 | BLP03282 hypothetical protein | | | |
| 3435 | leuD 3-isopropylmalate dehydratase (small subunit) | LeuD [Bacillus licheniformis DSM 13] | UniRef100_Q65GJ1 | Bacillus licheniformis DSM 13 |
| 3436 | leuC 3-isopropylmalate dehydratase (large subunit) | LeuC [Bacillus licheniformis DSM 13] | UniRef100_Q65GJ0 | Bacillus licheniformis DSM 13 |
| 3437 | leuB 3-isopropylmalate dehydrogenase | LeuB [Bacillus licheniformis DSM 13] | UniRef100_Q65GI9 | Bacillus licheniformis DSM 13 |
| 3438 | leuA 2-isopropylmalate synthase | LeuA [Bacillus licheniformis DSM 13] | UniRef100_Q65GI8 | Bacillus licheniformis DSM 13 |
| 3439 | ilvC ketol-acid reductoisomerase (acetohydroxy-acid isomeroreductase) | IlvC [Bacillus licheniformis DSM 13] | UniRef100_Q65GI7 | Bacillus licheniformis DSM 13 |
| 3440 | ilvH acetolactate synthase (acetohydroxy-acid | IlvH [Bacillus licheniformis DSM 13] | UniRef100_Q65GI6 | Bacillus licheniformis DSM 13 |

| | | | | |
|---|---|---|---|---|
| | synthase) (small subunit) | | | |
| 3441 | ilvB acetolactate synthase IlvB | IlvB [Bacillus licheniformis DSM 13] | UniRef100_Q65GI5 | Bacillus licheniformis DSM 13 |
| 3442 | BLP03290 hypothetical protein | | | |
| 3443 | BLP03291 putative branched-chain-amino-acid transaminase | Hypothetical protein [Bacillus licheniformis DSM 13] | UniRef100_Q65GI4 | Bacillus licheniformis DSM 13 |
| 3444 | BLP03292 conserved hypothetical protein | Hypothetical protein [Bacillus licheniformis DSM 13] | UniRef100_Q62RZ1 | Bacillus licheniformis DSM 13 |
| 3445 | BLP03293 putative glutamate dehydrogenase | RocG [Bacillus licheniformis DSM 13] | UniRef100_Q65GI2 | Bacillus licheniformis DSM 13 |
| 3446 | BLP04776 hypothetical protein | | | |
| 3447 | BLP03294 conserved hypothetical protein | Hypothetical protein [Bacillus licheniformis DSM 13] | UniRef100_Q65GI1 | Bacillus licheniformis DSM 13 |
| 3448 | BLP03295 conserved hypothetical protein | Hypothetical protein [Bacillus licheniformis DSM 13] | UniRef100_Q65GI0 | Bacillus licheniformis DSM 13 |
| 3449 | BLP03296 conserved hypothetical phage-like protein | Hypothetical protein [Bacillus licheniformis DSM 13] | UniRef100_Q65GH9 | Bacillus licheniformis DSM 13 |
| 3450 | yqaT Phage terminase, large subunit protein YqaT | YqaT [Bacillus licheniformis DSM 13] | UniRef100_Q65GH8 | Bacillus licheniformis DSM 13 |
| 3451 | BLP03298 conserved hypothetical phage protein | Hypothetical protein [Bacillus licheniformis DSM 13] | UniRef100_Q65GH7 | Bacillus licheniformis DSM 13 |
| 3452 | BLP03299 conserved hypothetical protein | Hypothetical protein [Bacillus licheniformis DSM 13] | UniRef100_Q65GH6 | Bacillus licheniformis DSM 13 |
| 3453 | BLP03300 conserved hypothetical protein | Hypothetical protein [Bacillus licheniformis DSM 13] | UniRef100_Q65GH5 | Bacillus licheniformis DSM 13 |
| 3454 | BLP03301 conserved hypothetical protein | Hypothetical protein [Bacillus licheniformis DSM 13] | UniRef100_Q65GH4 | Bacillus licheniformis DSM 13 |
| 3455 | mtbP modification methylase Bsu | Hypothetical protein [Bacillus licheniformis DSM 13] | UniRef100_Q65GH2 | Bacillus licheniformis DSM 13 |
| 3456 | BLP03304 putative DNA methylase | Hypothetical protein [Bacillus licheniformis DSM 13] | UniRef100_Q65GH1 | Bacillus licheniformis DSM 13 |
| 3457 | BLP03305 hypothetical protein | | | |
| 3458 | BLP03306 conserved hypothetical protein | Hypothetical protein [Bacillus cereus] | UniRef100_Q81BA9 | Bacillus cereus |
| 3459 | BLP03307 putative transposase | Hypothetical protein [Bacillus licheniformis DSM 13] | UniRef100_Q65GG9 | Bacillus licheniformis DSM 13 |
| 3460 | BLP03308 Response regulator aspartate phosphatase | Response regulator aspartate phosphatase [Bacillus licheniformis DSM 13] | UniRef100_Q62RX5 | Bacillus licheniformis DSM 13 |
| 3461 | BLP03309 hypothetical protein | Hypothetical protein [Bacillus licheniformis DSM 13] | UniRef100_Q65GG7 | Bacillus licheniformis DSM 13 |
| 3462 | BLP03310 phage protein | Hypothetical protein [Bacillus licheniformis DSM 13] | UniRef100_Q65GG6 | Bacillus licheniformis DSM 13 |
| 3463 | BLP03311 conserved hypothetical protein | Hypothetical protein [Bacillus licheniformis DSM 13] | UniRef100_Q62RX2 | Bacillus licheniformis DSM 13 |
| 3464 | ysnB Putative metallophosphoesterase YsnB | YsnB [Bacillus licheniformis DSM 13] | UniRef100_Q65GG4 | Bacillus licheniformis DSM 13 |
| 3465 | ysnA conserved protein YsnA | YsnA [Bacillus licheniformis DSM 13] | UniRef100_Q65GG3 | Bacillus licheniformis DSM 13 |
| 3466 | rph ribonuclease PH | Rph [Bacillus licheniformis DSM 13] | UniRef100_Q65GG2 | Bacillus licheniformis DSM 13 |
| 3467 | gerM spore germination protein GerM | Hypothetical protein gerM [Bacillus licheniformis DSM 13] | UniRef100_Q65GG1 | Bacillus licheniformis DSM 13 |
| 3468 | racE glutamate racemase | RacE [Bacillus licheniformis DSM 13] | UniRef100_Q65GG0 | Bacillus licheniformis DSM 13 |
| 3469 | ysmB probable transcriptional regulator YsmB | YsmB [Bacillus licheniformis DSM 13] | UniRef100_Q65GF9 | Bacillus licheniformis DSM 13 |
| 3470 | gerE transcriptional regulator | GerE [Bacillus licheniformis DSM 13] | UniRef100_Q65GF8 | Bacillus licheniformis DSM 13 |
| 3471 | BLP03319 hypothetical protein | | | |
| 3472 | BLP03320 Short-chain dehydrogenase_reductase SDR | Hypothetical protein [Bacillus licheniformis DSM 13] | UniRef100_Q65GF7 | Bacillus licheniformis DSM 13 |
| 3473 | ysmA conserved protein YsmA | YsmA [Bacillus licheniformis DSM 13] | UniRef100_Q65GF6 | Bacillus licheniformis DSM 13 |

| 3474 | sdhB succinate dehydrogenase (iron-sulfur protein) | SdhB [Bacillus licheniformis DSM 13] | UniRef100_Q65GF5 | Bacillus licheniformis DSM 13 |
|---|---|---|---|---|
| 3475 | sdhA succinate dehydrogenase (flavoprotein subunit) | SdhA [Bacillus licheniformis DSM 13] | UniRef100_Q65GF4 | Bacillus licheniformis DSM 13 |
| 3476 | sdhC succinate dehydrogenase (cytochrome b558 subunit) | SdhC [Bacillus licheniformis DSM 13] | UniRef100_Q65GF3 | Bacillus licheniformis DSM 13 |
| 3477 | yslB conserved protein YslB | Hypothetical protein yslB [Bacillus licheniformis DSM 13] | UniRef100_Q65GF2 | Bacillus licheniformis DSM 13 |
| 3478 | lysC aspartokinase II alpha subunit and beta subunit | LysC (Aspartokinase II alpha subunit (Aa 1->408) and beta subunit) [Bacillus licheniformis DSM 13] | UniRef100_Q65GF1 | Bacillus licheniformis DSM 13 |
| 3479 | uvrC excinuclease ABC (subunit C) | UvrC [Bacillus licheniformis DSM 13] | UniRef100_Q65GF0 | Bacillus licheniformis DSM 13 |
| 3480 | trxA thioredoxin | TrxA [Bacillus licheniformis DSM 13] | UniRef100_Q65GE9 | Bacillus licheniformis DSM 13 |
| 3481 | etfA electron transfer flavoprotein (alpha subunit) | EtfA [Bacillus licheniformis DSM 13] | UniRef100_Q65GE8 | Bacillus licheniformis DSM 13 |
| 3482 | etfB electron transfer flavoprotein (beta subunit) | EtfB [Bacillus licheniformis DSM 13] | UniRef100_Q65GE7 | Bacillus licheniformis DSM 13 |
| 3483 | ysiB Enoyl-CoA hydratase_isomerase YsiB | YsiB [Bacillus licheniformis DSM 13] | UniRef100_Q65GE6 | Bacillus licheniformis DSM 13 |
| 3484 | ysiA probable transcriptional regulator YsiA | YsiA [Bacillus licheniformis DSM 13] | UniRef100_Q65GE5 | Bacillus licheniformis DSM 13 |
| 3485 | lcfA long chain acyl-CoA synthetase | LcfA [Bacillus licheniformis DSM 13] | UniRef100_Q65GE4 | Bacillus licheniformis DSM 13 |
| 3486 | BLP04903 hypothetical protein | | | |
| 3487 | yshE conserved membrane protein YshE | Hypothetical protein yshE [Bacillus licheniformis DSM 13] | UniRef100_Q65GE3 | Bacillus licheniformis DSM 13 |
| 3488 | mutSB DNA mismatch repair protein MutSB | Hypothetical protein mutSB [Bacillus licheniformis DSM 13] | UniRef100_Q65GE2 | Bacillus licheniformis DSM 13 |
| 3489 | yshC putative DNA polymerase YshC | RuvA domain 2-like,DNA polymerase beta, N-terminal-like [Bacillus licheniformis DSM 13] | UniRef100_Q62RU7 | Bacillus licheniformis DSM 13 |
| 3490 | yshB conserved membrane protein YshB | YshB [Bacillus licheniformis DSM 13] | UniRef100_Q65GE0 | Bacillus licheniformis DSM 13 |
| 3491 | zapA ZapA | YshA [Bacillus licheniformis DSM 13] | UniRef100_Q65GD9 | Bacillus licheniformis DSM 13 |
| 3492 | rnhC ribonuclease HIII | RnhC [Bacillus licheniformis DSM 13] | UniRef100_Q65GD8 | Bacillus licheniformis DSM 13 |
| 3493 | BLP04942 hypothetical protein | | | |
| 3494 | BLP03340 conserved hypothetical protein | Hypothetical protein [Bacillus licheniformis DSM 13] | UniRef100_Q65GD7 | Bacillus licheniformis DSM 13 |
| 3495 | BLP03341 phenylalalanyl-tRNA synthetase (beta subunit) | Phenylalanyl-tRNA synthetase [Bacillus licheniformis DSM 13] | UniRef100_Q62RU1 | Bacillus licheniformis DSM 13 |
| 3496 | BLP04904 hypothetical protein | | | |
| 3497 | BLP03342 conserved hypothetical protein | Hypothetical protein [Bacillus licheniformis DSM 13] | UniRef100_Q65GD6 | Bacillus licheniformis DSM 13 |
| 3498 | BLP03343 ABC transporter,ATP_GTP-binding site motif A (P-loop) | Hypothetical protein [Bacillus licheniformis DSM 13] | UniRef100_Q65GD5 | Bacillus licheniformis DSM 13 |
| 3499 | BLP03344 hypothetical protein | | | |
| 3500 | BLP03345 conserved hypothetical protein | Hypothetical protein [Bacillus licheniformis DSM 13] | UniRef100_Q65GD4 | Bacillus licheniformis DSM 13 |
| 3501 | pheT phenylalanyl-tRNA synthetase (beta subunit) | PheT [Bacillus licheniformis DSM 13] | UniRef100_Q65GD3 | Bacillus licheniformis DSM 13 |
| 3502 | pheS phenylalanyl-tRNA synthetase (alpha subunit) | PheS [Bacillus licheniformis DSM 13] | UniRef100_Q65GD2 | Bacillus licheniformis DSM 13 |
| 3503 | BLP03348 hypothetical protein | | | |
| 3504 | BLP03349 hypothetical protein | | | |
| 3505 | ysgA putative RNA methylase YsgA | YsgA [Bacillus licheniformis DSM 13] | UniRef100_Q65GD1 | Bacillus licheniformis DSM 13 |
| 3506 | sspI small acid-soluble spore protein | SspI [Bacillus licheniformis DSM 13] | UniRef100_Q65GD0 | Bacillus licheniformis DSM 13 |

| 3507 | cstA carbon starvation-induced protein | CstA [Bacillus licheniformis DSM 13] | UniRef100_Q65GC9 | Bacillus licheniformis DSM 13 |
|---|---|---|---|---|
| 3508 | BLP03353 conserved hypothetical protein | Hypothetical protein [Bacillus licheniformis DSM 13] | UniRef100_Q65GC8 | Bacillus licheniformis DSM 13 |
| 3509 | BLP03354 hypothetical protein | | | |
| 3510 | abfA Glycoside Hydrolase Family 51 | AbfA [Bacillus licheniformis DSM 13] | UniRef100_Q65GC7 | Bacillus licheniformis DSM 13 |
| 3511 | araQ integral membrane protein | AraQ [Bacillus licheniformis DSM 13] | UniRef100_Q65GC6 | Bacillus licheniformis DSM 13 |
| 3512 | araP integral membrane protein | AraP [Bacillus licheniformis DSM 13] | UniRef100_Q65GC5 | Bacillus licheniformis DSM 13 |
| 3513 | araN sugar-binding protein | AraN [Bacillus licheniformis DSM 13] | UniRef100_Q65GC4 | Bacillus licheniformis DSM 13 |
| 3514 | araM AraM | Hypothetical protein araM [Bacillus licheniformis DSM 13] | UniRef100_Q65GC3 | Bacillus licheniformis DSM 13 |
| 3515 | araD L-ribulose-5-phosphate 4-epimerase | AraD [Bacillus licheniformis DSM 13] | UniRef100_Q65GC2 | Bacillus licheniformis DSM 13 |
| 3516 | araB L-ribulokinase | AraB [Bacillus licheniformis DSM 13] | UniRef100_Q65GC1 | Bacillus licheniformis DSM 13 |
| 3517 | araA L-arabinose isomerase | AraA [Bacillus licheniformis DSM 13] | UniRef100_Q65GC0 | Bacillus licheniformis DSM 13 |
| 3518 | abnA Glycoside Hydrolase Family 43 | Hypothetical protein [Bacillus licheniformis DSM 13] | UniRef100_Q65GB9 | Bacillus licheniformis DSM 13 |
| 3519 | BLP03364 hypothetical protein | | | |
| 3520 | ysdC peptidase M42 family YsdC | YsdC [Bacillus licheniformis DSM 13] | UniRef100_Q65GB8 | Bacillus licheniformis DSM 13 |
| 3521 | ysdB conserved protein YsdB | Hypothetical protein ysdB [Bacillus licheniformis DSM 13] | UniRef100_Q65GB7 | Bacillus licheniformis DSM 13 |
| 3522 | ysdA conserved membrane protein YsdA | Hypothetical protein ysdA [Bacillus licheniformis DSM 13] | UniRef100_Q65GB6 | Bacillus licheniformis DSM 13 |
| 3523 | rplT ribosomal protein L20 | RplT [Bacillus licheniformis DSM 13] | UniRef100_Q65GB5 | Bacillus licheniformis DSM 13 |
| 3524 | rpmI ribosomal protein L35 RpmI | RpmI [Bacillus licheniformis DSM 13] | UniRef100_Q65GB4 | Bacillus licheniformis DSM 13 |
| 3525 | infC initiation factor IF-3 | InfC [Bacillus licheniformis DSM 13] | UniRef100_Q65GB3 | Bacillus licheniformis DSM 13 |
| 3526 | lrgB LrgB-like protein | YsbB [Bacillus licheniformis DSM 13] | UniRef100_Q65GB2 | Bacillus licheniformis DSM 13 |
| 3527 | lrgA LrgA family | YsbA [Bacillus licheniformis DSM 13] | UniRef100_Q65GB1 | Bacillus licheniformis DSM 13 |
| 3528 | BLP03372 phosphodiesterase_alkaline phosphatase D | Hypothetical protein [Bacillus licheniformis DSM 13] | UniRef100_Q65GB0 | Bacillus licheniformis DSM 13 |
| 3529 | lytT two-component response regulator | LytT [Bacillus licheniformis DSM 13] | UniRef100_Q65GA9 | Bacillus licheniformis DSM 13 |
| 3530 | lytS two-component sensor histidine kinase | LytS [Bacillus licheniformis DSM 13] | UniRef100_Q65GA8 | Bacillus licheniformis DSM 13 |
| 3531 | BLP03375 conserved hypothetical protein | Hypothetical protein [Bacillus licheniformis DSM 13] | UniRef100_Q65GA7 | Bacillus licheniformis DSM 13 |
| 3532 | ysaA putative metallopeptidase YsaA | HAD-superfamily hydrolase, subfamily IA, variant 1,Peptidase M, neutral zinc metallopeptidases, zinc-binding site [Bacillus licheniformis DSM 13] | UniRef100_Q62RR1 | Bacillus licheniformis DSM 13 |
| 3533 | thrS threonyl-tRNA synthetase | ThrS [Bacillus licheniformis DSM 13] | UniRef100_Q65GA5 | Bacillus licheniformis DSM 13 |
| 3534 | ytxC conserved protein YtxC | Hypothetical protein ytxC [Bacillus licheniformis DSM 13] | UniRef100_Q65GA4 | Bacillus licheniformis DSM 13 |
| 3535 | ytxB conserved membrane protein YtxB | YtxB [Bacillus licheniformis DSM 13] | UniRef100_Q65GA3 | Bacillus licheniformis DSM 13 |
| 3536 | dnaI helicase loader | DnaI [Bacillus licheniformis DSM 13] | UniRef100_Q65GA2 | Bacillus licheniformis DSM 13 |
| 3537 | dnaB membrane attachment protein | DnaB [Bacillus licheniformis DSM 13] | UniRef100_Q65GA1 | Bacillus licheniformis DSM 13 |
| 3538 | ytcG putative transcriptional regulator | Hypothetical protein ytcG [Bacillus licheniformis DSM 13] | UniRef100_Q65GA0 | Bacillus licheniformis DSM 13 |
| 3539 | BLP03383 hypothetical protein | Hypothetical protein [Bacillus licheniformis DSM 13] | UniRef100_Q65G99 | Bacillus licheniformis DSM 13 |
| 3540 | speD S-adenosylmethionine decarboxylase | SpeD [Bacillus licheniformis DSM 13] | UniRef100_Q65G98 | Bacillus licheniformis DSM 13 |

| | | | | |
|---|---|---|---|---|
| 3541 | gapB glyceraldehyde-3-phosphate dehydrogenase | GapB [Bacillus licheniformis DSM 13] | UniRef100_Q65G97 | Bacillus licheniformis DSM 13 |
| 3542 | pelB pectate lyase, Polysaccharide Lyase Family 1 | PelB [Bacillus licheniformis DSM 13] | UniRef100_Q65G96 | Bacillus licheniformis DSM 13 |
| 3543 | coaE Dephospho-CoA kinase | YtaG [Bacillus licheniformis DSM 13] | UniRef100_Q65G95 | Bacillus licheniformis DSM 13 |
| 3544 | ytaF conserved membrane protein YtaF | YtaF [Bacillus licheniformis DSM 13] | UniRef100_Q65G94 | Bacillus licheniformis DSM 13 |
| 3545 | mutM formamidopyrimidine-DNA glycosidase | MutM [Bacillus licheniformis DSM 13] | UniRef100_Q65G93 | Bacillus licheniformis DSM 13 |
| 3546 | polA DNA polymerase I | PolA [Bacillus licheniformis DSM 13] | UniRef100_Q65G92 | Bacillus licheniformis DSM 13 |
| 3547 | phoR two-component sensor histidine kinase | PhoR [Bacillus licheniformis DSM 13] | UniRef100_Q65G91 | Bacillus licheniformis DSM 13 |
| 3548 | phoP two-component response regulator | Two-component response regulator [Bacillus licheniformis DSM 13] | UniRef100_Q62RP5 | Bacillus licheniformis DSM 13 |
| 3549 | BLP03393 hypothetical protein | | | |
| 3550 | mdh malate dehydrogenase | Mdh [Bacillus licheniformis DSM 13] | UniRef100_Q65G89 | Bacillus licheniformis DSM 13 |
| 3551 | icd isocitrate dehydrogenase | Icd [Bacillus licheniformis DSM 13] | UniRef100_Q65G88 | Bacillus licheniformis DSM 13 |
| 3552 | citZ citrate synthase II | CitZ [Bacillus licheniformis DSM 13] | UniRef100_Q65G87 | Bacillus licheniformis DSM 13 |
| 3553 | ytwI conserved membrane protein YtwI | Hypothetical protein ytwI [Bacillus licheniformis DSM 13] | UniRef100_Q65G86 | Bacillus licheniformis DSM 13 |
| 3554 | ytvI conserved membrane protein YtvI | Hypothetical protein ytvI [Bacillus licheniformis DSM 13] | UniRef100_Q65G85 | Bacillus licheniformis DSM 13 |
| 3555 | ytzA conserved membrane protein YtzA | YtzA [Bacillus licheniformis DSM 13] | UniRef100_Q65G84 | Bacillus licheniformis DSM 13 |
| 3556 | pyk pyruvate kinase | Pyruvate kinase [Bacillus licheniformis] | UniRef100_P51181 | Bacillus licheniformis |
| 3557 | pfkA 6-phosphofructokinase | 6-phosphofructokinase [Bacillus licheniformis DSM 13] | UniRef100_Q62RN7 | Bacillus licheniformis DSM 13 |
| 3558 | BLP04778 hypothetical protein | | | |
| 3559 | accA acetyl CoA carboxylase (alpha subunit) | AccA [Bacillus licheniformis DSM 13] | UniRef100_Q65G81 | Bacillus licheniformis DSM 13 |
| 3560 | accD acetyl-CoA carboxylase (beta subunit) | AccD [Bacillus licheniformis DSM 13] | UniRef100_Q65G80 | Bacillus licheniformis DSM 13 |
| 3561 | ytsJ malate dehydrogenase isozyme YtsJ | YtsJ [Bacillus licheniformis DSM 13] | UniRef100_Q65G79 | Bacillus licheniformis DSM 13 |
| 3562 | dnaE DNA polymerase III (alpha subunit) | DnaE [Bacillus licheniformis DSM 13] | UniRef100_Q65G78 | Bacillus licheniformis DSM 13 |
| 3563 | BLP03406 hypothetical protein | Hypothetical protein [Bacillus licheniformis DSM 13] | UniRef100_Q65G77 | Bacillus licheniformis DSM 13 |
| 3564 | ytrI conserved protein YtrI | Hypothetical protein ytrI [Bacillus licheniformis DSM 13] | UniRef100_Q65G76 | Bacillus licheniformis DSM 13 |
| 3565 | BLP03408 hypothetical protein | Hypothetical protein [Bacillus licheniformis DSM 13] | UniRef100_Q65G75 | Bacillus licheniformis DSM 13 |
| 3566 | ytqI YtqI | YtqI [Bacillus licheniformis DSM 13] | UniRef100_Q65G74 | Bacillus licheniformis DSM 13 |
| 3567 | ytpI conserved membrane protein YtpI | Hypothetical protein ytpI [Bacillus licheniformis DSM 13] | UniRef100_Q65G73 | Bacillus licheniformis DSM 13 |
| 3568 | ytoI conserved protein YtoI | YtoI [Bacillus licheniformis DSM 13] | UniRef100_Q65G72 | Bacillus licheniformis DSM 13 |
| 3569 | padR transcriptional regulator | PadR [Bacillus licheniformis DSM 13] | UniRef100_Q65G71 | Bacillus licheniformis DSM 13 |
| 3570 | ytkL conserved protein YtkL | YtkL [Bacillus licheniformis DSM 13] | UniRef100_Q65G70 | Bacillus licheniformis DSM 13 |
| 3571 | ytkK Short-chain dehydrogenase_reductase YtkK | YtkK [Bacillus licheniformis DSM 13] | UniRef100_Q65G69 | Bacillus licheniformis DSM 13 |
| 3572 | BLP03415 hypothetical protein | Hypothetical protein [Bacillus licheniformis DSM 13] | UniRef100_Q62RM3 | Bacillus licheniformis DSM 13 |
| 3573 | argH argininosuccinate lyase | ArgH [Bacillus licheniformis DSM 13] | UniRef100_Q65G68 | Bacillus licheniformis DSM 13 |
| 3574 | argG argininosuccinate synthase | ArgG [Bacillus licheniformis DSM 13] | UniRef100_Q65G67 | Bacillus licheniformis DSM 13 |
| 3575 | moaB molybdopterin precursor biosynthesis protein B | MoaB [Bacillus licheniformis DSM 13] | UniRef100_Q65G66 | Bacillus licheniformis DSM 13 |

| 3576 | ackA Acetate kinase | AckA [Bacillus licheniformis DSM 13] | UniRef100_Q65G65 | Bacillus licheniformis DSM 13 |
|---|---|---|---|---|
| 3577 | BLP03421 hypothetical protein | | | |
| 3578 | ytxK putative RNA methylase YtxK | YtxK (SAM (And some other nucleotide) binding motif) [Bacillus licheniformis DSM 13] | UniRef100_Q65G64 | Bacillus licheniformis DSM 13 |
| 3579 | tpx thiol peroxidase | Tpx [Bacillus licheniformis DSM 13] | UniRef100_Q65G63 | Bacillus licheniformis DSM 13 |
| 3580 | ytfJ conserved protein YtfJ | Hypothetical protein ytfJ [Bacillus licheniformis DSM 13] | UniRef100_Q65G62 | Bacillus licheniformis DSM 13 |
| 3581 | ytfI conserved protein YtfI | YtfI [Bacillus licheniformis DSM 13] | UniRef100_Q65G61 | Bacillus licheniformis DSM 13 |
| 3582 | yteJ conserved membrane protein YteJ | YteJ [Bacillus licheniformis DSM 13] | UniRef100_Q65G60 | Bacillus licheniformis DSM 13 |
| 3583 | sppA signal peptide peptidase | SppA [Bacillus licheniformis DSM 13] | UniRef100_Q65G59 | Bacillus licheniformis DSM 13 |
| 3584 | ppnK2 ATP-NAD kinase | YtdI [Bacillus licheniformis DSM 13] | UniRef100_Q65G58 | Bacillus licheniformis DSM 13 |
| 3585 | yhbJ conserved protein YhbJ | Hypothetical protein [Bacillus licheniformis DSM 13] | UniRef100_Q65G57 | Bacillus licheniformis DSM 13 |
| 3586 | yubD putative transporter YubD | YubD [Bacillus licheniformis DSM 13] | UniRef100_Q65G56 | Bacillus licheniformis DSM 13 |
| 3587 | BLP03431 hypothetical protein | | | |
| 3588 | yxaD probable transcriptional regulator YxaD | YxaD [Bacillus licheniformis DSM 13] | UniRef100_Q65G55 | Bacillus licheniformis DSM 13 |
| 3589 | ytcI AMP-dependent synthetase and ligase YtcI | YtcI [Bacillus licheniformis DSM 13] | UniRef100_Q65G54 | Bacillus licheniformis DSM 13 |
| 3590 | sspB small acid-soluble spore protein (beta-type SASP) | Hypothetical protein [Bacillus licheniformis DSM 13] | UniRef100_Q65G53 | Bacillus licheniformis DSM 13 |
| 3591 | thil Thiamine biosynthesis protein | YtbJ [Bacillus licheniformis DSM 13] | UniRef100_Q65G52 | Bacillus licheniformis DSM 13 |
| 3592 | nifZ NifZ | NifZ [Bacillus licheniformis DSM 13] | UniRef100_Q65G51 | Bacillus licheniformis DSM 13 |
| 3593 | BLP04780 hypothetical protein | | | |
| 3594 | braB branched-chain amino acid transporter | BraB [Bacillus licheniformis DSM 13] | UniRef100_Q65G50 | Bacillus licheniformis DSM 13 |
| 3595 | ezrA EzrA | Hypothetical protein ezrA [Bacillus licheniformis DSM 13] | UniRef100_Q65G47 | Bacillus licheniformis DSM 13 |
| 3596 | hisJ histidinol phosphate phosphatase | HisJ [Bacillus licheniformis DSM 13] | UniRef100_Q65G46 | Bacillus licheniformis DSM 13 |
| 3597 | yttP putative transcriptional regulator YttP | YttP [Bacillus licheniformis DSM 13] | UniRef100_Q65G45 | Bacillus licheniformis DSM 13 |
| 3598 | BLP03440 hypothetical protein | | | |
| 3599 | ytsP conserved protein YtsP | Hypothetical protein ytsP [Bacillus licheniformis DSM 13] | UniRef100_Q65G44 | Bacillus licheniformis DSM 13 |
| 3600 | ytrP YtrP | | | |
| 3601 | BLP03443 hypothetical protein | | | |
| 3602 | rpsD ribosomal protein S4 | RpsD [Bacillus licheniformis DSM 13] | UniRef100_Q65G42 | Bacillus licheniformis DSM 13 |
| 3603 | BLP04945 conserved hypothetical | Hypothetical protein [Bacillus licheniformis DSM 13] | UniRef100_Q65G41 | Bacillus licheniformis DSM 13 |
| 3604 | BLP03445 hypothetical protein | Hypothetical protein [Bacillus licheniformis DSM 13] | UniRef100_Q65G40 | Bacillus licheniformis DSM 13 |
| 3605 | BLP03446 hypothetical protein | Hypothetical protein [Bacillus licheniformis DSM 13] | UniRef100_Q65G39 | Bacillus licheniformis DSM 13 |
| 3606 | BLP04781 hypothetical protein | | | |
| 3607 | yddR putative Zn-dependent hydrolases of the beta-lactamase fold | YddR [Bacillus licheniformis DSM 13] | UniRef100_Q65G38 | Bacillus licheniformis DSM 13 |
| 3608 | lrpA transcriptional regulator (Lrp_AsnC family) | LrpA [Bacillus licheniformis DSM 13] | UniRef100_Q65G37 | Bacillus licheniformis DSM 13 |
| 3609 | BLP03449 hypothetical protein | Hypothetical protein [Bacillus licheniformis DSM 13] | UniRef100_Q65G36 | Bacillus licheniformis DSM 13 |

| 3610 | tyrS tyrosyl-tRNA synthetase | TyrS [Bacillus licheniformis DSM 13] | UniRef100_Q65G35 | Bacillus licheniformis DSM 13 |
|---|---|---|---|---|
| 3611 | BLP03451 hypothetical protein | | | |
| 3612 | acsAA acetyl-CoA synthetase | AcsA [Bacillus licheniformis DSM 13] | UniRef100_Q65G34 | Bacillus licheniformis DSM 13 |
| 3613 | acuA acetoin dehydrogenase | AcuA [Bacillus licheniformis DSM 13] | UniRef100_Q65G33 | Bacillus licheniformis DSM 13 |
| 3614 | acuB acetoin dehydrogenase | AcuB [Bacillus licheniformis DSM 13] | UniRef100_Q65G32 | Bacillus licheniformis DSM 13 |
| 3615 | acuC acetoin dehydrogenase | Acetoin dehydrogenase [Bacillus licheniformis DSM 13] | UniRef100_Q62RI6 | Bacillus licheniformis DSM 13 |
| 3616 | ytxE YtxE | YtxE [Bacillus licheniformis DSM 13] | UniRef100_Q65G30 | Bacillus licheniformis DSM 13 |
| 3617 | ytxD flagellar motor apparatus protein | YtxD [Bacillus licheniformis DSM 13] | UniRef100_Q65G29 | Bacillus licheniformis DSM 13 |
| 3618 | ccpA transcriptional regulator | CcpA [Bacillus licheniformis DSM 13] | UniRef100_Q65G28 | Bacillus licheniformis DSM 13 |
| 3619 | aroA 3-deoxy-D-arabino-heptulosonate 7-phosphate synthase and chorismate mutase-isozyme 3 | AroA [Bacillus licheniformis DSM 13] | UniRef100_Q65G27 | Bacillus licheniformis DSM 13 |
| 3620 | BLP03460 hypothetical protein | Hypothetical protein [Bacillus licheniformis DSM 13] | UniRef100_Q65G26 | Bacillus licheniformis DSM 13 |
| 3621 | ytxJ YtxJ | Hypothetical protein ytxJ [Bacillus licheniformis DSM 13] | UniRef100_Q65G25 | Bacillus licheniformis DSM 13 |
| 3622 | ytxH conserved protein YtxH | Hypothetical protein ytxH [Bacillus licheniformis DSM 13] | UniRef100_Q65G24 | Bacillus licheniformis DSM 13 |
| 3623 | ytxG conserved protein YtxG | Hypothetical protein ytxG [Bacillus licheniformis DSM 13] | UniRef100_Q65G23 | Bacillus licheniformis DSM 13 |
| 3624 | murC UDP-N-acetyl muramate-alanine ligase | MurC [Bacillus licheniformis DSM 13] | UniRef100_Q65G22 | Bacillus licheniformis DSM 13 |
| 3625 | ytpT YtpT | YtpT [Bacillus licheniformis DSM 13] | UniRef100_Q65G21 | Bacillus licheniformis DSM 13 |
| 3626 | ytpR putative tRNA binding protein YtpR | YtpR [Bacillus licheniformis DSM 13] | UniRef100_Q65G20 | Bacillus licheniformis DSM 13 |
| 3627 | ytpQ conserved protein YtpQ | Hypothetical protein ytpQ [Bacillus licheniformis DSM 13] | UniRef100_Q65G19 | Bacillus licheniformis DSM 13 |
| 3628 | ytpP putative thioredoxin YtpP | YtpP [Bacillus licheniformis DSM 13] | UniRef100_Q65G18 | Bacillus licheniformis DSM 13 |
| 3629 | ytoQ Nucleoside 2-deoxyribosyltransferase YtoQ | YtoQ [Bacillus licheniformis DSM 13] | UniRef100_Q65G17 | Bacillus licheniformis DSM 13 |
| 3630 | ytoP putative endo-1,4-glucanase YtoP | YtoP [Bacillus licheniformis DSM 13] | UniRef100_Q65G16 | Bacillus licheniformis DSM 13 |
| 3631 | ytzB conserved protein YtzB | Hypothetical protein ytzB [Bacillus licheniformis DSM 13] | UniRef100_Q65G15 | Bacillus licheniformis DSM 13 |
| 3632 | malS malate dehydrogenase (decarboxylating) | MalS [Bacillus licheniformis DSM 13] | UniRef100_Q65G14 | Bacillus licheniformis DSM 13 |
| 3633 | ytnP Beta-lactamase-like protein YtnP | YtnP [Bacillus licheniformis DSM 13] | UniRef100_Q65G13 | Bacillus licheniformis DSM 13 |
| 3634 | ytmQ putative methyltransferase YtmQ | Hypothetical protein ytmQ [Bacillus licheniformis DSM 13] | UniRef100_Q65G12 | Bacillus licheniformis DSM 13 |
| 3635 | ytzH YtzH | Hypothetical protein ytzH [Bacillus licheniformis DSM 13] | UniRef100_Q65G11 | Bacillus licheniformis DSM 13 |
| 3636 | ytmP YtmP | Protein kinase-like [Bacillus licheniformis DSM 13] | UniRef100_Q62RG5 | Bacillus licheniformis DSM 13 |
| 3637 | amyX putative pullulanase, Glycoside Hydrolase Family 13, AmyX | AmyX [Bacillus licheniformis DSM 13] | UniRef100_Q65G09 | Bacillus licheniformis DSM 13 |
| 3638 | ytlR conserved protein YtlR | Hypothetical protein ytlR [Bacillus licheniformis DSM 13] | UniRef100_Q65G08 | Bacillus licheniformis DSM 13 |
| 3639 | ytlQ conserved protein YtlQ | Hypothetical protein ytlQ [Bacillus licheniformis DSM 13] | UniRef100_Q65G07 | Bacillus licheniformis DSM 13 |
| 3640 | ytlP 2',5' RNA ligase YtlP | YtlP [Bacillus licheniformis DSM 13] | UniRef100_Q65G06 | Bacillus licheniformis DSM 13 |
| 3641 | ytkP cysteine synthase | YtkP [Bacillus licheniformis DSM 13] | UniRef100_Q65G05 | Bacillus licheniformis DSM 13 |
| 3642 | BLP03483 conserved hypothetical protein | Hypothetical protein [Bacillus licheniformis DSM 13] | UniRef100_Q65G04 | Bacillus licheniformis DSM 13 |
| 3643 | yncE YncE | Hypothetical protein yncE [Bacillus licheniformis DSM 13] | UniRef100_Q65G03 | Bacillus licheniformis DSM 13 |
| 3644 | ytjP putative peptidase YtjP | YtjP [Bacillus licheniformis DSM 13] | UniRef100_Q65G02 | Bacillus licheniformis DSM 13 |

| 3645 | pbuO Xanthine_uracil_vitamin C permease family | Xanthine/uracil/vitamin C permease family [Bacillus licheniformis DSM 13] | UniRef100_Q62RF8 | Bacillus licheniformis DSM 13 |
|---|---|---|---|---|
| 3646 | BLP04905 putative transcriptional regulator | YtzE [Bacillus licheniformis DSM 13] | UniRef100_Q65G00 | Bacillus licheniformis DSM 13 |
| 3647 | ytzF putative Pseudouridine synthase YtzF | Hypothetical protein [Bacillus licheniformis DSM 13] | UniRef100_Q65FZ9 | Bacillus licheniformis DSM 13 |
| 3648 | ytgP possible polysaccharide transporter YtgP | YtgP [Bacillus licheniformis DSM 13] | UniRef100_Q65FZ8 | Bacillus licheniformis DSM 13 |
| 3649 | ytfP conserved protein YtfP | YtfP [Bacillus licheniformis DSM 13] | UniRef100_Q65FZ7 | Bacillus licheniformis DSM 13 |
| 3650 | opuD glycine betaine transporter | OpuD [Bacillus licheniformis DSM 13] | UniRef100_Q65FZ6 | Bacillus licheniformis DSM 13 |
| 3651 | cse60 conserved hypothetical protein | YteV [Bacillus licheniformis DSM 13] | UniRef100_Q65FZ5 | Bacillus licheniformis DSM 13 |
| 3652 | ytwF conserved protein YtwF | Hypothetical protein [Bacillus licheniformis DSM 13] | UniRef100_Q65FZ4 | Bacillus licheniformis DSM 13 |
| 3653 | BLP03493 hypothetical protein | | | |
| 3654 | rapC response regulator aspartate phosphatase | RapC [Bacillus licheniformis DSM 13] | UniRef100_Q65FZ3 | Bacillus licheniformis DSM 13 |
| 3655 | BLP03495 hypothetical protein | | | |
| 3656 | BLP03496 conserved hypothetical protein | Hypothetical protein [Bacillus licheniformis DSM 13] | UniRef100_Q65FZ2 | Bacillus licheniformis DSM 13 |
| 3657 | yteU conserved membrane protein YteU | Hypothetical protein yteU [Bacillus licheniformis DSM 13] | UniRef100_Q65FZ1 | Bacillus licheniformis DSM 13 |
| 3658 | yteT putative oxidoreductase YteT | YteT [Bacillus licheniformis DSM 13] | UniRef100_Q65FZ0 | Bacillus licheniformis DSM 13 |
| 3659 | yteS conserved protein YteS | YteS [Bacillus licheniformis DSM 13] | UniRef100_Q65FY9 | Bacillus licheniformis DSM 13 |
| 3660 | yteR conserved protein YteR | YteR [Bacillus licheniformis DSM 13] | UniRef100_Q65FY8 | Bacillus licheniformis DSM 13 |
| 3661 | BLP03501 putative transmembrane lipoprotein | Hypothetical protein [Bacillus licheniformis DSM 13] | UniRef100_Q65FY7 | Bacillus licheniformis DSM 13 |
| 3662 | ytdP putative transcriptional regulator YtdP | YtdP [Bacillus licheniformis DSM 13] | UniRef100_Q65FY6 | Bacillus licheniformis DSM 13 |
| 3663 | ytcQ putative multiple sugar transport system substrate-binding protein YtcQ | YtcQ [Bacillus licheniformis DSM 13] | UniRef100_Q65FY5 | Bacillus licheniformis DSM 13 |
| 3664 | ytcP putative multiple sugar transport system permease protein YtcP | Binding-protein-dependent transport systems inner membrane component [Bacillus licheniformis DSM 13] | UniRef100_Q62RE2 | Bacillus licheniformis DSM 13 |
| 3665 | ytbQ YtbQ | Hypothetical protein ytbQ [Bacillus licheniformis DSM 13] | UniRef100_Q65FY3 | Bacillus licheniformis DSM 13 |
| 3666 | ytaP putative endopeptidase YtaP | YtaP [Bacillus licheniformis DSM 13] | UniRef100_Q65FY2 | Bacillus licheniformis DSM 13 |
| 3667 | yecA probable amino acid permease YecA | Hypothetical protein [Bacillus licheniformis DSM 13] | UniRef100_Q65FY1 | Bacillus licheniformis DSM 13 |
| 3668 | ywqM probable transcriptional regulator YwqM | YwqM [Bacillus licheniformis DSM 13] | UniRef100_Q65FY0 | Bacillus licheniformis DSM 13 |
| 3669 | yycE YycE | Glyoxalase/Bleomycin resistance protein/dioxygenase domain [Bacillus licheniformis DSM 13] | UniRef100_Q62RD7 | Bacillus licheniformis DSM 13 |
| 3670 | BLP03510 hypothetical protein | | | |
| 3671 | leuS leucyl-tRNA synthetase | LeuS [Bacillus licheniformis DSM 13] | UniRef100_Q65FX8 | Bacillus licheniformis DSM 13 |
| 3672 | ytvB YtvB | YtvB [Bacillus licheniformis DSM 13] | UniRef100_Q65FX7 | Bacillus licheniformis DSM 13 |
| 3673 | BLP03513 hypothetical protein | | | |
| 3674 | yttB Major facilitator superfamily YttB | YttB [Bacillus licheniformis DSM 13] | UniRef100_Q65FX6 | Bacillus licheniformis DSM 13 |
| 3675 | BLP03515 putative lipoprotein | Hypothetical protein [Bacillus licheniformis DSM 13] | UniRef100_Q65FX5 | Bacillus licheniformis DSM 13 |
| 3676 | yttA YttA | Hypothetical protein yttA [Bacillus licheniformis DSM 13] | UniRef100_Q65FX4 | Bacillus licheniformis DSM 13 |
| 3677 | ytrF possible acetoin transport system substrate binding protein YtrF | Hypothetical protein ytrF [Bacillus licheniformis DSM 13] | UniRef100_Q65FX3 | Bacillus licheniformis DSM 13 |

| 3678 | ytrE ABC transporter YtrE | YtrE [Bacillus licheniformis DSM 13] | UniRef100_Q65FX2 | Bacillus licheniformis DSM 13 |
|------|---------------------------|-------------------------------------|------------------|-------------------------------|
| 3679 | ytrC putative permease YtrC | Hypothetical protein ytrC [Bacillus licheniformis DSM 13] | UniRef100_Q65FX1 | Bacillus licheniformis DSM 13 |
| 3680 | ytrB ABC transporter YtrB | YtrB [Bacillus licheniformis DSM 13] | UniRef100_Q65FX0 | Bacillus licheniformis DSM 13 |
| 3681 | ytrA transcriptional regulator YtrA | YtrA [Bacillus licheniformis DSM 13] | UniRef100_Q65FW9 | Bacillus licheniformis DSM 13 |
| 3682 | BLP03522 hypothetical protein | | | |
| 3683 | ytzC conserved protein YtzC | Hypothetical protein ytzC [Bacillus licheniformis DSM 13] | UniRef100_Q65FW8 | Bacillus licheniformis DSM 13 |
| 3684 | ytqA Conserved hypothetical protein YtqA | Hypothetical protein ytqA [Bacillus licheniformis DSM 13] | UniRef100_Q65FW7 | Bacillus licheniformis DSM 13 |
| 3685 | ytqB conserved protein YtqB | YtqB [Bacillus licheniformis DSM 13] | UniRef100_Q65FW6 | Bacillus licheniformis DSM 13 |
| 3686 | gltP proton_glutamate symport protein | GltP [Bacillus licheniformis DSM 13] | UniRef100_Q65FW5 | Bacillus licheniformis DSM 13 |
| 3687 | ytpB conserved protein YtpB | Hypothetical protein ytpB [Bacillus licheniformis DSM 13] | UniRef100_Q65FW4 | Bacillus licheniformis DSM 13 |
| 3688 | ytpA Probable lysophospholipase YtpA | YtpA [Bacillus licheniformis DSM 13] | UniRef100_Q65FW3 | Bacillus licheniformis DSM 13 |
| 3689 | ytoA conserved protein YtoA | YtoA [Bacillus licheniformis DSM 13] | UniRef100_Q65FW2 | Bacillus licheniformis DSM 13 |
| 3690 | BLP04782 hypothetical protein | | | |
| 3691 | BLP03530 Ribosomal protein S2 | Ribosomal protein S2 [Bacillus licheniformis DSM 13] | UniRef100_Q62RB9 | Bacillus licheniformis DSM 13 |
| 3692 | BLP03531 putative sugar transferase, Glycosyl Transferase Family 4 | Hypothetical protein [Bacillus licheniformis DSM 13] | UniRef100_Q65FW0 | Bacillus licheniformis DSM 13 |
| 3693 | asnB asparagine synthetase | AsnB [Bacillus licheniformis DSM 13] | UniRef100_Q65FV9 | Bacillus licheniformis DSM 13 |
| 3694 | BLP03533 hypothetical protein | | | |
| 3695 | metK S-adenosylmethionine synthetase | MetK [Bacillus licheniformis DSM 13] | UniRef100_Q65FV8 | Bacillus licheniformis DSM 13 |
| 3696 | pckA phosphoenolpyruvate carboxykinase | PckA [Bacillus licheniformis DSM 13] | UniRef100_Q65FV7 | Bacillus licheniformis DSM 13 |
| 3697 | dctP C4-dicarboxylate transport protein | Hypothetical protein [Bacillus licheniformis DSM 13] | UniRef100_Q65FV6 | Bacillus licheniformis DSM 13 |
| 3698 | BLP03537 hypothetical protein | YtmB [Bacillus licheniformis DSM 13] | UniRef100_Q65FV5 | Bacillus licheniformis DSM 13 |
| 3699 | BLP03538 Peptidase S9, serine active site | YtmA [Bacillus licheniformis DSM 13] | UniRef100_Q65FV4 | Bacillus licheniformis DSM 13 |
| 3700 | ytlA putative sulfonate transport system substrate-binding protein YtlA | YtlA [Bacillus licheniformis DSM 13] | UniRef100_Q65FV3 | Bacillus licheniformis DSM 13 |
| 3701 | ytlC putative sulfonate transport system ATP-binding protein YtlC | YtlC [Bacillus licheniformis DSM 13] | UniRef100_Q65FV2 | Bacillus licheniformis DSM 13 |
| 3702 | ytlD putative sulfonate transport system permease protein YtlD | YtlD [Bacillus licheniformis DSM 13] | UniRef100_Q65FV1 | Bacillus licheniformis DSM 13 |
| 3703 | ytkD putative hydrolase YtkD | YtkD [Bacillus licheniformis DSM 13] | UniRef100_Q65FV0 | Bacillus licheniformis DSM 13 |
| 3704 | BLP03543 putative hydrolase | Hypothetical Glycoside hydrolase, family 1 [Bacillus licheniformis DSM 13] | UniRef100_Q62RA7 | Bacillus licheniformis DSM 13 |
| 3705 | ytkC conserved membrane protein YtkC | YtkC [Bacillus licheniformis DSM 13] | UniRef100_Q65FU8 | Bacillus licheniformis DSM 13 |
| 3706 | dps DNA-protecting protein | Dps [Bacillus licheniformis DSM 13] | UniRef100_Q65FU7 | Bacillus licheniformis DSM 13 |
| 3707 | BLP03546 hypothetical protein | | | |
| 3708 | ytkA conserved protein YtkA | Hypothetical protein ytkA [Bacillus licheniformis DSM 13] | UniRef100_Q65FU6 | Bacillus licheniformis DSM 13 |
| 3709 | BLP04783 hypothetical protein | | | |
| 3710 | luxS autoinducer-2 production protein | LuxS [Bacillus licheniformis DSM 13] | UniRef100_Q65FU5 | Bacillus licheniformis DSM 13 |
| 3711 | ytjA conserved protein YtjA | Hypothetical protein ytjA [Bacillus licheniformis DSM 13] | UniRef100_Q65FU4 | Bacillus licheniformis DSM 13 |

| 3712 | ytiB putative Carbonic anhydrase | YtiB [Bacillus licheniformis DSM 13] | UniRef100_Q65FU3 | Bacillus licheniformis DSM 13 |
|---|---|---|---|---|
| 3713 | BLP03551 Cobalamin synthesis protein_P47K | Hypothetical protein [Bacillus licheniformis DSM 13] | UniRef100_Q65FU2 | Bacillus licheniformis DSM 13 |
| 3714 | BLP03552 Periplasmic solute binding protein | YcdH [Bacillus licheniformis DSM 13] | UniRef100_Q65FU1 | Bacillus licheniformis DSM 13 |
| 3715 | rpmE2 Ribosomal protein L31 | YtiA [Bacillus licheniformis DSM 13] | UniRef100_Q65FU0 | Bacillus licheniformis DSM 13 |
| 3716 | BLP03554 hypothetical protein | | | |
| 3717 | ythA Cytochrome bd ubiquinol oxidase, subunit I | YthA [Bacillus licheniformis DSM 13] | UniRef100_Q65FT9 | Bacillus licheniformis DSM 13 |
| 3718 | ythB conserved hypothetical protein YthB | YthB [Bacillus licheniformis DSM 13] | UniRef100_Q65FT8 | Bacillus licheniformis DSM 13 |
| 3719 | BLP03557 hypothetical protein | Hypothetical protein [Bacillus licheniformis DSM 13] | UniRef100_Q62R95 | Bacillus licheniformis DSM 13 |
| 3720 | BLP03558 hypothetical protein | Hypothetical protein [Bacillus licheniformis DSM 13] | UniRef100_Q65FT7 | Bacillus licheniformis DSM 13 |
| 3721 | menC O-succinylbenzoate-CoA synthase | MenC [Bacillus licheniformis DSM 13] | UniRef100_Q65FT6 | Bacillus licheniformis DSM 13 |
| 3722 | menE O-succinylbenzoic acid-CoA ligase | MenE [Bacillus licheniformis DSM 13] | UniRef100_Q65FT5 | Bacillus licheniformis DSM 13 |
| 3723 | menB dihydroxynapthoic acid synthetase | MenB [Bacillus licheniformis DSM 13] | UniRef100_Q65FT4 | Bacillus licheniformis DSM 13 |
| 3724 | menHA o-succinylbenzoate synthase | YtxM [Bacillus licheniformis DSM 13] | UniRef100_Q65FT3 | Bacillus licheniformis DSM 13 |
| 3725 | menD 2-oxoglutarate decarboxylase and 2-succinyl-6-hydroxy-2,4-cyclohexadiene-1-carboxylate synthase | MenD [Bacillus licheniformis DSM 13] | UniRef100_Q65FT2 | Bacillus licheniformis DSM 13 |
| 3726 | menF menaquinone-specific isochorismate synthase | MenF [Bacillus licheniformis DSM 13] | UniRef100_Q65FT1 | Bacillus licheniformis DSM 13 |
| 3727 | BLP04784 hypothetical protein | | | |
| 3728 | menA 1,4-dihydroxy-2-naphthoate octaprenyltransferase | MenA [Bacillus licheniformis DSM 13] | UniRef100_Q65FT0 | Bacillus licheniformis DSM 13 |
| 3729 | BLP03566 conserved hypothetical protein | YteA [Bacillus licheniformis DSM 13] | UniRef100_Q65FS9 | Bacillus licheniformis DSM 13 |
| 3730 | glgP glycogen phosphorylase_glycosyl transferase family 35 | GlgP [Bacillus licheniformis DSM 13] | UniRef100_Q65FS8 | Bacillus licheniformis DSM 13 |
| 3731 | glgA starch synthase, glycosyl transferase family 5 | GlgA (Starch ( glycogen) synthase) [Bacillus licheniformis DSM 13] | UniRef100_Q65FS7 | Bacillus licheniformis DSM 13 |
| 3732 | glgD ADP-glucose pyrophosphorylase | GlgD [Bacillus licheniformis DSM 13] | UniRef100_Q65FS6 | Bacillus licheniformis DSM 13 |
| 3733 | glgC glucose-1-phosphate adenylyltransferase | GlgC [Bacillus licheniformis DSM 13] | UniRef100_Q65FS5 | Bacillus licheniformis DSM 13 |
| 3734 | glgB Glycoside Hydrolase Family 13 | GlgB [Bacillus licheniformis DSM 13] | UniRef100_Q65FS4 | Bacillus licheniformis DSM 13 |
| 3735 | BLP04785 hypothetical protein | | | |
| 3736 | BLP04786 hypothetical protein | Hypothetical protein [Bacillus licheniformis DSM 13] | UniRef100_Q65FS3 | Bacillus licheniformis DSM 13 |
| 3737 | BLP03572 hypothetical protein | | | |
| 3738 | BLP03574 transcriptional regulator | Hypothetical protein [Bacillus licheniformis DSM 13] | UniRef100_Q65FS1 | Bacillus licheniformis DSM 13 |
| 3739 | yuaJ hypothetical protein YuaJ | YuaJ [Bacillus licheniformis DSM 13] | UniRef100_Q65FS0 | Bacillus licheniformis DSM 13 |
| 3740 | BLP03576 putative peptidase, sortase like protein | Hypothetical protein [Bacillus licheniformis DSM 13] | UniRef100_Q65FR9 | Bacillus licheniformis DSM 13 |
| 3741 | BLP03577 hypothetical protein | Hypothetical Surface protein from Gram-positive cocci, anchor region [Bacillus licheniformis DSM 13] | UniRef100_Q65FR8 | Bacillus licheniformis DSM 13 |
| 3742 | BLP03578 hypothetical protein | Hypothetical protein [Bacillus licheniformis DSM 13] | UniRef100_Q62R76 | Bacillus licheniformis DSM 13 |
| 3743 | rapD response regulator aspartate phosphatase | RapD [Bacillus licheniformis DSM 13] | UniRef100_Q65FR7 | Bacillus licheniformis DSM 13 |
| 3744 | BLP03580 conserved hypothetical protein | Hypothetical protein [Bacillus licheniformis DSM 13] | UniRef100_Q65FR6 | Bacillus licheniformis DSM 13 |

| 3745 | pcp pyrrolidone-carboxylate peptidase | Pcp [Bacillus licheniformis DSM 13] | UniRef100_Q65FR5 | Bacillus licheniformis DSM 13 |
|---|---|---|---|---|
| 3746 | ktrA K transporter subunit peripherally bound to the cytoplasmic membrane | YuaA [Bacillus licheniformis DSM 13] | UniRef100_Q65FR4 | Bacillus licheniformis DSM 13 |
| 3747 | ktrB K transporter integral membrane subunit | Hypothetical protein [Bacillus licheniformis DSM 13] | UniRef100_Q65FR3 | Bacillus licheniformis DSM 13 |
| 3748 | BLP03584 hypothetical protein | Hypothetical protein [Bacillus licheniformis DSM 13] | UniRef100_Q62R71 | Bacillus licheniformis DSM 13 |
| 3749 | BLP03585 hypothetical protein | YuaE [Bacillus licheniformis DSM 13] | UniRef100_Q65FR1 | Bacillus licheniformis DSM 13 |
| 3750 | BLP04787 hypothetical protein | YuaD [Bacillus licheniformis DSM 13] | UniRef100_Q65FR0 | Bacillus licheniformis DSM 13 |
| 3751 | gbsB alcohol dehydrogenase | GbsB [Bacillus licheniformis DSM 13] | UniRef100_Q65FQ9 | Bacillus licheniformis DSM 13 |
| 3752 | gbsA glycine betaine aldehyde dehydrogenase | Glycine betaine aldehyde dehydrogenase [Bacillus licheniformis DSM 13] | UniRef100_Q62R67 | Bacillus licheniformis DSM 13 |
| 3753 | BLP04788 hypothetical protein | | | |
| 3754 | BLP03588 hypothetical protein | YuaC [Bacillus licheniformis DSM 13] | UniRef100_Q65FQ7 | Bacillus licheniformis DSM 13 |
| 3755 | BLP03589 proline transporter | Hypothetical protein [Bacillus licheniformis DSM 13] | UniRef100_Q65FQ6 | Bacillus licheniformis DSM 13 |
| 3756 | ytkD conserved hypothetical protein | YktD [Bacillus licheniformis DSM 13] | UniRef100_Q65FQ5 | Bacillus licheniformis DSM 13 |
| 3757 | alr2 putative alanine racemase | YncD [Bacillus licheniformis DSM 13] | UniRef100_Q65FQ4 | Bacillus licheniformis DSM 13 |
| 3758 | BLP03592 putative Oxalate decarboxylase | YoaN1 [Bacillus licheniformis DSM 13] | UniRef100_Q65FQ3 | Bacillus licheniformis DSM 13 |
| 3759 | BLP04825 hypothetical protein | Hypothetical protein [Bacillus licheniformis DSM 13] | UniRef100_Q65FQ1 | Bacillus licheniformis DSM 13 |
| 3760 | BLP03593 hypothetical protein | Hypothetical protein [Bacillus licheniformis DSM 13] | UniRef100_Q65FQ0 | Bacillus licheniformis DSM 13 |
| 3761 | BLP03594 hypothetical protein | Hypothetical protein [Bacillus licheniformis DSM 13] | UniRef100_Q65FP9 | Bacillus licheniformis DSM 13 |
| 3762 | BLP03595 hypothetical protein | Hypothetical protein [Bacillus licheniformis DSM 13] | UniRef100_Q65FP8 | Bacillus licheniformis DSM 13 |
| 3763 | BLP03596 hypothetical protein | Hypothetical protein [Bacillus licheniformis DSM 13] | UniRef100_Q65FP7 | Bacillus licheniformis DSM 13 |
| 3764 | BLP03597 hypothetical protein | Hypothetical protein [Bacillus licheniformis DSM 13] | UniRef100_Q65FP6 | Bacillus licheniformis DSM 13 |
| 3765 | BLP03598 hypothetical protein | | | |
| 3766 | BLP03599 hypothetical protein | Hypothetical protein [Bacillus licheniformis DSM 13] | UniRef100_Q65FP5 | Bacillus licheniformis DSM 13 |
| 3767 | BLP03600 hypothetical protein | Hypothetical protein [Bacillus licheniformis DSM 13] | UniRef100_Q62R54 | Bacillus licheniformis DSM 13 |
| 3768 | BLP03601 hypothetical protein | Hypothetical protein [Bacillus licheniformis DSM 13] | UniRef100_Q65FP4 | Bacillus licheniformis DSM 13 |
| 3769 | ykoN putative Glycosyl Transferase Family 28 | YkoN [Bacillus licheniformis DSM 13] | UniRef100_Q65FP3 | Bacillus licheniformis DSM 13 |
| 3770 | BLP03603 hypothetical protein | | | |
| 3771 | ykoP hypothetical protein | YkoP [Bacillus licheniformis DSM 13] | UniRef100_Q65FP2 | Bacillus licheniformis DSM 13 |
| 3772 | ykoQ Metallophosphoesterase | YkoQ [Bacillus licheniformis DSM 13] | UniRef100_Q65FP1 | Bacillus licheniformis DSM 13 |
| 3773 | upk probable undecaprenol kinase | YubB [Bacillus licheniformis DSM 13] | UniRef100_Q65FP0 | Bacillus licheniformis DSM 13 |
| 3774 | BLP03607 conserved hypothetical protein | YubA [Bacillus licheniformis DSM 13] | UniRef100_Q65FN9 | Bacillus licheniformis DSM 13 |
| 3775 | yulF putative oxidoreductase | YulF [Bacillus licheniformis DSM 13] | UniRef100_Q65FN7 | Bacillus licheniformis DSM 13 |
| 3776 | yraA probable intracellular protease | YraA [Bacillus licheniformis DSM 13] | UniRef100_Q65FN6 | Bacillus licheniformis DSM 13 |
| 3777 | mcpB methyl-accepting chemotaxis protein | Hypothetical protein [Bacillus licheniformis DSM 13] | UniRef100_Q65FN5 | Bacillus licheniformis DSM 13 |
| 3778 | mcpAA methyl-accepting chemotaxis protein | McpA [Bacillus licheniformis DSM 13] | UniRef100_Q65FN4 | Bacillus licheniformis DSM 13 |
| 3779 | mcpAB methyl-accepting chemotaxis protein | Hypothetical protein [Bacillus licheniformis DSM 13] | UniRef100_Q65FN3 | Bacillus licheniformis DSM 13 |

| 3780 | BLP03613 hypothetical protein | | | |
|---|---|---|---|---|
| 3781 | tgl transglutaminase | Tgl [Bacillus licheniformis DSM 13] | UniRef100_Q65FN2 | Bacillus licheniformis DSM 13 |
| 3782 | yrpB putative Dihydroorotate dehydrogenase YrpB | YrpB [Bacillus licheniformis DSM 13] | UniRef100_Q65FN1 | Bacillus licheniformis DSM 13 |
| 3783 | yugU conserved protein YugU | Hypothetical protein yugU [Bacillus licheniformis DSM 13] | UniRef100_Q65FN0 | Bacillus licheniformis DSM 13 |
| 3784 | yugT Glycoside Hydrolase Family 13, YugT | YugT [Bacillus licheniformis DSM 13] | UniRef100_Q65FM9 | Bacillus licheniformis DSM 13 |
| 3785 | yfiR putative transcriptional regulator YfiR | YfiR [Bacillus licheniformis DSM 13] | UniRef100_Q65FM8 | Bacillus licheniformis DSM 13 |
| 3786 | BLP03619 Ribosomal protein L29 | YqeB [Bacillus licheniformis DSM 13] | UniRef100_Q65FM7 | Bacillus licheniformis DSM 13 |
| 3787 | BLP03620 Glycoside Hydrolase Family 12 | Endo-beta-1,4-glucanase [Bacillus licheniformis] | UniRef100_Q7X4S4 | Bacillus licheniformis |
| 3788 | yugS YugS | YugS [Bacillus licheniformis DSM 13] | UniRef100_Q65FM5 | Bacillus licheniformis DSM 13 |
| 3789 | yugP conserved protein YugP | Hypothetical protein yugP [Bacillus licheniformis DSM 13] | UniRef100_Q65FM4 | Bacillus licheniformis DSM 13 |
| 3790 | BLP03623 conserved hypothetical protein | Hypothetical protein [Bacillus licheniformis DSM 13] | UniRef100_Q65FM3 | Bacillus licheniformis DSM 13 |
| 3791 | | Hypothetical protein yugO [Bacillus licheniformis DSM 13] | UniRef100_Q65FM2 | Bacillus licheniformis DSM 13 |
| 3792 | yugO conserved putative K+ channel protein YugO | YugO [Bacillus licheniformis DSM 13] | UniRef100_Q65FM1 | Bacillus licheniformis DSM 13 |
| 3793 | yugN conserved protein YugN | Hypothetical protein yugN [Bacillus licheniformis DSM 13] | UniRef100_Q65FM0 | Bacillus licheniformis DSM 13 |
| 3794 | BLP03626 hypothetical protein | YdfR [Bacillus licheniformis DSM 13] | UniRef100_Q65FL9 | Bacillus licheniformis DSM 13 |
| 3795 | ytaB hypothetical membrane protein | YtaB [Bacillus licheniformis DSM 13] | UniRef100_Q65FL8 | Bacillus licheniformis DSM 13 |
| 3796 | BLP03628 putative N-acetyltransferase | Hypothetical protein [Bacillus licheniformis DSM 13] | UniRef100_Q65FL7 | Bacillus licheniformis DSM 13 |
| 3797 | pgi glucose-6-phosphate isomerase | Pgi [Bacillus licheniformis DSM 13] | UniRef100_Q65FL6 | Bacillus licheniformis DSM 13 |
| 3798 | new hypothetical protein | Hypothetical protein [Bacillus licheniformis DSM 13] | UniRef100_Q65FL5 | Bacillus licheniformis DSM 13 |
| 3799 | BLP03630 hypothetical protein | | | |
| 3800 | yugJ Iron-containing alcohol dehydrogenase YugJ | YugJ [Bacillus licheniformis DSM 13] | UniRef100_Q65FL4 | Bacillus licheniformis DSM 13 |
| 3801 | yuzA conserved protein YuzA | Hypothetical protein yuzA [Bacillus licheniformis DSM 13] | UniRef100_Q65FL3 | Bacillus licheniformis DSM 13 |
| 3802 | yugI putative polyribonucleotide nucleotidyltransferase YugI | YugI [Bacillus licheniformis DSM 13] | UniRef100_Q65FL2 | Bacillus licheniformis DSM 13 |
| 3803 | alaT alanine transaminase | AlaT [Bacillus licheniformis DSM 13] | UniRef100_Q65FL1 | Bacillus licheniformis DSM 13 |
| 3804 | alaR transcriptional regulator (Lrp_AsnC family) | AlaR [Bacillus licheniformis DSM 13] | UniRef100_Q65FL0 | Bacillus licheniformis DSM 13 |
| 3805 | yugF putative hydrolase YugF | YugF [Bacillus licheniformis DSM 13] | UniRef100_Q65FK9 | Bacillus licheniformis DSM 13 |
| 3806 | yugE YugE | Hypothetical protein yugE [Bacillus licheniformis DSM 13] | UniRef100_Q62R20 | Bacillus licheniformis DSM 13 |
| 3807 | BLP03638 hypothetical protein | Hypothetical protein [Bacillus licheniformis DSM 13] | UniRef100_Q65FK7 | Bacillus licheniformis DSM 13 |
| 3808 | patB aminotransferase PatB | PatB [Bacillus licheniformis DSM 13] | UniRef100_Q65FK6 | Bacillus licheniformis DSM 13 |
| 3809 | BLP03640 hypothetical protein | Hypothetical protein [Bacillus licheniformis DSM 13] | UniRef100_Q65FK5 | Bacillus licheniformis DSM 13 |
| 3810 | kapB kinase-associated protein B | KapB [Bacillus licheniformis DSM 13] | UniRef100_Q65FK4 | Bacillus licheniformis DSM 13 |
| 3811 | kapD KapD | Hypothetical protein kapD [Bacillus licheniformis DSM 13] | UniRef100_Q65FK3 | Bacillus licheniformis DSM 13 |
| 3812 | BLP03643 putative transporter | YuxJ [Bacillus licheniformis DSM 13] | UniRef100_Q65FK2 | Bacillus licheniformis DSM 13 |
| 3813 | pbpD penicillin-binding protein_Glycosyl Transferase Family 51 | PbpD [Bacillus licheniformis DSM 13] | UniRef100_Q65FK1 | Bacillus licheniformis DSM 13 |
| 3814 | yuxK conserved protein YuxK | Hypothetical protein yuxK [Bacillus licheniformis DSM 13] | UniRef100_Q65FK0 | Bacillus licheniformis DSM 13 |

| | | | |
|---|---|---|---|
| 3815 | BLP03646 hypothetical protein | YufK [Bacillus licheniformis DSM 13] | UniRef100_Q65FJ9 | Bacillus licheniformis DSM 13 |
| 3816 | BLP03647 putative Histidine kinase | YufL [Bacillus licheniformis DSM 13] | UniRef100_Q65FJ8 | Bacillus licheniformis DSM 13 |
| 3817 | malR hypothetical conserved protein | YufM [Bacillus licheniformis DSM 13] | UniRef100_Q65FJ7 | Bacillus licheniformis DSM 13 |
| 3818 | BLP03649 hypothetical protein | | | |
| 3819 | yufQ conserved hypothetical YufQ | Hypothetical protein yufQ [Bacillus subtilis] | UniRef100_O05255 | Bacillus subtilis |
| 3820 | pssA phosphatidylserine synthase | PssA [Bacillus licheniformis DSM 13] | UniRef100_Q65FJ5 | Bacillus licheniformis DSM 13 |
| 3821 | ybfM hypothetical protein | YbfM [Bacillus licheniformis DSM 13] | UniRef100_Q65FJ4 | Bacillus licheniformis DSM 13 |
| 3822 | psd phosphatidylserine decarboxylase Psd | Psd [Bacillus licheniformis DSM 13] | UniRef100_Q65FJ3 | Bacillus licheniformis DSM 13 |
| 3823 | maeN Na _malate symporter MaeN | MaeN [Bacillus licheniformis DSM 13] | UniRef100_Q65FJ0 | Bacillus licheniformis DSM 13 |
| 3824 | BLP03654 hypothetical protein | Hypothetical protein [Bacillus licheniformis DSM 13] | UniRef100_Q65FI9 | Bacillus licheniformis DSM 13 |
| 3825 | BLP03655 hypothetical protein | | | |
| 3826 | mrpA Na _H antiporter MrpA | MrpA [Bacillus licheniformis DSM 13] | UniRef100_Q65FI8 | Bacillus licheniformis DSM 13 |
| 3827 | mrpB MrpA | Hypothetical protein mrpB [Bacillus licheniformis DSM 13] | UniRef100_Q65FI7 | Bacillus licheniformis DSM 13 |
| 3828 | mrpC MrpC | Hypothetical protein mrpC [Bacillus licheniformis DSM 13] | UniRef100_Q65FI6 | Bacillus licheniformis DSM 13 |
| 3829 | mrpD MrpD | MrpD [Bacillus licheniformis DSM 13] | UniRef100_Q65FI5 | Bacillus licheniformis DSM 13 |
| 3830 | mrpE MrpE | Hypothetical protein mrpE [Bacillus licheniformis DSM 13] | UniRef100_Q65FI4 | Bacillus licheniformis DSM 13 |
| 3831 | mrpF probable efflux system for Na and cholate MrpF | MrpF [Bacillus licheniformis DSM 13] | UniRef100_Q65FI3 | Bacillus licheniformis DSM 13 |
| 3832 | mrpG MrpG | Hypothetical protein mrpG [Bacillus licheniformis DSM 13] | UniRef100_Q65FI2 | Bacillus licheniformis DSM 13 |
| 3833 | yuxO comA operon protein 2 ComA2 | YuxO protein [Bacillus licheniformis] | UniRef100_Q8VQ60 | Bacillus licheniformis |
| 3834 | comA two-component response regulator | ComA [Bacillus licheniformis DSM 13] | UniRef100_Q65FI0 | Bacillus licheniformis DSM 13 |
| 3835 | comP ComP | Histidine kinase sensor protein [Bacillus mojavensis] | UniRef100_Q8VQE5 | Bacillus mojavensis |
| 3836 | | | | |
| 3837 | comQ transcriptional regulator | ComQ protein [Bacillus licheniformis] | UniRef100_Q8VQ63 | Bacillus licheniformis |
| 3838 | degQ DegQ | Degradation enzyme regulation protein degQ [Bacillus licheniformis] | UniRef100_P12051 | Bacillus licheniformis |
| 3839 | BLP03669 hypothetical protein | | | |
| 3840 | yuzC conserved protein YuzC | YuzC protein [Bacillus licheniformis] | UniRef100_Q8VQ64 | Bacillus licheniformis |
| 3841 | yuxH putative Acyl-coA-binding protein YuxH | YuxH [Bacillus licheniformis DSM 13] | UniRef100_Q65FH1 | Bacillus licheniformis DSM 13 |
| 3842 | pncB Nicotinate phosphoribosyltransferase related | YueK [Bacillus licheniformis DSM 13] | UniRef100_Q65FH0 | Bacillus licheniformis DSM 13 |
| 3843 | BLP03673 Isochorismatase hydrolase | YueJ [Bacillus licheniformis DSM 13] | UniRef100_Q65FG9 | Bacillus licheniformis DSM 13 |
| 3844 | yueI hypothetical protein | Hypothetical protein [Bacillus licheniformis DSM 13] | UniRef100_Q62QY3 | Bacillus licheniformis DSM 13 |
| 3845 | yueH hypothetical protein | YueH [Bacillus licheniformis DSM 13] | UniRef100_Q65FG7 | Bacillus licheniformis DSM 13 |
| 3846 | yueG conserved hypothetical protein YueG | YueG [Bacillus licheniformis DSM 13] | UniRef100_Q65FG6 | Bacillus licheniformis DSM 13 |
| 3847 | yueF hypothetical protein | Hypothetical protein [Bacillus licheniformis DSM 13] | UniRef100_Q62QY0 | Bacillus licheniformis DSM 13 |
| 3848 | BLP03678 conserved hypothetical protein | Hypothetical protein [Bacillus licheniformis DSM 13] | UniRef100_Q65FG4 | Bacillus licheniformis DSM 13 |
| 3849 | BLP03679 putative Esterase_lipase_thioesterase | Hypothetical protein [Bacillus licheniformis DSM 13] | UniRef100_Q65FG3 | Bacillus licheniformis DSM 13 |

| 3850 | BLP03680 conserved hypothetical protein | Hypothetical protein [Bacillus licheniformis DSM 13] | UniRef100_Q65FG2 | Bacillus licheniformis DSM 13 |
|------|------|------|------|------|
| 3851 | yueE hypothetical protein | YueE [Bacillus licheniformis DSM 13] | UniRef100_Q65FG1 | Bacillus licheniformis DSM 13 |
| 3852 | yueD putative benzil reductase | YueD [Bacillus licheniformis DSM 13] | UniRef100_Q65FG0 | Bacillus licheniformis DSM 13 |
| 3853 | yueC hypothetical protein | Hypothetical protein [Bacillus licheniformis DSM 13] | UniRef100_Q62QX4 | Bacillus licheniformis DSM 13 |
| 3854 | yueB Eukaryotic DNA topoisomerases I, dispensable insert | YueB [Bacillus licheniformis DSM 13] | UniRef100_Q65FF8 | Bacillus licheniformis DSM 13 |
| 3855 | yukA conserved protein YukA | YukA [Bacillus licheniformis DSM 13] | UniRef100_Q65FF7 | Bacillus licheniformis DSM 13 |
| 3856 | yukC conserved protein YukC | Hypothetical protein yukC [Bacillus licheniformis DSM 13] | UniRef100_Q65FF6 | Bacillus licheniformis DSM 13 |
| 3857 | yukD conserved ubiqutin-like protein YukD | Hypothetical protein yukD [Bacillus licheniformis DSM 13] | UniRef100_Q65FF5 | Bacillus licheniformis DSM 13 |
| 3858 | yukE conserved protein YukE | Hypothetical protein yukE [Bacillus licheniformis DSM 13] | UniRef100_Q65FF4 | Bacillus licheniformis DSM 13 |
| 3859 | BLP03689 putative Sodium:alanine symporter | Hypothetical protein [Bacillus licheniformis DSM 13] | UniRef100_Q65FF3 | Bacillus licheniformis DSM 13 |
| 3860 | yukF conserved protein YukF | YukF [Bacillus licheniformis DSM 13] | UniRef100_Q65FF2 | Bacillus licheniformis DSM 13 |
| 3861 | ald L-alanine dehydrogenase | Ald [Bacillus licheniformis DSM 13] | UniRef100_Q65FF1 | Bacillus licheniformis DSM 13 |
| 3862 | BLP03692 hypothetical protein | Hypothetical protein [Bacillus licheniformis DSM 13] | UniRef100_Q65FF0 | Bacillus licheniformis DSM 13 |
| 3863 | yuiH Oxidoreductase YuiH | YuiH [Bacillus licheniformis DSM 13] | UniRef100_Q65FE9 | Bacillus licheniformis DSM 13 |
| 3864 | bioY biotin metabolism protein BioY | YuiG [Bacillus licheniformis DSM 13] | UniRef100_Q65FE8 | Bacillus licheniformis DSM 13 |
| 3865 | yuiF Na _H  antiporter YuiF | YuiF [Bacillus licheniformis DSM 13] | UniRef100_Q65FE7 | Bacillus licheniformis DSM 13 |
| 3866 | pepA Peptidase M17, cytosol aminopeptidase, C-terminal | YuiE [Bacillus licheniformis DSM 13] | UniRef100_Q65FE6 | Bacillus licheniformis DSM 13 |
| 3867 | yuiD conserved membrane protein YuiD | Hypothetical protein yuiD [Bacillus licheniformis DSM 13] | UniRef100_Q65FE5 | Bacillus licheniformis DSM 13 |
| 3868 | yuiC conserved protein YuiC | 3.0 [Bacillus licheniformis DSM 13] | UniRef100_Q62QV9 | Bacillus licheniformis DSM 13 |
| 3869 | yuiB conserved membrane protein YuiB | Hypothetical protein yuiB [Bacillus licheniformis DSM 13] | UniRef100_Q65FE3 | Bacillus licheniformis DSM 13 |
| 3870 | yuiA conserved protein YuiA | Hypothetical protein yuiA [Bacillus licheniformis DSM 13] | UniRef100_Q65FE2 | Bacillus licheniformis DSM 13 |
| 3871 | yumB FAD-dependent pyridine nucleotide-disulphide oxidoreductase YumB | YumB [Bacillus licheniformis DSM 13] | UniRef100_Q65FE1 | Bacillus licheniformis DSM 13 |
| 3872 | BLP03701 hypothetical protein | | | |
| 3873 | yumC FAD-dependent pyridine nucleotide-disulphide oxidoreductase YumC | YumC [Bacillus licheniformis DSM 13] | UniRef100_Q65FE0 | Bacillus licheniformis DSM 13 |
| 3874 | BLP03703 hypothetical protein | Hypothetical protein [Bacillus licheniformis DSM 13] | UniRef100_Q62QV4 | Bacillus licheniformis DSM 13 |
| 3875 | ydjO YdjO | Hypothetical protein ydjO [Bacillus licheniformis DSM 13] | UniRef100_Q65FD9 | Bacillus licheniformis DSM 13 |
| 3876 | yxbD GCN5-related N-acetyltransferase | YxbD [Bacillus licheniformis DSM 13] | UniRef100_Q65FD8 | Bacillus licheniformis DSM 13 |
| 3877 | yutM conserved protein YutM | YutM [Bacillus licheniformis DSM 13] | UniRef100_Q65FD7 | Bacillus licheniformis DSM 13 |
| 3878 | dapF diaminopimelate epimerase | DapF [Bacillus licheniformis DSM 13] | UniRef100_Q65FD6 | Bacillus licheniformis DSM 13 |
| 3879 | BLP03707 hypothetical protein | | | |
| 3880 | yutK putative nucleoside uptake transporter YutK | YutK [Bacillus licheniformis DSM 13] | UniRef100_Q65FD5 | Bacillus licheniformis DSM 13 |
| 3881 | BLP03709 hypothetical protein | | | |
| 3882 | yuzB conserved protein YuzB | Hypothetical protein yuzB [Bacillus licheniformis DSM 13] | UniRef100_Q65FD4 | Bacillus licheniformis DSM 13 |
| 3883 | yutJ putative NADH dehydrogenase YutJ | YutJ [Bacillus licheniformis DSM 13] | UniRef100_Q65FD3 | Bacillus licheniformis DSM 13 |

| 3884 | ydhG conserved hypothetical protein YdhG | YdhG [Bacillus licheniformis DSM 13] | UniRef100_Q65FD2 | Bacillus licheniformis DSM 13 |
|---|---|---|---|---|
| 3885 | BLP03713 conserved hypothetical protein | Hypothetical protein [Bacillus licheniformis DSM 13] | UniRef100_Q65FD1 | Bacillus licheniformis DSM 13 |
| 3886 | BLP03714 response regulator aspartate phosphatase | Hypothetical protein [Bacillus licheniformis DSM 13] | UniRef100_Q65FD0 | Bacillus licheniformis DSM 13 |
| 3887 | padC phenolic acid decarboxylase | PadC [Bacillus licheniformis DSM 13] | UniRef100_Q65FC9 | Bacillus licheniformis DSM 13 |
| 3888 | BLP03716 hypothetical protein | YraG [Bacillus licheniformis DSM 13] | UniRef100_Q65FC8 | Bacillus licheniformis DSM 13 |
| 3889 | yuzD conserved protein YuzD | YuzD [Bacillus licheniformis DSM 13] | UniRef100_Q65FC7 | Bacillus licheniformis DSM 13 |
| 3890 | yutI Conserved protein YutI | YutI [Bacillus licheniformis DSM 13] | UniRef100_Q65FC6 | Bacillus licheniformis DSM 13 |
| 3891 | yuxL putative acylaminoacyl-peptidase YuxL | YuxL [Bacillus licheniformis DSM 13] | UniRef100_Q65FC5 | Bacillus licheniformis DSM 13 |
| 3892 | thrB homoserine kinase | ThrB [Bacillus licheniformis DSM 13] | UniRef100_Q65FC4 | Bacillus licheniformis DSM 13 |
| 3893 | thrC threonine synthase | ThrC [Bacillus licheniformis DSM 13] | UniRef100_Q65FC3 | Bacillus licheniformis DSM 13 |
| 3894 | hom homoserine dehydrogenase | Hom [Bacillus licheniformis DSM 13] | UniRef100_Q65FC2 | Bacillus licheniformis DSM 13 |
| 3895 | BLP03723 D-isomer specific 2-hydroxyacid dehydrogenase, NAD binding domain,D-isomer specific 2-hydroxyacid dehydrogenase, NAD binding domain | Hypothetical protein [Bacillus licheniformis DSM 13] | UniRef100_Q65FC1 | Bacillus licheniformis DSM 13 |
| 3896 | yutH conserved protein YutH | Hypothetical protein yutH [Bacillus licheniformis DSM 13] | UniRef100_Q62QT5 | Bacillus licheniformis DSM 13 |
| 3897 | yutG conserved protein YutG | Hypothetical protein yutG [Bacillus licheniformis DSM 13] | UniRef100_Q62QT4 | Bacillus licheniformis DSM 13 |
| 3898 | yutF putative HAD-superfamily subfamily IIA hydrolase | YutF [Bacillus licheniformis DSM 13] | UniRef100_Q65FB8 | Bacillus licheniformis DSM 13 |
| 3899 | yutE conserved protein YutE | Hypothetical protein yutE [Bacillus licheniformis DSM 13] | UniRef100_Q65FB7 | Bacillus licheniformis DSM 13 |
| 3900 | yutD conserved protein YutD | Hypothetical protein yutD [Bacillus licheniformis DSM 13] | UniRef100_Q65FB6 | Bacillus licheniformis DSM 13 |
| 3901 | yutC conserved protein YutC | Hypothetical protein yutC [Bacillus licheniformis DSM 13] | UniRef100_Q65FB5 | Bacillus licheniformis DSM 13 |
| 3902 | lipA lipoic acid synthetase | LipA [Bacillus licheniformis DSM 13] | UniRef100_Q65FB4 | Bacillus licheniformis DSM 13 |
| 3903 | yunA putative metallopeptidase | YunA [Bacillus licheniformis DSM 13] | UniRef100_Q65FB3 | Bacillus licheniformis DSM 13 |
| 3904 | BLP03732 hypothetical protein | | | |
| 3905 | BLP03733 putative sodium dependent transporter | Hypothetical protein [Bacillus licheniformis DSM 13] | UniRef100_Q65FB1 | Bacillus licheniformis DSM 13 |
| 3906 | yunB conserved protein YunB | Hypothetical protein yunB [Bacillus licheniformis DSM 13] | UniRef100_Q65FB0 | Bacillus licheniformis DSM 13 |
| 3907 | yunC conserved protein YunC | Hypothetical protein yunC [Bacillus licheniformis DSM 13] | UniRef100_Q65FA9 | Bacillus licheniformis DSM 13 |
| 3908 | yunD putative nucleotidase YunD | YunD [Bacillus licheniformis DSM 13] | UniRef100_Q65FA8 | Bacillus licheniformis DSM 13 |
| 3909 | yunE conserved protein YunE | Hypothetical protein yunE [Bacillus licheniformis DSM 13] | UniRef100_Q65FA7 | Bacillus licheniformis DSM 13 |
| 3910 | yunF conserved protein YunF | Hypothetical protein yunF [Bacillus licheniformis DSM 13] | UniRef100_Q65FA6 | Bacillus licheniformis DSM 13 |
| 3911 | BLP04789 hypothetical protein | | | |
| 3912 | ymcC hypothetical protein | YmcC [Bacillus licheniformis DSM 13] | UniRef100_Q65FA5 | Bacillus licheniformis DSM 13 |
| 3913 | pksA transcriptional regulator | Hypothetical protein [Bacillus licheniformis DSM 13] | UniRef100_Q65FA4 | Bacillus licheniformis DSM 13 |
| 3914 | BLP03741 hypothetical protein | Hypothetical protein [Bacillus licheniformis DSM 13] | UniRef100_Q65FA3 | Bacillus licheniformis DSM 13 |
| 3915 | BLP03742 hypothetical protein | | | |
| 3916 | BLP03743 hypothetical protein | | | |
| 3917 | BLP03744 hypothetical protein | YurG [Bacillus licheniformis DSM 13] | UniRef100_Q65FA2 | Bacillus licheniformis DSM 13 |

| 3918 | BLP03745 putative allointase_hydantoinase_amidohydrolase protein | YurH [Bacillus licheniformis DSM 13] | UniRef100_Q65FA1 | Bacillus licheniformis DSM 13 |
|------|------|------|------|------|
| 3919 | BLP03746 hypothetical protein | PucR [Bacillus licheniformis DSM 13] | UniRef100_Q65FA0 | Bacillus licheniformis DSM 13 |
| 3920 | blt multidrug-efflux transporter | Blt [Bacillus licheniformis DSM 13] | UniRef100_Q65F99 | Bacillus licheniformis DSM 13 |
| 3921 | BLP03748 C4-dicarboxylate anaerobic carrier | Hypothetical protein [Bacillus licheniformis DSM 13] | UniRef100_Q65F98 | Bacillus licheniformis DSM 13 |
| 3922 | BLP03749 hyopthetical protein | | | |
| 3923 | BLP03750 anthranilate phosphoribosyltransferase | Hypothetical protein [Bacillus licheniformis DSM 13] | UniRef100_Q65F97 | Bacillus licheniformis DSM 13 |
| 3924 | BLP03751 putative transferase | Hypothetical protein [Bacillus licheniformis DSM 13] | UniRef100_Q65F96 | Bacillus licheniformis DSM 13 |
| 3925 | bsn extracellular ribonuclease | Yurl [Bacillus licheniformis DSM 13] | UniRef100_Q65F95 | Bacillus licheniformis DSM 13 |
| 3926 | BLP03753 hypothetical protein | | | |
| 3927 | BLP03754 hypothetical protein | PucC [Bacillus licheniformis DSM 13] | UniRef100_Q65F94 | Bacillus licheniformis DSM 13 |
| 3928 | yurR YurR | YurR [Bacillus licheniformis DSM 13] | UniRef100_Q65F93 | Bacillus licheniformis DSM 13 |
| 3929 | BLP03756 hypothetical protein | Hypothetical protein [Bacillus licheniformis DSM 13] | UniRef100_Q65F92 | Bacillus licheniformis DSM 13 |
| 3930 | BLP03757 hypothetical protein | Hypothetical protein [Bacillus licheniformis DSM 13] | UniRef100_Q65F91 | Bacillus licheniformis DSM 13 |
| 3931 | BLP04790 hypothetical protein | | | |
| 3932 | rapl response regulator aspartate phosphatase | Rapl [Bacillus licheniformis DSM 13] | UniRef100_Q65F90 | Bacillus licheniformis DSM 13 |
| 3933 | sufB FeS assembly protein SufB | YurU [Bacillus licheniformis DSM 13] | UniRef100_Q65F89 | Bacillus licheniformis DSM 13 |
| 3934 | nifU Nitrogen-fixing protein NifU | YurV [Bacillus licheniformis DSM 13] | UniRef100_Q65F88 | Bacillus licheniformis DSM 13 |
| 3935 | csd cysteine desulfurase | Csd [Bacillus licheniformis DSM 13] | UniRef100_Q65F87 | Bacillus licheniformis DSM 13 |
| 3936 | yurX conserved protein involved in Fe-S cluster formation YurX | YurX [Bacillus licheniformis DSM 13] | UniRef100_Q65F86 | Bacillus licheniformis DSM 13 |
| 3937 | yurY ATP-binding protein involved in Fe-S cluster formation YurY | YurY [Bacillus licheniformis DSM 13] | UniRef100_Q65F85 | Bacillus licheniformis DSM 13 |
| 3938 | BLP03764 hypothetical protein | | | |
| 3939 | czcD cation-efflux system membrane protein | CzcD [Bacillus licheniformis DSM 13] | UniRef100_Q65F84 | Bacillus licheniformis DSM 13 |
| 3940 | BLP03766 hypothetical protein | Hypothetical protein [Bacillus licheniformis DSM 13] | UniRef100_Q62QP9 | Bacillus licheniformis DSM 13 |
| 3941 | yurZ conserved protein YurZ | YurZ [Bacillus licheniformis DSM 13] | UniRef100_Q65F83 | Bacillus licheniformis DSM 13 |
| 3942 | yusA putative ABC transport system substrate-binding protein YusA | YusA [Bacillus licheniformis DSM 13] | UniRef100_Q65F82 | Bacillus licheniformis DSM 13 |
| 3943 | yusB Binding-protein-dependent transport systems inner membrane component, YusB | YusB [Bacillus licheniformis DSM 13] | UniRef100_Q65F81 | Bacillus licheniformis DSM 13 |
| 3944 | yusC ABC transport system ATP-binding protein YusC | YusC [Bacillus licheniformis DSM 13] | UniRef100_Q65F80 | Bacillus licheniformis DSM 13 |
| 3945 | yusD YusD | Hypothetical protein yusD [Bacillus licheniformis DSM 13] | UniRef100_Q65F79 | Bacillus licheniformis DSM 13 |
| 3946 | yusE YusE | YusE [Bacillus licheniformis DSM 13] | UniRef100_Q65F78 | Bacillus licheniformis DSM 13 |
| 3947 | yusF conserved nucleic acid binding protein YusF | YusF [Bacillus licheniformis DSM 13] | UniRef100_Q65F77 | Bacillus licheniformis DSM 13 |
| 3948 | yusG conserved hypothetical protein YusG | Hypothetical protein yusG [Bacillus licheniformis DSM 13] | UniRef100_Q62QP1 | Bacillus licheniformis DSM 13 |
| 3949 | gcvH glycine cleavage system protein H | GcvH [Bacillus licheniformis DSM 13] | UniRef100_Q65F75 | Bacillus licheniformis DSM 13 |
| 3950 | yusl Conserved hypothetical protein Yusl | Hypothetical protein yusl [Bacillus licheniformis DSM 13] | UniRef100_Q65F74 | Bacillus licheniformis DSM 13 |
| 3951 | yusJ Acyl-CoA dehydrogenase YusJ | YusJ [Bacillus licheniformis DSM 13] | UniRef100_Q65F73 | Bacillus licheniformis DSM 13 |

| | | | | |
|---|---|---|---|---|
| 3952 | yusK putative acetyl-CoA C-acyltransferase YusK | YusK [Bacillus licheniformis DSM 13] | UniRef100_Q65F72 | Bacillus licheniformis DSM 13 |
| 3953 | yusL 3-hydroxyacyl-CoA dehydrogenase _ enoyl-CoA hydratase YusL | YusL [Bacillus licheniformis DSM 13] | UniRef100_Q65F71 | Bacillus licheniformis DSM 13 |
| 3954 | BLP03780 hypothetical protein | Hypothetical protein [Bacillus licheniformis DSM 13] | UniRef100_Q65F70 | Bacillus licheniformis DSM 13 |
| 3955 | BLP03781 hypothetical protein | Hypothetical protein [Bacillus licheniformis DSM 13] | UniRef100_Q65F69 | Bacillus licheniformis DSM 13 |
| 3956 | yusN conserved protein YusN | Hypothetical protein yusN [Bacillus licheniformis DSM 13] | UniRef100_Q65F68 | Bacillus licheniformis DSM 13 |
| 3957 | yusU conserved protein YusU | Hypothetical protein yusU [Bacillus licheniformis DSM 13] | UniRef100_Q65F67 | Bacillus licheniformis DSM 13 |
| 3958 | BLP03784 hypothetical protein | Hypothetical protein [Bacillus licheniformis DSM 13] | UniRef100_Q65F66 | Bacillus licheniformis DSM 13 |
| 3959 | yusV ABC transporter | YusV [Bacillus licheniformis DSM 13] | UniRef100_Q65F65 | Bacillus licheniformis DSM 13 |
| 3960 | yfhA ABC transport system permease protein | YfhA [Bacillus licheniformis DSM 13] | UniRef100_Q65F64 | Bacillus licheniformis DSM 13 |
| 3961 | yfiZ ABC transport system permease protein | YfiZ [Bacillus licheniformis DSM 13] | UniRef100_Q65F63 | Bacillus licheniformis DSM 13 |
| 3962 | yfiY ABC transport system substrate-binding protein | YfiY [Bacillus licheniformis DSM 13] | UniRef100_Q65F62 | Bacillus licheniformis DSM 13 |
| 3963 | yusW YusW | Hypothetical protein yusW [Bacillus licheniformis DSM 13] | UniRef100_Q65F61 | Bacillus licheniformis DSM 13 |
| 3964 | yusX Peptidase M, neutral zinc metallopeptidases | YusX [Bacillus licheniformis DSM 13] | UniRef100_Q65F60 | Bacillus licheniformis DSM 13 |
| 3965 | BLP03791 putative carboxypeptidase | Hypothetical protein [Bacillus licheniformis DSM 13] | UniRef100_Q65F59 | Bacillus licheniformis DSM 13 |
| 3966 | yusZ Short-chain dehydrogenase_reductase YusZ | YusZ [Bacillus licheniformis DSM 13] | UniRef100_Q65F58 | Bacillus licheniformis DSM 13 |
| 3967 | mrgA metalloregulation DNA-binding stress protein | MrgA [Bacillus licheniformis DSM 13] | UniRef100_Q65F57 | Bacillus licheniformis DSM 13 |
| 3968 | htrB putative serine protease | YvtA [Bacillus licheniformis DSM 13] | UniRef100_Q65F56 | Bacillus licheniformis DSM 13 |
| 3969 | BLP03795 hypothetical protein | | | |
| 3970 | cssR two-component response regulator | CssR [Bacillus licheniformis DSM 13] | UniRef100_Q65F55 | Bacillus licheniformis DSM 13 |
| 3971 | cssS two-component sensor histidine kinase | CssS [Bacillus licheniformis DSM 13] | UniRef100_Q65F54 | Bacillus licheniformis DSM 13 |
| 3972 | BLP03798 hypothetical protein | Hypothetical protein [Bacillus licheniformis DSM 13] | UniRef100_Q65F53 | Bacillus licheniformis DSM 13 |
| 3973 | yuxN probable transcriptional regulator YuxN | YuxN [Bacillus licheniformis DSM 13] | UniRef100_Q65F52 | Bacillus licheniformis DSM 13 |
| 3974 | citG fumarate hydratase class II CitG | CitG [Bacillus licheniformis DSM 13] | UniRef100_Q65F51 | Bacillus licheniformis DSM 13 |
| 3975 | BLP03801 hypothetical protein | Hypothetical protein [Bacillus licheniformis DSM 13] | UniRef100_Q65F50 | Bacillus licheniformis DSM 13 |
| 3976 | gerAA spore germination protein | Hypothetical protein gerAA [Bacillus licheniformis DSM 13] | UniRef100_Q65F49 | Bacillus licheniformis DSM 13 |
| 3977 | gerAB spore germination protein | Hypothetical protein gerAB [Bacillus licheniformis DSM 13] | UniRef100_Q65F48 | Bacillus licheniformis DSM 13 |
| 3978 | gerAC spore germination protein A3 precursor | Hypothetical protein gerAC [Bacillus licheniformis DSM 13] | UniRef100_Q65F47 | Bacillus licheniformis DSM 13 |
| 3979 | BLP03805 hypothetical protein | Hypothetical protein [Bacillus licheniformis DSM 13] | UniRef100_Q65F46 | Bacillus licheniformis DSM 13 |
| 3980 | liaR two-component response regulator LiaR | YvqC [Bacillus licheniformis DSM 13] | UniRef100_Q65F45 | Bacillus licheniformis DSM 13 |
| 3981 | liaS two-component sensor histidine kinase LiaS | YvqE [Bacillus licheniformis DSM 13] | UniRef100_Q65F44 | Bacillus licheniformis DSM 13 |
| 3982 | liaF conserved protein LiaF | Hypothetical protein yvqF [Bacillus licheniformis DSM 13] | UniRef100_Q65F43 | Bacillus licheniformis DSM 13 |
| 3983 | liaG conserved protein LiaG | YvqG [Bacillus licheniformis DSM 13] | UniRef100_Q65F42 | Bacillus licheniformis DSM 13 |
| 3984 | liaH putative phage shock protein YvqH | YvqH [Bacillus licheniformis DSM 13] | UniRef100_Q65F41 | Bacillus licheniformis DSM 13 |
| 3985 | liaI conserved membrane protein LiaI | Hypothetical protein yvqI [Bacillus licheniformis DSM 13] | UniRef100_Q65F40 | Bacillus licheniformis DSM 13 |
| 3986 | BLP03812 Carbohydrate Esterase Family 8 protein | Hypothetical protein [Bacillus licheniformis DSM 13] | UniRef100_Q65F39 | Bacillus licheniformis DSM 13 |

| 3987 | yvqK conserved protein YvqK | Hypothetical protein yvqK [Bacillus licheniformis DSM 13] | UniRef100_Q65F38 | Bacillus licheniformis DSM 13 |
|---|---|---|---|---|
| 3988 | BLP03814 putative acyl carrier protein reductase | Hypothetical protein [Bacillus licheniformis DSM 13] | UniRef100_Q65F37 | Bacillus licheniformis DSM 13 |
| 3989 | BLP03816 4-hydroxythreonine-4-phosphate dehydrogenase | | | |
| 3990 | BLP03817 putative dehydrogenase | Hypothetical protein [Bacillus licheniformis DSM 13] | UniRef100_Q65F35 | Bacillus licheniformis DSM 13 |
| 3991 | BLP03818 putative dihydrodipicolinate synthase | Hypothetical protein [Bacillus licheniformis DSM 13] | UniRef100_Q65F34 | Bacillus licheniformis DSM 13 |
| 3992 | yvrA putative iron transport system ATP-binding protein YvrA | YvrA [Bacillus licheniformis DSM 13] | UniRef100_Q65F33 | Bacillus licheniformis DSM 13 |
| 3993 | yvrB putative iron transport system permease protein YvrB | YvrB [Bacillus licheniformis DSM 13] | UniRef100_Q65F32 | Bacillus licheniformis DSM 13 |
| 3994 | yvrC putative iron transport system substrate-binding protein YvrC | YvrC [Bacillus licheniformis DSM 13] | UniRef100_Q65F31 | Bacillus licheniformis DSM 13 |
| 3995 | BLP03822 putative transcriptional regulator | Hypothetical protein [Bacillus licheniformis DSM 13] | UniRef100_Q65F30 | Bacillus licheniformis DSM 13 |
| 3996 | yvrD Short-chain dehydrogenase_reductase YvrD | YvrD [Bacillus licheniformis DSM 13] | UniRef100_Q65F29 | Bacillus licheniformis DSM 13 |
| 3997 | BLP03824 conserved hypothetical protein | Hypothetical protein [Bacillus licheniformis DSM 13] | UniRef100_Q65F28 | Bacillus licheniformis DSM 13 |
| 3998 | alsRA transcriptional regulator | Hypothetical protein [Bacillus licheniformis DSM 13] | UniRef100_Q65F27 | Bacillus licheniformis DSM 13 |
| 3999 | BLP03826 Glycoside Hydrolase Family 28 | Hypothetical protein [Bacillus licheniformis DSM 13] | UniRef100_Q65F26 | Bacillus licheniformis DSM 13 |
| 4000 | uxaA altronate hydrolase | UxaA [Bacillus licheniformis DSM 13] | UniRef100_Q65F25 | Bacillus licheniformis DSM 13 |
| 4001 | uxaB tagaturonate reductase (altronate oxidoreductase) | UxaB [Bacillus licheniformis DSM 13] | UniRef100_Q65F24 | Bacillus licheniformis DSM 13 |
| 4002 | exuR transcriptional regulator | ExuR [Bacillus licheniformis DSM 13] | UniRef100_Q65F23 | Bacillus licheniformis DSM 13 |
| 4003 | BLP03830 putative Sodium:galactoside symporter | YjmB [Bacillus licheniformis DSM 13] | UniRef100_Q65F22 | Bacillus licheniformis DSM 13 |
| 4004 | uxaC glucuronate isomerase. | UxaC [Bacillus licheniformis DSM 13] | UniRef100_Q65F21 | Bacillus licheniformis DSM 13 |
| 4005 | yvrG two-component sensor histidine kinase. | YvrG [Bacillus licheniformis DSM 13] | UniRef100_Q65F20 | Bacillus licheniformis DSM 13 |
| 4006 | yvrH two-component response regulator YvrH | YvrH [Bacillus licheniformis DSM 13] | UniRef100_Q65F19 | Bacillus licheniformis DSM 13 |
| 4007 | fhuC ferrichrome ABC transporter (ATP-binding protein) | FhuC [Bacillus licheniformis DSM 13] | UniRef100_Q65F18 | Bacillus licheniformis DSM 13 |
| 4008 | fhuG ferrichrome ABC transporter (permease) | FhuG [Bacillus licheniformis DSM 13] | UniRef100_Q65F17 | Bacillus licheniformis DSM 13 |
| 4009 | fhuB ferrichrome ABC transporter (permease) | FhuB [Bacillus licheniformis DSM 13] | UniRef100_Q65F16 | Bacillus licheniformis DSM 13 |
| 4010 | yvsH putative amino acid transporter YvsH | YvsH [Bacillus licheniformis DSM 13] | UniRef100_Q65F15 | Bacillus licheniformis DSM 13 |
| 4011 | BLP04829 hypothetical protein | Hypothetical protein [Bacillus licheniformis DSM 13] | UniRef100_Q65F13 | Bacillus licheniformis DSM 13 |
| 4012 | yvsG putative membrane-bound metal-dependent hydrolase | YvsG [Bacillus licheniformis DSM 13] | UniRef100_Q65F12 | Bacillus licheniformis DSM 13 |
| 4013 | yvgJ putative sulphatase YvgJ | YvgJ [Bacillus licheniformis DSM 13] | UniRef100_Q65F11 | Bacillus licheniformis DSM 13 |
| 4014 | ycnBA putative drug antiporter(transporter) | Hypothetical protein [Bacillus licheniformis DSM 13] | UniRef100_Q65F10 | Bacillus licheniformis DSM 13 |
| 4015 | yvgN putative 2,5-diketo-D-gluconic acid reductase YvgN | YvgN [Bacillus licheniformis DSM 13] | UniRef100_Q65F09 | Bacillus licheniformis DSM 13 |
| 4016 | BLP03842 conserved hypothetical protein | YdhR [Bacillus licheniformis DSM 13] | UniRef100_Q65F08 | Bacillus licheniformis DSM 13 |
| 4017 | BLP03843 hypothetical protein | | | |
| 4018 | BLP03844 hypothetical protein | | | |
| 4019 | ycbU putative Aminotransferase, class V | YcbU [Bacillus licheniformis DSM 13] | UniRef100_Q65F07 | Bacillus licheniformis DSM 13 |

| 4020 | BLP03846 conserved hypothetical protein | Hypothetical protein [Bacillus licheniformis DSM 13] | UniRef100_Q65F06 | Bacillus licheniformis DSM 13 |
|---|---|---|---|---|
| 4021 | BLP03847 putative DNA helicase | YvgS [Bacillus licheniformis DSM 13] | UniRef100_Q65F05 | Bacillus licheniformis DSM 13 |
| 4022 | yvgT conserved membrane protein YvgT | Hypothetical protein yvgT [Bacillus licheniformis DSM 13] | UniRef100_Q65F04 | Bacillus licheniformis DSM 13 |
| 4023 | BLP03849 conserved hypothetical protein | Hypothetical protein [Bacillus licheniformis DSM 13] | UniRef100_Q65F03 | Bacillus licheniformis DSM 13 |
| 4024 | BLP03850 putative GNAT family N-acetyltransferase | Hypothetical protein [Bacillus licheniformis DSM 13] | UniRef100_Q65F02 | Bacillus licheniformis DSM 13 |
| 4025 | bdbC thiol-disulfide oxidoreductase | BdbC [Bacillus licheniformis DSM 13] | UniRef100_Q65F01 | Bacillus licheniformis DSM 13 |
| 4026 | bdbD thiol-disulfide oxidoreductase | BdbD [Bacillus licheniformis DSM 13] | UniRef100_Q65F00 | Bacillus licheniformis DSM 13 |
| 4027 | yvgW Heavy metal-(Cd_Co_Hg_Pb_Zn)-translocating P-type ATPase,Heavy metal translocating P-type ATPase | YvgW (Heavy metal-(Cd/Co/Hg/Pb/Zn)-translocating P-type ATPase,Heavy metal translocating P-type ATPase) [Bacillus licheniformis DSM 13] | UniRef100_Q65EZ9 | Bacillus licheniformis DSM 13 |
| 4028 | BLP03854 putative Extracellular solute-binding protein | Hypothetical protein [Bacillus licheniformis DSM 13] | UniRef100_Q65EZ8 | Bacillus licheniformis DSM 13 |
| 4029 | BLP03855 Binding-protein-dependent transport systems inner membrane component | Hypothetical protein [Bacillus licheniformis DSM 13] | UniRef100_Q65EZ7 | Bacillus licheniformis DSM 13 |
| 4030 | BLP03856 Binding-protein-dependent transport systems inner membrane component | YurM [Bacillus licheniformis DSM 13] | UniRef100_Q65EZ6 | Bacillus licheniformis DSM 13 |
| 4031 | BLP03857 putative glycoside hydrolase family 31 | Hypothetical protein [Bacillus licheniformis DSM 13] | UniRef100_Q65EZ5 | Bacillus licheniformis DSM 13 |
| 4032 | BLP03858 putative Glycoside Hydrolase Family 3 | Hypothetical protein [Bacillus licheniformis DSM 13] | UniRef100_Q65EZ4 | Bacillus licheniformis DSM 13 |
| 4033 | BLP03859 hypothetical protein | | | |
| 4034 | mntB manganese ABC transporter (ATP-binding protein) | MntB [Bacillus licheniformis DSM 13] | UniRef100_Q65EZ3 | Bacillus licheniformis DSM 13 |
| 4035 | mntD manganese ABC transporter | MntD [Bacillus licheniformis DSM 13] | UniRef100_Q65EZ2 | Bacillus licheniformis DSM 13 |
| 4036 | mntA manganese transport system substrate-binding protein | MntA [Bacillus licheniformis DSM 13] | UniRef100_Q65EZ1 | Bacillus licheniformis DSM 13 |
| 4037 | BLP03863 hypothetical protein | | | |
| 4038 | BLP03864 hypothetical protein | Hypothetical protein [Bacillus licheniformis DSM 13] | UniRef100_Q62QF8 | Bacillus licheniformis DSM 13 |
| 4039 | BLP03865 putative transcriptional regulator | Hypothetical protein [Bacillus licheniformis DSM 13] | UniRef100_Q65EZ0 | Bacillus licheniformis DSM 13 |
| 4040 | BLP03866 fructose-1-phosphate kinase | Hypothetical protein [Bacillus licheniformis DSM 13] | UniRef100_Q65EY9 | Bacillus licheniformis DSM 13 |
| 4041 | BLP03867 phosphotransferase system (PTS) fructose-specific enzyme IIABC component | Hypothetical protein (Phosphotransferase system (PTS) fructose- specific enzyme IIABC component) [Bacillus licheniformis DSM 13] | UniRef100_Q65EY8 | Bacillus licheniformis DSM 13 |
| 4042 | BLP03868 hypothetical protein | Hypothetical protein (Phosphotransferase system (PTS) fructose- specific enzyme IIABC component) [Bacillus licheniformis DSM 13] | UniRef100_Q65EY7 | Bacillus licheniformis DSM 13 |
| 4043 | BLP03869 fructose-1,6-bisphosphate aldolase | Hypothetical protein [Bacillus licheniformis DSM 13] | UniRef100_Q65EY6 | Bacillus licheniformis DSM 13 |
| 4044 | BLP03870 hypothetical protein | Hypothetical protein [Bacillus licheniformis DSM 13] | UniRef100_Q62QF2 | Bacillus licheniformis DSM 13 |
| 4045 | copA Cu2 -exporting ATPase | Copper ion binding domain,Copper ion binding domain [Bacillus licheniformis DSM 13] | UniRef100_Q62QF1 | Bacillus licheniformis DSM 13 |
| 4046 | copZ copper binding protein CopZ | YvgY [Bacillus licheniformis DSM 13] | UniRef100_Q65EY4 | Bacillus licheniformis DSM 13 |
| 4047 | yvgZ conserved hypothetical protein YvgZ | Hypothetical protein yvgZ [Bacillus licheniformis DSM 13] | UniRef100_Q65EY3 | Bacillus licheniformis DSM 13 |

| 4048 | BLP04791 hypothetical protein | | | |
|---|---|---|---|---|
| 4049 | BLP03874 putative intracellular proteinase | Hypothetical protein [Bacillus licheniformis DSM 13] | UniRef100_Q65EY2 | Bacillus licheniformis DSM 13 |
| 4050 | yulB probable transcriptional regulator YulB | YulB [Bacillus licheniformis DSM 13] | UniRef100_Q65EY1 | Bacillus licheniformis DSM 13 |
| 4051 | yulC putative Carbohydrate kinase YulC | YulC [Bacillus licheniformis DSM 13] | UniRef100_Q65EY0 | Bacillus licheniformis DSM 13 |
| 4052 | xylA putative xylose isomerase | XylA [Bacillus licheniformis DSM 13] | UniRef100_Q65EX9 | Bacillus licheniformis DSM 13 |
| 4053 | BLP03878 conserved hypothetical protein | Hypothetical protein [Bacillus licheniformis DSM 13] | UniRef100_Q65EX8 | Bacillus licheniformis DSM 13 |
| 4054 | yuxG putative oxidoreductase YuxG | YuxG [Bacillus licheniformis DSM 13] | UniRef100_Q65EX7 | Bacillus licheniformis DSM 13 |
| 4055 | BLP03880 hypothetical protein | | | |
| 4056 | BLP03881 hypothetical protein | | | |
| 4057 | yfhl putative transporter | Hypothetical protein [Bacillus licheniformis DSM 13] | UniRef100_Q65EX6 | Bacillus licheniformis DSM 13 |
| 4058 | yvmA putative transporter YvmA | Major facilitator superfamily [Bacillus licheniformis DSM 13] | UniRef100_Q62QE1 | Bacillus licheniformis DSM 13 |
| 4059 | BLP03884 hypothetical protein | | | |
| 4060 | yvmC conserved hypothetical protein YvmC | Hypothetical protein yvmC [Bacillus licheniformis DSM 13] | UniRef100_Q65EX3 | Bacillus licheniformis DSM 13 |
| 4061 | cypX cytochrome P450-like enzyme CypX | CypX [Bacillus licheniformis DSM 13] | UniRef100_Q65EX2 | Bacillus licheniformis DSM 13 |
| 4062 | BLP03887 putative metallopeptidase | Hypothetical protein [Bacillus licheniformis DSM 13] | UniRef100_Q65EX1 | Bacillus licheniformis DSM 13 |
| 4063 | yvnA probable transcriptional regulator YvnA | YvnA [Bacillus licheniformis DSM 13] | UniRef100_Q65EX0 | Bacillus licheniformis DSM 13 |
| 4064 | BLP03889 conserved hypothetical protein similar to GatA | Hypothetical protein [Bacillus licheniformis DSM 13] | UniRef100_Q65EW9 | Bacillus licheniformis DSM 13 |
| 4065 | yvaA putative oxidoreductase | YvaA [Bacillus licheniformis DSM 13] | UniRef100_Q65EW7 | Bacillus licheniformis DSM 13 |
| 4066 | yvaB putative NAD(P)H dehydrogenase YvaB | YvaB (NAD(P)H dehydrogenase) [Bacillus licheniformis DSM 13] | UniRef100_Q65EW6 | Bacillus licheniformis DSM 13 |
| 4067 | ybfQ conserved hypothetical protein with rhodanese domain | YbfQ [Bacillus licheniformis DSM 13] | UniRef100_Q65EW5 | Bacillus licheniformis DSM 13 |
| 4068 | BLP03893 hypothetical protein | | | |
| 4069 | BLP03894 putative two-component response regulator | Hypothetical protein yvrHA [Bacillus licheniformis DSM 13] | UniRef100_Q65EW4 | Bacillus licheniformis DSM 13 |
| 4070 | yvrI Yvrl | Yvrl [Bacillus licheniformis DSM 13] | UniRef100_Q65EW3 | Bacillus licheniformis DSM 13 |
| 4071 | BLP03896 hypothetical protein | Hypothetical protein [Bacillus licheniformis DSM 13] | UniRef100_Q65EW2 | Bacillus licheniformis DSM 13 |
| 4072 | oxdC oxalate decarboxylase | OxdC [Bacillus licheniformis DSM 13] | UniRef100_Q65EW1 | Bacillus licheniformis DSM 13 |
| 4073 | yvrL putative endo-peptidase YvrL | Hypothetical protein [Bacillus licheniformis DSM 13] | UniRef100_Q65EW0 | Bacillus licheniformis DSM 13 |
| 4074 | BLP03899 conserved hypothetical protein | Hypothetical protein [Bacillus licheniformis DSM 13] | UniRef100_Q65EV9 | Bacillus licheniformis DSM 13 |
| 4075 | BLP03900 conserved hypothetical | Hypothetical protein [Bacillus licheniformis DSM 13] | UniRef100_Q65EV8 | Bacillus licheniformis DSM 13 |
| 4076 | BLP03901 hypothetical protein | | | |
| 4077 | BLP03902 hypothetical protein | Hypothetical protein ycxB [Bacillus subtilis] | UniRef100_Q08793 | Bacillus subtilis |
| 4078 | BLP03903 hypothetical protein | | | |
| 4079 | BLP03904 hypothetical protein | Hypothetical protein [Bacillus licheniformis DSM 13] | UniRef100_Q65EU5 | Bacillus licheniformis DSM 13 |
| 4080 | BLP03905 hypothetical protein | Hypothetical protein [Bacillus licheniformis DSM 13] | UniRef100_Q65EU5 | Bacillus licheniformis DSM 13 |
| 4081 | BLP03906 conserved hypothetical putative ATP binding protein | | | |

| 4082 | BLP03907 hypothetical protein | | | |
|---|---|---|---|---|
| 4083 | BLP03908 N-acetylmuramoyl-L-alanine amidase | N-acetylmuramoyl-L-alanine amidase cwlL precursor [Bacillus licheniformis] | UniRef100_P36550 | Bacillus licheniformis |
| 4084 | BLP03909 partial transposase | Hypothetical protein [Bacillus licheniformis DSM 13] | UniRef100_Q65EU5 | Bacillus licheniformis DSM 13 |
| 4085 | BLP03911 conserved hypothetical | Lmo0142 protein [Listeria monocytogenes] | UniRef100_Q8YAl4 | Listeria monocytogenes |
| 4086 | BLP03912 hypothetical protein | | | |
| 4087 | smpB tmRNA-binding protein | SmpB [Bacillus licheniformis DSM 13] | UniRef100_Q65EQ3 | Bacillus licheniformis DSM 13 |
| 4088 | rnr ribonuclease R | Rnr [Bacillus licheniformis DSM 13] | UniRef100_Q65EQ2 | Bacillus licheniformis DSM 13 |
| 4089 | yvaK Esterase_lipase_thioesterase | YvaK [Bacillus licheniformis DSM 13] | UniRef100_Q65EQ1 | Bacillus licheniformis DSM 13 |
| 4090 | BLP03916 hypothetical protein | | | |
| 4091 | secG preprotein translocase subunit | SecG [Bacillus licheniformis DSM 13] | UniRef100_Q65EQ0 | Bacillus licheniformis DSM 13 |
| 4092 | abhA transcriptional regulator | Hypothetical protein [Bacillus licheniformis DSM 13] | UniRef100_Q65EP9 | Bacillus licheniformis DSM 13 |
| 4093 | yvzC posible transcriptional regulator | YvzC [Bacillus licheniformis DSM 13] | UniRef100_Q65EP8 | Bacillus licheniformis DSM 13 |
| 4094 | yvaO posible transcriptional regulator | Lambda repressor-like, DNA-binding [Bacillus licheniformis DSM 13] | UniRef100_Q62Q60 | Bacillus licheniformis DSM 13 |
| 4095 | yvaOA possible transcriptional regulator | Hypothetical protein [Bacillus licheniformis DSM 13] | UniRef100_Q65EP6 | Bacillus licheniformis DSM 13 |
| 4096 | opuCD glycine betaine_carnitine_choline ABC transporter (membrane protein) | OpuCD [Bacillus licheniformis DSM 13] | UniRef100_Q65EP5 | Bacillus licheniformis DSM 13 |
| 4097 | opuCC glycine betaine_carnitine_choline ABC transporter (osmoprotectant-binding protein) | OpuCC [Bacillus licheniformis DSM 13] | UniRef100_Q65EP4 | Bacillus licheniformis DSM 13 |
| 4098 | opuCB glycine betaine_carnitine_choline ABC transporter (membrane protein) | OpuCB [Bacillus licheniformis DSM 13] | UniRef100_Q65EP3 | Bacillus licheniformis DSM 13 |
| 4099 | opuCA ABC transporter | OpuCA [Bacillus licheniformis DSM 13] | UniRef100_Q65EP2 | Bacillus licheniformis DSM 13 |
| 4100 | yvbF conserved protein YvbF | YvbF [Bacillus licheniformis DSM 13] | UniRef100_Q65EP1 | Bacillus licheniformis DSM 13 |
| 4101 | yvbG conserved membrane protein YvbG | YvbG [Bacillus licheniformis DSM 13] | UniRef100_Q65EP0 | Bacillus licheniformis DSM 13 |
| 4102 | yvbI YvbI | YvbI [Bacillus licheniformis DSM 13] | UniRef100_Q65EN9 | Bacillus licheniformis DSM 13 |
| 4103 | yvbJ hypothetical protein | YvbJ2 [Bacillus licheniformis DSM 13] | UniRef100_Q65EN7 | Bacillus licheniformis DSM 13 |
| 4104 | BLP03930 Periplasmic binding protein,putative iron transporter | Hypothetical protein [Bacillus licheniformis DSM 13] | UniRef100_Q65EN6 | Bacillus licheniformis DSM 13 |
| 4105 | BLP03931 hypothetical protein | Hypothetical protein [Bacillus licheniformis DSM 13] | UniRef100_Q65EN5 | Bacillus licheniformis DSM 13 |
| 4106 | BLP03932 hypothetical protein | | | |
| 4107 | BLP03933 conserved hypothetical protein | YoaZ [Bacillus subtilis] | UniRef100_O34947 | Bacillus subtilis |
| 4108 | eno enolase | Eno [Bacillus licheniformis DSM 13] | UniRef100_Q65EN2 | Bacillus licheniformis DSM 13 |
| 4109 | pgm phosphoglycerate mutase | Pgm [Bacillus licheniformis DSM 13] | UniRef100_Q65EN1 | Bacillus licheniformis DSM 13 |
| 4110 | tpiA triose phosphate isomerase | TpiA [Bacillus licheniformis DSM 13] | UniRef100_Q65EN0 | Bacillus licheniformis DSM 13 |
| 4111 | pgk phosphoglycerate kinase | Pgk [Bacillus licheniformis DSM 13] | UniRef100_Q65EM9 | Bacillus licheniformis DSM 13 |
| 4112 | BLP04792 hypothetical protein | | | |
| 4113 | gapA glyceraldehyde-3-phosphate dehydrogenase | GapA [Bacillus licheniformis DSM 13] | UniRef100_Q65EM8 | Bacillus licheniformis DSM 13 |

| 4114 | cggR transcriptional regulator | CggR [Bacillus licheniformis DSM 13] | UniRef100_Q65EM7 | Bacillus licheniformis DSM 13 |
|------|--------------------------------|--------------------------------------|------------------|-------------------------------|
| 4115 | yvbT putative oxygenase YvbT | YvbT2 [Bacillus licheniformis DSM 13] | UniRef100_Q65EM6 | Bacillus licheniformis DSM 13 |
| 4116 | yvbW putative Amino acid permease YvbW | YvbW [Bacillus licheniformis DSM 13] | UniRef100_Q65EM5 | Bacillus licheniformis DSM 13 |
| 4117 | BLP03942 hypothetical protein | | | |
| 4118 | BLP03943 hypothetical protein | Hypothetical protein [Bacillus licheniformis DSM 13] | UniRef100_Q65EM4 | Bacillus licheniformis DSM 13 |
| 4119 | BLP03944 putative ABC transporter | Hypothetical protein [Bacillus licheniformis DSM 13] | UniRef100_Q65EM3 | Bacillus licheniformis DSM 13 |
| 4120 | BLP03945 hypothetical protein | Hypothetical protein [Bacillus licheniformis DSM 13] | UniRef100_Q65EM2 | Bacillus licheniformis DSM 13 |
| 4121 | yvbY YvbY | Hypothetical protein yvbY [Bacillus licheniformis DSM 13] | UniRef100_Q65EM1 | Bacillus licheniformis DSM 13 |
| 4122 | yvfW Iron-sulfur cluster binding protein YvfW | YvfW [Bacillus licheniformis DSM 13] | UniRef100_Q65EM0 | Bacillus licheniformis DSM 13 |
| 4123 | yvfV YvfV | YvfV [Bacillus licheniformis DSM 13] | UniRef100_Q65EL9 | Bacillus licheniformis DSM 13 |
| 4124 | yvfI probable transcriptional regulator YvfI | YvfI [Bacillus licheniformis DSM 13] | UniRef100_Q65EL8 | Bacillus licheniformis DSM 13 |
| 4125 | yvfH putative L-lactate permease YvfH | YvfH [Bacillus licheniformis DSM 13] | UniRef100_Q65EL7 | Bacillus licheniformis DSM 13 |
| 4126 | sigL RNA polymerase sigma-54 factor (sigma-L) | SigL [Bacillus licheniformis DSM 13] | UniRef100_Q65EL6 | Bacillus licheniformis DSM 13 |
| 4127 | yvfG YvfG | Hypothetical protein yvfG [Bacillus licheniformis DSM 13] | UniRef100_Q65EL5 | Bacillus licheniformis DSM 13 |
| 4128 | yvfF Polysaccharide pyruvyl transferase YvfF | YvfF [Bacillus licheniformis DSM 13] | UniRef100_Q65EL4 | Bacillus licheniformis DSM 13 |
| 4129 | yvfE putative polysaccharide biosynthesis protein YvfE | YvfE [Bacillus licheniformis DSM 13] | UniRef100_Q65EL3 | Bacillus licheniformis DSM 13 |
| 4130 | yvfD acetyltransferase, possible polysaccharide biosynthesis protein YvfD | YvfD [Bacillus licheniformis DSM 13] | UniRef100_Q65EL2 | Bacillus licheniformis DSM 13 |
| 4131 | yvfC putative sugar transferase YvfC | YvfC [Bacillus licheniformis DSM 13] | UniRef100_Q65EL1 | Bacillus licheniformis DSM 13 |
| 4132 | yvfA YvfA | Hypothetical protein [Bacillus licheniformis DSM 13] | UniRef100_Q65EL0 | Bacillus licheniformis DSM 13 |
| 4133 | yveT putative capsular polysaccharide biosynthesis protein,Glycosyl Transferase Family 2, YveT | YveT [Bacillus licheniformis DSM 13] | UniRef100_Q65EK9 | Bacillus licheniformis DSM 13 |
| 4134 | yveS putative Polysaccharide pyruvyl transferase YveS | YveS [Bacillus licheniformis DSM 13] | UniRef100_Q65EK8 | Bacillus licheniformis DSM 13 |
| 4135 | yveR polysaccharide biosynthesis protein, Glycosyl transferase Family 2,YveR | YveR [Bacillus licheniformis DSM 13] | UniRef100_Q65EK7 | Bacillus licheniformis DSM 13 |
| 4136 | yveQ putative capsular polysaccharide biosynthesis protein YveQ | Hypothetical protein yveQ [Bacillus licheniformis DSM 13] | UniRef100_Q65EK6 | Bacillus licheniformis DSM 13 |
| 4137 | yveP putative capsular polysaccharide biosynthesis protein,Glycosyl Transferase Family 4, YveP | YveP [Bacillus licheniformis DSM 13] | UniRef100_Q65EK5 | Bacillus licheniformis DSM 13 |
| 4138 | yveO putative exopolysaccharide biosynthesis protein, Glycosyl Transferase Family 2,YveO | Putative glucosyl transferase [Bacillus licheniformis] | UniRef100_Q65EK4 | Bacillus licheniformis |
| 4139 | yveN putative capsular polysaccharide biosynthesis protein,Glycosyl transferase Family 4, YveN | YveN [Bacillus licheniformis DSM 13] | UniRef100_Q65EK3 | Bacillus licheniformis DSM 13 |
| 4140 | yveM putative capsular polysaccharide biosynthesis protein YveM | YveM [Bacillus licheniformis DSM 13] | UniRef100_Q65EK2 | Bacillus licheniformis DSM 13 |
| 4141 | yveL putative capsular polysaccharide biosynthesis protein YveL | YveL [Bacillus licheniformis DSM 13] | UniRef100_Q65EK1 | Bacillus licheniformis DSM 13 |
| 4142 | yveK putative capsular polysaccharide biosynthesis protein YveK | YveK [Bacillus licheniformis DSM 13] | UniRef100_Q65EK0 | Bacillus licheniformis DSM 13 |
| 4143 | slr transcriptional regulator Slr | Slr [Bacillus licheniformis DSM 13] | UniRef100_Q65EJ9 | Bacillus licheniformis DSM 13 |

| | | | | |
|---|---|---|---|---|
| 4144 | BLP03969 hypothetical protein | | | |
| 4145 | BLP03970 hypothetical protein | | | |
| 4146 | ywrO NAD(P)H dehydrogenase YwrO | NAD(P)H-FMN oxidoreductase [Bacillus subtilis] | UniRef100_Q75V96 | Bacillus subtilis |
| 4147 | BLP03972 putative transporter | Hypothetical protein [Bacillus licheniformis DSM 13] | UniRef100_Q65EJ7 | Bacillus licheniformis DSM 13 |
| 4148 | BLP03973 hypothetical protein | Hypothetical protein [Bacillus licheniformis DSM 13] | UniRef100_Q65EJ6 | Bacillus licheniformis DSM 13 |
| 4149 | BLP03974 putative 4-hydroxythreonine-4-phosphate dehydrogenase | Hypothetical protein [Bacillus licheniformis DSM 13] | UniRef100_Q65EJ5 | Bacillus licheniformis DSM 13 |
| 4150 | BLP03975 conserved hypothetical protein | Hypothetical protein [Bacillus licheniformis DSM 13] | UniRef100_Q65EJ4 | Bacillus licheniformis DSM 13 |
| 4151 | BLP03976 putative alcohol dehydrogenase | Hypothetical protein [Bacillus licheniformis DSM 13] | UniRef100_Q65EJ3 | Bacillus licheniformis DSM 13 |
| 4152 | BLP03977 putative dihydrodipicolinate synthase | Hypothetical protein [Bacillus licheniformis DSM 13] | UniRef100_Q65EJ2 | Bacillus licheniformis DSM 13 |
| 4153 | BLP03978 putative transcriptional regulator | Hypothetical protein [Bacillus licheniformis DSM 13] | UniRef100_Q65EJ1 | Bacillus licheniformis DSM 13 |
| 4154 | BLP03979 hypothetical protein | Hypothetical protein [Bacillus licheniformis DSM 13] | UniRef100_Q65EJ0 | Bacillus licheniformis DSM 13 |
| 4155 | yqiG putative NADH-dependent flavin oxidoreductase YqiG | YqiG [Bacillus licheniformis DSM 13] | UniRef100_Q65EI9 | Bacillus licheniformis DSM 13 |
| 4156 | sacB levansucrase, Glycoside Hydrolase Family 68 | SacB [Bacillus licheniformis DSM 13] | UniRef100_Q65EI8 | Bacillus licheniformis DSM 13 |
| 4157 | levB endolevanase, Glycoside Hydrolase Family 32 | YveB [Bacillus licheniformis DSM 13] | UniRef100_Q65EI7 | Bacillus licheniformis DSM 13 |
| 4158 | BLP03983 hypothetical protein | | | |
| 4159 | BLP03984 hypothetical phage tail like protein | ORF36 [Bacteriophage phi-105] | UniRef100_Q9ZXE7 | Bacteriophage phi-105 |
| 4160 | BLP04906 hypothetical protein | UPI000024ED56 UniRef100 entry | UniRef100_UPI000024ED56 | |
| 4161 | BLP03985 conserved hypothetical | Hypothetical protein MW1895 [Staphylococcus aureus] | UniRef100_Q8NVQ7 | Staphylococcus aureus |
| 4162 | BLP03986 hypothetical protein | | | |
| 4163 | BLP03987 hypothetical protein | | | |
| 4164 | BLP03988 hypothetical protein | | | |
| 4165 | BLP04793 hypothetical protein | | | |
| 4166 | BLP03989 hypothetical protein | | | |
| 4167 | BLP03990 hypothetical protein | | | |
| 4168 | BLP03991 hypothetical protein | | | |
| 4169 | BLP03992 hypothetical protein | | | |
| 4170 | BLP04907 hypothetical protein | | | |
| 4171 | BLP03993 hypothetical protein | | | |
| 4172 | BLP04794 hypothetical protein | | | |
| 4173 | BLP03994 hypothetical protein | | | |
| 4174 | BLP04908 conserved hypothetical protein | Phage protein [Bacillus cereus] | UniRef100_Q81EQ2 | Bacillus cereus |
| 4175 | BLP03995 hypothetical protein | | | |
| 4176 | BLP03996 hypothetical protein | Hypothetical protein [Bacillus licheniformis DSM 13] | UniRef100_Q65KH7 | Bacillus licheniformis DSM 13 |
| 4177 | BLP03997 hypothetical protein | Hypothetical protein [Bacillus licheniformis DSM 13] | UniRef100_Q65KD0 | Bacillus licheniformis DSM 13 |

| 4178 | BLP03998 hypothetical protein | Hypothetical protein [Bacillus licheniformis DSM 13] | UniRef100_Q65KH8 | Bacillus licheniformis DSM 13 |
|------|---|---|---|---|
| 4179 | BLP03999 putative recombinase_integrase | UPI00003CC585 UniRef100 entry | UniRef100_UPI00003CC585 | |
| 4180 | BLP04000 putative phage related protein | Hypothetical protein yolD [Bacillus licheniformis DSM 13] | UniRef100_Q65KE1 | Bacillus licheniformis DSM 13 |
| 4181 | BLP04001 hypothetical protein | Hypothetical protein [Bacillus licheniformis DSM 13] | UniRef100_Q65KE2 | Bacillus licheniformis DSM 13 |
| 4182 | BLP04002 putative integrase | Hypothetical protein [Bacillus licheniformis DSM 13] | UniRef100_Q65KI2 | Bacillus licheniformis DSM 13 |
| 4183 | BLP04003 hypothetical protein | Hypothetical protein [Bacillus licheniformis DSM 13] | UniRef100_Q65EI6 | Bacillus licheniformis DSM 13 |
| 4184 | clpP ATP-dependent Clp protease proteolytic subunit (class III heat-shock protein) | ClpP [Bacillus licheniformis DSM 13] | UniRef100_Q65EI5 | Bacillus licheniformis DSM 13 |
| 4185 | BLP04005 Conserved hypothetical protein | Hypothetical protein [Bacillus licheniformis DSM 13] | UniRef100_Q62Q01 | Bacillus licheniformis DSM 13 |
| 4186 | yvdC YvdC | YvdC [Bacillus licheniformis DSM 13] | UniRef100_Q65EI3 | Bacillus licheniformis DSM 13 |
| 4187 | BLP04007 putative Metal-dependent phosphohydrolase protein | YdhJ [Bacillus licheniformis DSM 13] | UniRef100_Q65EI2 | Bacillus licheniformis DSM 13 |
| 4188 | yvdB Sulfate transporter_antisigma-factor antagonist | YvdB [Bacillus licheniformis DSM 13] | UniRef100_Q65EI1 | Bacillus licheniformis DSM 13 |
| 4189 | yvdA Carbonic anhydrase, prokaryotic YvdA | YvdA [Bacillus licheniformis DSM 13] | UniRef100_Q65EI0 | Bacillus licheniformis DSM 13 |
| 4190 | yvcT probable 2-ketogluconate reductase YvcT | YvcT [Bacillus licheniformis DSM 13] | UniRef100_Q65EH9 | Bacillus licheniformis DSM 13 |
| 4191 | ydfE conserved hypothetical protein | YdfE [Bacillus licheniformis DSM 13] | UniRef100_Q65EH8 | Bacillus licheniformis DSM 13 |
| 4192 | ydfF hypothetical protein | YdfF [Bacillus licheniformis DSM 13] | UniRef100_Q65EH7 | Bacillus licheniformis DSM 13 |
| 4193 | BLP04013 putative ribonuclease | Hypothetical protein [Bacillus licheniformis DSM 13] | UniRef100_Q65EH6 | Bacillus licheniformis DSM 13 |
| 4194 | yrdF putative ribonuclease inhibitor YrdF | YrdF [Bacillus licheniformis DSM 13] | UniRef100_Q65EH5 | Bacillus licheniformis DSM 13 |
| 4195 | nat arylamine N-acetyltransferase | YvcN [Bacillus licheniformis DSM 13] | UniRef100_Q65EH4 | Bacillus licheniformis DSM 13 |
| 4196 | crh catabolite repression protein | Crh [Bacillus licheniformis DSM 13] | UniRef100_Q65EH3 | Bacillus licheniformis DSM 13 |
| 4197 | yvcL conserved protein YvcL | Hypothetical protein yvcL [Bacillus licheniformis DSM 13] | UniRef100_Q65EH2 | Bacillus licheniformis DSM 13 |
| 4198 | yvcK Conserved hypothetical protein YvcK | YvcK [Bacillus licheniformis DSM 13] | UniRef100_Q65EH1 | Bacillus licheniformis DSM 13 |
| 4199 | yvcJ Uncharacterised P-loop ATPase protein family UPF0042 | YvcJ [Bacillus licheniformis DSM 13] | UniRef100_Q65EH0 | Bacillus licheniformis DSM 13 |
| 4200 | yvcI NUDIX hydrolase | YvcI [Bacillus licheniformis DSM 13] | UniRef100_Q65EG9 | Bacillus licheniformis DSM 13 |
| 4201 | BLP04021 conserved hypothetical protein | Hypothetical protein [Bacillus licheniformis DSM 13] | UniRef100_Q65EG8 | Bacillus licheniformis DSM 13 |
| 4202 | trxB thioredoxin reductase | TrxB [Bacillus licheniformis DSM 13] | UniRef100_Q65EG7 | Bacillus licheniformis DSM 13 |
| 4203 | yvcD TPR-like,Putative DNA binding | YvcD [Bacillus licheniformis DSM 13] | UniRef100_Q65EG6 | Bacillus licheniformis DSM 13 |
| 4204 | ybbJ GCN5-related N-acetyltransferase | YbbJ [Bacillus licheniformis DSM 13] | UniRef100_Q65EG5 | Bacillus licheniformis DSM 13 |
| 4205 | hisI phosphoribosyl-AMP cyclohydrolase and phosphoribosyl-ATP pyrophosphohydrolase, Histidine biosynthesis bifunctional protein HisI | HisI [Bacillus licheniformis DSM 13] | UniRef100_Q65EG4 | Bacillus licheniformis DSM 13 |
| 4206 | hisF Imidazole glycerol phosphate synthase subunit HisF | HisF cyclase-like protein [Bacillus licheniformis DSM 13] | UniRef100_Q65EG3 | Bacillus licheniformis DSM 13 |
| 4207 | hisA phosphoribosylformimino-5-aminoimidazole carboxamide ribotide isomerase | HisA [Bacillus licheniformis DSM 13] | UniRef100_Q65EG2 | Bacillus licheniformis DSM 13 |
| 4208 | hisH Imidazole glycerol phosphate synthase subunit HisH | HisH [Bacillus licheniformis DSM 13] | UniRef100_Q65EG1 | Bacillus licheniformis DSM 13 |

| 4209 | hisB imidazoleglycerol-phosphate dehydratase | HisB [Bacillus licheniformis DSM 13] | UniRef100_Q65EG0 | Bacillus licheniformis DSM 13 |
|------|---|---|---|---|
| 4210 | hisD histidinol dehydrogenase | HisD [Bacillus licheniformis DSM 13] | UniRef100_Q65EF9 | Bacillus licheniformis DSM 13 |
| 4211 | hisG ATP phosphoribosyltransferase HisG | HisG [Bacillus licheniformis DSM 13] | UniRef100_Q65EF8 | Bacillus licheniformis DSM 13 |
| 4212 | hisZ histidyl-tRNA synthetase HisZ | HisZ [Bacillus licheniformis DSM 13] | UniRef100_Q65EF7 | Bacillus licheniformis DSM 13 |
| 4213 | yvpB putative cysteine protease YvpB | Hypothetical protein [Bacillus licheniformis DSM 13] | UniRef100_Q65EF6 | Bacillus licheniformis DSM 13 |
| 4214 | pelC Polysaccharide Lyase Family 3, putative pectate lyase | YvpA [Bacillus licheniformis DSM 13] | UniRef100_Q65EF5 | Bacillus licheniformis DSM 13 |
| 4215 | yvoF Trimeric LpxA-like | YvoF [Bacillus licheniformis DSM 13] | UniRef100_Q65EF4 | Bacillus licheniformis DSM 13 |
| 4216 | hprP,ppaX,ptsL, yvoE Pyrophosphatase PpaX | HprP e [Bacillus licheniformis DSM 13] | UniRef100_Q65EF3 | Bacillus licheniformis DSM 13 |
| 4217 | yvoD conserved membrane protein YvoD | Hypothetical protein yvoD [Bacillus licheniformis DSM 13] | UniRef100_Q65EF2 | Bacillus licheniformis DSM 13 |
| 4218 | lgt prolipoprotein diacylglyceryl transferase | Lgt [Bacillus licheniformis DSM 13] | UniRef100_Q65EF1 | Bacillus licheniformis DSM 13 |
| 4219 | hprK serine_threonine protein kinase | HprK [Bacillus licheniformis DSM 13] | UniRef100_Q65EF0 | Bacillus licheniformis DSM 13 |
| 4220 | yfiV putative transcriptional regulator YfiV | YfiV [Bacillus licheniformis DSM 13] | UniRef100_Q65EE9 | Bacillus licheniformis DSM 13 |
| 4221 | BLP04041 hypothetical protein | | | |
| 4222 | yfiU putative multidrug-efflux transporter YfiU | YfiU [Bacillus licheniformis DSM 13] | UniRef100_Q65EE8 | Bacillus licheniformis DSM 13 |
| 4223 | yvnB YvnB | YvnB [Bacillus licheniformis DSM 13] | UniRef100_Q65EE7 | Bacillus licheniformis DSM 13 |
| 4224 | yvlD conserved membrane protein YvlD | Hypothetical protein yvlD [Bacillus licheniformis DSM 13] | UniRef100_Q65EE6 | Bacillus licheniformis DSM 13 |
| 4225 | yvlC PspC | YvlC [Bacillus licheniformis DSM 13] | UniRef100_Q65EE5 | Bacillus licheniformis DSM 13 |
| 4226 | yvlB conserved hypothetical protein YvlB | Hypothetical protein yvlB [Bacillus licheniformis DSM 13] | UniRef100_Q65EE4 | Bacillus licheniformis DSM 13 |
| 4227 | yvlA YvlA | Hypothetical protein yvlA [Bacillus licheniformis DSM 13] | UniRef100_Q65EE3 | Bacillus licheniformis DSM 13 |
| 4228 | yvkN YvkN | Hypothetical protein yvkN [Bacillus licheniformis DSM 13] | UniRef100_Q65EE2 | Bacillus licheniformis DSM 13 |
| 4229 | BLP04049 putative dioxygenase protein | Hypothetical protein [Bacillus licheniformis DSM 13] | UniRef100_Q65EE0 | Bacillus licheniformis DSM 13 |
| 4230 | uvrA excinuclease ABC (subunit A) | UvrA2 [Bacillus licheniformis DSM 13] | UniRef100_Q65ED9 | Bacillus licheniformis DSM 13 |
| 4231 | uvrB excinuclease ABC (subunit B) | UvrB [Bacillus licheniformis DSM 13] | UniRef100_Q65ED8 | Bacillus licheniformis DSM 13 |
| 4232 | csbA putative membrane protein | CsbA [Bacillus licheniformis DSM 13] | UniRef100_Q65ED7 | Bacillus licheniformis DSM 13 |
| 4233 | BLP04054 conserved hypothetical protein | YwkF [Bacillus licheniformis DSM 13] | UniRef100_Q65ED6 | Bacillus licheniformis DSM 13 |
| 4234 | swrAB motility_swarming protein SwrAB | YvjD [Bacillus licheniformis DSM 13] | UniRef100_Q65ED5 | Bacillus licheniformis DSM 13 |
| 4235 | BLP04056 hypothetical protein | | | |
| 4236 | swrA Swarming motility protein SwrA | Hypothetical protein yvzD [Bacillus licheniformis DSM 13] | UniRef100_Q62PV3 | Bacillus licheniformis DSM 13 |
| 4237 | ctpB Peptidase S41A, C-terminal protease | YvjB [Bacillus licheniformis DSM 13] | UniRef100_Q65ED3 | Bacillus licheniformis DSM 13 |
| 4238 | ycsA putative dehydrogenase | Isocitrate/isopropylmalate dehydrogenase [Bacillus licheniformis DSM 13] | UniRef100_Q62PV1 | Bacillus licheniformis DSM 13 |
| 4239 | BLP04060 hypothetical protein | | | |
| 4240 | BLP04061 conserved hypothetical protein, putative peptidase | Hypothetical protein [Bacillus licheniformis DSM 13] | UniRef100_Q65ED1 | Bacillus licheniformis DSM 13 |
| 4241 | ftsX  Cell division ABC transporter, permease protein FtsX | FtsX [Bacillus licheniformis DSM 13] | UniRef100_Q65ED0 | Bacillus licheniformis DSM 13 |
| 4242 | ftsE cell-division ATP-binding protein | FtsE [Bacillus licheniformis DSM 13] | UniRef100_Q65EC9 | Bacillus licheniformis DSM 13 |

| 4243 | BLP04064 hypothetical protein | | | |
|---|---|---|---|---|
| 4244 | cccB putative cytochrome c551 heme binding protein | CccB [Bacillus licheniformis DSM 13] | UniRef100_Q65EC8 | Bacillus licheniformis DSM 13 |
| 4245 | yvjA conserved membrane protein YvjA | Hypothetical protein yvjA [Bacillus licheniformis DSM 13] | UniRef100_Q65EC7 | Bacillus licheniformis DSM 13 |
| 4246 | prfB peptide chain release factor 2 | PrfB [Bacillus licheniformis DSM 13] | UniRef100_Q65EC6 | Bacillus licheniformis DSM 13 |
| 4247 | secA translocase binding subunit (ATPase) | SecA [Bacillus licheniformis DSM 13] | UniRef100_Q65EC5 | Bacillus licheniformis DSM 13 |
| 4248 | yvyD general stress protein conserved hypothetical YvyD | Sigma 54 modulation protein/ribosomal protein S30EA [Bacillus licheniformis DSM 13] | UniRef100_Q62PU3 | Bacillus licheniformis DSM 13 |
| 4249 | BLP04070 hypothetical protein | Hypothetical protein [Bacillus licheniformis DSM 13] | UniRef100_Q65EC3 | Bacillus licheniformis DSM 13 |
| 4250 | fliT flagellar protein FliT | FliT [Bacillus licheniformis DSM 13] | UniRef100_Q65EC2 | Bacillus licheniformis DSM 13 |
| 4251 | fliS flagellar protein FliS | FliS [Bacillus licheniformis DSM 13] | UniRef100_Q65EC1 | Bacillus licheniformis DSM 13 |
| 4252 | fliD flagellar hook-associated protein FliD | FliD [Bacillus licheniformis DSM 13] | UniRef100_Q65EC0 | Bacillus licheniformis DSM 13 |
| 4253 | yvyC Flagellar protein YvyC | YvyC [Bacillus licheniformis DSM 13] | UniRef100_Q65EB9 | Bacillus licheniformis DSM 13 |
| 4254 | hag flagellin protein Hag | Hag [Bacillus licheniformis DSM 13] | UniRef100_Q65EB8 | Bacillus licheniformis DSM 13 |
| 4255 | csrA carbon storage regulator CsrA | CsrA [Bacillus licheniformis DSM 13] | UniRef100_Q65EB7 | Bacillus licheniformis DSM 13 |
| 4256 | yviF conserved membrane protein YviF | YviF [Bacillus licheniformis DSM 13] | UniRef100_Q65EB6 | Bacillus licheniformis DSM 13 |
| 4257 | yviE conserved protein YviE | Hypothetical protein yviE [Bacillus licheniformis DSM 13] | UniRef100_Q65EB5 | Bacillus licheniformis DSM 13 |
| 4258 | flgL flagellar hook-associated protein 3 | FlgL [Bacillus licheniformis DSM 13] | UniRef100_Q65EB4 | Bacillus licheniformis DSM 13 |
| 4259 | flgK flagellar hook-associated protein 1 | FlgK [Bacillus licheniformis DSM 13] | UniRef100_Q65EB3 | Bacillus licheniformis DSM 13 |
| 4260 | yvyG flagellar protein YvyG | Hypothetical protein yvyG [Bacillus licheniformis DSM 13] | UniRef100_Q65EB2 | Bacillus licheniformis DSM 13 |
| 4261 | flgM anti-sigma factor repressor of sigma-D-dependent transcription | FlgM [Bacillus licheniformis DSM 13] | UniRef100_Q65EB1 | Bacillus licheniformis DSM 13 |
| 4262 | yvyF flagellar protein YvyF | Hypothetical protein yvyF [Bacillus licheniformis DSM 13] | UniRef100_Q65EB0 | Bacillus licheniformis DSM 13 |
| 4263 | comFC late competence protein ComFC | Hypothetical protein comFC [Bacillus licheniformis DSM 13] | UniRef100_Q65EA9 | Bacillus licheniformis DSM 13 |
| 4264 | comFB late competence protein ComFB | Hypothetical protein comFB [Bacillus licheniformis DSM 13] | UniRef100_Q65EA8 | Bacillus licheniformis DSM 13 |
| 4265 | comFA late competence protein | Late competence protein ComFA [Bacillus licheniformis] | UniRef100_Q8VQ58 | Bacillus licheniformis |
| 4266 | degV DegV | Hypothetical protein yviA [Bacillus licheniformis] | UniRef100_Q8VQ59 | Bacillus licheniformis |
| 4267 | degU two-component response regulator | DegU [Bacillus licheniformis DSM 13] | UniRef100_Q65EA5 | Bacillus licheniformis DSM 13 |
| 4268 | degS two-component sensor histidine kinase | DegS [Bacillus licheniformis DSM 13] | UniRef100_Q65EA4 | Bacillus licheniformis DSM 13 |
| 4269 | yvyE YvyE | Hypothetical protein yvyE [Bacillus licheniformis DSM 13] | UniRef100_Q65EA3 | Bacillus licheniformis DSM 13 |
| 4270 | yvhJ putative transcriptional attenuator LytR family protein | YvhJ [Bacillus licheniformis DSM 13] | UniRef100_Q65EA2 | Bacillus licheniformis DSM 13 |
| 4271 | yunG YunG | Hypothetical protein yunG [Bacillus licheniformis DSM 13] | UniRef100_Q65EA1 | Bacillus licheniformis DSM 13 |
| 4272 | tagO TagO | Hypothetical protein tagO [Bacillus licheniformis DSM 13] | UniRef100_Q65EA0 | Bacillus licheniformis DSM 13 |
| 4273 | tuaH Glycosyl Transferase Family4, TuaH | Hypothetical protein tuaH [Bacillus licheniformis DSM 13] | UniRef100_Q65E99 | Bacillus licheniformis DSM 13 |
| 4274 | tuaG Glycosyl Transferase Family 2,TuaG | TuaG [Bacillus licheniformis DSM 13] | UniRef100_Q65E98 | Bacillus licheniformis DSM 13 |
| 4275 | tuaF TuaF | Hypothetical protein tuaF [Bacillus licheniformis DSM 13] | UniRef100_Q65E97 | Bacillus licheniformis DSM 13 |
| 4276 | tuaE TuaE | Hypothetical protein tuaE [Bacillus licheniformis DSM 13] | UniRef100_Q65E96 | Bacillus licheniformis DSM 13 |

| 4277 | BLP04098 UDP-glucose 4-epimerase | Hypothetical protein [Bacillus licheniformis DSM 13] | UniRef100_Q65E95 | Bacillus licheniformis DSM 13 |
|---|---|---|---|---|
| 4278 | tuaD UDP-glucose 6-dehydrogenase TuaD | TuaD [Bacillus licheniformis DSM 13] | UniRef100_Q65E94 | Bacillus licheniformis DSM 13 |
| 4279 | tuaC glycosyl transferase Family 4,TuaC | Hypothetical protein tuaC [Bacillus licheniformis DSM 13] | UniRef100_Q65E93 | Bacillus licheniformis DSM 13 |
| 4280 | tuaB TuaB | Hypothetical protein tuaB [Bacillus licheniformis DSM 13] | UniRef100_Q65E92 | Bacillus licheniformis DSM 13 |
| 4281 | tuaA TuaA | Hypothetical protein tuaA [Bacillus licheniformis DSM 13] | UniRef100_Q65E91 | Bacillus licheniformis DSM 13 |
| 4282 | lytC N-acetylmuramoyl-L-alanine amidase (major autolysin) (CWBP49) | LytC [Bacillus licheniformis DSM 13] | UniRef100_Q65E90 | Bacillus licheniformis DSM 13 |
| 4283 | lytB modifier protein of major autolysin LytC (CWBP76) | LytB [Bacillus licheniformis DSM 13] | UniRef100_Q65E89 | Bacillus licheniformis DSM 13 |
| 4284 | BLP04105 membrane bound lipoprotein | Hypothetical protein [Bacillus licheniformis DSM 13] | UniRef100_Q65E88 | Bacillus licheniformis DSM 13 |
| 4285 | BLP04795 hypothetical protein | | | |
| 4286 | lytR membrane-bound protein | LytR [Bacillus licheniformis DSM 13] | UniRef100_Q65E87 | Bacillus licheniformis DSM 13 |
| 4287 | mnaA UDP-N-acetylglucosamine 2-epimerase | YvyH [Bacillus licheniformis DSM 13] | UniRef100_Q65E86 | Bacillus licheniformis DSM 13 |
| 4288 | BLP04108 hypothetical protein | | | |
| 4289 | gtaB UTP-glucose-1-phosphate uridylyltransferase | GtaB [Bacillus licheniformis DSM 13] | UniRef100_Q65E85 | Bacillus licheniformis DSM 13 |
| 4290 | tagH ATP-binding protein | TagH [Bacillus licheniformis DSM 13] | UniRef100_Q65E84 | Bacillus licheniformis DSM 13 |
| 4291 | tagG permease | TagG [Bacillus licheniformis DSM 13] | UniRef100_Q65E83 | Bacillus licheniformis DSM 13 |
| 4292 | tagFA Glycosyl transferase family 4 | Hypothetical protein [Bacillus licheniformis DSM 13] | UniRef100_Q65E82 | Bacillus licheniformis DSM 13 |
| 4293 | tagFB CDP-glycerol:polyglycerol phosphate glycero-phosphotransferase | TagF [Bacillus licheniformis DSM 13] | UniRef100_Q65E81 | Bacillus licheniformis DSM 13 |
| 4294 | tagD glycerol-3-phosphate cytidylyltransferase | TagD [Bacillus licheniformis DSM 13] | UniRef100_Q65E80 | Bacillus licheniformis DSM 13 |
| 4295 | tagA Glycosyl Transferase Family 26 | TagA [Bacillus licheniformis DSM 13] | UniRef100_Q65E79 | Bacillus licheniformis DSM 13 |
| 4296 | tagB putative CDP-glycerol glycerophosphotransferase TagB | Hypothetical protein tagB [Bacillus licheniformis DSM 13] | UniRef100_Q65E78 | Bacillus licheniformis DSM 13 |
| 4297 | lytD N-acetylglucosaminidase Glycoside Hydrolase Family 73 | LytD [Bacillus licheniformis DSM 13] | UniRef100_Q65E77 | Bacillus licheniformis DSM 13 |
| 4298 | pmi mannose-6-phosphate isomerase | Pmi [Bacillus licheniformis DSM 13] | UniRef100_Q65E76 | Bacillus licheniformis DSM 13 |
| 4299 | BLP04119 iron-binding, putative oxidoreductase | Hypothetical protein [Bacillus licheniformis DSM 13] | UniRef100_Q65E75 | Bacillus licheniformis DSM 13 |
| 4300 | BLP04120 hypothetical protein | Hypothetical protein [Bacillus licheniformis DSM 13] | UniRef100_Q65E74 | Bacillus licheniformis DSM 13 |
| 4301 | kdgT 2-keto-3-deoxygluconate permease | KdgT [Bacillus licheniformis DSM 13] | UniRef100_Q65E73 | Bacillus licheniformis DSM 13 |
| 4302 | kdgA 2-keto-3-deoxygluconate-6-phosphate aldolase | 2-keto-3-deoxygluconate-6-phosphate aldolase [Bacillus licheniformis DSM 13] | UniRef100_Q62PP2 | Bacillus licheniformis DSM 13 |
| 4303 | kdgK 2-keto-3-deoxygluconate kinase | KdgK [Bacillus licheniformis DSM 13] | UniRef100_Q65E71 | Bacillus licheniformis DSM 13 |
| 4304 | kdgR transcriptional regulator | KdgR [Bacillus licheniformis DSM 13] | UniRef100_Q65E70 | Bacillus licheniformis DSM 13 |
| 4305 | BLP04125 hypothetical protein | | | |
| 4306 | kduI 5-keto-4-deoxyuronate isomerase | KduI [Bacillus licheniformis DSM 13] | UniRef100_Q65E69 | Bacillus licheniformis DSM 13 |
| 4307 | kduD 2-keto-3-deoxygluconate oxidoreductase | KduD [Bacillus licheniformis DSM 13] | UniRef100_Q65E68 | Bacillus licheniformis DSM 13 |
| 4308 | adhB alcohol dehydrogenase | AdhB [Bacillus licheniformis DSM 13] | UniRef100_Q65E67 | Bacillus licheniformis DSM 13 |
| 4309 | ywtG Sugar transporter YwtG | YwtG [Bacillus licheniformis DSM 13] | UniRef100_Q65E66 | Bacillus licheniformis DSM 13 |

| 4310 | ywtF Cell envelope-related transcriptional attenuator YwtF | YwtF [Bacillus licheniformis DSM 13] | UniRef100_Q65E65 | Bacillus licheniformis DSM 13 |
|---|---|---|---|---|
| 4311 | ywtE putative hydrolase YwtE | YwtE [Bacillus licheniformis DSM 13] | UniRef100_Q65E64 | Bacillus licheniformis DSM 13 |
| 4312 | pgdS gamma-DL-glutamyl hydrolase | YwtD [Bacillus licheniformis DSM 13] | UniRef100_Q65E63 | Bacillus licheniformis DSM 13 |
| 4313 | pgsE PgsE | Hypothetical protein ywtC [Bacillus licheniformis DSM 13] | UniRef100_Q65E62 | Bacillus licheniformis DSM 13 |
| 4314 | pgsAA poly-gamma-glutamate synthesis protein | YwtB [Bacillus licheniformis DSM 13] | UniRef100_Q65E61 | Bacillus licheniformis DSM 13 |
| 4315 | pgsC poly-gamma-glutamate synthesis, Capsule biosynthesis protein | YwtA [Bacillus licheniformis DSM 13] | UniRef100_Q65E60 | Bacillus licheniformis DSM 13 |
| 4316 | pgsB poly-gamma-glutamate synthesis protein | YwsC [Bacillus licheniformis DSM 13] | UniRef100_Q65E59 | Bacillus licheniformis DSM 13 |
| 4317 | rbsR transcriptional regulator (LacI family) | RbsR [Bacillus licheniformis DSM 13] | UniRef100_Q65E58 | Bacillus licheniformis DSM 13 |
| 4318 | rbsK ribokinase | RbsK [Bacillus licheniformis DSM 13] | UniRef100_Q65E57 | Bacillus licheniformis DSM 13 |
| 4319 | rbsD ribose ABC transporter (membrane protein) | RbsD [Bacillus licheniformis DSM 13] | UniRef100_Q65E56 | Bacillus licheniformis DSM 13 |
| 4320 | rbsA ribose ABC transporter (ATP-binding protein) | RbsA [Bacillus licheniformis DSM 13] | UniRef100_Q65E55 | Bacillus licheniformis DSM 13 |
| 4321 | rbsC ribose ABC transporter (permease) | RbsC [Bacillus licheniformis DSM 13] | UniRef100_Q65E54 | Bacillus licheniformis DSM 13 |
| 4322 | rbsB ribose ABC transporter (ribose-binding protein) | RbsB [Bacillus licheniformis DSM 13] | UniRef100_Q65E53 | Bacillus licheniformis DSM 13 |
| 4323 | BLP04143 hypothetical protein | | | |
| 4324 | alsD alpha-acetolactate decarboxylase | AlsD [Bacillus licheniformis DSM 13] | UniRef100_Q65E52 | Bacillus licheniformis DSM 13 |
| 4325 | alsS alpha-acetolactate synthase (pH6) | AlsS [Bacillus licheniformis DSM 13] | UniRef100_Q65E51 | Bacillus licheniformis DSM 13 |
| 4326 | alsR transcriptional regulator (LysR family) | AlsR [Bacillus licheniformis DSM 13] | UniRef100_Q65E50 | Bacillus licheniformis DSM 13 |
| 4327 | ywrD Peptidase T3, gamma-glutamyltranspeptidase | YwrD [Bacillus licheniformis DSM 13] | UniRef100_Q65E49 | Bacillus licheniformis DSM 13 |
| 4328 | ywrC transcriptional regulator YwrC | YwrC [Bacillus licheniformis DSM 13] | UniRef100_Q65E48 | Bacillus licheniformis DSM 13 |
| 4329 | ywrB Chromate transporter YwrB | YwrB [Bacillus licheniformis DSM 13] | UniRef100_Q65E47 | Bacillus licheniformis DSM 13 |
| 4330 | ywrA Chromate transporter YwrA | YwrA [Bacillus licheniformis DSM 13] | UniRef100_Q65E46 | Bacillus licheniformis DSM 13 |
| 4331 | ywqE protein tyrosine phoshpatase | YwqE [Bacillus licheniformis DSM 13] | UniRef100_Q65E45 | Bacillus licheniformis DSM 13 |
| 4332 | ywqC Modulator of tyrosine kinase | YwqC [Bacillus licheniformis DSM 13] | UniRef100_Q65E44 | Bacillus licheniformis DSM 13 |
| 4333 | BLP04153 conserved hypothetical protein | Hypothetical protein [Bacillus licheniformis DSM 13] | UniRef100_Q65E43 | Bacillus licheniformis DSM 13 |
| 4334 | ywqB YwqB | YwqB [Bacillus licheniformis DSM 13] | UniRef100_Q65E42 | Bacillus licheniformis DSM 13 |
| 4335 | ywqA putative helicase YwqA | YwqA [Bacillus licheniformis DSM 13] | UniRef100_Q65E41 | Bacillus licheniformis DSM 13 |
| 4336 | BLP04516 conserved hypothetical protein | Hypothetical protein [Bacillus licheniformis DSM 13] | UniRef100_Q65E40 | Bacillus licheniformis DSM 13 |
| 4337 | ywpJ putative hydrolase YwpJ | YwpJ [Bacillus licheniformis DSM 13] | UniRef100_Q65E39 | Bacillus licheniformis DSM 13 |
| 4338 | glcR transcriptional regulator (DeoR family) | GlcR [Bacillus licheniformis DSM 13] | UniRef100_Q65E38 | Bacillus licheniformis DSM 13 |
| 4339 | BLP04159 putative Transaldolase | Hypothetical protein [Bacillus licheniformis DSM 13] | UniRef100_Q65E37 | Bacillus licheniformis DSM 13 |
| 4340 | BLP04160 putative oxidoreductase | Hypothetical protein [Bacillus licheniformis DSM 13] | UniRef100_Q65E36 | Bacillus licheniformis DSM 13 |
| 4341 | BLP04161 putative phosphotransferase | Hypothetical protein [Bacillus licheniformis DSM 13] | UniRef100_Q65E35 | Bacillus licheniformis DSM 13 |
| 4342 | BLP04162 putative phosphotransferase | Hypothetical protein [Bacillus licheniformis DSM 13] | UniRef100_Q65E34 | Bacillus licheniformis DSM 13 |
| 4343 | BLP04163 Phosphotransferase enzyme II | Hypothetical protein [Bacillus licheniformis DSM 13] | UniRef100_Q65E33 | Bacillus licheniformis DSM 13 |
| 4344 | BLP04164 putative transcriptional activator | Hypothetical protein [Bacillus licheniformis DSM 13] | UniRef100_Q65E32 | Bacillus licheniformis DSM 13 |

| 4345 | BLP04165 transcriptional regulator | Hypothetical protein [Bacillus licheniformis DSM 13] | UniRef100_Q65E31 | Bacillus licheniformis DSM 13 |
|---|---|---|---|---|
| 4346 | ywpH Single-strand binding protein | YwpH [Bacillus licheniformis DSM 13] | UniRef100_Q65E30 | Bacillus licheniformis DSM 13 |
| 4347 | ywpG YwpG | Hypothetical protein ywpG [Bacillus licheniformis DSM 13] | UniRef100_Q65E29 | Bacillus licheniformis DSM 13 |
| 4348 | ywpF conserved protein YwpF | Hypothetical protein ywpF [Bacillus licheniformis DSM 13] | UniRef100_Q65E28 | Bacillus licheniformis DSM 13 |
| 4349 | mscL large conductance mechanosensitive channel protein | MscL [Bacillus licheniformis DSM 13] | UniRef100_Q65E27 | Bacillus licheniformis DSM 13 |
| 4350 | fabZ Beta-hydroxyacyl-(acyl-carrier-protein) dehydratase FabZ | YwpB (Beta-hydroxyacyl-(Acyl-carrier-protein) dehydratase FabZ) [Bacillus licheniformis DSM 13] | UniRef100_Q65E26 | Bacillus licheniformis DSM 13 |
| 4351 | flhP flagellar hook-basal body protein | FlhP [Bacillus licheniformis DSM 13] | UniRef100_Q65E25 | Bacillus licheniformis DSM 13 |
| 4352 | flhO flagellar basal-body rod protein | FlhO [Bacillus licheniformis DSM 13] | UniRef100_Q65E24 | Bacillus licheniformis DSM 13 |
| 4353 | BLP04174 hypothetical protein | | | |
| 4354 | mbl MreB-like protein | Mbl [Bacillus licheniformis DSM 13] | UniRef100_Q65E23 | Bacillus licheniformis DSM 13 |
| 4355 | spoIIID transcriptional regulator | SpoIIID [Bacillus licheniformis DSM 13] | UniRef100_Q65E22 | Bacillus licheniformis DSM 13 |
| 4356 | usd Usd | Hypothetical protein usd [Bacillus licheniformis DSM 13] | UniRef100_Q62PJ3 | Bacillus licheniformis DSM 13 |
| 4357 | ywoH putative transcriptional regulator (MarR family) | YwoH [Bacillus licheniformis DSM 13] | UniRef100_Q65E21 | Bacillus licheniformis DSM 13 |
| 4358 | ywoG putative transporter | YwoG [Bacillus licheniformis DSM 13] | UniRef100_Q65E20 | Bacillus licheniformis DSM 13 |
| 4359 | bcrC Acid phosphatase_vanadium-dependent haloperoxidase | YwoA [Bacillus licheniformis DSM 13] | UniRef100_Q65E19 | Bacillus licheniformis DSM 13 |
| 4360 | ppaC inorganic pyrophosphatase | PpaC [Bacillus licheniformis DSM 13] | UniRef100_Q65E18 | Bacillus licheniformis DSM 13 |
| 4361 | ydbD general stress protein,putative manganese-containing catalase | YdbD [Bacillus licheniformis DSM 13] | UniRef100_Q65E17 | Bacillus licheniformis DSM 13 |
| 4362 | yqjF conserved protein YqjF | Hypothetical protein yqjF [Bacillus licheniformis DSM 13] | UniRef100_Q65E16 | Bacillus licheniformis DSM 13 |
| 4363 | BLP04183 conserved hypothetical protein | Hypothetical protein [Bacillus licheniformis DSM 13] | UniRef100_Q65E15 | Bacillus licheniformis DSM 13 |
| 4364 | yqxL putative metal ion transporter protein, CorA-like protein | YqxL [Bacillus licheniformis DSM 13] | UniRef100_Q65E14 | Bacillus licheniformis DSM 13 |
| 4365 | yqhB 2-oxo acid dehydrogenase, lipoyl-binding site protein | YqhB [Bacillus licheniformis DSM 13] | UniRef100_Q65E13 | Bacillus licheniformis DSM 13 |
| 4366 | nrgAB ammonium transporter | NrgA [Bacillus licheniformis DSM 13] | UniRef100_Q65E12 | Bacillus licheniformis DSM 13 |
| 4367 | nrgBB nitrogen-regulated PII-like protein | NrgB [Bacillus licheniformis DSM 13] | UniRef100_Q65E11 | Bacillus licheniformis DSM 13 |
| 4368 | BLP04188 ATP-dependent Clp like protease proteolytic subunit (class III heat-shock protein) | Hypothetical protein [Bacillus licheniformis DSM 13] | UniRef100_Q65E10 | Bacillus licheniformis DSM 13 |
| 4369 | BLP04189 RNA polymerase ECF(extracytoplasmic function)-type sigma factor (sigma-M) | Hypothetical protein (RNA polymerase ECF(Extracytoplasmic function)- type sigma factor) [Bacillus licheniformis DSM 13] | UniRef100_Q65E09 | Bacillus licheniformis DSM 13 |
| 4370 | BLP04190 hypothetical protein | | | |
| 4371 | spoIIQ SpoIIQ | Hypothetical protein spoIIQ [Bacillus licheniformis DSM 13] | UniRef100_Q65E08 | Bacillus licheniformis DSM 13 |
| 4372 | ywnH putative phosphinothricin acetyltransferase | YwnH [Bacillus licheniformis DSM 13] | UniRef100_Q65E07 | Bacillus licheniformis DSM 13 |
| 4373 | ywnF putative Beta-ketoacyl synthase | YwnF [Bacillus licheniformis DSM 13] | UniRef100_Q65E06 | Bacillus licheniformis DSM 13 |
| 4374 | ywnE cardiolipin synthetase | YwnE [Bacillus licheniformis DSM 13] | UniRef100_Q65E05 | Bacillus licheniformis DSM 13 |
| 4375 | BLP04195 putative transporter EmrB_QacA subfamily | Hypothetical protein [Bacillus licheniformis DSM 13] | UniRef100_Q65E04 | Bacillus licheniformis DSM 13 |
| 4376 | BLP04196 putative siderophore biosynthesis MtbH like | Hypothetical protein [Bacillus licheniformis DSM 13] | UniRef100_Q65E03 | Bacillus licheniformis DSM 13 |

| | protein | | | |
|---|---|---|---|---|
| 4377 | dhbF putative Nonribosomal peptide synthetase DhbF | Hypothetical protein dhbF [Bacillus licheniformis DSM 13] | UniRef100_Q65E02 | Bacillus licheniformis DSM 13 |
| 4378 | dhbB isochorismatase | DhbB [Bacillus licheniformis DSM 13] | UniRef100_Q65E01 | Bacillus licheniformis DSM 13 |
| 4379 | dhbE 2,3-dihydroxybenzoate-AMP ligase (enterobactin synthetase component E) | DhbE [Bacillus licheniformis DSM 13] | UniRef100_Q65E00 | Bacillus licheniformis DSM 13 |
| 4380 | dhbC isochorismate synthase | DhbC [Bacillus licheniformis DSM 13] | UniRef100_Q65DZ9 | Bacillus licheniformis DSM 13 |
| 4381 | dhbA 2,3-dihydro-2,3-dihydroxybenzoate dehydrogenase | DhbA [Bacillus licheniformis DSM 13] | UniRef100_Q65DZ8 | Bacillus licheniformis DSM 13 |
| 4382 | yuiI Putative esterase | YuiI [Bacillus licheniformis DSM 13] | UniRef100_Q65DZ7 | Bacillus licheniformis DSM 13 |
| 4383 | feuC integral membrane protein | FeuC [Bacillus licheniformis DSM 13] | UniRef100_Q65DZ6 | Bacillus licheniformis DSM 13 |
| 4384 | feuB integral membrane protein | FeuB [Bacillus licheniformis DSM 13] | UniRef100_Q65DZ5 | Bacillus licheniformis DSM 13 |
| 4385 | feuA iron-binding protein | FeuA [Bacillus licheniformis DSM 13] | UniRef100_Q65DZ4 | Bacillus licheniformis DSM 13 |
| 4386 | ybbB probable transcriptional regulator (AraC_XylS family) | YbbB [Bacillus licheniformis DSM 13] | UniRef100_Q65DZ3 | Bacillus licheniformis DSM 13 |
| 4387 | ywnC YwnC | Hypothetical protein ywnG [Bacillus licheniformis DSM 13] | UniRef100_Q65DZ2 | Bacillus licheniformis DSM 13 |
| 4388 | ywnB YwnB | Hypothetical protein ywnB [Bacillus licheniformis DSM 13] | UniRef100_Q65DY9 | Bacillus licheniformis DSM 13 |
| 4389 | ywnA putative transcriptional regulator | YwnA [Bacillus licheniformis DSM 13] | UniRef100_Q65DY8 | Bacillus licheniformis DSM 13 |
| 4390 | BLP04909 conserved hypothetical protein | Hypothetical protein [Bacillus licheniformis DSM 13] | UniRef100_Q65DY7 | Bacillus licheniformis DSM 13 |
| 4391 | ywmF YwmF | Hypothetical protein ywmF [Bacillus licheniformis DSM 13] | UniRef100_Q65DY6 | Bacillus licheniformis DSM 13 |
| 4392 | moaA MoaA | MoaA [Bacillus licheniformis DSM 13] | UniRef100_Q65DY5 | Bacillus licheniformis DSM 13 |
| 4393 | fdhD FdhD | Hypothetical protein fdhD [Bacillus licheniformis DSM 13] | UniRef100_Q65DY4 | Bacillus licheniformis DSM 13 |
| 4394 | BLP04214 putative transporter | Hypothetical protein [Bacillus licheniformis DSM 13] | UniRef100_Q65DY3 | Bacillus licheniformis DSM 13 |
| 4395 | BLP04215 Oxidoreductase alpha (molybdopterin) subunit | Hypothetical protein (EC 1.2.1.2) (Oxidoreductase alpha (Molybdopterin) subunit) [Bacillus licheniformis DSM 13] | UniRef100_Q65DY2 | Bacillus licheniformis DSM 13 |
| 4396 | ywmD YwmD | Von Willebrand factor, type A [Bacillus licheniformis DSM 13] | UniRef100_Q62PF2 | Bacillus licheniformis DSM 13 |
| 4397 | ywmC YwmC | YwmC [Bacillus licheniformis DSM 13] | UniRef100_Q65DY0 | Bacillus licheniformis DSM 13 |
| 4398 | BLP04218 hypothetical protein | | | |
| 4399 | spoIID SpoIID | Hypothetical protein spoIID [Bacillus licheniformis DSM 13] | UniRef100_Q65DX9 | Bacillus licheniformis DSM 13 |
| 4400 | murAA UDP-N-acetylglucosamine 1-carboxyvinyltransferase | MurAA [Bacillus licheniformis DSM 13] | UniRef100_Q65DX8 | Bacillus licheniformis DSM 13 |
| 4401 | ywmB conserved protein YwmB | Hypothetical protein ywmB [Bacillus licheniformis DSM 13] | UniRef100_Q65DX7 | Bacillus licheniformis DSM 13 |
| 4402 | ywzB conserved protein YwzB | Hypothetical protein ywzB [Bacillus licheniformis DSM 13] | UniRef100_Q65DX6 | Bacillus licheniformis DSM 13 |
| 4403 | atpC ATP synthase (subunit epsilon) | AtpC [Bacillus licheniformis DSM 13] | UniRef100_Q65DX5 | Bacillus licheniformis DSM 13 |
| 4404 | atpD ATP synthase (subunit beta) | AtpD [Bacillus licheniformis DSM 13] | UniRef100_Q65DX4 | Bacillus licheniformis DSM 13 |
| 4405 | atpG ATP synthase (subunit gamma) | AtpG [Bacillus licheniformis DSM 13] | UniRef100_Q65DX3 | Bacillus licheniformis DSM 13 |
| 4406 | atpA ATP synthase (subunit alpha) | AtpA [Bacillus licheniformis DSM 13] | UniRef100_Q65DX2 | Bacillus licheniformis DSM 13 |
| 4407 | atpH ATP synthase (subunit delta) | AtpH [Bacillus licheniformis DSM 13] | UniRef100_Q65DX1 | Bacillus licheniformis DSM 13 |
| 4408 | atpF ATP synthase (subunit b) | AtpF [Bacillus licheniformis DSM 13] | UniRef100_Q65DX0 | Bacillus licheniformis DSM 13 |

| 4409 | atpE ATP synthase C chain AtpE | AtpE [Bacillus licheniformis DSM 13] | UniRef100_Q65DW9 | Bacillus licheniformis DSM 13 |
|---|---|---|---|---|
| 4410 | atpB ATP synthase (subunit a) | AtpB [Bacillus licheniformis DSM 13] | UniRef100_Q65DW8 | Bacillus licheniformis DSM 13 |
| 4411 | atpI ATP synthase (subunit i) | AtpI [Bacillus licheniformis DSM 13] | UniRef100_Q65DW7 | Bacillus licheniformis DSM 13 |
| 4412 | upp uracil phosphoribosyltransferase | Upp [Bacillus licheniformis DSM 13] | UniRef100_Q65DW6 | Bacillus licheniformis DSM 13 |
| 4413 | glyA serine hydroxymethyltransferase | GlyA [Bacillus licheniformis DSM 13] | UniRef100_Q65DW5 | Bacillus licheniformis DSM 13 |
| 4414 | ywlG conserved protein YwlG | Hypothetical protein ywlG [Bacillus licheniformis DSM 13] | UniRef100_Q65DW4 | Bacillus licheniformis DSM 13 |
| 4415 | ywlF Ribose_galactose isomerase | YwlF [Bacillus licheniformis DSM 13] | UniRef100_Q65DW3 | Bacillus licheniformis DSM 13 |
| 4416 | ywlE Low molecular weight phosphotyrosine protein phosphatase | YwlE [Bacillus licheniformis DSM 13] | UniRef100_Q65DW2 | Bacillus licheniformis DSM 13 |
| 4417 | BLP04236 hypothetical protein | | | |
| 4418 | ywlD YwlD | Hypothetical protein ywlD [Bacillus licheniformis DSM 13] | UniRef100_Q65DW1 | Bacillus licheniformis DSM 13 |
| 4419 | ywlC conserved protein YwlC | YwlC [Bacillus licheniformis DSM 13] | UniRef100_Q65DW0 | Bacillus licheniformis DSM 13 |
| 4420 | ywlB YwlB | Hypothetical protein ywlB [Bacillus licheniformis DSM 13] | UniRef100_Q65DV9 | Bacillus licheniformis DSM 13 |
| 4421 | BLP04240 hypothetical protein | | | |
| 4422 | spoIIR SpoIIR | SpoIIR [Bacillus licheniformis DSM 13] | UniRef100_Q65DV8 | Bacillus licheniformis DSM 13 |
| 4423 | BLP04796 hypothetical protein | | | |
| 4424 | glcP glucose_mannose:H symporter | GlcP [Bacillus licheniformis DSM 13] | UniRef100_Q65DV7 | Bacillus licheniformis DSM 13 |
| 4425 | ntdC putative oxidoreductase | YhjJ [Bacillus licheniformis DSM 13] | UniRef100_Q65DV6 | Bacillus licheniformis DSM 13 |
| 4426 | ntdB HAD-superfamily hydrolase, subfamily IIB | YhjK [Bacillus licheniformis DSM 13] | UniRef100_Q65DV5 | Bacillus licheniformis DSM 13 |
| 4427 | ntdA putative aminotransferase | YhjL [Bacillus licheniformis DSM 13] | UniRef100_Q65DV4 | Bacillus licheniformis DSM 13 |
| 4428 | ntdR transcriptional activator of ntdABC operon (LacI family) | YhjM [Bacillus licheniformis DSM 13] | UniRef100_Q65DV3 | Bacillus licheniformis DSM 13 |
| 4429 | BLP04247 hypothetical protein | | | |
| 4430 | ywkE YwkE | YwkE [Bacillus licheniformis DSM 13] | UniRef100_Q65DV2 | Bacillus licheniformis DSM 13 |
| 4431 | prfA peptide chain release factor 1 | PrfA [Bacillus licheniformis DSM 13] | UniRef100_Q65DV1 | Bacillus licheniformis DSM 13 |
| 4432 | ywkD YwkD | YwkD [Bacillus licheniformis DSM 13] | UniRef100_Q65DV0 | Bacillus licheniformis DSM 13 |
| 4433 | racA RacA | YwkC [Bacillus licheniformis DSM 13] | UniRef100_Q65DU9 | Bacillus licheniformis DSM 13 |
| 4434 | ywkB conserved membrane protein | YwkB [Bacillus licheniformis DSM 13] | UniRef100_Q65DU8 | Bacillus licheniformis DSM 13 |
| 4435 | BLP04797 hypothetical protein | | | |
| 4436 | ywkA malate dehyrogenase isozyme | Malic oxidoreductase [Bacillus licheniformis DSM 13] | UniRef100_Q62PB9 | Bacillus licheniformis DSM 13 |
| 4437 | tdk thymidine kinase | Tdk [Bacillus licheniformis DSM 13] | UniRef100_Q65DU6 | Bacillus licheniformis DSM 13 |
| 4438 | rpmE ribosomal protein L31 | RpmE [Bacillus licheniformis DSM 13] | UniRef100_Q65DU5 | Bacillus licheniformis DSM 13 |
| 4439 | rho transcriptional terminator Rho | Transcriptional terminator Rho [Bacillus licheniformis DSM 13] | UniRef100_Q65DU4 | Bacillus licheniformis DSM 13 |
| 4440 | ywjI GlpX | Ywjl [Bacillus licheniformis DSM 13] | UniRef100_Q65DU3 | Bacillus licheniformis DSM 13 |
| 4441 | murAB UDP-N-acetylglucosamine 1-carboxyvinyltransferase | MurAB [Bacillus licheniformis DSM 13] | UniRef100_Q65DU2 | Bacillus licheniformis DSM 13 |
| 4442 | tal Transaldolase C | YwjH [Bacillus licheniformis DSM 13] | UniRef100_Q65DU1 | Bacillus licheniformis DSM 13 |
| 4443 | fbaA fructose-1,6-bisphosphate aldolase | FbaA [Bacillus licheniformis DSM 13] | UniRef100_Q65DU0 | Bacillus licheniformis DSM 13 |

| 4444 | spo0F two-component response regulator | Spo0F [Bacillus licheniformis DSM 13] | UniRef100_Q65DT9 | Bacillus licheniformis DSM 13 |
|---|---|---|---|---|
| 4445 | ywjG YwjG | Hypothetical protein ywjG [Bacillus licheniformis DSM 13] | UniRef100_Q65DT8 | Bacillus licheniformis DSM 13 |
| 4446 | BLP04798 hypothetical protein | | | |
| 4447 | pyrG CTP synthetase | PyrG [Bacillus licheniformis DSM 13] | UniRef100_Q65DT7 | Bacillus licheniformis DSM 13 |
| 4448 | BLP04799 hypothetical protein | | | |
| 4449 | rpoE RNA polymerase (delta subunit) | RpoE [Bacillus licheniformis DSM 13] | UniRef100_Q65DT6 | Bacillus licheniformis DSM 13 |
| 4450 | BLP04265 hypothetical protein | | | |
| 4451 | acdA acyl-CoA dehydrogenase | AcdA [Bacillus licheniformis DSM 13] | UniRef100_Q65DT5 | Bacillus licheniformis DSM 13 |
| 4452 | mmgC acyl-CoA dehydrogenase | MmgC [Bacillus licheniformis DSM 13] | UniRef100_Q65DT4 | Bacillus licheniformis DSM 13 |
| 4453 | mmgB 3-hydroxybutyryl-CoA dehydrogenase | MmgB [Bacillus licheniformis DSM 13] | UniRef100_Q65DT3 | Bacillus licheniformis DSM 13 |
| 4454 | mmgA acetyl-CoA acetyltransferase | MmgA [Bacillus licheniformis DSM 13] | UniRef100_Q65DT2 | Bacillus licheniformis DSM 13 |
| 4455 | ywjF 4Fe-4S ferredoxin protein | YwjF [Bacillus licheniformis DSM 13] | UniRef100_Q65DT1 | Bacillus licheniformis DSM 13 |
| 4456 | ywjE putative Phospholipase | Phospholipase D/Transphosphatidylase,Phospholipase D/Transphosphatidylase [Bacillus licheniformis DSM 13] | UniRef100_Q62PA2 | Bacillus licheniformis DSM 13 |
| 4457 | ywjC YwjC | Hypothetical protein ywjC [Bacillus licheniformis DSM 13] | UniRef100_Q62PA1 | Bacillus licheniformis DSM 13 |
| 4458 | argS arginyl-tRNA synthetase | ArgS [Bacillus licheniformis DSM 13] | UniRef100_Q65DS9 | Bacillus licheniformis DSM 13 |
| 4459 | ywiB conserved protein YwiB | YwiB [Bacillus licheniformis DSM 13] | UniRef100_Q65DS8 | Bacillus licheniformis DSM 13 |
| 4460 | speB agmatinase | SpeB [Bacillus licheniformis DSM 13] | UniRef100_Q65DS7 | Bacillus licheniformis DSM 13 |
| 4461 | speE spermidine synthase | SpeE [Bacillus licheniformis DSM 13] | UniRef100_Q65DS6 | Bacillus licheniformis DSM 13 |
| 4462 | BLP04277 hypothetical protein | Hypothetical protein [Bacillus licheniformis DSM 13] | UniRef100_Q65DS5 | Bacillus licheniformis DSM 13 |
| 4463 | | Hypothetical protein [Bacillus licheniformis DSM 13] | UniRef100_Q65DS4 | Bacillus licheniformis DSM 13 |
| 4464 | ywhE Penicillin-binding protein, Glycosyl Transferase Family 51 | YwhE [Bacillus licheniformis DSM 13] | UniRef100_Q65DS3 | Bacillus licheniformis DSM 13 |
| 4465 | ywhD YwhD | Hypothetical protein ywhD [Bacillus licheniformis DSM 13] | UniRef100_Q65DS2 | Bacillus licheniformis DSM 13 |
| 4466 | ywhC Peptidase M50 | YwhC [Bacillus licheniformis DSM 13] | UniRef100_Q65DS1 | Bacillus licheniformis DSM 13 |
| 4467 | ywhB putative 4-oxalocrotonate tautomerase | YwhB [Bacillus licheniformis DSM 13] | UniRef100_Q65DS0 | Bacillus licheniformis DSM 13 |
| 4468 | BLP04282 hypothetical protein | Hypothetical protein [Bacillus licheniformis DSM 13] | UniRef100_Q65DR9 | Bacillus licheniformis DSM 13 |
| 4469 | BLP04283 hypothetical protein | | | |
| 4470 | ywgA YwgA | Hypothetical protein ywgA [Bacillus licheniformis DSM 13] | UniRef100_Q65DR8 | Bacillus licheniformis DSM 13 |
| 4471 | ywfO Metal-dependent phosphohydrolase, HD region | YwfO [Bacillus licheniformis DSM 13] | UniRef100_Q65DR7 | Bacillus licheniformis DSM 13 |
| 4472 | BLP04286 hypothetical protein | | | |
| 4473 | ywzC YwzC | Hypothetical protein ywzC [Bacillus licheniformis DSM 13] | UniRef100_Q65DR6 | Bacillus licheniformis DSM 13 |
| 4474 | BLP04800 hypothetical protein | | | |
| 4475 | rsfA putative transcriptional regulatory protein | RsfA [Bacillus licheniformis DSM 13] | UniRef100_Q65DR5 | Bacillus licheniformis DSM 13 |
| 4476 | BLP04801 hypothetical protein | | | |
| 4477 | BLP04289 hypothetical protein | YjdJ [Bacillus licheniformis DSM 13] | UniRef100_Q65DR4 | Bacillus licheniformis DSM 13 |
| 4478 | ywfL conserved protein YwfL | YwfL [Bacillus licheniformis DSM 13] | UniRef100_Q65DR3 | Bacillus licheniformis DSM 13 |

| 4479 | BLP04291 4-hydroxybenzoate 3-monooxygenase | Hypothetical protein [Bacillus licheniformis DSM 13] | UniRef100_Q65DR2 | Bacillus licheniformis DSM 13 |
|---|---|---|---|---|
| 4480 | BLP04292 putative transporter | Hypothetical protein [Bacillus licheniformis DSM 13] | UniRef100_Q65DR1 | Bacillus licheniformis DSM 13 |
| 4481 | BLP04293 aldehyde dehydrogenase | Hypothetical protein [Bacillus licheniformis DSM 13] | UniRef100_Q65DQ7 | Bacillus licheniformis DSM 13 |
| 4482 | BLP04294 hypothetical protein | | | |
| 4483 | BLP04295 transcriptional regulator (IclR family) | Hypothetical protein [Bacillus licheniformis DSM 13] | UniRef100_Q65DQ6 | Bacillus licheniformis DSM 13 |
| 4484 | BLP04296 putative decarboxylase | Hypothetical protein [Bacillus licheniformis DSM 13] | UniRef100_Q65DQ5 | Bacillus licheniformis DSM 13 |
| 4485 | pta phosphotransacetylase | Pta [Bacillus licheniformis DSM 13] | UniRef100_Q65DQ4 | Bacillus licheniformis DSM 13 |
| 4486 | ywfI Chlorite dismutase | YwfI [Bacillus licheniformis DSM 13] | UniRef100_Q65DQ3 | Bacillus licheniformis DSM 13 |
| 4487 | BLP04299 hypothetical protein | | | |
| 4488 | BLP04300 conserved hypothetical protein | Hypothetical protein [Bacillus licheniformis DSM 13] | UniRef100_Q65DQ1 | Bacillus licheniformis DSM 13 |
| 4489 | ktrB K transporter integral membrane subunit | YubG [Bacillus licheniformis DSM 13] | UniRef100_Q65DQ0 | Bacillus licheniformis DSM 13 |
| 4490 | gerQ GerQ | YwdL [Bacillus licheniformis DSM 13] | UniRef100_Q65DP9 | Bacillus licheniformis DSM 13 |
| 4491 | yfiN ABC transporter, family 2,ABC transporter, family 2 | YfiN [Bacillus licheniformis DSM 13] | UniRef100_Q65DP8 | Bacillus licheniformis DSM 13 |
| 4492 | yfiM ABC transport system permease protein | Hypothetical protein yfiM [Bacillus licheniformis DSM 13] | UniRef100_Q65DP7 | Bacillus licheniformis DSM 13 |
| 4493 | yfiL ABC transporter YfiL | YfiL [Bacillus licheniformis DSM 13] | UniRef100_Q65DP6 | Bacillus licheniformis DSM 13 |
| 4494 | BLP04306 Binding-protein-dependent transport systems inner membrane component | YxjM [Bacillus licheniformis DSM 13] | UniRef100_Q65DP5 | Bacillus licheniformis DSM 13 |
| 4495 | yfiK two-component response regulator | YfiK [Bacillus licheniformis DSM 13] | UniRef100_Q65DP4 | Bacillus licheniformis DSM 13 |
| 4496 | ywdK YwdK | YwdK [Bacillus licheniformis DSM 13] | UniRef100_Q65DP3 | Bacillus licheniformis DSM 13 |
| 4497 | ywdJ putative transporter YwdJ | YwdJ [Bacillus licheniformis DSM 13] | UniRef100_Q65DP2 | Bacillus licheniformis DSM 13 |
| 4498 | ywdl Ywdl | Hypothetical protein ywdl [Bacillus licheniformis DSM 13] | UniRef100_Q62P64 | Bacillus licheniformis DSM 13 |
| 4499 | BLP04802 hypothetical protein | | | |
| 4500 | ywdH Aldehyde dehydrogenase,Aldehyde dehydrogenase | YwdH [Bacillus licheniformis DSM 13] | UniRef100_Q65DP0 | Bacillus licheniformis DSM 13 |
| 4501 | ung uracil-DNA glycosylase | Ung [Bacillus licheniformis DSM 13] | UniRef100_Q65DN9 | Bacillus licheniformis DSM 13 |
| 4502 | ywdF Glycosyl transferase, family 2, YwdF | Glycosyl transferase, family 2 [Bacillus licheniformis DSM 13] | UniRef100_Q62P61 | Bacillus licheniformis DSM 13 |
| 4503 | thiD phosphomethylpyrimidine kinase | ThiD [Bacillus licheniformis DSM 13] | UniRef100_Q65DN7 | Bacillus licheniformis DSM 13 |
| 4504 | ywdA hypothetical protein | Hypothetical protein ywdA [Bacillus licheniformis DSM 13] | UniRef100_Q65DN6 | Bacillus licheniformis DSM 13 |
| 4505 | sacA Glycoside Hydrolase Family 32 | SacA [Bacillus licheniformis DSM 13] | UniRef100_Q65DN5 | Bacillus licheniformis DSM 13 |
| 4506 | sacP phosphotransferase system (PTS) sucrose-specific enzyme IIBC component | SacP (Phosphotransferase system (PTS) sucrose-specific enzyme IIBC component) [Bacillus licheniformis DSM 13] | UniRef100_Q65DN4 | Bacillus licheniformis DSM 13 |
| 4507 | sacT transcriptional antiterminator | SacT [Bacillus licheniformis DSM 13] | UniRef100_Q65DN3 | Bacillus licheniformis DSM 13 |
| 4508 | BLP04319 hypothetical protein | Hypothetical protein [Bacillus licheniformis DSM 13] | UniRef100_Q62P55 | Bacillus licheniformis DSM 13 |
| 4509 | vpr extracellular serine protease | | | |
| 4510 | BLP04321 hypothetical protein | | | |
| 4511 | BLP04322 hypothetical protein | Hypothetical protein [Bacillus licheniformis DSM 13] | UniRef100_Q62P53 | Bacillus licheniformis DSM 13 |
| 4512 | ywcH putative oxygenase | YwcH [Bacillus licheniformis DSM 13] | UniRef100_Q65DN0 | Bacillus licheniformis DSM 13 |

| 4513 | BLP04324 hypothetical protein | | | |
|------|-------------------------------|---|---|---|
| 4514 | nfrA1 FMN-containing NADPH-linked nitro_flavin reductase | NfrA [Bacillus licheniformis DSM 13] | UniRef100_Q65DM9 | Bacillus licheniformis DSM 13 |
| 4515 | BLP04326 Peptidase M20D, amidohydrolase | Hypothetical protein [Bacillus licheniformis DSM 13] | UniRef100_Q65DM8 | Bacillus licheniformis DSM 13 |
| 4516 | rodA RodA | Hypothetical protein rodA [Bacillus licheniformis DSM 13] | UniRef100_Q65DM7 | Bacillus licheniformis DSM 13 |
| 4517 | rodAA cell division protein | Hypothetical protein [Bacillus licheniformis DSM 13] | UniRef100_Q65DM6 | Bacillus licheniformis DSM 13 |
| 4518 | BLP04329 hypothetical protein | | | |
| 4519 | BLP04330 conserved hypothetical protein | Hypothetical protein [Bacillus licheniformis DSM 13] | UniRef100_Q65DM5 | Bacillus licheniformis DSM 13 |
| 4520 | ywcE conserved hypothetical protein YwcE | Hypothetical protein ywcE [Bacillus licheniformis DSM 13] | UniRef100_Q65DM3 | Bacillus licheniformis DSM 13 |
| 4521 | BLP04332 hypothetical protein | Hypothetical protein [Bacillus licheniformis DSM 13] | UniRef100_Q65DM2 | Bacillus licheniformis DSM 13 |
| 4522 | BLP04333 putative N-acetyltransferase | YfmK [Bacillus licheniformis DSM 13] | UniRef100_Q65DM1 | Bacillus licheniformis DSM 13 |
| 4523 | BLP04334 ABC transporter | Hypothetical protein [Bacillus licheniformis DSM 13] | UniRef100_Q65DM0 | Bacillus licheniformis DSM 13 |
| 4524 | BLP04335 ABC transporter | Hypothetical protein [Bacillus licheniformis DSM 13] | UniRef100_Q65DL9 | Bacillus licheniformis DSM 13 |
| 4525 | BLP04336 hypothetical protein | Hypothetical protein [Bacillus licheniformis DSM 13] | UniRef100_Q65DL8 | Bacillus licheniformis DSM 13 |
| 4526 | BLP04337 hypothetical protein | Hypothetical protein [Bacillus licheniformis DSM 13] | UniRef100_Q65DL7 | Bacillus licheniformis DSM 13 |
| 4527 | BLP04338 hypothetical protein | Hypothetical protein [Bacillus licheniformis DSM 13] | UniRef100_Q65DL6 | Bacillus licheniformis DSM 13 |
| 4528 | BLP04339 two-component response regulator | Hypothetical protein [Bacillus licheniformis DSM 13] | UniRef100_Q65DL5 | Bacillus licheniformis DSM 13 |
| 4529 | BLP04340 hypothetical protein | Hypothetical protein [Bacillus licheniformis DSM 13] | UniRef100_Q62P36 | Bacillus licheniformis DSM 13 |
| 4530 | qoxD cytochrome aa3 quinol oxidase (subunit IV) | QoxD [Bacillus licheniformis DSM 13] | UniRef100_Q65DL4 | Bacillus licheniformis DSM 13 |
| 4531 | qoxC cytochrome aa3 quinol oxidase (subunit III) | QoxC [Bacillus licheniformis DSM 13] | UniRef100_Q65DL3 | Bacillus licheniformis DSM 13 |
| 4532 | qoxB cytochrome aa3 quinol oxidase (subunit I) | QoxB [Bacillus licheniformis DSM 13] | UniRef100_Q65DL2 | Bacillus licheniformis DSM 13 |
| 4533 | qoxA cytochrome aa3 quinol oxidase (subunit II) | QoxA [Bacillus licheniformis DSM 13] | UniRef100_Q65DL1 | Bacillus licheniformis DSM 13 |
| 4534 | galE UDP-glucose 4-epimerase | GalE [Bacillus licheniformis DSM 13] | UniRef100_Q65DL0 | Bacillus licheniformis DSM 13 |
| 4535 | BLP04346 hypothetical protein | Hypothetical protein [Bacillus licheniformis DSM 13] | UniRef100_Q62P30 | Bacillus licheniformis DSM 13 |
| 4536 | BLP04347 sporulation inhibition like protein | Hypothetical protein [Bacillus licheniformis DSM 13] | UniRef100_Q65DK9 | Bacillus licheniformis DSM 13 |
| 4537 | BLP04348 hypothetical protein | Hypothetical protein [Bacillus licheniformis DSM 13] | UniRef100_Q62P28 | Bacillus licheniformis DSM 13 |
| 4538 | BLP04349 conserved hypothetical protein YwqN | YwqN [Bacillus licheniformis DSM 13] | UniRef100_Q65DK7 | Bacillus licheniformis DSM 13 |
| 4539 | ywcB conserved protein YwcB | Hypothetical protein ywcB [Bacillus licheniformis DSM 13] | UniRef100_Q65DK6 | Bacillus licheniformis DSM 13 |
| 4540 | ywcA Na _solute symporter | YwcA [Bacillus licheniformis DSM 13] | UniRef100_Q65DK5 | Bacillus licheniformis DSM 13 |
| 4541 | xylB xylulose kinase | XylB [Bacillus licheniformis DSM 13] | UniRef100_Q65DK4 | Bacillus licheniformis DSM 13 |
| 4542 | xylA xylose isomerase | Xylose isomerase [Bacillus licheniformis] | UniRef100_P77832 | Bacillus licheniformis |
| 4543 | xylR transcriptional regulator XylR | XylR [Bacillus licheniformis DSM 13] | UniRef100_Q65DK2 | Bacillus licheniformis DSM 13 |
| 4544 | BLP04803 hypothetical protein | Hypothetical protein [Bacillus licheniformis DSM 13] | UniRef100_Q65DK1 | Bacillus licheniformis DSM 13 |
| 4545 | thiE thiamine-phosphate pyrophosphorylase | ThiE [Bacillus licheniformis DSM 13] | UniRef100_Q65DK0 | Bacillus licheniformis DSM 13 |
| 4546 | thiM hydroxyethylthiazole kinase | ThiM [Bacillus licheniformis DSM 13] | UniRef100_Q65DJ9 | Bacillus licheniformis DSM 13 |
| 4547 | BLP04358 hypothetical protein | Putative HTH-type transcriptional regulator ywbl [Bacillus subtilis] | UniRef100_P39592 | Bacillus subtilis |

| 4548 | ywqIA conserved hypothetical protein | Hypothetical protein ywqIA [Bacillus licheniformis DSM 13] | UniRef100_Q65DJ6 | Bacillus licheniformis DSM 13 |
|---|---|---|---|---|
| 4549 | ywqH conserved hypothetical protein | Hypothetical protein [Bacillus licheniformis DSM 13] | UniRef100_Q62P15 | Bacillus licheniformis DSM 13 |
| 4550 | ywbH conserved membrane protein YwbH | YwbH [Bacillus licheniformis DSM 13] | UniRef100_Q65DJ4 | Bacillus licheniformis DSM 13 |
| 4551 | ywbG peptidoglycan hydrolase YwbG | Hypothetical protein ywbG [Bacillus licheniformis DSM 13] | UniRef100_Q65DJ3 | Bacillus licheniformis DSM 13 |
| 4552 | BLP04363 hypothetical protein | Hypothetical protein [Bacillus licheniformis DSM 13] | UniRef100_Q62P12 | Bacillus licheniformis DSM 13 |
| 4553 | BLP04364 proline transporter | Hypothetical protein [Bacillus licheniformis DSM 13] | UniRef100_Q65DJ2 | Bacillus licheniformis DSM 13 |
| 4554 | ywbD SAM (and some other nucleotide) binding motif,PUA domain | YwbD (SAM (And some other nucleotide) binding motif,PUA domain) [Bacillus licheniformis DSM 13] | UniRef100_Q65DJ1 | Bacillus licheniformis DSM 13 |
| 4555 | ywbC putative lyase YwbC | YwbC [Bacillus licheniformis DSM 13] | UniRef100_Q65DJ0 | Bacillus licheniformis DSM 13 |
| 4556 | BLP04367 D-isomer specific 2-hydroxyacid dehydrogenase, NAD binding domain | YoaD [Bacillus licheniformis DSM 13] | UniRef100_Q65DI9 | Bacillus licheniformis DSM 13 |
| 4557 | gspA Glycosyl Transferase Family 8 | GspA [Bacillus licheniformis DSM 13] | UniRef100_Q65DI7 | Bacillus licheniformis DSM 13 |
| 4558 | BLP04369 hypothetical protein | | | |
| 4559 | BLP04370 hypothetical protein | | | |
| 4560 | BLP04371 hypothetical protein | | | |
| 4561 | ydfH Histidine kinase YdfH | YdfH [Bacillus licheniformis DSM 13] | UniRef100_Q65DI6 | Bacillus licheniformis DSM 13 |
| 4562 | ydfI putative transcriptional regulator Ydfl | Ydfl [Bacillus licheniformis DSM 13] | UniRef100_Q65DI5 | Bacillus licheniformis DSM 13 |
| 4563 | ydfJ Sterol-sensing 5TM box YdfJ | YdfJ [Bacillus licheniformis DSM 13] | UniRef100_Q65DI4 | Bacillus licheniformis DSM 13 |
| 4564 | BLP04375 2-keto-3-deoxygluconate kinase | Hypothetical protein [Bacillus licheniformis DSM 13] | UniRef100_Q65DH9 | Bacillus licheniformis DSM 13 |
| 4565 | BLP04376 transcriptional regulator (IclR family) | Hypothetical protein [Bacillus licheniformis DSM 13] | UniRef100_Q65DH8 | Bacillus licheniformis DSM 13 |
| 4566 | ywaD Putative aminopeptidase | YwaD [Bacillus licheniformis DSM 13] | UniRef100_Q65DH7 | Bacillus licheniformis DSM 13 |
| 4567 | ywoD Major facilitator superfamily protein YwoD | YwoD [Bacillus licheniformis DSM 13] | UniRef100_Q65DH6 | Bacillus licheniformis DSM 13 |
| 4568 | BLP04379 hypothetical protein | Hypothetical protein [Bacillus licheniformis DSM 13] | UniRef100_Q65DH5 | Bacillus licheniformis DSM 13 |
| 4569 | BLP04380 hypothetical protein | | | |
| 4570 | ywaC putative GTP-pyrophosphokinase YwaC | YwaC [Bacillus licheniformis DSM 13] | UniRef100_Q65DH3 | Bacillus licheniformis DSM 13 |
| 4571 | BLP04382 hypothetical protein | | | |
| 4572 | BLP04383 hypothetical protein | DltA [Bacillus licheniformis DSM 13] | UniRef100_Q65DH2 | Bacillus licheniformis DSM 13 |
| 4573 | dltA D-alanyl-D-alanine carrier protein ligase (Dcl) | DltA [Bacillus licheniformis DSM 13] | UniRef100_Q65DH1 | Bacillus licheniformis DSM 13 |
| 4574 | dltB DltB | Hypothetical protein dltB [Bacillus licheniformis DSM 13] | UniRef100_Q65DH0 | Bacillus licheniformis DSM 13 |
| 4575 | dltC D-alanyl carrier protein | DltC [Bacillus licheniformis DSM 13] | UniRef100_Q65DG9 | Bacillus licheniformis DSM 13 |
| 4576 | dltD precusor DltD | Hypothetical protein dltD [Bacillus licheniformis DSM 13] | UniRef100_Q65DG8 | Bacillus licheniformis DSM 13 |
| 4577 | ywaA Branched-chain amino acid aminotransferase II | YwaA [Bacillus licheniformis DSM 13] | UniRef100_Q65DG7 | Bacillus licheniformis DSM 13 |
| 4578 | BLP04389 hypothetical protein | | | |
| 4579 | yvqJ putative macrolide-efflux protein | Hypothetical protein yvqJ [Bacillus licheniformis DSM 13] | UniRef100_Q65DG6 | Bacillus licheniformis DSM 13 |
| 4580 | licH Glycoside Hydrolase Family 4 | LicH [Bacillus licheniformis DSM 13] | UniRef100_Q65DG5 | Bacillus licheniformis DSM 13 |
| 4581 | licA phosphotransferase system (PTS) lichenan-specific enzyme IIA component | LicA (Phosphotransferase system (PTS) lichenan-specific enzyme IIA component) [Bacillus licheniformis DSM 13] | UniRef100_Q65DG4 | Bacillus licheniformis DSM 13 |

| 4582 | licC phosphotransferase system (PTS) lichenan-specific enzyme IIC component | LicC (Phosphotransferase system (PTS) lichenan-specific enzyme IIC component) [Bacillus licheniformis DSM 13] | UniRef100_Q65DG3 | Bacillus licheniformis DSM 13 |
|---|---|---|---|---|
| 4583 | licB phosphotransferase system (PTS) lichenan-specific enzyme IIB component | LicB (Phosphotransferase system (PTS) lichenan-specific enzyme IIB component) [Bacillus licheniformis DSM 13] | UniRef100_Q65DG2 | Bacillus licheniformis DSM 13 |
| 4584 | licR transcriptional regulator | LicR [Bacillus licheniformis DSM 13] | UniRef100_Q65DG1 | Bacillus licheniformis DSM 13 |
| 4585 | yxzF YxzF | Hypothetical protein yxzF [Bacillus licheniformis DSM 13] | UniRef100_Q65DG0 | Bacillus licheniformis DSM 13 |
| 4586 | yxlJ Methylpurine-DNA glycosylase (MPG) | YxlJ [Bacillus licheniformis DSM 13] | UniRef100_Q65DF9 | Bacillus licheniformis DSM 13 |
| 4587 | BLP04398 putative transcriptional regulator | Hypothetical protein [Bacillus licheniformis DSM 13] | UniRef100_Q65DF8 | Bacillus licheniformis DSM 13 |
| 4588 | mmgD citrate synthase III | MmgD [Bacillus licheniformis DSM 13] | UniRef100_Q65DF7 | Bacillus licheniformis DSM 13 |
| 4589 | mmgE 2-methylisocitrate dehydratase | Hypothetical protein mmgE [Bacillus licheniformis DSM 13] | UniRef100_Q65DF6 | Bacillus licheniformis DSM 13 |
| 4590 | yqiQ putative carboxyphosphonoenolpyruvate phosphonomutase | YqiQ [Bacillus licheniformis DSM 13] | UniRef100_Q65DF5 | Bacillus licheniformis DSM 13 |
| 4591 | BLP04402 Chromate transporter | Hypothetical protein [Bacillus licheniformis DSM 13] | UniRef100_Q65DF4 | Bacillus licheniformis DSM 13 |
| 4592 | BLP04403 conserved hypothetical protein | Hypothetical protein [Bacillus licheniformis DSM 13] | UniRef100_Q65DF3 | Bacillus licheniformis DSM 13 |
| 4593 | ywjA Glycoside hydrolase, family 1,ABC transporter | YwjA [Bacillus licheniformis DSM 13] | UniRef100_Q65DF2 | Bacillus licheniformis DSM 13 |
| 4594 | BLP04405 Carbohydrate Esterase Family 12, putative beta-tubulin | Hypothetical protein [Bacillus licheniformis DSM 13] | UniRef100_Q65DF1 | Bacillus licheniformis DSM 13 |
| 4595 | yweA YweA | Hypothetical protein yweA [Bacillus licheniformis DSM 13] | UniRef100_Q65DF0 | Bacillus licheniformis DSM 13 |
| 4596 | BLP04407 hypothetical protein | YweA [Bacillus licheniformis DSM 13] | UniRef100_Q65DE9 | Bacillus licheniformis DSM 13 |
| 4597 | BLP04408 Drug resistance transporter | Hypothetical protein [Bacillus licheniformis DSM 13] | UniRef100_Q65DE8 | Bacillus licheniformis DSM 13 |
| 4598 | yxlG YxlG | Hypothetical protein yxlG [Bacillus licheniformis DSM 13] | UniRef100_Q65DE7 | Bacillus licheniformis DSM 13 |
| 4599 | yxlF ABC transporter | YxlF [Bacillus licheniformis DSM 13] | UniRef100_Q65DE6 | Bacillus licheniformis DSM 13 |
| 4600 | yxlE YxlE | | | |
| 4601 | yxlD YxlD | Hypothetical protein yxlD [Bacillus licheniformis DSM 13] | UniRef100_Q65DE4 | Bacillus licheniformis DSM 13 |
| 4602 | yxlC YxlC | Hypothetical protein yxlC [Bacillus licheniformis DSM 13] | UniRef100_Q65DE3 | Bacillus licheniformis DSM 13 |
| 4603 | sigY RNA polymerase ECF(extracytoplasmic function)-type sigma factor (sigma-Y) | SigY (RNA polymerase ECF(Extracytoplasmic function)-type sigma factor) [Bacillus licheniformis DSM 13] | UniRef100_Q65DE2 | Bacillus licheniformis DSM 13 |
| 4604 | yxlH putative electron transport protein YxlH | YxlH [Bacillus licheniformis DSM 13] | UniRef100_Q65DE1 | Bacillus licheniformis DSM 13 |
| 4605 | BLP04416 hypothetical protein | | | |
| 4606 | katX major catalase in spores | KatX [Bacillus licheniformis DSM 13] | UniRef100_Q65DE0 | Bacillus licheniformis DSM 13 |
| 4607 | BLP04418 conserved hypothetical protein | Hypothetical protein [Bacillus licheniformis DSM 13] | UniRef100_Q65DD9 | Bacillus licheniformis DSM 13 |
| 4608 | BLP04419 hypothetical protein | KatA [Bacillus licheniformis DSM 13] | UniRef100_Q65DD8 | Bacillus licheniformis DSM 13 |
| 4609 | BLP04420 transcriptional regulator (Fur family) | Hypothetical protein [Bacillus licheniformis DSM 13] | UniRef100_Q65DD7 | Bacillus licheniformis DSM 13 |
| 4610 | BLP04421 putative ferrochelatase | Hypothetical protein [Bacillus licheniformis DSM 13] | UniRef100_Q65DD6 | Bacillus licheniformis DSM 13 |
| 4611 | lanI lantibiotic immunity protein | Hypothetical protein [Bacillus licheniformis DSM 13] | UniRef100_Q65DD5 | Bacillus licheniformis DSM 13 |
| 4612 | lanH lantibiotic immunity protein,ABC transporter | Hypothetical protein [Bacillus licheniformis DSM 13] | UniRef100_Q65DD4 | Bacillus licheniformis DSM 13 |
| 4613 | lanE lantibiotic immunity protein | Hypothetical protein [Bacillus licheniformis DSM 13] | UniRef100_Q65DD3 | Bacillus licheniformis DSM 13 |
| 4614 | lanG lantibiotic immunity protein, MFS type transporter | Hypothetical protein [Bacillus licheniformis DSM 13] | UniRef100_Q65DD2 | Bacillus licheniformis DSM 13 |

| 4615 | lanF lantibiotic immunity protein,ABC transporter | Hypothetical protein [Bacillus licheniformis DSM 13] | UniRef100_Q65DD1 | Bacillus licheniformis DSM 13 |
|------|---------------------------------------------------|-----------------------------------------------------|-------------------|-------------------------------|
| 4616 | lanY LanY | Hypothetical protein [Bacillus licheniformis DSM 13] | UniRef100_Q65DD0 | Bacillus licheniformis DSM 13 |
| 4617 | lanR transcriptional regulator | Hypothetical protein [Bacillus licheniformis DSM 13] | UniRef100_Q65DC9 | Bacillus licheniformis DSM 13 |
| 4618 | lanX conserved hypothetical protein | Hypothetical protein [Bacillus licheniformis DSM 13] | UniRef100_Q65DC8 | Bacillus licheniformis DSM 13 |
| 4619 | lanP intracellular serine protease | Hypothetical protein [Bacillus licheniformis DSM 13] | UniRef100_Q65DC7 | Bacillus licheniformis DSM 13 |
| 4620 | lanT lantibiotic transport protein | Hypothetical protein [Bacillus licheniformis DSM 13] | UniRef100_Q65DC6 | Bacillus licheniformis DSM 13 |
| 4621 | lanM1 lantibiotic modifying enzyme | Hypothetical protein [Bacillus licheniformis DSM 13] | UniRef100_Q65DC5 | Bacillus licheniformis DSM 13 |
| 4622 | BLP04804 hypothetical protein | Lantibiotic cytolysin [Bacillus halodurans] | UniRef100_Q9KFM6 | Bacillus halodurans |
| 4623 | lanA lichenicidin prepeptide | Hypothetical protein [Bacillus licheniformis DSM 13] | UniRef100_Q65DC4 | Bacillus licheniformis DSM 13 |
| 4624 | lanM2 lantibiotic modifying enzyme | Hypothetical protein [Bacillus licheniformis DSM 13] | UniRef100_Q65DC3 | Bacillus licheniformis DSM 13 |
| 4625 | pelII pectate lyase, Polysaccharide Lyase Family 1 | Pectate lyase Pel-34K [Bacillus sp. P-2850] | UniRef100_Q8L0S6 | Bacillus sp. P-2850 |
| 4626 | qdoI putative ribokinase | Hypothetical protein yxkO [Bacillus licheniformis DSM 13] | UniRef100_Q65DC1 | Bacillus licheniformis DSM 13 |
| 4627 | cydD ABC membrane transporter (ATP-binding protein) | CydD [Bacillus licheniformis DSM 13] | UniRef100_Q65DC0 | Bacillus licheniformis DSM 13 |
| 4628 | cydC ABC membrane transporter (ATP-binding protein) | CydC [Bacillus licheniformis DSM 13] | UniRef100_Q65DB9 | Bacillus licheniformis DSM 13 |
| 4629 | cydB cytochrome bd ubiquinol oxidase (subunit II) | CydB [Bacillus licheniformis DSM 13] | UniRef100_Q65DB8 | Bacillus licheniformis DSM 13 |
| 4630 | cydA cytochrome bd ubiquinol oxidase (subunit I) | CydA [Bacillus licheniformis DSM 13] | UniRef100_Q65DB7 | Bacillus licheniformis DSM 13 |
| 4631 | BLP04441 hypothetical protein | | | |
| 4632 | ywcJ Formate_nitrite transporter | YwcJ [Bacillus licheniformis DSM 13] | UniRef100_Q65DB6 | Bacillus licheniformis DSM 13 |
| 4633 | BLP04443 hypothetical protein | | | |
| 4634 | BLP04444 hypothetical protein | Hypothetical protein [Bacillus licheniformis DSM 13] | UniRef100_Q65DB5 | Bacillus licheniformis DSM 13 |
| 4635 | mleA malolactic enzyme | MleA [Bacillus licheniformis DSM 13] | UniRef100_Q65DB4 | Bacillus licheniformis DSM 13 |
| 4636 | mleN malate-H _Na -lactate antiporter | MleN [Bacillus licheniformis DSM 13] | UniRef100_Q65DB3 | Bacillus licheniformis DSM 13 |
| 4637 | ansB L-aspartase | Hypothetical protein [Bacillus licheniformis DSM 13] | UniRef100_Q65DB2 | Bacillus licheniformis DSM 13 |
| 4638 | ansA L-asparaginase | AnsA [Bacillus licheniformis DSM 13] | UniRef100_Q65DB1 | Bacillus licheniformis DSM 13 |
| 4639 | ansR transcriptional regulator | AnsR [Bacillus licheniformis DSM 13] | UniRef100_Q65DB0 | Bacillus licheniformis DSM 13 |
| 4640 | ykgB YkgB | YkgB [Bacillus licheniformis DSM 13] | UniRef100_Q65DA9 | Bacillus licheniformis DSM 13 |
| 4641 | BLP04451 putative two-component reponse regulator | Hypothetical sensory transduction protein yxdJ [Bacillus subtilis] | UniRef100_P42421 | Bacillus subtilis |
| 4642 | yxdK Histidine kinase, homodimeric YxdK | YxdK [Bacillus licheniformis DSM 13] | UniRef100_Q65DA6 | Bacillus licheniformis DSM 13 |
| 4643 | BLP04453 hypothetical protein | | | |
| 4644 | yxdL ABC transporter YxdL | YxdL [Bacillus licheniformis DSM 13] | UniRef100_Q65DA5 | Bacillus licheniformis DSM 13 |
| 4645 | yxdM GTP-binding signal recognition particle (SRP54) G-domain | | | |
| 4646 | BLP04457 conserved hypothetical protein | YxeA [Bacillus licheniformis DSM 13] | UniRef100_Q65DA3 | Bacillus licheniformis DSM 13 |
| 4647 | BLP04458 hypothetical protein | | | |
| 4648 | BLP04459 conserved hypothetical protein | Hypothetical protein [Bacillus licheniformis DSM 13] | UniRef100_Q65DA1 | Bacillus licheniformis DSM 13 |

| 4649 | yxkH Polysaccharide deacetylase, Carbohydrate Esterase Family 4 | | | |
|---|---|---|---|---|
| 4650 | BLP04461 conserved hypothetical protein | Hypothetical protein [Bacillus licheniformis DSM 13] | UniRef100_Q65D99 | Bacillus licheniformis DSM 13 |
| 4651 | ydeL hypothetical DNA-binding protein YdeL | Hypothetical protein [Bacillus licheniformis DSM 13] | UniRef100_Q65D98 | Bacillus licheniformis DSM 13 |
| 4652 | BLP04463 putative Glycosyl transferase, family 2 | Hypothetical protein [Bacillus licheniformis DSM 13] | UniRef100_Q65D97 | Bacillus licheniformis DSM 13 |
| 4653 | cimH Citrate carrier protein | YxkJ [Bacillus licheniformis DSM 13] | UniRef100_Q65D96 | Bacillus licheniformis DSM 13 |
| 4654 | BLP04465 putative Glycerophosphoryl diester phosphodiesterase | Hypothetical protein [Bacillus licheniformis DSM 13] | UniRef100_Q65D95 | Bacillus licheniformis DSM 13 |
| 4655 | yhjA conserved hypothetical protein | YhjA [Bacillus licheniformis DSM 13] | UniRef100_Q65D94 | Bacillus licheniformis DSM 13 |
| 4656 | BLP04467 hypothetical protein | Hypothetical protein [Bacillus licheniformis DSM 13] | UniRef100_Q62NR9 | Bacillus licheniformis DSM 13 |
| 4657 | yfnI putative sulfatase | YfnI [Bacillus licheniformis DSM 13] | UniRef100_Q65D92 | Bacillus licheniformis DSM 13 |
| 4658 | BLP04469 hypothetical protein | | | |
| 4659 | yfjD hypothetical protein | Hypothetical protein yfjD [Bacillus licheniformis DSM 13] | UniRef100_Q65D91 | Bacillus licheniformis DSM 13 |
| 4660 | yxkD YxkD | Hypothetical protein yxkD [Bacillus licheniformis DSM 13] | UniRef100_Q65D90 | Bacillus licheniformis DSM 13 |
| 4661 | argR transcriptional regulator of arginine metabolism expression | Transcriptional regulator [Bacillus licheniformis DSM 13] | UniRef100_Q62NR5 | Bacillus licheniformis DSM 13 |
| 4662 | arcA Arginine deiminase | Arginine deiminase [Bacillus licheniformis] | UniRef100_O86131 | Bacillus licheniformis |
| 4663 | arcB ornithine carbamoyltransferase | Ornithine carbamoyltransferase, catabolic [Bacillus licheniformis] | UniRef100_O86132 | Bacillus licheniformis |
| 4664 | arcD Amino acid_polyamine transporter permease | Permease [Bacillus licheniformis] | UniRef100_O86133 | Bacillus licheniformis |
| 4665 | arcC carbamate kinase | Carbamate kinase [Bacillus licheniformis] | UniRef100_O86134 | Bacillus licheniformis |
| 4666 | BLP04477 transcriptional regulator Fnr family protein | Crp/Fnr family protein [Bacillus licheniformis] | UniRef100_Q9K5F3 | Bacillus licheniformis |
| 4667 | BLP04478 Glycoside hydrolase, family 1 | Hypothetical protein [Bacillus licheniformis DSM 13] | UniRef100_Q65D83 | Bacillus licheniformis DSM 13 |
| 4668 | BLP04479 phosphotransferase system (PTS) lichenan-specific enzyme IIC component | Hypothetical protein (Phosphotransferase system (PTS) lichenan- specific enzyme IIC component) [Bacillus licheniformis DSM 13] | UniRef100_Q65D82 | Bacillus licheniformis DSM 13 |
| 4669 | BLP04480 hypothetical protein | | | |
| 4670 | BLP04481 hypothetical protein | Hypothetical protein [Bacillus licheniformis DSM 13] | UniRef100_Q65D81 | Bacillus licheniformis DSM 13 |
| 4671 | BLP04482 putative RNA polymerase | Hypothetical protein [Bacillus licheniformis DSM 13] | UniRef100_Q65D80 | Bacillus licheniformis DSM 13 |
| 4672 | BLP04483 hypothetical protein | Hypothetical protein [Bacillus licheniformis DSM 13] | UniRef100_Q65D78 | Bacillus licheniformis DSM 13 |
| 4673 | pepT peptidase T (tripeptidase) | PepT [Bacillus licheniformis DSM 13] | UniRef100_Q65D74 | Bacillus licheniformis DSM 13 |
| 4674 | BLP04485 Glycoside Hydrolase Family 32 | Hypothetical protein [Bacillus licheniformis DSM 13] | UniRef100_Q65D73 | Bacillus licheniformis DSM 13 |
| 4675 | ywbF putative sugar permease YwbF | YwbF [Bacillus licheniformis DSM 13] | UniRef100_Q65D72 | Bacillus licheniformis DSM 13 |
| 4676 | BLP04487 Binding-protein-dependent transport systems inner membrane component | Hypothetical protein [Bacillus licheniformis DSM 13] | UniRef100_Q65D71 | Bacillus licheniformis DSM 13 |
| 4677 | BLP04488 Binding-protein-dependent transport systems inner membrane component | YurN [Bacillus licheniformis DSM 13] | UniRef100_Q65D70 | Bacillus licheniformis DSM 13 |
| 4678 | BLP04489 putative extracellular solute-binding protein | Hypothetical protein [Bacillus licheniformis DSM 13] | UniRef100_Q65D69 | Bacillus licheniformis DSM 13 |
| 4679 | BLP04490 putative transcriptional regulator | Hypothetical protein [Bacillus licheniformis DSM 13] | UniRef100_Q65D68 | Bacillus licheniformis DSM 13 |

| 4680 | BLP04491 hypothetical protein | Hypothetical protein [Bacillus licheniformis DSM 13] | UniRef100_Q65D67 | Bacillus licheniformis DSM 13 |
|---|---|---|---|---|
| 4681 | BLP04492 hypothetical protein | Hypothetical protein [Bacillus licheniformis DSM 13] | UniRef100_Q65D66 | Bacillus licheniformis DSM 13 |
| 4682 | BLP04493 conserved hypothetical protein | Hypothetical protein [Bacillus licheniformis DSM 13] | UniRef100_Q65D65 | Bacillus licheniformis DSM 13 |
| 4683 | yxjG methionine synthase | YxjG [Bacillus licheniformis DSM 13] | UniRef100_Q65D64 | Bacillus licheniformis DSM 13 |
| 4684 | ykcB Glycosyl transferase, family 39 | YkcB [Bacillus licheniformis DSM 13] | UniRef100_Q65D63 | Bacillus licheniformis DSM 13 |
| 4685 | ykcC Glycosyl transferase, family 2 | YkcC [Bacillus licheniformis DSM 13] | UniRef100_Q65D62 | Bacillus licheniformis DSM 13 |
| 4686 | BLP04497 UDP-glucose 4-epimerase | Hypothetical protein [Bacillus licheniformis DSM 13] | UniRef100_Q65D61 | Bacillus licheniformis DSM 13 |
| 4687 | BLP04498 partial protein | | | |
| 4688 | BLP04499 hypothetical protein | Hypothetical protein [Bacillus licheniformis DSM 13] | UniRef100_Q65D60 | Bacillus licheniformis DSM 13 |
| 4689 | BLP04500 hypothetical protein | Hypothetical protein [Bacillus licheniformis DSM 13] | UniRef100_Q65D60 | Bacillus licheniformis DSM 13 |
| 4690 | BLP04501 hypothetical protein | Hypothetical protein [Bacillus licheniformis DSM 13] | UniRef100_Q65D59 | Bacillus licheniformis DSM 13 |
| 4691 | BLP04502 hypothetical protein | Hypothetical protein [Bacillus licheniformis DSM 13] | UniRef100_Q65D58 | Bacillus licheniformis DSM 13 |
| 4692 | BLP04503 conserved hypothetical protein | Hypothetical protein [Bacillus licheniformis DSM 13] | UniRef100_Q65D57 | Bacillus licheniformis DSM 13 |
| 4693 | BLP04504 hypothetical protein | | | |
| 4694 | yxjA Concentrative nucleoside transporter | YxjA [Bacillus licheniformis DSM 13] | UniRef100_Q65D56 | Bacillus licheniformis DSM 13 |
| 4695 | katA vegetative catalase 1 | Catalase 2 [Bacillus subtilis] | UniRef100_P42234 | Bacillus subtilis |
| 4696 | BLP04507 putative DNA Gyrase inhibitor | YosT [Bacillus licheniformis DSM 13] | UniRef100_Q65D53 | Bacillus licheniformis DSM 13 |
| 4697 | BLP04508 Glycoside hydrolase, family 1 | Hypothetical protein [Bacillus licheniformis DSM 13] | UniRef100_Q65D52 | Bacillus licheniformis DSM 13 |
| 4698 | bgIPA phosphotransferase system (PTS) beta-glucoside-specific enzyme IIBCA component | Hypothetical protein (Phosphotransferase system (PTS) beta-glucoside- specific enzyme IIBCA component) [Bacillus licheniformis DSM 13] | UniRef100_Q65D51 | Bacillus licheniformis DSM 13 |
| 4699 | licT transcriptional antiterminator | LicT [Bacillus licheniformis DSM 13] | UniRef100_Q65D50 | Bacillus licheniformis DSM 13 |
| 4700 | BLP04511 Cobalt transport protein | YkoC [Bacillus licheniformis DSM 13] | UniRef100_Q65D49 | Bacillus licheniformis DSM 13 |
| 4701 | BLP04512 ABC transporter | YkoD [Bacillus licheniformis DSM 13] | UniRef100_Q65D48 | Bacillus licheniformis DSM 13 |
| 4702 | BLP04513 conserved hypothetical protein | YkoE [Bacillus licheniformis DSM 13] | UniRef100_Q65D47 | Bacillus licheniformis DSM 13 |
| 4703 | tenAA transcriptional regulator | Hypothetical protein [Bacillus licheniformis DSM 13] | UniRef100_Q65D46 | Bacillus licheniformis DSM 13 |
| 4704 | BLP04515 conserved hypothetical protein | Hypothetical protein [Bacillus licheniformis DSM 13] | UniRef100_Q65D45 | Bacillus licheniformis DSM 13 |
| 4705 | aceA Isocitrate lyase | Hypothetical protein [Bacillus licheniformis DSM 13] | UniRef100_Q65D44 | Bacillus licheniformis DSM 13 |
| 4706 | aceB Malate synthase A | Hypothetical protein [Bacillus licheniformis DSM 13] | UniRef100_Q65D43 | Bacillus licheniformis DSM 13 |
| 4707 | BLP04518 conserved hypothetical protein | Hypothetical protein [Bacillus licheniformis DSM 13] | UniRef100_Q62NL9 | Bacillus licheniformis DSM 13 |
| 4708 | BLP04519 hypothetical protein | | | |
| 4709 | yycA Glycosyl transferase, family 39 | YycA [Bacillus licheniformis DSM 13] | UniRef100_Q65D42 | Bacillus licheniformis DSM 13 |
| 4710 | bceB bacitracine efflux ABC transport system permease | YtsD [Bacillus licheniformis DSM 13] | UniRef100_Q65D41 | Bacillus licheniformis DSM 13 |
| 4711 | bceA ABC transporter,ATP_GTP-binding site motif A (P-loop) | YtsC [Bacillus licheniformis DSM 13] | UniRef100_Q65D40 | Bacillus licheniformis DSM 13 |
| 4712 | bceS Histidine kinase, homodimeric | Histidine kinase, homodimeric [Bacillus licheniformis DSM 13] | UniRef100_Q62NL5 | Bacillus licheniformis DSM 13 |

| 4713 | bceR two-component response regulator | YtsA [Bacillus licheniformis DSM 13] | UniRef100_Q65D38 | Bacillus licheniformis DSM 13 |
|---|---|---|---|---|
| 4714 | bglH beta-glucosidase, Glycoside Hydrolase Family 1 | BglH [Bacillus licheniformis DSM 13] | UniRef100_Q65D37 | Bacillus licheniformis DSM 13 |
| 4715 | bglP phosphotransferase system (PTS) beta-glucoside-specific enzyme IIBCA component | BglP (Phosphotransferase system (PTS) beta-glucoside-specific enzyme IIBCA component) [Bacillus licheniformis DSM 13] | UniRef100_Q65D36 | Bacillus licheniformis DSM 13 |
| 4716 | yxeG YxeG | Hypothetical protein yxeG [Bacillus licheniformis DSM 13] | UniRef100_Q65D35 | Bacillus licheniformis DSM 13 |
| 4717 | yxeI Choloylglycine hydrolase | YxeI [Bacillus licheniformis DSM 13] | UniRef100_Q65D34 | Bacillus licheniformis DSM 13 |
| 4718 | BLP04529 hypothetical protein | | | |
| 4719 | BLP04530 hypothetical protein | Hypothetical protein [Bacillus licheniformis DSM 13] | UniRef100_Q65D33 | Bacillus licheniformis DSM 13 |
| 4720 | BLP04531 hypothetical protein | Hypothetical protein [Bacillus licheniformis DSM 13] | UniRef100_Q65D32 | Bacillus licheniformis DSM 13 |
| 4721 | yxiA Glycoside hydrolase, family 43 YxiA | YxiA [Bacillus licheniformis DSM 13] | UniRef100_Q65D31 | Bacillus licheniformis DSM 13 |
| 4722 | BLP04533 hypothetical protein | | | |
| 4723 | deaD ATP-dependent RNA helicase | DeaD [Bacillus licheniformis DSM 13] | UniRef100_Q65D30 | Bacillus licheniformis DSM 13 |
| 4724 | BLP04535 hypothetical protein | Hypothetical protein [Bacillus licheniformis DSM 13] | UniRef100_Q65D29 | Bacillus licheniformis DSM 13 |
| 4725 | BLP04536 hypothetical protein | Hypothetical protein [Bacillus licheniformis DSM 13] | UniRef100_Q65D28 | Bacillus licheniformis DSM 13 |
| 4726 | BLP04537 putative transposase protein | Hypothetical protein [Bacillus licheniformis DSM 13] | UniRef100_Q65D27 | Bacillus licheniformis DSM 13 |
| 4727 | BLP04538 hypothetical protein | Hypothetical protein [Bacillus licheniformis DSM 13] | UniRef100_Q65D26 | Bacillus licheniformis DSM 13 |
| 4728 | BLP04539 hypothetical protein | Hypothetical protein yxiB [Bacillus licheniformis DSM 13] | UniRef100_Q65D25 | Bacillus licheniformis DSM 13 |
| 4729 | pdp pyrimidine-nucleoside phosphorylase | Pdp [Bacillus licheniformis DSM 13] | UniRef100_Q65D24 | Bacillus licheniformis DSM 13 |
| 4730 | nupC pyrimidine-nucleoside transport protein | NupC [Bacillus licheniformis DSM 13] | UniRef100_Q65D23 | Bacillus licheniformis DSM 13 |
| 4731 | deoC deoxyribose-phosphate aldolase | Dra [Bacillus licheniformis DSM 13] | UniRef100_Q65D22 | Bacillus licheniformis DSM 13 |
| 4732 | deoR transcriptional regulator | DeoR [Bacillus licheniformis DSM 13] | UniRef100_Q65D21 | Bacillus licheniformis DSM 13 |
| 4733 | BLP04544 hypothetical protein | Hypothetical protein [Bacillus licheniformis DSM 13] | UniRef100_Q65D20 | Bacillus licheniformis DSM 13 |
| 4734 | ydaJ putative Glycoside transferase | YdaJ [Bacillus licheniformis DSM 13] | UniRef100_Q65D19 | Bacillus licheniformis DSM 13 |
| 4735 | ydaK conserved membrane protein YdaK | YdaK [Bacillus licheniformis DSM 13] | UniRef100_Q65D18 | Bacillus licheniformis DSM 13 |
| 4736 | ydaL YdaL | Hypothetical protein ydaL [Bacillus licheniformis DSM 13] | UniRef100_Q65D17 | Bacillus licheniformis DSM 13 |
| 4737 | ydaM Glycosyl transferase, family 2 | YdaM [Bacillus licheniformis DSM 13] | UniRef100_Q65D16 | Bacillus licheniformis DSM 13 |
| 4738 | ydaN putative cellulose synthase | Hypothetical protein ydaN [Bacillus licheniformis DSM 13] | UniRef100_Q65D15 | Bacillus licheniformis DSM 13 |
| 4739 | BLP04550 putative alanine transaminase | YwfG [Bacillus licheniformis DSM 13] | UniRef100_Q65D14 | Bacillus licheniformis DSM 13 |
| 4740 | BLP04551 hypothetical protein | Hypothetical protein [Bacillus licheniformis DSM 13] | UniRef100_Q65D13 | Bacillus licheniformis DSM 13 |
| 4741 | BLP04552 conserved hypothetical protein | Hypothetical protein ywfB [Bacillus licheniformis DSM 13] | UniRef100_Q65D12 | Bacillus licheniformis DSM 13 |
| 4742 | BLP04553 conserved hypothetical protein | Hypothetical protein ywfE [Bacillus licheniformis DSM 13] | UniRef100_Q65D11 | Bacillus licheniformis DSM 13 |
| 4743 | BLP04554 hypothetical protein, putative transporter | Hypothetical protein [Bacillus licheniformis DSM 13] | UniRef100_Q65D10 | Bacillus licheniformis DSM 13 |
| 4744 | fbaB fructose-1,6-bisphosphate aldolase | FbaB [Bacillus licheniformis DSM 13] | UniRef100_Q65D09 | Bacillus licheniformis DSM 13 |
| 4745 | iolI IolI | Hypothetical protein iolI [Bacillus licheniformis DSM 13] | UniRef100_Q65D08 | Bacillus licheniformis DSM 13 |
| 4746 | iolH IolH | Hypothetical protein iolH [Bacillus licheniformis DSM 13] | UniRef100_Q65D07 | Bacillus licheniformis DSM 13 |

| 4747 | idh myo-inositol 2-dehydrogenase | Idh [Bacillus licheniformis DSM 13] | UniRef100_Q65D06 | Bacillus licheniformis DSM 13 |
|---|---|---|---|---|
| 4748 | iolF inositol transport protein | IolF [Bacillus licheniformis DSM 13] | UniRef100_Q65D05 | Bacillus licheniformis DSM 13 |
| 4749 | iolE IolE | Hypothetical protein iolE [Bacillus licheniformis DSM 13] | UniRef100_Q65D04 | Bacillus licheniformis DSM 13 |
| 4750 | iolD IolD | Hypothetical protein iolD [Bacillus licheniformis DSM 13] | UniRef100_Q65D03 | Bacillus licheniformis DSM 13 |
| 4751 | iolC putative kinase,myo-inositol catabolism protein | Hypothetical protein iolC [Bacillus licheniformis DSM 13] | UniRef100_Q65D02 | Bacillus licheniformis DSM 13 |
| 4752 | iolB myo-inositol catabolism protein | Hypothetical protein iolB [Bacillus licheniformis DSM 13] | UniRef100_Q65D01 | Bacillus licheniformis DSM 13 |
| 4753 | iolA methylmalonate-semialdehyde dehydrogenase | Hypothetical protein [Bacillus licheniformis DSM 13] | UniRef100_Q65D00 | Bacillus licheniformis DSM 13 |
| 4754 | BLP04565 hypothetical protein | | | |
| 4755 | iolR transcriptional regulator (DeoR family) | IolR [Bacillus licheniformis DSM 13] | UniRef100_Q65CZ9 | Bacillus licheniformis DSM 13 |
| 4756 | iolS aldo_keto reductase family 2 protein | Hypothetical protein iolS [Bacillus licheniformis DSM 13] | UniRef100_Q65CZ8 | Bacillus licheniformis DSM 13 |
| 4757 | glpQ glycerophosphoryl diester phosphodiesterase | GlpQ [Bacillus licheniformis DSM 13] | UniRef100_Q65CZ7 | Bacillus licheniformis DSM 13 |
| 4758 | glpT glycerol-3-phosphate permease | GlpT [Bacillus licheniformis DSM 13] | UniRef100_Q65CZ6 | Bacillus licheniformis DSM 13 |
| 4759 | htpG class III heat-shock protein (molecular chaperone) | HtpG [Bacillus licheniformis DSM 13] | UniRef100_Q65CZ5 | Bacillus licheniformis DSM 13 |
| 4760 | BLP04571 ABC transport system ATP-binding protein | Hypothetical protein [Bacillus licheniformis DSM 13] | UniRef100_Q65CZ4 | Bacillus licheniformis DSM 13 |
| 4761 | yxeH HAD-superfamily hydrolase YxeH | YxeH [Bacillus licheniformis DSM 13] | UniRef100_Q65CZ3 | Bacillus licheniformis DSM 13 |
| 4762 | BLP04573 putative Glycerol dehydrogenase | Hypothetical protein [Bacillus licheniformis DSM 13] | UniRef100_Q65CZ2 | Bacillus licheniformis DSM 13 |
| 4763 | BLP04574 putative D-alpha-glycerophosphatase | YkrX (HAD-superfamily hydrolase, subfamily IB (PSPase-like),HAD- superfamily subfamily IB hydrolase, hypothetical 1) [Bacillus licheniformis DSM 13] | UniRef100_Q65CZ1 | Bacillus licheniformis DSM 13 |
| 4764 | BLP04575 conserved hypothetical protein | Hypothetical protein [Bacillus licheniformis DSM 13] | UniRef100_Q65CZ0 | Bacillus licheniformis DSM 13 |
| 4765 | yxeB Periplasmic binding protein YxeB | YxeB [Bacillus licheniformis DSM 13] | UniRef100_Q65CY9 | Bacillus licheniformis DSM 13 |
| 4766 | BLP04577 hypothetical protein | Hypothetical protein [Bacillus licheniformis DSM 13] | UniRef100_Q65CY8 | Bacillus licheniformis DSM 13 |
| 4767 | BLP04578 hypothetical protein | | | |
| 4768 | BLP04579 Glyoxalase I | YraH [Bacillus licheniformis DSM 13] | UniRef100_Q65CY7 | Bacillus licheniformis DSM 13 |
| 4769 | BLP04580 hypothetical protein | Hypothetical protein [Bacillus licheniformis DSM 13] | UniRef100_Q62NG2 | Bacillus licheniformis DSM 13 |
| 4770 | BLP04581 ferrous ion transporter | Hypothetical Ferrous iron transport protein B [Bacillus licheniformis DSM 13] | UniRef100_Q65CY6 | Bacillus licheniformis DSM 13 |
| 4771 | BLP04582 transcriptional repressor | Hypothetical protein [Bacillus licheniformis DSM 13] | UniRef100_Q65CY5 | Bacillus licheniformis DSM 13 |
| 4772 | yfiQ putative Acyltransferase 3 YfiQ | YfiQ [Bacillus licheniformis DSM 13] | UniRef100_Q65CY4 | Bacillus licheniformis DSM 13 |
| 4773 | yxdM ABC transport system permease protein | Hypothetical protein yxdM [Bacillus licheniformis DSM 13] | UniRef100_Q65CY3 | Bacillus licheniformis DSM 13 |
| 4774 | yxdL ABC transporter | Hypothetical protein [Bacillus licheniformis DSM 13] | UniRef100_Q65CY2 | Bacillus licheniformis DSM 13 |
| 4775 | yxdK two-component sensor histidine kinase. potential cognate response regulator is YxdJ | Hypothetical protein [Bacillus licheniformis DSM 13] | UniRef100_Q65CY1 | Bacillus licheniformis DSM 13 |
| 4776 | yxdJ two-component response regulator. potential cognate sensor kinase is YxdK | YxdJ [Bacillus licheniformis DSM 13] | UniRef100_Q65CY0 | Bacillus licheniformis DSM 13 |
| 4777 | yxeA conserved hypothetical protein | Hypothetical protein yxeA [Bacillus licheniformis DSM 13] | UniRef100_Q65CX9 | Bacillus licheniformis DSM 13 |
| 4778 | yoaH putative methyl-accepting chemotaxis protein | YoaH [Bacillus licheniformis DSM 13] | UniRef100_Q65CX8 | Bacillus licheniformis DSM 13 |

| | | YoaH | | | |
|---|---|---|---|---|---|
| | 4779 | BLP04590 putative alanine:Na symporter | Hypothetical protein [Bacillus licheniformis DSM 13] | UniRef100_Q65CX7 | Bacillus licheniformis DSM 13 |
| | 4780 | aldA alanine dehydrogenase (stage V sporulation protein N) | Hypothetical protein [Bacillus licheniformis DSM 13] | UniRef100_Q65CX6 | Bacillus licheniformis DSM 13 |
| | 4781 | yvfO Glycosyl Hydrolase Family 53 | Hypothetical protein yvfO [Bacillus licheniformis DSM 13] | UniRef100_Q62NF0 | Bacillus licheniformis DSM 13 |
| | 4782 | lacA Glycoside Hydrolase Family 42 | LacA [Bacillus licheniformis DSM 13] | UniRef100_Q65CX4 | Bacillus licheniformis DSM 13 |
| | 4783 | yvfM Binding-protein-dependent transport system, inner membrane component protein | YvfM [Bacillus licheniformis DSM 13] | UniRef100_Q65CX3 | Bacillus licheniformis DSM 13 |
| | 4784 | yvfL Binding-protein-dependent transport systems inner membrane component | YvfL [Bacillus licheniformis DSM 13] | UniRef100_Q65CX2 | Bacillus licheniformis DSM 13 |
| | 4785 | cycB putative extracellular solute-binding protein, family 1 CycB | YvfK [Bacillus licheniformis DSM 13] | UniRef100_Q65CX1 | Bacillus licheniformis DSM 13 |
| | 4786 | BLP04597 hypothetical protein | | | |
| | 4787 | lacR transcriptional regulator LacR | LacR [Bacillus licheniformis DSM 13] | UniRef100_Q65CX0 | Bacillus licheniformis DSM 13 |
| | 4788 | galK galactokinase | GalK [Bacillus licheniformis DSM 13] | UniRef100_Q65CW9 | Bacillus licheniformis DSM 13 |
| | 4789 | galE UDP-glucose 4-epimerase | Hypothetical protein [Bacillus licheniformis DSM 13] | UniRef100_Q65CW8 | Bacillus licheniformis DSM 13 |
| | 4790 | galT galactose-1-phosphate uridyltransferase | GalT [Bacillus licheniformis DSM 13] | UniRef100_Q65CW7 | Bacillus licheniformis DSM 13 |
| | 4791 | xylR transcriptional regulator | Hypothetical protein [Bacillus licheniformis DSM 13] | UniRef100_Q65CW6 | Bacillus licheniformis DSM 13 |
| | 4792 | BLP04603 hypothetical protein | | | |
| | 4793 | gntR transcriptional regulator | GntR family [Bacillus licheniformis DSM 13] | UniRef100_Q65CW5 | Bacillus licheniformis DSM 13 |
| | 4794 | gntK gluconate kinase | GntK [Bacillus licheniformis DSM 13] | UniRef100_Q65CW4 | Bacillus licheniformis DSM 13 |
| | 4795 | gntP gluconate permease | GntP [Bacillus licheniformis DSM 13] | UniRef100_Q65CW3 | Bacillus licheniformis DSM 13 |
| | 4796 | gntZ 6-phosphogluconate dehydrogenase | GntZ [Bacillus licheniformis DSM 13] | UniRef100_Q65CW2 | Bacillus licheniformis DSM 13 |
| | 4797 | BLP04608 hypothetical protein | | | |
| | 4798 | BLP04609 putative Aldehyde-alcohol dehydrogenase | Hypothetical protein [Bacillus licheniformis DSM 13] | UniRef100_Q65CW1 | Bacillus licheniformis DSM 13 |
| | 4799 | ahpC alkyl hydroperoxide reductase (small subunit) | AhpC [Bacillus licheniformis DSM 13] | UniRef100_Q65CW0 | Bacillus licheniformis DSM 13 |
| | 4800 | ahpF alkyl hydroperoxide reductase (large subunit) and NADH dehydrogenase | AhpF (Alkyl hydroperoxide reductase (Large subunit) and NADH dehydrogenase) [Bacillus licheniformis DSM 13] | UniRef100_Q65CV9 | Bacillus licheniformis DSM 13 |
| | 4801 | BLP04612 hypothetical protein | Hypothetical protein [Bacillus licheniformis DSM 13] | UniRef100_Q65CV8 | Bacillus licheniformis DSM 13 |
| | 4802 | BLP04613 hypothetical protein | Hypothetical protein [Bacillus licheniformis DSM 13] | UniRef100_Q65CV7 | Bacillus licheniformis DSM 13 |
| | 4803 | abiBL11 AbiBL11 | AbiBL11 [Bacillus licheniformis] | UniRef100_Q9F5X7 | Bacillus licheniformis |
| | 4804 | BLP04615 highly conserved hypothetical protein | Hypothetical protein [Bacillus licheniformis] | UniRef100_Q9F5X6 | Bacillus licheniformis |
| | 4805 | yvfR ABC transporter | YvfR [Bacillus licheniformis DSM 13] | UniRef100_Q65CV3 | Bacillus licheniformis DSM 13 |
| | 4806 | yvfS conserved hypothetical protein YvfS | Hypothetical protein yvfS [Bacillus subtilis] | UniRef100_O07017 | Bacillus subtilis |
| | 4807 | desK two-component sensor histidine kinase esponsible for cold induction of the des gene | Hypothetical ATP-binding region, ATPase-like [Bacillus licheniformis DSM 13] | UniRef100_Q62NC9 | Bacillus licheniformis DSM 13 |
| | 4808 | yvfU CheY-like | Response regulator [Bacillus licheniformis] | UniRef100_Q65CU8 | Bacillus licheniformis |
| | 4809 | BLP04620 hypothetical protein | Hypothetical protein [Bacillus licheniformis DSM 13] | UniRef100_Q62NC7 | Bacillus licheniformis DSM 13 |

| 4810 | BLP04621 hypothetical protein | Hypothetical protein [Bacillus licheniformis DSM 13] | UniRef100_Q65CU6 | Bacillus licheniformis DSM 13 |
|------|-------------------------------|------------------------------------------------------|------------------|-------------------------------|
| 4811 | BLP04622 ABC-type antimicrobial peptide transport system ATPase component | Hypothetical protein [Bacillus licheniformis DSM 13] | UniRef100_Q65CU5 | Bacillus licheniformis DSM 13 |
| 4812 | BLP04805 hypothetical protein | | | |
| 4813 | BLP04836 L12 L12 | Hypothetical protein [Bacillus licheniformis DSM 13] | UniRef100_Q65CU4 | Bacillus licheniformis DSM 13 |
| 4814 | BLP04806 hypothetical protein | | | |
| 4815 | BLP04623 hypothetical protein | Hypothetical protein [Bacillus licheniformis DSM 13] | UniRef100_Q65CU3 | Bacillus licheniformis DSM 13 |
| 4816 | BLP04624 hypothetical protein | Hypothetical protein [Bacillus licheniformis DSM 13] | UniRef100_Q62NC2 | Bacillus licheniformis DSM 13 |
| 4817 | yydA conserved hypothetical protein containing DUF163 domain YydA | Hypothetical protein yydA [Bacillus licheniformis DSM 13] | UniRef100_Q65CS8 | Bacillus licheniformis DSM 13 |
| 4818 | BLP04626 conserved hypothetical protein | Hypothetical protein [Bacillus licheniformis DSM 13] | UniRef100_Q65CS7 | Bacillus licheniformis DSM 13 |
| 4819 | BLP04627 conserved hypothetical protein | Hypothetical protein [Bacillus licheniformis DSM 13] | UniRef100_Q65CS6 | Bacillus licheniformis DSM 13 |
| 4820 | yycN GCN5-related N-acetyltransferase | GCN5-related N-acetyltransferase [Bacillus licheniformis DSM 13] | UniRef100_Q62NA3 | Bacillus licheniformis DSM 13 |
| 4821 | BLP04629 conserved hypothetical protein | Hypothetical protein [Bacillus licheniformis DSM 13] | UniRef100_Q65CS4 | Bacillus licheniformis DSM 13 |
| 4822 | BLP04630 putative Molybdate_tungstate binding protein | Hypothetical Molybdate/tungstate binding protein, MOP [Bacillus licheniformis DSM 13] | UniRef100_Q65CS3 | Bacillus licheniformis DSM 13 |
| 4823 | BLP04631 hypothetical protein | | | |
| 4824 | yyxA putative serine protease YyxA | YyxA [Bacillus licheniformis DSM 13] | UniRef100_Q65CS2 | Bacillus licheniformis DSM 13 |
| 4825 | yycJ conserved hypothetical protein YycJ | YycJ [Bacillus licheniformis DSM 13] | UniRef100_Q65CS1 | Bacillus licheniformis DSM 13 |
| 4826 | yycI putative extracytoplasmic proein YycI | Hypothetical protein yycI [Bacillus licheniformis DSM 13] | UniRef100_Q65CS0 | Bacillus licheniformis DSM 13 |
| 4827 | yycH conserved protein YycH | YycH [Bacillus licheniformis DSM 13] | UniRef100_Q65CR9 | Bacillus licheniformis DSM 13 |
| 4828 | yycG two-component sensor histidine kinase | YycG [Bacillus licheniformis DSM 13] | UniRef100_Q65CR8 | Bacillus licheniformis DSM 13 |
| 4829 | yycF putative two-component response regulator YycF | YycF [Bacillus licheniformis DSM 13] | UniRef100_Q65CR7 | Bacillus licheniformis DSM 13 |
| 4830 | BLP04638 hypothetical protein | | | |
| 4831 | BLP04639 putative Phosphohydrolase | Hypothetical protein [Bacillus licheniformis DSM 13] | UniRef100_Q65CR6 | Bacillus licheniformis DSM 13 |
| 4832 | purA adenylosuccinate synthetase | PurA [Bacillus licheniformis DSM 13] | UniRef100_Q65CR5 | Bacillus licheniformis DSM 13 |
| 4833 | BLP04641 hypothetical protein | | | |
| 4834 | BLP04642 conserved hypothetical protein | Hypothetical protein [Bacillus licheniformis DSM 13] | UniRef100_Q65CR4 | Bacillus licheniformis DSM 13 |
| 4835 | dnaC replicative DNA helicase | DnaC [Bacillus licheniformis DSM 13] | UniRef100_Q65CR3 | Bacillus licheniformis DSM 13 |
| 4836 | yycD YycD | Hypothetical protein yycD [Bacillus licheniformis DSM 13] | UniRef100_Q65CR2 | Bacillus licheniformis DSM 13 |
| 4837 | yyzB YyzB | Hypothetical protein yyzB [Bacillus licheniformis DSM 13] | UniRef100_Q65CR1 | Bacillus licheniformis DSM 13 |
| 4838 | yycC conserved hypothetical protein YycC | YycC [Bacillus licheniformis DSM 13] | UniRef100_Q65CR0 | Bacillus licheniformis DSM 13 |
| 4839 | yycB Cyanate transport system protein | YycB [Bacillus licheniformis DSM 13] | UniRef100_Q65CQ9 | Bacillus licheniformis DSM 13 |
| 4840 | nagA N-acetylglucosamine-6-phosphate deacetylase (Carbohydrate esterase Family 9) | NagA [Bacillus licheniformis DSM 13] | UniRef100_Q65CQ8 | Bacillus licheniformis DSM 13 |
| 4841 | nagB N-acetylglucosamine-6-phosphate isomerase | Hypothetical protein [Bacillus licheniformis DSM 13] | UniRef100_Q65CQ7 | Bacillus licheniformis DSM 13 |

| | | | | |
|---|---|---|---|---|
| 4842 | yvoA putative transcriptional regulator (GntR family) YvoA | YvoA [Bacillus licheniformis DSM 13] | UniRef100_Q65CQ6 | Bacillus licheniformis DSM 13 |
| 4843 | BLP04651 conserved hypothetical protein | Hypothetical protein [Bacillus licheniformis DSM 13] | UniRef100_Q65CQ5 | Bacillus licheniformis DSM 13 |
| 4844 | nagP phosphotransferase system (PTS) N-acetylglucosamine-specific enzyme IICB component | NagP (Phosphotransferase system (PTS) N-acetylglucosamine-specific enzyme IICB component) [Bacillus licheniformis DSM 13] | UniRef100_Q65CQ4 | Bacillus licheniformis DSM 13 |
| 4845 | rplI ribosomal protein L9 | RplI [Bacillus licheniformis DSM 13] | UniRef100_Q65CQ3 | Bacillus licheniformis DSM 13 |
| 4846 | yybT putative phosphoesterase family protein YybT | YybT [Bacillus licheniformis DSM 13] | UniRef100_Q65CQ2 | Bacillus licheniformis DSM 13 |
| 4847 | yybS conserved hypothetical protein YybS | Hypothetical protein yybS [Bacillus licheniformis DSM 13] | UniRef100_Q65CQ1 | Bacillus licheniformis DSM 13 |
| 4848 | BLP04661 hypothetical protein | | | |
| 4849 | cotF2 spore coat protein CotF2 | | | |
| 4850 | cotF spore coat protein F | CotF [Bacillus licheniformis DSM 13] | UniRef100_Q65CP9 | Bacillus licheniformis DSM 13 |
| 4851 | yxiE conserved protein containing universal stress protein domain YxiE | YxiE [Bacillus licheniformis DSM 13] | UniRef100_Q65CP8 | Bacillus licheniformis DSM 13 |
| 4852 | ybaR putative Sulfate transporter_antisigma-factor antagonist YbaR | YbaR [Bacillus licheniformis DSM 13] | UniRef100_Q65CP7 | Bacillus licheniformis DSM 13 |
| 4853 | yybO Major facilitator superfamily | YybO [Bacillus licheniformis DSM 13] | UniRef100_Q65CP6 | Bacillus licheniformis DSM 13 |
| 4854 | BLP04661 hypothetical protein | | | |
| 4855 | rpsR 30S ribosomal protein RpsR | RpsR [Bacillus licheniformis DSM 13] | UniRef100_Q65CP5 | Bacillus licheniformis DSM 13 |
| 4856 | ssb single-strand DNA-binding protein | Ssb [Bacillus licheniformis DSM 13] | UniRef100_Q65CP4 | Bacillus licheniformis DSM 13 |
| 4857 | rpsF ribosomal protein S6 (BS9) | RpsF [Bacillus licheniformis DSM 13] | UniRef100_Q65CP3 | Bacillus licheniformis DSM 13 |
| 4858 | engD GTP-dependent nucleic acid-binding protein EngD | YyaF [Bacillus licheniformis DSM 13] | UniRef100_Q65CP2 | Bacillus licheniformis DSM 13 |
| 4859 | BLP04665 hypothetical protein | | | |
| 4860 | BLP04666 conserved hypothetical protein containing domain DUF951 | Hypothetical protein [Bacillus licheniformis DSM 13] | UniRef100_Q65CP1 | Bacillus licheniformis DSM 13 |
| 4861 | yyaD conserved hypothetical membrane protein YyaD | Hypothetical protein yyaD [Bacillus licheniformis DSM 13] | UniRef100_Q65CP0 | Bacillus licheniformis DSM 13 |
| 4862 | BLP04808 hypothetical protein | | | |
| 4863 | yyaC conserved hypothetical protein containing domain DUF1256 | Hypothetical protein yyaC [Bacillus licheniformis DSM 13] | UniRef100_Q65CN9 | Bacillus licheniformis DSM 13 |
| 4864 | spo0J Stage 0 sporulation protein J | Spo0J [Bacillus licheniformis DSM 13] | UniRef100_Q65CN8 | Bacillus licheniformis DSM 13 |
| 4865 | soj chromosome partitioning protein transcriptional regulator | Chromosome partitioning protein transcriptional regulator [Bacillus licheniformis DSM 13] | UniRef100_Q62N66 | Bacillus licheniformis DSM 13 |
| 4866 | ydfG conserved hypothetical protein containing Alkylhydroperoxidase AhpD core domain YdfG | YdfG [Bacillus licheniformis DSM 13] | UniRef100_Q65CN6 | Bacillus licheniformis DSM 13 |
| 4867 | BLP04672 putative RNA polymerase, ECF sigma subunit | Hypothetical protein [Bacillus licheniformis DSM 13] | UniRef100_Q65CN5 | Bacillus licheniformis DSM 13 |
| 4868 | yyaA putative DNA binding partioning protein | YyaA [Bacillus licheniformis DSM 13] | UniRef100_Q65CN4 | Bacillus licheniformis DSM 13 |
| 4869 | gidB glucose-inhibited division protein | GidB [Bacillus licheniformis DSM 13] | UniRef100_Q65CN3 | Bacillus licheniformis DSM 13 |
| 4870 | gidA glucose-inhibited division protein | GidA [Bacillus licheniformis DSM 13] | UniRef100_Q65CN2 | Bacillus licheniformis DSM 13 |
| 4871 | thdF ThdF protein - tRNA modification | Hypothetical protein thdF [Bacillus licheniformis DSM 13] | UniRef100_Q65CN1 | Bacillus licheniformis DSM 13 |

| | | | | |
|---|---|---|---|---|
| 4872 | BLP04677 hypothetical protein | | | |
| 4873 | jag SpoIIIJ-associated protein | Jag [Bacillus licheniformis DSM 13] | UniRef100_Q65CN0 | Bacillus licheniformis DSM 13 |
| 4874 | spoIIIJ SpoIIIJ protein involved in stage III sporulation | SpoIIIJ [Bacillus licheniformis DSM 13] | UniRef100_Q65CM9 | Bacillus licheniformis DSM 13 |
| 4875 | rnpA protein component of ribonuclease P (RNase P) (substrate specificity) | RnpA [Bacillus licheniformis DSM 13] | UniRef100_Q65CM8 | Bacillus licheniformis DSM 13 |
| 4876 | rpmH ribosomal protein L34 | 50S ribosomal protein L34 [Bacillus subtilis] | UniRef100_P05647 | Bacillus subtilis |

**Table 2. Features of the *Bacillus licheniformis* SJ1904 genome and comparison with genome of *Bacillus licheniformis* ATCC 14580**

| Properties | *B. licheniformis* SJ1904 | *B. licheniformis* ATCC 14580 |
|---|---|---|
| Total base pairs | 4,345,159 | 4,222,336 |
| Gene models w/o frameshifts | 4,707 | 4,208 |
| Gene models partial or w/confirmed frameshifts | 168 | 45 |
| rRNA operons | 7 | 7 |
| tRNA genes | 72 | 72 |
| Avg G+C content | 46.7 % | 46.2% |
| Avg gene length | 789 bp | 873 bp |
| Gene density | 1.12 genes/kb | 0.98 genes/kb |

**Table 3. Features of the *Bacillus licheniformis* SJ1904 not predicted in *Bacillus licheniformis* ATCC 14580**

| SEQID | Gene Name | Gene Product |
|---|---|---|
| 50 | BLP04682 | BLP04682 hypothetical protein |
| 55 | BLP00052 | BLP00052 hypothetical protein |
| 77 | BLP00074 | BLP00074 hypothetical protein |
| 82 | BLP04683 | BLP04683 hypothetical protein |
| 91 | BLP00087 | BLP00087 hypothetical protein |
| 98 | BLP00094 | BLP00094 hypothetical protein |
| 103 | BLP04684 | BLP04684 hypothetical protein |
| 125 | BLP04685 | BLP04685 hypothetical protein |
| 169 | BLP00152 | BLP00152 Putative transcriptional regulator |
| 171 | BLP00154 | BLP00154 conserved hypothetical protein |
| 172 | BLP00155 | BLP00155 putative phage protein |
| 174 | BLP04687 | BLP04687 hypothetical protein |
| 177 | BLP00158 | BLP00158 conserved hypothetical phage protein |
| 178 | BLP00159 | BLP00159 conserved hypothetical phage protein |
| 179 | BLP00160 | BLP00160 hypothetical protein |
| 180 | BLP04686 | BLP04686 hypothetical protein |
| 181 | BLP00161 | BLP00161 hypothetical protein |
| 182 | BLP00162 | BLP00162 conserved hypothetical phage protein |
| 185 | BLP00166 | BLP00166 conserved hypothetical protein |
| 186 | BLP00167 | BLP00167 conserved hypothetical protein |
| 187 | BLP00168 | BLP00168 hypothetical protein |
| 192 | BLP00173 | BLP00173 hypothetical protein |
| 194 | BLP00175 | BLP00175 hypothetical protein |
| 195 | BLP00176 | BLP00175 hypothetical protein |
| 196 | BLP00177 | BLP00177 hypothetical protein |
| 198 | BLP04690 | BLP04690 hypothetical protein |
| 199 | BLP00179 | BLP00179 conserved hypothetical protein |
| 203 | BLP00183 | BLP00183 hypothetical protein |
| 205 | BLP00185 | BLP00185 putative phage terminase, large subunit, PBSX family |
| 206 | BLP00186 | BLP00186 hypothetical protein |
| 207 | BLP00187 | BLP00187 putative Phage portal protein, A118 family |
| 208 | BLP00189 | BLP00189 putative Gp4-like protein |
| 209 | BLP00190 | BLP00190 hypothetical protein |
| 210 | BLP00191 | BLP00191 hypothetical protein |

(continued)

| SEQID | Gene Name | Gene Product |
|---|---|---|
| 211 | BLP00192 | BLP00192 conserved hypothetical phage protein |
| 212 | BLP00193 | BLP00193 conserved hypothetical phage-like protein |
| 213 | BLP00194 | BLP00194 conserved phage protein |
| 214 | BLP00195 | BLP00195 conserved phage protein |
| 215 | BLP00196 | BLP00196 conserved phage protein |
| 216 | BLP04843 | BLP04843 conserved hypothetical phage protein |
| 217 | BLP00197 | BLP00197 hypothetical protein |
| 218 | BLP00198 | BLP00198 hypothetical protein |
| 219 | BLP04691 | BLP04691 hypothetical protein |
| 220 | BLP00199 | BLP00199 hypothetical protein |
| 221 | BLP00200 | BLP00200 hypothetical protein |
| 225 | BLP00205 | BLP00205 hypothetical protein |
| 226 | BLP00206 | BLP00206 hypothetical protein |
| 227 | BLP00207 | BLP00207 hypothetical protein |
| 228 | BLP00208 | BLP00208 hypothetical protein |
| 229 | BLP00209 | BLP00209 hypothetical protein |
| 230 | BLP00210 | BLP00210 hypothetical protein |
| 241 | BLP00220 | BLP00220 hypothetical protein |
| 243 | BLP00222 | BLP00222 hypothetical protein |
| 244 | BLP04911 | BLP04911 conserved hyopothetical protein |
| 245 | BLP04912 | BLP04912 hypothetical protein |
| 246 | BLP04693 | BLP04693 hypothetical protein |
| 247 | BLP04913 | BLP04913 hypothetical protein |
| 248 | BLP00223 | BLP00223 hypothetical protein |
| 249 | BLP04914 | BLP04914 hypothetical protein |
| 250 | BLP00224 | BLP00224 hypothetical protein |
| 251 | BLP04915 | BLP04915 hypothetical protein |
| 252 | BLP00225 | BLP00225 hypothetical protein |
| 253 | BLP04916 | BLP04916 hypothetical protein |
| 254 | BLP00226 | BLP00226 hypothetical protein |
| 255 | BLP00227 | BLP00227 hypothetical protein |
| 256 | BLP04917 | BLP04917 hypothetical protein |
| 257 | BLP04918 | BLP04918 hypothetical protein |
| 258 | BLP00228 | BLP00228 hypothetical protein |
| 259 | BLP04694 | BLP04694 hypothetical protein |
| 260 | BLP00229 | BLP00229 hypothetical protein |

(continued)

| SEQID | Gene Name | Gene Product |
|---|---|---|
| 263 | BLP00231 | BLP00231 hypothetical protein |
| 264 | BLP00232 | BLP00232 hypothetical protein |
| 265 | BLP00233 | BLP00233 hypothetical protein |
| 266 | BLP04695 | BLP04695 hypothetical protein |
| 287 | BLP00254 | BLP00254 hypothetical protein |
| 306 | BLP00272 | BLP00272 hypothetical protein |
| 308 | BLP00274 | BLP00274 hypothetical protein |
| 323 | BLP04697 | BLP04697 hypothetical protein |
| 350 | BLP00315 | BLP00315 hypothetical protein |
| 354 | BLP00319 | BLP00319 hypothetical protein |
| 360 | BLP00325 | BLP00325 hypothetical protein |
| 363 | BLP00328 | BLP00328 hypothetical protein |
| 367 | BLP00332 | BLP00332 hypothetical protein |
| 381 | BLP00347 | BLP00347 hypothetical protein |
| 445 | BLP00411 | BLP00411 conserved hypothetical protein |
| 469 | BLP00435 | BLP00435 hypothetical protein |
| 487 | BLP00453 | BLP00453 hypothetical protein |
| 488 | BLP04698 | BLP04698 hypothetical protein |
| 516 | BLP00481 | BLP00481 hypothetical protein |
| 523 | BLP00488 | BLP00488 conserved hypothetical protein |
| 524 | BLP00489 | BLP00489 hypothetical protein |
| 537 | BLP00502 | BLP00502 hypothetical protein |
| 541 | BLP00506 | BLP00506 conserved hypothetical protein |
| 575 | BLP00538 | BLP00538 hypothetical protein |
| 592 | BLP00554 | BLP00554 hypothetical protein |
| 607 | BLP00569 | BLP00569 hypothetical protein |
| 621 | BLP00582 | BLP00582 hypothetical protein |
| 627 | BLP04701 | BLP04701 hypothetical protein |
| 628 | BLP00588 | BLP00588 hypothetical protein |
| 666 | BLP04703 | BLP04703 hypothetical protein |
| 672 | BLP00631 | BLP00631 hypothetical protein |
| 688 | BLP04706 | BLP04706 hypothetical protein |
| 691 | BLP00650 | BLP00650 hypothetical protein |
| 702 | BLP00661 | BLP00661 hypothetical protein |
| 710 | BLP00669 | BLP00669 hypothetical protein |
| 719 | BLP04707 | BLP04707 hypothetical protein |
| 726 | BLP00683 | BLP00683 hypothetical protein |
| 748 | BLP00705 | BLP00705 hypothetical protein |

(continued)

| SEQID | Gene Name | Gene Product |
|---|---|---|
| 751 | BLP00708 | BLP00708 hypothetical protein |
| 773 | BLP00731 | BLP00731 conserved hypothetical protein |
| 774 | BLP00732 | BLP00732 hypothetical protein |
| 777 | BLP00735 | BLP00735 hypothetical protein |
| 791 | BLP00749 | BLP00749 hypothetical protein |
| 831 | BLP00787 | BLP00787 hypothetical protein |
| 833 | BLP00789 | BLP00789 hypothetical protein |
| 842 | BLP00798 | BLP00798 conserved hypothetical protein |
| 847 | BLP00803 | BLP00803 hypothetical protein |
| 855 | BLP04709 | BLP04709 hypothetical protein |
| 857 | BLP00812 | BLP00812 hypothetical protein |
| 885 | BLP00840 | BLP00840 Putative Type IIc bacteriocin lichenein |
| 887 | BLP00842 | BLP00842 ABC transporter, permease subunit |
| 901 | BLP00856 | BLP00856 hypothetical protein |
| 944 | BLP00900 | BLP00900 conserved hypothetical protein |
| 982 | BLP00938 | BLP00938 hypothetical protein |
| 991 | BLP00947 | BLP00947 hypothetical protein |
| 994 | BLP00950 | BLP00950 hypothetical protein |
| 1018 | BLP00973 | BLP00973 hypothetical protein |
| 1032 | BLP00985 | BLP00985 hypothetical protein |
| 1034 | BLP00987 | BLP00987 hypothetical protein |
| 1058 | BLP01011 | BLP01011 hypothetical protein |
| 1078 | BLP01032 | BLP01032 hypothetical protein |
| 1088 | BLP04710 | BLP04710 hypothetical protein |
| 1098 | BLP01051 | BLP01051 hypothetical protein |
| 1108 | BLP04711 | BLP04711 hypothetical protein |
| 1112 | BLP04712 | BLP04712 hypothetical protein |
| 1128 | BLP01079 | BLP01079 hypothetical protein |
| 1148 | BLP01099 | BLP01099 hypothetical protein |
| 1153 | BLP01105 | BLP01105 hypothetical protein |
| 1154 | BLP01106 | BLP01106 hypothetical protein |
| 1158 | BLP01110 | BLP01110 hypothetical protein |
| 1160 | BLP01112 | BLP01112 hypothetical protein |
| 1164 | BLP01116 | BLP01116 hypothetical protein |
| 1175 | BLP01127 | BLP01127 hypothetical protein |
| 1176 | BLP04814 | BLP04814 unassigned |
| 1177 | BLP01128 | BLP01128 hypothetical protein |

(continued)

| SEQID | Gene Name | Gene Product |
|---|---|---|
| 1180 | BLP01131 | BLP01131 hypothetical protein |
| 1183 | BLP01134 | BLP01134 hypothetical protein |
| 1184 | BLP01135 | BLP01135 conserved hypothetical protein |
| 1193 | BLP04850 | BLP04850 conserved hypothetical protein |
| 1194 | BLP01144 | BLP01144 conserved hypothetical protein |
| 1199 | BLP01149 | BLP01149 conserved hypothetical protein |
| 1237 | BLP01185 | BLP01185 hypothetical protein |
| 1244 | BLP04714 | BLP04714 hypothetical protein |
| 1262 | BLP01209 | BLP01209 hypothetical protein |
| 1263 | BLP04715 | BLP04715 hypothetical protein |
| 1265 | BLP01210 | BLP01210 hypothetical protein |
| 1266 | BLP01211 | BLP01211 hypothetical protein |
| 1302 | BLP01246 | BLP01246 hypothetical protein |
| 1313 | BLP01257 | BLP01257 hypothetical protein |
| 1314 | BLP01258 | BLP01258 hypothetical protein |
| 1315 | BLP01259 | BLP01259 hypothetical protein |
| 1316 | BLP01260 | BLP01260 hypothetical protein |
| 1318 | BLP01262 | BLP01262 hypothetical protein |
| 1345 | BLP01290 | BLP01290 hypothetical protein |
| 1355 | BLP01300 | BLP01300 hypothetical protein |
| 1373 | BLP01318 | BLP01318 hypothetical protein |
| 1374 | BLP01319 | BLP01319 hypothetical protein |
| 1401 | BLP04717 | BLP04717 hypothetical protein |
| 1424 | BLP01368 | BLP01368 hypothetical protein |
| 1432 | BLP04816 | BLP04816 conserved hypothetical protein |
| 1433 | BLP01376 | BLP01376 hypothetical protein |
| 1440 | BLP01383 | BLP01383 conserved hypothetical protein |
| 1442 | BLP04719 | BLP04719 hypothetical protein |
| 1448 | BLP01390 | BLP01390 hypothetical protein |
| 1455 | BLP04720 | BLP04720 hypothetical protein |
| 1470 | BLP01410 | BLP01410 conserved hypothetical |
| 1509 | BLP01451 | BLP01451 hypothetical protein |
| 1527 | BLP01469 | BLP01469 hypothetical protein |
| 1539 | BLP01481 | BLP01481 hypothetical protein |
| 1556 | BLP04721 | BLP04721 hypothetical protein |
| 1565 | BLP01506 | BLP01506 hypothetical protein |
| 1589 | BLP01530 | BLP01530 hypothetical protein |
| 1619 | BLP01561 | BLP01561 hypothetical protein |

(continued)

| SEQID | Gene Name | Gene Product |
|---|---|---|
| 1634 | BLP01575 | BLP01575 hypothetical protein |
| 1635 | BLP01576 | BLP01576 hypothetical protein |
| 1636 | BLP04851 | BLP04851 conserved hypothetical protein |
| 1638 | BLP04852 | BLP04852 hypothetical protein |
| 1639 | BLP01578 | BLP01578 hypothetical protein |
| 1640 | BLP04853 | BLP04853 hypothetical protein |
| 1641 | BLP04722 | BLP04722 hypothetical protein |
| 1642 | BLP01579 | BLP01579 hypothetical protein |
| 1643 | BLP04854 | BLP04854 hypothetical protein |
| 1644 | BLP04855 | BLP04855 hypothetical protein |
| 1645 | BLP04724 | BLP04724 hypothetical protein |
| 1646 | BLP01580 | BLP01580 hypothetical protein |
| 1647 | BLP01581 | BLP01581 hypothetical protein |
| 1649 | BLP04856 | BLP04856 hypothetical protein |
| 1650 | BLP01583 | BLP01583 hypothetical protein |
| 1651 | BLP01584 | BLP01584 hypothetical protein |
| 1652 | BLP04857 | BLP04857 hypothetical protein |
| 1653 | BLP01585 | BLP01585 hypothetical protein |
| 1654 | BLP01586 | BLP01586 hypothetical protein |
| 1655 | BLP04725 | BLP04725 hypothetical protein |
| 1657 | BLP01589 | BLP01589 integrase like protein |
| 1658 | BLP01590 | BLP01590 phage protein |
| 1659 | BLP01591 | BLP01591 phage protein |
| 1660 | BLP01592 | BLP01592 phage portal like protein |
| 1661 | BLP01593 | BLP01593 phage head protein |
| 1662 | BLP01594 | BLP01594 phage protein |
| 1663 | BLP01595 | BLP01595 phage protein minor capsid-like |
| 1664 | BLP01596 | BLP01596 phage protein |
| 1665 | BLP01597 | BLP01597 hypothetical protein |
| 1666 | BLP01598 | BLP01598 hypothetical protein |
| 1667 | BLP04858 | BLP04858 hypothetical protein |
| 1668 | BLP01599 | BLP01599 hypothetical protein |
| 1669 | BLP01600 | BLP01600 hypothetical protein |
| 1677 | BLP01609 | BLP01609 hypothetical protein |
| 1684 | BLP01616 | BLP01616 hypothetical protein |
| 1688 | BLP04726 | BLP04726 hypothetical protein |
| 1692 | BLP01623 | BLP01623 hypothetical protein |
| 1694 | BLP01625 | BLP01625 hypothetical protein |

(continued)

| SEQID | Gene Name | Gene Product |
|---|---|---|
| 1703 | BLP01634 | BLP01634 hypothetical protein |
| 1704 | BLP01635 | BLP01635 hypothetical protein |
| 1728 | BLP01660 | BLP01660 hypothetical protein |
| 1732 | BLP01663 | BLP01663 hypothetical protein |
| 1734 | BLP01665 | BLP01665 hypothetical protein |
| 1736 | BLP01667 | BLP01667 hypothetical protein |
| 1775 | BLP01705 | BLP01705 hypothetical protein |
| 1783 | BLP04727 | BLP04727 hypothetical protein |
| 1816 | BLP01743 | BLP01743 hypothetical protein |
| 1831 | BLP01758 | BLP01758 hypothetical protein |
| 1838 | BLP01765 | BLP01765 hypothetical protein |
| 1851 | BLP01777 | BLP01777 hypothetical protein |
| 1853 | BLP01779 | BLP01779 hypothetical protein |
| 1866 | BLP01792 | BLP01792 hypothetical protein |
| 1887 | BLP01812 | BLP01812 hypothetical protein |
| 2002 | BLP01924 | BLP01924 hypothetical protein |
| 2007 | BLP01929 | BLP01929 hypothetical protein |
| 2022 | BLP04730 | BLP04730 hypothetical protein |
| 2038 | BLP01957 | BLP01957 hypothetical protein |
| 2044 | BLP01963 | BLP01963 hypothetical protein |
| 2067 | BLP01985 | BLP01985 hypothetical protein |
| 2072 | BLP01990 | BLP01990 hypothetical protein |
| 2076 | BLP04731 | BLP04731 hypothetical protein |
| 2094 | BLP04732 | BLP04732 hypothetical protein |
| 2106 | BLP02022 | BLP02022 hypothetical protein |
| 2119 | BLP02034 | BLP02034 hypothetical protein |
| 2120 | BLP02035 | BLP02035 hypothetical protein |
| 2121 | BLP02036 | BLP02036 hypothetical protein |
| 2124 | BLP02038 | BLP02038 hypothetical protein |
| 2131 | BLP02045 | BLP02045 conserved hypothetical protein |
| 2139 | BLP02049 | BLP02049 hypothetical protein |
| 2147 | BLP02056 | BLP02056 hypothetical protein |
| 2148 | BLP02057 | BLP02057 hypothetical protein |
| 2166 | BLP02075 | BLP02075 hypothetical protein |
| 2171 | BLP02080 | BLP02080 hypothetical protein |
| 2194 | BLP02103 | BLP02103 hypothetical protein |
| 2196 | BLP04733 | BLP04733 hypothetical protein |
| 2198 | BLP02106 | BLP02106 hypothetical protein |

(continued)

| SEQID | Gene Name | Gene Product |
|-------|-----------|--------------|
| 2205 | BLP04735 | BLP04735 hypothetical protein |
| 2211 | BLP02117 | BLP02117 hypothetical protein |
| 2213 | BLP02119 | BLP02019 conserved hypothetical protein |
| 2216 | BLP02122 | BLP02122 conserved hypothetical protein |
| 2221 | BLP02127 | BLP02127 hypothetical protein |
| 2230 | BLP04736 | BLP04736 hypothetical protein |
| 2236 | BLP02142 | BLP02142 hypothetical protein |
| 2240 | BLP02146 | BLP02146 putative transcriptional regulator |
| 2255 | BLP02162 | BLP02162 hypothetical protein |
| 2258 | BLP04737 | BLP04737 hypothetical protein |
| 2263 | BLP04738 | BLP04738 hypothetical protein |
| 2273 | BLP04739 | BLP04739 hypothetical protein |
| 2277 | BLP02179 | BLP02179 hypothetical protein |
| 2286 | BLP04740 | BLP04740 hypothetical protein |
| 2292 | BLP04866 | BLP04866 conserved hypothetical protein |
| 2296 | BLP02195 | BLP02195 conserved hypothetical protein |
| 2297 | BLP04867 | BLP04867 highly conserved hypothetical protein |
| 2298 | BLP04868 | BLP04868 conserved hypothetical, putative oxidoreductase |
| 2300 | BLP04821 | BLP04821 conserved hypothetical |
| 2303 | BLP04869 | BLP04869 hypothetical protein |
| 2304 | BLP02200 | BLP02200 resolvase |
| 2305 | BLP02201 | BLP02201 putative transposase |
| 2324 | BLP02222 | BLP02222 hypothetical protein |
| 2327 | BLP02225 | BLP02225 hypothetical protein |
| 2337 | BLP02235 | BLP02235 hypothetical protein |
| 2341 | BLP02239 | BLP02239 hypothetical protein |
| 2347 | BLP02245 | BLP02245 hypothetical protein |
| 2351 | BLP02249 | BLP02249 hypothetical protein |
| 2364 | BLP02262 | BLP02262 hypothetical protein |
| 2381 | BLP02279 | BLP02279 hypothetical protein |
| 2388 | BLP04741 | BLP04741 hypothetical protein |
| 2400 | BLP02297 | BLP02297 hypothetical protein |
| 2403 | BLP04743 | BLP04743 Adenine methyltransferase |
| 2408 | BLP02303 | BLP02303 hypothetical protein |
| 2410 | BLP02305 | BLP02305 hypothetical protein |
| 2411 | BLP04870 | BLP04870 hypothetical protein |

(continued)

| SEQID | Gene Name | Gene Product |
|---|---|---|
| 2414 | BLP04744 | BLP04744 hypothetical protein |
| 2415 | BLP02308 | BLP02308 hypothetical protein |
| 2424 | BLP02316 | BLP02316 hypothetical protein |
| 2425 | BLP04745 | BLP04745 hypothetical protein |
| 2430 | BLP02320 | BLP02320 hypothetical protein |
| 2435 | BLP04748 | BLP04748 hypothetical protein |
| 2439 | BLP04749 | BLP04749 hypothetical protein |
| 2457 | BLP02344 | BLP02344 hypothetical protein |
| 2467 | BLP02354 | BLP02354 hypothetical protein |
| 2468 | BLP04751 | BLP04751 hypothetical protein |
| 2469 | BLP04752 | BLP04752 hypothetical protein |
| 2483 | BLP02368 | BLP02368 hypothetical protein |
| 2491 | BLP02376 | BLP02376 hypothetical protein |
| 2498 | BLP02383 | BLP02383 hypothetical protein |
| 2504 | BLP02389 | yodV, yokI phage protein |
| 2505 | BLP04872 | yotK phage related sp-beta protein YotK |
| 2506 | BLP02390 | BLP02390 hypothetical phage protein |
| 2507 | BLP02391 | BLP02391 hypothetical phage protein |
| 2508 | BLP02392 | BLP02392 hypothetical phage protein |
| 2509 | BLP02393 | yotH phage protein YotH |
| 2510 | BLP04754 | BLP04754 hypothetical phage protein |
| 2511 | BLP02394 | yotB phage protein YotB |
| 2512 | BLP02395 | BLP02395 hypothetical protein |
| 2514 | BLP02396 | BLP02396 hypothetical protein |
| 2515 | BLP02397 | BLP02397 hypothetical protein |
| 2516 | BLP02398 | yosX phage protein |
| 2518 | BLP02400 | yosV YosV |
| 2519 | BLP02401 | BLP02401 hypothetical protein |
| 2520 | BLP02402 | yncF phage protein |
| 2521 | BLP02403 | BLP02403 hypothetical protein |
| 2525 | BLP02407 | BLP02407 hypothetical protein |
| 2526 | BLP02409 | BLP02408 HNH endonuclease |
| 2528 | BLP02411 | yosL YosL |
| 2529 | BLP02412 | BLP02412 hypothetical protein |
| 2530 | BLP02413 | yosI conserved hypothetical YosI |
| 2531 | BLP02414 | yosH phage like conserved hyopothetical YosH |
| 2532 | BLP02415 | BLP02415 conserved hypothetical protein |

(continued)

| SEQID | Gene Name | Gene Product |
|-------|-----------|--------------|
| 2533 | BLP04755 | BLP04755 hypothetical protein |
| 2534 | BLP02416 | BLP02416 hypothetical protein |
| 2535 | BLP02417 | BLP02417 phage hypothetical protein |
| 2536 | BLP02418 | BLP02418 phage hypothetical protein |
| 2537 | BLP02419 | BLP02419 hypothetical protein |
| 2538 | BLP02420 | yorZ YorZ |
| 2539 | BLP02421 | yorY YorY |
| 2540 | BLP04875 | BLP04875 hypothetical protein |
| 2541 | BLP02422 | BLP02422 conserved hyopothetical |
| 2542 | BLP02423 | yorQ YorQ |
| 2543 | BLP02424 | BLP02424 hypothetical protein |
| 2544 | BLP04756 | yorP YorP |
| 2547 | BLP02427 | BLP02427 putative HNH endonuclease |
| 2549 | BLP02430 | yorJ spbeta like phage protein YorJ |
| 2551 | BLP02432 | yorH YorH |
| 2552 | BLP02433 | yorG ATP_GTP binding phage protein YorG |
| 2553 | BLP02434 | yorF conserved hypothetical phage protein YorF |
| 2554 | BLP04876 | yorE conserved hypothetical protein YorE |
| 2555 | BLP04758 | BLP04758 hypothetical protein |
| 2556 | BLP02435 | BLP02435 hypothetical protein |
| 2557 | BLP02436 | BLP02436 hypothetical protein |
| 2558 | BLP04877 | BLP04877 hypothetical protein |
| 2559 | BLP02437 | BLP02437 hypothetical protein |
| 2560 | BLP04759 | BLP04759 hypothetical protein |
| 2561 | BLP02438 | BLP02438 hypothetical protein |
| 2562 | BLP02439 | BLP02439 hypothetical protein |
| 2564 | BLP02441 | BLP02441 hypothetical protein |
| 2565 | BLP02442 | yoqX conserved hypothetical phage protein YoqX |
| 2567 | BLP02444 | BLP02444 hypothetical protein |
| 2568 | BLP02445 | BLP02445 hypothetical protein |
| 2569 | BLP02446 | BLP02446 hypothetical protein |
| 2570 | BLP02447 | BLP02447 hypothetical protein |
| 2571 | BLP04760 | BLP04760 hypothetical protein |
| 2572 | BLP04878 | BLP04878 hypothetical protein |
| 2573 | BLP02448 | BLP02448 hypothetical protein |
| 2575 | BLP02450 | BLP02450 hypothetical protein |

(continued)

| SEQID | Gene Name | Gene Product |
|---|---|---|
| 2576 | BLP02451 | BLP02451 hypothetical protein |
| 2577 | BLP02452 | BLP02452 hypothetical protein |
| 2579 | BLP02454 | BLP02454 hypothetical protein |
| 2580 | BLP02455 | BLP02455 hypothetical protein |
| 2581 | BLP04761 | BLP04761 hypothetical protein |
| 2582 | BLP02456 | yopR YopR |
| 2583 | BLP02457 | yopQ YopQ |
| 2584 | BLP02458 | yopP integrase family phage protein YopP |
| 2585 | BLP02459 | yopN SPBc2-like prophage-derived hypothetical protein YopN |
| 2586 | BLP02460 | yopM Hypothetical protein YopM |
| 2587 | BLP02461 | BLP02461 hypothetical protein |
| 2588 | BLP02462 | yopK conserved hypothetical protein YopK |
| 2590 | BLP02463 | BLP02463 hypothetical protein |
| 2591 | BLP02464 | BLP02464 conserved hypothetical protein |
| 2592 | BLP02465 | BLP02465 hypothetical protein |
| 2594 | BLP04880 | BLP04880 hypothetical protein |
| 2595 | BLP02467 | BLP02467 conserved hypothetical protein |
| 2596 | BLP02468 | BLP02468 conserved hypothetical protein |
| 2597 | BLP02469 | BLP02469 hypothetical protein |
| 2598 | BLP02471 | BLP02471 hypothetical protein |
| 2599 | BLP02472 | BLP02472 hypothetical protein |
| 2600 | BLP02473 | BLP02473 hypothetical protein |
| 2601 | BLP02474 | BLP02474 hypothetical protein |
| 2602 | BLP02475 | BLP02475 hypothetical protein |
| 2603 | BLP02476 | BLP02476 hypothetical protein |
| 2604 | BLP02477 | BLP02477 conserved hypothetical protein |
| 2605 | BLP02478 | BLP02478 hypothetical protein |
| 2606 | BLP02479 | BLP02479 conserved hypothetical protein |
| 2607 | BLP02480 | BLP02476 hypothetical protein |
| 2608 | BLP02481 | BLP02476 hypothetical protein |
| 2609 | BLP02482 | BLP02482 hypothetical protein |
| 2610 | BLP02483 | BLP02483 hypothetical protein |
| 2611 | BLP02484 | BLP02483 hypothetical protein |
| 2612 | BLP02485 | BLP02485 hypothetical protein |
| 2613 | BLP02486 | yopB conserved phage hypothetical protein YopB |
| 2614 | BLP02487 | BLP02487 hypothetical protein |

(continued)

| SEQID | Gene Name | Gene Product |
|-------|-----------|--------------|
| 2615 | BLP02488 | BLP02488 hypothetical protein |
| 2616 | BLP02489 | BLP02489 hypothetical protein |
| 2617 | BLP04881 | BLP04881 hypothetical protein |
| 2618 | BLP04882 | BLP04882 hypothetical protein |
| 2619 | BLP02490 | BLP02490 hypothetical protein |
| 2620 | BLP02491 | BLP02491 hypothetical protein |
| 2621 | BLP02492 | BLP02492 hypothetical protein |
| 2622 | BLP02493 | BLP02493 hypothetical protein |
| 2623 | BLP02494 | BLP02494 hypothetical protein |
| 2624 | BLP02495 | BLP02495 hypothetical protein |
| 2625 | BLP02496 | BLP02496 hypothetical protein |
| 2626 | BLP02497 | BLP02497 hypothetical protein |
| 2627 | BLP02498 | yonV hypothetical phage protein YonV |
| 2628 | BLP02499 | BLP02499 Putative GIY-YIG endonuclease |
| 2629 | BLP04762 | BLP04762 hypothetical protein |
| 2630 | BLP04763 | BLP04763 hypothetical protein |
| 2631 | BLP04883 | BLP04883 conserved hypothetical phage protein |
| 2633 | BLP04885 | BLP04885 hypothetical protein |
| 2634 | BLP02500 | BLP02500 hypothetical protein |
| 2635 | BLP02501 | BLP02501 conserved phage like protein |
| 2636 | BLP02502 | BLP02502 conserved phage related protein |
| 2638 | BLP02504 | BLP02504 hypothetical protein |
| 2639 | BLP02505 | BLP02505 conserved hypothetical protein |
| 2640 | BLP02506 | yonK YonK |
| 2641 | BLP02507 | yonJ phage protein YonJ |
| 2642 | BLP02508 | BLP02508 hypothetical protein |
| 2643 | BLP02509 | BLP02509 hypothetical protein |
| 2644 | BLP02510 | BLP02510 hypothetical protein |
| 2645 | BLP04886 | BLP04886 hypothetical protein |
| 2646 | BLP02511 | BLP02511 hypothetical protein |
| 2647 | BLP02512 | yonG YonG |
| 2648 | BLP02513 | yonF YonF |
| 2649 | BLP02514 | yonE YonE |
| 2650 | BLP02515 | yonD YonD |
| 2651 | BLP02516 | yonC YonC |
| 2652 | BLP02517 | yonB YonB |
| 2653 | BLP02518 | yonA YonA |

(continued)

| SEQID | Gene Name | Gene Product |
|---|---|---|
| 2654 | BLP02519 | yomZ YomZ |
| 2655 | BLP04822 | yomY YomY |
| 2656 | BLP02520 | yomX YomX |
| 2657 | BLP02521 | yomW YomW |
| 2658 | BLP02522 | yomV YomV |
| 2659 | BLP02523 | yomU YomU |
| 2661 | BLP02525 | yomS YomS |
| 2664 | BLP02527 | yomO conserved hypothetical phage protein YomO |
| 2665 | BLP04888 | yomN conserved hypothetical protein YomN |
| 2667 | BLP02529 | BLP02529 hypothetical protein |
| 2669 | BLP04889 | BLP04889 conserved hypothetical protein |
| 2670 | BLP02531 | yomK conserved hypothetical phage protein YomK |
| 2671 | BLP02532 | BLP02532 hypothetical protein |
| 2672 | BLP02533 | BLP02533 conserved hypothetical phage protein |
| 2673 | BLP02534 | yomJ YomJ |
| 2674 | BLP02535 | BLP02535 hypothetical protein |
| 2675 | BLP04890 | BLP04890 hypothetical protein |
| 2676 | BLP02536 | BLP02536 conserved hypothetical protein |
| 2677 | BLP02537 | BLP02537 hypothetical protein |
| 2679 | BLP02540 | yomH conserved phage protein YomH |
| 2680 | BLP02541 | yomG conserved phage protein YomG |
| 2681 | BLP02542 | yomF conserved phage protein YomF |
| 2684 | BLP02545 | BLP02545 hypothetical protein |
| 2685 | BLP04764 | BLP04764 putative holin |
| 2686 | BLP02546 | BLP02546 hypothetical protein |
| 2689 | BLP02549 | BLP02549 hypothetical protein |
| 2691 | BLP02551 | BLP02551 conserved hypothetical protein |
| 2692 | BLP04891 | yobK conserved hypothetical protein YobK |
| 2693 | BLP04892 | BLP04892 conserved hypothetical |
| 2696 | BLP02554 | yokH YokH |
| 2697 | BLP02555 | BLP02555 conserved hypothetical protein |
| 2706 | BLP02565 | BLP02565 conserved hypothetical protein |
| 2723 | BLP02581 | BLP02581 hypothetical protein |
| 2730 | BLP02588 | BLP02588 hypothetical protein |
| 2733 | BLP04893 | BLP04893 hypothetical protein |

(continued)

| SEQID | Gene Name | Gene Product |
|---|---|---|
| 2751 | BLP02608 | sspL Small, acid-soluble spore protein L (SASP L) SspL |
| 2762 | BLP02619 | BLP02619 hypothetical protein |
| 2795 | BLP02651 | BLP02651 hypothetical protein |
| 2806 | BLP02662 | BLP02662 hypothetical protein |
| 2813 | BLP02669 | BLP02669 hypothetical protein |
| 2834 | BLP04766 | BLP04766 hypothetical protein |
| 2835 | BLP02690 | BLP02690 hypothetical protein |
| 2841 | BLP02696 | BLP02696 hypothetical protein |
| 2850 | BLP02705 | BLP02705 hypothetical protein |
| 2866 | BLP02722 | BLP02722 hypothetical protein |
| 2867 | BLP02723 | BLP02723 hypothetical protein |
| 2874 | BLP02730 | BLP02730 hypothetical protein |
| 2878 | BLP04767 | BLP04767 hypothetical protein |
| 2898 | BLP02752 | BLP02752 hypothetical protein |
| 2902 | BLP02756 | BLP02756 conserved hypothetical protein |
| 2904 | BLP02758 | BLP02758 hypothetical protein |
| 2917 | BLP02771 | BLP02771 hypothetical protein |
| 2947 | BLP04769 | BLP04769 hypothetical protein |
| 2957 | BLP04939 | BLP04939 hypothetical protein |
| 2960 | BLP02811 | BLP02811 hypothetical protein |
| 2969 | BLP02820 | BLP02820 hypothetical protein |
| 2972 | BLP04770 | BLP04770 hypothetical protein |
| 2973 | BLP02823 | BLP02823 hypothetical protein |
| 2999 | BLP02851 | BLP02851 hypothetical protein |
| 3004 | BLP02857 | BLP02857 hypothetical protein |
| 3019 | BLP02873 | BLP02873 hypothetical protein |
| 3028 | BLP02882 | BLP02882 hypothetical protein |
| 3054 | BLP02908 | BLP02908 hypothetical protein |
| 3064 | BLP02918 | BLP02918 conserved hypothetical protein |
| 3082 | BLP02936 | BLP02936 hypothetical protein |
| 3093 | BLP02947 | BLP02947 hypothetical protein |
| 3123 | BLP02976 | BLP02976 conserved hypothetical protein |
| 3137 | BLP04897 | BLP04897 conserved hypothetical protein |
| 3141 | BLP02993 | BLP02993 conserved hypothetical |
| 3142 | BLP04898 | BLP04898 conserved hypothetical protein |
| 3144 | BLP04824 | BLP04824 conserved hypothetical protein |
| 3148 | BLP02998 | BLP02998 hypothetical protein |

(continued)

| SEQID | Gene Name | Gene Product |
|---|---|---|
| 3149 | BLP02999 | BLP02999 putative resolvase |
| 3150 | BLP03000 | BLP03000 putative transposase |
| 3162 | BLP03011 | BLP03011 hypothetical protein |
| 3222 | BLP03071 | BLP03071 hypothetical protein |
| 3226 | BLP04902 | BLP04902 hypothetical protein |
| 3227 | BLP03076 | BLP03076 hypothetical protein |
| 3229 | BLP03078 | BLP03078 hypothetical protein |
| 3231 | BLP09003 | hypothetical protein |
| 3244 | BLP03092 | BLP03092 hypothetical protein |
| 3248 | BLP03097 | BLP03097 hypothetical protein |
| 3259 | BLP03108 | BLP03108 hypothetical protein |
| 3264 | BLP03113 | BLP03113 conserved hypothetical protein |
| 3287 | BLP03137 | BLP03137 hypothetical protein |
| 3291 | BLP04772 | BLP04772 hypothetical protein |
| 3303 | BLP04773 | BLP04773 hypothetical protein |
| 3310 | BLP03158 | BLP03158 hypothetical protein |
| 3311 | BLP03159 | BLP03159 hypothetical protein |
| 3321 | BLP03169 | BLP03169 hypothetical protein |
| 3324 | BLP03172 | BLP03172 hypothetical protein |
| 3355 | BLP03204 | BLP03204 hypothetical protein |
| 3362 | BLP03211 | BLP03211 hypothetical protein |
| 3371 | BLP04774 | BLP04774 hypothetical protein |
| 3429 | BLP03277 | BLP03277 hypothetical protein |
| 3434 | BLP03282 | BLP03282 hypothetical protein |
| 3442 | BLP03290 | BLP03290 hypothetical protein |
| 3446 | BLP04776 | BLP04776 hypothetical protein |
| 3458 | BLP03306 | BLP03306 conserved hypothetical protein |
| 3471 | BLP03319 | BLP03319 hypothetical protein |
| 3486 | BLP04903 | BLP04903 hypothetical protein |
| 3496 | BLP04904 | BLP04904 hypothetical protein |
| 3499 | BLP03344 | BLP03344 hypothetical protein |
| 3503 | BLP03348 | BLP03348 hypothetical protein |
| 3504 | BLP03349 | BLP03349 hypothetical protein |
| 3508 | BLP03353 | BLP03353 conserved hypothetical protein |
| 3549 | BLP03393 | BLP03393 hypothetical protein |
| 3558 | BLP04778 | BLP04778 hypothetical protein |
| 3577 | BLP03421 | BLP03421 hypothetical protein |
| 3593 | BLP04780 | BLP04780 hypothetical protein |

(continued)

| SEQID | Gene Name | Gene Product |
|---|---|---|
| 3598 | BLP03440 | BLP03440 hypothetical protein |
| 3601 | BLP03443 | BLP03443 hypothetical protein |
| 3603 | BLP04945 | BLP04945 conserved hypothetical |
| 3606 | BLP04781 | BLP04781 hypothetical protein |
| 3611 | BLP03451 | BLP03451 hypothetical protein |
| 3642 | BLP03483 | BLP03483 conserved hypothetical protein |
| 3653 | BLP03493 | BLP03493 hypothetical protein |
| 3655 | BLP03495 | BLP03495 hypothetical protein |
| 3670 | BLP03510 | BLP03510 hypothetical protein |
| 3673 | BLP03513 | BLP03513 hypothetical protein |
| 3682 | BLP03522 | BLP03522 hypothetical protein |
| 3690 | BLP04782 | BLP04782 hypothetical protein |
| 3694 | BLP03533 | BLP03533 hypothetical protein |
| 3707 | BLP03546 | BLP03546 hypothetical protein |
| 3716 | BLP03554 | BLP03554 hypothetical protein |
| 3727 | BLP04784 | BLP04784 hypothetical protein |
| 3736 | BLP04786 | BLP04786 hypothetical protein |
| 3737 | BLP03572 | BLP03572 hypothetical protein |
| 3744 | BLP03580 | BLP03580 conserved hypothetical protein |
| 3753 | BLP04788 | BLP04788 hypothetical protein |
| 3765 | BLP03598 | BLP03598 hypothetical protein |
| 3790 | BLP03623 | BLP03623 conserved hypothetical protein |
| 3798 | BLP09002 | hypothetical protein |
| 3799 | BLP03630 | BLP03630 hypothetical protein |
| 3872 | BLP03701 | BLP03701 hypothetical protein |
| 3881 | BLP03709 | BLP03709 hypothetical protein |
| 3885 | BLP03713 | BLP03713 conserved hypothetical protein |
| 3915 | BLP03742 | BLP03742 hypothetical protein |
| 3916 | BLP03743 | BLP03743 hypothetical protein |
| 3922 | BLP03749 | BLP03749 hyopthetical protein |
| 3931 | BLP04790 | BLP04790 hypothetical protein |
| 3938 | BLP03764 | BLP03764 hypothetical protein |
| 3969 | BLP03795 | BLP03795 hypothetical protein |
| 4011 | BLP04829 | BLP04829 hypothetical protein |
| 4017 | BLP03843 | BLP03843 hypothetical protein |
| 4056 | BLP03881 | BLP03881 hypothetical protein |
| 4059 | BLP03884 | BLP03884 hypothetical protein |
| 4068 | BLP03893 | BLP03893 hypothetical protein |

(continued)

| SEQID | Gene Name | Gene Product |
|---|---|---|
| 4076 | BLP03901 | BLP03901 hypothetical protein |
| 4077 | BLP03902 | BLP03902 hypothetical protein |
| 4081 | BLP03906 | BLP03906 conserved hypothetical putative ATP binding protein |
| 4082 | BLP03907 | BLP03907 hypothetical protein |
| 4085 | BLP03911 | BLP03911 conserved hypothetical |
| 4090 | BLP03916 | BLP03916 hypothetical protein |
| 4106 | BLP03932 | BLP03932 hypothetical protein |
| 4112 | BLP04792 | BLP04792 hypothetical protein |
| 4117 | BLP03942 | BLP03942 hypothetical protein |
| 4144 | BLP03969 | BLP03969 hypothetical protein |
| 4145 | BLP03970 | BLP03970 hypothetical protein |
| 4158 | BLP03983 | BLP03983 hypothetical protein |
| 4162 | BLP03986 | BLP03986 hypothetical protein |
| 4163 | BLP03987 | BLP03987 hypothetical protein |
| 4164 | BLP03988 | BLP03988 hypothetical protein |
| 4165 | BLP04793 | BLP04793 hypothetical protein |
| 4166 | BLP03989 | BLP03989 hypothetical protein |
| 4167 | BLP03990 | BLP03990 hypothetical protein |
| 4168 | BLP03991 | BLP03991 hypothetical protein |
| 4169 | BLP03992 | BLP03992 hypothetical protein |
| 4170 | BLP04907 | BLP04907 hypothetical protein |
| 4171 | BLP03993 | BLP03993 hypothetical protein |
| 4172 | BLP04794 | BLP04794 hypothetical protein |
| 4173 | BLP03994 | BLP03994 hypothetical protein |
| 4175 | BLP03995 | BLP03995 hypothetical protein |
| 4201 | BLP04021 | BLP04021 conserved hypothetical protein |
| 4235 | BLP04056 | BLP04056 hypothetical protein |
| 4285 | BLP04795 | BLP04795 hypothetical protein |
| 4288 | BLP04108 | BLP04108 hypothetical protein |
| 4305 | BLP04125 | BLP04125 hypothetical protein |
| 4323 | BLP04143 | BLP04143 hypothetical protein |
| 4333 | BLP04153 | BLP04153 conserved hypothetical protein |
| 4336 | BLP04156 | BLP04516 conserved hypothetical protein |
| 4353 | BLP04174 | BLP04174 hypothetical protein |
| 4398 | BLP04218 | BLP04218 hypothetical protein |
| 4409 | BLP04830 | atpE ATP synthase C chain AtpE |
| 4417 | BLP04236 | BLP04236 hypothetical protein |

(continued)

| SEQID | Gene Name | Gene Product |
|---|---|---|
| 4421 | BLP04240 | BLP04240 hypothetical protein |
| 4423 | BLP04796 | BLP04796 hypothetical protein |
| 4429 | BLP04247 | BLP04247 hypothetical protein |
| 4435 | BLP04797 | BLP04797 hypothetical protein |
| 4448 | BLP04799 | BLP04799 hypothetical protein |
| 4450 | BLP04265 | BLP04265 hypothetical protein |
| 4469 | BLP04283 | BLP04283 hypothetical protein |
| 4472 | BLP04286 | BLP04286 hypothetical protein |
| 4476 | BLP04801 | BLP04801 hypothetical protein |
| 4482 | BLP04294 | BLP04294 hypothetical protein |
| 4488 | BLP04300 | BLP04300 conserved hypothetical protein |
| 4513 | BLP04324 | BLP04324 hypothetical protein |
| 4518 | BLP04329 | BLP04329 hypothetical protein |
| 4544 | BLP04803 | BLP04803 hypothetical protein |
| 4559 | BLP04370 | BLP04370 hypothetical protein |
| 4560 | BLP04371 | BLP04371 hypothetical protein |
| 4569 | BLP04380 | BLP04380 hypothetical protein |
| 4571 | BLP04382 | BLP04382 hypothetical protein |
| 4578 | BLP04389 | BLP04389 hypothetical protein |
| 4607 | BLP04418 | BLP04418 conserved hypothetical protein |
| 4622 | BLP04804 | BLP04804 hypothetical protein |
| 4631 | BLP04441 | BLP04441 hypothetical protein |
| 4643 | BLP04453 | BLP04453 hypothetical protein |
| 4647 | BLP04458 | BLP04458 hypothetical protein |
| 4648 | BLP04459 | BLP04459 conserved hypothetical protein |
| 4658 | BLP04469 | BLP04469 hypothetical protein |
| 4669 | BLP04480 | BLP04480 hypothetical protein |
| 4682 | BLP04493 | BLP04493 conserved hypothetical protein |
| 4704 | BLP04515 | BLP04515 conserved hypothetical protein |
| 4718 | BLP04529 | BLP04529 hypothetical protein |
| 4754 | BLP04565 | BLP04565 hypothetical protein |
| 4767 | BLP04578 | BLP04578 hypothetical protein |
| 4786 | BLP04597 | BLP04597 hypothetical protein |
| 4792 | BLP04603 | BLP04603 hypothetical protein |
| 4797 | BLP04608 | BLP04608 hypothetical protein |
| 4804 | BLP04615 | BLP04615 highly conserved hypothetical protein |
| 4812 | BLP04805 | BLP04805 hypothetical protein |

(continued)

| SEQID | Gene Name | Gene Product |
|---|---|---|
| 4814 | BLP04806 | BLP04806 hypothetical protein |
| 4823 | BLP04631 | BLP04631 hypothetical protein |
| 4830 | BLP04638 | BLP04638 hypothetical protein |
| 4833 | BLP04641 | BLP04641 hypothetical protein |
| 4838 | BLP04646 | yycC conserved hypothetical protein YycC |
| 4848 | BLP04807 | BLP04661 hypothetical protein |
| 4849 | BLP04656 | cotF2 spore coat protein CotF2 |
| 4859 | BLP04665 | BLP04665 hypothetical protein |
| 4862 | BLP04808 | BLP04808 hypothetical protein |
| 4872 | BLP04677 | BLP04677 hypothetical protein |

**Table 4. Extracellular proteins predicted in the *Bacillus licheniformis* SJ1904 genome**

| SEQ ID | Gene Name & Product | Predicted Signal Peptide Length |
|---|---|---|
| 166 | BLP00149 conserved hypothetical phage protein | 31 |
| 200 | BLP00180 hypothetical protein | 28 |
| 268 | pbpX penicillin-binding protein | 52 |
| 274 | ybbD Glycoside hydrolase, family 3 | 27 |
| 275 | ybbE putative beta-lactamase YbbE | 22 |
| 309 | ylfP conserved hypothetical protein | 39 |
| 326 | yubF conserved protein YubF | 34 |
| 339 | BLP00304 putative lytic transglycosylase YomI | 32 |
| 362 | penP beta-lactamase precursor | 35 |
| 385 | BLP00351 putative Proteinase inhibititor 14, serpin | 26 |
| 423 | yvbX conserved protein, Glycoside Hydrolase Family 18, YvbX | 36 |
| 424 | BLP00390 Chitinase precursor, Glycoside Hydrolase Family 18 | 27 |
| 425 | mpr Glutamyl Endo peptidase | 31 |
| 433 | yvcE putative peptidoglycan hydrolase, DL-endopeptidase II family | 31 |
| 483 | BLP00449 putative extracellular solute-binding protein | 32 |
| 500 | BLP04844 conserved hypothetical protein | 25 |
| 508 | yckK putative extracellular solute-binding protein, family 3 YckK | 25 |
| 539 | phy phytase | 30 |
| 571 | BLP00534 hypothetical protein | 26 |
| 620 | BLP00581 putative chitin binding protein | 42 |
| 700 | ywpE conserved protein YwpE | 26 |
| 720 | BLP04846 conserved hypothetical protein | 34 |
| 752 | yjeAA similarilty to polysacharide deacetylase | 37 |

(continued)

| SEQ ID | Gene Name & Product | Predicted Signal Peptide Length |
|---|---|---|
| 753 | yjeA Putative Carbohydrate Esterase Family 4 protein | 33 |
| 754 | amyL alpha amylase, Glycoside Hydrolase Family 13 | 30 |
| 764 | BLP00721 hypothetical protein | 18 |
| 767 | BLP00724 hypothetical protein | 37 |
| 768 | BLP00725 hypothetical protein | 28 |
| 770 | ydjN conserved hypothetical protein YdjN | 21 |
| 813 | yerB conserved hypothetical protein YerB | 28 |
| 818 | yerH conserved hypothetical YerH | 26 |
| 891 | BLP00846 putative carboxypeptidase | 29 |
| 934 | yfkD conserved protein YfkD | 26 |
| 937 | yfjS putative Carbohydrate Esterase Family 4 YfjS | 24 |
| 949 | BLP00905 hypothetical protein | 26 |
| 976 | BLP00932 putative extracellular solute-binding protein, family 1 | 26 |
| 1064 | yhcC conserved hypothetical protein YhcC | 28 |
| 1073 | yhcM conserved hypothetical protein YhcM | 31 |
| 1116 | BLP01067 putative glucose dehydrogenase | 26 |
| 1153 | BLP01105 hypothetical protein | 28 |
| 1183 | BLP01134 hypothetical protein | 24 |
| 1192 | prsA molecular chaperone PrsA | 30 |
| 1208 | BLP04713 hypothetical protein | 23 |
| 1231 | kerA KerA | 29 |
| 1233 | yhfQ putative transferase | 32 |
| 1247 | epr extracellular serine protease | 27 |
| 1264 | BLP04716 conserved hypothetical protein | 24 |
| 1279 | BLP01224 conserved hypothetical protein | 28 |
| 1313 | BLP01257 hypothetical protein | 31 |
| 1384 | BLP01329 conserved hypothetical protein | 31 |
| 1443 | abnA Glycoside Hydrolase Family 43 | 33 |
| 1446 | BLP01388 hypothetical protein | 30 |
| 1454 | yoeB conserved hypothetical protein YoeB | 23 |
| 1456 | yocH Peptidoglycan-binding protein | 26 |
| 1460 | BLP01401 hypothetical protein | 40 |
| 1514 | ggt gamma-glutamyltranspeptidase | 26 |
| 1557 | pell pectate lyase family 1,Pell | 25 |
| 1572 | BLP01513 hypothetical protein | 30 |
| 1581 | BLP01522 hypothetical protein | 27 |
| 1598 | pbpC penicillin-binding protein 3 | 30 |

(continued)

| SEQ ID | Gene Name & Product | Predicted Signal Peptide Length |
|---|---|---|
| 1629 | BLP01571 conserved hypothetical protein | 30 |
| 1632 | BLP01573 conserved hypothetical protein | 31 |
| 1682 | BLP01614 hypothetical protein | 26 |
| 1698 | ykvT putative Cell wall hydrolase YkvT | 25 |
| 1703 | BLP01634 hypothetical protein | 20 |
| 1705 | BLP01636 conserved hypothetical protein | 22 |
| 1717 | ykwD Allergen V5_Tpx-1 related YkwD | 27 |
| 1733 | BLP01664 hypothetical protein | 28 |
| 1867 | bprA bacillopeptidase F | 28 |
| 1868 | bprB bacillopeptidase F | 31 |
| 2003 | celA Glycoside Hydrolase Family 9 | 41 |
| 2005 | celC Glycoside Hydrolase Family 5 | 34 |
| 2006 | celD Glycoside Hydrolase family 5 | 32 |
| 2038 | BLP01957 hypothetical protein | 31 |
| 2064 | BLP01982 hypothetical protein | 29 |
| 2132 | BLP04819 hypothetical protein | 25 |
| 2145 | BLP04934 hypothetical protein | 31 |
| 2154 | yoaW conserved hypothetical protein YoaW | 26 |
| 2166 | BLP02075 hypothetical protein | 33 |
| 2167 | BLP02076 phage-like protein | 38 |
| 2237 | bglC endo-1,4-beta-glucanase, Glycoside hydrolase Family 5 | 50 |
| 2242 | ywoF Polysaccharide Lyase Family 9,YwoF | 22 |
| 2247 | BLP02154 conserved hypothetical protein | 27 |
| 2271 | dctB possible C4-dicarboxylate binding protein | 29 |
| 2287 | dacC penicillin-binding protein (D-alanyl-D-alanine carboxypeptidase) | 29 |
| 2343 | BLP02241 hypothetical protein | 37 |
| 2387 | yocA putative Glycoside Hydrolase Family 23 YocA | 29 |
| 2394 | BLP02291 conserved hypothetical protein | 33 |
| 2397 | yocH conserved hypothetical protein YocH | 26 |
| 2412 | BLP02306 conserved hypothetical protein | 34 |
| 2425 | BLP04745 hypothetical protein | 24 |
| 2432 | BLP02322 hypothetical protein | 24 |
| 2456 | yvgO conserved protein YvgO | 25 |
| 2466 | yojL gamma-D-glutamate-meso-diaminopimelate muropeptidase YojL | 27 |
| 2486 | ctpA carboxy-terminal processing protease | 37 |
| 2489 | yodJ putative carboxypeptidase | 31 |

(continued)

| SEQ ID | Gene Name & Product | Predicted Signal Peptide Length |
|---|---|---|
| 2540 | BLP04875 hypothetical protein | 27 |
| 2556 | BLP02435 hypothetical protein | 24 |
| 2587 | BLP02461 hypothetical protein | 23 |
| 2592 | BLP02465 hypothetical protein | 26 |
| 2593 | BLP02466 Putative subtilase family protease | 37 |
| 2632 | BLP04884 conserved hypothetical protein | 29 |
| 2646 | BLP02511 hypothetical protein | 26 |
| 2697 | BLP02555 conserved hypothetical protein | 29 |
| 2719 | ypmR conserved protein YpmR | 24 |
| 2721 | ypmQ conserved hypothetical YpmQ | 25 |
| 2732 | ypjP conserved hypothetical protein YpjP | 30 |
| 2788 | aspB aspartate aminotransferase | 23 |
| 2789 | ypmB conserved protein YpmB | 25 |
| 2806 | BLP02662 hypothetical protein | 28 |
| 2837 | yphF conserved hypothetical protein YphF | 29 |
| 2852 | sleB spore cortex-lytic enzyme, secreted across spore | 34 |
| 2854 | BLP02710 L-asparaginase | 18 |
| 2858 | ypbG putative hydrolase YpbG | 27 |
| 2869 | BLP02725 putative peptidogycan hydrolase | 29 |
| 2873 | yheN Carbohydrate Esterase Family 4,YheN | 53 |
| 2875 | BLP02731 conserved hypothetical protein | 29 |
| 2887 | dacB D-alanyl-D-alanine carboxypeptidase (penicillin-binding protein 5*) | 28 |
| 2902 | BLP02756 conserved hypothetical protein | 33 |
| 2906 | ypuA conserved hypothetical protein YpuA | 90 |
| 2921 | dacF penicilin binding protein (putative D-alanyl-D-alanine carboxypeptidase) | 28 |
| 2948 | lip lipase | 31 |
| 2967 | lacA3 LacA3 | 24 |
| 2985 | BLP02835 conserved hypothetical protein | 31 |
| 2999 | BLP02851 hypothetical protein | 29 |
| 3017 | yqiI N-acetylmuramoyl-L-alanine amidase autolysin YqiI | 36 |
| 3021 | BLP04895 conserved hypothetical protein | 26 |
| 3070 | tasA translocation-dependent antimicrobial spore component | 28 |
| 3072 | yqxM secreted biofilm formation protein YqxM | 44 |
| 3073 | yqzG conserved hypothetical protein YqzG | 24 |
| 3090 | yqgU conserved protein YqgU | 28 |
| 3116 | yqfZ conserved protein YqfZ | 40 |

(continued)

| SEQ ID | Gene Name & Product | Predicted Signal Peptide Length |
|---|---|---|
| 3145 | gerKC putative spore germination protein | 34 |
| 3181 | yqfA conserved hypothetical protein YqfA | 37 |
| 3216 | yqeF conserved hypothetical YqeF | 31 |
| 3285 | BLP03135 putative phosphoesterase | 32 |
| 3286 | BLP03136 Esterase_lipase_thioesterase family protein | 27 |
| 3290 | yndA conserved hypothetical protein | 28 |
| 3293 | sacC Glycoside Hydrolase Family 32 | 24 |
| 3332 | yrrS conserved protein YrrS | 50 |
| 3359 | yrvJ N-acetylmuramoyl-L-alanine amidase YrvJ | 29 |
| 3375 | BLP03223 conserved hypothetical protein | 28 |
| 3383 | ymaC phage-related protein YmaC | 24 |
| 3457 | BLP03305 hypothetical protein | 33 |
| 3461 | BLP03309 hypothetical protein | 28 |
| 3494 | BLP03340 conserved hypothetical protein | 33 |
| 3496 | BLP04904 hypothetical protein | 68 |
| 3518 | abnA-like Glycoside Hydrolase Family 43 | 28 |
| 3653 | BLP03493 hypothetical protein | 27 |
| 3659 | yteS conserved protein YteS | 26 |
| 3682 | BLP03522 hypothetical protein | 28 |
| 3707 | BLP03546 hypothetical protein | 28 |
| 3708 | ytkA conserved protein YtkA | 29 |
| 3744 | BLP03580 conserved hypothetical protein | 28 |
| 3787 | BLP03620 Glycoside Hydrolase Family 12 | 30 |
| 3824 | BLP03654 hypothetical protein | 30 |
| 3850 | BLP03680 conserved hypothetical protein | 22 |
| 3868 | yuiC conserved protein YuiC | 34 |
| 3885 | BLP03713 conserved hypothetical protein | 28 |
| 3903 | yunA putative metallopeptidase | 22 |
| 3925 | bsn extracellular ribonuclease | 40 |
| 3963 | yusW conserved hypothetical protein YusW | 28 |
| 3983 | liaG conserved protein LiaG | 39 |
| 4026 | bdbD thiol-disulfide oxidoreductase | 36 |
| 4028 | BLP03854 putative Extracellular solute-binding protein | 27 |
| 4032 | BLP03858 putative Glycoside Hydrolase Family 3 | 31 |
| 4044 | BLP03870 hypothetical protein | 31 |
| 4118 | BLP03943 hypothetical protein | 26 |
| 4156 | sacB levansucrase, Glycoside Hydrolase Family 68 | 30 |

(continued)

| SEQ ID | Gene Name & Product | Predicted Signal Peptide Length |
|---|---|---|
| 4157 | IevB endolevanase, Glycoside Hydrolase Family 32 | 33 |
| 4193 | BLP04013 putative ribonuclease | 29 |
| 4213 | yvpB putative cysteine protease YvpB | 39 |
| 4214 | pelC Polysaccharide Lyase Family 3, putative pectate lyase | 29 |
| 4223 | yvnB conserved hypothetical protein YvnB | 29 |
| 4237 | ctpB Peptidase S41A, C-terminal protease | 37 |
| 4240 | BLP04061 conserved hypothetical protein, putative peptidase | 29 |
| 4244 | cccB putative cytochrome c551 heme binding protein | 27 |
| 4283 | lytb modifier protein of major autolysin LytC (CWBP76) | 26 |
| 4297 | lytD N-acetylglucosaminidase Glycoside Hydrolase Family 73 | 29 |
| 4312 | pgdS gamma-DL-glutamyl hydrolase | 34 |
| 4396 | ywmD conserved hypothetical protein containing VWFA domain YwmD | 25 |
| 4397 | ywmC conserved hypothetical protein containing VWFA domain YwmC | 24 |
| 4401 | ywmB conserved protein YwmB | 30 |
| 4468 | BLP04282 hypothetical protein | 25 |
| 4566 | ywaD Putative aminopeptidase | 31 |
| 4619 | lanP intracellular serine protease | 25 |
| 4625 | pelII pectate lyase, Polysaccharide Lyase Family 1 | 28 |
| 4638 | ansA L-asparaginase | 29 |
| 4646 | BLP04457 conserved hypothetical protein | 25 |
| 4647 | BLP04458 hypothetical protein | 30 |
| 4654 | BLP04465 putative Glycerophosphoryl diester phosphodiesterase | 30 |
| 4655 | yhjA conserved hypothetical protein | 28 |
| 4670 | BLP04481 hypothetical protein | 73 |
| 4678 | BLP04489 putative extracellular solute-binding protein | 29 |
| 4681 | BLP04492 hypothetical protein | 34 |
| 4689 | BLP04500 hypothetical protein | 30 |
| 4721 | yxiA Glycoside hydrolase, family 43 YxiA | 29 |
| 4734 | ydaJ putative Glycoside transferase | 29 |
| 4777 | yxeA conserved hypothetical protein | 34 |
| 4781 | yvfO Glyccosyl Hydrolase Family 53 | 28 |
| 4785 | cycB putative extracellular solute-binding protein, family 1 CycB | 32 |
| 4802 | BLP04613 hypothetical protein | 27 |
| 4810 | BLP04621 hypothetical protein | 34 |
| 4813 | BLP04836 putative bacteriocin | 42 |

**Table 5. Codon usage table for the *Bacillus licheniformis* SJ1904 chromosome assembled using Artemis software (K. Rutherford, J. Parkhill, J. Crook, T. Horsnell, P. Rice, M-A. Rajandream and B. Barrell. 2000. Artemis: sequence visualisation and annotation. *Bioinformatics* 16: 944-945). The numbers in parentheses denote the total occurrences of a particular codon among all CDSs, and the number to the right of each codon reflects its frequency per 1000 codons**

| | | | |
|---|---|---|---|
| ttt 29.8 (37864) | tct 9.1 (11536) | tat 20.5 (26064) | tgt 2.9 (3758) |
| ttc 16.3 (20684) | tcc 9.3 (11879) | tac 13.3 (16932) | tgc 5.4 (6918) |
| tta 14.4 (18259) | tca 12.5 (15847) | taa 0.2 (368) | tga 0.5 (642) |
| ttg 17.9 (22781) | tcg 10.3 (13048) | tag 0.1 (172) | tgg 10.3 (13070) |
| ctt 22.3 (28263) | cct 9.4 (11912) | cat 13.5 (17194) | cgt 5.1 (6472) |
| ctc 14.7 (18656) | ccc 4.5 (5810) | cac 8.4 (10668) | cgc 11.1 (14112) |
| cta 3.3 (4238) | cca 4.3 (5535) | caa 16.8 (21289) | cga 3.5 (4528) |
| ctg 25.0 (31731) | ccg 18.9 (24037) | cag 18.9 (24027) | cgg 8.6 (10955) |
| att 29.5 (37460) | act 6.1 (7825) | aat 19.3 (24529) | agt 4.0 (5161) |
| atc 34.0 (43124) | acc 9.6 (12284) | aac 19.2 (24386) | agc 15.8 (20074) |
| ata 7.7 (9850) | aca 17.0 (21587) | aaa 49.9 (63316) | aga 10.1 (12863) |
| atg 26.5 (33584) | acg 18.9 (23979) | aag 21.9 (27854) | agg 6.7 (8550) |
| gtt 17.4 (22162) | gct 16.0 (20317) | gat 29.1 (36898) | ggt 10.0 (12684) |
| gtc 23.6 (30011) | gcc 21.1 (26730) | gac 21.7 (27552) | ggc 26.1 (33149) |
| gta 9.6 (12286) | gca 18.3 (23209) | gaa 51.5 (65281) | gga 22.3 (28354) |
| gtg 15.7 (19894) | gcg 23.4 (29646) | gag 21.6 (27398) | ggg 12.1 (15429) |

**Sequence Listings**

Incorporated herein by reference are 2 copies of the Sequence Listing on compact disk. Copy 1 is done on a Intel x86 machine format, in Windows XP operating system compatibility, there is one file saved as 01 Seq List 29-NOV-2006.txt, and is 20,404 KB, and was created on November 16, 2006. Copy 2 is identical to Copy 1. The content of the attached compact disks are the same and includes no new matter.

**Deposit of Biological Material**

The following biological material has been deposited under the terms of the Budapest Treaty with the American Type Culture Collection, 10801 University Blvd. Manassas, Virginia 20110-2209 USA, and given the following accession number:

| Deposit | Accession Number | Date of Deposit |
|---|---|---|
| *B. licheniformis* SJ1904 DNA | ATCC PTA-7992 | November 8, 2006 |

The strain has been deposited under conditions that assure that access to the culture will be available during the pendency of this patent application to one determined by the Commissioner of Patents and Trademarks to be entitled thereto under 37 C.F.R. §1.14 and 35 U.S.C. §122. The deposit represents a substantially pure culture of the deposited strain. The deposit is available as required by foreign patent laws in countries wherein counterparts of the subject application, or its progeny are filed. However, it should be understood that the availability of a deposit does not constitute a license to practice the subject invention in derogation of patent rights granted by governmental action.

The invention described and claimed herein is not to be limited in scope by the specific aspects herein disclosed, since these aspects are intended as illustrations of several aspects of the invention. Any equivalent aspects are intended to be within the scope of this invention. Indeed, various modifications of the invention in addition to those shown and described herein will become apparent to those skilled in the art from the foregoing description. Such modifications are also intended to fall within the scope of the appended claims. In the case of conflict, the present disclosure including definitions will control.

Various references are cited herein, the disclosures of which are incorporated by reference in their entireties.

**Preferred embodiments**

1. An isolated polynucleotide encoding a biologically active substance, selected from the group consisting of:

(a) a polynucleotide comprising a nucleotide sequence having preferably at least 60% identity, more preferably at least 65% identity, more preferably at least 70% identity, more preferably at least 75% identity, more preferably at least 80% identity, more preferably at least 85% identity, more preferably at least 90% identity, even more preferably at least 90% identity, most preferably at least 95% identity, and even most preferably at least 97% identity with a polynucleotide selected from the group consisting of SEQ ID NOs: 2-4876 and subsequences thereof encoding fragments having biological activity;
(b) a polynucleotide comprising a nucleotide sequence that hybridizes under preferably at least medium stringency conditions, more preferably at least medium-high stringency conditions, or most preferably at least high stringency conditions with a polynucleotide selected from the group consisting of SEQ ID NOs: 2-4876 and full-length complementary strands thereof;
(c) a polynucleotide encoding a biologically active substance comprising an amino acid sequence having preferably at least 60% identity, more preferably at least 65% identity, more preferably at least 70% identity, more preferably at least 75% identity, more preferably at least 80% identity, more preferably at least 85% identity, more preferably at least 90% identity, even more preferably at least 90% identity, most preferably at least 95% identity, and even most preferably at least 97% identity with an amino acid sequence selected from the group consisting of SEQ ID NOs: 4877-9751 and fragments thereof retaining biological activity; and
(d) a polynucleotide encoding an artificial variant comprising a substitution, deletion, and/or insertion of one or more amino acids of an amino acid sequence selected from the group consisting of SEQ ID NOs: 4877-9751 and fragments thereof retaining biological activity.

2. The isolated polynucleotide of claim 1, comprising a nucleotide sequence having preferably at least 60% identity, more preferably at least 65% identity, more preferably at least 70% identity, more preferably at least 75% identity, more preferably at least 80% identity, more preferably at least 85% identity, even more preferably at least 90% identity, most preferably at least 95% identity, and even most preferably at least 97% identity with a polynucleotide selected from the group consisting of SEQ ID NOs: 50, 55, 77, 82, 91, 98, 103, 110, 125, 169, 171, 172, 174, 176, 177, 178, 179, 180, 181, 182, 183, 185, 186, 187, 189, 192, 194, 195, 196, 198, 199, 201, 203, 204, 205, 206, 207, 208, 209, 210, 211, 212, 213, 214, 215, 216, 217, 218, 219, 220, 221, 222, 223, 225, 226, 227, 228, 229, 230, 240, 241, 242, 243, 244, 245, 246, 247, 248, 249, 250, 251, 252, 253, 254, 255, 256, 257, 258, 259, 260, 261, 262, 263, 264, 265, 266, 287, 306, 308, 322, 323, 329, 350, 354, 360, 363, 367, 381, 445, 469, 487, 488, 516, 523, 524, 537, 541, 575, 592, 607, 621, 627, 628, 666, 672, 688, 691, 702, 710, 719, 726, 729, 748, 751, 773, 774, 777, 791, 831, 833, 842, 847, 848, 849, 850, 851, 855, 857, 885, 886, 887, 901, 944, 982, 991, 994, 1018, 1032, 1034, 1058, 1078, 1088, 1098, 1108, 1112, 1128, 1148, 1153, 1154, 1158, 1160, 1164, 1175, 1176, 1177, 1180, 1181, 1183, 1184, 1193, 1194, 1199, 1222, 1237, 1244, 1262, 1263, 1265, 1266, 1278, 1302, 1313, 1314, 1315, 1316, 1317, 1318, 1345, 1355, 1373, 1374, 1401, 1424, 1432, 1433, 1440, 1442, 1448, 1455, 1470, 1496, 1497, 1501, 1509, 1527, 1539, 1556, 1565, 1589, 1619, 1629, 1630, 1634, 1635, 1636, 1638, 1639, 1640, 1641, 1642, 1643, 1644, 1645, 1646, 1647, 1649, 1650, 1651, 1652, 1653, 1654, 1655, 1657, 1658, 1659, 1660, 1661, 1662, 1663, 1664, 1665, 1666, 1667, 1668, 1669, 1670, 1671, 1672, 1673, 1675, 1676, 1677, 1679, 1684, 1688, 1692, 1694, 1703, 1704, 1728, 1732, 1734, 1736, 1775, 1783, 1816, 1831, 1838, 1851, 1853, 1866, 1887, 2002, 2007, 2022, 2038, 2044, 2067, 2070, 2072, 2076, 2094, 2106, 2119, 2120, 2121, 2124, 2131, 2139, 2147, 2148, 2166, 2171, 2194, 2196, 2198, 2205, 2211, 2213, 2216, 2221, 2230, 2236, 2240, 2241, 2255, 2258, 2263, 2273, 2277, 2286, 2288, 2289, 2290, 2291, 2292, 2293, 2294, 2295, 2296, 2297, 2298, 2299, 2300, 2301, 2302, 2303, 2304, 2305, 2324, 2327, 2337, 2341, 2347, 2351, 2364, 2381, 2388, 2400, 2403, 2408, 2410, 2411, 2414, 2415, 2424, 2425, 2430, 2435, 2439, 2457, 2467, 2468, 2469, 2483, 2491, 2498, 2504, 2505, 2506, 2507, 2508, 2509, 2510, 2511, 2512, 2514, 2515, 2516, 2517, 2518, 2519, 2520, 2521, 2522, 2524, 2525, 2526, 2527, 2528, 2529, 2530, 2531, 2532, 2533, 2534, 2535, 2536, 2537, 2538, 2539, 2540, 2541, 2542, 2543, 2544, 2545, 2546, 2547, 2548, 2549, 2550, 2551, 2552, 2553, 2554, 2555, 2556, 2557, 2558, 2559, 2560, 2561, 2562, 2564, 2565, 2566, 2567, 2568, 2569, 2570, 2571, 2572, 2573, 2574, 2575, 2576, 2577, 2578, 2579, 2580, 2581, 2582, 2583, 2584, 2585, 2586, 2587, 2588, 2590, 2591, 2592, 2593, 2594, 2595, 2596, 2597, 2598, 2599, 2600, 2601, 2602, 2603, 2604, 2605, 2606, 2607, 2608, 2609, 2610, 2611, 2612, 2613, 2614, 2615, 2616, 2617, 2618, 2619, 2620, 2621, 2622, 2623, 2624, 2625, 2626, 2627, 2628, 2629, 2630, 2631, 2632, 2633, 2634, 2635, 2636, 2637, 2638, 2639, 2640, 2641, 2642, 2643, 2644, 2645, 2646, 2647, 2648, 2649, 2650, 2651, 2652, 2653, 2654, 2655, 2656, 2657, 2658, 2659, 2661, 2664, 2665, 2666, 2667, 2669, 2670, 2671, 2672, 2673, 2674, 2675, 2676, 2677, 2679, 2680, 2681, 2684, 2685, 2686, 2688, 2689, 2690, 2691, 2692, 2693, 2696, 2697, 2698, 2706, 2723, 2730, 2733, 2751, 2762, 2795, 2806, 2813, 2834, 2835, 2841, 2850, 2866, 2867, 2874, 2878, 2898, 2902, 2904, 2917, 2947, 2957, 2960, 2969, 2972, 2973, 2999, 3004, 3019, 3028, 3054, 3064, 3082, 3093, 3123, 3133, 3134, 3135, 3136, 3137, 3138, 3139, 3140, 3141, 3142, 3143, 3144, 3145, 3146, 3147, 3148, 3149, 3150, 3162, 3222, 3224, 3225, 3226, 3227, 3228, 3229, 3231, 3244, 3248, 3259, 3264, 3265, 3287, 3291, 3303, 3310, 3311, 3321, 3324, 3355, 3362, 3371, 3429, 3434, 3442, 3446, 3458, 3471, 3486, 3496, 3499, 3503, 3504, 3508, 3549, 3558, 3577, 3593, 3598, 3601, 3603, 3606, 3611, 3642, 3653, 3655, 3670, 3673, 3682, 3690, 3694, 3707, 3716, 3727, 3736, 3737, 3744, 3753, 3765, 3790, 3798, 3799, , 3872, 3881, 3885, 3915, 3916, 3922, 3931, 3938, 3969, 4011, 4017, 4056, 4059, 4068, 4076, 4077, 4081, 4082, 4085, 4090, 4106, 4112, 4117, 4144, 4145, 4158, 4159, 4160, 4161, 4162, 4163, 4164, 4165, 4166, 4167, 4168, 4169, 4170, 4171, 4172, 4173, 4174, 4175, 4176, 4179, 4181, 4182, 4201, 4235, 4285, 4288, 4305, 4323, 4333, 4336, 4353, 4398, 4409, 4417, 4421, 4423, 4429, 4435, 4448, 4450, 4469, 4472, 4476, 4482, 4488, 4513, 4518, 4544, 4559, 4560, 4569, 4571, 4578, 4607, 4622, 4631, 4643, 4647, 4648, 4650, 4658, 4669, 4672, 4682, 4687, 4688, 4704, 4718, 4754, 4767, 4786, 4792, 4797, 4804, 4812, 4814, 4823, 4830, 4833, 4838, 4848, 4849, 4859, 4862, and 4872; preferably at least 75% identity, more preferably at least 80% identity, more preferably at least 85% identity, even more preferably at least 90% identity, most preferably at least 95% identity, and even most preferably at least 97% identity with a polynucleotide selected from the group consisting of SEQ ID NOs: 909, 911, 1367, 1498, 2663, 2668, 2682, and 2683; preferably at least 80% identity, more preferably at least 85% identity, even more preferably at least 90% identity, most preferably at least 95% identity, and even most preferably at least 97% identity with a polynucleotide selected from the group consisting of SEQ ID NOs: 167, 654, 2513, 2589, and 2694; preferably at least 85% identity, more preferably at least 90% identity, most preferably at least 95% identity, and even most preferably at least 97% identity with a polynucleotide selected from the group consisting of SEQ ID NOs: 163, 184, 910, 1674, 2412, 2660, 2662, 2695, and 3231; preferably at least 90% identity, more preferably at least 95% identity, and most preferably at least 97% identity with a polynucleotide selected from the group consisting of SEQ ID NOs: 27, 166, 168, 170, 193, 202, 231, 543, 776, 888, 889, 890, 898, 908, 916, 1028, 1123, 1133, 1155, 1156, 1157, 1173, 1211, 1212, 1218, 1229, 1230, 1277, 1356, 1357, 1359, 1360, 1368, 1372, 1391, 1500, 1584, 1604, 1627, 1631, 1632, 1637, 1648, 1680, 2101, 2102, 2239, 2358, 2359, 2360, 2404, 2417, 2429, 2494, 2563, 2678, 2724, 3111, 3539, 3850, 4078, 4079, 4080, 4083, 4084, 4180, 4221, 4642, 4689, 4707, and 4727; or preferably at least 95% identity and more preferably at least 97% identity with a polynucleotide selected from the group consisting of SEQ ID NOs: 2, 3, 4, 5, 6, 7, 8, 9, 10, 11, 12, 13, 14, 15, 16, 17, 18, 19, 20, 21, 22, 23, 24, 25, 26, 28, 29, 30, 31, 32, 33, 34, 35, 36, 37, 38, 39, 40, 41, 42, 43, 44, 45, 46, 47, 48, 49, 51, 52, 53, 54, 56, 57, 58, 59, 60, 61, 62, 63, 64, 65, 66, 67, 68, 69, 70, 71, 72, 73, 74, 75, 76, 78, 79, 80, 81, 83, 84, 85, 86, 87, 88, 89, 90, 92, 93, 94, 95, 96, 97, 99, 100, 101, 102, 104, 105, 106, 107, 108, 109, 111, 112, 113, 114, 115, 116, 117, 118, 119, 120, 121, 122, 123, 124, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 164, 165, 173, 175, 188, 190, 191, 197, 200, 224, 232, 233, 234, 235, 236, 237, 238, 239, 267, 268, 269, 270, 271, 272, 273, 274, 275, 276, 277, 278, 279, 280, 281, 282, 283, 284, 285, 286, 288, 289, 290, 291, 292, 293, 294, 295, 296, 297, 298, 299, 300, 301, 302, 303, 304, 305, 307, 309, 310, 311, 312, 313, 314, 315, 316, 317, 318, 319, 320, 321, 324, 325, 326, 327, 328, 330, 331, 332, 333, 334, 335, 336, 337, 338, 339, 340, 341, 342, 343, 344, 345, 346, 347, 348, 349, 351, 352, 353, 355, 356, 357, 358, 359, 361, 362, 364, 365, 366, 368, 369, 370, 371, 372, 373, 374, 375, 376, 377, 378, 379, 380, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 446, 447, 448, 449, 450, 451, 452, 453, 454, 455, 456, 457, 458, 459, 460, 461, 462, 463, 464, 465, 466, 467, 468, 470, 471, 472, 473, 474, 475, 476, 477, 478, 479, 480, 481, 482, 483, 484, 485, 486, 489, 490, 491, 492, 493, 494, 495, 496, 497, 498, 499, 500, 501, 502, 503, 504, 505, 506, 507, 508, 509, 510, 511, 512, 513, 514, 515, 517, 518, 519, 520, 521, 522, 525, 526, 527, 528, 529, 530, 531, 532, 533, 534, 535, 536, 538, 539, 540, 542, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 576, 577, 578, 579, 580, 581, 582, 583, 584, 585, 586, 587, 588, 589, 590, 591, 593, 594, 595, 596, 597, 598, 599, 600, 601, 602, 603, 604, 605, 606, 608, 609, 610, 611, 612, 613, 614, 615, 616, 617, 618, 619, 620, 622, 623, 624, 625, 626, 629, 630, 631, 632, 633, 634, 635, 636, 637, 638, 639, 640, 641, 642, 643, 644, 645, 646, 647, 648, 649, 650, 651, 652, 653, 655, 656, 657, 658, 659, 660, 661, 662, 663, 664, 665, 667, 668, 669, 670, 671, 673, 674, 675, 676, 677, 678, 679, 680, 681, 682, 683, 684, 685, 686, 687, 689, 690, 692, 693, 694, 695, 696, 697, 698, 699, 700, 701, 703, 704, 705, 706, 707, 708, 709, 711, 712, 713, 714, 715, 716, 717, 718, 720, 721, 722, 723, 724, 725, 727, 728, 730, 731, 732, 733, 734, 735, 736, 737, 738, 739, 740, 741, 742, 743, 744, 745, 746, 747, 749, 750, 752, 753, 754, 755, 756, 757, 758, 759, 760, 761, 762, 763, 764, 765, 766, 767, 768, 769, 770, 771, 772, 775, 778, 779, 780, 781, 782, 783, 784, 785, 786, 787, 788, 789, 790, 792, 793, 794, 795, 796, 797, 798, 799, 800, 801, 802, 803, 804, 805, 806, 807, 808, 809, 810, 811, 812, 813, 814, 815, 816, 817, 818, 819, 820, 821, 822, 823, 824, 825, 826, 827, 828, 829, 830, 832, 834, 835, 836, 837, 838, 839, 840, 841, 843, 844, 845, 846, 852, 853, 854, 856, 858, 859, 860, 861, 862, 863, 864, 865, 866, 867, 868, 869, 870, 871, 872, 873, 874, 875, 876, 877, 878, 879, 880, 881, 882, 883, 884, 891, 892, 893, 894, 895, 896, 897, 899, 900, 902, 903, 904, 905, 906, 907, 912, 913, 914, 915, 917, 918, 919, 920, 921, 922, 923, 924, 925, 926, 927, 928, 929, 930, 931, 932, 933, 934, 935, 936, 937, 938, 939, 940, 941, 942, 943, 945, 946, 947, 948, 949, 950, 951, 952, 953, 954, 955, 956, 957, 958, 959, 960, 961, 962, 963, 964, 965, 966, 967, 968, 969, 970, 971, 972, 973, 974, 975, 976, 977, 978, 979, 980, 981, 983, 984, 985, 986, 987, 988, 989, 990, 992, 993, 995, 996, 997, 998, 999, 1000, 1001, 1002, 1003, 1004, 1005, 1006, 1007, 1008, 1009, 1010, 1011, 1012, 1013, 1014, 1015, 1016, 1017, 1019, 1020, 1021, 1022, 1023, 1024, 1025, 1026, 1027, 1029, 1030, 1031, 1033, 1035, 1036, 1037, 1038, 1039, 1040, 1041, 1042, 1043, 1044, 1045, 1046, 1047, 1048, 1049, 1050, 1051, 1052, 1053, 1054, 1055, 1056, 1057, 1059, 1060, 1061, 1062, 1063, 1064, 1065, 1066, 1067, 1068, 1069, 1070, 1071, 1072, 1073, 1074, 1075, 1076, 1077, 1079, 1080, 1081, 1082, 1083, 1084, 1085, 1086, 1087, 1089, 1090, 1091, 1092, 1093, 1094, 1095, 1096, 1097, 1099, 1100, 1101, 1102, 1103, 1104, 1105, 1106, 1107, 1109, 1110, 1111, 1113, 1114, 1115, 1116, 1117, 1118, 1119, 1120, 1121, 1122, 1124, 1125, 1126, 1127, 1129, 1130, 1131, 1132, 1134, 1135, 1136, 1137, 1138, 1139, 1140, 1141, 1142, 1143, 1144, 1145, 1146, 1147, 1149, 1150, 1151, 1152, 1159, 1161, 1162, 1163, 1165, 1166, 1167, 1168, 1169, 1170, 1171, 1172, 1174, 1178, 1179, 1182, 1185, 1186, 1187, 1188, 1189, 1190, 1191, 1192, 1195, 1196, 1197, 1198, 1200, 1201, 1202, 1203, 1204, 1205, 1206, 1207, 1208, 1209, 1210, 1213, 1214, 1215, 1216, 1217, 1219, 1220, 1221, 1223, 1224, 1225, 1226, 1227, 1228, 1231, 1232, 1233, 1234, 1235, 1236, 1238, 1239, 1240, 1241, 1242, 1243, 1245, 1246, 1247, 1248, 1249, 1250, 1251, 1252, 1253, 1254, 1255, 1256, 1257, 1258, 1259, 1260, 1261, 1264, 1267, 1268, 1269, 1270, 1271, 1272, 1273, 1274, 1275, 1276, 1279, 1280, 1281, 1282, 1283, 1284, 1285, 1286, 1287, 1288, 1289, 1290, 1291, 1292, 1293, 1294, 1295, 1296, 1297, 1298, 1299, 1300, 1301, 1303, 1304, 1305, 1306, 1307, 1308, 1309, 1310, 1311, 1312, 1319, 1320, 1321, 1322, 1323, 1324, 1325, 1326, 1327, 1328, 1329, 1330, 1331, 1332, 1333, 1334, 1335, 1336, 1337, 1338, 1339, 1340, 1341, 1342, 1343, 1344, 1346, 1347, 1348, 1349, 1350, 1351, 1352, 1353, 1354, 1358, 1361, 1362, 1363, 1364, 1365, 1366, 1369, 1370, 1371, 1375, 1376, 1377, 1378, 1379, 1380, 1381, 1382, 1383, 1384, 1385, 1386, 1387, 1388, 1389, 1390, 1392, 1393, 1394, 1395, 1396, 1397, 1398, 1399, 1400, 1402, 1403, 1404, 1405, 1406, 1407, 1408, 1409, 1410, 1411, 1412, 1413, 1414, 1415, 1416, 1417, 1418, 1419, 1420, 1421, 1422, 1423, 1425, 1426, 1427, 1428, 1429, 1430, 1431, 1434, 1435, 1436, 1437, 1438, 1439, 1441, 1443, 1444, 1445, 1446, 1447, 1449, 1450, 1451, 1452, 1453, 1454, 1456, 1457, 1458, 1459, 1460, 1461, 1462, 1463, 1464, 1465, 1466, 1467, 1468, 1469, 1471, 1472, 1473, 1474, 1475, 1476, 1477, 1478, 1479, 1480, 1481, 1482, 1483, 1484, 1485, 1486, 1487, 1488, 1489, 1490, 1491, 1492, 1493, 1494, 1495, 1499, 1502, 1503, 1504, 1505, 1506, 1507, 1508, 1510, 1511, 1512, 1513, 1514, 1515, 1516, 1517, 1518, 1519, 1520, 1521, 1522, 1523, 1524, 1525, 1526, 1528, 1529, 1530, 1531, 1532, 1533, 1534, 1535, 1536, 1537, 1538, 1540, 1541, 1542, 1543, 1544, 1545, 1546, 1547, 1548, 1549, 1550, 1551, 1552, 1553, 1554, 1555, 1557, 1558, 1559, 1560, 1561, 1562, 1563, 1564, 1566, 1567, 1568, 1569, 1570, 1571, 1572, 1573, 1574, 1575, 1576, 1577, 1578, 1579, 1580, 1581, 1582, 1583, 1585, 1586, 1587, 1588, 1590, 1591, 1592, 1593, 1594, 1595, 1596, 1597, 1598, 1599, 1600, 1601, 1602, 1603, 1605, 1606, 1607, 1608, 1609, 1610, 1611, 1612, 1613, 1614, 1615, 1616, 1617, 1618, 1620, 1621, 1622, 1623, 1624, 1625, 1626, 1628, 1633, 1656, 1678, 1681, 1682, 1683, 1685, 1686, 1687, 1689, 1690, 1691, 1693, 1695, 1696, 1697, 1698, 1699, 1700, 1701, 1702, 1705, 1706, 1707, 1708, 1709, 1710, 1711, 1712, 1713, 1714, 1715, 1716, 1717, 1718, 1719, 1720, 1721, 1722, 1723, 1724, 1725, 1726, 1727, 1729, 1730, 1731, 1733, 1735, 1737, 1738, 1739, 1740, 1741, 1742, 1743, 1744, 1745, 1746, 1747, 1748, 1749, 1750, 1751, 1752, 1753, 1754, 1755, 1756, 1757, 1758, 1759, 1760, 1761, 1762, 1763, 1764, 1765, 1766, 1767, 1768, 1769, 1770, 1771, 1772, 1773, 1774, 1776, 1777, 1778, 1779, 1780, 1781, 1782, 1784, 1785, 1786, 1787, 1788, 1789, 1790, 1791, 1792, 1793, 1794, 1795, 1796, 1797, 1798, 1799, 1800, 1801, 1802, 1803, 1804, 1805, 1806, 1807, 1808, 1809, 1810, 1811, 1812, 1813, 1814, 1815, 1817, 1818, 1819, 1820, 1821, 1822, 1823, 1824, 1825, 1826, 1827, 1828, 1829, 1830, 1832, 1833, 1834, 1835, 1836, 1837, 1839, 1840, 1841, 1842, 1843, 1844, 1845, 1846, 1847, 1848, 1849, 1850, 1852, 1854, 1855, 1856, 1857, 1858, 1859, 1860, 1861, 1862, 1863, 1864, 1865, 1867, 1868, 1869, 1870, 1871, 1872, 1873, 1874, 1875, 1876, 1877, 1878, 1879, 1880, 1881, 1882, 1883, 1884, 1885, 1886, 1888, 1889, 1890, 1891, 1892, 1893, 1894, 1895, 1896, 1897, 1898, 1899, 1900, 1901, 1902, 1903, 1904, 1905, 1906, 1907, 1908, 1909, 1910, 1911, 1912, 1913, 1914, 1915, 1916, 1917, 1918, 1919, 1920, 1921, 1922, 1923, 1924, 1925, 1926, 1927, 1928, 1929, 1930, 1931, 1932, 1933, 1934, 1935, 1936, 1937, 1938, 1939, 1940, 1941, 1942, 1943, 1944, 1945, 1946, 1947, 1948, 1949, 1950, 1951, 1952, 1953, 1954, 1955, 1956, 1957, 1958, 1959, 1960, 1961, 1962, 1963, 1964, 1965, 1966, 1967, 1968, 1969, 1970, 1971, 1972, 1973, 1974, 1975, 1976, 1977, 1978, 1979, 1980, 1981, 1982, 1983, 1984, 1985, 1986, 1987, 1988, 1989, 1990, 1991, 1992, 1993, 1994, 1995, 1996, 1997, 1998, 1999, 2000, 2001, 2003, 2004, 2005, 2006, 2008, 2009, 2010, 2011, 2012, 2013, 2014, 2015, 2016, 2017, 2018, 2019, 2020, 2021, 2023, 2024, 2025, 2026, 2027, 2028, 2029, 2030, 2031, 2032, 2033, 2034, 2035, 2036, 2037, 2039, 2040, 2041, 2042, 2043, 2045, 2046, 2047, 2048, 2049, 2050, 2051, 2052, 2053, 2054, 2055, 2056, 2057, 2058, 2059, 2060, 2061, 2062, 2063, 2064, 2065, 2066, 2068, 2069, 2071, 2073, 2074, 2075, 2077, 2078, 2079, 2080, 2081, 2082, 2083, 2084, 2085, 2086, 2087, 2088, 2089, 2090, 2091, 2092, 2093, 2095, 2096, 2097, 2098, 2099, 2100, 2103, 2104, 2105, 2107, 2108, 2109, 2110, 2111, 2112, 2113, 2114, 2115, 2116, 2117, 2118, 2122, 2123, 2125, 2126, 2127, 2128, 2129, 2130, 2132, 2133, 2134, 2135, 2136, 2137, 2138, 2140, 2141, 2142, 2143, 2144, 2145, 2146, 2149, 2150, 2151, 2152, 2153, 2154, 2155, 2156, 2157, 2158, 2159, 2160, 2161, 2162, 2163, 2164, 2165, 2167, 2168, 2169, 2170, 2172, 2173, 2174, 2175, 2176, 2177, 2178, 2179, 2180, 2181, 2182, 2183, 2184, 2185, 2186, 2187, 2188, 2189, 2190, 2191, 2192, 2193, 2195, 2197, 2199, 2200, 2201, 2202, 2203, 2204, 2206, 2207, 2208, 2209, 2210, 2212, 2214, 2215, 2217, 2218, 2219, 2220, 2222, 2223, 2224, 2225, 2226, 2227, 2228, 2229, 2231, 2232, 2233, 2234, 2235, 2237, 2238, 2242, 2243, 2244, 2245, 2246, 2247, 2248, 2249, 2250, 2251, 2252, 2253, 2254, 2256, 2257, 2259, 2260, 2261, 2262, 2264, 2265, 2266, 2267, 2268, 2269, 2270, 2271, 2272, 2274, 2275, 2276, 2278, 2279, 2280, 2281, 2282, 2283, 2284, 2285, 2287, 2306, 2307, 2308, 2309, 2310, 2311, 2312, 2313, 2314, 3231, 2316, 2317, 2318, 2319, 2320, 2321, 2322, 2323, 2325, 2326, 2328, 2329, 2330, 2331, 2332, 2333, 2334, 2335, 2336, 2338, 2339, 2340, 2342, 2343, 2344, 2345, 2346, 2348, 2349, 2350, 2352, 2353, 2354, 2355, 2356, 2357, 2361, 2362, 2363, 2365, 2366, 2367, 2368, 2369, 2370, 2371, 2372, 2373, 2374, 2375, 2376, 2377, 2378, 2379, 2380, 2382, 2383, 2384, 2385, 2386, 2387, 2389, 2390, 2391, 2392, 2393, 2394, 2395, 2396, 2397, 2398, 2399, 2401, 2402, 2405, 2406, 2407, 2409, 2413, 2416, 2418, 2419, 2420, 2421, 2422, 2423, 2426, 2427, 2428, 2431, 2432, 2433, 2434, 2436, 2437, 2438, 2440, 2441, 2442, 2443, 2444, 2445, 2446, 2447, 2448, 2449, 2450, 2451, 2452, 2453, 2454, 2455, 2456, 2458, 2459, 2460, 2461, 2462, 2463, 2464, 2465, 2466, 2470, 2471, 2472, 2473, 2474, 2475, 2476, 2477, 2478, 2479, 2480, 2481, 2482, 2484, 2485, 2486, 2487, 2488, 2489, 2490, 2492, 2493, 2495, 2496, 2497, 2499, 2500, 2501, 2502, 2503, 2523, 2687, 3231, 2700, 2701, 2702, 2703, 2704, 2705, 2707, 2708, 2709, 2710, 2711, 2712, 2713, 2714, 2715, 2716, 2717, 2718, 2719, 2720, 2721, 2722, 2725, 2726, 2727, 2728, 2729, 2731, 2732, 2734, 2735, 2736, 2737, 2738, 2739, 2740, 2741, 2742, 2743, 2744, 2745, 2746, 2747, 2748, 2749, 2750, 2752, 2753, 2754, 2755, 2756, 2757, 2758, 2759, 2760, 2761, 2763, 2764, 2765, 2766, 2767, 2768, 2769, 2770, 2771, 2772, 2773, 2774, 2775, 2776, 2777, 2778, 2779, 2780, 2781, 2782, 2783, 2784, 2785, 2786, 2787, 2788, 2789, 2790, 2791, 2792, 2793, 2794, 2796, 2797, 2798, 2799, 2800, 2801, 2802, 2803, 2804, 2805, 2807, 2808, 2809, 2810, 2811, 2812, 2814, 2815, 2816, 2817, 2818, 2819, 2820, 2821, 2822, 2823, 2824, 2825, 2826, 2827, 2828, 2829, 2830, 2831, 2832, 2833, 2836, 2837, 2838, 2839, 2840, 2842, 2843, 2844, 2845, 2846, 2847, 2848, 2849, 2851, 2852, 2853, 2854, 2855, 2856, 2857, 2858, 2859, 2860, 2861, 2862, 2863, 2864, 2865, 2868, 2869, 2870, 2871, 2872, 2873, 2875, 2876, 2877, 2879, 2880, 2881, 2882, 2883, 2884, 2885, 2886, 2887, 2888, 2889, 2890, 2891, 2892, 2893, 2894, 2895, 2896, 2897, 2899, 2900, 2901, 2903, 2905, 2906, 2907, 2908, 2909, 2910, 2911, 2912, 2913, 2914, 2915, 2916, 2918, 2919, 2920, 2921, 2922, 2923, 2924, 2925, 2926, 2927, 2928, 2929, 2930, 2931, 2932, 2933, 2934, 2935, 2936, 2937, 2938, 2939, 2940, 2941, 2942, 2943, 2944, 2945, 2946, 2948, 2949, 2950, 2951, 2952, 2953, 2954, 2955, 2956, 2958, 2959, 2961, 2962, 2963, 2964, 2965, 2966, 2967, 2968, 2970, 2971, 2974, 2975, 2976, 2977, 2978, 2979, 2980, 2981, 2982, 2983, 2984, 2985, 2986, 2987, 2988, 2989, 2990, 2991, 2992, 2993, 2994, 2995, 2996, 2997, 2998, 3000, 3001, 3002, 3003, 3005, 3006, 3007, 3008, 3009, 3010, 3011, 3012, 3013, 3014, 3015, 3016, 3017, 3018, 3020, 3021, 3022, 3023, 3024, 3025, 3026, 3027, 3029, 3030, 3031, 3032, 3033, 3034, 3035, 3036, 3037, 3038, 3039, 3040, 3041, 3042, 3043, 3044, 3045, 3046, 3047, 3048, 3049, 3050, 3051, 3052, 3053, 3055, 3056, 3057, 3058, 3059, 3060, 3061, 3062, 3063, 3065, 3066, 3067, 3068, 3069, 3070, 3071, 3072, 3073, 3074, 3075, 3076, 3077, 3078, 3079, 3080, 3081, 3083, 3084, 3085, 3086, 3087, 3088, 3089, 3090, 3091, 3092, 3094, 3095, 3096, 3097, 3098, 3099, 3100, 3101, 3102, 3103, 3104, 3105, 3106, 3107, 3108, 3109, 3110, 3112, 3113, 3114, 3115, 3116, 3117, 3118, 3119, 3120, 3121, 3122, 3124, 3125, 3126, 3127, 3128, 3129, 3130, 3131, 3132, 3151, 3152, 3153, 3154, 3155, 3156, 3157, 3158, 3159, 3160, 3161, 3163, 3164, 3165, 3166, 3167, 3168, 3169, 3170, 3171, 3172, 3173, 3174, 3175, 3176, 3177, 3178, 3179, 3180, 3181, 3182, 3183, 3184, 3185, 3186, 3187, 3188, 3189, 3190, 3191, 3192, 3193, 3194, 3195, 3196, 3197, 3198, 3199, 3200, 3201, 3202, 3203, 3204, 3205, 3206, 3207, 3208, 3209, 3210, 3211, 3212, 3213, 3214, 3215, 3216, 3217, 3218, 3219, 3220, 3221, 3223, 3232, 3233, 3234, 3235, 3236, 3237, 3238, 3239, 3240, 3241, 3242, 3243, 3245, 3246, 3247, 3249, 3250, 3251, 3252, 3253, 3254, 3255, 3256, 3257, 3258, 3260, 3261, 3262, 3263, 3266, 3267, 3268, 3269, 3270, 3271, 3272, 3273, 3274, 3275, 3276, 3277, 3278, 3279, 3280, 3281, 3282, 3283, 3284, 3285, 3286, 3288, 3289, 3290, 3292, 3293, 3294, 3295, 3296, 3297, 3298, 3299, 3300, 3301, 3302, 3304, 3305, 3306, 3307, 3308, 3309, 3312, 3313, 3314, 3315, 3316, 3317, 3318, 3319, 3320, 3322, 3323, 3325, 3326, 3327, 3328, 3329, 3330, 3331, 3332, 3333, 3334, 3335, 3336, 3337, 3338, 3339, 3340, 3341, 3342, 3343, 3344, 3345, 3346, 3347, 3348, 3349, 3350, 3351, 3352, 3353, 3354, 3356, 3357, 3358, 3359, 3360, 3361, 3363, 3364, 3365, 3366, 3367, 3368, 3369, 3370, 3372, 3373, 3374, 3375, 3376, 3377, 3378, 3379, 3380, 3381, 3382, 3383, 3384, 3385, 3386, 3387, 3388, 3389, 3390, 3391, 3392, 3393, 3394, 3395, 3396, 3397, 3398, 3399, 3400, 3401, 3402, 3403, 3404, 3405, 3406, 3407, 3408, 3409, 3410, 3411, 3412, 3413, 3414, 3415, 3416, 3417, 3418, 3419, 3420, 3421, 3422, 3423, 3424, 3425, 3426, 3427, 3428, 3430, 3431, 3432, 3433, 3435, 3436, 3437, 3438, 3439, 3440, 3441, 3443, 3444, 3445, 3447, 3448, 3449, 3450, 3451, 3452, 3453, 3454, 3455, 3456, 3457, 3459, 3460, 3461, 3462, 3463, 3464, 3465, 3466, 3467, 3468, 3469, 3470, 3472, 3473, 3474, 3475, 3476, 3477, 3478, 3479, 3480, 3481, 3482, 3483, 3484, 3485, 3487, 3488, 3489, 3490, 3491, 3492, 3493, 3494, 3495, 3497, 3498, 3500, 3501, 3502, 3505, 3506, 3507, 3509, 3510, 3511, 3512, 3513, 3514, 3515, 3516, 3517, 3518, 3519, 3520, 3521, 3522, 3523, 3524, 3525, 3526, 3527, 3528, 3529, 3530, 3531, 3532, 3533, 3534, 3535, 3536, 3537, 3538, 3540, 3541, 3542, 3543, 3544, 3545, 3546, 3547, 3548, 3550, 3551, 3552, 3553, 3554, 3555, 3556, 3557, 3559, 3560, 3561, 3562, 3563, 3564, 3565, 3566, 3567, 3568, 3569, 3570, 3571, 3572, 3573, 3574, 3575, 3576, 3578, 3579, 3580, 3581, 3582, 3583, 3584, 3585, 3586, 3587, 3588, 3589, 3590, 3591, 3592, 3594, 3595, 3596, 3597, 3599, 3600, 3602, 3604, 3605, 3607, 3608, 3609, 3610, 3612, 3613, 3614, 3615, 3616, 3617, 3618, 3619, 3620, 3621, 3622, 3623, 3624, 3625, 3626, 3627, 3628, 3629, 3630, 3631, 3632, 3633, 3634, 3635, 3636, 3637, 3638, 3639, 3640, 3641, 3643, 3644, 3645, 3646, 3647, 3648, 3649, 3650, 3651, 3652, 3654, 3656, 3657, 3658, 3659, 3660, 3661, 3662, 3663, 3664, 3665, 3666, 3667, 3668, 3669, 3671, 3672, 3674, 3675, 3676, 3677, 3678, 3679, 3680, 3681, 3683, 3684, 3685, 3686, 3687, 3688, 3689, 3691, 3692, 3693, 3695, 3696, 3697, 3698, 3699, 3700, 3701, 3702, 3703, 3704, 3705, 3706, 3708, 3709, 3710, 3711, 3712, 3713, 3714, 3715, 3717, 3718, 3719, 3720, 3721, 3722, 3723, 3724, 3725, 3726, 3728, 3729, 3730, 3731, 3732, 3733, 3734, 3735, 3738, 3739, 3740, 3741, 3742, 3743, 3745, 3746, 3747, 3748, 3749, 3750, 3751, 3752, 3754, 3755, 3756, 3757, 3758, 3759, 3760, 3761, 3762, 3763, 3764, 3766, 3767, 3768, 3769, 3770, 3771, 3772, 3773, 3774, 3775, 3776, 3777, 3778, 3779, 3780, 3781, 3782, 3783, 3784, 3785, 3786, 3787, 3788, 3789, 4463, 3792, 3793, 3794, 3795, 3796, 3797, 3800, 3801, 3802, 3803, 3804, 3805, 3806, 3807, 3808, 3809, 3810, 3811, 3812, 3813, 3814, 3815, 3816, 3817, 3818, 3819, 3820, 3821, 3822, 3823, 3824, 3825, 3826, 3827, 3828, 3829, 3830, 3831, 3832, 3833, 3834, 3835, 3837, 3838, 3839, 3840, 3841, 3842, 3843, 3844, 3845, 3846, 3847, 3848, 3849, 3851, 3852, 3853, 3854, 3855, 3856, 3857, 3858, 3859, 3860, 3861, 3862, 3863, 3864, 3865, 3866, 3867, 3868, 3869, 3870, 3871, 3873, 3874, 3875, 3876, 3877, 3878, 3879, 3880, 3882, 3883, 3884, 3886, 3887, 3888, 3889, 3890, 3891, 3892, 3893, 3894, 3895, 3896, 3897, 3898, 3899, 3900, 3901, 3902, 3903, 3904, 3905, 3906, 3907, 3908, 3909, 3910, 3911, 3912, 3913, 3914, 3917, 3918, 3919, 3920, 3921, 3923, 3924, 3925, 3926, 3927, 3928, 3929, 3930, 3932, 3933, 3934, 3935, 3936, 3937, 3939, 3940, 3941, 3942, 3943, 3944, 3945, 3946, 3947, 3948, 3949, 3950, 3951, 3952, 3953, 3954, 3955, 3956, 3957, 3958, 3959, 3960, 3961, 3962, 3963, 3964, 3965, 3966, 3967, 3968, 3970, 3971, 3972, 3973, 3974, 3975, 3976, 3977, 3978, 3979, 3980, 3981, 3982, 3983, 3984, 3985, 3986, 3987, 3988, 3989, 3990, 3991, 3992, 3993, 3994, 3995, 3996, 3997, 3998, 3999, 4000, 4001, 4002, 4003, 4004, 4005, 4006, 4007, 4008, 4009, 4010, 4012, 4013, 4014, 4015, 4016, 4018, 4019, 4020, 4021, 4022, 4023, 4024, 4025, 4026, 4027, 4028, 4029, 4030, 4031, 4032, 4033, 4034, 4035, 4036, 4037, 4038, 4039, 4040, 4041, 4042, 4043, 4044, 4045, 4046, 4047, 4048, 4049, 4050, 4051, 4052, 4053, 4054, 4055, 4057, 4058, 4060, 4061, 4062, 4063, 4064, 4065, 4066, 4067, 4069, 4070, 4071, 4072, 4073, 4074, 4075, 4086, 4087, 4088, 4089, 4091, 4092, 4093, 4094, 4095, 4096, 4097, 4098, 4099, 4100, 4101, 4102, 4103, 4104, 4105, 4107, 4108, 4109, 4110, 4111, 4113, 4114, 4115, 4116, 4118, 4119, 4120, 4121, 4122, 4123, 4124, 4125, 4126, 4127, 4128, 4129, 4130, 4131, 4132, 4133, 4134, 4135, 4136, 4137, 4138, 4139, 4140, 4141, 4142, 4143, 4146, 4147, 4148, 4149, 4150, 4151, 4152, 4153, 4154, 4155, 4156, 4157, 4177, 4178, 4183, 4184, 4185, 4186, 4187, 4188, 4189, 4190, 4191, 4192, 4193, 4194, 4195, 4196, 4197, 4198, 4199, 4200, 4202, 4203, 4204, 4205, 4206, 4207, 4208, 4209, 4210, 4211, 4212, 4213, 4214, 4215, 4216, 4217, 4218, 4219, 4220, 4222, 4223, 4224, 4225, 4226, 4227, 4228, 4229, 4230, 4231, 4232, 4233, 4234, 4236, 4237, 4238, 4239, 4240, 4241, 4242, 4243, 4244, 4245, 4246, 4247, 4248, 4249, 4250, 4251, 4252, 4253, 4254, 4255, 4256, 4257, 4258, 4259, 4260, 4261, 4262, 4263, 4264, 4265, 4266, 4267, 4268, 4269, 4270, 4271, 4272, 4273, 4274, 4275, 4276, 4277, 4278, 4279, 4280, 4281, 4282, 4283, 4284, 4286, 4287, 4289, 4290, 4291, 4292, 4293, 4294, 4295, 4296, 4297, 4298, 4299, 4300, 4301, 4302, 4303, 4304, 4306, 4307, 4308, 4309, 4310, 4311, 4312, 4313, 4314, 4315, 4316, 4317, 4318, 4319, 4320, 4321, 4322, 4324, 4325, 4326, 4327, 4328, 4329, 4330, 4331, 4332, 4334, 4335, 4337, 4338, 4339, 4340, 4341, 4342, 4343, 4344, 4345, 4346, 4347, 4348, 4349, 4350, 4351, 4352, 4354, 4355, 4356, 4357, 4358, 4359, 4360, 4361, 4362, 4363, 4364, 4365, 4366, 4367, 4368, 4369, 4370, 4371, 4372, 4373, 4374, 4375, 4376, 4377, 4378, 4379, 4380, 4381, 4382, 4383, 4384, 4385, 4386, 4387, 4388, 4389, 4390, 4391, 4392, 4393, 4394, 4395, 4396, 4397, 4399, 4400, 4401, 4402, 4403, 4404, 4405, 4406, 4407, 4408, 4410, 4411, 4412, 4413, 4414, 4415, 4416, 4418, 4419, 4420, 4422, 4424, 4425, 4426, 4427, 4428, 4430, 4431, 4432, 4433, 4434, 4436, 4437, 4438, 4439, 4440, 4441, 4442, 4443, 4444, 4445, 4446, 4447, 4449, 4451, 4452, 4453, 4454, 4455, 4456, 4457, 4458, 4459, 4460, 4461, 4462, 4463, 4464, 4465, 4466, 4467, 4468, 4470, 4471, 4473, 4474, 4475, 4477, 4478, 4479, 4480, 4481, 4483, 4484, 4485, 4486, 4487, 4489, 4490, 4491, 4492, 4493, 4494, 4495, 4496, 4497, 4498, 4499, 4500, 4501, 4502, 4503, 4504, 4505, 4506, 4507, 4508, 4509, 4510, 4511, 4512, 4514, 4515, 4516, 4517, 4519, 4520, 4521, 4522, 4523, 4524, 4525, 4526, 4527, 4528, 4529, 4530, 4531, 4532, 4533, 4534, 4535, 4536, 4537, 4538, 4539, 4540, 4541, 4542, 4543, 4545, 4546, 4547, 4548, 4549, 4550, 4551, 4552, 4553, 4554, 4555, 4556, 4557, 4558, 4561, 4562, 4563, 4564, 4565, 4566, 4567, 4568, 4570, 4572, 4573, 4574, 4575, 4576, 4577, 4579, 4580, 4581, 4582, 4583, 4584, 4585, 4586, 4587, 4588, 4589, 4590, 4591, 4592, 4593, 4594, 4595, 4596, 4597, 4598, 4599, 4600, 4601, 4602, 4603, 4604, 4605, 4606, 4608, 4609, 4610, 4611, 4612, 4613, 4614, 4615, 4616, 4617, 4618, 4619, 4620, 4621, 4623, 4624, 4625, 4626, 4627, 4628, 4629, 4630, 4632, 4633, 4634, 4635, 4636, 4637, 4638, 4639, 4640, 4641, 4644, 4645, 4646, 4649, 4651, 4652, 4653, 4654, 4655, 4656, 4657, 4659, 4660, 4661, 4662, 4663, 4664, 4665, 4666, 4667, 4668, 4670, 4671, 4673, 4674, 4675, 4676, 4677, 4678, 4679, 4680, 4681, 4683, 4684, 4685, 4686, 4690, 4691, 4692, 4693, 4694, 4695, 4696, 4697, 4698, 4699, 4700, 4701, 4702, 4703, 4705, 4706, 4708, 4709, 4710, 4711, 4712, 4713, 4714, 4715, 4716, 4717, 4719, 4720, 4721, 4722, 4723, 4724, 4725, 4726, 4728, 4729, 4730, 4731, 4732, 4733, 4734, 4735, 4736, 4737, 4738, 4739, 4740, 4741, 4742, 4743, 4744, 4745, 4746, 4747, 4748, 4749, 4750, 4751, 4752, 4753, 4755, 4756, 4757, 4758, 4759, 4760, 4761, 4762, 4763, 4764, 4765, 4766, 4768, 4769, 4770, 4771, 4772, 4773, 4774, 4775, 4776, 4777, 4778, 4779, 4780, 4781, 4782, 4783, 4784, 4785, 4787, 4788, 4789, 4790, 4791, 4793, 4794, 4795, 4796, 4798, 4799, 4800, 4801, 4802, 4803, 4805, 4806, 4807, 4808, 4809, 4810, 4811, 4813, 4815, 4816, 4817, 4818, 4819, 4820, 4821, 4822, 4824, 4825, 4826, 4827, 4828, 4829, 4831, 4832, 4834, 4835, 4836, 4837, 4839, 4840, 4841, 4842, 4843, 4844, 4845, 4846, 4847, 4850, 4851, 4852, 4853, 4854, 4855, 4856, 4857, 4858, 4860, 4861, 4863, 4864, 4865, 4866, 4867, 4868, 4869, 4870, 4871, 4873, 4874, 4875, and 4876.

3. The isolated polynucleotide of claim 1, which comprises or consists of any of SEQ ID NOs: 2-4876.

4. The isolated polynucleotide of claim 1, comprising a nucleotide sequence that hybridizes under preferably at least medium stringency conditions, more preferably at least medium-high stringency conditions, or most preferably at least high stringency conditions with a polynucleotide selected from the group consisting of SEQ ID NOs: 2-4876 and full-length complementary strands thereof.

5. The isolated polynucleotide of claim 1, which encodes a biologically active substance comprising an amino acid sequence having preferably an amino acid sequence having a degree of sequence identity of preferably at least 60% identity, more preferably at least 65% identity, more preferably at least 70% identity, more preferably at least 75% identity, more preferably at least 80%, more preferably at least 85%, even more preferably at least 90%, most preferably at least 95%, and even most preferably at least 97% with an amino acid sequence selected from the group consisting of SEQ ID NOs: 4944, 4985, 5051, 5058, 5064, 5076, 5079, 5097, 5098, 5099, 5106, 5115, 5117, 5136, 5137, 5197, 5204, 5286, 5459, 5529, 5604, 5723, 5724, 5725, 5726, 5761, 5923, 6056, 6097, 6153, 6192, 6371, 6372, 6376, 6456, 6504, 6531, 6545, 6546, 6547, 6548, 6550, 6945, 6965, 7053, 7116, 7163, 7164, 7165, 7166, 7169, 7170, 7174, 7176, 7177, 7363, 7392, 7397, 7399, 7402, 7420, 7421, 7423, 7425, 7438, 7449, 7453, 7468, 7507, 7562, 7563, 7565, 7573, 7641, 7815, 8003, 8008, 8009, 8010, 8011, 8014, 8015, 8018, 8020, 8021, 8022, 8046, 8099, 8100, 8103, 8140, 8323, 8710, 8711, 8966, 9034, 9035, 9036, 9049, 9051, 9054, 9056, 9057, 9224, 9516, 9525, 9547, 9562, 9564, 4900, 4925, 4930, 4952, 4957, 4966, 4973, 4978, 5000, 5010, 5044, 5046, 5047, 5049, 5052, 5053, 5054, 5055, 5056, 5057, 5060, 5061, 5062, 5067, 5069, 5070, 5071, 5073, 5074, 5078, 5080, 5081, 5082, 5083, 5084, 5085, 5086, 5087, 5088, 5089, 5090, 5091, 5092, 5093, 5094, 5095, 5096, 5100, 5101, 5102, 5103, 5104, 5105, 5112, 5116, 5118, 5119, 5120, 5121, 5122, 5123, 5124, 5125, 5126, 5127, 5128, 5129, 5130, 5131, 5132, 5133, 5134, 5135, 5138, 5139, 5140, 5141, 5151, 5162, 5173, 5180, 5181, 5183, 5192, 5198, 5225, 5229, 5235, 5236, 5238, 5242, 5249, 5256, 5290, 5297, 5307, 5320, 5344, 5347, 5353, 5354, 5362, 5363, 5366, 5373, 5391, 5396, 5398, 5399, 5412, 5416, 5428, 5445, 5450, 5456, 5467, 5482, 5493, 5496, 5502, 5503, 5506, 5541, 5547, 5563, 5566, 5574, 5577, 5585, 5594, 5601, 5609, 5623, 5626, 5639, 5648, 5649, 5652, 5664, 5666, 5702, 5704, 5706, 5708, 5717, 5722, 5730, 5732, 5760, 5762, 5776, 5784, 5819, 5832, 5852, 5857, 5866, 5869, 5890, 5893, 5897, 5907, 5909, 5912, 5933, 5953, 5955, 5963, 5966, 5973, 5983, 5986, 5987, 5993, 6003, 6005, 6023, 6026, 6028, 6029, 6033, 6035, 6039, 6045, 6050, 6051, 6052, 6055, 6058, 6059, 6068, 6069, 6074, 6083, 6090, 6095, 6112, 6119, 6137, 6138, 6140, 6141, 6167, 6177, 6188, 6189, 6190, 6191, 6193, 6195, 6209, 6220, 6230, 6245, 6248, 6249, 6276, 6286, 6294, 6296, 6299, 6307, 6308, 6315, 6317, 6323, 6330, 6341, 6345, 6384, 6402, 6414, 6431, 6440, 6445, 6464, 6494, 6506, 6509, 6510, 6511, 6513, 6514, 6515, 6516, 6517, 6518, 6519, 6520, 6521, 6522, 6524, 6525, 6526, 6527, 6528, 6529, 6530, 6532, 6533, 6534, 6535, 6536, 6537, 6538, 6539, 6540, 6541, 6542, 6543, 6544, 6552, 6557, 6559, 6563, 6567, 6569, 6572, 6578, 6579, 6603, 6607, 6609, 6611, 6626, 6650, 6654, 6658, 6661, 6667, 6670, 6673, 6691, 6706, 6711, 6713, 6726, 6728, 6741, 6758, 6762, 6819, 6821, 6877, 6882, 6897, 6913, 6919, 6923, 6940, 6942, 6947, 6951, 6958, 6969, 6981, 6984, 6994, 6995, 6996, 6999, 7006, 7014, 7022, 7023, 7041, 7046, 7069, 7071, 7073, 7079, 7080, 7086, 7088, 7091, 7096, 7100, 7105, 7111, 7115, 7129, 7130, 7133, 7138, 7148, 7152, 7157, 7161, 7167, 7171, 7172, 7173, 7175, 7178, 7179, 7180, 7199, 7200, 7202, 7211, 7212, 7216, 7222, 7226, 7229, 7237, 7239, 7256, 7263, 7275, 7278, 7279, 7283, 7285, 7286, 7287, 7289, 7290, 7299, 7300, 7305, 7310, 7314, 7321, 7324, 7332, 7339, 7342, 7343, 7344, 7351, 7358, 7366, 7373, 7375, 7379, 7380, 7381, 7382, 7383, 7384, 7385, 7386, 7387, 7388, 7389, 7390, 7391, 7393, 7394, 7395, 7396, 7400, 7401, 7403, 7404, 7405, 7406, 7407, 7408, 7409, 7410, 7411, 7412, 7413, 7414, 7415, 7416, 7417, 7418, 7419, 7422, 7424, 7426, 7427, 7428, 7429, 7430, 7431, 7432, 7433, 7434, 7435, 7436, 7437, 7439, 7440, 7442, 7443, 7444, 7445, 7446, 7447, 7448, 7450, 7451, 7452, 7454, 7455, 7456, 7457, 7458, 7459, 7460, 7461, 7462, 7463, 7464, 7465, 7466, 7467, 7469, 7470, 7471, 7472, 7473, 7474, 7475, 7476, 7477, 7478, 7479, 7480, 7481, 7482, 7483, 7484, 7485, 7486, 7487, 7488, 7489, 7490, 7491, 7492, 7493, 7494, 7495, 7496, 7497, 7498, 7499, 7500, 7501, 7502, 7503, 7504, 7505, 7506, 7508, 7509, 7510, 7511, 7513, 7514, 7515, 7516, 7517, 7518, 7519, 7520, 7521, 7522, 7523, 7524, 7525, 7526, 7527, 7528, 7529, 7530, 7531, 7532, 7533, 7534, 7536, 7539, 7540, 7542, 7544, 7545, 7546, 7547, 7548, 7549, 7550, 7551, 7552, 7553, 7554, 7555, 7556, 7559, 7560, 7561, 7564, 7566, 7567, 7568, 7571, 7572, 7581, 7583, 7592, 7595, 7598, 7605, 7608, 7626, 7637, 7646, 7670, 7681, 7688, 7709, 7710, 7716, 7725, 7741, 7742, 7749, 7753, 7758, 7766, 7773, 7777, 7779, 7792, 7822, 7832, 7835, 7844, 7847, 7848, 7874, 7879, 7893, 7894, 7903, 7921, 7929, 7939, 7944, 7957, 7962, 7968, 7987, 7998, 8012, 8016, 8017, 8019, 8023, 8024, 8025, 8037, 8090, 8097, 8101, 8102, 8104, 8106, 8119, 8123, 8134, 8136, 8139, 8162, 8166, 8174, 8178, 8185, 8186, 8196, 8198, 8199, 8208, 8230, 8237, 8246, 8299, 8304, 8306, 8309, 8317, 8321, 8332, 8333, 8346, 8361, 8368, 8371, 8374, 8378, 8379, 8383, 8384, 8394, 8424, 8433, 8452, 8462, 8468, 8473, 8476, 8478, 8481, 8486, 8517, 8528, 8530, 8545, 8548, 8557, 8565, 8569, 8582, 8584, 8591, 8602, 8610, 8611, 8612, 8619, 8628, 8640, 8645, 8655, 8665, 8667, 8673, 8674, 8693, 8700, 8714, 8747, 8754, 8756, 8760, 8779, 8786, 8790, 8791, 8797, 8801, 8806, 8813, 8844, 8886, 8892, 8893, 8908, 8912, 8923, 8930, 8931, 8934, 8943, 8951, 8952, 8953, 8956, 8957, 8960, 8961, 8965, 8981, 8987, 8992, 9019, 9020, 9033, 9037, 9038, 9039, 9040, 9041, 9042, 9043, 9044, 9045, 9046, 9047, 9048, 9050, 9076, 9096, 9110, 9114, 9118, 9160, 9163, 9180, 9198, 9208, 9211, 9228, 9245, 9273, 9284, 9292, 9296, 9298, 9304, 9310, 9321, 9323, 9325, 9337, 9344, 9347, 9349, 9351, 9357, 9362, 9363, 9374, 9384, 9385, 9388, 9393, 9419, 9433, 9434, 9435, 9436, 9444, 9446, 9453, 9475, 9480, 9482, 9497, 9506, 9508, 9518, 9520, 9522, 9523, 9524, 9533, 9544, 9557, 9568, 9579, 9583, 9593, 9597, 9629, 9642, 9661, 9667, 9672, 9679, 9687, 9689, 9698, 9705, 9708, 9713, 9723, 9724, 9729, 9734, 9737, and 9747 ; preferably at least 65% identity, more preferably at least 70% identity, more preferably at least 75% identity, more preferably at least 80%, more preferably at least 85%, even more preferably at least 90%, most preferably at least 95%, and even most preferably at least 97% with an amino acid sequence selected from the group consisting of SEQ ID NOs: 4991, 5068, 5308, 5463, 5919, 6549, 6648, 6680, 6948, 7298, 7541, 7896, 7911, 8497, 8702, 8729, 8737, 9052, 9690, and 9709 ; preferably at least 70% identity, more preferably at least 75% identity, more preferably at least 80%, more preferably at least 85%, even more preferably at least 90%, most preferably at least 95%, and even most preferably at least 97% with an amino acid sequence selected from the group consisting of SEO ID NOs: 4986, 5041, 5043, 5418, 5427, 5500, 5515, 5696, 5713, 5785, 5939, 6106, 6109, 6171, 6260, 6503, 6677, 6724, 7054, 7260, 7295, 7316, 7341, 7512, 7538, 7591, 7628, 7735, 7912, 8050, 8076, 8206, 8207, 8240, 8327, 8406, 8428, 8860, 8955, 9061, 9099, 9115, 9262, 9591, 9641, and 9720; preferably at least 75% identity, more preferably at least 80%, more preferably at least 85%, even more preferably at least 90%, most preferably at least 95%, and even most preferably at least 97% with an amino acid sequence selected from the group consisting of SEQ ID NOs: 5038, 5059, 5240, 5403, 5444, 5508, 5559, 5562, 5642, 5651, 5846, 5885, 5921, 5942, 6015, 6067, 6071, 6164, 6215, 6293, 6373, 6390, 6446, 6624, 6704, 6717, 6749, 6759, 6808, 6847, 6848, 6852, 6854, 7292, 7297, 7308, 7317, 7537, 7543, 7557, 7603, 7651, 7858, 7949, 7954, 7996, 8074, 8200, 8221, 8263, 8288, 8614, 8684, 8720, 8728, 8740, 8749, 8787, 8864, 8958, 9055, 9176, 9196, 9283, 9308, 9352, 9491, 9512, 9602, 9653, 9684, 9719, and 9731; preferably at least 80% identity, more preferably at least 85% identity, even more preferably at least 90% identity, most preferably at least 95% identity, and even most preferably at least 97% identity with an amino acid sequence selected from the group consisting of SEQ ID NOs: 4891, 4956, 5042, 5072, 5169, 5190, 5195, 5217, 5241, 5266, 5305, 5369, 5374, 5377, 5409, 5435, 5512, 5524, 5538, 5595, 5613, 5647, 5657, 5665, 5667, 5735, 5743, 5830, 5878, 5900, 5903, 5977, 5988, 6000, 6060, 6066, 6070, 6073, 6075, 6105, 6120, 6139, 6152, 6174, 6291, 6335, 6375, 6437, 6466, 6492, 6505, 6507, 6523, 6553, 6592, 6651, 6694, 6730, 6732, 6774, 6843, 6929, 6943, 6964, 7093, 7132, 7144, 7224, 7349, 7374, 7398, 7570, 7578, 7632, 7635, 7643, 7680, 7682, 7685, 7700, 7713, 7727, 7800, 7843, 7864, 7918, 7947, 7982, 7989, 7991, 7999, 8055, 8057, 8085, 8095, 8170, 8182, 8191, 8210, 8255, 8261, 8328, 8365, 8399, 8410, 8479, 8551, 8561, 8577, 8589, 8649, 8679, 8742, 8744, 8784, 8789, 8796, 8818, 8832, 8835, 8845, 8854, 8858, 8868, 8878, 8900, 8950, 9007, 9102, 9108, 9151, 9169, 9207, 9241, 9246, 9248, 9250, 9299, 9341, 9343, 9412, 9443, 9470, 9476, 9511, 9528, 9559, 9565, 9577, 9584, 9594, 9595, 9682, and 9714; preferably at least 85% identity, more preferably at least 90% identity, most preferably at least 95% identity, and even most preferably at least 97% identity with an amino acid sequence selected from the group consisting of SEQ ID NOs: 4913, 4948, 4968, 5025, 5034, 5066, 5109, 5110, 5146, 5160, 5199, 5200, 5203, 5211, 5221, 5223, 5226, 5252, 5269, 5273, 5293, 5309, 5318, 5342, 5352, 5393, 5394, 5421, 5430, 5431, 5434, 5447, 5458, 5492, 5507, 5511, 5514, 5525, 5526, 5530, 5544, 5570, 5616, 5617, 5621, 5640, 5661, 5663, 5691, 5716, 5738, 5751, 5753, 5757, 5759, 5786, 5802, 5820, 5826, 5855, 5858, 5871, 5872, 5902, 5905, 5908, 5915, 5936, 5937, 5938, 5947, 6006, 6011, 6012, 6025, 6030, 6032, 6034, 6057, 6080, 6084, 6093, 6096, 6110, 6131, 6178, 6183, 6199, 6232, 6236, 6243, 6257, 6258, 6313, 6331, 6354, 6360, 6362, 6386, 6417, 6421, 6435, 6455, 6468, 6472, 6475, 6476, 6478, 6495, 6555, 6566, 6568, 6604, 6608, 6641, 6662, 6663, 6665, 6692, 6699, 6736, 6752, 6763, 6807, 6809, 6810, 6840, 6842, 6844, 6853, 6855, 6857, 6866, 6906, 6908, 6915, 6922, 6953, 6962, 6977, 6989, 7004, 7025, 7032, 7042, 7045, 7058, 7072, 7085, 7134, 7139, 7145, 7182, 7188, 7194, 7208, 7227, 7249, 7261, 7272, 7293, 7309, 7320, 7331, 7333, 7334, 7354, 7367, 7371, 7558, 7597, 7602, 7623, 7664, 7677, 7679, 7687, 7732, 7734, 7739, 7744, 7752, 7765, 7769, 7790, 7801, 7818, 7823, 7833, 7837, 7860, 7867, 7877, 7882, 7915, 7931, 7932, 7946, 7948, 7959, 7967, 7973, 7975, 7977, 7978, 7986, 8071, 8072, 8096, 8107, 8129, 8146, 8148, 8153, 8164, 8165, 8169, 8203, 8211, 8216, 8218, 8234, 8245, 8253, 8275, 8297, 8315, 8334, 8340, 8357, 8362, 8372, 8386, 8398, 8425, 8438, 8455, 8460, 8470, 8480, 8490, 8506, 8531, 8533, 8572, 8593, 8600, 8615, 8623, 8627, 8630, 8634, 8664, 8687, 8703, 8704, 8705, 8726, 8739, 8750, 8752, 8753, 8769, 8780, 8781, 8805, 8816, 8825, 8846, 8869, 8870, 8883, 8913, 8938, 8944, 8945, 8948, 8959, 8962, 8973, 8976, 8980, 8991, 9000, 9006, 9017, 9068, 9093, 9097, 9103, 9109, 9146, 9155, 9159, 9255, 9258, 9260, 9266, 9269, 9271, 9278, 9293, 9350, 9373, 9394, 9415, 9420, 9449, 9466, 9472, 9477, 9486, 9500, 9504, 9514, 9517, 9535, 9573, 9590, 9599, 9601, 9623, 9645, 9648, 9652, 9654, 9659, 9726, 9728, 9736, and 9748; preferably at least 90% identity, more preferably at least 95% identity, and most preferably at least 97% identity with an amino acid sequence selected from the group consisting of SEQ ID NOs: 4890, 4895, 4921, 4923, 4927, 4933, 4934, 4937, 4939, 4943, 4946, 4950, 4951, 4955, 4958, 4967, 4970, 4981, 4982, 4987, 4989, 4999, 5017, 5022, 5029, 5048, 5111, 5113, 5145, 5152, 5157, 5165, 5171, 5178, 5179, 5182, 5186, 5202, 5213, 5214, 5215, 5218, 5227, 5230, 5233, 5245, 5268, 5272, 5274, 5279, 5281, 5283, 5298, 5303, 5315, 5324, 5326, 5328, 5329, 5330, 5336, 5337, 5338, 5339, 5346, 5357, 5360, 5376, 5380, 5387, 5400, 5401, 5414, 5417, 5419, 5422, 5424, 5436, 5442, 5446, 5448, 5455, 5457, 5466, 5475, 5499, 5501, 5510, 5537, 5539, 5542, 5550, 5552, 5557, 5565, 5571, 5573, 5586, 5589, 5612, 5615, 5620, 5622, 5628, 5632, 5634, 5638, 5645, 5646, 5660, 5670, 5677, 5678, 5681, 5688, 5705, 5707, 5710, 5739, 5748, 5754, 5756, 5763, 5764, 5765, 5766, 5768, 5779, 5782, 5783, 5787, 5791, 5792, 5793, 5799, 5808, 5813, 5821, 5827, 5828, 5834, 5835, 5837, 5842, 5850, 5856, 5859, 5864, 5870, 5884, 5906, 5914, 5917, 5945, 5946, 5958, 5964, 5965, 5985, 5989, 5994, 5998, 6007, 6009, 6016, 6017, 6019, 6022, 6031, 6048, 6062, 6064, 6079, 6081, 6086, 6089, 6098, 6103, 6107, 6113, 6121, 6122, 6125, 6156, 6163, 6165, 6181, 6205, 6212, 6223, 6235, 6238, 6239, 6242, 6244, 6250, 6251, 6255, 6261, 6262, 6265, 6267, 6269, 6281, 6283, 6284, 6290, 6292, 6300, 6301, 6318, 6324, 6327, 6332, 6333, 6338, 6339, 6344, 6349, 6353, 6361, 6363, 6374, 6377, 6383, 6387, 6396, 6397, 6399, 6400, 6413, 6423, 6449, 6450, 6462, 6465, 6467, 6471, 6484, 6493, 6501, 6512, 6565, 6573, 6574, 6575, 6576, 6580, 6583, 6585, 6589, 6594, 6598, 6600, 6605, 6619, 6629, 6635, 6636, 6637, 6638, 6642, 6647, 6664, 6669, 6686, 6688, 6695, 6696, 6697, 6698, 6700, 6710, 6712, 6729, 6735, 6740, 6742, 6743, 6744, 6745, 6768, 6779, 6780, 6781, 6782, 6786, 6792, 6793, 6795, 6800, 6801, 6802, 6804, 6823, 6827, 6829, 6833, 6834, 6836, 6838, 6861, 6862, 6863, 6867, 6868, 6871, 6872, 6887, 6903, 6918, 6920, 6927, 6933, 6939, 6944, 6949, 6960, 6966, 6973, 6976, 6979, 7000, 7002, 7015, 7017, 7019, 7036, 7040, 7051, 7055, 7056, 7063, 7075, 7077, 7087, 7090, 7092, 7097, 7102, 7103, 7104, 7109, 7112, 7117, 7118, 7122, 7123, 7125, 7126, 7127, 7158, 7162, 7181, 7186, 7196, 7201, 7203, 7206, 7209, 7210, 7219, 7221, 7230, 7231, 7232, 7233, 7235, 7236, 7244, 7248, 7251, 7252, 7257, 7268, 7271, 7274, 7281, 7304, 7326, 7335, 7352, 7356, 7357, 7360, 7364, 7577, 7579, 7601, 7607, 7615, 7624, 7625, 7639, 7650, 7658, 7672, 7696, 7712, 7718, 7728, 7729, 7730, 7745, 7747, 7748, 7751, 7754, 7756, 7774, 7780, 7786, 7808, 7810, 7826, 7831, 7840, 7849, 7854, 7871, 7872, 7886, 7892, 7914, 7925, 7928, 7933, 7934, 7937, 7941, 7945, 7969, 7970, 7980, 8032, 8039, 8040, 8060, 8063, 8065, 8066, 8075, 8077, 8078, 8086, 8122, 8133, 8138, 8149, 8157, 8158, 8159, 8177, 8180, 8184, 8188, 8195, 8205, 8209, 8220, 8223, 8235, 8241, 8243, 8247, 8250, 8252, 8257, 8271, 8280, 8282, 8283, 8295, 8298, 8305, 8310, 8325, 8342, 8344, 8347, 8358, 8363, 8366, 8373, 8377, 8382, 8387, 8392, 8400, 8404, 8405, 8407, 8413, 8417, 8419, 8421, 8426, 8429, 8434, 8436, 8442, 8450, 8457, 8458, 8461, 8463, 8469, 8491, 8494, 8501, 8504, 8507, 8508, 8516, 8523, 8532, 8536, 8544, 8550, 8552, 8554, 8564, 8566, 8587, 8603, 8622, 8629, 8632, 8635, 8636, 8646, 8647, 8652, 8653, 8654, 8657, 8682, 8690, 8698, 8701, 8709, 8712, 8724, 8725, 8736, 8755, 8767, 8772, 8776, 8798, 8803, 8804, 8817, 8836, 8843, 8852, 8857, 8859, 8861, 8866, 8880, 8881, 8882, 8884, 8885, 8891, 8894, 8901, 8904, 8905, 8910, 8911, 8927, 8933, 8954, 8963, 8971, 8975, 8977, 8984, 8995, 8997, 9004, 9008, 9023, 9026, 9031, 9063, 9078, 9080, 9083, 9091, 9092, 9112, 9120, 9127, 9134, 9140, 9149, 9156, 9164, 9168, 9185, 9189, 9190, 9193, 9197, 9202, 9204, 9214, 9217, 9220, 9232, 9233, 9236, 9243, 9259, 9274, 9275, 9280, 9286, 9291, 9300, 9305, 9306, 9317, 9329, 9356, 9364, 9366, 9367, 9378, 9382, 9389, 9390, 9392, 9396, 9399, 9405, 9417, 9422, 9425, 9428, 9438, 9441, 9451, 9454, 9462, 9463, 9468, 9473, 9496, 9499, 9501, 9502, 9510, 9539, 9540, 9550, 9556, 9561, 9570, 9574, 9585, 9587, 9604, 9605, 9606, 9613, 9616, 9618, 9630, 9632, 9633, 9635, 9650, 9651, 9655, 9656, 9670, 9680, 9683, 9686, 9699, 9704, 9716, 9727, and 9749; or preferably at least 95% identity and more preferably at least 97% identity with an amino acid sequence selected from the group consisting of SEQ ID NOs: 4877, 4878, 4879, 4880, 4881, 4882, 4883, 4884, 4885, 4886, 4887, 4888, 4889, 4892, 4893, 4894, 4896, 4897, 4898, 4899, 4901, 4902, 4903, 4904, 4905, 4906, 4907, 4908, 4909, 4910, 4911, 4912, 4914, 4915, 4916, 4917, 4918, 4919, 4920, 4922, 4924, 4926, 4928, 4929, 4931, 4932, 4935, 4936, 4938, 4940, 4941, 4942, 4945, 4947, 4949, 4953, 4954, 4959, 4960, 4961, 4962, 4963, 4964, 4965, 4969, 4971, 4972, 4974, 4975, 4976, 4977, 4979, 4980, 4983, 4984, 4988, 4990, 4992, 4993, 4994, 4995, 4996, 4997, 4998, 5001, 5002, 5003, 5004, 5005, 5006, 5007, 5008, 5009, 5011, 5012, 5013, 5014, 5015, 5016, 5018, 5019, 5020, 5021, 5023, 5024, 5026, 5027, 5028, 5030, 5031, 5032, 5033, 5035, 5036, 5037, 5039, 5045, 5050, 5063, 5065, 5075, 5077, 5107, 5108, 5114, 5142, 5143, 5144, 5147, 5148, 5149, 5150, 5153, 5154, 5155, 5156, 5158, 5159, 5161, 5163, 5164, 5166, 5167, 5168, 5170, 5172, 5174, 5175, 5176, 5177, 5184, 5185, 5187, 5188, 5189, 5191, 5193, 5194, 5196, 5201, 5205, 5206, 5207, 5208, 5209, 5210, 5212, 5216, 5219, 5220, 5222, 5224, 5228, 5231, 5232, 5234, 5237, 5239, 5243, 5244, 5246, 5247, 5248, 5250, 5251, 5254, 5255, 5257, 5258, 5259, 5260, 5261, 5262, 5263, 5264, 5265, 5267, 5270, 5271, 5275, 5276, 5277, 5278, 5280, 5282, 5284, 5285, 5287, 5288, 5289, 5291, 5292, 5294, 5295, 5296, 5299, 5300, 5301, 5302, 5304, 5306, 5310, 5311, 5312, 5313, 5314, 5316, 5317, 5319, 5321, 5322, 5323, 5325, 5327, 5331, 5332, 5333, 5334, 5335, 5340, 5341, 5343, 5345, 5348, 5349, 5350, 5351, 5355, 5356, 5358, 5359, 5361, 5364, 5365, 5367, 5368, 5370, 5371, 5372, 5375, 5379, 5381, 5382, 5383, 5384, 5385, 5386, 5388, 5389, 5390, 5392, 5395, 5397, 5402, 5404, 5405, 5406, 5407, 5408, 5410, 5411, 5413, 5415, 5420, 5423, 5425, 5426, 5429, 5432, 5433, 5437, 5438, 5439, 5440, 5441, 5443, 5449, 5451, 5452, 5453, 5454, 5460, 5461, 5462, 5464, 5465, 5468, 5469, 5470, 5471, 5472, 5473, 5474, 5476, 5477, 5478, 5479, 5480, 5481, 5483, 5484, 5485, 5486, 5487, 5488, 5489, 5490, 5491, 5494, 5495, 5497, 5498, 5504, 5505, 5509, 5513, 5516, 5517, 5518, 5519, 5520, 5521, 5522, 5523, 5527, 5528, 5531, 5532, 5533, 5534, 5535, 5536, 5540, 5543, 5545, 5546, 5548, 5549, 5551, 5553, 5554, 5555, 5556, 5558, 5560, 5561, 5564, 5567, 5568, 5569, 5572, 5575, 5576, 5578, 5579, 5580, 5581, 5582, 5583, 5584, 5587, 5588, 5590, 5591, 5592, 5593, 5596, 5597, 5598, 5599, 5600, 5602, 5603, 5605, 5606, 5607, 5608, 5610, 5611, 5614, 5618, 5619, 5624, 5625, 5627, 5629, 5630, 5631, 5633, 5635, 5636, 5637, 5641, 5643, 5644, 5650, 5653, 5654, 5655, 5656, 5658, 5659, 5662, 5668, 5669, 5671, 5672, 5673, 5674, 5675, 5676, 5679, 5680, 5682, 5683, 5684, 5685, 5686, 5687, 5689, 5690, 5692, 5693, 5694, 5695, 5697, 5698, 5699, 5700, 5701, 5703, 5709, 5711, 5712, 5714, 5715, 5718, 5719, 5720, 5721, 5728, 5729, 5731, 5733, 5734, 5736, 5737, 5740, 5741, 5742, 5744, 5745, 5746, 5747, 5749, 5750, 5752, 5755, 5758, 5767, 5769, 5770, 5771, 5772, 5773, 5774, 5775, 5777, 5778, 5780, 5781, 5788, 5789, 5790, 5794, 5795, 5796, 5797, 5798, 5800, 5801, 5803, 5804, 5805, 5806, 5807, 5809, 5810, 5811, 5812, 5814, 5815, 5816, 5817, 5818, 5822, 5823, 5824, 5825, 5829, 5831, 5833, 5836, 5838, 5839, 5840, 5841, 5843, 5844, 5845, 5847, 5848, 5849, 5851, 5853, 5854, 5860, 5861, 5862, 5863, 5865, 5867, 5868, 5873, 5874, 5875, 5876, 5877, 5879, 5880, 5881, 5882, 5883, 5886, 5887, 5888, 5889, 5891, 5892, 5894, 5895, 5896, 5899, 5901, 5904, 5910, 5911, 5913, 5916, 5918, 5920, 5922, 5924, 5925, 5926, 5927, 5928, 5929, 5930, 5931, 5932, 5934, 5935, 5940, 5941, 5943, 5944, 5948, 5949, 5950, 5951, 5952, 5954, 5956, 5957, 5959, 5960, 5961, 5962, 5967, 5968, 5969, 5970, 5971, 5972, 5974, 5975, 5976, 5978, 5979, 5980, 5981, 5982, 5984, 5990, 5991, 5992, 5995, 5996, 5997, 5999, 6001, 6002, 6004, 6008, 6010, 6013, 6014, 6018, 6020, 6021, 6024, 6027, 6036, 6037, 6038, 6040, 6041, 6042, 6043, 6044, 6046, 6047, 6049, 6053, 6054, 6061, 6063, 6065, 6072, 6076, 6077, 6078, 6082, 6085, 6087, 6088, 6091, 6092, 6094, 6099, 6100, 6101, 6102, 6104, 6108, 6111, 6114, 6115, 6116, 6117, 6118, 6123, 6124, 6126, 6127, 6128, 6129, 6130, 6132, 6133, 6134, 6135, 6136, 6142, 6143, 6144, 6145, 6146, 6147, 6148, 6149, 6150, 6151, 6154, 6155, 6157, 6158, 6159, 6160, 6161, 6162, 6166, 6168, 6169, 6170, 6172, 6173, 6175, 6176, 6179, 6180, 6182, 6184, 6185, 6186, 6187, 6194, 6196, 6197, 6198, 6200, 6201, 6202, 6203, 6204, 6206, 6207, 6208, 6210, 6211, 6213, 6214, 6216, 6217, 6218, 6219, 6221, 6222, 6224, 6225, 6226, 6227, 6228, 6229, 6231, 6233, 6234, 6237, 6240, 6246, 6247, 6252, 6253, 6254, 6256, 6259, 6263, 6264, 6266, 6268, 6270, 6271, 6272, 6273, 6274, 6275, 6277, 6278, 6279, 6280, 6282, 6285, 6287, 6288, 6289, 6295, 6297, 6298, 6302, 6303, 6304, 6305, 6306, 6309, 6310, 6311, 6312, 6314, 6316, 6319, 6320, 6321, 6322, 6325, 6326, 6328, 6329, 6336, 6337, 6340, 6342, 6343, 6346, 6347, 6348, 6350, 6351, 6352, 6355, 6356, 6357, 6358, 6359, 6364, 6365, 6366, 6367, 6368, 6369, 6378, 6379, 6380, 6381, 6382, 6385, 6388, 6389, 6391, 6392, 6393, 6394, 6395, 6398, 6401, 6403, 6404, 6405, 6406, 6407, 6408, 6409, 6410, 6411, 6412, 6415, 6416, 6418, 6419, 6420, 6422, 6424, 6425, 6426, 6427, 6428, 6429, 6430, 6432, 6433, 6434, 6436, 6438, 6439, 6441, 6442, 6443, 6444, 6447, 6448, 6451, 6452, 6453, 6454, 6457, 6458, 6459, 6460, 6461, 6463, 6469, 6470, 6473, 6474, 6477, 6479, 6480, 6481, 6482, 6483, 6485, 6486, 6487, 6488, 6489, 6490, 6491, 6496, 6497, 6498, 6499, 6500, 6502, 6508, 6556, 6558, 6560, 6561, 6562, 6564, 6570, 6571, 6577, 6581, 6582, 6584, 6586, 6587, 6588, 6590, 6591, 6593, 6595, 6596, 6597, 6599, 6601, 6602, 6606, 6610, 6612, 6613, 6614, 6615, 6616, 6617, 6618, 6620, 6621, 6622, 6623, 6625, 6627, 6628, 6630, 6631, 6632, 6633, 6634, 6639, 6640, 6643, 6644, 6645, 6646, 6649, 6652, 6653, 6655, 6656, 6657, 6659, 6660, 6666, 6668, 6671, 6672, 6674, 6675, 6676, 6678, 6679, 6681, 6682, 6683, 6684, 6685, 6687, 6689, 6690, 6693, 6701, 6702, 6703, 6705, 6707, 6708, 6709, 6714, 6715, 6716, 6718, 6719, 6720, 6721, 6722, 6723, 6725, 6727, 6731, 6733, 6734, 6737, 6738, 6739, 6746, 6747, 6748, 6750, 6751, 6753, 6754, 6755, 6756, 6757, 6760, 6761, 6764, 6765, 6766, 6767, 6769, 6770, 6771, 6772, 6773, 6775, 6776, 6777, 6778, 6783, 6784, 6785, 6787, 6788, 6789, 6790, 6791, 6794, 6796, 6797, 6798, 6799, 6803, 6805, 6806, 6811, 6812, 6813, 6814, 6815, 6816, 6817, 6818, 6820, 6822, 6824, 6825, 6826, 6828, 6830, 6831, 6832, 6835, 6837, 6839, 6841, 6845, 6846, 6849, 6850, 6851, 6856, 6858, 6859, 6860, 6864, 6865, 6869, 6870, 6873, 6874, 6875, 6876, 6878, 6879, 6880, 6881, 6883, 6884, 6885, 6886, 6888, 6889, 6890, 6891, 6892, 6893, 6894, 6895, 6896, 6898, 6899, 6900, 6901, 6902, 6904, 6905, 6907, 6909, 6910, 6911, 6912, 6914, 6916, 6917, 6921, 6924, 6925, 6926, 6928, 6930, 6931, 6932, 6934, 6935, 6936, 6937, 6938, 6941, 6950, 6952, 6954, 6955, 6956, 6957, 6959, 6961, 6967, 6968, 6970, 6971, 6972, 6974, 6975, 6978, 6980, 6982, 6983, 6985, 6986, 6987, 6988, 6990, 6991, 6992, 6993, 6997, 6998, 7001, 7003, 7005, 7007, 7008, 7009, 7010, 7011, 7012, 7013, 7016, 7018, 7020, 7021, 7024, 7026, 7027, 7028, 7029, 7030, 7031, 7033, 7034, 7035, 7037, 7038, 7039, 7043, 7044, 7047, 7048, 7049, 7050, 7052, 7057, 7059, 7060, 7061, 7062, 7064, 7065, 7066, 7067, 7068, 7070, 7074, 7076, 7078, 7081, 7082, 7083, 7084, 7089, 7094, 7095, 7098, 7099, 7101, 7106, 7107, 7108, 7110, 7113, 7114, 7119, 7120, 7121, 7124, 7128, 7131, 7135, 7136, 7137, 7140, 7141, 7142, 7143, 7146, 7147, 7149, 7150, 7151, 7153, 7154, 7155, 7156, 7159, 7160, 7183, 7184, 7185, 7187, 7189, 7190, 7191, 7192, 7193, 7197, 7198, 7204, 7205, 7207, 7213, 7214, 7215, 7217, 7218, 7220, 7223, 7225, 7228, 7234, 7238, 7240, 7241, 7242, 7243, 7245, 7246, 7247, 7250, 7253, 7254, 7255, 7258, 7259, 7262, 7264, 7265, 7266, 7267, 7269, 7270, 7273, 7276, 7277, 7280, 7282, 7284, 7288, 7291, 7294, 7296, 7301, 7302, 7303, 7306, 7307, 7311, 7312, 7313, 7315, 7318, 7319, 7322, 7323, 7325, 7327, 7328, 7329, 7330, 7336, 7337, 7338, 7340, 7345, 7346, 7347, 7348, 7350, 7355, 7359, 7361, 7362, 7365, 7368, 7369, 7370, 7372, 7376, 7377, 7378, 7574, 7575, 7576, 7580, 7582, 7584, 7585, 7586, 7587, 7588, 7589, 7590, 7593, 7594, 7596, 7599, 7600, 7604, 7606, 7609, 7610, 7611, 7612, 7613, 7614, 7616, 7617, 7618, 7619, 7620, 7621, 7622, 7627, 7629, 7630, 7631, 7633, 7634, 7636, 7638, 7640, 7642, 7644, 7645, 7647, 7648, 7649, 7652, 7653, 7654, 7655, 7656, 7657, 7659, 7660, 7661, 7662, 7663, 7665, 7666, 7667, 7668, 7669, 7671, 7673, 7674, 7675, 7676, 7678, 7683, 7684, 7686, 7689, 7690, 7691, 7692, 7693, 7694, 7695, 7697, 7698, 7699, 7701, 7702, 7703, 7704, 7705, 7706, 7707, 7708, 7711, 7714, 7715, 7717, 7719, 7720, 7721, 7722, 7723, 7724, 7726, 7731, 7733, 7736, 7737, 7738, 7740, 7743, 7746, 7750, 7755, 7757, 7759, 7760, 7761, 7762, 7763, 7764, 7767, 7768, 7770, 7771, 7772, 7775, 7776, 7778, 7781, 7782, 7783, 7784, 7785, 7787, 7788, 7789, 7791, 7793, 7794, 7795, 7796, 7797, 7798, 7799, 7802, 7803, 7804, 7805, 7806, 7807, 7809, 7811, 7812, 7813, 7814, 7816, 7817, 7819, 7820, 7821, 7824, 7825, 7827, 7828, 7829, 7830, 7834, 7836, 7838, 7839, 7841, 7842, 7845, 7846, 7850, 7851, 7852, 7853, 7855, 7856, 7857, 7859, 7861, 7862, 7863, 7865, 7866, 7868, 7869, 7870, 7873, 7875, 7876, 7878, 7880, 7881, 7883, 7884, 7885, 7887, 7888, 7889, 7891, 7895, 7897, 7898, 7899, 7900, 7901, 7902, 7904, 7905, 7906, 7907, 7908, 7909, 7910, 7913, 7916, 7917, 7919, 7920, 7922, 7923, 7924, 7926, 7927, 7930, 7935, 7936, 7938, 7940, 7942, 7943, 7950, 7951, 7952, 7953, 7955, 7956, 7958, 7960, 7961, 7963, 7964, 7965, 7966, 7971, 7972, 7974, 7976, 7979, 7981, 7983, 7984, 7985, 7988, 7990, 7992, 7993, 7994, 7995, 7997, 8000, 8001, 8002, 8004, 8005, 8006, 8007, 8026, 8027, 8028, 8029, 8030, 8031, 8033, 8034, 8035, 8036, 8038, 8041, 8042, 8043, 8044, 8045, 8047, 8048, 8049, 8051, 8052, 8053, 8054, 8056, 8058, 8059, 8061, 8062, 8064, 8067, 8068, 8069, 8070, 8073, 8079, 8080, 8081, 8082, 8083, 8084, 8087, 8088, 8089, 8091, 8092, 8093, 8094, 8098, 8105, 8108, 8109, 8110, 8111, 8112, 8113, 8114, 8115, 8116, 8117, 8118, 8120, 8121, 8124, 8125, 8126, 8127, 8128, 8130, 8131, 8132, 8135, 8137, 8141, 8142, 8143, 8144, 8145, 8147, 8150, 8151, 8152, 8154, 8155, 8156, 8160, 8161, 8163, 8167, 8168, 8171, 8172, 8173, 8175, 8176, 8179, 8181, 8183, 8187, 8189, 8190, 8192, 8193, 8194, 8197, 8201, 8202, 8204, 8212, 8213, 8214, 8215, 8217, 8219, 8222, 8224, 8225, 8226, 8227, 8228, 8229, 8231, 8232, 8233, 8236, 8238, 8239, 8242, 8244, 8248, 8249, 8251, 8254, 8256, 8258, 8259, 8260, 8262, 8264, 8265, 8266, 8267, 8268, 8269, 8270, 8272, 8273, 8274, 8276, 8277, 8278, 8279, 8281, 8284, 8285, 8286, 8287, 8289, 8290, 8291, 8292, 8293, 8294, 8296, 8300, 8301, 8302, 8303, 8307, 8308, 8311, 8312, 8313, 8314, 8316, 8318, 8319, 8320, 8322, 8324, 8326, 8329, 8330, 8331, 8335, 8336, 8337, 8338, 8339, 8341, 8343, 8345, 8348, 8349, 8350, 8351, 8352, 8353, 8354, 8355, 8356, 8359, 8360, 8364, 8367, 8369, 8370, 8375, 8376, 8380, 8381, 8385, 8388, 8389, 8390, 8391, 8393, 8395, 8396, 8397, 8401, 8402, 8403, 8408, 8409, 8411, 8412, 8414, 8415, 8416, 8418, 8420, 8422, 8423, 8427, 8430, 8431, 8432, 8435, 8437, 8439, 8440, 8441, 8443, 8444, 8445, 8446, 8447, 8448, 8449, 8451, 8453, 8454, 8456, 8459, 8464, 8465, 8466, 8467, 8471, 8472, 8474, 8475, 8477, 8482, 8483, 8484, 8485, 8487, 8488, 8489, 8492, 8493, 8495, 8496, 8498, 8499, 8500, 8502, 8503, 8505, 8509, 8510, 8511, 8512, 8513, 8514, 8515, 8518, 8519, 8520, 8521, 8522, 8524, 8525, 8526, 8527, 8529, 8534, 8535, 8537, 8538, 8539, 8540, 8541, 8542, 8543, 8546, 8547, 8549, 8553, 8555, 8556, 8558, 8559, 8560, 8562, 8563, 8567, 8568, 8570, 8571, 8573, 8574, 8575, 8576, 8578, 8579, 8580, 8581, 8583, 8585, 8586, 8588, 8590, 8592, 8594, 8595, 8596, 8597, 8598, 8599, 8601, 8604, 8605, 8606, 8607, 8608, 8609, 8613, 8616, 8617, 8618, 8620, 8621, 8624, 8625, 8626, 8631, 8633, 8637, 8638, 8639, 8641, 8642, 8643, 8644, 8648, 8650, 8651, 8656, 8658, 8659, 8660, 8661, 8662, 8663, 8666, 8668, 8669, 8670, 8671, 8672, 8675, 8676, 8677, 8678, 8680, 8681, 8683, 8685, 8686, 8688, 8689, 8691, 8692, 8694, 8695, 8696, 8697, 8699, 8706, 8707, 8708, 8713, 8715, 8716, 8717, 8718, 8719, 8721, 8722, 8723, 8727, 8730, 8731, 8732, 8733, 8734, 8735, 8738, 8741, 8743, 8745, 8746, 8748, 8751, 8757, 8758, 8759, 8761, 8762, 8763, 8764, 8765, 8766, 8768, 8770, 8771, 8773, 8774, 8775, 8777, 8778, 8782, 8783, 8785, 8788, 8792, 8793, 8794, 8795, 8799, 8800, 8802, 8807, 8808, 8809, 8810, 8811, 8812, 8814, 8815, 8819, 8820, 8821, 8822, 8823, 8824, 8826, 8827, 8828, 8829, 8830, 8831, 8833, 8834, 8837, 8838, 8839, 8840, 8841, 8842, 8847, 8848, 8849, 8850, 8851, 8853, 8855, 8856, 8862, 8863, 8865, 8867, 8871, 8872, 8873, 8874, 8875, 8876, 8877, 8879, 8887, 8888, 8889, 8890, 8895, 8896, 8897, 8898, 8899, 8902, 8903, 8906, 8907, 8909, 8914, 8915, 8916, 8917, 8918, 8919, 8920, 8921, 8922, 8924, 8925, 8926, 8928, 8929, 8932, 8935, 8936, 8937, 8939, 8940, 8941, 8942, 8946, 8947, 8949, 8964, 8967, 8968, 8969, 8970, 8972, 8974, 8979, 8982, 8983, 8985, 8986, 8988, 8989, 8990, 8993, 8994, 8996, 8998, 8999, 9001, 9002, 9003, 9005, 9009, 9010, 9011, 9012, 9013, 9014, 9015, 9016, 9018, 9021, 9022, 9024, 9025, 9027, 9028, 9029, 9030, 9032, 9053, 9058, 9059, 9060, 9062, 9064, 9065, 9066, 9067, 9069, 9070, 9071, 9072, 9073, 9074, 9075, 9077, 9079, 9081, 9082, 9084, 9085, 9086, 9087, 9088, 9089, 9090, 9094, 9095, 9098, 9100, 9101, 9104, 9105, 9106, 9107, 9111, 9113, 9116, 9117, 9119, 9121, 9122, 9123, 9124, 9125, 9126, 9128, 9129, 9130, 9131, 9132, 9133, 9135, 9136, 9137, 9138, 9139, 9141, 9142, 9143, 9144, 9145, 9147, 9148, 9150, 9152, 9153, 9154, 9157, 9158, 9161, 9162, 9165, 9166, 9167, 9170, 9171, 9172, 9173, 9174, 9175, 9177, 9178, 9179, 9181, 9182, 9183, 9184, 9186, 9187, 9188, 9191, 9192, 9194, 9195, 9199, 9200, 9201, 9203, 9205, 9206, 9209, 9210, 9212, 9213, 9215, 9216, 9218, 9219, 9221, 9222, 9223, 9225, 9226, 9227, 9229, 9230, 9231, 9234, 9235, 9237, 9238, 9239, 9240, 9242, 9244, 9247, 9249, 9251, 9252, 9253, 9254, 9256, 9257, 9261, 9263, 9264, 9265, 9267, 9268, 9270, 9272, 9276, 9277, 9279, 9281, 9282, 9285, 9287, 9288, 9289, 9290, 9294, 9295, 9297, 9301, 9302, 9303, 9307, 9309, 9311, 9312, 9313, 9314, 9315, 9316, 9318, 9319, 9320, 9322, 9324, 9326, 9327, 9328, 9330, 9331, 9332, 9333, 9334, 9335, 9336, 9338, 9339, 9340, 9342, 9345, 9346, 9348, 9353, 9354, 9355, 9358, 9359, 9360, 9361, 9365, 9368, 9369, 9370, 9371, 9372, 9375, 9376, 9377, 9379, 9380, 9381, 9383, 9386, 9387, 9391, 9395, 9397, 9398, 9400, 9401, 9402, 9403, 9404, 9406, 9407, 9408, 9409, 9410, 9411, 9413, 9414, 9416, 9418, 9421, 9423, 9424, 9426, 9427, 9429, 9430, 9431, 9432, 9437, 9439, 9440, 9442, 9445, 9448, 9450, 9452, 9455, 9456, 9457, 9458, 9459, 9460, 9461, 9464, 9465, 9467, 9469, 9471, 9474, 9478, 9479, 9481, 9483, 9484, 9485, 9487, 9488, 9489, 9490, 9492, 9493, 9494, 9495, 9498, 9503, 9505, 9507, 9509, 9513, 9515, 9519, 9521, 9526, 9527, 9529, 9530, 9531, 9532, 9534, 9536, 9537, 9538, 9541, 9542, 9543, 9545, 9546, 9548, 9549, 9551, 9552, 9553, 9554, 9555, 9558, 9560, 9566, 9567, 9569, 9571, 9572, 9575, 9576, 9578, 9580, 9581, 9582, 9586, 9588, 9589, 9592, 9596, 9598, 9600, 9603, 9607, 9608, 9609, 9610, 9611, 9612, 9614, 9615, 9617, 9619, 9620, 9621, 9622, 9624, 9625, 9626, 9627, 9628, 9631, 9634, 9636, 9637, 9638, 9639, 9640, 9643, 9644, 9646, 9647, 9649, 9657, 9658, 9660, 9662, 9663, 9664, 9665, 9666, 9668, 9669, 9671, 9673, 9674, 9675, 9676, 9677, 9678, 9681, 9685, 9688, 9691, 9692, 9693, 9694, 9695, 9696, 9697, 9700, 9701, 9702, 9703, 9706, 9707, 9710, 9711, 9712, 9715, 9717, 9718, 9721, 9722, 9725, 9730, 9732, 9733, 9735, 9738, 9739, 9740, 9741, 9742, 9743, 9744, 9745, 9746, 9750, and 9751

6. The isolated polynucleotide of claim 1, which encodes an artificial variant comprising a substitution, deletion, and/or insertion of one or more amino acids of an amino acid sequence selected from the group consisting of SEQ ID NOs: 4877-9751 and fragments thereof retaining biological activity.

7. The isolated polynucleotide of any of claims 1-6, which is contained in the chomosome of *Bacillus licheniformis* SJ 1904 (ATCC PTA-7992).

8. A nucleic acid construct comprising the isolated polynucleotide of any of claims 1-7 operably linked to one or more control sequences that direct the production of the biologically active substance in an expression host.

9. A recombinant expression vector comprising the nucleic acid construct of claim 8.

10. A recombinant host cell comprising the nucleic acid construct of claim 8.

11. An isolated biologically active substance, selected from the group consisting of:

(a) a biologically active substance having an amino acid sequence having preferably at least 60% identity, more preferably at least 65% identity, more preferably at least 70% identity, more preferably at least 75% identity, more preferably at least 80% identity, more preferably at least 85% identity, more preferably at least 90% identity, even more preferably at least 90% identity, most preferably at least 95% identity, and even most preferably at least 97% identity with an amino acid sequence selected from the group consisting of SEQ ID NOs: 4877-9751 and fragments thereof retaining biological activity;
(b) a biologically active substance encoded by a polynucleotide comprising a nucleotide sequence having preferably at least 60% identity, more preferably at least 65% identity, more preferably at least 70% identity, more preferably at least 75% identity, more preferably at least 80% identity, more preferably at least 85% identity, more preferably at least 90% identity, even more preferably at least 90% identity, most preferably at least 95% identity, and even most preferably at least 97% identity with a polynucleotide selected from the group consisting of SEQ ID NOs: 2-4876 and subsequences thereof encoding fragments having biological activity;
(c) a biologically active substance encoded by a polynucleotide comprising a nucleotide sequence that hybridizes under preferably at least medium stringency conditions, more preferably at least medium-high stringency conditions, or most preferably at least high stringency conditions with a polynucleotide selected from the group consisting of SEQ ID NOs: 2-4876 and full-length complementary strands thereof; and
(d) an artificial variant comprising a substitution, deletion, and/or insertion of one or more amino acids of an amino acid sequence selected from the group consisting of SEQ ID NOs: 4877-9751 and fragments thereof retaining biological activity.

12. The biologically active substance of claim 11, comprising an amino acid sequence having a degree of sequence identity of preferably at least 60% identity, more preferably at least 65% identity, more preferably at least 70% identity, more preferably at least 75% identity, more preferably at least 80%, more preferably at least 85%, even more preferably at least 90%, most preferably at least 95%, and even most preferably at least 97% with an amino acid sequence selected from the group consisting of SEQ ID NOs: 4944, 4985, 5051, 5058, 5064, 5076, 5079, 5097, 5098, 5099, 5106, 5115, 5117, 5136, 5137, 5197, 5204, 5286, 5459, 5529, 5604, 5723, 5724, 5725, 5726, 5761, 5923, 6056, 6097, 6153, 6192, 6371, 6372, 6376, 6456, 6504, 6531, 6545, 6546, 6547, 6548, 6550, 6945, 6965, 7053, 7116, 7163, 7164, 7165, 7166, 7169, 7170, 7174, 7176, 7177, 7363, 7392, 7397, 7399, 7402, 7420, 7421, 7423, 7425, 7438, 7449, 7453, 7468, 7507, 7562, 7563, 7565, 7573, 7641, 7815, 8003, 8008, 8009, 8010, 8011, 8014, 8015, 8018, 8020, 8021, 8022, 8046, 8099, 8100, 8103, 8140, 8323, 8710, 8711, 8966, 9034, 9035, 9036, 9049, 9051, 9054, 9056, 9057, 9224, 9516, 9525, 9547, 9562, 9564, 4900, 4925, 4930, 4952, 4957, 4966, 4973, 4978, 5000, 5010, 5044, 5046, 5047, 5049, 5052, 5053, 5054, 5055, 5056, 5057, 5060, 5061, 5062, 5067, 5069, 5070, 5071, 5073, 5074, 5078, 5080, 5081, 5082, 5083, 5084, 5085, 5086, 5087, 5088, 5089, 5090, 5091, 5092, 5093, 5094, 5095, 5096, 5100, 5101, 5102, 5103, 5104, 5105, 5112, 5116, 5118, 5119, 5120, 5121, 5122, 5123, 5124, 5125, 5126, 5127, 5128, 5129, 5130, 5131, 5132, 5133, 5134, 5135, 5138, 5139, 5140, 5141, 5151, 5162, 5173, 5180, 5181, 5183, 5192, 5198, 5225, 5229, 5235, 5236, 5238, 5242, 5249, 5256, 5290, 5297, 5307, 5320, 5344, 5347, 5353, 5354, 5362, 5363, 5366, 5373, 5391, 5396, 5398, 5399, 5412, 5416, 5428, 5445, 5450, 5456, 5467, 5482, 5493, 5496, 5502, 5503, 5506, 5541, 5547, 5563, 5566, 5574, 5577, 5585, 5594, 5601, 5609, 5623, 5626, 5639, 5648, 5649, 5652, 5664, 5666, 5702, 5704, 5706, 5708, 5717, 5722, 5730, 5732, 5760, 5762, 5776, 5784, 5819, 5832, 5852, 5857, 5866, 5869, 5890, 5893, 5897, 5907, 5909, 5912, 5933, 5953, 5955, 5963, 5966, 5973, 5983, 5986, 5987, 5993, 6003, 6005, 6023, 6026, 6028, 6029, 6033, 6035, 6039, 6045, 6050, 6051, 6052, 6055, 6058, 6059, 6068, 6069, 6074, 6083, 6090, 6095, 6112, 6119, 6137, 6138, 6140, 6141, 6167, 6177, 6188, 6189, 6190, 6191, 6193, 6195, 6209, 6220, 6230, 6245, 6248, 6249, 6276, 6286, 6294, 6296, 6299, 6307, 6308, 6315, 6317, 6323, 6330, 6341, 6345, 6384, 6402, 6414, 6431, 6440, 6445, 6464, 6494, 6506, 6509, 6510, 6511, 6513, 6514, 6515, 6516, 6517, 6518, 6519, 6520, 6521, 6522, 6524, 6525, 6526, 6527, 6528, 6529, 6530, 6532, 6533, 6534, 6535, 6536, 6537, 6538, 6539, 6540, 6541, 6542, 6543, 6544, 6552, 6557, 6559, 6563, 6567, 6569, 6572, 6578, 6579, 6603, 6607, 6609, 6611, 6626, 6650, 6654, 6658, 6661, 6667, 6670, 6673, 6691, 6706, 6711, 6713, 6726, 6728, 6741, 6758, 6762, 6819, 6821, 6877, 6882, 6897, 6913, 6919, 6923, 6940, 6942, 6947, 6951, 6958, 6969, 6981, 6984, 6994, 6995, 6996, 6999, 7006, 7014, 7022, 7023, 7041, 7046, 7069, 7071, 7073, 7079, 7080, 7086, 7088, 7091, 7096, 7100, 7105, 7111, 7115, 7129, 7130, 7133, 7138, 7148, 7152, 7157, 7161, 7167, 7171, 7172, 7173, 7175, 7178, 7179, 7180, 7199, 7200, 7202, 7211, 7212, 7216, 7222, 7226, 7229, 7237, 7239, 7256, 7263, 7275, 7278, 7279, 7283, 7285, 7286, 7287, 7289, 7290, 7299, 7300, 7305, 7310, 7314, 7321, 7324, 7332, 7339, 7342, 7343, 7344, 7351, 7358, 7366, 7373, 7375, 7379, 7380, 7381, 7382, 7383, 7384, 7385, 7386, 7387, 7388, 7389, 7390, 7391, 7393, 7394, 7395, 7396, 7400, 7401, 7403, 7404, 7405, 7406, 7407, 7408, 7409, 7410, 7411, 7412, 7413, 7414, 7415, 7416, 7417, 7418, 7419, 7422, 7424, 7426, 7427, 7428, 7429, 7430, 7431, 7432, 7433, 7434, 7435, 7436, 7437, 7439, 7440, 7442, 7443, 7444, 7445, 7446, 7447, 7448, 7450, 7451, 7452, 7454, 7455, 7456, 7457, 7458, 7459, 7460, 7461, 7462, 7463, 7464, 7465, 7466, 7467, 7469, 7470, 7471, 7472, 7473, 7474, 7475, 7476, 7477, 7478, 7479, 7480, 7481, 7482, 7483, 7484, 7485, 7486, 7487, 7488, 7489, 7490, 7491, 7492, 7493, 7494, 7495, 7496, 7497, 7498, 7499, 7500, 7501, 7502, 7503, 7504, 7505, 7506, 7508, 7509, 7510, 7511, 7513, 7514, 7515, 7516, 7517, 7518, 7519, 7520, 7521, 7522, 7523, 7524, 7525, 7526, 7527, 7528, 7529, 7530, 7531, 7532, 7533, 7534, 7536, 7539, 7540, 7542, 7544, 7545, 7546, 7547, 7548, 7549, 7550, 7551, 7552, 7553, 7554, 7555, 7556, 7559, 7560, 7561, 7564, 7566, 7567, 7568, 7571, 7572, 7581, 7583, 7592, 7595, 7598, 7605, 7608, 7626, 7637, 7646, 7670, 7681, 7688, 7709, 7710, 7716, 7725, 7741, 7742, 7749, 7753, 7758, 7766, 7773, 7777, 7779, 7792, 7822, 7832, 7835, 7844, 7847, 7848, 7874, 7879, 7893, 7894, 7903, 7921, 7929, 7939, 7944, 7957, 7962, 7968, 7987, 7998, 8012, 8016, 8017, 8019, 8023, 8024, 8025, 8037, 8090, 8097, 8101, 8102, 8104, 8106, 8119, 8123, 8134, 8136, 8139, 8162, 8166, 8174, 8178, 8185, 8186, 8196, 8198, 8199, 8208, 8230, 8237, 8246, 8299, 8304, 8306, 8309, 8317, 8321, 8332, 8333, 8346, 8361, 8368, 8371, 8374, 8378, 8379, 8383, 8384, 8394, 8424, 8433, 8452, 8462, 8468, 8473, 8476, 8478, 8481, 8486, 8517, 8528, 8530, 8545, 8548, 8557, 8565, 8569, 8582, 8584, 8591, 8602, 8610, 8611, 8612, 8619, 8628, 8640, 8645, 8655, 8665, 8667, 8673, 8674, 8693, 8700, 8714, 8747, 8754, 8756, 8760, 8779, 8786, 8790, 8791, 8797, 8801, 8806, 8813, 8844, 8886, 8892, 8893, 8908, 8912, 8923, 8930, 8931, 8934, 8943, 8951, 8952, 8953, 8956, 8957, 8960, 8961, 8965, 8981, 8987, 8992, 9019, 9020, 9033, 9037, 9038, 9039, 9040, 9041, 9042, 9043, 9044, 9045, 9046, 9047, 9048, 9050, 9076, 9096, 9110, 9114, 9118, 9160, 9163, 9180, 9198, 9208, 9211, 9228, 9245, 9273, 9284, 9292, 9296, 9298, 9304, 9310, 9321, 9323, 9325, 9337, 9344, 9347, 9349, 9351, 9357, 9362, 9363, 9374, 9384, 9385, 9388, 9393, 9419, 9433, 9434, 9435, 9436, 9444, 9446, 9453, 9475, 9480, 9482, 9497, 9506, 9508, 9518, 9520, 9522, 9523, 9524, 9533, 9544, 9557, 9568, 9579, 9583, 9593, 9597, 9629, 9642, 9661, 9667, 9672, 9679, 9687, 9689, 9698, 9705, 9708, 9713, 9723, 9724, 9729, 9734, 9737, and 9747 ; preferably at least 65% identity, more preferably at least 70% identity, more preferably at least 75% identity, more preferably at least 80%, more preferably at least 85%, even more preferably at least 90%, most preferably at least 95%, and even most preferably at least 97% with an amino acid sequence selected from the group consisting of SEQ ID NOs: 4991, 5068, 5308, 5463, 5919, 6549, 6648, 6680, 6948, 7298, 7541, 7896, 7911, 8497, 8702, 8729, 8737, 9052, 9690, and 9709 ; preferably at least 70% identity, more preferably at least 75% identity, more preferably at least 80%, more preferably at least 85%, even more preferably at least 90%, most preferably at least 95%, and even most preferably at least 97% with an amino acid sequence selected from the group consisting of SEQ ID NOs: 4986, 5041, 5043, 5418, 5427, 5500, 5515, 5696, 5713, 5785, 5939, 6106, 6109, 6171, 6260, 6503, 6677, 6724, 7054, 7260, 7295, 7316, 7341, 7512, 7538, 7591, 7628, 7735, 7912, 8050, 8076, 8206, 8207, 8240, 8327, 8406, 8428, 8860, 8955, 9061, 9099, 9115, 9262, 9591, 9641, and 9720; preferably at least 75% identity, more preferably at least 80%, more preferably at least 85%, even more preferably at least 90%, most preferably at least 95%, and even most preferably at least 97% with an amino acid sequence selected from the group consisting of SEQ ID NOs: 5038, 5059, 5240, 5403, 5444, 5508, 5559, 5562, 5642, 5651, 5846, 5885, 5921, 5942, 6015, 6067, 6071, 6164, 6215, 6293, 6373, 6390, 6446, 6624, 6704, 6717, 6749, 6759, 6808, 6847, 6848, 6852, 6854, 7292, 7297, 7308, 7317, 7537, 7543, 7557, 7603, 7651, 7858, 7949, 7954, 7996, 8074, 8200, 8221, 8263, 8288, 8614, 8684, 8720, 8728, 8740, 8749, 8787, 8864, 8958, 9055, 9176, 9196, 9283, 9308, 9352, 9491, 9512, 9602, 9653, 9684, 9719, and 9731; preferably at least 80% identity, more preferably at least 85% identity, even more preferably at least 90% identity, most preferably at least 95% identity, and even most preferably at least 97% identity with an amino acid sequence selected from the group consisting of SEQ ID NOs: 4891, 4956, 5042, 5072, 5169, 5190, 5195, 5217, 5241, 5266, 5305, 5369, 5374, 5377, 5409, 5435, 5512, 5524, 5538, 5595, 5613, 5647, 5657, 5665, 5667, 5735, 5743, 5830, 5878, 5900, 5903, 5977, 5988, 6000, 6060, 6066, 6070, 6073, 6075, 6105, 6120, 6139, 6152, 6174, 6291, 6335, 6375, 6437, 6466, 6492, 6505, 6507, 6523, 6553, 6592, 6651, 6694, 6730, 6732, 6774, 6843, 6929, 6943, 6964, 7093, 7132, 7144, 7224, 7349, 7374, 7398, 7570, 7578, 7632, 7635, 7643, 7680, 7682, 7685, 7700, 7713, 7727, 7800, 7843, 7864, 7918, 7947, 7982, 7989, 7991, 7999, 8055, 8057, 8085, 8095, 8170, 8182, 8191, 8210, 8255, 8261, 8328, 8365, 8399, 8410, 8479, 8551, 8561, 8577, 8589, 8649, 8679, 8742, 8744, 8784, 8789, 8796, 8818, 8832, 8835, 8845, 8854, 8858, 8868, 8878, 8900, 8950, 9007, 9102, 9108, 9151, 9169, 9207, 9241, 9246, 9248, 9250, 9299, 9341, 9343, 9412, 9443, 9470, 9476, 9511, 9528, 9559, 9565, 9577, 9584, 9594, 9595, 9682, and 9714; preferably at least 85% identity, more preferably at least 90% identity, most preferably at least 95% identity, and even most preferably at least 97% identity with an amino acid sequence selected from the group consisting of SEQ ID NOs: 4913, 4948, 4968, 5025, 5034, 5066, 5109, 5110, 5146, 5160, 5199, 5200, 5203, 5211, 5221, 5223, 5226, 5252, 5269, 5273, 5293, 5309, 5318, 5342, 5352, 5393, 5394, 5421, 5430, 5431, 5434, 5447, 5458, 5492, 5507, 5511, 5514, 5525, 5526, 5530, 5544, 5570, 5616, 5617, 5621, 5640, 5661, 5663, 5691, 5716, 5738, 5751, 5753, 5757, 5759, 5786, 5802, 5820, 5826, 5855, 5858, 5871, 5872, 5902, 5905, 5908, 5915, 5936, 5937, 5938, 5947, 6006, 6011, 6012, 6025, 6030, 6032, 6034, 6057, 6080, 6084, 6093, 6096, 6110, 6131, 6178, 6183, 6199, 6232, 6236, 6243, 6257, 6258, 6313, 6331, 6354, 6360, 6362, 6386, 6417, 6421, 6435, 6455, 6468, 6472, 6475, 6476, 6478, 6495, 6555, 6566, 6568, 6604, 6608, 6641, 6662, 6663, 6665, 6692, 6699, 6736, 6752, 6763, 6807, 6809, 6810, 6840, 6842, 6844, 6853, 6855, 6857, 6866, 6906, 6908, 6915, 6922, 6953, 6962, 6977, 6989, 7004, 7025, 7032, 7042, 7045, 7058, 7072, 7085, 7134, 7139, 7145, 7182, 7188, 7194, 7208, 7227, 7249, 7261, 7272, 7293, 7309, 7320, 7331, 7333, 7334, 7354, 7367, 7371, 7558, 7597, 7602, 7623, 7664, 7677, 7679, 7687, 7732, 7734, 7739, 7744, 7752, 7765, 7769, 7790, 7801, 7818, 7823, 7833, 7837, 7860, 7867, 7877, 7882, 7915, 7931, 7932, 7946, 7948, 7959, 7967, 7973, 7975, 7977, 7978, 7986, 8071, 8072, 8096, 8107, 8129, 8146, 8148, 8153, 8164, 8165, 8169, 8203, 8211, 8216, 8218, 8234, 8245, 8253, 8275, 8297, 8315, 8334, 8340, 8357, 8362, 8372, 8386, 8398, 8425, 8438, 8455, 8460, 8470, 8480, 8490, 8506, 8531, 8533, 8572, 8593, 8600, 8615, 8623, 8627, 8630, 8634, 8664, 8687, 8703, 8704, 8705, 8726, 8739, 8750, 8752, 8753, 8769, 8780, 8781, 8805, 8816, 8825, 8846, 8869, 8870, 8883, 8913, 8938, 8944, 8945, 8948, 8959, 8962, 8973, 8976, 8980, 8991, 9000, 9006, 9017, 9068, 9093, 9097, 9103, 9109, 9146, 9155, 9159, 9255, 9258, 9260, 9266, 9269, 9271, 9278, 9293, 9350, 9373, 9394, 9415, 9420, 9449, 9466, 9472, 9477, 9486, 9500, 9504, 9514, 9517, 9535, 9573, 9590, 9599, 9601, 9623, 9645, 9648, 9652, 9654, 9659, 9726, 9728, 9736, and 9748; preferably at least 90% identity, more preferably at least 95% identity, and most preferably at least 97% identity with an amino acid sequence selected from the group consisting ofSEQ ID NOs: 4890, 4895, 4921, 4923, 4927, 4933, 4934, 4937, 4939, 4943, 4946, 4950, 4951, 4955, 4958, 4967, 4970, 4981, 4982, 4987, 4989, 4999, 5017, 5022, 5029, 5048, 5111, 5113, 5145, 5152, 5157, 5165, 5171, 5178, 5179, 5182, 5186, 5202, 5213, 5214, 5215, 5218, 5227, 5230, 5233, 5245, 5268, 5272, 5274, 5279, 5281, 5283, 5298, 5303, 5315, 5324, 5326, 5328, 5329, 5330, 5336, 5337, 5338, 5339, 5346, 5357, 5360, 5376, 5380, 5387, 5400, 5401, 5414, 5417, 5419, 5422, 5424, 5436, 5442, 5446, 5448, 5455, 5457, 5466, 5475, 5499, 5501, 5510, 5537, 5539, 5542, 5550, 5552, 5557, 5565, 5571, 5573, 5586, 5589, 5612, 5615, 5620, 5622, 5628, 5632, 5634, 5638, 5645, 5646, 5660, 5670, 5677, 5678, 5681, 5688, 5705, 5707, 5710, 5739, 5748, 5754, 5756, 5763, 5764, 5765, 5766, 5768, 5779, 5782, 5783, 5787, 5791, 5792, 5793, 5799, 5808, 5813, 5821, 5827, 5828, 5834, 5835, 5837, 5842, 5850, 5856, 5859, 5864, 5870, 5884, 5906, 5914, 5917, 5945, 5946, 5958, 5964, 5965, 5985, 5989, 5994, 5998, 6007, 6009, 6016, 6017, 6019, 6022, 6031, 6048, 6062, 6064, 6079, 6081, 6086, 6089, 6098, 6103, 6107, 6113, 6121, 6122, 6125, 6156, 6163, 6165, 6181, 6205, 6212, 6223, 6235, 6238, 6239, 6242, 6244, 6250, 6251, 6255, 6261, 6262, 6265, 6267, 6269, 6281, 6283, 6284, 6290, 6292, 6300, 6301, 6318, 6324, 6327, 6332, 6333, 6338, 6339, 6344, 6349, 6353, 6361, 6363, 6374, 6377, 6383, 6387, 6396, 6397, 6399, 6400, 6413, 6423, 6449, 6450, 6462, 6465, 6467, 6471, 6484, 6493, 6501, 6512, 6565, 6573, 6574, 6575, 6576, 6580, 6583, 6585, 6589, 6594, 6598, 6600, 6605, 6619, 6629, 6635, 6636, 6637, 6638, 6642, 6647, 6664, 6669, 6686, 6688, 6695, 6696, 6697, 6698, 6700, 6710, 6712, 6729, 6735, 6740, 6742, 6743, 6744, 6745, 6768, 6779, 6780, 6781, 6782, 6786, 6792, 6793, 6795, 6800, 6801, 6802, 6804, 6823, 6827, 6829, 6833, 6834, 6836, 6838, 6861, 6862, 6863, 6867, 6868, 6871, 6872, 6887, 6903, 6918, 6920, 6927, 6933, 6939, 6944, 6949, 6960, 6966, 6973, 6976, 6979, 7000, 7002, 7015, 7017, 7019, 7036, 7040, 7051, 7055, 7056, 7063, 7075, 7077, 7087, 7090, 7092, 7097, 7102, 7103, 7104, 7109, 7112, 7117, 7118, 7122, 7123, 7125, 7126, 7127, 7158, 7162, 7181, 7186, 7196, 7201, 7203, 7206, 7209, 7210, 7219, 7221, 7230, 7231, 7232, 7233, 7235, 7236, 7244, 7248, 7251, 7252, 7257, 7268, 7271, 7274, 7281, 7304, 7326, 7335, 7352, 7356, 7357, 7360, 7364, 7577, 7579, 7601, 7607, 7615, 7624, 7625, 7639, 7650, 7658, 7672, 7696, 7712, 7718, 7728, 7729, 7730, 7745, 7747, 7748, 7751, 7754, 7756, 7774, 7780, 7786, 7808, 7810, 7826, 7831, 7840, 7849, 7854, 7871, 7872, 7886, 7892, 7914, 7925, 7928, 7933, 7934, 7937, 7941, 7945, 7969, 7970, 7980, 8032, 8039, 8040, 8060, 8063, 8065, 8066, 8075, 8077, 8078, 8086, 8122, 8133, 8138, 8149, 8157, 8158, 8159, 8177, 8180, 8184, 8188, 8195, 8205, 8209, 8220, 8223, 8235, 8241, 8243, 8247, 8250, 8252, 8257, 8271, 8280, 8282, 8283, 8295, 8298, 8305, 8310, 8325, 8342, 8344, 8347, 8358, 8363, 8366, 8373, 8377, 8382, 8387, 8392, 8400, 8404, 8405, 8407, 8413, 8417, 8419, 8421, 8426, 8429, 8434, 8436, 8442, 8450, 8457, 8458, 8461, 8463, 8469, 8491, 8494, 8501, 8504, 8507, 8508, 8516, 8523, 8532, 8536, 8544, 8550, 8552, 8554, 8564, 8566, 8587, 8603, 8622, 8629, 8632, 8635, 8636, 8646, 8647, 8652, 8653, 8654, 8657, 8682, 8690, 8698, 8701, 8709, 8712, 8724, 8725, 8736, 8755, 8767, 8772, 8776, 8798, 8803, 8804, 8817, 8836, 8843, 8852, 8857, 8859, 8861, 8866, 8880, 8881, 8882, 8884, 8885, 8891, 8894, 8901, 8904, 8905, 8910, 8911, 8927, 8933, 8954, 8963, 8971, 8975, 8977, 8984, 8995, 8997, 9004, 9008, 9023, 9026, 9031, 9063, 9078, 9080, 9083, 9091, 9092, 9112, 9120, 9127, 9134, 9140, 9149, 9156, 9164, 9168, 9185, 9189, 9190, 9193, 9197, 9202, 9204, 9214, 9217, 9220, 9232, 9233, 9236, 9243, 9259, 9274, 9275, 9280, 9286, 9291, 9300, 9305, 9306, 9317, 9329, 9356, 9364, 9366, 9367, 9378, 9382, 9389, 9390, 9392, 9396, 9399, 9405, 9417, 9422, 9425, 9428, 9438, 9441, 9451, 9454, 9462, 9463, 9468, 9473, 9496, 9499, 9501, 9502, 9510, 9539, 9540, 9550, 9556, 9561, 9570, 9574, 9585, 9587, 9604, 9605, 9606, 9613, 9616, 9618, 9630, 9632, 9633, 9635, 9650, 9651, 9655, 9656, 9670, 9680, 9683, 9686, 9699, 9704, 9716, 9727, and 9749; or preferably at least 95% identity and more preferably at least 97% identity with an amino acid sequence selected from the group consisting of SEQ ID NOs: 4877, 4878, 4879, 4880, 4881, 4882, 4883, 4884, 4885, 4886, 4887, 4888, 4889, 4892, 4893, 4894, 4896, 4897, 4898, 4899, 4901, 4902, 4903, 4904, 4905, 4906, 4907, 4908, 4909, 4910, 4911, 4912, 4914, 4915, 4916, 4917, 4918, 4919, 4920, 4922, 4924, 4926, 4928, 4929, 4931, 4932, 4935, 4936, 4938, 4940, 4941, 4942, 4945, 4947, 4949, 4953, 4954, 4959, 4960, 4961, 4962, 4963, 4964, 4965, 4969, 4971, 4972, 4974, 4975, 4976, 4977, 4979, 4980, 4983, 4984, 4988, 4990, 4992, 4993, 4994, 4995, 4996, 4997, 4998, 5001, 5002, 5003, 5004, 5005, 5006, 5007, 5008, 5009, 5011, 5012, 5013, 5014, 5015, 5016, 5018, 5019, 5020, 5021, 5023, 5024, 5026, 5027, 5028, 5030, 5031, 5032, 5033, 5035, 5036, 5037, 5039, 5045, 5050, 5063, 5065, 5075, 5077, 5107, 5108, 5114, 5142, 5143, 5144, 5147, 5148, 5149, 5150, 5153, 5154, 5155, 5156, 5158, 5159, 5161, 5163, 5164, 5166, 5167, 5168, 5170, 5172, 5174, 5175, 5176, 5177, 5184, 5185, 5187, 5188, 5189, 5191, 5193, 5194, 5196, 5201, 5205, 5206, 5207, 5208, 5209, 5210, 5212, 5216, 5219, 5220, 5222, 5224, 5228, 5231, 5232, 5234, 5237, 5239, 5243, 5244, 5246, 5247, 5248, 5250, 5251, 5254, 5255, 5257, 5258, 5259, 5260, 5261, 5262, 5263, 5264, 5265, 5267, 5270, 5271, 5275, 5276, 5277, 5278, 5280, 5282, 5284, 5285, 5287, 5288, 5289, 5291, 5292, 5294, 5295, 5296, 5299, 5300, 5301, 5302, 5304, 5306, 5310, 5311, 5312, 5313, 5314, 5316, 5317, 5319, 5321, 5322, 5323, 5325, 5327, 5331, 5332, 5333, 5334, 5335, 5340, 5341, 5343, 5345, 5348, 5349, 5350, 5351, 5355, 5356, 5358, 5359, 5361, 5364, 5365, 5367, 5368, 5370, 5371, 5372, 5375, 5379, 5381, 5382, 5383, 5384, 5385, 5386, 5388, 5389, 5390, 5392, 5395, 5397, 5402, 5404, 5405, 5406, 5407, 5408, 5410, 5411, 5413, 5415, 5420, 5423, 5425, 5426, 5429, 5432, 5433, 5437, 5438, 5439, 5440, 5441, 5443, 5449, 5451, 5452, 5453, 5454, 5460, 5461, 5462, 5464, 5465, 5468, 5469, 5470, 5471, 5472, 5473, 5474, 5476, 5477, 5478, 5479, 5480, 5481, 5483, 5484, 5485, 5486, 5487, 5488, 5489, 5490, 5491, 5494, 5495, 5497, 5498, 5504, 5505, 5509, 5513, 5516, 5517, 5518, 5519, 5520, 5521, 5522, 5523, 5527, 5528, 5531, 5532, 5533, 5534, 5535, 5536, 5540, 5543, 5545, 5546, 5548, 5549, 5551, 5553, 5554, 5555, 5556, 5558, 5560, 5561, 5564, 5567, 5568, 5569, 5572, 5575, 5576, 5578, 5579, 5580, 5581, 5582, 5583, 5584, 5587, 5588, 5590, 5591, 5592, 5593, 5596, 5597, 5598, 5599, 5600, 5602, 5603, 5605, 5606, 5607, 5608, 5610, 5611, 5614, 5618, 5619, 5624, 5625, 5627, 5629, 5630, 5631, 5633, 5635, 5636, 5637, 5641, 5643, 5644, 5650, 5653, 5654, 5655, 5656, 5658, 5659, 5662, 5668, 5669, 5671, 5672, 5673, 5674, 5675, 5676, 5679, 5680, 5682, 5683, 5684, 5685, 5686, 5687, 5689, 5690, 5692, 5693, 5694, 5695, 5697, 5698, 5699, 5700, 5701, 5703, 5709, 5711, 5712, 5714, 5715, 5718, 5719, 5720, 5721, 5728, 5729, 5731, 5733, 5734, 5736, 5737, 5740, 5741, 5742, 5744, 5745, 5746, 5747, 5749, 5750, 5752, 5755, 5758, 5767, 5769, 5770, 5771, 5772, 5773, 5774, 5775, 5777, 5778, 5780, 5781, 5788, 5789, 5790, 5794, 5795, 5796, 5797, 5798, 5800, 5801, 5803, 5804, 5805, 5806, 5807, 5809, 5810, 5811, 5812, 5814, 5815, 5816, 5817, 5818, 5822, 5823, 5824, 5825, 5829, 5831, 5833, 5836, 5838, 5839, 5840, 5841, 5843, 5844, 5845, 5847, 5848, 5849, 5851, 5853, 5854, 5860, 5861, 5862, 5863, 5865, 5867, 5868, 5873, 5874, 5875, 5876, 5877, 5879, 5880, 5881, 5882, 5883, 5886, 5887, 5888, 5889, 5891, 5892, 5894, 5895, 5896, 5899, 5901, 5904, 5910, 5911, 5913, 5916, 5918, 5920, 5922, 5924, 5925, 5926, 5927, 5928, 5929, 5930, 5931, 5932, 5934, 5935, 5940, 5941, 5943, 5944, 5948, 5949, 5950, 5951, 5952, 5954, 5956, 5957, 5959, 5960, 5961, 5962, 5967, 5968, 5969, 5970, 5971, 5972, 5974, 5975, 5976, 5978, 5979, 5980, 5981, 5982, 5984, 5990, 5991, 5992, 5995, 5996, 5997, 5999, 6001, 6002, 6004, 6008, 6010, 6013, 6014, 6018, 6020, 6021, 6024, 6027, 6036, 6037, 6038, 6040, 6041, 6042, 6043, 6044, 6046, 6047, 6049, 6053, 6054, 6061, 6063, 6065, 6072, 6076, 6077, 6078, 6082, 6085, 6087, 6088, 6091, 6092, 6094, 6099, 6100, 6101, 6102, 6104, 6108, 6111, 6114, 6115, 6116, 6117, 6118, 6123, 6124, 6126, 6127, 6128, 6129, 6130, 6132, 6133, 6134, 6135, 6136, 6142, 6143, 6144, 6145, 6146, 6147, 6148, 6149, 6150, 6151, 6154, 6155, 6157, 6158, 6159, 6160, 6161, 6162, 6166, 6168, 6169, 6170, 6172, 6173, 6175, 6176, 6179, 6180, 6182, 6184, 6185, 6186, 6187, 6194, 6196, 6197, 6198, 6200, 6201, 6202, 6203, 6204, 6206, 6207, 6208, 6210, 6211, 6213, 6214, 6216, 6217, 6218, 6219, 6221, 6222, 6224, 6225, 6226, 6227, 6228, 6229, 6231, 6233, 6234, 6237, 6240, 6246, 6247, 6252, 6253, 6254, 6256, 6259, 6263, 6264, 6266, 6268, 6270, 6271, 6272, 6273, 6274, 6275, 6277, 6278, 6279, 6280, 6282, 6285, 6287, 6288, 6289, 6295, 6297, 6298, 6302, 6303, 6304, 6305, 6306, 6309, 6310, 6311, 6312, 6314, 6316, 6319, 6320, 6321, 6322, 6325, 6326, 6328, 6329, 6336, 6337, 6340, 6342, 6343, 6346, 6347, 6348, 6350, 6351, 6352, 6355, 6356, 6357, 6358, 6359, 6364, 6365, 6366, 6367, 6368, 6369, 6378, 6379, 6380, 6381, 6382, 6385, 6388, 6389, 6391, 6392, 6393, 6394, 6395, 6398, 6401, 6403, 6404, 6405, 6406, 6407, 6408, 6409, 6410, 6411, 6412, 6415, 6416, 6418, 6419, 6420, 6422, 6424, 6425, 6426, 6427, 6428, 6429, 6430, 6432, 6433, 6434, 6436, 6438, 6439, 6441, 6442, 6443, 6444, 6447, 6448, 6451, 6452, 6453, 6454, 6457, 6458, 6459, 6460, 6461, 6463, 6469, 6470, 6473, 6474, 6477, 6479, 6480, 6481, 6482, 6483, 6485, 6486, 6487, 6488, 6489, 6490, 6491, 6496, 6497, 6498, 6499, 6500, 6502, 6508, 6556, 6558, 6560, 6561, 6562, 6564, 6570, 6571, 6577, 6581, 6582, 6584, 6586, 6587, 6588, 6590, 6591, 6593, 6595, 6596, 6597, 6599, 6601, 6602, 6606, 6610, 6612, 6613, 6614, 6615, 6616, 6617, 6618, 6620, 6621, 6622, 6623, 6625, 6627, 6628, 6630, 6631, 6632, 6633, 6634, 6639, 6640, 6643, 6644, 6645, 6646, 6649, 6652, 6653, 6655, 6656, 6657, 6659, 6660, 6666, 6668, 6671, 6672, 6674, 6675, 6676, 6678, 6679, 6681, 6682, 6683, 6684, 6685, 6687, 6689, 6690, 6693, 6701, 6702, 6703, 6705, 6707, 6708, 6709, 6714, 6715, 6716, 6718, 6719, 6720, 6721, 6722, 6723, 6725, 6727, 6731, 6733, 6734, 6737, 6738, 6739, 6746, 6747, 6748, 6750, 6751, 6753, 6754, 6755, 6756, 6757, 6760, 6761, 6764, 6765, 6766, 6767, 6769, 6770, 6771, 6772, 6773, 6775, 6776, 6777, 6778, 6783, 6784, 6785, 6787, 6788, 6789, 6790, 6791, 6794, 6796, 6797, 6798, 6799, 6803, 6805, 6806, 6811, 6812, 6813, 6814, 6815, 6816, 6817, 6818, 6820, 6822, 6824, 6825, 6826, 6828, 6830, 6831, 6832, 6835, 6837, 6839, 6841, 6845, 6846, 6849, 6850, 6851, 6856, 6858, 6859, 6860, 6864, 6865, 6869, 6870, 6873, 6874, 6875, 6876, 6878, 6879, 6880, 6881, 6883, 6884, 6885, 6886, 6888, 6889, 6890, 6891, 6892, 6893, 6894, 6895, 6896, 6898, 6899, 6900, 6901, 6902, 6904, 6905, 6907, 6909, 6910, 6911, 6912, 6914, 6916, 6917, 6921, 6924, 6925, 6926, 6928, 6930, 6931, 6932, 6934, 6935, 6936, 6937, 6938, 6941, 6950, 6952, 6954, 6955, 6956, 6957, 6959, 6961, 6967, 6968, 6970, 6971, 6972, 6974, 6975, 6978, 6980, 6982, 6983, 6985, 6986, 6987, 6988, 6990, 6991, 6992, 6993, 6997, 6998, 7001, 7003, 7005, 7007, 7008, 7009, 7010, 7011, 7012, 7013, 7016, 7018, 7020, 7021, 7024, 7026, 7027, 7028, 7029, 7030, 7031, 7033, 7034, 7035, 7037, 7038, 7039, 7043, 7044, 7047, 7048, 7049, 7050, 7052, 7057, 7059, 7060, 7061, 7062, 7064, 7065, 7066, 7067, 7068, 7070, 7074, 7076, 7078, 7081, 7082, 7083, 7084, 7089, 7094, 7095, 7098, 7099, 7101, 7106, 7107, 7108, 7110, 7113, 7114, 7119, 7120, 7121, 7124, 7128, 7131, 7135, 7136, 7137, 7140, 7141, 7142, 7143, 7146, 7147, 7149, 7150, 7151, 7153, 7154, 7155, 7156, 7159, 7160, 7183, 7184, 7185, 7187, 7189, 7190, 7191, 7192, 7193, 7197, 7198, 7204, 7205, 7207, 7213, 7214, 7215, 7217, 7218, 7220, 7223, 7225, 7228, 7234, 7238, 7240, 7241, 7242, 7243, 7245, 7246, 7247, 7250, 7253, 7254, 7255, 7258, 7259, 7262, 7264, 7265, 7266, 7267, 7269, 7270, 7273, 7276, 7277, 7280, 7282, 7284, 7288, 7291, 7294, 7296, 7301, 7302, 7303, 7306, 7307, 7311, 7312, 7313, 7315, 7318, 7319, 7322, 7323, 7325, 7327, 7328, 7329, 7330, 7336, 7337, 7338, 7340, 7345, 7346, 7347, 7348, 7350, 7355, 7359, 7361, 7362, 7365, 7368, 7369, 7370, 7372, 7376, 7377, 7378, 7574, 7575, 7576, 7580, 7582, 7584, 7585, 7586, 7587, 7588, 7589, 7590, 7593, 7594, 7596, 7599, 7600, 7604, 7606, 7609, 7610, 7611, 7612, 7613, 7614, 7616, 7617, 7618, 7619, 7620, 7621, 7622, 7627, 7629, 7630, 7631, 7633, 7634, 7636, 7638, 7640, 7642, 7644, 7645, 7647, 7648, 7649, 7652, 7653, 7654, 7655, 7656, 7657, 7659, 7660, 7661, 7662, 7663, 7665, 7666, 7667, 7668, 7669, 7671, 7673, 7674, 7675, 7676, 7678, 7683, 7684, 7686, 7689, 7690, 7691, 7692, 7693, 7694, 7695, 7697, 7698, 7699, 7701, 7702, 7703, 7704, 7705, 7706, 7707, 7708, 7711, 7714, 7715, 7717, 7719, 7720, 7721, 7722, 7723, 7724, 7726, 7731, 7733, 7736, 7737, 7738, 7740, 7743, 7746, 7750, 7755, 7757, 7759, 7760, 7761, 7762, 7763, 7764, 7767, 7768, 7770, 7771, 7772, 7775, 7776, 7778, 7781, 7782, 7783, 7784, 7785, 7787, 7788, 7789, 7791, 7793, 7794, 7795, 7796, 7797, 7798, 7799, 7802, 7803, 7804, 7805, 7806, 7807, 7809, 7811, 7812, 7813, 7814, 7816, 7817, 7819, 7820, 7821, 7824, 7825, 7827, 7828, 7829, 7830, 7834, 7836, 7838, 7839, 7841, 7842, 7845, 7846, 7850, 7851, 7852, 7853, 7855, 7856, 7857, 7859, 7861, 7862, 7863, 7865, 7866, 7868, 7869, 7870, 7873, 7875, 7876, 7878, 7880, 7881, 7883, 7884, 7885, 7887, 7888, 7889, 7891, 7895, 7897, 7898, 7899, 7900, 7901, 7902, 7904, 7905, 7906, 7907, 7908, 7909, 7910, 7913, 7916, 7917, 7919, 7920, 7922, 7923, 7924, 7926, 7927, 7930, 7935, 7936, 7938, 7940, 7942, 7943, 7950, 7951, 7952, 7953, 7955, 7956, 7958, 7960, 7961, 7963, 7964, 7965, 7966, 7971, 7972, 7974, 7976, 7979, 7981, 7983, 7984, 7985, 7988, 7990, 7992, 7993, 7994, 7995, 7997, 8000, 8001, 8002, 8004, 8005, 8006, 8007, 8026, 8027, 8028, 8029, 8030, 8031, 8033, 8034, 8035, 8036, 8038, 8041, 8042, 8043, 8044, 8045, 8047, 8048, 8049, 8051, 8052, 8053, 8054, 8056, 8058, 8059, 8061, 8062, 8064, 8067, 8068, 8069, 8070, 8073, 8079, 8080, 8081, 8082, 8083, 8084, 8087, 8088, 8089, 8091, 8092, 8093, 8094, 8098, 8105, 8108, 8109, 8110, 8111, 8112, 8113, 8114, 8115, 8116, 8117, 8118, 8120, 8121, 8124, 8125, 8126, 8127, 8128, 8130, 8131, 8132, 8135, 8137, 8141, 8142, 8143, 8144, 8145, 8147, 8150, 8151, 8152, 8154, 8155, 8156, 8160, 8161, 8163, 8167, 8168, 8171, 8172, 8173, 8175, 8176, 8179, 8181, 8183, 8187, 8189, 8190, 8192, 8193, 8194, 8197, 8201, 8202, 8204, 8212, 8213, 8214, 8215, 8217, 8219, 8222, 8224, 8225, 8226, 8227, 8228, 8229, 8231, 8232, 8233, 8236, 8238, 8239, 8242, 8244, 8248, 8249, 8251, 8254, 8256, 8258, 8259, 8260, 8262, 8264, 8265, 8266, 8267, 8268, 8269, 8270, 8272, 8273, 8274, 8276, 8277, 8278, 8279, 8281, 8284, 8285, 8286, 8287, 8289, 8290, 8291, 8292, 8293, 8294, 8296, 8300, 8301, 8302, 8303, 8307, 8308, 8311, 8312, 8313, 8314, 8316, 8318, 8319, 8320, 8322, 8324, 8326, 8329, 8330, 8331, 8335, 8336, 8337, 8338, 8339, 8341, 8343, 8345, 8348, 8349, 8350, 8351, 8352, 8353, 8354, 8355, 8356, 8359, 8360, 8364, 8367, 8369, 8370, 8375, 8376, 8380, 8381, 8385, 8388, 8389, 8390, 8391, 8393, 8395, 8396, 8397, 8401, 8402, 8403, 8408, 8409, 8411, 8412, 8414, 8415, 8416, 8418, 8420, 8422, 8423, 8427, 8430, 8431, 8432, 8435, 8437, 8439, 8440, 8441, 8443, 8444, 8445, 8446, 8447, 8448, 8449, 8451, 8453, 8454, 8456, 8459, 8464, 8465, 8466, 8467, 8471, 8472, 8474, 8475, 8477, 8482, 8483, 8484, 8485, 8487, 8488, 8489, 8492, 8493, 8495, 8496, 8498, 8499, 8500, 8502, 8503, 8505, 8509, 8510, 8511, 8512, 8513, 8514, 8515, 8518, 8519, 8520, 8521, 8522, 8524, 8525, 8526, 8527, 8529, 8534, 8535, 8537, 8538, 8539, 8540, 8541, 8542, 8543, 8546, 8547, 8549, 8553, 8555, 8556, 8558, 8559, 8560, 8562, 8563, 8567, 8568, 8570, 8571, 8573, 8574, 8575, 8576, 8578, 8579, 8580, 8581, 8583, 8585, 8586, 8588, 8590, 8592, 8594, 8595, 8596, 8597, 8598, 8599, 8601, 8604, 8605, 8606, 8607, 8608, 8609, 8613, 8616, 8617, 8618, 8620, 8621, 8624, 8625, 8626, 8631, 8633, 8637, 8638, 8639, 8641, 8642, 8643, 8644, 8648, 8650, 8651, 8656, 8658, 8659, 8660, 8661, 8662, 8663, 8666, 8668, 8669, 8670, 8671, 8672, 8675, 8676, 8677, 8678, 8680, 8681, 8683, 8685, 8686, 8688, 8689, 8691, 8692, 8694, 8695, 8696, 8697, 8699, 8706, 8707, 8708, 8713, 8715, 8716, 8717, 8718, 8719, 8721, 8722, 8723, 8727, 8730, 8731, 8732, 8733, 8734, 8735, 8738, 8741, 8743, 8745, 8746, 8748, 8751, 8757, 8758, 8759, 8761, 8762, 8763, 8764, 8765, 8766, 8768, 8770, 8771, 8773, 8774, 8775, 8777, 8778, 8782, 8783, 8785, 8788, 8792, 8793, 8794, 8795, 8799, 8800, 8802, 8807, 8808, 8809, 8810, 8811, 8812, 8814, 8815, 8819, 8820, 8821, 8822, 8823, 8824, 8826, 8827, 8828, 8829, 8830, 8831, 8833, 8834, 8837, 8838, 8839, 8840, 8841, 8842, 8847, 8848, 8849, 8850, 8851, 8853, 8855, 8856, 8862, 8863, 8865, 8867, 8871, 8872, 8873, 8874, 8875, 8876, 8877, 8879, 8887, 8888, 8889, 8890, 8895, 8896, 8897, 8898, 8899, 8902, 8903, 8906, 8907, 8909, 8914, 8915, 8916, 8917, 8918, 8919, 8920, 8921, 8922, 8924, 8925, 8926, 8928, 8929, 8932, 8935, 8936, 8937, 8939, 8940, 8941, 8942, 8946, 8947, 8949, 8964, 8967, 8968, 8969, 8970, 8972, 8974, 8979, 8982, 8983, 8985, 8986, 8988, 8989, 8990, 8993, 8994, 8996, 8998, 8999, 9001, 9002, 9003, 9005, 9009, 9010, 9011, 9012, 9013, 9014, 9015, 9016, 9018, 9021, 9022, 9024, 9025, 9027, 9028, 9029, 9030, 9032, 9053, 9058, 9059, 9060, 9062, 9064, 9065, 9066, 9067, 9069, 9070, 9071, 9072, 9073, 9074, 9075, 9077, 9079, 9081, 9082, 9084, 9085, 9086, 9087, 9088, 9089, 9090, 9094, 9095, 9098, 9100, 9101, 9104, 9105, 9106, 9107, 9111, 9113, 9116, 9117, 9119, 9121, 9122, 9123, 9124, 9125, 9126, 9128, 9129, 9130, 9131, 9132, 9133, 9135, 9136, 9137, 9138, 9139, 9141, 9142, 9143, 9144, 9145, 9147, 9148, 9150, 9152, 9153, 9154, 9157, 9158, 9161, 9162, 9165, 9166, 9167, 9170, 9171, 9172, 9173, 9174, 9175, 9177, 9178, 9179, 9181, 9182, 9183, 9184, 9186, 9187, 9188, 9191, 9192, 9194, 9195, 9199, 9200, 9201, 9203, 9205, 9206, 9209, 9210, 9212, 9213, 9215, 9216, 9218, 9219, 9221, 9222, 9223, 9225, 9226, 9227, 9229, 9230, 9231, 9234, 9235, 9237, 9238, 9239, 9240, 9242, 9244, 9247, 9249, 9251, 9252, 9253, 9254, 9256, 9257, 9261, 9263, 9264, 9265, 9267, 9268, 9270, 9272, 9276, 9277, 9279, 9281, 9282, 9285, 9287, 9288, 9289, 9290, 9294, 9295, 9297, 9301, 9302, 9303, 9307, 9309, 9311, 9312, 9313, 9314, 9315, 9316, 9318, 9319, 9320, 9322, 9324, 9326, 9327, 9328, 9330, 9331, 9332, 9333, 9334, 9335, 9336, 9338, 9339, 9340, 9342, 9345, 9346, 9348, 9353, 9354, 9355, 9358, 9359, 9360, 9361, 9365, 9368, 9369, 9370, 9371, 9372, 9375, 9376, 9377, 9379, 9380, 9381, 9383, 9386, 9387, 9391, 9395, 9397, 9398, 9400, 9401, 9402, 9403, 9404, 9406, 9407, 9408, 9409, 9410, 9411, 9413, 9414, 9416, 9418, 9421, 9423, 9424, 9426, 9427, 9429, 9430, 9431, 9432, 9437, 9439, 9440, 9442, 9445, 9448, 9450, 9452, 9455, 9456, 9457, 9458, 9459, 9460, 9461, 9464, 9465, 9467, 9469, 9471, 9474, 9478, 9479, 9481, 9483, 9484, 9485, 9487, 9488, 9489, 9490, 9492, 9493, 9494, 9495, 9498, 9503, 9505, 9507, 9509, 9513, 9515, 9519, 9521, 9526, 9527, 9529, 9530, 9531, 9532, 9534, 9536, 9537, 9538, 9541, 9542, 9543, 9545, 9546, 9548, 9549, 9551, 9552, 9553, 9554, 9555, 9558, 9560, 9566, 9567, 9569, 9571, 9572, 9575, 9576, 9578, 9580, 9581, 9582, 9586, 9588, 9589, 9592, 9596, 9598, 9600, 9603, 9607, 9608, 9609, 9610, 9611, 9612, 9614, 9615, 9617, 9619, 9620, 9621, 9622, 9624, 9625, 9626, 9627, 9628, 9631, 9634, 9636, 9637, 9638, 9639, 9640, 9643, 9644, 9646, 9647, 9649, 9657, 9658, 9660, 9662, 9663, 9664, 9665, 9666, 9668, 9669, 9671, 9673, 9674, 9675, 9676, 9677, 9678, 9681, 9685, 9688, 9691, 9692, 9693, 9694, 9695, 9696, 9697, 9700, 9701, 9702, 9703, 9706, 9707, 9710, 9711, 9712, 9715, 9717, 9718, 9721, 9722, 9725, 9730, 9732, 9733, 9735, 9738, 9739, 9740, 9741, 9742, 9743, 9744, 9745, 9746, 9750, and 9751.

13. The biologically active substance of claim 11, comprising or consisting of an amino acid sequence selected from the group consisting of SEQ ID NOs: 4877-9751 and fragments thereof retaining biological activity.

14. The biologically active substance of claim 11, which is encoded by a nucleotide sequence having preferably at least 60% identity, more preferably at least 65% identity, more preferably at least 70% identity, more preferably at least 75% identity, more preferably at least 80% identity, more preferably at least 85% identity, even more preferably at least 90% identity, most preferably at least 95% identity, and even most preferably at least 97% identity with a polynucleotide selected from the group consisting of SEQ ID NOs: 50, 55, 77, 82, 91, 98, 103, 110, 125, 169, 171, 172, 174, 176, 177, 178, 179, 180, 181, 182, 183, 185, 186, 187, 189, 192, 194, 195, 196, 198, 199, 201, 203, 204, 205, 206, 207, 208, 209, 210, 211, 212, 213, 214, 215, 216, 217, 218, 219, 220, 221, 222, 223, 225, 226, 227, 228, 229, 230, 240, 241, 242, 243, 244, 245, 246, 247, 248, 249, 250, 251, 252, 253, 254, 255, 256, 257, 258, 259, 260, 261, 262, 263, 264, 265, 266, 287, 306, 308, 322, 323, 329, 350, 354, 360, 363, 367, 381, 445, 469, 487, 488, 516, 523, 524, 537, 541, 575, 592, 607, 621, 627, 628, 666, 672, 688, 691, 702, 710, 719, 726, 729, 748, 751, 773, 774, 777, 791, 831, 833, 842, 847, 848, 849, 850, 851, 855, 857, 885, 886, 887, 901, 944, 982, 991, 994, 1018, 1032, 1034, 1058, 1078, 1088, 1098, 1108, 1112, 1128, 1148, 1153, 1154, 1158, 1160, 1164, 1175, 1176, 1177, 1180, 1181, 1183, 1184, 1193, 1194, 1199, 1222, 1237, 1244, 1262, 1263, 1265, 1266, 1278, 1302, 1313, 1314, 1315, 1316, 1317, 1318, 1345, 1355, 1373, 1374, 1401, 1424, 1432, 1433, 1440, 1442, 1448, 1455, 1470, 1496, 1497, 1501, 1509, 1527, 1539, 1556, 1565, 1589, 1619, 1629, 1630, 1634, 1635, 1636, 1638, 1639, 1640, 1641, 1642, 1643, 1644, 1645, 1646, 1647, 1649, 1650, 1651, 1652, 1653, 1654, 1655, 1657, 1658, 1659, 1660, 1661, 1662, 1663, 1664, 1665, 1666, 1667, 1668, 1669, 1670, 1671, 1672, 1673, 1675, 1676, 1677, 1679, 1684, 1688, 1692, 1694, 1703, 1704, 1728, 1732, 1734, 1736, 1775, 1783, 1816, 1831, 1838, 1851, 1853, 1866, 1887, 2002, 2007, 2022, 2038, 2044, 2067, 2070, 2072, 2076, 2094, 2106, 2119, 2120, 2121, 2124, 2131, 2139, 2147, 2148, 2166, 2171, 2194, 2196, 2198, 2205, 2211, 2213, 2216, 2221, 2230, 2236, 2240, 2241, 2255, 2258, 2263, 2273, 2277, 2286, 2288, 2289, 2290, 2291, 2292, 2293, 2294, 2295, 2296, 2297, 2298, 2299, 2300, 2301, 2302, 2303, 2304, 2305, 2324, 2327, 2337, 2341, 2347, 2351, 2364, 2381, 2388, 2400, 2403, 2408, 2410, 2411, 2414, 2415, 2424, 2425, 2430, 2435, 2439, 2457, 2467, 2468, 2469, 2483, 2491, 2498, 2504, 2505, 2506, 2507, 2508, 2509, 2510, 2511, 2512, 2514, 2515, 2516, 2517, 2518, 2519, 2520, 2521, 2522, 2524, 2525, 2526, 2527, 2528, 2529, 2530, 2531, 2532, 2533, 2534, 2535, 2536, 2537, 2538, 2539, 2540, 2541, 2542, 2543, 2544, 2545, 2546, 2547, 2548, 2549, 2550, 2551, 2552, 2553, 2554, 2555, 2556, 2557, 2558, 2559, 2560, 2561, 2562, 2564, 2565, 2566, 2567, 2568, 2569, 2570, 2571, 2572, 2573, 2574, 2575, 2576, 2577, 2578, 2579, 2580, 2581, 2582, 2583, 2584, 2585, 2586, 2587, 2588, 2590, 2591, 2592, 2593, 2594, 2595, 2596, 2597, 2598, 2599, 2600, 2601, 2602, 2603, 2604, 2605, 2606, 2607, 2608, 2609, 2610, 2611, 2612, 2613, 2614, 2615, 2616, 2617, 2618, 2619, 2620, 2621, 2622, 2623, 2624, 2625, 2626, 2627, 2628, 2629, 2630, 2631, 2632, 2633, 2634, 2635, 2636, 2637, 2638, 2639, 2640, 2641, 2642, 2643, 2644, 2645, 2646, 2647, 2648, 2649, 2650, 2651, 2652, 2653, 2654, 2655, 2656, 2657, 2658, 2659, 2661, 2664, 2665, 2666, 2667, 2669, 2670, 2671, 2672, 2673, 2674, 2675, 2676, 2677, 2679, 2680, 2681, 2684, 2685, 2686, 2688, 2689, 2690, 2691, 2692, 2693, 2696, 2697, 2698, 2706, 2723, 2730, 2733, 2751, 2762, 2795, 2806, 2813, 2834, 2835, 2841, 2850, 2866, 2867, 2874, 2878, 2898, 2902, 2904, 2917, 2947, 2957, 2960, 2969, 2972, 2973, 2999, 3004, 3019, 3028, 3054, 3064, 3082, 3093, 3123, 3133, 3134, 3135, 3136, 3137, 3138, 3139, 3140, 3141, 3142, 3143, 3144, 3145, 3146, 3147, 3148, 3149, 3150, 3162, 3222, 3224, 3225, 3226, 3227, 3228, 3229, 3231, 3244, 3248, 3259, 3264, 3265, 3287, 3291, 3303, 3310, 3311, 3321, 3324, 3355, 3362, 3371, 3429, 3434, 3442, 3446, 3458, 3471, 3486, 3496, 3499, 3503, 3504, 3508, 3549, 3558, 3577, 3593, 3598, 3601, 3603, 3606, 3611, 3642, 3653, 3655, 3670, 3673, 3682, 3690, 3694, 3707, 3716, 3727, 3736, 3737, 3744, 3753, 3765, 3790, 3798, 3799, , 3872, 3881, 3885, 3915, 3916, 3922, 3931, 3938, 3969, 4011, 4017, 4056, 4059, 4068, 4076, 4077, 4081, 4082, 4085, 4090, 4106, 4112, 4117, 4144, 4145, 4158, 4159, 4160, 4161, 4162, 4163, 4164, 4165, 4166, 4167, 4168, 4169, 4170, 4171, 4172, 4173, 4174, 4175, 4176, 4179, 4181, 4182, 4201, 4235, 4285, 4288, 4305, 4323, 4333, 4336, 4353, 4398, 4409, 4417, 4421, 4423, 4429, 4435, 4448, 4450, 4469, 4472, 4476, 4482, 4488, 4513, 4518, 4544, 4559, 4560, 4569, 4571, 4578, 4607, 4622, 4631, 4643, 4647, 4648, 4650, 4658, 4669, 4672, 4682, 4687, 4688, 4704, 4718, 4754, 4767, 4786, 4792, 4797, 4804, 4812, 4814, 4823, 4830, 4833, 4838, 4848, 4849, 4859, 4862, and 4872; preferably at least 75% identity, more preferably at least 80% identity, more preferably at least 85% identity, even more preferably at least 90% identity, most preferably at least 95% identity, and even most preferably at least 97% identity with a polynucleotide selected from the group consisting of SEQ ID NOs: 909, 911, 1367, 1498, 2663, 2668, 2682, and 2683; preferably at least 80% identity, more preferably at least 85% identity, even more preferably at least 90% identity, most preferably at least 95% identity, and even most preferably at least 97% identity with a polynucleotide selected from the group consisting of SEQ ID NOs: 167, 654, 2513, 2589, and 2694; preferably at least 85% identity, more preferably at least 90% identity, most preferably at least 95% identity, and even most preferably at least 97% identity with a polynucleotide selected from the group consisting of SEQ ID NOs: 163, 184, 910, 1674, 2412, 2660, 2662, 2695, and 3231; preferably at least 90% identity, more preferably at least 95% identity, and most preferably at least 97% identity with a polynucleotide selected from the group consisting of SEQ ID NOs: 27, 166, 168, 170, 193, 202, 231, 543, 776, 888, 889, 890, 898, 908, 916, 1028, 1123, 1133, 1155, 1156, 1157, 1173, 1211, 1212, 1218, 1229, 1230, 1277, 1356, 1357, 1359, 1360, 1368, 1372, 1391, 1500, 1584, 1604, 1627, 1631, 1632, 1637, 1648, 1680, 2101, 2102, 2239, 2358, 2359, 2360, 2404, 2417, 2429, 2494, 2563, 2678, 2724, 3111, 3539, 3850, 4078, 4079, 4080, 4083, 4084, 4180, 4221, 4642, 4689, 4707, and 4727; or preferably at least 95% identity and more preferably at least 97% identity with a polynucleotide selected from the group consisting of SEQ ID NOs: 2, 3, 4, 5, 6, 7, 8, 9, 10, 11, 12, 13, 14, 15, 16, 17, 18, 19, 20, 21, 22, 23, 24, 25, 26, 28, 29, 30, 31, 32, 33, 34, 35, 36, 37, 38, 39, 40, 41, 42, 43, 44, 45, 46, 47, 48, 49, 51, 52, 53, 54, 56, 57, 58, 59, 60, 61, 62, 63, 64, 65, 66, 67, 68, 69, 70, 71, 72, 73, 74, 75, 76, 78, 79, 80, 81, 83, 84, 85, 86, 87, 88, 89, 90, 92, 93, 94, 95, 96, 97, 99, 100, 101, 102, 104, 105, 106, 107, 108, 109, 111, 112, 113, 114, 115, 116, 117, 118, 119, 120, 121, 122, 123, 124, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 164, 165, 173, 175, 188, 190, 191, 197, 200, 224, 232, 233, 234, 235, 236, 237, 238, 239, 267, 268, 269, 270, 271, 272, 273, 274, 275, 276, 277, 278, 279, 280, 281, 282, 283, 284, 285, 286, 288, 289, 290, 291, 292, 293, 294, 295, 296, 297, 298, 299, 300, 301, 302, 303, 304, 305, 307, 309, 310, 311, 312, 313, 314, 315, 316, 317, 318, 319, 320, 321, 324, 325, 326, 327, 328, 330, 331, 332, 333, 334, 335, 336, 337, 338, 339, 340, 341, 342, 343, 344, 345, 346, 347, 348, 349, 351, 352, 353, 355, 356, 357, 358, 359, 361, 362, 364, 365, 366, 368, 369, 370, 371, 372, 373, 374, 375, 376, 377, 378, 379, 380, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 446, 447, 448, 449, 450, 451, 452, 453, 454, 455, 456, 457, 458, 459, 460, 461, 462, 463, 464, 465, 466, 467, 468, 470, 471, 472, 473, 474, 475, 476, 477, 478, 479, 480, 481, 482, 483, 484, 485, 486, 489, 490, 491, 492, 493, 494, 495, 496, 497, 498, 499, 500, 501, 502, 503, 504, 505, 506, 507, 508, 509, 510, 511, 512, 513, 514, 515, 517, 518, 519, 520, 521, 522, 525, 526, 527, 528, 529, 530, 531, 532, 533, 534, 535, 536, 538, 539, 540, 542, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 576, 577, 578, 579, 580, 581, 582, 583, 584, 585, 586, 587, 588, 589, 590, 591, 593, 594, 595, 596, 597, 598, 599, 600, 601, 602, 603, 604, 605, 606, 608, 609, 610, 611, 612, 613, 614, 615, 616, 617, 618, 619, 620, 622, 623, 624, 625, 626, 629, 630, 631, 632, 633, 634, 635, 636, 637, 638, 639, 640, 641, 642, 643, 644, 645, 646, 647, 648, 649, 650, 651, 652, 653, 655, 656, 657, 658, 659, 660, 661, 662, 663, 664, 665, 667, 668, 669, 670, 671, 673, 674, 675, 676, 677, 678, 679, 680, 681, 682, 683, 684, 685, 686, 687, 689, 690, 692, 693, 694, 695, 696, 697, 698, 699, 700, 701, 703, 704, 705, 706, 707, 708, 709, 711, 712, 713, 714, 715, 716, 717, 718, 720, 721, 722, 723, 724, 725, 727, 728, 730, 731, 732, 733, 734, 735, 736, 737, 738, 739, 740, 741, 742, 743, 744, 745, 746, 747, 749, 750, 752, 753, 754, 755, 756, 757, 758, 759, 760, 761, 762, 763, 764, 765, 766, 767, 768, 769, 770, 771, 772, 775, 778, 779, 780, 781, 782, 783, 784, 785, 786, 787, 788, 789, 790, 792, 793, 794, 795, 796, 797, 798, 799, 800, 801, 802, 803, 804, 805, 806, 807, 808, 809, 810, 811, 812, 813, 814, 815, 816, 817, 818, 819, 820, 821, 822, 823, 824, 825, 826, 827, 828, 829, 830, 832, 834, 835, 836, 837, 838, 839, 840, 841, 843, 844, 845, 846, 852, 853, 854, 856, 858, 859, 860, 861, 862, 863, 864, 865, 866, 867, 868, 869, 870, 871, 872, 873, 874, 875, 876, 877, 878, 879, 880, 881, 882, 883, 884, 891, 892, 893, 894, 895, 896, 897, 899, 900, 902, 903, 904, 905, 906, 907, 912, 913, 914, 915, 917, 918, 919, 920, 921, 922, 923, 924, 925, 926, 927, 928, 929, 930, 931, 932, 933, 934, 935, 936, 937, 938, 939, 940, 941, 942, 943, 945, 946, 947, 948, 949, 950, 951, 952, 953, 954, 955, 956, 957, 958, 959, 960, 961, 962, 963, 964, 965, 966, 967, 968, 969, 970, 971, 972, 973, 974, 975, 976, 977, 978, 979, 980, 981, 983, 984, 985, 986, 987, 988, 989, 990, 992, 993, 995, 996, 997, 998, 999, 1000, 1001, 1002, 1003, 1004, 1005, 1006, 1007, 1008, 1009, 1010, 1011, 1012, 1013, 1014, 1015, 1016, 1017, 1019, 1020, 1021, 1022, 1023, 1024, 1025, 1026, 1027, 1029, 1030, 1031, 1033, 1035, 1036, 1037, 1038, 1039, 1040, 1041, 1042, 1043, 1044, 1045, 1046, 1047, 1048, 1049, 1050, 1051, 1052, 1053, 1054, 1055, 1056, 1057, 1059, 1060, 1061, 1062, 1063, 1064, 1065, 1066, 1067, 1068, 1069, 1070, 1071, 1072, 1073, 1074, 1075, 1076, 1077, 1079, 1080, 1081, 1082, 1083, 1084, 1085, 1086, 1087, 1089, 1090, 1091, 1092, 1093, 1094, 1095, 1096, 1097, 1099, 1100, 1101, 1102, 1103, 1104, 1105, 1106, 1107, 1109, 1110, 1111, 1113, 1114, 1115, 1116, 1117, 1118, 1119, 1120, 1121, 1122, 1124, 1125, 1126, 1127, 1129, 1130, 1131, 1132, 1134, 1135, 1136, 1137, 1138, 1139, 1140, 1141, 1142, 1143, 1144, 1145, 1146, 1147, 1149, 1150, 1151, 1152, 1159, 1161, 1162, 1163, 1165, 1166, 1167, 1168, 1169, 1170, 1171, 1172, 1174, 1178, 1179, 1182, 1185, 1186, 1187, 1188, 1189, 1190, 1191, 1192, 1195, 1196, 1197, 1198, 1200, 1201, 1202, 1203, 1204, 1205, 1206, 1207, 1208, 1209, 1210, 1213, 1214, 1215, 1216, 1217, 1219, 1220, 1221, 1223, 1224, 1225, 1226, 1227, 1228, 1231, 1232, 1233, 1234, 1235, 1236, 1238, 1239, 1240, 1241, 1242, 1243, 1245, 1246, 1247, 1248, 1249, 1250, 1251, 1252, 1253, 1254, 1255, 1256, 1257, 1258, 1259, 1260, 1261, 1264, 1267, 1268, 1269, 1270, 1271, 1272, 1273, 1274, 1275, 1276, 1279, 1280, 1281, 1282, 1283, 1284, 1285, 1286, 1287, 1288, 1289, 1290, 1291, 1292, 1293, 1294, 1295, 1296, 1297, 1298, 1299, 1300, 1301, 1303, 1304, 1305, 1306, 1307, 1308, 1309, 1310, 1311, 1312, 1319, 1320, 1321, 1322, 1323, 1324, 1325, 1326, 1327, 1328, 1329, 1330, 1331, 1332, 1333, 1334, 1335, 1336, 1337, 1338, 1339, 1340, 1341, 1342, 1343, 1344, 1346, 1347, 1348, 1349, 1350, 1351, 1352, 1353, 1354, 1358, 1361, 1362, 1363, 1364, 1365, 1366, 1369, 1370, 1371, 1375, 1376, 1377, 1378, 1379, 1380, 1381, 1382, 1383, 1384, 1385, 1386, 1387, 1388, 1389, 1390, 1392, 1393, 1394, 1395, 1396, 1397, 1398, 1399, 1400, 1402, 1403, 1404, 1405, 1406, 1407, 1408, 1409, 1410, 1411, 1412, 1413, 1414, 1415, 1416, 1417, 1418, 1419, 1420, 1421, 1422, 1423, 1425, 1426, 1427, 1428, 1429, 1430, 1431, 1434, 1435, 1436, 1437, 1438, 1439, 1441, 1443, 1444, 1445, 1446, 1447, 1449, 1450, 1451, 1452, 1453, 1454, 1456, 1457, 1458, 1459, 1460, 1461, 1462, 1463, 1464, 1465, 1466, 1467, 1468, 1469, 1471, 1472, 1473, 1474, 1475, 1476, 1477, 1478, 1479, 1480, 1481, 1482, 1483, 1484, 1485, 1486, 1487, 1488, 1489, 1490, 1491, 1492, 1493, 1494, 1495, 1499, 1502, 1503, 1504, 1505, 1506, 1507, 1508, 1510, 1511, 1512, 1513, 1514, 1515, 1516, 1517, 1518, 1519, 1520, 1521, 1522, 1523, 1524, 1525, 1526, 1528, 1529, 1530, 1531, 1532, 1533, 1534, 1535, 1536, 1537, 1538, 1540, 1541, 1542, 1543, 1544, 1545, 1546, 1547, 1548, 1549, 1550, 1551, 1552, 1553, 1554, 1555, 1557, 1558, 1559, 1560, 1561, 1562, 1563, 1564, 1566, 1567, 1568, 1569, 1570, 1571, 1572, 1573, 1574, 1575, 1576, 1577, 1578, 1579, 1580, 1581, 1582, 1583, 1585, 1586, 1587, 1588, 1590, 1591, 1592, 1593, 1594, 1595, 1596, 1597, 1598, 1599, 1600, 1601, 1602, 1603, 1605, 1606, 1607, 1608, 1609, 1610, 1611, 1612, 1613, 1614, 1615, 1616, 1617, 1618, 1620, 1621, 1622, 1623, 1624, 1625, 1626, 1628, 1633, 1656, 1678, 1681, 1682, 1683, 1685, 1686, 1687, 1689, 1690, 1691, 1693, 1695, 1696, 1697, 1698, 1699, 1700, 1701, 1702, 1705, 1706, 1707, 1708, 1709, 1710, 1711, 1712, 1713, 1714, 1715, 1716, 1717, 1718, 1719, 1720, 1721, 1722, 1723, 1724, 1725, 1726, 1727, 1729, 1730, 1731, 1733, 1735, 1737, 1738, 1739, 1740, 1741, 1742, 1743, 1744, 1745, 1746, 1747, 1748, 1749, 1750, 1751, 1752, 1753, 1754, 1755, 1756, 1757, 1758, 1759, 1760, 1761, 1762, 1763, 1764, 1765, 1766, 1767, 1768, 1769, 1770, 1771, 1772, 1773, 1774, 1776, 1777, 1778, 1779, 1780, 1781, 1782, 1784, 1785, 1786, 1787, 1788, 1789, 1790, 1791, 1792, 1793, 1794, 1795, 1796, 1797, 1798, 1799, 1800, 1801, 1802, 1803, 1804, 1805, 1806, 1807, 1808, 1809, 1810, 1811, 1812, 1813, 1814, 1815, 1817, 1818, 1819, 1820, 1821, 1822, 1823, 1824, 1825, 1826, 1827, 1828, 1829, 1830, 1832, 1833, 1834, 1835, 1836, 1837, 1839, 1840, 1841, 1842, 1843, 1844, 1845, 1846, 1847, 1848, 1849, 1850, 1852, 1854, 1855, 1856, 1857, 1858, 1859, 1860, 1861, 1862, 1863, 1864, 1865, 1867, 1868, 1869, 1870, 1871, 1872, 1873, 1874, 1875, 1876, 1877, 1878, 1879, 1880, 1881, 1882, 1883, 1884, 1885, 1886, 1888, 1889, 1890, 1891, 1892, 1893, 1894, 1895, 1896, 1897, 1898, 1899, 1900, 1901, 1902, 1903, 1904, 1905, 1906, 1907, 1908, 1909, 1910, 1911, 1912, 1913, 1914, 1915, 1916, 1917, 1918, 1919, 1920, 1921, 1922, 1923, 1924, 1925, 1926, 1927, 1928, 1929, 1930, 1931, 1932, 1933, 1934, 1935, 1936, 1937, 1938, 1939, 1940, 1941, 1942, 1943, 1944, 1945, 1946, 1947, 1948, 1949, 1950, 1951, 1952, 1953, 1954, 1955, 1956, 1957, 1958, 1959, 1960, 1961, 1962, 1963, 1964, 1965, 1966, 1967, 1968, 1969, 1970, 1971, 1972, 1973, 1974, 1975, 1976, 1977, 1978, 1979, 1980, 1981, 1982, 1983, 1984, 1985, 1986, 1987, 1988, 1989, 1990, 1991, 1992, 1993, 1994, 1995, 1996, 1997, 1998, 1999, 2000, 2001, 2003, 2004, 2005, 2006, 2008, 2009, 2010, 2011, 2012, 2013, 2014, 2015, 2016, 2017, 2018, 2019, 2020, 2021, 2023, 2024, 2025, 2026, 2027, 2028, 2029, 2030, 2031, 2032, 2033, 2034, 2035, 2036, 2037, 2039, 2040, 2041, 2042, 2043, 2045, 2046, 2047, 2048, 2049, 2050, 2051, 2052, 2053, 2054, 2055, 2056, 2057, 2058, 2059, 2060, 2061, 2062, 2063, 2064, 2065, 2066, 2068, 2069, 2071, 2073, 2074, 2075, 2077, 2078, 2079, 2080, 2081, 2082, 2083, 2084, 2085, 2086, 2087, 2088, 2089, 2090, 2091, 2092, 2093, 2095, 2096, 2097, 2098, 2099, 2100, 2103, 2104, 2105, 2107, 2108, 2109, 2110, 2111, 2112, 2113, 2114, 2115, 2116, 2117, 2118, 2122, 2123, 2125, 2126, 2127, 2128, 2129, 2130, 2132, 2133, 2134, 2135, 2136, 2137, 2138, 2140, 2141, 2142, 2143, 2144, 2145, 2146, 2149, 2150, 2151, 2152, 2153, 2154, 2155, 2156, 2157, 2158, 2159, 2160, 2161, 2162, 2163, 2164, 2165, 2167, 2168, 2169, 2170, 2172, 2173, 2174, 2175, 2176, 2177, 2178, 2179, 2180, 2181, 2182, 2183, 2184, 2185, 2186, 2187, 2188, 2189, 2190, 2191, 2192, 2193, 2195, 2197, 2199, 2200, 2201, 2202, 2203, 2204, 2206, 2207, 2208, 2209, 2210, 2212, 2214, 2215, 2217, 2218, 2219, 2220, 2222, 2223, 2224, 2225, 2226, 2227, 2228, 2229, 2231, 2232, 2233, 2234, 2235, 2237, 2238, 2242, 2243, 2244, 2245, 2246, 2247, 2248, 2249, 2250, 2251, 2252, 2253, 2254, 2256, 2257, 2259, 2260, 2261, 2262, 2264, 2265, 2266, 2267, 2268, 2269, 2270, 2271, 2272, 2274, 2275, 2276, 2278, 2279, 2280, 2281, 2282, 2283, 2284, 2285, 2287, 2306, 2307, 2308, 2309, 2310, 2311, 2312, 2313, 2314, 3231, 2316, 2317, 2318, 2319, 2320, 2321, 2322, 2323, 2325, 2326, 2328, 2329, 2330, 2331, 2332, 2333, 2334, 2335, 2336, 2338, 2339, 2340, 2342, 2343, 2344, 2345, 2346, 2348, 2349, 2350, 2352, 2353, 2354, 2355, 2356, 2357, 2361, 2362, 2363, 2365, 2366, 2367, 2368, 2369, 2370, 2371, 2372, 2373, 2374, 2375, 2376, 2377, 2378, 2379, 2380, 2382, 2383, 2384, 2385, 2386, 2387, 2389, 2390, 2391, 2392, 2393, 2394, 2395, 2396, 2397, 2398, 2399, 2401, 2402, 2405, 2406, 2407, 2409, 2413, 2416, 2418, 2419, 2420, 2421, 2422, 2423, 2426, 2427, 2428, 2431, 2432, 2433, 2434, 2436, 2437, 2438, 2440, 2441, 2442, 2443, 2444, 2445, 2446, 2447, 2448, 2449, 2450, 2451, 2452, 2453, 2454, 2455, 2456, 2458, 2459, 2460, 2461, 2462, 2463, 2464, 2465, 2466, 2470, 2471, 2472, 2473, 2474, 2475, 2476, 2477, 2478, 2479, 2480, 2481, 2482, 2484, 2485, 2486, 2487, 2488, 2489, 2490, 2492, 2493, 2495, 2496, 2497, 2499, 2500, 2501, 2502, 2503, 2523, 2687, 3231, 2700, 2701, 2702, 2703, 2704, 2705, 2707, 2708, 2709, 2710, 2711, 2712, 2713, 2714, 2715, 2716, 2717, 2718, 2719, 2720, 2721, 2722, 2725, 2726, 2727, 2728, 2729, 2731, 2732, 2734, 2735, 2736, 2737, 2738, 2739, 2740, 2741, 2742, 2743, 2744, 2745, 2746, 2747, 2748, 2749, 2750, 2752, 2753, 2754, 2755, 2756, 2757, 2758, 2759, 2760, 2761, 2763, 2764, 2765, 2766, 2767, 2768, 2769, 2770, 2771, 2772, 2773, 2774, 2775, 2776, 2777, 2778, 2779, 2780, 2781, 2782, 2783, 2784, 2785, 2786, 2787, 2788, 2789, 2790, 2791, 2792, 2793, 2794, 2796, 2797, 2798, 2799, 2800, 2801, 2802, 2803, 2804, 2805, 2807, 2808, 2809, 2810, 2811, 2812, 2814, 2815, 2816, 2817, 2818, 2819, 2820, 2821, 2822, 2823, 2824, 2825, 2826, 2827, 2828, 2829, 2830, 2831, 2832, 2833, 2836, 2837, 2838, 2839, 2840, 2842, 2843, 2844, 2845, 2846, 2847, 2848, 2849, 2851, 2852, 2853, 2854, 2855, 2856, 2857, 2858, 2859, 2860, 2861, 2862, 2863, 2864, 2865, 2868, 2869, 2870, 2871, 2872, 2873, 2875, 2876, 2877, 2879, 2880, 2881, 2882, 2883, 2884, 2885, 2886, 2887, 2888, 2889, 2890, 2891, 2892, 2893, 2894, 2895, 2896, 2897, 2899, 2900, 2901, 2903, 2905, 2906, 2907, 2908, 2909, 2910, 2911, 2912, 2913, 2914, 2915, 2916, 2918, 2919, 2920, 2921, 2922, 2923, 2924, 2925, 2926, 2927, 2928, 2929, 2930, 2931, 2932, 2933, 2934, 2935, 2936, 2937, 2938, 2939, 2940, 2941, 2942, 2943, 2944, 2945, 2946, 2948, 2949, 2950, 2951, 2952, 2953, 2954, 2955, 2956, 2958, 2959, 2961, 2962, 2963, 2964, 2965, 2966, 2967, 2968, 2970, 2971, 2974, 2975, 2976, 2977, 2978, 2979, 2980, 2981, 2982, 2983, 2984, 2985, 2986, 2987, 2988, 2989, 2990, 2991, 2992, 2993, 2994, 2995, 2996, 2997, 2998, 3000, 3001, 3002, 3003, 3005, 3006, 3007, 3008, 3009, 3010, 3011, 3012, 3013, 3014, 3015, 3016, 3017, 3018, 3020, 3021, 3022, 3023, 3024, 3025, 3026, 3027, 3029, 3030, 3031, 3032, 3033, 3034, 3035, 3036, 3037, 3038, 3039, 3040, 3041, 3042, 3043, 3044, 3045, 3046, 3047, 3048, 3049, 3050, 3051, 3052, 3053, 3055, 3056, 3057, 3058, 3059, 3060, 3061, 3062, 3063, 3065, 3066, 3067, 3068, 3069, 3070, 3071, 3072, 3073, 3074, 3075, 3076, 3077, 3078, 3079, 3080, 3081, 3083, 3084, 3085, 3086, 3087, 3088, 3089, 3090, 3091, 3092, 3094, 3095, 3096, 3097, 3098, 3099, 3100, 3101, 3102, 3103, 3104, 3105, 3106, 3107, 3108, 3109, 3110, 3112, 3113, 3114, 3115, 3116, 3117, 3118, 3119, 3120, 3121, 3122, 3124, 3125, 3126, 3127, 3128, 3129, 3130, 3131, 3132, 3151, 3152, 3153, 3154, 3155, 3156, 3157, 3158, 3159, 3160, 3161, 3163, 3164, 3165, 3166, 3167, 3168, 3169, 3170, 3171, 3172, 3173, 3174, 3175, 3176, 3177, 3178, 3179, 3180, 3181, 3182, 3183, 3184, 3185, 3186, 3187, 3188, 3189, 3190, 3191, 3192, 3193, 3194, 3195, 3196, 3197, 3198, 3199, 3200, 3201, 3202, 3203, 3204, 3205, 3206, 3207, 3208, 3209, 3210, 3211, 3212, 3213, 3214, 3215, 3216, 3217, 3218, 3219, 3220, 3221, 3223, 3232, 3233, 3234, 3235, 3236, 3237, 3238, 3239, 3240, 3241, 3242, 3243, 3245, 3246, 3247, 3249, 3250, 3251, 3252, 3253, 3254, 3255, 3256, 3257, 3258, 3260, 3261, 3262, 3263, 3266, 3267, 3268, 3269, 3270, 3271, 3272, 3273, 3274, 3275, 3276, 3277, 3278, 3279, 3280, 3281, 3282, 3283, 3284, 3285, 3286, 3288, 3289, 3290, 3292, 3293, 3294, 3295, 3296, 3297, 3298, 3299, 3300, 3301, 3302, 3304, 3305, 3306, 3307, 3308, 3309, 3312, 3313, 3314, 3315, 3316, 3317, 3318, 3319, 3320, 3322, 3323, 3325, 3326, 3327, 3328, 3329, 3330, 3331, 3332, 3333, 3334, 3335, 3336, 3337, 3338, 3339, 3340, 3341, 3342, 3343, 3344, 3345, 3346, 3347, 3348, 3349, 3350, 3351, 3352, 3353, 3354, 3356, 3357, 3358, 3359, 3360, 3361, 3363, 3364, 3365, 3366, 3367, 3368, 3369, 3370, 3372, 3373, 3374, 3375, 3376, 3377, 3378, 3379, 3380, 3381, 3382, 3383, 3384, 3385, 3386, 3387, 3388, 3389, 3390, 3391, 3392, 3393, 3394, 3395, 3396, 3397, 3398, 3399, 3400, 3401, 3402, 3403, 3404, 3405, 3406, 3407, 3408, 3409, 3410, 3411, 3412, 3413, 3414, 3415, 3416, 3417, 3418, 3419, 3420, 3421, 3422, 3423, 3424, 3425, 3426, 3427, 3428, 3430, 3431, 3432, 3433, 3435, 3436, 3437, 3438, 3439, 3440, 3441, 3443, 3444, 3445, 3447, 3448, 3449, 3450, 3451, 3452, 3453, 3454, 3455, 3456, 3457, 3459, 3460, 3461, 3462, 3463, 3464, 3465, 3466, 3467, 3468, 3469, 3470, 3472, 3473, 3474, 3475, 3476, 3477, 3478, 3479, 3480, 3481, 3482, 3483, 3484, 3485, 3487, 3488, 3489, 3490, 3491, 3492, 3493, 3494, 3495, 3497, 3498, 3500, 3501, 3502, 3505, 3506, 3507, 3509, 3510, 3511, 3512, 3513, 3514, 3515, 3516, 3517, 3518, 3519, 3520, 3521, 3522, 3523, 3524, 3525, 3526, 3527, 3528, 3529, 3530, 3531, 3532, 3533, 3534, 3535, 3536, 3537, 3538, 3540, 3541, 3542, 3543, 3544, 3545, 3546, 3547, 3548, 3550, 3551, 3552, 3553, 3554, 3555, 3556, 3557, 3559, 3560, 3561, 3562, 3563, 3564, 3565, 3566, 3567, 3568, 3569, 3570, 3571, 3572, 3573, 3574, 3575, 3576, 3578, 3579, 3580, 3581, 3582, 3583, 3584, 3585, 3586, 3587, 3588, 3589, 3590, 3591, 3592, 3594, 3595, 3596, 3597, 3599, 3600, 3602, 3604, 3605, 3607, 3608, 3609, 3610, 3612, 3613, 3614, 3615, 3616, 3617, 3618, 3619, 3620, 3621, 3622, 3623, 3624, 3625, 3626, 3627, 3628, 3629, 3630, 3631, 3632, 3633, 3634, 3635, 3636, 3637, 3638, 3639, 3640, 3641, 3643, 3644, 3645, 3646, 3647, 3648, 3649, 3650, 3651, 3652, 3654, 3656, 3657, 3658, 3659, 3660, 3661, 3662, 3663, 3664, 3665, 3666, 3667, 3668, 3669, 3671, 3672, 3674, 3675, 3676, 3677, 3678, 3679, 3680, 3681, 3683, 3684, 3685, 3686, 3687, 3688, 3689, 3691, 3692, 3693, 3695, 3696, 3697, 3698, 3699, 3700, 3701, 3702, 3703, 3704, 3705, 3706, 3708, 3709, 3710, 3711, 3712, 3713, 3714, 3715, 3717, 3718, 3719, 3720, 3721, 3722, 3723, 3724, 3725, 3726, 3728, 3729, 3730, 3731, 3732, 3733, 3734, 3735, 3738, 3739, 3740, 3741, 3742, 3743, 3745, 3746, 3747, 3748, 3749, 3750, 3751, 3752, 3754, 3755, 3756, 3757, 3758, 3759, 3760, 3761, 3762, 3763, 3764, 3766, 3767, 3768, 3769, 3770, 3771, 3772, 3773, 3774, 3775, 3776, 3777, 3778, 3779, 3780, 3781, 3782, 3783, 3784, 3785, 3786, 3787, 3788, 3789, 4463, 3792, 3793, 3794, 3795, 3796, 3797, 3800, 3801, 3802, 3803, 3804, 3805, 3806, 3807, 3808, 3809, 3810, 3811, 3812, 3813, 3814, 3815, 3816, 3817, 3818, 3819, 3820, 3821, 3822, 3823, 3824, 3825, 3826, 3827, 3828, 3829, 3830, 3831, 3832, 3833, 3834, 3835, 3837, 3838, 3839, 3840, 3841, 3842, 3843, 3844, 3845, 3846, 3847, 3848, 3849, 3851, 3852, 3853, 3854, 3855, 3856, 3857, 3858, 3859, 3860, 3861, 3862, 3863, 3864, 3865, 3866, 3867, 3868, 3869, 3870, 3871, 3873, 3874, 3875, 3876, 3877, 3878, 3879, 3880, 3882, 3883, 3884, 3886, 3887, 3888, 3889, 3890, 3891, 3892, 3893, 3894, 3895, 3896, 3897, 3898, 3899, 3900, 3901, 3902, 3903, 3904, 3905, 3906, 3907, 3908, 3909, 3910, 3911, 3912, 3913, 3914, 3917, 3918, 3919, 3920, 3921, 3923, 3924, 3925, 3926, 3927, 3928, 3929, 3930, 3932, 3933, 3934, 3935, 3936, 3937, 3939, 3940, 3941, 3942, 3943, 3944, 3945, 3946, 3947, 3948, 3949, 3950, 3951, 3952, 3953, 3954, 3955, 3956, 3957, 3958, 3959, 3960, 3961, 3962, 3963, 3964, 3965, 3966, 3967, 3968, 3970, 3971, 3972, 3973, 3974, 3975, 3976, 3977, 3978, 3979, 3980, 3981, 3982, 3983, 3984, 3985, 3986, 3987, 3988, 3989, 3990, 3991, 3992, 3993, 3994, 3995, 3996, 3997, 3998, 3999, 4000, 4001, 4002, 4003, 4004, 4005, 4006, 4007, 4008, 4009, 4010, 4012, 4013, 4014, 4015, 4016, 4018, 4019, 4020, 4021, 4022, 4023, 4024, 4025, 4026, 4027, 4028, 4029, 4030, 4031, 4032, 4033, 4034, 4035, 4036, 4037, 4038, 4039, 4040, 4041, 4042, 4043, 4044, 4045, 4046, 4047, 4048, 4049, 4050, 4051, 4052, 4053, 4054, 4055, 4057, 4058, 4060, 4061, 4062, 4063, 4064, 4065, 4066, 4067, 4069, 4070, 4071, 4072, 4073, 4074, 4075, 4086, 4087, 4088, 4089, 4091, 4092, 4093, 4094, 4095, 4096, 4097, 4098, 4099, 4100, 4101, 4102, 4103, 4104, 4105, 4107, 4108, 4109, 4110, 4111, 4113, 4114, 4115, 4116, 4118, 4119, 4120, 4121, 4122, 4123, 4124, 4125, 4126, 4127, 4128, 4129, 4130, 4131, 4132, 4133, 4134, 4135, 4136, 4137, 4138, 4139, 4140, 4141, 4142, 4143, 4146, 4147, 4148, 4149, 4150, 4151, 4152, 4153, 4154, 4155, 4156, 4157, 4177, 4178, 4183, 4184, 4185, 4186, 4187, 4188, 4189, 4190, 4191, 4192, 4193, 4194, 4195, 4196, 4197, 4198, 4199, 4200, 4202, 4203, 4204, 4205, 4206, 4207, 4208, 4209, 4210, 4211, 4212, 4213, 4214, 4215, 4216, 4217, 4218, 4219, 4220, 4222, 4223, 4224, 4225, 4226, 4227, 4228, 4229, 4230, 4231, 4232, 4233, 4234, 4236, 4237, 4238, 4239, 4240, 4241, 4242, 4243, 4244, 4245, 4246, 4247, 4248, 4249, 4250, 4251, 4252, 4253, 4254, 4255, 4256, 4257, 4258, 4259, 4260, 4261, 4262, 4263, 4264, 4265, 4266, 4267, 4268, 4269, 4270, 4271, 4272, 4273, 4274, 4275, 4276, 4277, 4278, 4279, 4280, 4281, 4282, 4283, 4284, 4286, 4287, 4289, 4290, 4291, 4292, 4293, 4294, 4295, 4296, 4297, 4298, 4299, 4300, 4301, 4302, 4303, 4304, 4306, 4307, 4308, 4309, 4310, 4311, 4312, 4313, 4314, 4315, 4316, 4317, 4318, 4319, 4320, 4321, 4322, 4324, 4325, 4326, 4327, 4328, 4329, 4330, 4331, 4332, 4334, 4335, 4337, 4338, 4339, 4340, 4341, 4342, 4343, 4344, 4345, 4346, 4347, 4348, 4349, 4350, 4351, 4352, 4354, 4355, 4356, 4357, 4358, 4359, 4360, 4361, 4362, 4363, 4364, 4365, 4366, 4367, 4368, 4369, 4370, 4371, 4372, 4373, 4374, 4375, 4376, 4377, 4378, 4379, 4380, 4381, 4382, 4383, 4384, 4385, 4386, 4387, 4388, 4389, 4390, 4391, 4392, 4393, 4394, 4395, 4396, 4397, 4399, 4400, 4401, 4402, 4403, 4404, 4405, 4406, 4407, 4408, 4410, 4411, 4412, 4413, 4414, 4415, 4416, 4418, 4419, 4420, 4422, 4424, 4425, 4426, 4427, 4428, 4430, 4431, 4432, 4433, 4434, 4436, 4437, 4438, 4439, 4440, 4441, 4442, 4443, 4444, 4445, 4446, 4447, 4449, 4451, 4452, 4453, 4454, 4455, 4456, 4457, 4458, 4459, 4460, 4461, 4462, 4463, 4464, 4465, 4466, 4467, 4468, 4470, 4471, 4473, 4474, 4475, 4477, 4478, 4479, 4480, 4481, 4483, 4484, 4485, 4486, 4487, 4489, 4490, 4491, 4492, 4493, 4494, 4495, 4496, 4497, 4498, 4499, 4500, 4501, 4502, 4503, 4504, 4505, 4506, 4507, 4508, 4509, 4510, 4511, 4512, 4514, 4515, 4516, 4517, 4519, 4520, 4521, 4522, 4523, 4524, 4525, 4526, 4527, 4528, 4529, 4530, 4531, 4532, 4533, 4534, 4535, 4536, 4537, 4538, 4539, 4540, 4541, 4542, 4543, 4545, 4546, 4547, 4548, 4549, 4550, 4551, 4552, 4553, 4554, 4555, 4556, 4557, 4558, 4561, 4562, 4563, 4564, 4565, 4566, 4567, 4568, 4570, 4572, 4573, 4574, 4575, 4576, 4577, 4579, 4580, 4581, 4582, 4583, 4584, 4585, 4586, 4587, 4588, 4589, 4590, 4591, 4592, 4593, 4594, 4595, 4596, 4597, 4598, 4599, 4600, 4601, 4602, 4603, 4604, 4605, 4606, 4608, 4609, 4610, 4611, 4612, 4613, 4614, 4615, 4616, 4617, 4618, 4619, 4620, 4621, 4623, 4624, 4625, 4626, 4627, 4628, 4629, 4630, 4632, 4633, 4634, 4635, 4636, 4637, 4638, 4639, 4640, 4641, 4644, 4645, 4646, 4649, 4651, 4652, 4653, 4654, 4655, 4656, 4657, 4659, 4660, 4661, 4662, 4663, 4664, 4665, 4666, 4667, 4668, 4670, 4671, 4673, 4674, 4675, 4676, 4677, 4678, 4679, 4680, 4681, 4683, 4684, 4685, 4686, 4690, 4691, 4692, 4693, 4694, 4695, 4696, 4697, 4698, 4699, 4700, 4701, 4702, 4703, 4705, 4706, 4708, 4709, 4710, 4711, 4712, 4713, 4714, 4715, 4716, 4717, 4719, 4720, 4721, 4722, 4723, 4724, 4725, 4726, 4728, 4729, 4730, 4731, 4732, 4733, 4734, 4735, 4736, 4737, 4738, 4739, 4740, 4741, 4742, 4743, 4744, 4745, 4746, 4747, 4748, 4749, 4750, 4751, 4752, 4753, 4755, 4756, 4757, 4758, 4759, 4760, 4761, 4762, 4763, 4764, 4765, 4766, 4768, 4769, 4770, 4771, 4772, 4773, 4774, 4775, 4776, 4777, 4778, 4779, 4780, 4781, 4782, 4783, 4784, 4785, 4787, 4788, 4789, 4790, 4791, 4793, 4794, 4795, 4796, 4798, 4799, 4800, 4801, 4802, 4803, 4805, 4806, 4807, 4808, 4809, 4810, 4811, 4813, 4815, 4816, 4817, 4818, 4819, 4820, 4821, 4822, 4824, 4825, 4826, 4827, 4828, 4829, 4831, 4832, 4834, 4835, 4836, 4837, 4839, 4840, 4841, 4842, 4843, 4844, 4845, 4846, 4847, 4850, 4851, 4852, 4853, 4854, 4855, 4856, 4857, 4858, 4860, 4861, 4863, 4864, 4865, 4866, 4867, 4868, 4869, 4870, 4871, 4873, 4874, 4875, and 4876.

15. The biologically active substance of claim 11, which is encoded by a polynucleotide comprising a nucleotide sequence that hybridizes under preferably at least medium stringency conditions, more preferably at least medium-high stringency conditions, or most preferably at least high stringency conditions with a polynucleotide selected from the group consisting of SEQ ID NOs: 2-4876 and full-length complementary strands thereof.

16. The biologically active substance of claim 11, which is an artificial variant comprising a substitution, deletion, and/or insertion of one or more amino acids of an amino acid sequence selected from the group consisting of SEQ ID NOs: 4877-9751 and fragments thereof retaining biological activity.

17. The biologically active substance of any of claims 11-16, which is encoded by a polynucleotide contained in the chromosome of *Bacillus licheniformis* SJ1904 (ATCC PTA-7992).

18. A method for producing the biologically active substance of any of claims 11-17 comprising (a) cultivating a strain, which in its wild-type form is capable of producing the biologically active substance, under conditions conducive for production of the biologically active substance; and (b) recovering the biologically active substance.

19. A method for producing the biologically active substance of any of claims 11-17, comprising (a) cultivating a host cell comprising a nucleic acid construct comprising a polynucleotide encoding the biologically active substance under conditions conducive for production of the biologically active substance; and (b) recovering the biologically active substance.

20. A method for producing a mutant of a parent cell, which comprises disrupting or deleting the polynucleotide of any of claims 1-7, which results in the mutant producing less of the biologically active substance than the parent cell.

21. A mutant cell produced by the method of claim 20.

22. The mutant cell of claim 21, which further comprises a nucleotide sequence encoding a native or heterologous protein.

23. A method for producing a protein comprising (a) cultivating the mutant cell of claim 22 under conditions conducive for production of the protein; and (b) recovering the protein.

24. An isolated polynucleotide obtained by (a) hybridizing a population of DNA under preferably at least medium stringency conditions, more preferably at least medium-high stringency conditions, or most preferably at least high stringency conditions with a polynucleotide selected from the group consisting of SEQ ID NOs: 2-4876 and full-length complementary strands thereof; and (b) isolating the hybridizing nucleotide sequence, which encodes a biologically active substance.

25. A transgenic plant, plant part or plant cell, which has been transformed with the isolated polynucleotide of any of claims 1-7.

26. A double-stranded inhibitory RNA (dsRNA) molecule comprising a subsequence of the polynucleotide of any of claims 1-7, wherein optionally the dsRNA is a siRNA or a miRNA molecule.

27. The double-stranded inhibitory RNA (dsRNA) molecule of claim 26, which is about 15, 16, 17, 18, 19, 20, 21, 22, 23, 24, 25 or more duplex nucleotides in length.

28. A method of inhibiting the expression of a polypeptide in a cell, comprising administering to the cell or expressing in the cell a double-stranded RNA (dsRNA) molecule, wherein the dsRNA comprises a subsequence of the polynucleotide of any of claims 1-7.

29. The method of claim 28, wherein the dsRNA is about 15, 16, 17, 18, 19, 20, 21, 22, 23, 24, 25 or more duplex nucleotides in length.

30. A method for isolating a gene encoding an enzyme, comprising:

(a) adding a mixture of labeled first nucleic acid probes, isolated from a microbial strain cultured on medium without an inducing substrate, and labeled second nucleic acid probes, isolated from the microbial strain cultured on medium with the inducing substrate, to an array of *Bacillus licheniformis* polynucleotides selected from the group consisting of SEQ ID NOs: 2-4876, full-length complementary strands of SEQ ID NOs: 2-4876, and fragments of SEQ ID NOs: 2-4876, under conditions where the labeled nucleic acids hybridize to full-length complementary sequences of the *Bacillus licheniformis* polynucleotides on the array, wherein the first nucleic acid probes are labeled with a first reporter and the second nucleic acid probes are labeled with a second reporter;
(b) examining the array under conditions wherein the relative expression of the genes of the microbial strain is determined by the observed hybridization reporter signal of each spot on the array in which (i) the *Bacillus licheniformis* polynucleotides on the array that hybridize to the first nucleic acid probes produce a distinct first hybridization reporter signal or to the second nucleic acid probes produce a distinct second hybridization reporter signal indicating induction of a gene by the presence of the inducing substrate in the culture medium of the microorganism, and (ii) the *Bacillus licheniformis* polynucleotides on the array that hybridize to both the first and second nucleic acid probes produce a distinct combined hybridization reporter signal; and
(c) isolating from the microbial strain a gene induced by the presence of the substrate, wherein the gene encodes an enzyme that is involved in the degradtion or conversion of the substrate.

31. The method of claim 30, wherein the inducing substrate is selected from the group consisting of cellulose, hemicellulose, lignin, phytic acid, starch, pectin, and protein.

32. The method of claim 30, wherein the enzyme is an oxidoreductase, transferase, hydrolase, lyase, isomerase, or ligase.

33. The method of claim 30, wherein the microbial strain is a bacterium.

34. The method of claim 33, wherein the bacterium is a *Bacillus, Pseudomonas, Streptococcus,* or *Streptomyces* strain.

35. A method for monitoring differential expression of a plurality of genes in a first bacterial cell relative to expression of the same genes in one or more second bacterial cells, comprising:

(a) adding a mixture of detection reporter-labeled nucleic acids isolated from the bacterial cells to a substrate containing an array of *Bacillus licheniformis* polynucleotides selected from the group consisting of SEQ ID NOs: 2-4876, full-length complementary strands of SEQ ID NOs: 2-4876, and fragments of SEQ ID NOs: 2-4876, under conditions where the detection reporter-labeled nucleic acids hybridize to full-length complementary sequences of the *Bacillus licheniformis* polynucleotides on the array, wherein the nucleic acids from the first bacterial cell and the one or more second bacterial cells are labeled with a first detection reporter and one or more different second detection reporters, respectively; and
(b) examining the array under conditions wherein the relative expression of the genes in the bacterial cells is determined by the observed detection signal of each spot on the array in which (i) the *Bacillus licheniformis* polynucleotides on the array that hybridize to the nucleic acids obtained from either the first or the one or more second bacterial cells produce a distinct first detection signal or one or more second detection signals, respectively, and (ii) the *Bacillus licheniformis* polynucleotides on the array that hybridize to the nucleic acids obtained from both the first and one or more second bacterial produce a distinct combined detection signal.

36. The method of claim 35, wherein one or more bacterial cells are selected from the group consisting of a *Bacillus, Pseudomonas, Streptococcus,* and *Streptomyces* cell.

37. The method of claim 35, wherein the two or more bacterial cells are the same cell.

38. The method of claim 35, wherein the two or more bacterial cells are different cells.

39. A computer readable medium having recorded thereon an array of *Bacillus licheniformis* polynucleotides selected from the group consisting of SEQ ID NOs: 2-4876, full-length complementary strands of SEQ ID NOs: 2-4876, and fragments of SEQ ID NOs: 2-4876, for monitoring differential expression of a plurality of genes in a first bacterial cell relative to expression of the same genes in one or more second bacterial cells.

40. The computer readable medium of claim 39, wherein one or more bacterial cells are selected from the group consisting of a *Bacillus, Pseudomonas, Streptococcus,* and *Streptomyces* cell.

41. The computer readable medium of claim 39, wherein the two or more bacterial cells are the same cell.

42. The computer readable medium of claim 39, wherein the two or more bacterial cells are different cells.

43. The computer readable medium of claim 39, wherein the medium is selected from the group consisting of a floppy dick, a hard disk, random access memeory (RAM), read only memory (ROM), and CD-ROM.

44. A computer-based system for monitoring differential expression of a plurality of genes in a first bacterial cell relative to expression of the same genes in one or more second bacterial cells comprising the following elements:

(a) a data storage means comprising *Bacillus licheniformis* polynucleotides selected from the group consisting of SEQ ID NOs: 2-4876, full-length complementary strands of SEQ ID NOs: 2-4876, and fragments of SEQ ID NOs: 2-4876;

(b) a search means for comparing a target sequence to a *Bacillus licheniformis* polynucleotide sequence of the data storage means of step (a) to identify homologous sequences; and

(c) a retrieval means for obtaining the homologous sequence(s) of step (b).

45. A substrate comprising an array of *Bacillus licheniformis* polynucleotides selected from the group consisting of SEQ ID NOs: 2-4876, full-length complementary strands of SEQ ID NOs: 2-4876, and fragments of SEQ ID NOs: 2-4876, for monitoring differential expression of a plurality of genes in a first bacterial cell relative to expression of the same genes in one or more second bacterial cells.

46. A method for preparing a synthetic gene, comprising:

(a) generating a codon usage table based on codons used in one or more open reading frames or portions thereof of SEQ ID NO: 1;

(b) constructing a synthetic gene or portion thereof that contains in place of one or more native codons one or more preferred codons from the codon usage table; and

(c) recovering the synthetic gene.

47. The method of claim 46, wherein the codon usage table is Table 5.

48. A synthetic gene obtained by the method of claim 46.

49. A nucleic acid construct comprising the synthetic gene of claim 48 operably linked to one or more control sequences that direct the expression of the synthetic gene in an expression host.

50. A recombinant expression vector comprising the nucleic acid construct of claim 49.

51. A recombinant host cell comprising the nucleic acid construct of claim 49.

52. A method for producing a polypeptide encoded by the synthetic gene of claim 48 comprising (a) cultivating a host cell comprising the synthetic gene under conditions conducive for production of the polypeptide; and (b) recovering the polypeptide.

53. An isolated polynucleotide of the complete chromosomal DNA molecule of *Bacillus licheniformis* SJ1904 (ATCC PTA-7992) having the nucleotide sequence of SEQ ID NO: 1.

**Claims**

**1.** An isolated polynucleotide encoding a biologically active substance, selected from the group consisting of:

(a) a polynucleotide comprising a nucleotide sequence having preferably at least 60% identity, more preferably at least 65% identity, more preferably at least 70% identity, more preferably at least 75% identity, more preferably at least 80% identity, more preferably at least 85% identity, more preferably at least 90% identity, even more preferably at least 90% identity, most preferably at least 95% identity, and even most preferably at least 97% identity with SEQ ID NO: 885 and subsequences thereof encoding fragments having biological activity;

(b) a polynucleotide comprising a nucleotide sequence that hybridizes under preferably at least medium stringency conditions, more preferably at least medium-high stringency conditions, and most preferably at least high stringency conditions with the polynucleotide of SEQ ID NO: 885 or a full-length complementary strand thereof;

(c) a polynucleotide encoding a biologically active substance comprising an amino acid sequence having preferably at least 60% identity, more preferably at least 65% identity, more preferably at least 70% identity, more preferably at least 75% identity, more preferably at least 80% identity, more preferably at least 85% identity, more preferably at least 90% identity, even more preferably at least 90% identity, most preferably at least 95% identity, and even most preferably at least 97% identity with the amino acid sequence of SEQ ID NO: 5760 or fragments thereof retaining biological activity;

(d) a polynucleotide encoding an artificial variant comprising a substitution, deletion, and/or insertion of one or more amino acids of the amino acid sequence of SEQ ID NO: 5760 or fragments thereof retaining biological activity; and

(e) a polynucleotide comprising or consisting of SEQ ID NO: 885.

2. The isolated polynucleotide of claim 1, which is contained in the chomosome of *Bacillus licheniformis* SJ1904 (ATCC PTA-7992).

3. A recombinant host cell comprising the isolated polynucleotide of claim 1.

4. An isolated biologically active substance, selected from the group consisting of:

(a) a biologically active substance comprising an amino acid sequence having preferably at least 60% identity, more preferably at least 65% identity, more preferably at least 70% identity, more preferably at least 75% identity, more preferably at least 80% identity, more preferably at least 85% identity, more preferably at least 90% identity, even more preferably at least 90% identity, most preferably at least 95% identity, and even most preferably at least 97% identity with the amino acid sequence of SEQ ID NO: 5760 or fragments thereof retaining biological activity;

(b) a biologically active substance encoded by a polynucleotide comprising a nucleotide sequence having preferably at least 60% identity, more preferably at least 65% identity, more preferably at least 70% identity, more preferably at least 75% identity, more preferably at least 80% identity, more preferably at least 85% identity, more preferably at least 90% identity, even more preferably at least 90% identity, most preferably at least 95% identity, and even most preferably at least 97% identity with the polynucleotide of SEQ ID NO: 885 or subsequences thereof encoding fragments having biological activity;

(c) a biologically active substance encoded by a polynucleotide comprising a nucleotide sequence that hybridizes under preferably at least medium stringency conditions, more preferably at least medium-high stringency conditions, and most preferably at least high stringency conditions with the polynucleotide of SEQ ID NO: 885 or the full-length complementary strand thereof;

(d) an artificial variant comprising a substitution, deletion, and/or insertion of one or more amino acids of the amino acid sequence of SEQ ID NO: 5760 or fragments thereof retaining biological activity; and

(e) a biologically active substance comprising or consisting of the amino acid sequence of SEQ ID NO: 5760 or fragments thereof retaining biological activity.

5. The biologically active substance of claim 4, which is encoded by a polynucleotide contained in the chromosome of *Bacillus licheniformis* SJ1904 (ATCC PTA-7992).

6. A method for producing the biologically active substance of claim 4, comprising (a) cultivating a host cell comprising a nucleic acid construct comprising a polynucleotide encoding the biologically active substance under conditions conducive for production of the biologically active substance; and (b) recovering the biologically active substance.

7. A method for producing a mutant of a parent cell, which comprises disrupting or deleting the polynucleotide of claim 1, which results in the mutant producing less of the biologically active substance than the parent cell.

8. A mutant cell produced by the method of claim 7.

9. A transgenic plant, plant part or plant cell, which has been transformed with the isolated polynucleotide of claim 1.

10. A double-stranded inhibitory RNA (dsRNA) molecule comprising a subsequence of the polynucleotide of claim 1, wherein optionally the dsRNA is a siRNA or a miRNA molecule.

11. A method of inhibiting the expression of a polypeptide having biological activity in a cell, comprising administering to the cell or expressing in the cell a double-stranded RNA (dsRNA) molecule, wherein the dsRNA comprises a subsequence of the polynucleotide of claim 1.

12. A method for isolating a gene encoding an enzyme, comprising:

(a) adding a mixture of labeled first nucleic acid probes, isolated from a microbial strain cultured on medium without an inducing substrate, and labeled second nucleic acid probes, isolated from the microbial strain cultured on medium with the inducing substrate, to an array comprising the *Bacillus licheniformis* polynucleotide of claim 1, under conditions where the labeled nucleic acids hybridize to full-length complementary sequences of the *Bacillus licheniformis* polynucleotides on the array, wherein the first nucleic acid probes are labeled with a first reporter and the second nucleic acid probes are labeled with a second reporter;
(b) examining the array under conditions wherein the relative expression of the genes of the microbial strain is determined by the observed hybridization reporter signal of each spot on the array in which (i) the *Bacillus licheniformis* polynucleotides on the array that hybridize to the first nucleic acid probes produce a distinct first hybridization reporter signal or to the second nucleic acid probes produce a distinct second hybridization reporter signal indicating induction of a gene by the presence of the inducing substrate in the culture medium of the microorganism, and (ii) the *Bacillus licheniformis* polynucleotides on the array that hybridize to both the first and second nucleic acid probes produce a distinct combined hybridization reporter signal; and
(c) isolating from the microbial strain a gene induced by the presence of the substrate, wherein the gene encodes an enzyme that is involved in the degradation or conversion of the substrate.

13. A method for monitoring differential expression of a plurality of genes in a first bacterial cell relative to expression of the same genes in one or more second bacterial cells, comprising:

(a) adding a mixture of detection reporter-labeled nucleic acids isolated from the bacterial cells to a substrate containing an array comprising a *Bacillus licheniformis* polynucleotide of claim 1, under conditions where the detection reporter-labeled nucleic acids hybridize to full-length complementary sequences of the *Bacillus licheniformis* polynucleotides on the array, wherein the nucleic acids from the first bacterial cell and the one or more second bacterial cells are labeled with a first detection reporter and one or more different second detection reporters, respectively; and
(b) examining the array under conditions wherein the relative expression of the genes in the bacterial cells is determined by the observed detection signal of each spot on the array in which (i) the *Bacillus licheniformis* polynucleotides on the array that hybridize to the nucleic acids obtained from either the first or the one or more second bacterial cells produce a distinct first detection signal or one or more second detection signals, respectively, and (ii) the *Bacillus licheniformis* polynucleotides on the array that hybridize to the nucleic acids obtained from both the first and one or more second bacterial produce a distinct combined detection signal.

14. A computer readable medium having recorded thereon an array comprising a *Bacillus licheniformis* polynucleotide of claim 1, for monitoring differential expression of a plurality of genes in a first bacterial cell relative to expression of the same genes in one or more second bacterial cells.

15. A computer-based system for monitoring differential expression of a plurality of genes in a first bacterial cell relative to expression of the same genes in one or more second bacterial cells comprising the following elements:

(a) a data storage means comprising a *Bacillus licheniformis* polynucleotide of claim 1;
(b) a search means for comparing a target sequence to a *Bacillus licheniformis* polynucleotide sequence of the data storage means of step (a) to identify homologous sequences; and
(c) a retrieval means for obtaining the homologous sequence(s) of step (b).

16. A substrate comprising an array comprising a *Bacillus licheniformis* polynucleotide of claim 1, for monitoring differential expression of a plurality of genes in a first bacterial cell relative to expression of the same genes in one or more second bacterial cells.

**Europäisches Patentamt**
**European Patent Office**
**Office européen des brevets**

# EUROPEAN SEARCH REPORT

Application Number

EP 10 15 9320

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE Geneseq [Online] 24 February 2005 (2005-02-24), "Mouse inositol-1,4,5-triphosphate receptor CTT domain." XP002585283 retrieved from EBI accession no. GSP:ADU97725 Database accession no. ADU97725 * the whole document * ----- | 1-16 | INV. C07K14/32 C07K14/195 C12N15/31 |
| X | WO 2004/104038 A1 (JAPAN SCIENCE & TECH AGENCY [JP]; MIKOSHIBA KATSUHIKO [JP]; ZHANG SONG) 2 December 2004 (2004-12-02) * the whole document * ----- | 1-16 | |

TECHNICAL FIELDS SEARCHED (IPC)

C07K
C12N

The present search report has been drawn up for all claims

| Place of search | Date of completion of the search | Examiner |
|---|---|---|
| Munich | 2 June 2010 | Grosskopf, Ruediger |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 10 15 9320

This annex lists the patent family members relating to the patent documents cited in the above-mentioned European search report.
The members are as contained in the European Patent Office EDP file on
The European Patent Office is in no way liable for these particulars which are merely given for the purpose of information.

02-06-2010

| Patent document cited in search report | Publication date | Patent family member(s) | Publication date |
|---|---|---|---|
| WO 2004104038 A1 | 02-12-2004 | JP 2005035890 A | 10-02-2005 |

For more details about this annex : see Official Journal of the European Patent Office, No. 12/82

## REFERENCES CITED IN THE DESCRIPTION

### Patent documents cited in the description

- US 5589381 A
- US 5665354 A
- WO 9517413 A
- WO 9522625 A
- US 5223409 A
- WO 9206204 A
- WO 9114772 A
- US 6506559 B
- US 6511824 B
- US 6515109 B
- US 6489127 B
- US 5807522 A
- US 5700637 A
- US 5770151 A
- US 5770367 A

### Non-patent literature cited in the description

- **Claus, D. ; Berkeley, R.C.W. et al.** Bergey's Manual of Systematic Bacteriology. Williams and Wilkins Co, 1986, vol. 2, 1105-1139
- **Alexander, M.** Introduction to Soil Microbiology. John Wiley and Sons, Inc, 1977
- **Eveleigh, D.E.** *Scientific American,* 1981, vol. 245, 155-178
- **Erickson, R.J.** Microbiology. Am. Soc. Microbiol, 1976, 406-419
- **Logan, N.A. ; Berkeley, R.C.W.** The Aerobic Endospore-Forming Bacteria: Classification and Identification. Academic Press, Inc, 1981, 106-140
- **O'Donnell, A.G. ; Norris, J.R. ; Berkeley, R.C.W. ; Claus, D. ; Kanero, T. ; Logan, N.A. ; Nozaki, R.** *Internat. J. Systematic Bacteriol.,* 1980, vol. 30, 448-459
- **Lapidus, A. ; Galleron, N. ; Andersen, J.T. ; Jørgensen, P.L. ; Ehrlich, S.D. ; Sorokin, A.** *FEMS Microbiol. Lett.,* 2002, vol. 209, 23-30
- **Rey, M.W ; Ramaiya, P ; Nelson, B.A. ; Brody-Karpin, S.D. ; Zaretsky, E.J. ; Tang, M. ; Lopez de Leon, A. ; Xiang, H. ; Gusti, V. ; Clausen, I.G.** Complete genome sequence of the industrial bacterium Bacillus licheniformis and comparisons with closely related Bacillus species. *Genome Biol.,* 2004, vol. 5, R77
- **Welch et al.** *Proc. Nat. Acad. Sci. USA,* 2002, vol. 99, 17020-17024
- **Perna et al.** *Nature,* 2001, vol. 409, 529-533
- **Hayashi et al.** *DNA Res.,* 2001, vol. 8, 11-22
- **Blattner et al.** *Science,* 1997, vol. 277, 1453-1474
- **Brzuszkiewicz et al.** *Proc. Nat. Acad. Sci. USA,* 2006, vol. 103, 12879-12884
- **Carpenter ; Sabatini.** *Nature,* 2004, vol. 5, 11-22
- **Sordie et al.** *Proceedings of the National Academy of Sciences USA,* 2003, vol. 100, 11964-11969
- **LaBaer.** *TRENDS in Biotechnology,* 2003, vol. 21, 383-388
- **Kaberdin ; McDowall.** *Genome Research,* 2003, vol. 13, 1961-1965
- **Altschul et al.** *Journal of Molecular Biology,* 1990, vol. 215, 403-410
- **J. Sambrook ; E.F. Fritsch ; T. Maniatus.** Molecular Cloning, A Laboratory Manual. Cold Spring Harbor, 1989
- **Bolton ; McCarthy.** *Proceedings of the National Academy of Sciences USA,* 1962, vol. 48, 1390
- **H. Neurath ; R.L. Hill.** The Proteins. Academic Press, 1979
- **Cunningham ; Wells.** *Science,* 1989, vol. 244, 1081-1085
- **Hilton et al.** *J. Biol. Chem.,* 1996, vol. 271, 4699-4708
- **de Vos et al.** *Science,* 1992, vol. 255, 306-312
- **Smith et al.** *J. Mol. Biol.,* 1992, vol. 224, 899-904
- **Wlodaver et al.** *FEBS Lett.,* 1992, vol. 309, 59-64
- **Reidhaar-Olson ; Sauer.** *Science,* 1988, vol. 241, 53-57
- **Bowie ; Sauer.** *Proc. Natl. Acad. Sci. USA,* 1989, vol. 86, 2152-2156
- **Lowman et al.** *Biochem.,* 1991, vol. 30, 10832-10837
- **Derbyshire et al.** *Gene,* 1986, vol. 46, 145
- **Ner et al.** *DNA,* 1988, vol. 7, 127
- **Ness et al.** *Nature Biotechnology,* 1999, vol. 17, 893-896

- **Villa-Kamaroff et al.** *Proceedings of the National Academy of Sciences USA,* 1978, vol. 75, 3727-3731
- **DeBoer et al.** *Proceedings of the National Academy of Sciences USA,* 1983, vol. 80, 21-25
- Useful proteins from recombinant bacteria. *Scientific American,* 1980, vol. 242, 74-94
- **Simonen ; Palva.** *Microbiological Reviews,* 1993, vol. 57, 109-137
- **Chang ; Cohen.** *Molecular General Genetics,* 1979, vol. 168, 111-115
- **Young ; Spizizen.** *Journal of Bacteriology,* 1961, vol. 81, 823-829
- **Dubnau ; Davidoff-Abelson.** *Journal of Molecular Biology,* 1971, vol. 56, 209-221
- **Shigekawa ; Dower.** *Biotechniques,* 1988, vol. 6, 742-751
- **Koehler ; Thorne.** *Journal of Bacteriology,* 1987, vol. 169, 5271-5278
- **Hanahan.** *J. Mol. Biol.,* 1983, vol. 166, 557-580
- **Dower et al.** *Nucleic Acids Res.,* 1988, vol. 16, 6127-6145
- **Gong et al.** *Folia Microbiol.,* 2004, vol. 49, 399-405
- **Mazodier et al.** *J. Bacteriol.,* 1989, vol. 171, 3583-3585
- **Burke et al.** *Proc. Natl. Acad. Sci. USA,* 2001, vol. 98, 6289-6294
- **Choi et al.** *J. Microbiol. Methods,* 2006, vol. 64, 391-397
- **Pinedo ; Smets.** *Appl. Environ. Microbiol.,* 2005, vol. 71, 51-57
- **Perry ; Kuramitsu.** *Infect. Immun.,* 1981, vol. 32, 1295-1297
- **Catt ; Jollick.** *Microbios,* 1991, vol. 68, 189-2070
- **Buckley et al.** *Appl. Environ. Microbiol.,* 1999, vol. 65, 3800-3804
- **Clewell.** *Microbiol. Rev.,* 1981, vol. 45, 409-436
- Enzyme Nomenclature. Academic Press, Inc, 2007
- Protein Purification. VCH Publishers, 1989
- **Tague et al.** *Plant Physiology,* 1988, vol. 86, 506
- **Franck et al.** *Cell,* 1980, vol. 21, 285-294
- **Christensen AH ; Sharrock RA ; Quail.** *Plant Mo. Biol.,* 1992, vol. 18, 675-689
- **Zhang W ; McElroy D. ; Wu R.** *Plant Cell,* 1991, vol. 3, 1155-1165
- **Edwards ; Coruzzi.** *Ann. Rev. Genet.,* 1990, vol. 24, 275-303
- **Ito et al.** *Plant Mol. Biol.,* 1994, vol. 24, 863-878
- **Wu et al.** *Plant and Cell Physiology,* 1998, vol. 39, 885-889
- **Conrad et al.** *Journal of Plant Physiology,* 1998, vol. 152, 708-711
- **Chen et al.** *Plant and Cell Physiology,* 1998, vol. 39, 935-941
- **Kyozuka et al.** *Plant Physiology,* 1993, vol. 102, 991-1000
- **Mitra ; Higgins.** *Plant Molecular Biology,* 1994, vol. 26, 85-93
- **Kagaya et al.** *Molecular and General Genetics,* 1995, vol. 248, 668-674
- **Xu et al.** *Plant Molecular Biology,* 1993, vol. 22, 573-588
- **Gasser et al.** *Science,* 1990, vol. 244, 1293
- **Potrykus.** *Bio/Technology,* 1990, vol. 8, 535
- **Shimamoto et al.** *Nature,* 1989, vol. 338, 274
- **Hooykas ; Schilperoort.** *Plant Molecular Biology,* 1992, vol. 19, 15-38
- **Christou.** *Plant Journal,* 1992, vol. 2, 275-281
- **Shimamoto.** *Current Opinion Biotechnology,* 1994, vol. 5, 158-162
- **Vasil et al.** *Bio/Technology,* 1992, vol. 10, 667-674
- **Omirulleh et al.** *Plant Molecular Biology,* 1993, vol. 21, 415-428
- **Danchin, A.** The bag or the spindle: the cell factory at the time of systems' biology. *Microb. Cell Fact.,* 2004, vol. 3, 13
- **Venter et al.** *Proc. Nat. Acad. Sci. USA,* 2003, vol. 100, 15440-15445
- **Schena et al.** *Proceedings of the National Academy of Science USA,* 1996, vol. 93, 10614-10619
- **Heller.** *Proceedings of the National Academy of Science USA,* 1997, vol. 94, 2150-2155
- **Chen et al.** *Genomics,* 1998, vol. 51, 313-324
- **DeRisi et al.** *Science,* 1997, vol. 278, 680-686
- **Eisen ; Brown.** *Methods of Enzymology,* 1999, vol. 303, 179-205
- **Kane et al.** *Nucleic Acids Research,* 2000, vol. 28, 4552-4557
- **Cheung et al.** *Nat. Genet.,* 1998, vol. 18, 225-230
- **Chen et al.** *Journal of Biomedical Optics,* 1997, vol. 2, 364-374
- **Eisen et al.** *Proc. Nat. Acad. Sci. USA,* 1998, vol. 95, 14863-14868
- **Spellman et al.** *Mol. Biol. Cell,* 1998, vol. 9, 3273-3297
- **Chu et al.** *Science,* 1998, vol. 282, 699-705
- **Tusher et al.** *Proc. Natl. Acad. Sci. USA,* 2001, vol. 98, 5116-5121
- **Tibshirani et al.** *Proc. Natl. Acad. Sci. USA,* 2002, vol. 99, 6567-6572
- **Tamayo et al.** *Proc. Natl. Acad. Sci. USA,* 1999, vol. 96, 2907-2912
- **Innis et al.** PCR: A Guide to Methods and Application. Academic Press, 1990
- **Brutlag et al.** *Comp. Chem.,* 1993, vol. 17, 203-207

- **Lukashin ; Borodovsky.** *Nucleic Acids Research,* 1998, vol. 26, 1107-1115
- **Burge ; Karlin.** *Journal of Molecular Biology,* 1997, vol. 268, 78-94
- **Salzberg et al.** *Nucleic Acids Research,* 1998, vol. 26, 544-548
- **Xu et al.** *Comput. Appl. Biosci.,* 1994, vol. 10, 613-623
- **Fuchs.** *Comput. Appl. Biosci.,* 1991, vol. 7, 105-106
- **Wilson, R.K. ; Mardis, E.R.** Genome Analysis: A Laboratory Manual. Cold Spring Harbor Press, 1997, vol. 1, 397-454
- **Margulies et al.** Genome sequencing in microfabricated high-density picolitre reactors. *Nature,* 2005, vol. 437, 376-380
- **Davis, R.W. ; Botstein, D. ; Roth, J.R.** Advanced Bacterial Genetics. Cold Spring Harbor Press, 1980
- **Berka, R.M. ; Schneider, P. ; Golightly, E.J. ; Brown, S.H. ; Madden, M. ; Brown, K.M. ; Halkier, T. ; Mondorf, K. ; Xu, F.** *Appl. Environ. Microbiol.,* 1997, vol. 63, 3151-3157
- **Gordon D. ; Abajian C. ; Green P.** *Genome Res.,* 1998, vol. 8, 195-202
- **Delcher, A,L. ; Harmon, D. ; Kasif, S. ; White, O. ; Salzberg, S.L.** *Nucleic Acids Res.,* 1999, vol. 27, 4636-4641
- **Tech M ; Morgenstern B ; Meinicke P.** *Nucleic Acids Res.,* 2006, vol. 34, W588-90
- **Bairoch A ; Apweiler R ; Wu CH ; Barker WC ; Boeckmann B ; Ferro S ; Gasteiger E ; Huang H ; Lopez R ; Magrane M.** The Universal Protein Resource [UniProt]. *Nucleic Acids Res.,* 2005, vol. 33, D154-159
- **Zdobnov, E.M. ; Apweiler, R.** *Bioinformatics,* 2001, vol. 17, 847-848
- **Bateman, A. ; Coin, L. ; Durbin, R. ; Finn, R.D. ; Hollich, V. ; Griffiths-Jones, S. ; Khanna, A. ; Sonnhammer, E.L. et al.** *Nucleic Acids Res.,* 2004, vol. 32, D138-D141
- **Haft, D.J. ; Selengut, J.D. ; White, O.** *Nucleic Acids Res.,* 2003, vol. 31, 371-373
- **Apweiler, R. ; Attwood, T.K. ; Bairock, A. ; Bateman, A. ; Birney, E. ; Biswas, M. ; Bucher, P. ; Cerutti, L. ; Corpet, F. ; Croning, M.D. et al.** *Nucleic Acids Res.,* 2001, vol. 29, 37-40
- **Nielsen, H. ; Engelbrecht, J. ; Brunak, S. ; von Heijne, G.** *Protein Engineering,* 1997, vol. 10, 1-6
- **Krogh, A. ; Larsson, B. ; Heijne, G. ; Sonnhammer, E.L.L.** *J. Mol. Biol.,* 2000, vol. 305, 567-580
- **Tatusov, R.L. ; Natale, D.A. ; Garkavtsev, I.V. ; Tatusova, T.A. ; Shankavarum, U.T. ; Rao, B.S. ; Kiryutin, B. ; Galperin, M.Y. ; Federova, N.D. ; Koonin, E.V.** The COG database: new developments in phylogenetic classification of proteins from complete genomes. *Nucleic Acids Res.,* 2001, vol. 29, 22-28
- **Tatusov, R.L. ; Koonin, E.V. ; Lipman, D.J.** *Science,* 1997, vol. 278, 631-637
- **Koonin, E.V. ; Galperin, M.Y.** Sequence - Evolution - Function: Computational Approaches in Comparative Genomics. Kluwer, 2002
- **Lowe, T.M. ; Eddy, S.R.** *Nucleic Acids Res.,* 1997, vol. 25, 955-964
- **Henrik Nielsen ; Jacob Engelbrecht ; Søren Brunak ; Gunnar von Heijne.** *Protein Engineering,* 1997, vol. 10, 1-6
- **J.L. Gardy ; M.R. Laird ; F. Chen ; S. Rey ; C.J. Walsh ; M. Ester ; F.S.L. Brinkman.** PSORTb v.2.0: expanded prediction of bacterial protein subcellular localization and insights gained from comparative proteome analysis. *Bioinformatics,* 2005, vol. 21, 617-623
- **A. Krogh ; B. Larsson ; G. von Heijne ; E.L.L. Sonnhammer.** *Journal of Molecular Biology,* 2000, vol. 305, 567-580
- **Jiang ; Mannervik.** *Protein Expression and Purification,* 1999, vol. 15, 92-98
- **Wong et al.** *J. Immunol.,* 1995, vol. 154, 3351-3358
- **Kaji, H. et al.** *J. Biochem.,* 1999, vol. 126, 769-775
- **Libertini ; Di Donato.** *Protein Engineering,* 1992, vol. 5, 821-825
- **Feng et al.** *Biochem.,* 2000, vol. 39, 15399-15409
- **Crombie et al.** *J. Mol. Biol.,* 1992, vol. 228, 7-12
- **Carlini ; Stephan.** *Genetics,* 2003, vol. 163, 239-243
- **K. Rutherford ; J. Parkhill ; J. Crook ; T. Horsnell ; P. Rice ; M-A. Rajandream ; B. Barrell.** Artemis: sequence visualisation and annotation. *Bioinformatics,* 2000, vol. 16, 944-945